# FASCICULE DE BREVET EUROPEEN

(11) **EP 1 888 579 B1**
(45) Date de publication et mention de la délivrance du brevet: **03.08.2011**
(21) Numéro de dépôt: 06755509.4
(22) Date de dépôt: 19.05.2006
(51) Int. Cl.: C07D 471/04

(54) **NOUVEAUX DERIVES DU FLUORENE, COMPOSITIONS LES CONTENANT ET UTILISATION**
NEUE FLUORENDERIVATE, ZUSAMMENSETZUNG MIT DIESEN DERIVATEN UND VERWENDUNG
NOVEL FLUORENE DERIVATIVES, COMPOSITION CONTAINING SAID DERIVATIVES AND THE USE THEREOF

(30) Priorité: 19.05.2005 FR 0505037
(43) Date de publication de la demande: 20.02.2008
(73) Titulaire: Aventis Pharma S.A., 92160 Antony (FR)
(72) Inventeur: MAILLIET, Patrick, F-94120 Fontenay Sous Bois (FR); BERTIN, Luc, F-91560 Crosnes (FR); GUYON, Thierry, F-91270 Vigneux Sur Seine (FR); THOMPSON, Fabienne, F-75012 Paris (FR); RUXER, Jean-Marie, F-92130 Issy Les Moulineaux (FR); PILORGE, Fabienne, F-77220 Tournan En Brie (FR); BENARD, Didier, F-95560 Montsoult (FR); MINOUX, Hervé, F-94320 Thiais (FR); CARREZ, Chantal, F-94320 Thiais (FR); GOULAOUIC, Hélène, F-75012 Paris (FR)
(74) Mandataire: Le Coupanec, Pascale A.M.P.
(86) Numéro de dépôt international: PCT/FR2006/001137
(87) Numéro de publication internationale: WO 2006/123061

(56) Documents cités:
- WO-A-2004/072080
- WO-A-2005/034950
- WALTER S ET AL: "Molecular chaperones--cellular machines for protein folding" ANGEWANDTE CHEMIE, WILEY-VCH, WEINHEIM, DE, vol. 41, no. 7, 2 avril 2002 (2002-04-02), pages 1098-1113, XP002337733 ISSN: 1433-7851
- JANIN, Y.L.: "Heat Shock Protein 90 Inhibitors. A Text Book Example of Medicinal Chemistry?" JOURNAL OF MEDICINAL CHEMISTRY, vol. 48, no. 24, 2005, pages 7503-7512, XP002373723

## Description

La présente invention concerne de nouveaux composés chimiques, dérivés du fluorène et plus particulièrement de nouveaux dérivés de 4-(benzimidazol-2-yl)fluorène ou de 4-(azabenzimidazol-2-yl)fluorène, des compositions qui les contiennent, et leur utilisation comme médicaments.

Plus particulièrement, l'invention concerne, selon un premier aspect, de nouveaux dérivés de 4-(benzimidazol-2-yl)fluorène ou de 4-(aza-benzimidazol-2-yl)fluorène présentant une activité anticancéreuse, et en particulier une activité inhibitrice de la protéine chaperone Hsp90, et plus particulièrement via l'inhibition de l'activité catalytique de type ATPasique de la protéine chaperone Hsp90.

### Protéines Chaperones :

Les chaperones moléculaires de la famille « Heat Schock Proteins » (HSPs), classées en fonction de leur masse moléculaire (Hsp27, Hsp70, Hsp90...), sont des éléments clés de l'équilibre entre la synthèse et la dégradation des protéines cellulaires, responsables du repliement correct des protéines. Elles jouent un rôle vital en réponse au stress cellulaire. Les HSPs, et en particulier Hsp90, sont également impliquées dans la régulation de diverses fonctions majeures de la cellule, via leur association avec diverses protéines clients impliquées dans la prolifération cellulaire ou l'apoptose (Jolly C. et Morimoto R.I., J. N. Cancer Inst. (2000), 92, 1564-72; Smith D.F. et al., Pharmacological Rev. (1998), 50, 493-513; Smith D.F. , Molecular Chaperones in the Cell, 165-178, Oxford University Press 2001).

Diverses pathologies humaines sont la conséquence d'un repliement incorrect de protéines clés, conduisant notamment à des maladies neurodégénératives suite à l'agrégation de certaines protéines comme dans les maladies d'Alzheimer et de Huntington ou les maladies liées aux prions (Tytell M. et Hooper P.L., Emerging Ther. Targets (2001), 5, 3788-3796 ;). Dans ces pathologies, des approches visant à inhiber Hsp90 dans le but d'activer les voies de stress (Hsp70 par exemple) pourraient être bénéfiques Chaperone Hsp90 et inhibiteurs d'Hsp 90 dans le traitement des cancers : La chaperone Hsp90, qui représente 1 à 2% du contenu protéique de la cellule a été récemment mise en évidence comme une cible particulièrement prometteuse en thérapie anticancéreuse (cf pour revue : Moloney A. et Workman P., Expert Opin. Biol. Ther. (2002), 2(1), 3-24 ; Choisis et al, Drug Discovery Today (2004), 9, 881-888). Cet intérêt tient en particulier aux interactions cytoplasmiques d'Hsp90 avec les principales protéines clients d'Hsp90, protéines qui sont impliquées dans les six mécanismes de progression des tumeurs, tels que définis par Hanahan D. et Weinberg R.A. (Cell (2002), 100, 57-70), à savoir :
- une capacité à proliférer en l'absence de facteurs de croissance : EGFR-R/HER2, Src, Akt, Raf, MEK, Bcr-Abl, Flt-3 ...
- une capacité à échapper à l'apoptose : forme mutée de p53, Akt, survivine
- une insensibilité aux signaux d'arrêt de prolifération : Cdk4, Plk , Wee1 ...
- une capacité à activer l'angiogénèse : VEGF-R, FAK, HIF-1, Akt ...
- une capacité à proliférer sans limite réplicative : hTert ...
- une capacité à envahir de nouveaux tissus et à métastaser : c-Met

Parmi les autres protéines clients de Hsp90, des récepteurs aux hormones stéroïdiennes, tels que le récepteur à l'estrogène ou le récepteur à l'androgène, présentent également un intérêt important dans le cadre des thérapies anticancéreuses.

II a été montré récemment que la forme alpha d'Hsp90 avait également un rôle extracellulaire via son interaction avec la métalloprotéase MMP-2, elle-même impliquée dans l'invasion tumorale (Eustace B.K. et al, Nature Cell Biology (2004), 6, 507-514.

Hsp90 est constituée de deux domaines N- et C-terminaux séparés par une région fortement chargée. L'interaction dynamique entre ces deux domaines, coordonnée par la fixation de nucléotides et de co-chaperones, détermine la conformation de la chaperone et son état d'activation. L'association des protéines clients dépend principalement de la nature des co-chaperones Hsp70/Hsp40, Hop60 etc ... et de la nature du nucléotide ADP ou ATP liée au domaine N-terminal de Hsp 90. Ainsi l'hydrolyse de l'ATP en ADP et le facteur d'échange ADP/ATP contrôlent l'ensemble de la « machinerie » chaperone, et il a été montré qu'il suffit de prévenir l'hydrolyse de l'ATP en ADP - activité ATPasique d'Hsp90 - pour libérer dans le cytoplasme des protéines-clients qui seront alors dégradées au protéasome (Neckers L et Neckers K, Expert Opin. Emerging Drugs (2002), 7, 277-288 ; Neckers L, Current Medicinal Chemistry, (2003), 10, 733-739 ; Piper P.W., Current Opin. lnvest. New Drugs (2001), 2, 1606-1610) .

### Rôle d'Hsp 90 et de ses inhibiteurs dans des pathologies autres que le cancer:

i) La maladie de Huntington : Cette maladie neurodégénérative est due à une extension de triplets CAG dans l'exon 1 du gène cofant pour la protéine huntingtin. Il a été montré que la geldanamycine inhibe l'aggrégation de cette protéine du fait de la surexpression des chaperones Hsp70 et Hsp40 (Human Molecular Genetics 10 : 1307, 2001).
ii) La maladie de Parkinson : Cette maladie est due à la perte progressive des neurones dopaminergiques et caractérisée par l'aggrégation de la protéine alpha-synuclein. II a été montré que la geldanamycine est capable de protéger la drosophile contre la toxicité de l'alpha-synuclein sur les neurones dopaminergiques.
iii) L'ischémie cérébrale focale : Il a été montré dans un modèle animal de rat que la geldanamycine protège le cerveau contre l'ischémie cérébrale, et ce du fait de l'effet de stimulation de la transcription des gènes codant pour les « heat-shock protéines » par un inhibiteur d'Hsp90.
iv) Les maladies d'Alzheimer et la sclérose en plaques : Ces maladies sont due en partie à l'expression de cytokines pro-inflammatoires et de la forme inductible de la NOS (Nitric oxide synthase) dans le cerveau, et cette expression délétaire est supprimée par la réponse au stress. En particulier, les inhibiteurs d'Hsp90 sont capables d'engranger cette réponse au stress, et il a été montré in vitro que la geldanamycine et le 17-AAG présentent une activité anti-inflammatoire dans des cellules gliales de cerveau (J. Neuroscience Res. 67 : 461, 2002).
v) La sclérose latérale amyotrophique : Cette maladie neurodégénérative est due à la perte progressive des neurones moteurs. II a été montré que l'arimoclomol, un inducteur des protéines de heat-shock, retarde l'évolution de la maladie dans un modèle animal (Nature Medicine 10: 402, 2004). Etant donné qu'un inhibiteur d'Hsp90 est également un inducteur des protéines heat-shock (Mol. Cell Biol. 19: 8033, 1999 ; Mol. Cell Biol. 18 : 4949, 1998), il est probable qu'un effet bénéfique pourrait être obtenu également dans cette pathologie pour ce type d'inhibiteurs.
   D'autre part, un inhibiteur de la protéine Hsp90 pourrait être potentiellement utile dans diverses maladies, autres que le cancer citées précedemment, telles que des infections parasitaires, virales, fongiques, ou des maladies neurodégénératives et ce par une action directe sur Hsp90 et des protéines clients particuliers. Quelques exemples sont présentés ci-dessous :
vi) la malaria : la protéine Hsp90 de Plasmodium falciparum présente 59% d'identité et 69% de similarité avec la protéine Hsp90 humaine, et il a été montré que la geldanamycine inhibe la croissance du parasite in vitro (Malaria Journal 2 : 30, 2003 ; J. Biol. Chem. 278 : 18336, 2003 ; J. Biol. Chem. 279: 46692, 2004).
vii) Les filarioses de Brugia et de Bancroft : ces parasites filaires lymphatiques possèdent une protéine Hsp90 pouvant potentiellement être inhibée par des inhibiteurs de la protéine humaine. En effet, il a été montré pour un autre parasite proche, le Brugia pahangi, que ce dernier est sensible à l'inhibition par la geldanamycine. Les séquences de B. pahangi et humaines sont identiques à 80% et similaires à 87%. (Int. J. for Parasitology 35: 627, 2005)
viii)La toxoplasmose : Toxoplasma gondii, le parasite responsable de la toxoplasmose, possède une protéine chaperone Hsp90, pour laquelle il a été montré l'induction au cours de la conversion tachyzoite-bradyzoite, correspondant au passage de l'infection chronique vers la toxoplasmose active. De plus, la geldanamycine bloque in vitro cette conversion tachyzoite-bradyzoite (J. Mol. Biol. 350 : 723, 2005)
ix) Les mycoses résistantes aux traitements : II est possible que la protéine Hsp90 potentialise l'évolution de la résistance aux drogues, en permettant à de nouvelles mutations de se développer. Par conséquent, un inhibiteur de Hsp90, seul ou en combinaison avec un autre traitement antifongique, pourrait se révéler utile dans le traitement de certaines souches résistantes (Science 309: 2185, 2005). De plus, l'anticorps anti-Hsp90 développé par Neu Tec Pharma démontre une activité contre C ; albicans, sensible et résistant au fluconazole, C. krusei, C. tropicalis, C. glabrata, C. lusitaniae et C. parapsilosis in vivo (Current Molecular Medicine 5: 403, 2005).
x) L'hépatite B : Hsp90 est l'une des protéines de l'hôte qui interagit avec la transcriptase inverse du virus de l'hépatite B au cours du cycle de réplication du virus. II a été montré que la geldanamycine inhibe la réplication de l'ADN viral et l'encapsulation de l'ARN viral (Proc. Natl. Acad. Sci. USA 93: 1060, 1996)
xi) L'hépatite C : La protéine Hsp90 humaine participe à l'étape de clivage entre les protéines NS2 et NS3 par la protéase virale. La geldanamycine et le radicicol sont capables d'inhiber ce clivage NS2/3 in vitro (Proc. Natl. Acad. Sci. USA 98 : 13931, 2001)
xii) Le virus de l'Herpes : La geldanamycine a démontré des activités d'inhibition de la réplication du virus HSV-1 in vitro, et ce avec un bon index thérapeutique (Antimicrobial Agents and Chemotherapy 48 : 867, 2004). Les auteurs ont également trouvé une activité de la geldanamycine sur les autres virus HSV-2, VSV, Cox B3, HIV-1 et le coronavirus SARS (données non montrées).
xiii) La dengue (ou grippe tropicale) : II a été montré que la protéine humaine Hsp90 participe à l'étape d'entrée du virus, en formant un complexe contenant également Hsp70 qui sert de récepteur au virus ; Un anticorps anti-Hsp90 diminue le pouvoir infectieux du virus in vitro (J. of Virology 79 : 4557, 2005)
xiv) L'atrophie musculaire spinale et bulbaire: (SBMA : Spinal and bulbar muscular atrophy), une maladie neurodégénérative héréditaire caractérisée par une extension de triplets CAG dans le gène du récepteur aux androgènes. II a été montré que le 17 -AAG, un dérivé de la geldanamycine, présente une activité in vivo sur des animaux transgéniques servant de modèles expérimentaux de cette maladie (Nature Medicine 11 : 1088, 2005).

### Inhibiteurs d'Hsp90 :

Les premiers inhibiteurs connus d'Hsp90 sont des composés de la famille des amsamycines, en particulier la Geldanamycine (1) et l'Herbimycine A. Des études de rayon X ont montré que la Geldanamycine se lie au site ATP du domaine N-terminal d'Hsp90 où elle inhibe l'activité ATPasique de la chaperone (Prodromou C. et al, Cell (1997), 90, 65-75)

Actuellement le NIH et Kosan BioSciences assurent le développement clinique du 17AAG (2), qui est un inhibiteur d'Hsp90 dérivé de la geldanamycine (1), qui bloque l'activité ATPasique d'Hsp 90, en se liant au site de reconnaissance de N-terminal de l'ATP. Les résultats des essais cliniques de phase I du 17AAG (1) conduisent aujourd'hui à initier des essais de phase II, mais orientent aussi les recherches vers des dérivés plus solubles tel que l'analogue (3) (17DMAG de Kosan BioSciences), porteur d'une chaîne diméthylaminée à la place du résidu méthoxy, et vers des formulations optimisées du 17AAG (CF1010 de Conforma Therapeutics) :

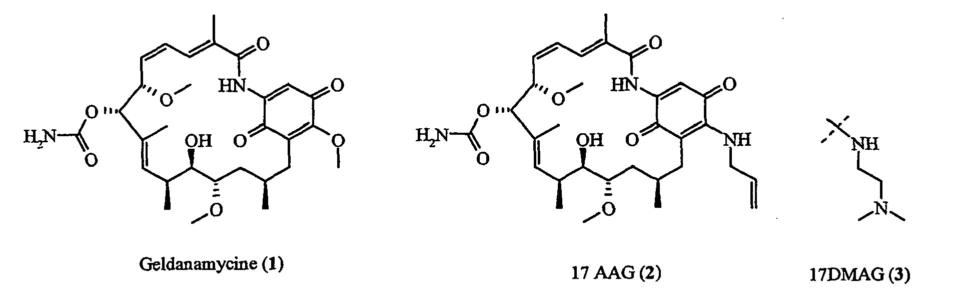

Le radicicol (4) est également un inhibiteur d'Hsp 90 d'origine naturelle (Roe S.M. et al, J. Med Chem. (1999), 42, 260-66). Toutefois, si celui-ci est de loin le meilleur inhibiteur in vitro d'Hsp90, son instabilité métabolique vis à vis des nucléophiles souffrés le rend difficilement utilisable in vivo. Des dérivés oximes bien plus stables tel que le KF 55823 (5) ou le KF 25706 ont été développés par la société Kyowa Hakko Kogyo (Soga et al, Cancer Research (1999), 59, 2931-2938)

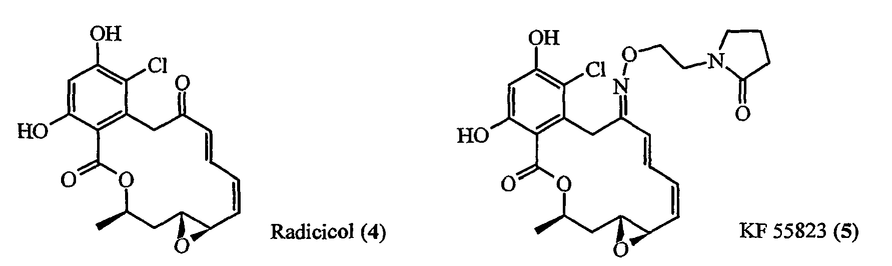

Des structures d'origine naturelle apparentées au radicicol ont également été récemment décrites, comme la zéaralénone (6) par la société Conforma Therapeutics (WO 03041643) ou les composés (7-9).

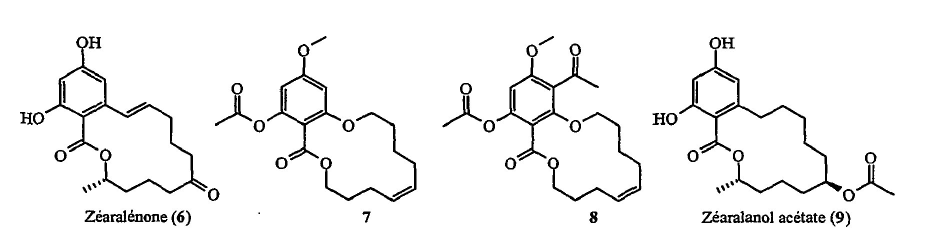

Un inhibiteur d'Hsp90 d'origine naturelle, la novobiocine (10) se lie à un site ATP différent situé dans le domaine C-terminal de la protéine (Itoh H. et al, Biochem J. (1999), 343, 697-703).

Un depsipeptide, nommé Pipalamycin ou IC1101 vient d'être récemment 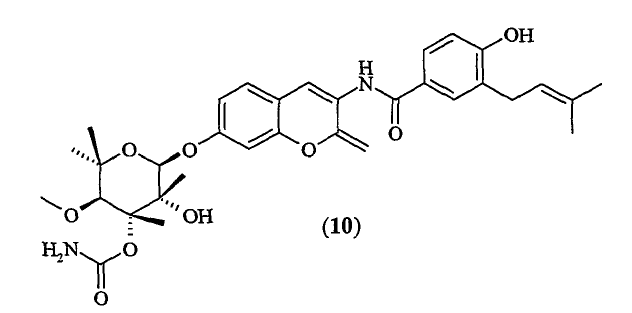 décrit comme inhibiteur non compétitif du site ATP d'Hsp90 (J. Pharmacol. Exp. Ther. (2004), 310, 1288-1295).

Des purines, comme les composés PU3 (11) (Chiosis et al, Chem. Biol. (2001), 8, 289-299) et PU24FCl (12) (Chiosis et al, Curr. Canc. Drug Targets (2003), 3, 371-376) ont été également été décrites comme inhibiteurs d'Hsp90 :

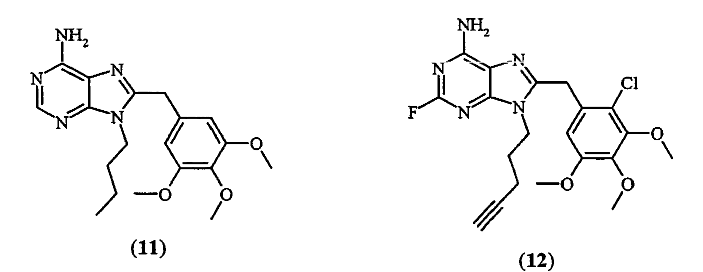

La demande de brevet WO2004/072080 (Cellular Genomics) revendique une famille de 8-hétéroaryl-6-phényl-imidazo[1,2-a]pyrazines comme modulateurs de l'activité d'hsp90.

La demande de brevet WO2004/050087 (Ribotarget/Vernalis) revendique une famille de pyrazoles utiles pour traiter des pathologies liées à l'inhibition des « Heat Shock Proteins » tels que la chaperone Hsp90.

La demande de brevet WO2004/056782 (Vernalis) revendique une nouvelle famille de pyrazoles utiles pour traiter des pathologies liées à l'inhibition des « Heat Shock Proteins » tels que la chaperone Hsp90.

La demande de brevet WO2004/07051 (Vernalis) revendique des dérivés d'arylisoxazoles utiles pour traiter des pathologies liées à l'inhibition des « Heat Shock Proteins » tels que la chaperone Hsp90.

La demande de brevet WO2004/096212 (Vernalis) revendique une troisième famille de pyrazoles utiles pour traiter des pathologies liées à l'inhibition des « Heat Shock Proteins » tels que la chaperone Hsp90.

La demande de brevet WO2005/00300 (Vernalis) revendique de manière plus générale des hétérocycles à 5 chaînons, substitués par des radicaux aryles, utiles pour traiter des pathologies liées à l'inhibition des « Heat Shock Proteins » tels que la chaperone Hsp90.

La demande de brevet WO2005/00778 (Kyowa Hakko Kogyo) revendique une famille de dérivés de benzophénone comme inhibiteurs d'HsP90, utiles pour le traitement des tumeurs.

La demande de brevet WO2005/215528 (Vernalis) revendique une famille de dérivés de pyrimidothiophène, utiles pour traiter des pathologies liées à l'inhibition des « Heat Shock Proteins » tels que la chaperone Hsp90.

European Journal of Medicinal Chemistry 1988, 23(2), 165-172 décrit la préparation et l'activité inhibitrice d'aldole réductase du composé CAS 116792-62-2 ci-dessous :

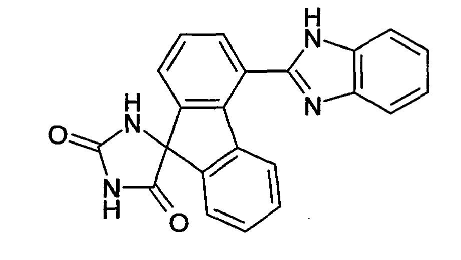

La présente invention concerne des produits de formule (I) : 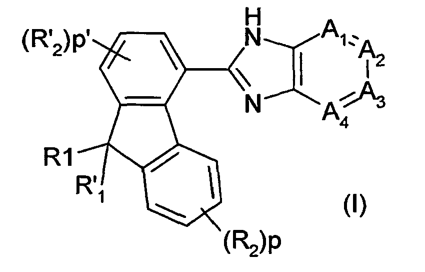 dans laquelle :
A1, A2, A3 et A4, identiques ou différents, représentent CRa ou N ou NRb; avec Rb représente alkyle, alcoxy ou OH,
R1 et R1' sont tels que : soit R1 et R1' identiques ou différents sont tels que l'un de R1 et R1' représente un atome d'hydrogène ou d'halogène ou un radical choisi parmi C1C3-alkyle, C1C3-alcoxy, alkyl-OH, CF3, cyano, carboxy et carboxamido ;
et l'autre de R1 et R1' est choisi dans le groupe constitué par H ; halogène ; CF3 ; hydroxyle ; mercapto ; nitro ; amino ; NH-OH ; NH-CO-H ; NH-CO-OH, NH-CO-Oalkyle , NH-CO-NH2 ; carboxy ; CN ; CO-NH2 ; X-(CH2)m-alkyle; X-(CH2)m-cycloalkyle; X-(CH2)m-hétérocycloalkyle ; X-(CH2)m-aryle ou X-(CH2)m-hétéroaryle avec X = simple liaison, CH2, CH=CH, CH2-O CH2-NH, . CH2-C(O), CH2-C(O)-O, CH2-C(O)-NH, CH2-NH-(CO), CH2-NH-S(O), CH2-NH-S(O)2, O, S, NH, O-C(O), C(O)-NH, -NH-C(O), -NH-C(O)-C(O)-, -NH-C(O)-NH-, ; NH-CS, NH-S(O) ou NH-S(O)2, -NH-CO-CH2-O- ; -NH-CO-CH2-S-CH2-CO-NH- ; -NH-CO-(CH2)2-S02- ; -NH-CO-CH2-N(CH3)-CO- ; avec m = 0, 1 ou 2, tous les radicaux alkyle, cycloalkyle, hétérocycloalkyle, aryle et hétéroaryle étant éventuellement substitués, le radical cycloalkyle contenant de 3 à 10 chainons, le radical aryle contenant de 6 à 10 chainons, et les radicaux hétérocycloalkyle et hétéroaryle, éventuellement substitués, contenant de 4 à 10 chainons dont 1 à 4 hétéroatomes choisis parmi O, S, N ou NR3 avec R3 représente H ou alkyle lui-même éventuellement substitué, soit R1 et R'1 forment ensemble avec l'atome de carbone auquels ils sont liés un radical =O ; =S ; =N-OH ; =N-NH2 ; =N-NH-CO-NH2, =CH-OH ; =Y1-(CH2)m-aryle ou =Y1-(CH2)m -hétéroaryle, dans lequel Y1 représente CH, CH-CO-, CH-CO-NH, N, N-O ou N-NH-, avec m = 0, 1 ou 2 et dans lesquels aryle et hétéroaryle sont tels que définis ci-dessus et éventuellement substitués,
soit R1 et R'1 forment ensemble avec l'atome de carbone auquels ils sont liés un cycle partiellement saturé constitué de 4 à 6 chainons et contenant éventuellement de 1 à 3 hétéroatomes choisis parmi O, S, N ou NR4 avec R4 représente H ou alkyle lui-même éventuellement substitué,
R2 et R'2, identiques ou différents, sont indépendamment sélectionnés dans le groupe constitué par H, halogène, CF3, nitro, cyano, alkyle, hydroxy, mercapto, amino, alkylamino, dialkylamino, alkoxy, alkylthio (méthylthio), carboxy libre ou estérifié par un radical alkyle , carboxamide, CO-NH(alkyl) et CO N(alkyl)2, tous les radicaux alkyle, alcoxy et alkylthio étant éventuellement substitués,
p et p' identiques ou différents représentent respectivement les entiers 1 à 4 et 1 à 3 ;
Ra est sélectionné dans le groupe constitué par H ; halogène ; CF3 ; hydroxy; OCF3; S02-NH2 ; SO2-NH(alk), SO2-N(alk)2 ; mercapto ; nitro ; amino ; NH(alk); N(alk)2; NH-OH ; NH-CO-H ; NH-CO-NH2 ; carboxy libre ou estérifié par un radical alkyle lui-même éventuellement substitué; CN; CO-NH2 ; Y-(CH2)n-alkyle ; Y-(CH2)n-cycloalkyle, Y-(CH2)n-hétérocycloalkyle, Y-(CH2)n-aryle ou Y-(CH2)n-hétéroaryle, avec Y représente une simple liaison ou bien = O, S, NH, O-C(O), C(O)-NH, -C(O)N(CH3)- ; CO ; NH-C(O), NH-S(O) ou NH-S(O)2, avec n = 0, 1, 2, ou 3 radicaux dans lesquels les radicaux alkyle, cycloalkyle, hétérocycloalkyle, aryle et hétéroaryle sont éventuellement substitués, le radical cycloalkyle contient de 3 à 10 chainons, le radical aryle contient de 6 à 10 chainons, et les radicaux hétérocycloalkyle et hétéroaryle, éventuellement substitué, contiennent de 4 à 10 chainons dont 1 à 4 hétéroatomes choisis parmi O, S, N ou NR3 avec R3 représente H ou alkyle lui-même éventuellement substitué,
tous les radicaux alkyle, alcoxy, alkylthio, cycloalkyle, hétérocycloalkyle, aryle et hétéroaryle des substituants des produits de formule (I) étant éventuellement substitués,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

La présente invention concerne ainsi des produits de formule (I) dans lesquels
A1, A2, A3 et A4, identiques ou différents, représentent CRa ou N ou NRb avec Rb représente CH3 ou OH ;
Ra est sélectionné dans le groupe constitué par H ;halogène ; CF3 ; hydroxy ; OCF3; SO2-NH2 ; SO2-NHCH3, SO2-N(CH3)2; mercapto ; nitro; amino ; NH(CH3); N(CH3)2; NH-OH ; NH-CO-H ; NH-CO-NH2; carboxy libre ou estérifié par un radical alkyle lui-même éventuellement substitué; CO2-CH3 ; CO2-(CH2)3-N(CH3)2 ; CN ; CO-NH2 ; Y-(CH2)n-alkyle ; Y-(CH2)n-cycloalkyle, Y-(CH2)n-hétérocycloalkyle, Y-(CH2)n-aryle ou Y-(CH2)n-hétéroaryle, avec Y représente une simple liaison ou bien = O, -C(O)-NH-, - C(O)N(CH3)- ; CO, avec n = 0, 1, 2, ou 3 radicaux dans lesquels les radicaux alkyle, cycloalkyle, hétérocycloalkyle, aryle et hétéroaryle sont éventuellement substitués, le radical cycloalkyle contient de 3 à 10 chainons, le radical aryle contient de 6 à 10 chainons, et les radicaux hétérocycloalkyle et hétéroaryle, éventuellement substitué, contiennent de 4 à 10 chainons dont 1 à 4 hétéroatomes choisis parmi O, S, N ou NR3 avec R3 représente H ou alkyle lui-même éventuellement substitué,
les autres substituants R1,R1',R2,R2' desdits produits de formule (I) étant choisis parmi l'une quelconque des définitions ci-dessus,
tous les radicaux alkyle, alcoxy, alkylthio, cycloalkyle, hétérocycloalkyle, aryle et hétéroaryle des substituants des produits de formule (I) étant éventuellement substitués,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

La présente invention concerne des produits de formule (I) tels que définis ci-dessus dans laquelle :
A1, A2, A3 et A4, identiques ou différents, représentent CRa ou N ;
R1 et R1' sont tels que :
   soit R1 et R1' identiques ou différents sont tels que l'un de R1 et R1' représente un atome d'hydrogène ou d'halogène ou un radical choisi parmi C1C3-alkyle, C1C3-alcoxy, alkyl-OH, CF3, cyano, carboxy et carboxamido;
   et l'autre de R1 et R1' est choisi dans le groupe constitué par H ; halogène ; CF3 ; hydroxyle ; mercapto ; nitro ; amino ; NH-OH ; NH-CO-H ; NH-CO-OH, NH-CO-Oalkyle , NH-CO-NH2 ; carboxy ; CN ; CO-NH2 ; X-(CH2)m-alkyle; X-(CH2)m-cycloalkyle; X-(CH2)m-hétérocycloalkyle; X-(CH2)m-aryle ou X-(CH2)m-hétéroaryle avec X = simple liaison, CH2, CH=CH, CH2-O, CH2-NH, CH2-C(O), CH2-C(O)-O, CH2-C(O)-NH, CH2-NH-(CO), CH2-NH-S(O), CH2-NH-S(O)2, O, S, NH, O-C(O), C(O)-NH, -NH-C(O), -NH-C(O)-C(O)-, -NH-C(O)-NH-, ; NH-CS, NH-S(O) ou NH-S(O)2, avec m = 0, 1 ou 2, tous les radicaux alkyle, cycloalkyle, hétérocycloalkyle, aryle et hétéroaryle étant éventuellement substitués, le radical cycloalkyle contenant de 3 à 10 chainons, le radical aryle contenant de 6 à 10 chainons, et les radicaux hétérocycloalkyle et hétéroaryle, éventuellement substitués, contenant de 4 à 10 chainons dont 1 à 4 hétéroatomes choisis parmi O, S, N ou NR3 avec R3 représente H ou alkyle lui-même éventuellement substitué,
   soit R1 et R'1 forment ensemble avec l'atome de carbone auquels ils sont liés un radical =O ; =S ; =N-OH ; =N-NH2 ; =N-NH-CO-NH2, =CH-OH ; =Y1-(CH2)m-aryle ou =Y1-(CH2)m -hétéroaryle, dans lequel Y1 représente CH, CH-CO-, CH-CO-NH, N, N-O ou N-NH-, avec m = 0, 1 ou 2 et dans lesquels aryle et hétéroaryle sont tels que définis ci-dessus et éventuellement substitués,
   soit R1 et R'1 forment ensemble avec l'atome de carbone auquels ils sont liés un cycle partiellement saturé constitué de 4 à 6 chainons et contenant éventuellement de 1 à 3 hétéroatomes choisis parmi O, S, N ou NR4 avec R4 représente H ou alkyle lui-même éventuellement substitué,
   R2 et R'2, identiques ou différents, sont indépendamment sélectionnés dans le groupe constitué par H, halogène, CF3, nitro, cyano, alkyle, hydroxy, mercapto, amino, alkylamino, dialkylamino, alkoxy, alkylthio, carboxy libre ou estérifié, carboxamide, CO-NH(alkyl) et CO N(alkyl)2,
   p et p' identiques ou différents représentent respectivement les entiers 1 à 4 et 1 à 3 ;
   Ra est sélectionné dans le groupe constitué par H ; halogène ; CF3 ; hydroxy ; mercapto ; nitro ; amino ; NH-OH ; NH-CO-H ; NH-CO-NH2 ; carboxy ; CN ; CO-NH2 ; Y-(CH2)n-alkyle ; Y-(CH2)n-cycloalkyle, Y-(CH2)n-hétérocycloalkyle, Y-(CH2)n-aryle ou Y-(CH2)n-hétéroaryle, avec Y = O, S, NH, O-C(O), C(O)-NH, NH-C(O), NH-S(O) ou NH-S(O)2, avec n = 0, 1, 2, ou 3, radicaux dans lesquels les radicaux alkyle, cycloalkyle, hétérocycloalkyle, aryle et hétéroaryle sont éventuellement substitués, le radical cycloalkyle contient de 3 à 10 chainons, le radical aryle contient de 6 à 10 chainons, et les radicaux hétérocycloalkyle et hétéroaryle, éventuellement substitué, contiennent de 4 à 10 chainons dont 1 à 4 hétéroatomes choisis parmi O, S, N ou NR3 avec R3 représente H ou alkyle lui-même éventuellement substitué,
   tous les radicaux alkyle, alcoxy, alkylthio, cycloalkyle, hétérocycloalkyle, aryle et hétéroaryle des substituants des produits de formule (I) étant éventuellement substitués,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

Dans les produits de formule (I) et dans ce qui suit, les termes indiqués ont les significations qui suivent :
- le terme halogène désigne les atomes de fluor, de chlore, de brome ou d'iode et de préférence de fluor, chlore ou brome.
- le terme radical alkyle désigne un radical linéaire ou ramifié renfermant au plus 12 atomes de carbone choisi parmi les radicaux méthyle, éthyle, propyle, isopropyle, butyle, isobutyle, sec-butyle, tert-butyle, pentyle, isopentyle, sec-pentyle, tert-pentyle, néo-pentyle, hexyle, isohexyle, sec-hexyle, tert-hexyle et également heptyle, octyle, nonyle, décyle, undécyle et dodécyle, ainsi que leurs isomères de position linéaires ou ramifiés. On cite plus particulièrement les radicaux alkyle ayant au plus 6 atomes de carbone et notamment les radicaux méthyle, éthyle, propyle, isopropyle, n-butyle, isobutyle, terbutyle, pentyle linéaire ou ramifié, hexyle linéaires ou ramifiés.
- le terme radical alkényle désigne un radical linéaire ou ramifié renfermant au plus 12 atomes de carbone et préférentiellement 4 atomes de carbone choisi par exemple parmi les valeurs suivantes: éthényle ou vinyle, propényl ou allyle, 1-propényle, n-butényle, i-butényle, 3-méthylbut-2-ényle, n-pentényle, hexényle, heptényle, octényle, cyclohexylbutényle et décényle ainsi que leurs isomères de position linéaires ou ramifiés. Parmi les valeurs alkényle, on cite plus particulièrement les valeurs allyle ou butényle.
- le terme radical alkynyle désigne un radical linéaire ou ramifié renfermant au plus 12 atomes de carbone et préférentiellement 4 atomes de carbone choisi

par exemple parmi les valeurs suivantes: éthynyle, propynyle ou propargyle, butynyle, n-butynyle, i-butynyle, 3-méthylbut-2-ynyle, pentynyle ou hexynyle ainsi que leurs isomères de position linéaires ou ramifiés. Parmi les valeurs alkynyle, on cite plus particulièrement la valeur propargyle.
- le terme radical alcoxy désigne un radical linéaire ou ramifié renfermant au plus 12 atomes de carbone et préférentiellement 6 atomes de carbone choisi par exemple parmi les radicaux méthoxy, éthoxy, propoxy, isopropoxy, butoxy linéaire, secondaire ou tertiaire, pentoxy, hexoxy et heptoxy ainsi que leurs isomères de position linéaires ou ramifiés,
- Le terme alkylthio ou alkyl-S- désigne un radical linéaire ou ramifié renfermant au plus 12 atomes de carbone et représente notamment les radicaux méthylthio, éthylthio, isopropylthio et heptylthio. Dans les radicaux comportant un atome de soufre, l'atome de soufre peut être oxydé en radical SO ou S(O)2.
- le terme radical acyle ou r-CO- désigne un radical linéaire ou ramifié renfermant au plus 12 atomes de carbone dans lequel le radical r représente un atome d'hydrogène, un radical alkyle, cycloalkyle, cycloalkényle, cycloalkyle, hétérocycloalkyle ou aryle, ces radicaux ayant les valeurs indiquées ci-dessus et étant éventuellement substitués comme indiqué : on cite par exemple les radicaux formyle, acétyle, propionyle, butyryle ou benzoyle, ou encore valéryle, hexanoyle, acryloyle, crotonoyle ou carbamoyle
- le terme radical cycloalkyle désigne un radical carbocyclique monocyclique ou bicyclique renfermant de 3 à 10 chaînons et désigne notamment les radicaux cyclopropyle, cyclobutyle, cyclopentyle et cyclohexyle,
- le terme radical cycloalkylalkyle désigne un radical dans lequel cycloalkyle et alkyle sont choisis parmi les valeurs indiquées ci-dessus : ce radical désigne ainsi par exemple les radicaux cyclopropylméthyle, cyclopentylméthyle, cyclohexylméthyle et cycloheptylméthyle.
- par radical acyloxy, on entend les radicaux acyl-O- dans lesquels acyl a la signification indiquée ci-dessus : on cite par exemple les radicaux acétoxy ou propionyloxy.
- par radical acylamino, on entend les radicaux acyl-N- dans lesquels acyl a la signification indiquée ci-dessus.
- le terme radical aryle désigne les radicaux insaturés, monocycliques ou constitués de cycles condensés, carbocycliques. Comme exemples de tel radical aryle, on peut citer les radicaux phényle ou naphtyle.
- Par arylalkyle on entend les radicaux résultant de la combinaison des radicaux alkyle cités précédemment éventuellement substitués et les radicaux aryles également cités ci-dessus, éventuellement substitués : on cite par exemple les radicaux benzyle, phényléthyle, 2-phénéthyle, triphénylméthyle ou naphthlèneméthyle.
- le terme radical hétérocyclique désigne un radical carbocylique saturé (hétérocycloalkyle) ou partiellement ou totalement insaturé (hétéroaryle), monocyclique ou bicyclique, constitué de 4 à 10 chaînons interrompus par un ou plusieurs hétéroatomes, identiques ou différents, choisis parmi les atomes d'oxygène, d'azote ou de soufre.

Comme radicaux hétérocycloalkyles, on peut citer notamment les radicaux dioxolane, dioxane, dithiolane, thiooxolane, thiooxane, oxirannyle, oxolannyle, dioxolannyle, pipérazinyle, pipéridyle, pyrrolidinyle, imidazolidinyle, diazépinyl, imidazolidine-2,4-dione, pyrazolidinyle, morpholinyle ou encore tétrahydrofuryle, hexahydropyranne , tétrahydrothiényle, chromanyle, dihydrobenzofuranyle, indolinyle, perhydropyranyle, pyrindolinyle, tétrahydroquinoléinyle, tétrahydroisoquinoléinyle ou thioazolidinyle, tous ces radicaux étant éventuellement substitués.

Parmi les radicaux hétérocycloalkyles, on peut citer notamment les radicaux pipérazinyle éventuellement substitué, N-méthylpipérazinyle, pipéridyle, éventuellement substitués, pyrrolidinyle éventuellement substitué, imidazolidinyle, pyrazolidinyle, morpholinyle, hexahydropyranne ou thioazolidinyle.

Par radical hétérocycloalkylalkyle, on entend les radicaux dans lesquels les restes hétérocycloalkyle et alkyle ont les significations précédentes parmi les radicaux hétéroaryles à 5 chaînons on peut citer les radicaux 2-furyle, 3-furyle, 2-pyrrolyle, 3-pyrrolyle, tétrazolyle, thiazolyle, isothiazolyle, diazolyle, thiadiazolyle, 1,3,4-thiadiazolyle, thiatriazolyle, oxazolyle, oxadiazolyle, isoxazolyle, 3-isoxazolyle, 4-isoxazolyle, imidazolyle, pyrazolyle, thiényle, 2-thiényle, 3-thiényle, groupes triazolyle,

Parmi les radicaux hétéroaryles à 6 chaînons on peut citer notamment les radicaux pyridyle tel que 2-pyridyle, 3-pyridyle et 4-pyridyle, pyrimidyle tel que par exemple 2-pyrimidyle, pyrimidinyle tel que par exemple 4-pyrimidinyle, 5-pyrimidinyle, pyridazinyle, pyrazinyle tel que par exemple 3-pyrazinyle, 4-pyrazinyle.

Comme radicaux hétéroaryles condensés contenant au moins un hétéroatome choisi parmi le soufre, l'azote et l'oxygène, on peut citer par exemple benzothiényle tel que 3-benzothiényle, benzofuryle, benzofurannyle, benzopyrrolyle, benzimidazolyle, benzoxazolyle, thionaphtyle, indolyle, purinyle, quinolyle, isoquinolyle, azaindolyle et naphtyridinyle.

Parmi les radicaux hétéroaryles condensés, on peut citer plus particulièrement les radicaux benzothiényle, benzofurannyle, indolyle, benzimidazolyle, benzothiazolyle, naphtyridinyle, indazolyle, quinolyle tel que 4-quinolyl, 5-quinolyl, isoquinolyle, azaindolyle tel que 4-azaindolyl, 3 azaindolyl, imidazo(4,5)pyridine, chroményle, indolizinyle, quinazolinyle, ces radicaux étant éventuellement substitués comme indiqué pour les radicaux hétéroaryles.

Par radical alkylamino, on entend les radicaux dans lesquels le radical alkyl est choisi parmi les radicaux alkyle cités ci-dessus . On préfère les radicaux alkyle ayant au plus 4 atomes de carbone et on peut citer par exemple les radicaux. méthylamino, éthylamino, propylamino ou butylamino, linéaire ou ramifié.

Par radical dialkylamino, on entend les radicaux dans lesquels les radicaux alkyl identiques ou différents sont choisis parmi les radicaux alkyle cités ci-dessus. Comme précédemment on préfère les radicaux alkyle ayant au plus 4 atomes de carbone et on peut citer par exemple les radicaux diméthylamino, diéthylamino, méthyléthylamino linéaire ou ramifié.

Le terme amine cyclique désigne un radical monocyclique ou bicyclique renfermant de 3 à 10 chaînons dans lequel au moins un atome de carbone est remplacé par un atome d'azote, ce radical cyclique pouvant renfermer également un ou plusieurs autres hétéroatomes choisi(s) parmi O, S, SO2, N ou NR3 avec R3 tel que défini ci-dessus : comme exemples de telles amines cycliques, on peut citer par exemple les radicaux pyrrolyle, imidazolyle, indolyle, pipéridyle, morpholinyle, pipérazinyle, pyrrolidinyle, indolinyle, pyrindolinyle ou tétrahydroquinolyle. On préfère les radicaux pipéridinyle, morpholinyle ou pipérazinyle.

Le terme patient désigne les êtres humains mais aussi les autres mammifères.

Le terme "Prodrug" désigne un produit qui peut être transformé in vivo par des mécanismes métaboliques (tel que l'hydrolyse) en un produit de formule (I). Par exemple, un ester d'un produit de formule (I) contenant un groupe hydroxyle peut être converti par hydrolyse in vivo en sa molécule mère. Ou encore un ester d'un produit de formule (I) contenant un groupe carboxy peut être converti par hydrolyse in vivo en sa molécule mère.

On peut citer à titre d'exemples des esters de produits de formule (1) contenant un groupe hydroxyle tels que les acétates, citrates, lactates, tartrates, malonates, oxalates, salicylates, propionates, succinates, fumarates, maléates, méthylene-bis-b-hydroxynaphthoates, gentisates, iséthionates, di-p-toluoyltartrates, méthanesulfonates, éthanesulfonates, benzenesulfonates, p-toluènesulfonates, cyclohexylsulfamates et quinates. Des esters de produits de formule (I) particulièrement utiles contenant un groupe hydroxyle peuvent être préparés à partir de restes acides tels que ceux décrits par Bundgaard et. al., J. Med. Chem., 1989, 32, page 2503-2507: ces esters incluent notamment des (aminométhyl)-benzoates substitués, dialkylamino-méthylbenzoates dans lesquels les deux groupements alkyle peuvent être liés ensemble ou peuvent être interrompus par un atome d'oxygène ou par un atome d'azote éventuellement substitué soit un atome d'azote alkylé ou encore des morpholino-méthyl)benzoates, e.g. 3- ou 4-(morpholinométhyl)-benzoates, et (4-alkylpiperazin-1-yl)benzoates, e.g. 3- ou 4-(4-alkylpiperazin-1-yl)benzoates.

Le ou les radicaux carboxy des produits de formule (I) peuvent être salifiés ou estérifiés par les groupements divers connus de l'homme du métier parmi lesquels on peut citer, à titre d'exemples non limitatifs, les composés suivants.
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxy-carbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxy-méthyle, propionyloxyméthyle, méthylthiométhyle, diméthyl-aminoéthyle, benzyle ou phénéthyle.

Par carboxy estérifié on entend par exemple les radicaux tels que les radicaux alkyloxycarbonyle par exemple méthoxycarbonyle, éthoxycarbonyle, propoxycarbonyle, butyl ou tert-butyloxycarbonyle, cyclobutyloxycarbonyle, cyclopentyloxycarbonyle ou cyclohexyloxycarbonyle.

On peut également citer des radicaux formés avec les restes esters facilement clivables tels que les radicaux méthoxyméthyle, éthoxyméthyle ; les radicaux acyloxyalkyle tels que pivaloyloxyméthyle, pivaloyloxyéthyle, acétoxyméthyle ou acétoxyéthyle ; les radicaux alkyloxycarbonyloxy alkyle tels que les radicaux méthoxycarbonyloxy méthyle ou éthyle, les radicaux isopropyloxycarbonyloxy méthyle ou éthyle.

Une liste de tels radicaux esters peut-être trouvée par exemple dans le brevet européen EP 0 034 536.

Par carboxy amidifié, on entend les radicaux du type -CONH2 dont les atomes d'hydrogène sont éventuellement substitués par un ou deux radicaux alkyle pour former des radicaux alkylamino ou dialkylamino eux-mêmes éventuellement substitués comme indiqué ci-dessus ou ci-dessous, ces radicaux pouvant également former avec l'atome d'azote auquel ils sont liés une amine cyclique tel que définie ci-dessus.

Par carboxy salifié on entend les sels formés par exemple avec un équivalent de sodium, de potassium, de lithium, de calcium, de magnésium ou d'ammonium. On peut également citer les sels formés avec les bases organiques telles que la méthylamine, la propylamine, la triméthylamine, la diéthylamine, la triéthylamine. On préfère le sel de sodium.

Lorsque les produits de formule (I) comportent un radical amino salifiable par un acide il est bien entendu que ces sels d'acides font également partie de l'invention. On peut citer les sels fournis avec les acides chlorhydrique ou méthanesulfonique par exemple.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

On peut rappeler que la stéréoisomérie peut être définie dans son sens large comme l'isomérie de composés ayant mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomère est utilisé dans la présente demande dans son sens le plus large et concerne donc l'ensemble des composés indiqués ci-dessus.

La présente invention a notamment pour objet les produits de formule (I) ci-dessus dans lesquels
A1, A2, A3 et A4, identiques ou différents, représentent CRa ou N ;
Ra est sélectionné dans le groupe constitué par H ; halogène ; hydroxy ; mercapto; amino; Y-(CH2)n-alkyle ; Y-(CH2)n-cycloalkyle, Y-(CH2)n-hétérocycloalkyle, Y-(CH2)n-aryle ou Y-(CH2)n-hétéroaryle, avec Y = O, avec n = 0, 1, 2, ou 3, radicaux dans lesquels tous les radicaux alkyle, cycloalkyle, hétérocycloalkyle, aryle et hétéroaryle sont éventuellement substitués et les radicaux hétérocycloalkyle et hétéroaryle contiennent de 4 à 10 chainons dont 1 à 4 hétéroatomes choisis parmi O, S, N ou NR3 avec R3 représente H ou alkyle lui-même éventuellement substitué,
les autres substituants R1,R1',R2,R2' desdits produits de formule (I) étant choisis parmi l'une quelconque des définitions ci-dessus,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

La présente invention a notamment pour objet les produits de formule (I) ci-dessus dans lesquels A1, A2, A3 et A4, identiques ou différents, représentent CRa ou N ;
Ra est sélectionné dans le groupe constitué par H ; halogène ; hydroxy ; Y-(CH2)n-alkyle ; Y-(CH2)n-cycloalkyle, Y-(CH2)n-hétérocycloalkyle, Y-(CH2)n-aryle ou Y-(CH2)n-hétéroaryle, avec Y = O, et avec n = 2 ou 3, tous les radicaux alkyle, cycloalkyle, hétérocycloalkyle, aryle et hétéroaryle étant éventuellement substitués, et les radicaux hétérocycloalkyle et hétéroaryle, contenant de 4 à 10 chainons dont 1 à 4 hétéroatomes choisis parmi O, S, N ou NR3 avec R3 représente H ou alkyle lui-même éventuellement substitué, les autres substituants R1,R1',R2,R2' desdits produits de formule (I) étant choisis parmi l'une quelconque des définitions ci-dessus,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

La présente invention a notamment pour objet les produits de formule (I) ci-dessus dans lesquels
A1, A2, A3 et A4 sont tels que soit tous les 4 représentent CRa soit l'un représente CRa et les trois autres, identiques ou différents, représentent N ou CRa , avec Ra représente H ; halogène ; hydroxy ou alcoxy;
les autres substituants R1,R1',R2,R2' desdits produits de formule (I) étant choisis parmi l'une quelconque des définitions ci-dessus,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

Lorsque Ra représente alcoxy, Ra représente notamment méthoxy.

Plus particulièrement la présente invention a pour objet les produits de formule (I) ci-dessus dans lesquels l'un de A2 ou A3 représente N et l'autre de A2 ou A3 ainsi que A1 et A4 représente CH,
les autres substituants R1,R1',R2,R2' desdits produits de formule (I) étant choisis parmi l'une quelconque des définitions ci-dessus,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

La présente invention a notamment pour objet les produits de formule (I) tels que ci-dessus dans lesquels R2 et R'2, identiques ou différents, sont indépendamment sélectionnés dans le groupe constitué par H, halogène, méthyle, éthyle, amino, méthoxy, CH2-NH2, CH2-NHalk, CH2-N(alk)2, CH2-OH, CH2-Oalk,.
p et p' identiques ou différents représentent respectivement les entiers 1 à 4 et 1 à 3 ;
les autres substituants A1, A2, A3, A4, R1 et R1' desdits produits de formule (I) étant choisis parmi l'une quelconque des définitions ci-dessus,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

La présente invention a notamment pour objet les produits de formule (I) ci-dessus dans lesquels R2 et R'2, identiques ou différents, sont indépendamment sélectionnés dans le groupe constitué par H, halogène, méthyle, éthyle, amino, méthoxy,.
p et p' identiques ou différents représentent respectivement les entiers 1 à 4 et 1 à 3 ;
les autres substituants A1, A2, A3, A4, R1 et R1' desdits produits de formule (I) étant choisis parmi l'une quelconque des définitions ci-dessus,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

La présente invention a notamment pour objet les produits de formule (I) ci-dessus dans lesquels R2 et R'2, identiques ou différents, sont indépendamment sélectionnés dans le groupe constitué par H, méthyle,
p et p' identiques ou différents représentent respectivement les entiers 1 à 4 et 1 à 3 ;
les autres substituants A1, A2, A3, A4, R1 et R1' desdits produits de formule (I) étant choisis parmi l'une quelconque des définitions ci-dessus,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

Notamment dans les produits de formule (I) de la présente invention, R2 et R2' peuvent être tels que R2' représente hydrogène et R2 est choisi parmi toutes les valeurs indiquées ci-dessus pour R2 et R2'.

Notamment R2 et R2' représentent tous deux hydrogène.

La présente invention a notamment pour objet les produits de formule (I) ci-dessus dans lesquels R1 et R1' sont tels que :
soit R1 et R1' identiques ou différents sont tels que l'un de R1 et R1' représente un atome d'hydrogène,
et l'autre de R1 et R1' est choisi dans le groupe constitué par H ; halogène ; hydroxyle ; amino ; NH-CO-H ; NH-CO-OH, NH-CO-Oalkyle , NH-CO-NH2 ; carboxy; CO-NH2; X-(CH2)m-alkyle; X-(CH2)m-cycloalkyle ; X-(CH2)m-hétérocycloalkyle; X-(CH2)m-aryle et X-(CH2)m-hétéroaryle, avec X = simple liaison, CH2, CH=CH, CH2-C(O), NH, O-C(O), C(O)-NH, -NH-C(O), -NH-C(O)-C(O)-, -NH-C(O)-NH-, ; NH-CS, NH-S(O) ou NH-S(O)2, avec m = 0, tous les radicaux alkyle, cycloalkyle, hétérocycloalkyle, aryle et hétéroaryle étant éventuellement substitués, le radical cycloalkyle contenant de 3 à 10 chainons, le radical aryle contenant de 6 à 10 chainons, et les radicaux hétérocycloalkyle et hétéroaryle, éventuellement substitués contenant de 4 à 10 chainons dont 1 à 4 hétéroatomes choisis parmi O, S, N ou NR3 avec R3 représente H ou alkyle lui-même éventuellement substitué,
soit R1 et R'1 forment ensemble avec l'atome de carbone auquels ils sont liés un radical =O ; =S ; =N-OH ; =N-NH2 ; =N-NH-CO-NH2, =CH-OH ; =Y1-(CH2)m-aryle ou =Y1-(CH2)m -hétéroaryle, dans lequel Y1 représente CH, CH-CO-, CH-CO-NH, N, N-O ou N-NH-, avec m = 0, 1 ou 2 et dans lesquels aryle, et hétéroaryle sont tels que définis ci-dessus et éventuellement substitués,
soit R1 et R'1 forment ensemble avec l'atome de carbone auquels ils sont liés un hétérocycle partiellement saturé constitué de 5 à 6 chainons et contenant de 1 à 3 hétéroatomes choisis parmi O, S, N ou NR4 avec R4 représente H ou alkyle lui-même éventuellement substitué,
les autres substituants A1, A2, A3, A4, R2 et R2' desdits produits de formule (I) étant choisis parmi l'une quelconque des définitions ci-dessus,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

Notamment dans les produits de formule (I) tels que définis ci-dessus, lorsque R1 et R1' sont tels que l'un de R1 et R1' représente un atome d'hydrogène et l'autre de R1 et R1' représente X-(CH2)m-cycloalkyle ; X-(CH2)m-hétérocycloalkyle ; X-(CH2)m-aryle ou X-(CH2)m-hétéroaryle alors X représente notamment simple liaison NH, O-C(O), C(O)-NH, -NH-C(O), -NH-C(O)-C(O)-, -NH-C(O)-NH-, ; NH-CS ou NH-S(O)2, avec m = 0, tous les radicaux alkyle, cycloalkyle, hétérocycloalkyle, aryle et hétéroaryle étant éventuellement substitués.

La présente invention a plus particulièrement pour objet les produits de formule (I) ci-dessus dans lesquels A1, A2, A3 et A4, identiques ou différents, représentent CRa ou N ou NRb avec Rb représente CH3 ou OH ;
Ra est sélectionné dans le groupe constitué par H ; CH3; CH2-NH2 ; halogène ; CF3 ; hydroxy ; OCF3; SO2-NH2 ; SO2-N(CH3)2; mercapto ; nitro; amino ; NH(CH3); N(CH3)2; NH-OH ; NH-CO-H ; NH-CO-NH2 ; carboxy libre ou estérifié par un radical alkyle lui-même éventuellement substitué; CO2-CH3 ; CO2-(CH2)3-N(CH3)2 ; CN ; CO-NH2 ; CO-N(CH3)2 ; CO-CH3, CO-(CH2)3-O-CH3) ; morpholinyle ; pipérazinyl-CH3; imidazolinyl-CH3; diazépin-CH3 ; -CO-pipérazinyl-CH3 ; -CO-pyrrolidinyle; Y-(CH2)n-alkyle ; Y-(CH2)n-cycloalkyle, Y-(CH2)n-hétérocycloalkyle, Y-(CH2)n-aryle ou Y-(CH2)n-hétéroaryle, avec Y représente une simple liaison ou bien = O, -C(O)-NH-, - C(O)N(CH3)- ; CO, avec n = 0, 1, 2, ou 3 radicaux dans lesquels les radicaux alkyle, cycloalkyle, hétérocycloalkyle, aryle et hétéroaryle sont éventuellement substitués, le radical cycloalkyle contient de 3 à 10 chainons, le radical aryle contient de 6 à 10 chainons, et les radicaux hétérocycloalkyle et hétéroaryle, éventuellement substitué, contiennent de 4 à 10 chainons dont 1 à 4 hétéroatomes choisis parmi O, S, N ou NR3 avec R3 représente H ou alkyle lui-même éventuellement substitué,
R1 et R1' sont tels que : soit l'un de R1 et R1' représente un atome d'hydrogène
et l'autre de R1 et R1' est choisi dans le groupe constitué par X-(CH2)m-hétérocycloalkyle, X-(CH2)m-aryle et X-(CH2)m-hétéroaryle, et notamment X-(CH2)m-hétéroaryle , avec X représente -O-C(O), -NH-C(O), ou NH-CS, - NH-CO-CH2-O- ; -NH-CO-CH2-S-CH2-CO-NH-; -NH-CO-(CH2)2-SO2-; - NH-CO-CH2-N(CH3)-CO- ; et m = 0,
soit R1 et R'1 forment ensemble avec l'atome de carbone auquels ils sont liés un radical =N-OH ou =N-NH2,
R2 et R'2, identiques ou différents, sont indépendamment sélectionnés dans le groupe constitué par H, halogène, méthyle, éthyle, amino, méthoxy, CH2-NH2, CH2-NHalk, CH2-N(alk)2, CH2-OH, CH2-Oalk,.
p et p' identiques ou différents représentent respectivement les entiers 1 à 4 et1à3;
tous les radicaux alkyle, alcoxy, alkylthio, cycloalkyle, hétérocycloalkyle, aryle et hétéroaryle des substituants des produits de formule (I) étant éventuellement substitués,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

La présente invention a plus particulièrement pour objet les produits de formule (I) ci-dessus dans lesquels R1 et R1' sont tels que : soit l'un de R1 et R1' représente un atome d'hydrogène et l'autre de R1 et R1' est choisi dans le groupe constitué par X-(CH2)m-hétérocycloalkyle, X-(CH2)m-aryle et X-(CH2)m-hétéroaryle, et notammment X-(CH2)m-hétéroaryle , avec X représente -O-C(O), -NH-C(O) ou NH-CS et m = 0,
soit R1 et R'1 forment ensemble avec l'atome de carbone auquels ils sont liés un radical =N-OH ou =N-NH2,
tous les radicaux hétérocycloalkyle, aryle et hétéroaryle étant éventuellement substitués,
les autres substituants A1, A2, A3, A4, R2 et R2' desdits produits de formule (I) étant choisis parmi l'une quelconque des définitions ci-dessus,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

Dans les produits de formule (I) tels que définis ci-dessus et ci-dessous, tous les radicaux alkyle, alcoxy, alkylthio, cycloalkyle, hétérocycloalkyle, aryle et hétéroaryle sont éventuellement substitués par un ou plusieurs radicaux, identiques ou différents, choisis parmi les atomes d'halogène ; les radicaux hydroxyle ; cyano ; mercapto, nitro ; carboxy libre, salifié ou estérifié ; tétrazolyle ; -NH2, -NH(alk), -N(alk)(alk) ; -S02-NH-CO-NH-alkyle ; -SO2-NH-CO-NH-phényle; -C(O)-NH2 ; COalkyle, CONH2, -C(O)-NH(alk) ; -C(O)-N(alk)(alk), CO-NH-alk-O-alk, -NH-C(O)-(alk), -N(alk)-C(O)-(alk); -NH-COOalkyle, NH-CO-NH2, alkyle, acyle; alkylthio, cycloalkyle, hétérocycloalkyle, aryle, hétéroaryle, alcoxy et phénoxy eux-mêmes éventuellement substitués par un ou plusieurs radicaux choisis parmi les atomes d'halogène et les radicaux hydroxyle, alcoxy, alkyle, -NH2, -NH(alk) et -N(alk)(alk) .

Notamment tous les radicaux alkyle, alcoxy, alkylthio, cycloalkyle, hétérocycloalkyle, aryle et hétéroaryle définis ci-dessus peuvent être éventuellement substitués par un ou plusieurs substituants identiques ou différents choisis parmi halogène, OH, CN, SH, NH2, NHalk, N(alk)2, COOH, COOalk, CONH2, CO-NH-alkyle, CO-NH-alk-O-alk, COalkyle, NH-COOalkyle, NH-CO-NH2, NHCOalk, alkyle, hydroxyalkyle, alcoxy, cycloalkyle, hétérocycloalkyle, aryle et hétéroaryle.

Notamment alkyle est éventuellement substitué par un ou plusieurs substituants identiques ou différents choisis parmi NH2, COOH, COOalk, CONH2, COalkyle, NH-COOalkyle, NH-CO-NH2, aryle, hétéroaryle. Notamment hétéroaryle est éventuellement substitué par un ou plusieurs substituants identiques ou différents choisis parmi halogène, OH, CN, SH, NH2, NHCOalk, NHalk, N(alk)2, alkyle, hydroxyalkyle(CH2OH), alcoxy ,COOH, COOalk, CONH2, CO-NH-alkyle, CO-NH-Alk-O-Alk, COalkyle, NH-COOalkyle, NH-CO-NH2, cycloalkyle, hétérocycloalkyle, aryle, hétéroaryle. Notamment hétéroaryle est éventuellement substitué par un ou plusieurs substituants identiques ou différents choisis parmi F, Cl, Br, OH, NH2, NHCOalk tel que NHCOCH3, NHalk, N(alk)2, alkyle, hydroxyalkyle tel que CH2OH ou COOalk.

La présente invention a plus particulièrement pour objet les produits de formule (I) ci-dessus dans lesquels A1, A2, A3 et A4, identiques ou différents, représentent CRa ou N ou NRb avec Rb représente CH3 ou OH ;
Ra est sélectionné dans le groupe constitué par H ; CH3; CH2-NH2 ; halogène ; CF3 ; hydroxy ; OCF3; S02-NH2 ; SO2-N(CH3)2; mercapto ; nitro; amino ; NH(CH3); N(CH3)2; NH-OH ; NH-CO-H ; NH-CO-NH2 ; carboxy libre ou estérifié par un radical alkyle lui-même éventuellement substitué; CO2-CH3 ; CO2-(CH2)3-N(CH3)2 ; CN ; CO-NH2 ; CO-N(CH3)2 ; CO-CH3, CO-(CH2)3-O-CH3) ; morpholinyle ; pipérazinyl-CH3; imidazolinyl-CH3; diazépin-CH3 ; -CO-pipérazinyl-CH3 ; -CO-pyrrolidinyle; Y-(CH2)n-alkyle ; Y-(CH2)n-cycloalkyle, Y-(CH2)n-hétérocycloalkyle, Y-(CH2)n-aryle ou Y-(CH2)n-hétéroaryle, avec Y représente une simple liaison ou bien = O, -C(O)-NH-, - C(O)N(CH3)- ; CO, avec n = 0, 1, 2, ou 3 radicaux dans lesquels les radicaux alkyle, cycloalkyle, hétérocycloalkyle, aryle et hétéroaryle sont éventuellement substitués, le radical cycloalkyle contient de 3 à 10 chainons, le radical aryle contient de 6 à 10 chainons, et les radicaux hétérocycloalkyle et hétéroaryle, éventuellement substitué, contiennent de 4 à 10 chainons dont 1 à 4 hétéroatomes choisis parmi O, S, N ou NR3 avec R3 représente H ou alkyle lui-même éventuellement substitué,
R1 et R1' sont tels que: soit l'un de R1 et R1' représente un atome d'hydrogène
et l'autre de R1 et R1' est choisi dans le groupe constitué par X-(CH2)m-hétérocycloalkyle, X-(CH2)m-aryle et X-(CH2)m-hétéroaryle, et notammment X-(CH2)m-hétéroaryle , avec X représente -O-C(O), -NH-C(O), NH-CS, - NH-CO-CH2-O- ; -NH-CO-CH2-S-CH2-CO-NH-; -NH-CO-(CH2)2-SO2-; - NH-CO-CH2-N(CH3)-CO- ; et m = 0,
soit R1 et R'1 forment ensemble avec l'atome de carbone auquels ils sont liés un radical =N-OH ou =N-NH2,
R2 et R'2, identiques ou différents, sont indépendamment sélectionnés dans le groupe constitué par H, halogène, méthyle, éthyle, amino, méthoxy, CH2-NH2, CH2-NHalk, CH2-N(alk)2, CH2-OH, CH2-Oalk,.
p et p' identiques ou différents représentent respectivement les entiers 1 à 4 et 1 à 3 ;
tous les radicaux alkyle, alcoxy, alkylthio, cycloalkyle, hétérocycloalkyle, aryle et hétéroaryle des substituants des produits de formule (I) étant éventuellement substitués,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

La présente invention a plus particulièrement pour objet les produits de formule (I) ci-dessus dans lesquels R1 et R1' sont tels que : soit l'un de R1 et R1' représente un atome d'hydrogène et l'autre de R1 et R1' est choisi dans le groupe constitué par X-(CH2)m-hétérocycloalkyle, X-(CH2)m-aryle et X-(CH2)m-hétéroaryle, et notammment X-(CH2)m-hétéroaryle , avec X représente -O-C(O), -NH-C(O) ou NH-CS et m = 0,
soit R1 et R'1 forment ensemble avec l'atome de carbone auquels ils sont liés un radical =N-OH ou =N-NH2,
tous les radicaux hétérocycloalkyle, aryle et hétéroaryle étant éventuellement substitués,
les autres substituants A1, A2, A3, A4, R2 et R2' desdits produits de formule (I) étant choisis parmi l'une quelconque des définitions ci-dessus,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

Dans les produits de formule (I) tels que définis ci-dessus et ci-dessous, tous les radicaux alkyle, alcoxy, alkylthio, cycloalkyle, hétérocycloalkyle, aryle et hétéroaryle sont éventuellement substitués par un ou plusieurs radicaux, identiques ou différents, choisis parmi les atomes d'halogène ; les radicaux hydroxyle ; cyano ; mercapto, nitro ; carboxy libre, salifié ou estérifié ; tétrazolyle ; -NH2, -NH(alk), -N(alk)(alk) ; -S02-NH-CO-NH-alkyle ; -SO2-NH-CO-NH-phényle; -C(O)-NH2 ; COalkyle, CONH2, -C(O)-NH(alk) ; -C(O)-N(alk)(alk), CO-NH-alk-O-alk, -NH-C(O)-(alk), -N(alk)-C(O)-(alk); -NH-COOalkyle, NH-CO-NH2, alkyle, acyle; alkylthio, cycloalkyle, hétérocycloalkyle, aryle, hétéroaryle, alcoxy et phénoxy eux-mêmes éventuellement substitués par un ou plusieurs radicaux choisis parmi les atomes d'halogène et les radicaux hydroxyle, alcoxy, alkyle, -NH2, -NH(alk) et -N(alk)(a1k) .

Notamment tous les radicaux alkyle, alcoxy, alkylthio, cycloalkyle, hétérocycloalkyle, aryle et hétéroaryle définis ci-dessus peuvent être éventuellement substitués par un ou plusieurs substituants identiques ou différents choisis parmi halogène, OH, CN, SH, NH2, NHalk, N(alk)2, COOH, COOalk, CONH2, CO-NH-alkyle, CO-NH-alk-O-alk, COalkyle, NH-COOalkyle, NH-CO-NH2, NHCOalk, alkyle, hydroxyalkyle, alcoxy, cycloalkyle, hétérocycloalkyle, aryle et hétéroaryle.

Notamment alkyle est éventuellement substitué par un ou plusieurs substituants identiques ou différents choisis parmi NH2, COOH, COOalk, CONH2, COalkyle, NH-COOalkyle, NH-CO-NH2, aryle, hétéroaryle. Notamment hétéroaryle est éventuellement substitué par un ou plusieurs substituants identiques ou différents choisis parmi halogène, OH, CN, SH, NH2, NHCOalk, NHalk, N(alk)2, alkyle, hydroxyalkyle(CH2OH), alcoxy ,COOH, COOalk, CONH2, CO-NH-alkyle, CO-NH-Alk-O-Alk, COalkyle, NH-COOalkyle, NH-CO-NH2, cycloalkyle, hétérocycloalkyle, aryle, hétéroaryle. Notamment hétéroaryle est éventuellement substitué par un ou plusieurs substituants identiques ou différents choisis parmi F, Cl, Br, OH, NH2, NHCOalk tel que NHCOCH3, NHalk, N(alk)2, alkyle, hydroxyalkyle tel que CH2OH ou COOalk.

Dans les produits de formule (I) ci-dessus, notamment les radicaux alkyle, alcoxy, alkylthio, cycloalkyle, hétérocycloalkyle, aryle et hétéroaryle des substituants des produits de formule (I) sont éventuellement substitués par un ou plusieurs substituants identiques ou différents choisis parmi les atomes d'halogène et les radicaux alkyle, OH, Oalkyle, NH2, NH(alk), N(alk)2, et les radicaux phényle, pipérazinyle et pyrrolidinyle eux-mêmes éventuellement substitués par un ou plusieurs substituants choisis parmi les atomes d'halogène et les radicaux alk, OH, Oalkyle, NH2, NH(alk) et N(alk)2 ;

Dans les produits de formule (I) ci-dessus, notamment les radicaux alkyle, alcoxy, alkylthio, cycloalkyle, hétérocycloalkyle, aryle et hétéroaryle des substituants des produits de formule (I) sont éventuellement substitués par un ou plusieurs substituants identiques ou différents choisis parmi les atomes d'halogène et les radicaux CH3, OH, OCH3, NH2, NH(CH3), N(CH3)2 et les radicaux phényle, pipérazinyle et pyrrolidinyle eux-mêmes éventuellement substitués par un ou plusieurs substituants choisis parmi les atomes d'halogène et les radicaux CH3, OH, OCH3, NH2, NH(CH3) et N(CH3)2 ;

La présente invention concerne notamment les composés dans lesquels R1 et R'1 sont tels que l'un de R1 et R'1 représente un atome d'hydrogène et l'autre représente le radical X-(CH2)m-hétéroaryle, dans lequel X représente -CO-NH- et m et hétéroaryle ont les significations indiquées ci-dessus : on indique à titre d'exemples les radicaux -X--(CH2)m-hétéroaryfe ci-après :

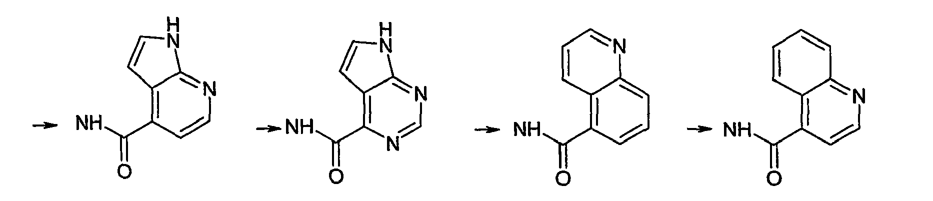

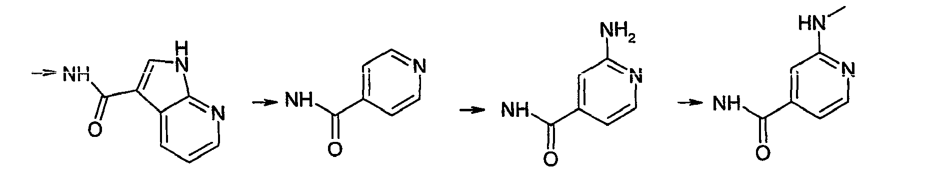

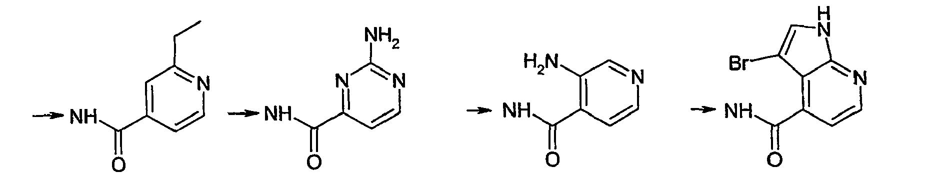

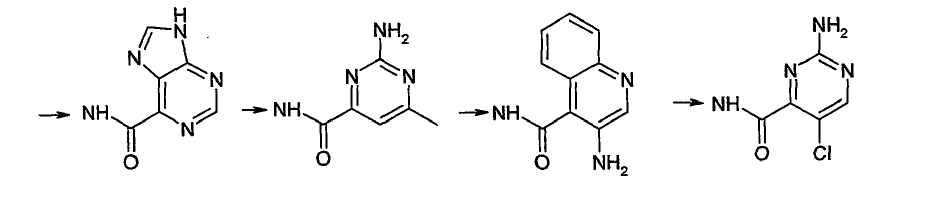

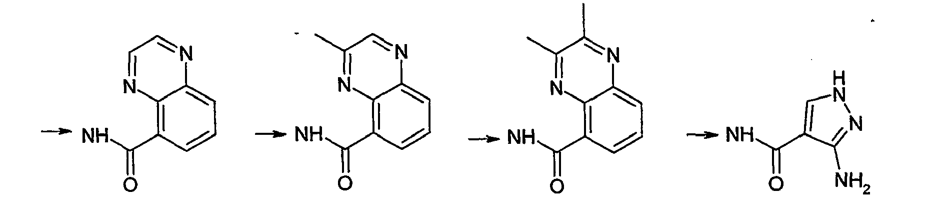

La présente invention concerne notamment les radicaux -X--(CH2)m-hétéroaryle ci-après

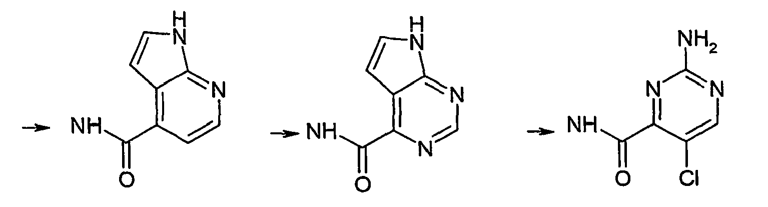

Sous un autre aspect, la présente invention concerne également particulièrement les composés dans lesquels le radical 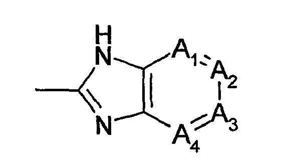 représente soit un radical benzimidazole dans lequel A1, A2, A3 et A4 identiques ou différents sont choisis parmi les valeurs de CRa telles que définies ci-dessus soit un radical azabenzimidazole dans lequel un ou deux de A1, A2, A3 et A4 représente N et les trois autres ou les deux autres identiques ou différents sont choisis parmi les valeurs de CRa telles que définies ci-dessus.

On indique ainsi à titre d'exemples les radicaux de type benzimidazole ou azabenzimidazole ci-après :

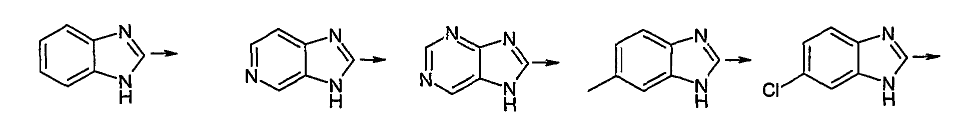

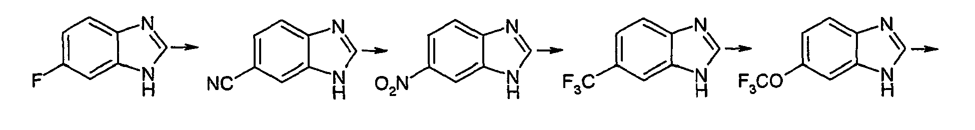

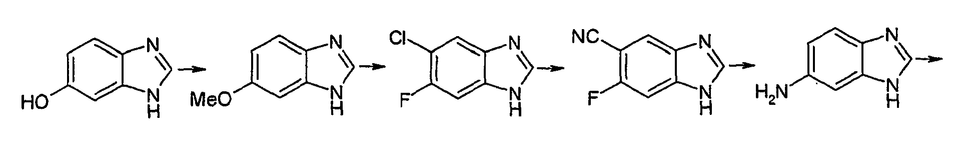

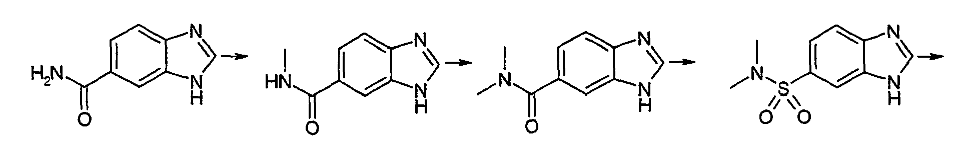

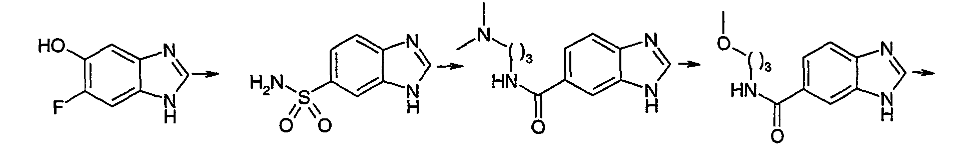

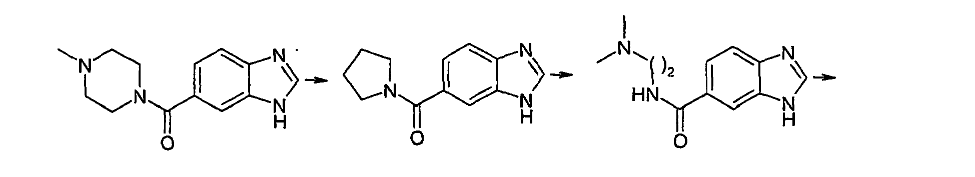

On indique plus particulièrement à titre d'exemples les radicaux de type benzimidazole ou azabenzimidazole ci-après :

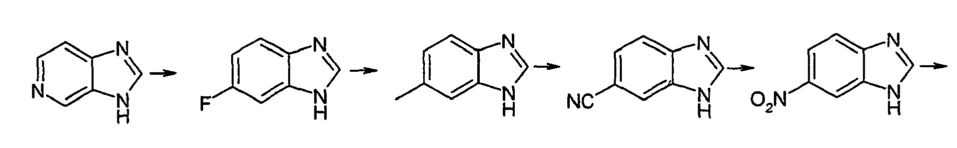

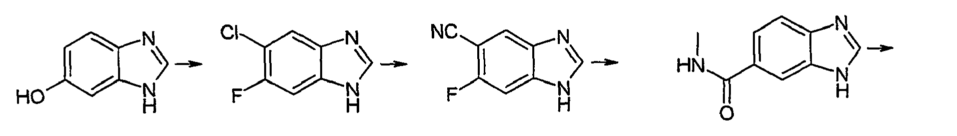

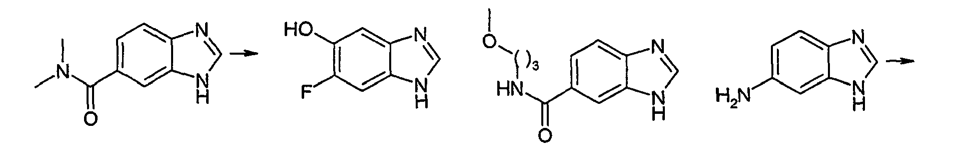

La présente invention concerne notamment les produits de formule générale (I) tels que définis ci-dessus à l'une quelconque des revendications dans lesquels R'2 représente un atome d'hydrogène, avec p' égal à 3, et R2 représente un atome d'hydrogène ou un radical amino étant entendu que les positions 5, 6 et 8 du noyau fluorène sont substituées préférentiellement par un atome d'hydrogène et que la position 7 du noyau fluorène est préférentiellement substituée par un atome d'hydrogène ou un radical amino. La présente invention concerne notamment les produits de formule générale (I) tels que définis ci-dessus dans lesquels :
R'2 représente un atome d'hydrogène, avec p' égal à 3, et R2 représente un atome d'hydrogène ou un radical amino étant entendu que les positions 5, 6 et 8 du noyau fluorène sont substituées préférentiellement par un atome d'hydrogène et que la position 7 du noyau fluorène est préférentiellement substituée par un atome d'hydrogène ou un radical amino,
R1 et R'1 sont tels que l'un de R1 et R'1 représente un atome d'hydrogène et l'autre est sélectionné parmi le groupe constitué de : 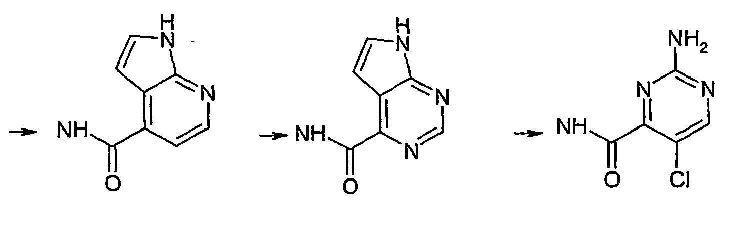
et le radical: 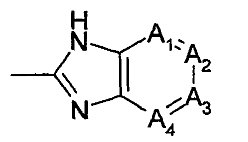
représente notamment les radicaux benzimidazole et azabenzimidazoles suivants:

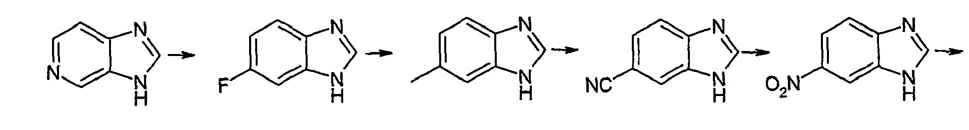

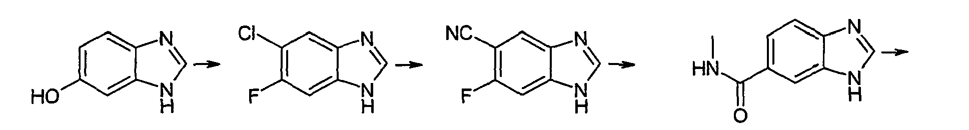

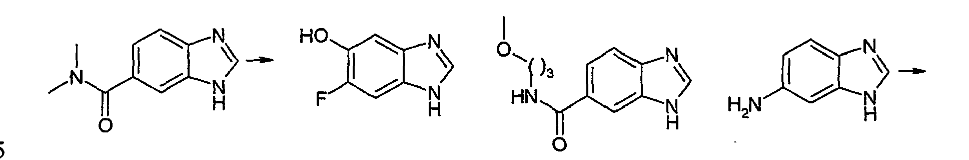

La présente invention concerne notamment les énantiomères dextrogyres des composés ci-dessus.

La présente invention a notamment pour objet les produits de formule (I) ci-dessus dont les noms suivent :
- la 4-(3H-imidazo[4,5-c]pyridin-2-yl)-fluorène-9-one oxime (Z, E).
- la N-[4-(1H-imidazo[4,5-c]pyridin-2-yl)-fluorèn-9-ylidène]-hydrazine, mélange 60/40 d'isomères E et Z
- le [4-(3H-imidazo[4,5-c]pyridin-2-yl)-9H-fluorèn-9(R,S)-yl]-amide de l'acide quinoléine-5-carboxylique.
- énantiomère dextrogyre du [4-(3H-imidazo[4,5-c]pyridin-2-yl)-9H-fluorèn-9(R,S)-yl]-amide de l'acide quinoléine-5-carboxylique
- la 4-(3H-lmidazo[4,5-c]pyridin-2-yl)-fluorène-9-one oxime (E).
- le trifluoroacétate du [4-(3H-imidazo[4,5-c]pyridin-2-yl)-9H-fluorèn-9(R,S)-yl]-amide de l'acide 1H-pyrazole-4-carboxylique
- le trifluoroacétate du N-[4-(3H-imidazo[4,5-c]pyridin-2-yl)-9H-fluorèn-9(R,S)-yl]-succinamide.
- le trifluoroacétate du [4-(3H-imidazo[4,5-c]pyridin-2-yl)-9H-fluorèn-9(R,S)-yl]-amide de l'acide 1H-benzotriazole-5-carboxylique.
- le [4-(3H-imidazo[4,5-c]pyridin-2-yl)-9H-fluorèn-9(R,S)-yl]-amide de l'acide 1H-indazole-5-carboxylique
- le [4-(3H-imidazo[4,5-c]pyridin-2-yl)-9H-fluorèn-9(R,S)-yl]-amide de l'acide 1H-indole-6-carboxylique
- le N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide 1H-indole-4-carboxylique
- le trifluoroacétate du [4-(3H-imidazo[4,5-c]pyridin-2-yl)-9H-fluorèn-9(R,S)-yl]-amide de l'acide quinoléine-5-carbothioique
- le 2-acétylamino-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide quinoléine-4-carboxylique
- le 2-amino-N-[4-(3H-imidazo[4,5-c]pyridin-2-yl)-9H-fluorèn-9(R,S)-yl]-isonicotinamide.
- le 2-éthyl-N-[4-(3H-imidazo[4,5-c]pyridin-2-yl)-9H-fluorèn-9(R,S)-yl]-isonicotinamide
- le [4-(6-fluoro-1H-benzimidazol-2-yl)-9H-fluorèn-9(R,S)-yl]-amide de l'acide quinoléine-5-carboxylique
- le 2-chloro-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluoren-9(R,S)-yl]-amide de l'acide 2-amino-pyrimidine-4-carboxylique
- le [4-(3H-imidazo[4,5-c]pyridin-2-yl)-9H-fluorèn-9(R,S)-yl]-amide de l'acide 1H-pyrrolo[2,3-b]pyridine-4-carboxylique
- le 2-hydroxyméthyl-N-[4-(3H-imidazo[4,5-c]pyridin-2-yi)-9H-fluorèn-9(R,S)-yl]-isonicotinamide
- le N-[4-(3H-imidazo[4,5-c]pyridin-2-yl)-9H-fluorèn-9(R,S)-yl]-3-méthyl-isonicotinamide
- le chlorhydrate du [4-(3H-imidazo[4,5-c]pyridin-2-yl)-9H-fluorèn-9(R,S)-yl]-amide de l'acide 1,8-naphtyridine-4-carboxylique
- le [4-(3H-imidazo[4,5-c]pyridin-2-yl)-9H-fluorèn)-9(R,S)-yl]-amide de l'acide 1H-pyrrolo[2,3-b]pyridine-3-carboxylique
- le [4-(3H-imidazo[4,5-c]pyridin-2-yl)-9H-fluorèn-9(R,S)-yl]-amide de l'acide 1-méthyl-1H-pyrrolo[2,3-b]pyridine-3-carboxylique
- le 2-bromo-N-[4-(3H-imidazo[4,5-c]pyridin-2-yl)-9H-fluorèn-9(R,S)-yl]-isonicotinamide
- le 3-hydroxy-N-[4-(3H-imidazo[4,5-c]pyridin-2-yl)-9H-fluorèn-9(R,S)-yl]-isonicotinamide
- le 3-amino-N-[4-(3H-imidazo[4,5-c]pyridin-2-yl)-9H-fluorèn-9(R,S)-yl]-isonicotinamide
- ester de méthyle de l'acide 4-[4-(3H-imidazo[4,5-c]pyridin-2-yl)-9H-fluorèn-9(R,S)-ylcarbamoyl]-pyridine-2-carboxylique
- le [4-(3H-imidazo[4,5-c]pyridin-2-yl)-9H-fluorèn-9(R,S)-yl]-amide de l'acide 1H-indazole-4-carboxylique
- le N-[4-(3H-imidazo[4,5-c]pyridin-2-yl)-9H-fluorèn-9(R,S)-yl]-2-méthylamino-isonicotinamide
- le N-[4-(6-hydroxy-1H-benzimidazol-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide 6-bromo-3H-imidazo[4,5-b]pyridine-7-carboxylique
- ester de 4-(3H-imidazo[4,5-c]pyridin-2-yl)-9H-fluoren-9(R,S)-yle de l'acide isonicotinique,
- le N-[4-(3H-imidazo[4,5-c]pyridin-2-yl)-9H-fluorèn-9(R,S)-yl]-amide de l'acide 2-tert-butoxycarbonylamino-isonicotinique.
- le N-[4-(3H-imidazo[4,5-c]pyridin-2-yl)-9H-fluorèn-9(R,S)-yl]-amide de l'acide 3-chloro-6-méthoxy-quinoléine-4-carboxylique.
- le N-[4-(3H-imidazo[4,5-c]pyridin-2-yl)-9H-fluorèn-9(R,S)-yl]-amidede l'acide 3-hydroxy-quinoléine-4-carboxylique.
- le N-[4-(3H-imidazo[4,5-c]pyridin-2-yl)-9H-fluorèn-9(R,S)-yl]-amide de l'acide 2-amino-5-chloro-pyrimidine-4-carboxylique.
- le [4-(3H-imidazo[4,5-c]pyridin-2-yl)-9H-fluorèn-9(R,S)-yl]-amide de l'acide 3-bromo-1H-pyrrolo[2,3-b]pyridine-4-carboxylique.
- l'énantiomère dextrogyre du [4-(3H-imidazo[4,5-c]pyridin-2-yl)-9H-fluorèn-9(R,S)-yl]-amide de l'acide 1H-pyrrolo[2,3-b]pyridine-4-carboxylique.
- le N-[4-(3H-imidazo[4,5-c]pyridin-2-yl)-9H-fluorèn-9(R,S)-yl]-amide de l'acide 9H-purine-6-carboxylique.
- le N-[4-(3H-imidazo[4,5-c]pyridin-2-yl)-9H-fluorèn-9(R,S)-yl]-amide de l'acide 2-amino-6-méthyl-pyrimidine-4-carboxylique.
- le [4-(3H-imidazo[4,5-c]pyridin-2-yl)-9H-fluorène-9(R,S)-yl]-amide de l'acide 5-amino-3H-1,2,3-triazole-4-carboxylique.
- le N-[4-(3H-imidazo[4,5-c]pyridin-2-yl)-9H-fluorèn-9(R,S)-yl]-amide de l'acide 6-méthyl-2-méthylamino-pyrimidine-4-carboxylique.
- le N-[4-(3H-imidazo[4,5-c]pyridin-2-yl)-9H-fluorèn-9(R,S)-yl]-amide de l'acide 6-méthoxy-quinoléine-4-carboxylique.
- le 3,5-dihydroxy-N-[4-(3H-imidazo[4,5-c]pyridin-2-yl)-9H-fluorèn-9(R,S)-yl]-benzamide.
- le N-[4-(3H-imidazo[4,5-c]pyridin-2-yl)-9H-fluorèn-9(R,S)-yl]-amide de l'acide pyrimidine-4-carboxylique.
- le 4-hydroxy-N-[4-(3H-imidazo[4,5-c]pyridin-2-yf)-9H-fluorèn-9(R,S)-yl]-benzamide.
- le 2,4-dihydroxy-N-[4-(3H-imidazo[4,5-c]pyridin-2-yl)-9H-fluorèn-9(R,S)-yl]-benzamide.
- le [4-(5-cyano-6-fluoro-1H-benzimidazol-2-yl)-9H-fluorén-9(R,S)-yl]-amide de l'acide 1H-pyrrolo[2,3-b]pyridine-4-carboxylique.
- le 4-(1H-benzimidazol-2-yl)-9H-fluorène-9(R,S)-yl]-amide de l'acide 2-amino-5-chloro-pyrimidine-4-carboxylique.
- le [4-(5-fluoro-1H-benzimidazol-2-yl)-9H-fluorène-9(R,S)-yl]-amide de l'acide 2-amino-5-chloro-pyrimidine-4-carboxylique.
- le [4-(5-fluoro-1H-benzimidazol-2-yl)-9H-fluorène-9(R,S)-yl]-amide de l'acide 1H-pyrrolo[2,3-b]pyridine-4-carboxylique.
- le [4-(3H-imidazo[4,5-c]pyridin-2-yl)-9H-fluorène-9(R,S)-yl]-amide de l'acide 2,3-diméthyl-quinoxaline-5-carboxylique.
- le [4-(3H-imidazo[4,5-c]pyridin-2-yl)-9H-fluorène-9(R,S)-yl]-amide de l'acide 3-amino-1H-pyrazole-4-carboxylique.
- l'énantiomère dextrogyre du N-[4-(3H-imidazo[4,5-c]pyridin-2-yl)-9H-fluorèn-9(R,S)-yl]-amide de l'acide 2-amino-5-chloro-pyrimidine-4-carboxylique.
- l'énantiomère lévogyre du N-[4-(3H-imidazo[4,5-c]pyridin-2-yl)-9H-fluorèn-9(R,S)-yl]-amide de l'acide 2-amino-5-chloro-pyrimidine-4-carboxylique.
- le [4-(3H-imidazo[4,5-c]pyridin-2-yl)-9H-fluorène-9(R,S)-yl]-amide de l'acide 3-méthyl-quinoxaline-5-carboxylique.
- le [4-(3H-imidazo[4,5-c]pyridin-2-yl)-9H-fluorène-9(R,S)-yl]-amide de l'acide quinoxaline-5-carboxylique.
- le [4-(9H-purine-8-yl)-9H-fluorène-9(R,S)-yl]-amide de l'acide 1H-pyrrolo[2,3-b]pyridine-4-carboxylique
- le [4-(1H-benzimidazol-2-yl)-9H-fluorène-9(R,S)-yl]-amide de l'acide 1H-pyrrolo[2,3-b]pyridine-4-carboxylique
- le [4-(5-trifluorométhyl-1H-benzimidazol-2-yl)-9H-fluorène-9(R,S)-yl]-amide de l'acide 1H-pyrrolo[2,3-b]pyridine-4-carboxylique
- le [4-(5-méthoxycarbonyl-1H-benzimidazol-2-yl)-9H-fluorène-9(R,S)-yl]-amide de l'acide 1H-pyrrolo[2,3-b]pyridine-4-carboxylique
- le [4-(5-carboxy-1H-benzimidazol-2-yl)-9H-fluorène-9(R,S)-yl]-amide de l'acide 1H-pyrrolo[2,3-b]pyridine-4-carboxylique
- le [4-(4-méthyl-1H-benzimidazol-2-yl)-9H-fluorène-9(R,S)-yl]-amide de l'acide 1H-pyrrolo[2,3-b]pyridine-4-carboxylique
- le [4-(5-carboxamido-1H-benzimidazol-2-yl)-9H-fluorène-9(R,S)-yl]-amide de l'acide 1H-pyrrolo[2,3-b]pyridine-4-carboxylique
- le [4-(6-sulfamoyl-1H-benzimidazol-2-yl)-9H-fluorèn-9(R,S)-yl]-amide de l'acide 1H-pyrrolo[2,3-b]pyridine-4-carboxylique
- le [4-(5-trifluorométhoxy-1H-benzimidazol-2-yl)-9H-fluorène-9(R,S)-yl]-amide de l'acide 1H-pyrrolo[2,3-b]pyridine-4-carboxylique
- le [4-(5-cyano-1H-benzimidazol-2-yl)-9H-fluorène-9(R,S)-yl]-amide de l'acide 1H-pyrrolo[2,3-b]pyridine-4-carboxylique
- le diastéréosisomère D du [4-(3H-imidazo[4,5-c]pyridin-2-yl)-9H-fluorèn-9(R,S)-yl]-amide de l'acide 2-(3-acétyl-2,2-diméthyl-cyclobutan-1-yl) acétique
- le [4-(6-chloro-1H-benzimidazol-2-yl)-9H-fluorèn-9(R,S)-yl]-amide de l'acide 1H-pyrrolo[2,3-b]pyridine-4-carboxylique :
- le [4-(5-fluoro-6-morpholino-1H-benzimidazole-2-yl)-9H-fluorène-9(R,S)-yl]-amide de l'acide 1H-pyrrolo[2,3-b]pyridine-4-carboxylique
- le [4-(6-chloro-5-fluoro-1H-benzimidazole-2-yl)-9H-fluorène-9(R,S)-yl]-amide de l'acide 1H-pyrrolo[2,3-b]pyridine-4-carboxylique
- le [4-(4,5,6,7-tétrahydro-1H-benzimidazol-2-yl)-9H-fluorèn-9(R,S)-yl]-amide de l'acide 1H-pyrrolo[2,3-b]pyridine-4-carboxylique
- le [2-amino-5-(1H-benzimidazol-2-yl)-9H-fluorène-9(R,S)-yl]-amide de l'acide 1H-pyrrolo[2,3-b]pyridine-4-carboxylique
- le [4-(5-hydroxy-5H-imidazo[4,5-c]pyridin-2-yl)-9H-fluorèn-9(R,S)-yl]-amide de l'acide 1H-pyrrolo[2,3-b]pyridine-4-carboxylique
- le [4-(5-hydroxy-1H-benzimidazole-2-yl)-9H-fluorène-9(R,S)-yl]-amide de l'acide 1H-pyrrolo[2,3-b]pyridine-4-carboxylique
- le [4-(5-méthylaminocarbonyl-1H-benzimidazol-2-yl)-9H-fluorène-9(R,S)-yl]-amide de l'acide 1H-pyrrolo[2,3-b]pyridine-4-carboxylique
- le [4-(5-diméthylaminocarbonyl-1H-benzimidazol-2-yl)-9H-fluorène-9(R,S)-yl]-amide de l'acide 1H-pyrrolo[2,3-b]pyridine-4-carboxylique
- le [4-5-(2-diméthylamino-éthyl)aminocarbonyl-1H-benzimidazol-2-yl)-9H-fluorène-9(R,S)-yl]-amide de l'acide 1H-pyrrolo[2,3-b]pyridine-4-carboxylique
- le [4-(4,5,6,7-tétrafluoro-1H-benzimidazol-2-yl)-9H-fluorèn-9(R,S)-yl]-amide de l'acide 1H-pyrrolo[2,3-b]pyridine-4-carboxylique
- le 4-[5-(3-méthoxypropyl)aminocarbonyl-1H-benzimidazol-2-yl)-9H-fluorène-9(R,S)-yl]-amide de l'acide 1H-pyrrolo[2,3-b]pyridine-4-carboxylique
- le 4-[5-(4-méthyl-pipérazine-1-yl)carbonyl-1H-benzimidazol-2-yl)-9H-fluorène-9(R,S)-yl]-amide de l'acide 1H-pyrrolo[2,3-b]pyridine-4-carboxylique
- le [4-(6-diméthylsulfamoyl-1H-benzimidazol-2-yl)-9H-fluorèn-9(R,S)-yl]-amide de l'acide 1H-pyrrolo[2,3-b]pyridine-4-carboxylique
- le [4-[5-(pyrrolidin-1-yl)carbonyl)-1H-benzimidazol-2-yl)-9H-fluorène-9(R,S)-yl]-amide de l'acide 1H-pyrrolo[2,3-b]pyridine-4-carboxylique
- l'énantiomère dextrogyre de l'acide 2-{9(R,S)-[(1H-pyrrolo[2,3-b]pyridine-4-carbonyl)-amino]-9H-fluorèn-4-yl}-1H-benzimidazole-5-carboxylique
- le {{4-{5-[2-(pyrrolidin-1-yl)éthylaminocarbonyl]-1H-benzimidazole-2-yl}-9H-fluorène-9(R,S)-yl}}amide de l'acide 1H-pyrrolo[2,3-b]pyridine-4-carboxylique
- le [4-(6-diméthylamino-1H-benzimidazol-2-yl)-9H-fluoren-9(R,S)-yl]-amide de l'acide 1H-pyrrolo[2,3-b]pyridine-4-carboxylique
- le {4-[6-(4-méthyl-pipérazin-1-yl)-1H-benzimidazol-2-yl]-9H-fluorèn-9(R,S)-yl}-amide de l'acide 1H-pyrrolo[2,3-b]pyridine-4-carboxylique
- le [4-[6-(méthyl-4(5)-imidazolin-2-yl)-1H-benzimidazol-2-yl]-9H-fluorèn-9(R,S)-yl]-amide de l'acide 1H-pyrrolo[2,3-b]pyridine-4-carboxylique
- le {{4-{5-[(3-diméthylamino-propyl)aminocarbonyl]-1H-benzimidazol-2-yl}-9H-fluorène-9(R,S)-yl}}-amide de l'acide 1H-pyrrolo[2,3-b]pyridine-4-carboxylique
- le {4-[5-fluoro-6-(4-méthyl-pipérazin-1-yl)-1H-benzimidazol-2-yl]-9H-fluorèn-9(R,S)-yl}-amide de l'acide 1H-pyrrolo[2,3-b]pyridine-4-carboxylique
- le {{4-{5-[(3-diméthylamino-propyl)carbonyloxy]-1H-benzimidazol-2-yl}-9H-fluorène-9(R,S)-yl}}-amide de l'acide 1H-pyrrolo[2,3-b]pyridine-4-carboxylique
- le [4-(6-amino-1H-benzimidazol-2-yl)-9H-fluorèn-9-(R,S)yl]-amide de l'acide 1H-pyrrolo[2,3-b]pyridine-4-carboxylique.
- le [4-(6-méthyl-1H-benzimidazol-2-yl)-9H-fluorène-9(R,S)-yl]-amide de l'acide 1H-pyrrolo[2,3-b]pyridine-4-carboxylique
- le {{4-{5-[(3-hydroxy-propyl)aminocarbonyl]-1H-benzimidazole-2-yl}-9H-fluorène-9(R,S)-yl}}-amide de l'acide 1H-pyrrolo[2,3-b]pyridine-4-carboxylique
- l'énantiomère dextrogyre du {4-[5-cyano-1H-benzimidazol-2-yl]-9H-fluorène-9(R,S)-yl}-amide de l'acide 1H-pyrrolo[2,3-b]pyridine-4-carboxylique
- le [4-(3H-imidazo[4,5-c]pyridin-2-yl)-9H-fluorèn-9(R,S)-yl]-amide de l'acide 7H-pyrrolo[2,3-d]pyrimidine-4-carboxylique.
- le [4-(6-amino-1H-benzimidazol-2-yl)-9H-fluorèn-9(R,S)-yl]-amide de l'acide 1H-pyrrolo[2,3-b]pyridine-4-carboxylique
- l'énantiomère dextrogyre du {4-[5-fluoro-1H-benzimidazol-2-yl]-9H-fluorène-9(R,S)-yl}-amide de l'acide 1H-pyrrolo[2,3-b]pyridine-4-carboxylique
- l'ester méthylique de l'acide 2-{9(R,S)-[(1H-pyrrolo[2,3-b]pyridine-4-carbonyl)-amino]-9H-fluorèn-4-yl}-1H-imidazo[4,5-c]pyridine-6-carboxylique
- le [4-(6-fluoro-5-methoxy-1H-benzimidazol-2-yl)-9H-fluorène-9(R,S)-yl]-amide de l'acide 1H-pyrrolo[2,3-b]pyridine-4-carboxylique
- le [4-(6-fluoro-5-hydroxy-1H-benzimidazol-2-yl)-9H-fluorène-9(R,S)-yl]-amide de l'acide 1H-pyrrolo[2,3-b]pyridine-4-carboxylique
- le {4-[5-fluoro-6-(3-méthoxy-propoxy)-1H-benzimidazol-2-yl]-9H-fluorèn-9(R,S)-yl}-amide de l'acide 1H-pyrrolo[2,3-b]pyridine-4-carboxylique
- le {4-[6-(3-diméthylamino-propoxy)-5-fluoro-1H-benzimidazol-2-yl]-9H-fluorèn-9(R,S)-yl}-amide de l'acide 1H-pyrrolo[2,3-b]pyridine-4-carboxylique
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

La présente invention a notamment pour objet les produits de formule (I) ci-dessus dont les noms suivent :
- la 4-(3H-imidazo[4,5-c]pyridin-2-yl)-fluorène-9-one oxime (Z, E).
- la N-[4-(1H-imidazo[4,5-c]pyridin-2-yl)-fluorèn-9-ylidène]-hydrazine, mélange 60/40 d'isomères E et Z
- le [4-(3H-imidazo[4,5-c]pyridin-2-yl)-9H-fluorèn-9(R,S)-yl]-amide de l'acide quinoléine-5-carboxylique.
- énantiomère dextrogyre du [4-(3H-imidazo[4,5-c]pyridin-2-yl)-9H-fluorèn-9(R,S)-yl]-amide de l'acide quinoléine-5-carboxylique
- la 4-(3H-Imidazo[4,5-c]pyridin-2-yl)-fluorène-9-one oxime (E).
- le trifluoroacétate du [4-(3H-imidazo[4,5-c]pyridin-2-yl)-9H-fluorèn-9(R,S)-yl]-amide de l'acide 1H-pyrazole-4-carboxylique
- le trifluoroacétate du N-[4-(3H-imidazo[4,5-c]pyridin-2-yl)-9H-fluorèn-9(R,S)-yl]-succinamide.
- le trifluoroacétate du [4-(3H-imidazo[4,5-c]pyridin-2-yl)-9H-fluorèn-9(R,S)-yl]-amide de l'acide 1H-benzotriazole-5-carboxylique.
- le [4-(3H-imidazo[4,5-c]pyridin-2-yl)-9H-fluorèn-9(R,S)-yl]-amide de l'acide 1H-indazole-5-carboxylique
- le [4-(3H-imidazo[4,5-c]pyridin-2-yl)-9H-fluorèn-9(R,S)-yl]-amide de l'acide 1H-indole-6-carboxylique
- le N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide 1H-indole-4-carboxylique
- le trifluoroacétate du [4-(3H-imidazo[4,5-c]pyridin-2-yl)-9H-fluorèn-9(R,S)-yl]-amide de l'acide quinoléine-5-carbothioique
- le 2-acétylamino-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide quinoléine-4-carboxylique
- le 2-amino-N-[4-(3H-imidazo[4,5-c]pyridin-2-yl)-9H-fluorèn-9(R,S)-yl]-isonicotinamide.
- le 2-éthyl-N-[4-(3H-imidazo[4,5-c]pyridin-2-yl)-9H-fluorèn-9(R,S)-yl]-isonicotinamide
- le [4-(6-fluoro-1H-benzimidazol-2-yl)-9H-fluorèn-9(R,S)-yl]-amide de l'acide quinoléine-5-carboxylique
- le 2-chloro-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluoren-9(R,S)-yl]-amide de l'acide 2-amino-pyrimidine-4-carboxylique
- le [4-(3H-imidazo[4,5-c]pyridin-2-yl)-9H-fluorèn-9(R,S)-yl]-amide de l'acide 1H-pyrrolo[2,3-b]pyridine-4-carboxylique
- le 2-hydroxyméthyl-N-[4-(3H-imidazo[4,5-c]pyridin-2-yl)-9H-fluorèn-9(R,S)-yl]-isonicotinamide
- le N-[4-(3H-imidazo[4,5-c]pyridin-2-yl)-9H-fluorèn-9(R,S)-yl]-3-méthyl-isonicotinamide
- le chlorhydrate du [4-(3H-imidazo[4,5-c]pyridin-2-yl)-9H-fluorèn-9(R,S)-yl]-amide de l'acide 1,8-naphtyridine-4-carboxylique
- le [4-(3H-imidazo[4,5-c]pyridin-2-yl)-9H-fluorèn-9(R,S)-yl]-amide de l'acide 1H-pyrrolo[2,3-b]pyridine-3-carboxylique
- le [4-(3H-imidazo[4,5-c]pyridin-2-yl)-9H-fluorèn-9(R,S)-yl]-amide de l'acide 1-méthyl-1H-pyrrolo[2,3-b]pyridine-3-carboxylique
- le 2-bromo-N-[4-(3H-imidazo[4,5-c]pyridin-2-yl)-9H-fluorèn-9(R,S)-yl]-isonicotinamide
- le 3-hydroxy-N-[4-(3H-imidazo[4,5-c]pyridin-2-yl)-9H-fluorèn-9(R,S)-yl]-isonicotinamide
- le 3-amino-N-[4-(3H-imidazo[4,5-c]pyridin-2-yl)-9H-fluorèn-9(R,S)-yl]-isonicotinamide
- ester de méthyle de l'acide 4-[4-(3H-imidazo[4,5-c]pyridin-2-yl)-9H-fluorèn-9(R,S)-ylcarbamoyl]-pyridine-2-carboxylique
- le [4-(3H-imidazo[4,5-c]pyridin-2-yl)-9H-fluorèn-9(R,S)-yl]-amide de l'acide 1H-indazole-4-carboxylique
- le N-[4-(3H-imidazo[4,5-c]pyridin-2-yl)-9H-fluorèn-9(R,S)-yl]-2-méthylamino-isonicotinamide
- le N-[4-(6-hydroxy-1H-benzimidazol-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide 6-bromo-3H-imidazo[4,5-b]pyridine-7-carboxylique
- ester de 4-(3H-imidazo[4,5-c]pyridin-2-yl)-9H-fluorèn-9-yle de l'acide isonicotinique,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

La présente invention a notamment pour objet les produits de formule (I) ci-dessus dont les noms suivent :
- le [4-(3H-imidazo[4,5-c]pyridin-2-yl)-9H-fluorèn-9(R,S)-yl]-amide de l'acide quinoléine-5-carboxylique.
- énantiomère dextrogyre du [4-(3H-imidazo[4,5-c]pyridin-2-yl)-9H-fluorèn-9(R,S)-yl]-amide de l'acide quinoléine-5-carboxylique
- la 4-(3H-imidazo[4,5-c]pyridin-2-yl)-fluorène-9-one oxime (E).
- le trifluoroacétate du [4-(3H-imidazo[4,5-c]pyridin-2-yl)-9H-fluorèn-9(R,S)-yl]-amide de l'acide 1H-benzotriazole-5-carboxylique.
- le N-[4-(3H-imidazo[4,5-c]pyridin-2-yl)-9H-fluorèn-9(R,S)-yl]-isonicotinamide
- le trifluoroacétate du [4-(3H-imidazo[4,5-c]pyridin-2-yl)-9H-fluorèn-9(R,S)-yl]-amide de l'acide quinoléine-5-carbothioique
- le 2-acétylamino-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide quinoléine-4-carboxylique
- le 2-amino-N-[4-(3H-imidazo[4,5-c]pyridin-2-yl)-9H-fluorèn-9(R,S)-yl]-isonicotinamide.
- le 2-éthyl-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide 2-amino-pyrimidine-4-carboxylique
- le [4-(3H-imidazo[4,5-c]pyridin-2-yl)-9H-fluorèn-9(R,S)-yl]-amide de l'acide 1H-pyrrolo[2,3-b]pyridine-4-carboxylique
- le [4-(3H-imidazo[4,5-c]pyridin-2-yl)-9H-fluorèn-9(R,S)-yl]-amide de l'acide 1H-pyrrolo[2,3-b]pyridine-3-carboxylique
- le 2-bromo-N-[4-(3H-imidazo[4,5-c]pyridin-2-yl)-9H-fluorèn-9(R,S)-yl]-isonicotinamide
- le 3-hydroxy-N-[4-(3H-imidazo[4,5-c]pyridin-2-yl)-9H-fluorèn-9(R,S)-yl]-isonicotinamide
- le 3-amino-N-[4-(3H-imidazo[4,5-c]pyridin-2-yl)-9H-fluorèn-9(R,S)-yl]-isonicotinamide
- ester de méthyle de l'acide 4-[4-(3H-imidazo[4,5-c]pyridin-2-yl)-9H-fluorèn-9(R,S)-ylcarbamoyl]-pyridine-2-carboxylique
- le [4-(3H-imidazo[4,5-c]pyridin-2-yl)-9H-fluorèn-9(R,S)-yl]-amide de l'acide 1H-indazole-4-carboxylique
- le [4-(3H-imidazo[4,5-c]pyridin-2-yl)-9H-fluorèn-9(R,S)-yl]-amide de l'acide 6-bromo-3H-imidazo[4,5-b]pyridine-7-carboxylique
- ester de 4-(3H-imidazo[4,5-c]pyridin-2-yl)-9H-fluoren-9(R,S)-yle de l'acide isonicotinique,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

La présente invention a notamment pour objet les produits de formule (I) ci-dessus dont les noms suivent :
- le [4-(3H-imidazo[4,5-c]pyridin-2-yl)-9H-fluorèn-9(R,S)-yl]-amide de l'acide quinoléine-5-carboxylique.
- le N-[4-(3H-imidazo[4,5-c]pyridin-2-yl)-9H-fluorèn-9(R,S)-yl]-isonicotinamide
- le 2-amino-N-[4-(3H-imidazo[4,5-c]pyridin-2-yl)-9H-fluorèn-9(R,S)-yl]-isonicotinamide.
- le 2-éthyl-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluoren-9(R,S)-yl]-amide de l'acide 2-amino-pyrimidine-4-carboxylique
- le [4-(3H-imidazo[4,5-c]pyridin-2-yl)-9H-fluorèn-9(R,S)-yl]-amide de l'acide 1H-pyrrolo[2,3-b]pyridine-4-carboxylique
- le [4-(3H-imidazo[4,5-c]pyridin-2-yl)-9H-fluorèn-9(R,S)-yl]-amide de l'acide 1H-pyrrolo[2,3-b]pyridine-3-carboxylique
- le 3-amino-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide 6-bromo-3H-imidazo[4,5-b]pyridine-7-carboxylique
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

L'invention a également pour objet des procédés de préparation de produits de formule (I) tels que définis ci-dessus.

De manière générale, des produits de formule générale (I) conformes à l'invention peuvent être préparés selon les diverses méthodes décrites par K. H. Wünsch et A. J. Boulton dans Advances in Heterocyclic Chemistry Vol. 8, 277-302.

De manière générale, des produits de formule générale (I) conformes à l'invention peuvent être avantageusement préparés par l'une au moins des quatre méthodes générales de synthèse indiquées ci-dessous.

### Méthodes générales de synthèses :

Une première méthode générale de synthèse a été développée à partir d'un acide 9-H-fluorèn-9-one-4-carboxylique ou de son chlorure - tel que le chlorure de l'acide 9-H-fluorèn-9-one-4-carboxylique commercial - par formation du noyau de type benzimidazole. Il a été trouvé particulièrement avantageux, dans le cadre de la présente invention, d'opérer en deux étapes successives selon le schéma 1 :

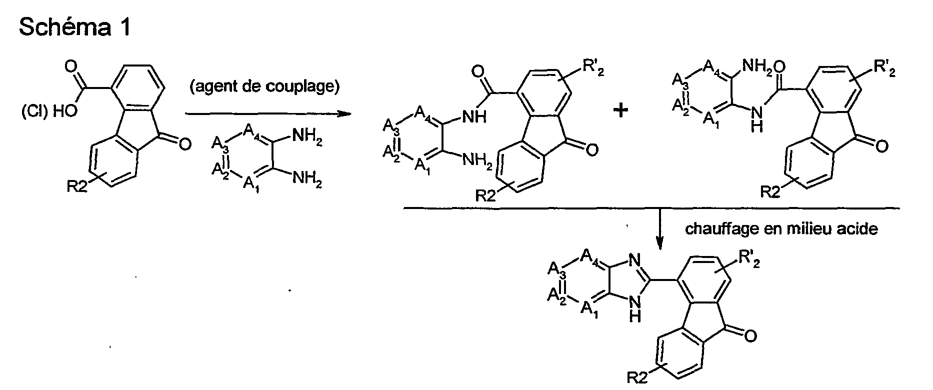

Lorsqu'on utilise un acide fluorèn-9-one-4-carboxylique, il est particulièrement avantageux d'activer cet acide à l'aide d'un agent de couplage connu de l'homme de l'art, tel que chlorhydrate de 1-(3-diméthylaminopropyl)-3-éthylcarbodiimide (EDCI), en présence de 1-hydroxybenzotriazole (HOBT). Diverses conditions de cyclisation du mélange d'amides intermédiaires peuvent être utilisées dans le cadre de l'invention, tels que l'acide acétique ou un mélange d'acide et d'anhydride trifluoroacétiques. Il est également particulièrement avantageux dans le cadre de l'invention d'effectuer ce type de cyclisation thermique en milieu acide par chauffage dans un réacteur micro-onde.

La réaction peut être également effectuée en une seule étape par chauffage dans un agent tel que l'acide polyphosphorique ou dans le trichlorure de phosphore.

Une seconde méthode générale de synthèse avantageuse dans le cadre de l'invention consiste à partir d'un 9-H-fluorèn-9-one-4-carboxaldéhyde - tel que le 9-H-fluorèn-9-one-4-carboxaldéhyde, qui peut être obtenue selon Helv. Chim. Acta 1972, 55, 1973-8 - par formation du noyau de type benzimidazole ou aza-benzimidazole selon le schéma 2 :

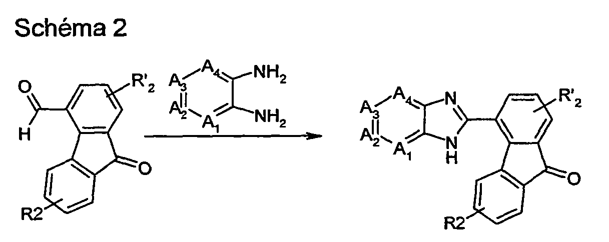

Dans ce cas, il est particulièrement avantageux dans le cadre de l'invention, d'opérer :
- soit par chauffage micro-onde en présence de silice, selon Tetrahedron Lett. 1998 , 39, 4481-84 ;
- soit en présence de dichloro-dicyano-benzoquinone (DDQ), selon Tetrahedron 1995, 51, 5813-18 ;
- soit en présence d'un mélange de chlorure de thionyle et de pyridine, selon E.P. 511187 ;

Dans ce cas, il est plus particulièrement avantageux dans le cadre de l'invention, d'opérer en présence de chlorure ferrique, selon Eur. J. Med. Chem. 2006, 31, 635-42.

Une troisième méthode générale de synthèse consiste à coupler une 9H-4-halo-fluorèn-9-one - telle que la 9H-4-bromo-fluorèn-9-one, qui peut être obtenue selon J. Amer. Chem. Soc. 1935, , 2443-6 ou la 9H-4-iodo-fluorèn-9-one, qui peut être obtenue selon Helv. Chim. Acta 1973, 3044-9 - avec un dérivé organométallique dérivé d'un benzimidazole - ou d'un aza-benzimidazole - dont la fonction NH est protégée, selon le schéma 3 :

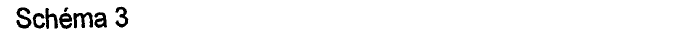

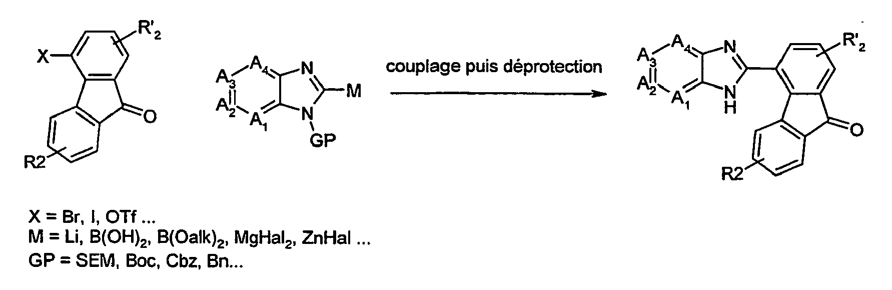

Dans le cadre de l'invention, il est particulièrement avantageux d'utiliser un groupe benzyle (Bn) ou (triméthylsilyléthyl)oxyméthyle (SEM) pour protéger le dérivé de benzimidazole.

Dans le cadre de l'invention, il est particulièrement avantageux d'utiliser soit un lithien soit un acide boronique comme dérivé organométallique de benzimidazole.

Dans le cadre de l'invention, il est particulièrement avantageux d'effectuer le couplage en présence d'un catalyseur dérivé de palladium (0), dans les conditions d'une réaction de type Suzuki.

Le couplage inverse peut également être envisagé, en particulier en utilisant des dérivés de 2-iodo-benzimidazole - ou d'aza-analogue de benzimidazoles

- dont la fonction NH est protégée, avec des dérivés organométalliques de fluorèn-9-ones - dont la fonction carbonyle est protégée selon le schéma 3' :

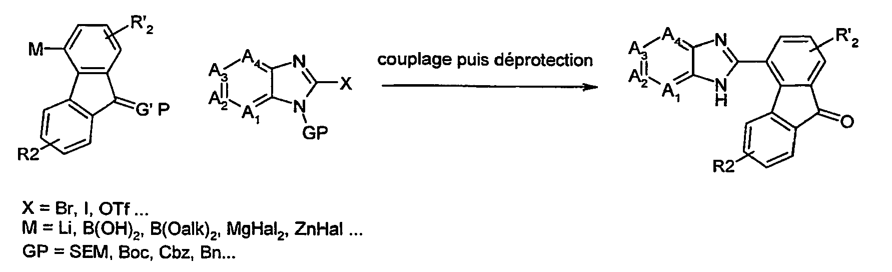

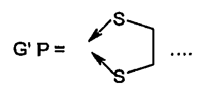

La transformation du radical C=O en radicaux CR1R'1 tels que définis dans la formule générale (I) peut être effectuée selon les méthodes générales connues de l'homme de l'art, en particulier celles décrites dans : Comprehensive Organic Chemistry, par D. Barton et al. (Pergamon Press) ;

Advanced Organic Chemistry, par J. Marsh (Wiley Interscience).

Une quatrième méthode générale de synthèse consiste à effectuer d'abord la transformation du radical C=O d'un ester d'acide de 9-oxo-fluorène-4-carboxylique en radicaux CR1R'1, tels que définis dans la formule générale (I), puis à former le radical de type benzimidazole ou aza-benzimidazole selon le schéma ci-dessous :

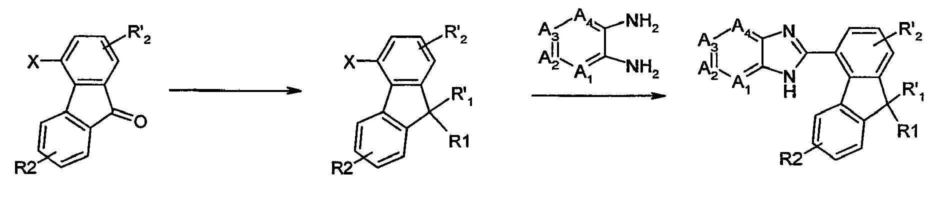

Dans le cadre de l'invention, il est particulièrement avantageux de former le radical de type benzimidazole ou aza-benzimidazole par réaction :
- soit directement à partir de l'ester (X= CO2Me ou CO2Et), en opérant au reflux d'un solvant, tel que toluène, en présence de triméthylaluminium ;
- soit à transformer l'ester en acide (X= CO2H) ou en chlorure d'acide (X = COCI) et à opérer dans les conditions décrites dans la méthode générale 1 ;
- soit à transformer l'ester en aldéhyde (X = CHO) et à opérer dans les conditions décrites dans la méthode générale 2.

Dans ce dernier cas, il est particulièrement avantageux dans le cadre de l'invention d'opérer en deux étapes : en réduisant d'abord l'ester en alcool primaire (X= CH2OH), en particulier à l'aide d'hydrure de diisopropylaluminium ; puis en réoxydant l'alcool primaire formé en aldéhyde (X = CHO), en particulier à l'aide de N-oxyde de 2,2,6,6-tétraméthyl-pipéridine.

Les produits objet de la présente invention sont doués de propriétés pharmacologiques intéressantes: on a constaté qu'ils possédaient notamment des propriétés inhibitrices de l'activité ATPasique de protéines chaperones. Parmi ces protéines chaperones, on cite notamment HSP90.

Les produits répondant à la formule générale (I) tels que définis ci-dessus présentent ainsi une activité inhibitrice de la chaperone Hsp90 importante. Des tests donnés dans la partie expérimentale ci-après illustrent l'activité inhibitrice de produits de la présente invention vis-à-vis de telles protéines. Ces propriétés rendent donc les produits de formule générale (I) de la présente invention utilisables comme médicaments pour le traitement de tumeurs malignes.

Les produits de formule (I) peuvent également être utilisés dans le domaine vétérinaire.

L'invention a donc pour objet l'application, à titre de médicaments, des produits de formule générale (I) pharmaceutiquement acceptables. L'invention a particulièrement pour objet l'application à titre de médicaments, des produits dont les noms suivent
- la 4-(3H-imidazo[4,5-c]pyridin-2-yl)-fluorène-9-one oxime (Z, E).
- la N-[4-(1H-imidazo[4,5-c]pyridin-2-yl)-fluorèn-9-ylidène]-hydrazine, mélange 60/40 d'isomères E et Z
- le [4-(3H-imidazo[4,5-c]pyridin-2-yl)-9H-fluorèn-9(R,S)-yl]-amide de l'acide quinoléine-5-carboxylique.
- énantiomère dextrogyre du [4-(3H-imidazo[4,5-c]pyridin-2-yl)-9H-fluorèn-9(R,S)-yl]-amide de l'acide quinoléine-5-carboxylique
- la 4-(3H-Imidazo[4,5-c]pyridin-2-yl)-fluorène-9-one oxime (E).
- le trifluoroacétate du [4-(3H-imidazo[4,5-c]pyridin-2-yl)-9H-fluorèn-9(R,S)-yl]-amide de l'acide 1H-pyrazole-4-carboxylique
- le trifluoroacétate du N-[4-(3H-imidazo[4,5-c]pyridin-2-yl)-9H-fluorèn-9(R,S)-yl]-succinamide.
- le trifluoroacétate du [4-(3H-imidazo[4,5-c]pyridin-2-yl)-9H-fluorèn-9(R,S)-yl]-amide de l'acide 1H-benzotriazole-5-carboxylique.
- le [4-(3H-imidazo[4,5-c]pyridin-2-yl)-9H-fluorèn-9(R,S)-yl]-amide de l'acide 1H-indazole-5-carboxylique
- le [4-(3H-imidazo[4,5-c]pyridin-2-yl)-9H-fluorèn-9(R,S)-yl]-amide de l'acide 1H-indole-6-carboxylique
- le N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide 1H-indole-4-carboxylique
- le trifluoroacétate du [4-(3H-imidazo[4,5-c]pyridin-2-yl)-9H-fluorèn-9(R,S)-yl]-amide de l'acide quinoléine-5-carbothioique
- le 2-acétylamino-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide quinoléine-4-carboxylique
- le 2-amino-N-[4-(3H-imidazo[4,5-c]pyridin-2-yl)-9H-fluorèn-9(R,S)-yl]-isonicotinamide.
- le 2-éthyl-N-[4-(3H-imidazo[4,5-c]pyridin-2-yl)-9H-fluorèn-9(R,S)-yl]-isonicotinamide
- le [4-(6-fluoro-1H-benzimidazol-2-yl)-9H-fluorèn-9(R,S)-yl]-amide de l'acide quinoléine-5-carboxylique
- le 2-chloro-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluoren-9(R,S)-yl]-amide de l'acide 2-amino-pyrimidine-4-carboxylique
- le [4-(3H-imidazo[4,5-c]pyridin-2-yl)-9H-fluorèn-9(R,S)-yl]-amide de l'acide 1H-pyrrolo[2,3-b]pyridine-4-carboxylique
- le 2-hydroxyméthyl-N-[4-(3H-imidazo[4,5-c]pyridin-2-yl)-9H-fluorèn-9(R,S)-yl]-isonicotinamide
- le N-[4-(3H-imidazo[4,5-c]pyridin-2-yl)-9H-fluorèn-9(R,S)-yl]-3-méthyl-isonicotinamide
- le chlorhydrate du [4-(3H-imidazo[4,5-c]pyridin-2-yl)-9H-fluorèn-9(R,S)-yl]-amide de l'acide 1,8-naphtyridine-4-carboxylique
- le [4-(3H-imidazo[4,5-c]pyridin-2-yl)-9H-fluorèn-9(R,S)-yl]-amide de l'acide 1H-pyrrolo[2,3-b]pyridine-3-carboxylique
- le [4-(3H-imidazo[4,5-c]pyridin-2-yl)-9H-fluorèn-9(R,S)-yl]-amide de l'acide 1-méthyl-1H-pyrrolo[2,3-b]pyridine-3-carboxylique
- le 2-bromo-N-[4-(3H-imidazo[4,5-c]pyridin-2-yl)-9H-fluorèn-9(R,S)-yl]-isonicotinamide
- le 3-hydroxy-N-[4-(3H-imidazo[4,5-c]pyridin-2-yl)-9H-fluorèn-9(R,S)-yl]-isonicotinamide
- le 3-amino-N-[4-(3H-imidazo[4,5-c]pyridin-2-yl)-9H-fluorèn-9(R,S)-yl]-isonicotinamide
- l'ester de méthyle de l'acide 4-[4-(3H-imidazo[4,5-c]pyridin-2-yl)-9H-fluorèn-9(R,S)-ylcarbamoyl]-pyridine-2-carboxylique
- le [4-(3H-imidazo[4,5-c]pyridin-2-yl)-9H-fluorèn-9(R,S)-yl]-amide de l'acide 1H-indazole-4-carboxylique
- le N-[4-(3H-imidazo[4,5-c]pyridin-2-yl)-9H-fluorèn-9(R,S)-yl]-2-méthylamino-isonicotinamide
- le N-[4-(6-hydroxy-1H-benzimidazol-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide 6-bromo-3H-imidazo[4,5-b]pyridine-7-carboxylique
- l'ester de 4-(3H-imidazo[4,5-c]pyridin-2-yl)-9H-fluorèn-9(R,S)-yle de l'acide isonicotinique,
- le N-[4-(3H-imidazo[4,5-c]pyridin-2-yl)-9H-fluorèn-9(R,S)-yl]-amide de l'acide 2-tert-butoxycarbonylamino-isonicotinique.
- le N-[4-(3H-imidazo[4,5-c]pyridin-2-yl)-9H-fluorèn-9(R,S)-yl]-amide de l'acide 3-chloro-6-méthoxy-quinoléine-4-carboxylique.
- le N-[4-(3H-imidazo[4,5-c]pyridin-2-yl)-9H-fluorèn-9(R,S)-yl]-amide de l'acide 3-hydroxy-quinoléine-4-carboxylique.
- le N-[4-(3H-imidazo[4,5-c]pyridin-2-yl)-9H-fluorèn-9(R,S)-yl]-amide de l'acide 2-amino-5-chloro-pyrimidine-4-carboxylique.
- le [4-(3H-imidazo[4,5-c]pyridin-2-yl)-9H-fluorèn-9(R,S)-yl]-amide de l'acide 3-bromo-1H-pyrrolo[2,3-b]pyridine-4-carboxylique.
- l'énantiomère dextrogyre du [4-(3H-imidazo[4,5-c]pyridin-2-yl)-9H-fluorèn-9(R,S)-yl]-amide de l'acide 1H-pyrrolo[2,3-b]pyridine-4-carboxylique.
- le N-[4-(3H-imidazo[4,5-c]pyridin-2-yl)-9H-fluorèn-9(R,S)-yl]-amide de l'acide 9H-purine-6-carboxylique.
- le N-[4-(3H-imidazo[4,5-c]pyridin-2-yl)-9H-fluorèn-9(R,S)-yl]-amide de l'acide 2-amino-6-méthyl-pyrimidine-4-carboxylique.
- le [4-(3H-imidazo[4,5-c]pyridin-2-yl)-9H-fluorène-9(R,S)-yl]-amide de l'acide 5-amino-3H-1,2,3-triazole-4-carboxylique.
- le N-[4-(3H-imidazo[4,5-c]pyridin-2-yl)-9H-fluorèn-9(R,S)-yl]-amide de l'acide 6-méthyl-2-méthylamino-pyrimidine-4-carboxylique.
- le N-[4-(3H-imidazo[4,5-c]pyridin-2-yl)-9H-fluorèn-9(R,S)-yl]-amide de l'acide 6-méthoxy-quinoléine-4-carboxylique.
- le 3,5-dihydroxy-N-[4-(3H-imidazo[4,5-c]pyridin-2-yl)-9H-fluorèn-9(R,S)-yl]-benzamide.
- le N-[4-(3H-imidazo[4,5-c]pyridin-2-yl)-9H-fluorèn-9(R,S)-yl]-amide de l'acide pyrimidine-4-carboxylique.
- le 4-hydroxy-N-[4-(3H-imidazo[4,5-c]pyridin-2-yl)-9H-fluorèn-9(R,S)-yl]-benzamide.
- le 2,4-dihydroxy-N-[4-(3H-imidazo[4,5-c]pyridin-2-yl)-9H-fluorèn-9(R,S)-yl]-benzamide.
- le [4-(5-cyano-6-fluoro-1H-benzimidazol-2-yl)-9H-fluorén-9(R,S)-yl]-amide de l'acide 1H-pyrrolo[2,3-b]pyridine-4-carboxylique.
- le 4-(1H-benzimidazol-2-yl)-9H-fluorène-9(R,S)-yl]-amide de l'acide 2-amino-5-chloro-pyrimidine-4-carboxylique.
- le [4-(5-fluoro-1H-benzimidazol-2-yl)-9H-fluorène-9(R,S)-yl]-amide de l'acide 2-amino-5-chloro-pyrimidine-4-carboxylique.
- le [4-(5-fluoro-1H-benzimidazol-2-yl)-9H-fluorène-9(R,S)-yl]-amide de l'acide 1H-pyrrolo[2,3-b]pyridine-4-carboxylique.
- le [4-(3H-imidazo[4,5-c]pyridin-2-yl)-9H-fluorène-9(R,S)-yl]-amide de l'acide 2,3-diméthyl-quinoxaline-5-carboxylique.
- le [4-(3H-imidazo[4,5-c]pyridin-2-yl)-9H-fluorène-9(R,S)-yl]-amide de l'acide 3-amino-1H-pyrazole-4-carboxylique.
- l'énantiomère dextrogyre du N-[4-(3H-imidazo[4,5-c]pyridin-2-yl)-9H-fluorèn-9(R,S)-yl]-amide de l'acide 2-amino-5-chloro-pyrimidine-4-carboxylique.
- l'énantiomère lévogyre du N-[4-(3H-imidazo[4,5-c]pyridin-2-yl)-9H-fluorèn-9(R,S)-yl]-amide de l'acide 2-amino-5-chloro-pyrimidine-4-carboxylique.
- le [4-(3H-imidazo[4,5-c]pyridin-2-yl)-9H-fluorène-9(R,S)-yl]-amide de l'acide 3-méthyl-quinoxaline-5-carboxylique.
- le [4-(3H-imidazo[4,5-c]pyridin-2-yl)-9H-fluorène-9(R,S)-yl]-amide de l'acide quinoxaline-5-carboxylique.
- le [4-(9H-purine-8-yl)-9H-fluorène-9(R,S)-yl]-amide de l'acide 1H-pyrrolo[2,3-b]pyridine-4-carboxylique
- le [4-(1H-benzimidazol-2-yl)-9H-fluorène-9(R,S)-yl]-amide de l'acide 1H-pyrrolo[2,3-b]pyridine-4-carboxylique
- ie [4-(5-trifluorométhyl-1H-benzimidazol-2-yl)-9H-fluorène-9(R,S)-yl]-amide de l'acide 1H-pyrrolo[2,3-b]pyridine-4-carboxylique
- le [4-(5-méthoxycarbonyl-1H-benzimidazol-2-yl)-9H-fluorène-9(R,S)-yl]-amide de l'acide 1H-pyrrolo[2,3-b]pyridine-4-carboxylique
- le [4-(5-carboxy-1H-benzimidazol-2-yl)-9H-fluorène-9(R,S)-yl]-amide de l'acide 1H-pyrrolo[2,3-b]pyridine-4-carboxylique
- le [4-(4-méthyl-1H-benzimidazol-2-yl)-9H-fluorène-9(R,S)-yl]-amide de l'acide 1H-pyrrolo[2,3-b]pyridine-4-carboxylique
- le [4-(5-carboxamido-1H-benzimidazol-2-yl)-9H-fluorène-9(R,S)-yl]-amide de l'acide 1H-pyrrolo[2,3-b]pyridine-4-carboxylique
- le [4-(6-sulfamoyl-1H-benzimidazol-2-yl)-9H-fluorèn-9(R,S)-yl]-amide de l'acide 1H-pyrrolo[2,3-b]pyridine-4-carboxylique
- le [4-(5-trifluorométhoxy-1H-benzimidazol-2-yl) -9H-fluorène-9(R,S)-yl]-amide de l'acide 1H-pyrrolo[2,3-b]pyridine-4-carboxylique
- le [4-(5-cyano-1H-benzimidazol-2-yl)-9H-fluorène-9(R,S)-yl]-amide de l'acide 1H-pyrrolo[2,3-b]pyridine-4-carboxylique
- le diastéréosisomère D du [4-(3H-imidazo[4,5-c]pyridin-2-yl)-9H-fluorèn-9(R,S)-yl]-amide de l'acide 2-(3-acétyl-2,2-diméthyl-cyclobutan-1-yl) acétique
- le [4-(6-chloro-1H-benzimidazol-2-yl)-9H-fluorèn-9(R,S)-yl]-amide de l'acide 1H-pyrrolo[2,3-b]pyridine-4-carboxylique :
- le [4-(5-fluoro-6-morpholino-1H-benzimidazole-2-yl)-9H-fluorène-9(R,S)-yl]-amide de l'acide 1H-pyrrolo[2,3-b]pyridine-4-carboxylique
- le [4-(6-chloro-5-fluoro-1H-benzimidazole-2-yl)-9H-fluorène-9(R,S)-yl]-amide de l'acide 1H-pyrrolo[2,3-b]pyridine-4-carboxylique
- le [4-(4,5,6,7-tétrahydro-1H-benzimidazol-2-yl)-9H-fluorèn-9(R,S)-yl]-amide de l'acide 1H-pyrrolo[2,3-b]pyridine-4-carboxylique
- le [2-amino-5-(1H-benzimidazol-2-yl)-9H-fluorène-9(R,S)-yl]-amide de l'acide 1H-pyrrolo[2,3-b]pyridine-4-carboxylique
- le [4-(5-hydroxy-5H-imidazo[4,5-c]pyridin-2-yl)-9H-fluorèn-9(R,S)-yl]-amide de l'acide 1H-pyrrolo[2,3-b]pyridine-4-carboxylique
- le [4-(5-hydroxy-1H-benzimidazole-2-yl)-9H-fluorène-9(R,S)-yl]-amide de l'acide 1H-pyrrolo[2,3-b]pyridine-4-carboxylique
- ie [4-(5-méthylaminocarbonyl-1H-benzimidazol-2-yl)-9H-fluorène-9(R,S)-yl]-amide de l'acide 1H-pyrrolo[2,3-b]pyridine-4-carboxylique
- le [4-(5-diméthylaminocarbonyl-1H-benzimidazol-2-yl)-9H-fluorène-9(R,S)-yl]-amide de l'acide 1H-pyrrolo[2,3-b]pyridine-4-carboxylique
- le [4-5-(2-diméthylamino-éthyl)aminocarbonyl-1H-benzimidazol-2-yl)-9H-fluorène-9(R,S)-yl]-amide de l'acide 1H-pyrrolo[2,3-b]pyridine-4-carboxylique
- le [4-(4,5,6,7-tétrafluoro-1H-benzimidazol-2-yl)-9H-fluorèn-9(R,S)-yl]-amide de l'acide 1H-pyrrolo[2,3-b]pyridine-4-carboxylique
- le 4-[5-(3-méthoxypropyl)aminocarbonyl-1H-benzimidazol-2-yl)-9H-fluorène-9(R,S)-yl]-amide de l'acide 1H-pyrrolo[2,3-b]pyridine-4-carboxylique
- le [4-(5-(4-méthyl-pipérazine-1-yl)carbonyl-1H-benzimidazol-2-yl)-9H-fluorène-9(R,S)-yl]-amide de l'acide 1H-pyrrolo[2,3-b]pyridine-4-carboxylique
- le [4-(6-diméthylsulfamoyl-1H-benzimidazol-2-yl)-9H-fluorèn-9(R,S)-yl]-amide de l'acide 1H-pyrrolo[2,3-b]pyridine-4-carboxylique
- le [4-(5-(pyrrolidin-1-yl)carbonyl)-1H-benzimidazol-2-yl)-9H-fluorène-9(R,S)-yl]-amide de l'acide 1H-pyrrolo[2,3-b]pyridine-4-carboxylique
- l'énantiomère dextrogyre, de l'acide 2-{9(R,S)-[(1H-pyrrolo[2,3-b]pyridine-4-carbonyl)-amino]-9H-fluorèn-4-yl}-1H-benzimidazole-5-carboxylique
- le {{4-{5-[2-(pyrrolidin-1-yl)éthylaminocarbony)]-1H-benzimidazole-2-yl}-9H-fluorène-9(R,S)-yl}}amide de l'acide 1H-pyrrolo[2,3-b]pyridine-4-carboxylique
- le [4-(6-diméthylamino-1H-benzimidazol-2-yl)-9H-fluoren-9(R,S)-yl]-amide de l'acide 1H-pyrrolo[2,3-b]pyridine-4-carboxylique
- le {4-[6-(4-méthyl-pipérazin-1-yl)-1H-benzimidazol-2-yl]-9H-fluorèn-9(R,S)-yl}-amide de l'acide 1H-pyrrolo[2,3-b]pyridine-4-carboxylique
- le [4-[6-(méthyl-4(5)-imidazolin-2-yl)-1H-benzimidazol-2-yl]-9H-fluorèn-9(R,S)-yl]-amide de l'acide 1H-pyrrolo[2,3-b]pyridine-4-carboxylique
- le {{4-{5-[(3-diméthylamino-propyl)aminocarbonyl]-1H-benzimidazol-2-yl}-9H-fluorène-9(R,S)-yl}}-amide de l'acide 1H-pyrrolo[2,3-b]pyridine-4-carboxylique
- le {4-[5-fluoro-6-(4-méthyl-pipérazin-1-yl)-1H-benzimidazol-2-yl]-9H-fluorèn-9(R,S)-yl}-amide de l'acide 1H-pyrrolo[2,3-b]pyridine-4-carboxylique
- le {{4-{5-[(3-diméthylamino-propyl)carbonyloxy]-1H-benzimidazol-2-yl}-9H-fluorène-9(R,S)-yl}}-amide de l'acide 1H-pyrrolo[2,3-b]pyridine-4-carboxylique
- le [4-(6-amino-1H-benzimidazol-2-yl)-9H-fluorèn-9-(R,S)-yl]-amide de l'acide 1H-pyrrolo[2,3-b]pyridine-4-carboxylique.
- le [4-(6-méthyl-1H-benzimidazol-2-yl)-9H-fluorène-9(R,S)-yl]-amide de l'acide 1H-pyrrolo[2,3-b]pyridine-4-carboxylique
- le {{4-{5-[(3-hydroxy-propyl)aminocarbonyl]-1H-benzimidazole-2-yl}-9H-fluorène-9(R,S)-yl}}-amide de l'acide 1H-pyrrolo[2,3-b]pyridine-4-carboxylique
- l'énantiomère dextrogyre du {4-[5-cyano-1H-benzimidazol-2-yl]-9H-fluorène-9(R,S)-yl}-amide de l'acide 1H-pyrrolo[2,3-b]pyridine-4-carboxylique
- le [4-(3H-imidazo[4,5-c]pyridin-2-yl)-9H-fluorèn-9(R,S)-yl]-amide de l'acide 7H-pyrrolo[2,3-d]pyrimidine-4-carboxylique.
- le [4-(6-amino-1H-benzimidazol-2-yl)-9H-fluorèn-9(R,S)-yl]-amide de l'acide 1H-pyrrolo[2,3-b]pyridine-4-carboxylique
- l'énantiomère dextrogyre du {4-[5-fluoro-1H-benzimidazol-2-yl]-9H-fluorène-9(R,S)-yl}-amide de l'acide 1H-pyrrolo[2,3-b]pyridine-4-carboxylique
- l'ester méthylique de l'acide 2-{9(R,S)-[(1H-pyrrolo[2,3-b]pyridine-4-carbonyl)-amino]-9H-fluorèn-4-yl}-1H-imidazo[4,5-c]pyridine-6-carboxylique
- le [4-(6-fluoro-5-methoxy-1H-benzimidazol-2-yl)-9H-fluorène-9(R,S)-yl]-amide de l'acide 1H-Pyrrolo[2,3-b]pyridine-4-carboxylique
- le [4-(6-fluoro-5-hydroxy-1H-benzimidazol-2-yl)-9H-fluorène-9(R,S)-yl]-amide de l'acide 1H-pyrrolo[2,3-b]pyridine-4-carboxylique
- le {4-[5-fluoro-6-(3-méthoxy-propoxy)-1H-benzimidazol-2-yl]-9H-fluorèn-9(R,S)-yl}-amide de l'acide 1H-pyrrolo[2,3-b]pyridine-4-carboxylique
- le {4-[6-(3-diméthylamino-propoxy)-5-fluoro-1H-benzimidazol-2-yl]-9H-fluorèn-9(R,S)-yl}-amide de l'acide 1H-pyrrolo[2,3-b]pyridine-4-carboxylique

ainsi que leurs prodrugs, lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

L'invention a plus particulièrement pour objet l'application à titre de médicaments, des produits dont les noms suivent :
- le [4-(3H-imidazo[4,5-c]pyridin-2-yl)-9H-fluorèn-9(R,S)-yl]-amide de l'acide quinoléine-5-carboxylique.
- énantiomère dextrogyre du [4-(3H-imidazo[4,5-c]pyridin-2-yl)-9H-fluorèn-9(R,S)-yl]-amide de l'acide quinoléine-5-carboxylique
- la 4-(3H-Imidazo[4,5-c]pyridin-2-yl)-fluorène-9-one oxime (E).
- le trifluoroacétate du [4-(3H-imidazo[4,5-c]pyridin-2-yl)-9H-fluorèn-9(R,S)-yl]-amide de l'acide 1H-benzotriazole-5-carboxylique.
- le N-[4-(3H-imidazo[4,5-c]pyridin-2-yl)-9H-fluorèn-9(R,S)-yl]-isonicotinamide
- le trifluoroacétate du [4-(3H-imidazo[4,5-c]pyridin-2-yl)-9H-fluorèn-9(R,S)-yl]-amide de l'acide quinoléine-5-carbothioique
- le 2-acétylamino-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide quinoléine-4-carboxylique
- le 2-amino-N-[4-(3H-imidazo[4,5-c]pyridin-2-yl)-9H-fluorèn-9(R,S)-yl]-isonicotinamide.
- le 2-éthyl-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluoren-9(R,S)-yl]-amide de l'acide 2-amino-pyrimidine-4-carboxylique
- le [4-(3H-imidazo[4,5-c]pyridin-2-yl)-9H-fluorèn-9(R,S)-yl]-amide de l'acide 1H-pyrrolo[2,3-b]pyridine-4-carboxylique
- le [4-(3H-imidazo[4,5-c]pyridin-2-yl)-9H-fluorèn-9(R,S)-yl]-amide de l'acide 1H-pyrrolo[2,3-b]pyridine-3-carboxylique
- le 2-bromo-N-[4-(3H-imidazo[4,5-c]pyridin-2-yl)-9H-fluorèn-9(R,S)-yl]-isonicotinamide
- le 3-hydroxy-N-[4-(3H-imidazo[4,5-c]pyridin-2-yl)-9H-fluorèn-9(R,S)-yl]-isonicotinamide
- le 3-amino-N-[4-(3H-imidazo[4,5-c]pyridin-2-yl)-9H-fluorèn-9(R,S)-yl]-isonicotinamide
- l'ester de méthyle de l'acide 4-[4-(3H-imidazo[4,5-c]pyridin-2-yl)-9H-fluorèn-9(R,S)-ylcarbamoyl]-pyridine-2-carboxylique
- le [4-(3H-imidazo[4,5-c]pyridin-2-yl)-9H-fluorèn-9(R,S)-yl]-amide de l'acide 1H-indazole-4-carboxylique
- le [4-(3H-imidazo[4,5-c]pyridin-2-yl)-9H-fluorèn-9(R,S)-yl]-amide de l'acide 6-bromo-3H-imidazo[4,5-b]pyridine-7-carboxylique
- l'ester de 4-(3H-imidazo[4,5-c]pyridin-2-yl)-9H-fluorèn-9(R,S)-yle de l'acide isonicotinique,
ainsi que leurs prodrugs, lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

L'invention a encore plus particulièrement pour objet l'application à titre de médicaments, des produits dont les noms suivent :
- le [4-(3H-imidazo[4,5-c]pyridin-2-yl)-9H-fluorèn-9(R,S)-yl]-amide de l'acide quinoléine-5-carboxylique
- le N-[4-(3H-imidazo[4,5-c]pyridin-2-yl)-9H-fluorèn-9(R,S)-yl]-isonicotinamide
- le 2-amino-N-[4-(3H-imidazo[4,5-c]pyridin-2-yl)-9H-fluorèn-9(R,S)-yl]-isonicotinamide.
- le 2-éthyl-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluoren-9(R,S)-yl]-amide de l'acide 2-amino-pyrimidine-4-carboxylique
- le [4-(3H-imidazo[4,5-c]pyridin-2-yl)-9H-fluorèn-9(R,S)-yl]-amide de l'acide 1H-pyrrolo[2,3-b]pyridine-4-carboxylique
- le [4-(3H-imidazo[4,5-c]pyridin-2-yl)-9H-fluorèn-9(R,S)-yl]-amide de l'acide 1H-pyrrolo[2,3-b]pyridine-3-carboxylique
- le 3-amino-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide 6-bromo-3H-imidazo[4,5-b]pyridine-7-carboxylique
ainsi que leurs prodrugs, lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

Les produits peuvent être administrés par voie parentérale, buccale, perlinguale, rectale ou topique.

L'invention a aussi pour objet les compositions pharmaceutiques, caractérisées en ce qu'elles renferment, à titre de principe actif, un au moins des médicaments de formule générale (I).

Ces compositions peuvent être présentées sous forme de solutions ou de suspensions injectables, de comprimés, de comprimés enrobés, de capsules, de sirops, de suppositoires, de crèmes, de pommades et de lotions. Ces formes pharmaceutiques sont préparées selon les méthodes usuelles. Le principe actif peut être incorporé à des excipients habituellement employés dans ces compositions, tels que les véhicules aqueux ou non, le talc, la gomme arabique, le lactose, l'amidon, le stéarate de magnésium, le beurre de cacao, les corps gras d'origine animale ou végétale, les dérivés paraffiniques, les glycols, les divers agents mouillants, dispersants ou émulsifiants, les conservateurs.

La dose usuelle, variable selon le sujet traité et l'affection en cause, peut être, par exemple, de 10 mg à 500 mg par jour chez l'homme, par voie orale.

La présente invention concerne ainsi l'utilisation de produits de formule (I) tels que définis ci -dessus ou de sels pharmaceutiquement acceptables desdits produits de formule (I) pour la préparation de médicaments destinés à inhiber l'activité de protéines chaperones

La présente invention concerne ainsi particulièrement l'utilisation de produits de formule (I) telle que définie ci-dessus ou de sels pharmaceutiquement acceptables desdits produits de formule (I) dans laquelle la protéine chaperone est HSP90.

La présente invention concerne également l'utilisation de produits de formule (I) telle que définie ci-dessus ou de sels pharmaceutiquement acceptables desdits produits de formule (I) dans laquelle la protéine kinase est dans une culture cellulaire et également cette utilisation dans un mammifère.

La présente invention concerne ainsi l'utilisation de produits de formule (I) telle que définie ci-dessus ou de sels pharmaceutiquement acceptables desdits produits de formule (I) pour la préparation d'un médicament destiné à prévenir ou traiter une maladie caractérisée par le dérèglement de l'activité d'une protéine kinase et notamment une telle maladie chez un mammifère. La présente invention concerne l'utilisation de produits de formule (I) telle que définie ci-dessus ou de sels pharmaceutiquement acceptables desdits produits de formule (I) pour la préparation d'un médicament destiné à prévenir ou traiter une maladie appartenant au groupe suivant: désordres de la prolifération de vaisseaux sanguins, désordres fibrotiques, désordres de la prolifération de cellules mésangiales, désordres métaboliques, allergies, asthme, thromboses, maladies du système nerveux, rétinopathies, psoriasis, arthrite rhumatoïde, diabètes, dégénération musculaire, maladies en oncologie, cancers.

La présente invention concerne ainsi l'utilisation de produits de formule (I) telle que définie ci-dessus ou de sels pharmaceutiquement acceptables desdits produits de formule (I) pour la préparation d'un médicament destiné à traiter des maladies en oncologie.

La présente invention concerne particulièrement l'utilisation de produits de formule (I) telle que définie ci-dessus ou de sels pharmaceutiquement acceptables desdits produits de formule (I) pour la préparation d'un médicament destiné à traiter des cancers.

Parmi ces cancers, la présente invention s'intéresse tout particulièrement au traitement des tumeurs solides et au traitement de cancers résistants aux agents cytotoxiques

La présente invention concerne ainsi notamment l'utilisation de produits de formule (I) telle que définie à l'une quelconque des revendications précédentes ou de sels pharmaceutiquement acceptables desdits produits de formule (I) pour la préparation d'un médicament destiné à traiter des cancers parmi lesquels les cancers du poumon, du sein et de l'ovaire, les glioblastomes, les leucémies myéloîdes chroniques, les leucémies lymphoblastiques aigûes, les cancers de la prostate, du pancréas et du colon, les mélanomes métastatiques, les tumeurs de la thyroîde et les carcinomes rénaux.

Aussi parmi les principales indications potentielles d'inhibiteurs d'Hsp90, peut-on citer, à titre non limitatif:
- les cancers du poumon « à non petites cellules », les cancers du sein, les cancers de l'ovaire ete les glioblastomes qui surexpriment EGF-R ou HER2 ;
- les leucémies myéloîdes chroniques qui surexpriment Bcr-Abl ;
- les leucémies lymphoblastiques aigûes qui surexpriment Flt-3 ;
- les cancers du sein, de la prostate, du poumon, du pancrés, du colon ou de l'ovaire qui surexpriment Akt ;
- les mélanomes metastatiques et les tumeurs de la thyroîde qui surexpriment la forme mutée de la protéine B-Raf ;
- les cancers de la prostate androgène-dépendants et androgène-indépendants ;
- les cancers du sein estrogène-dépendants et estrogène-indépendants ;
- les carcinomes rénaux qui surexpriment HIF-1a ou la protéine c-met mutée. La présente invention s'intéresse encore plus particulièrement au traitement du cancer du sein, du colon et des poumons.

La présente invention concerne aussi l'utilisation de produits de formule (I) telle que définie ci-dessus ou de sels pharmaceutiquement acceptables desdits produits de formule (I) pour la préparation d'un médicament destiné à la chimiothérapie de cancers.

A titre de médicaments selon la présente invention destinés à la chimiothérapie de cancers, les produits de formule (I) selon la présente invention peuvent être utilisés seuls ou en association avec chimiothérapie ou radiothérapie ou alternativement en association avec d'autres agents thérapeutiques.

La présente invention concerne ainsi notamment les compositions pharmaceutiques telles que définies ci-dessus contenant en plus, des principes actifs d'autres médicaments de chimiothérapie contre le cancer. De tels agents thérapeutiques peuvent être des agents anti-tumoraux utilisés communément.

Comme exemples d'inhibiteurs connus de protéines kinases, on peut citer notamment la butyrolactone, le flavopiridol, la 2-(2-hydroxyéthylamino)-6-benzylamino-9-méthylpurine, l'olomucine, le Glivec ainsi que l'Iressa.

Les produits de formule (I) selon la présente invention peuvent ainsi également être avantageusement utilisés en combinaison avec des agents anti-prolifératifs : à titre d'exemples de tels agents anti-prolifératifs mais sans toutefois se limiter à cette liste, on peut citer les inhibiteurs d'aromatase, les antiestrogènes, les inhibiteurs de topoisomérase I, les inhibiteurs de topoisomérase II, les agents actifs sur les microtubules, les agents d'alkylation, les inhibiteurs d'histone désacétylase, les inhibiteurs de farnésyl transférase, les inhibiteurs de COX-2, les inhibiteurs de MMP, les inhibiteurs de mTOR, les antimétabolites antinéoplastique, les composés du platine, les composés faisant décroître l'activité des protéines kinases et également les composés anti-angiogéniques, les agonistes de la gonadoréline, les anti-androgènes, les bengamides, les biphophonates et le trastuzumab.

On peut citer ainsi à titre d'exemples, des agents anti-microtubules comme les taxoides, vinka-alkaloides, des agents d'alkylation tels que cyclophosphamide, des agents DNA-intercalant comme le cis-platinum, des agents interactifs sur topoisomérase comme la camptothécine et dérivés, les anthracyclines comme l'adriamycine, des antimétabolites comme le 5-fluorouracile et dérivés et analogues.

La présente invention concerne donc des produits de formule (I) comme inhibiteurs de protéines chaperones, lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques pharmaceutiquement acceptables desdits produits de formule (I) ainsi que leurs prodrugs.

La présente invention concerne particulièrement des produits de formule (I) tels que définis ci-dessus comme inhibiteurs de HSP90.

Les produits de formule (I) selon la présente invention peuvent être préparés par l'application ou l'adaptation de méthodes connues et notamment des méthodes décrites dans la littérature comme par exemple celles décrites par R.C.Larock dans: Comprehensive Organic Transformations, VCH publishers, 1989.

Dans les réactions décrites ci-après, il peut être nécessaire de protéger des groupes fonctionnels réactifs tels que par exemple des groupements hydroxy, amino, imino, thio ou carboxy, quand ceux-ci sont désirés dans le produit final mais quand leur participation n'est pas souhaitée dans les réactions de synthèse des produits de formule (I). On peut utiliser des groupes protecteurs conventionnels en accord avec les pratiques usuelles standard comme ceux décrits par exemple par T.W. Greene and P.G.M.Wuts dans "Protective Groups in Organic Chemistry" John Wiley and Sons, 1991.

La partie expérimentale ci-après donne des exemples non limitatifs de produits de départ: d'autres produits de départ peuvent être trouvés dans le commerce ou préparés selon les méthodes usuelles connues de l'homme du métier.

### Exemples illustrant l'invention : Les exemples dont la préparation suit illustrent la présente invention sans toutefois la limiter.

Tous les exemples décrits ont été caractérisés par spectroscopie RMN du proton et par spectroscopie de masse, la majorité de ces exemples ont également été caractérisés en spectroscopie Infra Rouge.

### Exemple 1 : Synthèse de la 4-(3H-Imidazo[4,5-c]pyridin-2-yl)-fluorène-9-one

Etape 1 : Dans un tricol de 1 L, on dissout 6,75 g de 3,4-diamino-pyridine dans 500 mL de dichlorométhane, puis on ajoute, successivement à température ambiante, 11,49 mL de triéthylamine, préalablement séchée sur potasse, et 10 g de chlorure de l'acide fluorèn-9-one-4-carboxylique. Après 4 heures d'agitation à température ambiante, le précipité formé est essoré, lavé avec une solution d'hydrogénocarbonate de sodium puis à l'eau et séché en étuve à 50° pendant une nuit. On obtient ainsi 10,5 g du mélange équimoléculaire d'amides utilisé tel quel à l'étape suivante.

Etape 2 : Dans un réacteur micro-onde de 100 W, on chauffe, à 109°C pendant 20 minutes, une solution de 20 g du mélange d'amide, obtenu précédemment, dans un mélange de 40 mL d'anhydride trifluoroacétique, 80 mL d'acide chlorhydrique à 36% et 312 mL d'acide trifluoroacétique, la réaction étant réalisée par fractions de 50 mL. Après refroidissement, les diverses opérations sont jointes et additionnées de 1 L d'eau et 1 L de dichlorométhane. La phase organique est séparée, puis la phase aqueuse est amenée à pH = 8-9 par addition d'une solution saturée d'hydrogénocarbonate de potassium. Le précipité formé est essoré, lavé à l'eau et séché en étuve à 50°C. On obtient ainsi 18,8 g de 4-(3H-Imidazo[4,5-c]pyridin-2-yl)-fluorène-9-one, sous forme d'une poudre jaune dont les caractéristiques sont les suivantes :
Point de fusion Kofler = 236-38°C
Spectre de masse (E/I) : m/z = 297 (M+)
Spectre RMN 1H (300 MHz, □ en ppm, DMSO) : 7,40 (td, J = 7,5 et 1,0 Hz, 1H) ; 7,49 (td, J = 7,5 et 1,0 Hz, 1H) ; 7,59 (t, J = 7,5 Hz, 1H) ; de 7,62 à 7,73 (m, 3H) ; 7,82 (dd, J = 7,5 et 1,0 Hz, 1H) ; 7,90 (dd, J = 7,5 et 1,0 Hz, 1H) ; 8,41 (d large, J = 5,5 Hz, 1H) ; 9,08 (s large, 1H) ; de 12,5 à 13,5 (m très étalé, 1H).

La 4-(3H-Imidazo[4,5-c]pyridin-2-yl)-fluorène-9-one peut également être obtenue en une étape soit par chauffage à 200° dans l'acide polyphosphorique d'un mélange équimoléculaire de 3,4-diamino-pyridine et d'acide fluorène-4-one-9-carboxylique, soit par chauffage dans l'acide acétique entre 90 et 100°C.

### Exemple 2 : Synthèse du (6-chloro-pyridin-3-yl-méthyl)-amide de l'acide 4-(1H-imidazo[4,5-c]pyridin-2-yl)-9H-fluorène-9(R,S)-carboxylique.

Dans un tube rodé de 5 mL sous atmosphère d'argon, on dissout 100 mg de chlorhydrate de l'acide 4-(1H-Imidazo[4,5-c]pyridin-2-yl)-9H-fluorène-9(R,S)-carboxylique, obtenu à l'exemple 29, dans 1 mL de dichlorométhane puis on ajoute successivement 46 µL de triéthylamine, 47 µL de N,N'-diisopropylcarbodiimide et 41 mg de 1-hydroxybenzotriazole et on agite 10 minutes. Puis on ajoute une solution de 43 mg de 5-aminométhyl-2-chloropyridine dans 1 mL et on agite 6 heures à température ambiante. Le milieu réactionnel est versé sur 20 mL d'une solution aqueuse saturée d'hydrogénocarbonate de sodium et extrait 2 fois avec 10 mL de dichlorométhane puis avec 10 mL d'acétate d'éthyle. Les phases organiques jointes sont lavées à l'eau, séchées sur sulfate de sodium et concentrées sous pression réduite. Après purification par flash-chromatographie sur gel de silice (40-63 µm), en éluant avec un mélange de dichlorométhane et de méthanol (95-5 puis 90-10 en volumes), on obtient 21 mg du (6-chloro-pyridin-3-ylmethyl)-amide de l'acide 4-(1H-imidazo[4,5-c]pyridin-2-yl)-9H-fluorene-9(R,S)-carboxylique, sous forme d'une meringue beige dont les caractéristiques sont les suivantes :
Spectre de masse (E/I) : m/z = 451 (M+)

### Exemple 3 : Synthèse du 4-(3H-Imidazo[4,5-c]pyridin-2-yl)-9H-fluorène-9(R,S)-ol.

Dans un ballon de 25 mL sous atmosphère d'argon, on dissout 100 mg de 4-(3H-Imidazo[4,5-c]pyridin-2-yl)-fluorèn-9(R,S)-one, obtenue à l'exemple 1, dans 3 mL de méthanol. Après refroidissement à 0°C, on ajoute en 2 fois 13 mg de borohydrure de sodium et on agite pendant 15 minutes jusqu'à dissolution totale. Le milieu réactionnel est versé sur de l'eau, puis extrait 2 fois avec 10 mL d'acétate d'éthyle. Les phases organiques jointes sont lavées à l'eau, séchées sur sulfate de sodium puis concentrées sous pression réduite. Après purification par cristallisation dans le minimum d'oxyde de diéthyle, on obtient 88 mg de 4-(3H-Imidazo[4,5-c]pyridin-2-yl)-fluorène-9(R,S)-ol, sous forme d'une poudre jaune clair dont les caractéristiques sont les suivantes :
Spectre de masse (E/I) : m/z = 299 (M+)
Spectre RMN 1H (300 MHz, □ en ppm, DMSO) : 5,58 (d, J = 8,0 Hz, 1H) ; 6,00 (d, J = 8,0 Hz, 1H) ; 7,19 (t large, J = 7,5 Hz, 1H) ; 7,31 (t large, J = 7,5 Hz, 2H) ; 7,50 (t, J = 7,5 Hz, 1H); de 7,58 à 7,70 (m, 3H) ; 7,80 (d large, J = 7,5 Hz, 1H) ; 8,39 (d, J = 5,5 Hz, 1H) ; 9,02 (s large, 1H) ; 13,4 (m, 1H).

### Exemple 4 : Synthèse du chlorhydrate du (pyridin-4-ylmethyl)-amide de l'acide 4-(3H-Imidazo[4,5-c]pyridin-2-yl)-9H-fluorène-9(R,S)-carboxylique

On opère comme à l'exemple 2, à partir de 100 mg de chlorhydrate de l'acide 4-(1H-Imidazo[4,5-c]pyridin-2-yl)-9H-fluorène-9(R,S)-carboxylique, préparé à l'exemple 29, mais avec 31µL de 4-picolylamine. Après purification par chromatographie sur gel de silice (40-63 µm) en éluant avec un mélange de dichlorométhane et de méthanol (90-10 en volumes), on obtient 29 mg du chlorhydrate du (pyridin-4-ylmethyl)-amide de l'acide 4-(3H-lmidazo[4,5-c]pyridin-2-yl)-9H-fluorène-9(R,S)-carboxylique, sous forme d'une meringue beige dont les caractéristiques sont les suivantes :
Spectre de masse (E/I) : m/z = 453 (M+)

### Exemple 5 : Synthèse de la 4-(3H-imidazo[4,5-c]pyridin-2-yl)-fluorène-9-one oxime (Z, E).

Dans un tricol de 500 mL, on dissout 20 g 4-(3H-Imidazo[4,5-c]pyridin-2-yl)-fluorène-9-one, obtenue à l'exemple 1, dans 328 mL d'éthanol, puis on ajoute successivement 14,02 g de chlorhydrate d'hydroxylamine et 27,59 g d'acétate de sodium sec. Après une nuit d'agitation à température ambiante, on dilue avec 328 mL d'eau. Le précipité formé est essoré, lavé à l'eau et séché en étuve à 50°. On obtient ainsi 18,6 g du mélange équimoléculaire des oximes Z et E de la 4-(3H-Imidazo[4,5-c]pyridin-2-yl)-fluorène-9-one, sous forme d'une poudre jaune-pâle, de pureté RMN supérieure à 95%, dont les caractéristiques sont les suivantes :
Spectre de masse (E/I) : m/z = 312 (M+)
Spectre RMN 1H (400 MHz, □ en ppm, DMSO) :7,24 (m, 1H) ; de 7,30 à 7,37 (m, 1H) ; 7,40 (m, 0,5H) ; 7,49 (m, 0,5H) ; 7,54 (t, J = 7,5 Hz, 0,5H) ; 7,49 (t, J = 7,5 Hz, 0,5H) ; de 7,67 à 7,80 (m, 3H) ; 7,95 (m, 0,5H) ; de 8,40 à 8,47 (m, 2H) ; 8,63 (m, 0,5H) ; 9,11 (s large, 1H) ; de 12,7 à 12,8 (m large, 1H).

### Exemple 6 : Synthèse de la 4-(3H-Imidazo[4,5-c]pyridin-2-yl)-9H-fluorène-9(R,S)-amine.

Dans un autoclave de 211 mL, on dissout 6,99 g de mélange équimoléculaire des oximes Z et E de la 4-(3H-Imidazo[4,5-c]pyridin-2-yl)-fluorène-9(R,S)-one, obtenue à l'exemple 5, dans un mélange de 30 mL d'éthanol et 30 mL de tétrahydrofurane, on ajoute 108 mg de Nickel activé selon Raney puis on soumet à une pression initiale d'hydrogène de 1 bar et on chauffe l'autoclave à 60° pendant 20 heures. Après refroidissement, le volume d'hydrogène absorbé est de 175 mL. Après des purges à l'argon, l'autoclave est ouvert, on ajoute 10 g de Célite et on filtre (catalyseur + célite). Le filtrat est concentré sous pression réduite. On obtient ainsi 5,15 g de 4-(3H-imidazo[4,5-c]pyridin-2-yl)-fluorène-9(R,S)-amine, sous forme d'une poudre beige, dont les caractéristiques sont les suivantes :
Spectre de masse (E/I) : m/z = 298 (M+)
Spectre RMN 1H (400 MHz, □ en ppm, CD3OD) :4,94 (s, 1H) ; 7,05 (d large, J = 7,5 Hz, 1H) ; 7,14 (t large, J = 7,5 Hz, 1H) ; 7,31 (t large, J = 7,5 Hz, 1H) ; 7,51 (t, J = 7,5 Hz, 1H) ; 7,61 (d, J = 7,5 Hz, 1H) ; 7,72 (m, 2H) ; 7,92 (d, J = 7,5 Hz, 1H) ; 8,40 (d, J = 6,0 Hz, 1H) ; 8,99 (s, 1H).

### Exemple 7 : Synthèse de la 2-(9H-fluorèn-4-yl)-3H-imidazo[4,5-c]pyridine

Etape 1 : Dans un tricol de 250 mL, on agite, pendant 1 heure à température ambiante, 1,99 g d"acide fluorène-4-carboxylique dans 40 mL de chlorure de thionyle. Après concentration sous pression réduite, le chlorure d'acide obtenu est dissout dans 40 mL de dichlorométhane, puis on ajoute 1,55g de 3,4-diaminopyridine et 2,65 mL de triéthylamine. On agite une nuit à température ambiante. Le précipité formé est essoré et lavé au dichlorméthane. On obtient ainsi 3,08 g d'un produit contenant majoritairement un mélange équimoléculaire d'amides, utilisé tel quel à l'étape suivante.

Etape 2: Dans un tricol de 25 mL, on dissout 1,3 g du mélange d'amides obtenu à l'étape précédente dans 50 mL de dioxane et 5 mL d'oxychlorure de phosphore. On agite alors pendant 2 heures à 120°C, puis on rajoute 20 mL d'oxychlorure de phosphore et on chauffe de nouveau pendant 20 heures à 120°C. Après concentration sous pression réduite, le résidu est purifié par flash-chromatographie sur gel de silice (70-230 Mesh) en éluant avec un mélange de dichlorométhane, de méthanol et d'ammoniaque (95-5-0,2 en volumes). On obtient ainsi 1,68 g de 2-(9H-fluorèn-4-yl)-3H-imidazo[4,5-c]pyridine, sous forme d'une poudre écrue dont les caractéristiques sont les suivantes :
Spectre de masse (E/I) : m/z = 283 (M+)
Spectre RMN 1H (400 MHz, □ en ppm, DMSO) : 4,06 (s, 2H) ; 7,18 (t large, J = 7,5 Hz, 1H) ; 7,31 (t large, J = 7,5 Hz, 1H) ; 7,37 (d large, J = 7,5 Hz, 1H) ; 7,50 (t, J = 7,5 Hz, 1H) ; de 7,60 à 7,68 (m, 3H) ; 7,81 (d large, J = 7,5 Hz, 1H) ; 8,39 (d, J = 6,0 Hz, 1H) ; 9,03 (s large, 1H).

### Exemple 8 : Synthèse de la N-benzyl-N'-[4-(1H-imidazo[4,5-c]pyridin-2-yl)-fluorèn-9-ylidène]-hydrazine, mélange 55/45 d'isomères E et Z.

Dans un ballon de 10 mL, on dissout 100 mg 4-(3H-imidazo[4,5-c]pyridin-2-yl)-fluorène-9-one, obtenue à l'exemple 1, dans 3 mL de méthanol, puis on ajoute successivement 197 mg de dichlorhydrate de benzylhydrazine et 138 mg d'acétate de sodium sec et on chauffe à 50-55°C pendant 5 heures. Le milieu réactionnel refroidi est coulé sur 10 mL d'eau, extrait à l'acétate d'éthyle puis 2 fois au dichlorométhane. Les phases organiques jointes sont lavées avec une solution aqueuse saturée de chlorure de sodium, séchées sur sulfate de sodium puis concentrées sous pression réduite. Après purification par chromatographie sur gel de silice (40-63 µm) en éluant avec un mélange de dichlorométhane et de méthanol (93-7 en volumes), on obtient 37 mg de N-benzyl-N'-[4-(1H-imidazo[4,5-c]pyridin-2-yl)-fluorèn-9-ylidène]-hydrazine, sous forme d'une meringue jaune dont les caractéristiques sont les suivantes :
Spectre de masse (E/I) : m/z = 401 (M+)

### Exemple 9 : Synthèse de la N-[4-(1H-imidazo[4,5-c]pyridin-2-yl)-fluorèn-9-ylidène]-hydrazine, mélange 60/40 d'isomères E et Z

Dans un ballon de 10 mL, on dissout 100 mg de 4-(3H-imidazo[4,5-c]pyridin-2-yl)-fluorène-9-one, obtenue à l'exemple 1, dans 3 mL de méthanol, puis on ajoute successivement 96 µL d'hydrate d'hydrazine et 57,6 µL d'acide acétique puis on chauffe à 50-55°C pendant 3 heures. Le milieu réactionnel refroidi est coulé sur 10 mL d'eau. Le précipité formé est filtré, redissout dans un mélange de méthanol et de dichlorométhane. Après séchage sur sulfate de sodium puis concentration sous pression réduite, le résidu est purifié par cristallisation dans le minimum d'oxyde diéthyle. On obtient ainsi 70 mg de N-[4-(1H-imidazo[4,5-c]pyridin-2-yl)-fluorèn-9-ylidène]-hydrazine, en mélange des isomères Z et E, sous forme d'une poudre beige dont les caractéristiques sont les suivantes :
Spectre de masse (E/I) : m/z = 311 (M+)
Spectre RMN 1H (400 MHz, □ en ppm, DMSO-d6) : de 7,00 à 7,30 (m, 2H) ; 7,39 (t, J = 7,5 Hz, 0,5H) ; de 7,45 à 7,76 (m, 3H) ; 7,88 (m, 0,5 H) ; de 8,21 à 8,47 (m, 3H) ; 9,06 (s large, 1H) ; de 13,1 à 13,7 (m très étalé, 1H).

### Exemple 10 : Synthèse de la 2-[9(R,S)-fluoro-9H-fluorèn-4-yl]-1H-imidazo[4,5-c]pyridine

Dans un ballon de 10 mL sous atmosphère d'argon, on dissout 85 mg de 4-(3H-Imidazo[4,5-c]pyridin-2-yl)-fluorène-9-ol, obtenu à l'exemple 3, dans 3 mL de dichlorométhane. On refroidit la solution obtenue vers -60°C puis on ajoute, goutte à goutte à la seringue, 41 µL de diéthylamino-trifluorosulfure (DAST). On laisse revenir à température ambiante et on agite 45 minutes, puis on coule le milieu réactionnel sur une solution aqueuse à 10% d'hydrogénocarbonate de sodium et on extrait 2 fois au dichlorométhane. Les phases organiques jointes sont séchées sur sulfate de sodium et concentrées sous pression réduite. Après purification par chromatographie sur gel de silice (40-63 µm) en éluant avec un mélange de dichlorométhane et de méthanol (95-5 en volumes), on obtient 44 mg de 2-[9(R,S)-fluoro-9Hfluorèn-4-yl]-1H-imidazo[4,5-c]pyridine, sous forme d'une poudre blanche dont les caractéristiques sont les suivantes :
Spectre de masse (E/I) : m/z = 301 (M+)
Spectre RMN 1H (400 MHz, d en ppm, DMSO-d6) : 6,60 (d, J = 52,0 Hz, 1H) ; 7,31 (t large, J = 7,5 Hz, 1H) ; 7,38 (t large, J = 7,5 Hz, 1H) ; 7,47 (d large, J = 7,5 Hz, 1H) ; 7,57 (t, J = 7,5 Hz, 1H) ; 7,67 (d large, J = 5,5 Hz, 1H) ; 7,70 (d large, J = 7,5 Hz, 1H) ; 7,77 (d large, J = 7,5 Hz, 1H) ; 7,78 (d large, J = 7,5 Hz, 1H) ; 8,38 (d, J = 5,5 Hz, 1H) ; 9,04 (s large, 1H) ; de 13,2 à 13,6 (m très étalé, 1H).

### Exemple 11 : Synthèse de la O-(pyridin-3-yl)méthyl 4-(3H-Imidazo[4,5-c]pyridin-2-yl)-fluorène-9-one oxime (Z, E).

Dans un ballon de 10 mL, on dissout 100 mg 4-(3H-Imidazo[4,5-c]pyridin-2-yl)-fluorène-9-one, obtenue à l'exemple 1, dans 3 mL d'éthanol, puis on ajoute successivement 66,2 mg de dichlorhydrate de [(pyridin-3-yl)méthyl]hydroxylamine, qui peut être obtenue selon DE 2119012, et 276 mg d'acétate de sodium sec. Après une nuit d'agitation à température ambiante, on dilue avec 30 mL d'eau. Le précipité formé est essoré, lavé à l'eau et séché en étuve à 50°. On obtient ainsi 43 mg du mélange équimoléculaire des isomères Z et E de la de la O-(pyridin-3-yl)méthyl 4-(3H-Imidazo[4,5-c]pyridin-2-yl)-fluorène-9-one oxime (Z, E), sous forme d'une poudre jaune, dont les caractéristiques sont les suivantes :
Spectre de masse (E/I) : m/z = 476 (M+).

### Exemple 12 : Synthèse du N-[4-(3H-Imidazo[4,5-c]pyridin-2-yl)-9H-fluorèn-9(R,S)-yl]-formamide

La 4-(3H-imidazo[4,5-c]pyridin-2-yl)-fluorène-9(R,S)-amine, obtenue à l'exemple 6, peut être cristallisée sous forme de triformiate par traitement avec excès d'acide formique dans le méthanol. 65 mg de triformiate de 4-(3H-imidazo[4,5-c]pyridin-2-yl)-fluorène-9(R,S)-amine sont dissous dans 1,5 mL d'un mélange de diméthylformamide et de dichlorméthane (50-50 en volumes), puis on ajoute 43 mg d'EDCI, 30 mg d'HOBT et 200µL de diéthylisopropylamine et on agite à température ambiante pendant 22 heures. Le milieu réactionnel est versé dans 5 mL d'une solution aqueuse saturée de dihydrogénophosphate de potassium, puis extrait 3 fois par 10 mL d'acétate d'éthyle. Après purification par HPLC/MS sur une colonne de silice X-Terra, en éluant par un gradient eau 100% (tamponnée à pH = 9) à acétonitrile 100%, puis lyophilisation des fractions contenant le produit de masse attendue, on obtient 6 mg de N-[4-(3H-Imidazo[4,5-c]pyridin-2-yl)-9H-fluorèn-9(R,S)-yl]-formamide, sous forme d'un solide blanc dont les caractéristiques sont les suivantes :
Spectre de masse (E/I) : m/z = 326 (M+).

### Exemple 13 : Synthèse du [4-(1H-Imidazo[4,5-c]pyridin-2-yl)-fluorèn-(9)-ylidene]-methanol, mélange équimoléculaire des isomères Z et E.

Dans un ballon de 50 mL sous atmosphère d'argon, on dissout 200 mg de 2-(9H-fluorèn-4-yl)-3H-imidazo[4,5-c]pyridine, obtenue à l'exemple 7, dans 10 ml de tetrahydrofurane et on refroidit la solution vers -10°C. On ajoute alors 2,83 ml d'une solution molaire de tert.butylate de potassium dans le tetrahydrofurane puis 628 mg de formiate d'éthyle. On agite pendant 12 minutes vers -10°C, puis on ajoute une solution aqueuse normale d'acide chlorhydrique jusqu'à pH neutre et enfin 20 mL d'acétate d'éthyle. La phase organique est décantée, lavée à l'eau, séchée sur sulfate de magnésium et concentrée sous pression réduite. On obtient ainsi 95 mg de [4-(1H-Imidazo[4,5-c]pyridin-2-yl)-fluorèn-(9)-ylidene]-methanol, en mélange équimoléculaire des isomères Z et E, sous forme d'une meringue jaune, dont les caractéristiques sont les suivantes :
Spectre de masse (E/I) : m/z = 476 (M+).

### Exemple 14 : Synthèse du [4-(3H-imidazo[4,5-c]pyridin-2-yl)-9H-fluorèn-9(R,S)-yl]-amide de l'acide quinoléine-5-carboxylique.

Dans un tricol de 50 mL, on dissout 605 mg de 4-(3H-imidazo[4,5-c]pyridin-2-yl)-fluorène-9(R,S)-amine, obtenue à l'exemple 6, dans 30,17 mL de diméthylformamide, puis on ajoute successivement 530 mg de chlorhydrate de 1-(3-diméthylaminopropyl)-3-éthylcarbodiimide (EDCI), 373 mg de 1-hydroxybenzotriazole (HOBT) et 319 mg d'acide quinoléine-5-carboxylique puis on agite 20 heures à température ambiante. On ajoute alors 100 mL d'eau, et on essore le précipité formé qui est ensuite lavé à l'eau puis avec une solution saturée d'hydrogénocarbonate de sodium. Le solide brut obtenu est purifié par flash-chromatographie sur gel de silice (70-230 Mesh) en éluant avec un mélange de dichlorométhane et de méthanol (95-5 en volumes). On obtient ainsi 650 mg (78%) de [4-(3H-imidazo[4,5-c]pyridin-2-yl)-9H-fluorèn-9(R,S)-yl]-amide de l'acide quinoléine-5-carboxylique, sous forme d'une poudre jaune pâle dont les caractéristiques sont les suivantes :
Point de fusion (Kofler) = 254-8°C (décomposition).
Spectre de masse (E/I) : m/z = 453 (M+)
Spectre RMN 1H (400 MHz, d en ppm, DMSO-d6) : 6,44 (d, J = 8,5 Hz, 1H) ; 7,27 (t large, J = 7,5 Hz, 1H) ; 7,39 (t large, J = 7,5 Hz, 1H) ; 7,48 (m large, 1H) ; 7,57 (t, J = 8,0 Hz, 1H) ; de 7,60 à 7,85 (m, 5H) ; 7,87 (d large, J = 7,5 Hz, 1H) ; 7,92 (d large, J = 8,0 Hz, 1H) ; 8,14 (d large, J = 8,5 Hz, 1H) ; 8,40 (d large, J = 5,5 Hz, 1H) ; 8,87 (d large, J = 8,5 Hz, 1H) ; de 8,93 à 9,10 (m étalé, 1H) ; 8,99 (dd, J = 2,0 et 4,0 Hz, 1H) ; 9,39 (d, J = 8,5 Hz, 1H) ; de 13,3 à 13,5 (m étalé, 1H).

### Exemple 14A : Synthèse de l'énantiomère dextrogyre du [4-(3H-imidazo[4,5-c]pyridin-2-yl)-9H-fluorèn-9(R,S)-yl]-amide de l'acide quinoléine-5-carboxylique.

103 mg de [4-(3H-imidazo[4,5-c]pyridin-2-yl)-9H-fluorèn-9(R,S)-yl]-amide de l'acide quinoléine-5-carboxylique, obtenue à l'exemple 14, sont injectés sur une colonne chirale préparative contenant 600 g de silice Chiracel OJ. On élue avec un mélange de n.heptane, de méthanol, d'éthanol et de triéthylamine (50-25-25-0,1 en volumes). En récupérant et concentrant sous pression réduite la première fraction éluée, on obtient 41,8 mg d'énantiomère dextrogyre dont les caractéristiques sont les suivantes :
α^{D}₂₀ = +131,1 +/- 1,8°(c = 0,5 ; MeOH).

### Exemple 14B : Synthèse de l'énantiomère lévogyre du [4-(3H-imidazo[4,5-c]pyridin-2-yl)-9H-fluorèn-9(R,S)-yl]-amide de l'acide quinoléine-5-carboxylique.

En opérant comme à l'exemple 14A, mais en récupérant et concentrant sous pression réduite la seconde fraction éluée, on obtient 33,1 mg d'énantiomère lévogyre dont les caractéristiques sont les suivantes :
α^{D}₂₀ = -123,5 +/- 1,8°(c = 0,5 ; MeOH).

### Exemple 15 : Synthèse de la 4-(3H-Imidazo[4,5-b]pyridin-2-yl)-fluorène-9-one O-(4-bromo-3-hydroxy-benzyl)-oxime.

On opère comme à l'exemple 11, à partir de 100 mg de 4-(3H-Imidazo[4,5-c]pyridin-2-yl)-fluorène-9-one, obtenue à l'exemple 1, et de 42 mg (4-bromo-3-hydroxy-benzyl)hydroxylamine, qui peut être obtenue selon J. Pharm. Exp. Ther. 1971, 179, 619-33, dans 3 mL de méthanol. On obtient ainsi 22 mg du mélange 60/40 des isomères Z et E de la 4-(3H-Imidazo[4,5-b]pyridin-2-yl)-fluorène-9-one O-(4-bromo-3-hydroxy-benzyl)-oxime), sous forme d'une poudre jaune-orangée, dont les caractéristiques sont les suivantes :
Spectre de masse (E/I) : m/z = 496 (M+).

### Exemple 16 : Synthèse de la N-[4-(3H-imidazo[4,5-c]pyridin-2-yl)-9H-fluorèn-9(R,S)-yl]-phénéthyl-amine.

Dans un réacteur microonde, à une solution de 84 mg de 4-(3H-Imidazo[4,5-c]pyridin-2-yl)-fluorène-9-one, obtenue à l'exemple 1, dans 0,5 mL d'éthanol on ajoute 81 µL de 2-phényl-éthylamine,18 µL de diéthylisopropylamine et 182 µL tetra-isopropylate de titane. Puis on porte successivement à 60°C pendant 5 minutes, à 70°C pendant 20 minutes, à 100°C pendant 15 minutes et enfin à 130°C pendant 30 minutes. Après refroidissement à température ambiante, on ajoute 25 mg de cyanoborohydrure de sodium et on agite 16 heures à température ambiante. Le milieu réactionnel est versé dans 5 mL d'une solution aqueuse saturée de dihydrogénophosphate de potassium, puis extrait 3 fois par 10 mL de dichlorométhane.. Après purification sur gel de silice, en éluant avec des mélanges de dichlorométhane et de méthanol (98-2, puis 96-4 en volumes), on obtient 4,8 mg de N-[4-(3H-imidazo[4,5-c]pyridin-2-yl)-9H-fluorèn-9(R,S)-yl]-phénéthyl-amine, sous forme d'un solide blanc dont les caractéristiques sont les suivantes :
Spectre de masse (E/I) : m/z = 402 (M+).

### Exemple 17 : Synthèse du 2-[4-(3H-imidazo[4,5-c]pyridin-2-yl)-fluorèn-(9E,Z)-ylidène]-N-pyridin-3-yl-acétamide.

Etape 1 : Dans un tricol de 100 mL, on dissout 594 mg de 4-(3H-Imidazo[4,5-c]pyridin-2-yl)-fluorène-9-one, obtenue à l'exemple 1, dans 15 mL d'acétonitrile et on ajoute successivement 654 mg d'anhydride de tert.butyloxycarbonyle et 12 mg de 4-diméthylamino-pyridine. La solution obtenue est agitée 20 heures à température ambiante, puis le milieu réactionnel est coulé dans 100 mL d'eau glacée et extrait 3 fois à l'acétate d'éthyle. Les phases organiques jointes sont lavées à l'eau, séchées sur sulfate de magnésium et concentrées sous pression réduite. On obtient ainsi 695 mg d'ester tert.butylique de l'acide 2-(9-oxo-9H-fluorèn-4-yl)-imidazo[4,5-c]pyridine-3-carboxylique, sous forme de cristaux bruns utilisés tels quels à l'étape suivante.

Etape 2 : Dans un tricol de 25 mL sous atmosphère d'azote, on dissout 695 mg d'ester tert.butylique de l'acide 2-(9-oxo-9H-fluorèn-4-yl)-imidazo[4,5-c]pyridine-3-carboxylique, obtenu à l'étape précédente, dans 5 mL de tetrahydrofurane anhydre, puis on ajoute 126 mg d'hydrure de sodium et on agite 10 minutes. On coule ensuite, goutte à goutte en 30 minutes, une solution de 588 mg de phosphonoacétate de triéthyle dans 5 mL de tetrahydrofurane anhydre, puis on agite 16 heures à température ambiante. Le milieu réactionnel est concentré sous pression réduite, puis repris par un mélange d'eau et d'acétate d'éthyle. La phase organique est décantée, lavée à l'eau, séchée sur sulfate de magnésium et concentrée sous pression réduite. Après purification sur gel de silice, en éluant avec des mélanges de dichlorométhane et d'acétate d'éthyle (90-10, puis 80-20, puis 70-30 en volumes), on obtient 432 mg d'ester tert.butylique de l'acide 2-{9-[1-éthoxycarbonyl-méthylidène]-9H-fluorèn-4-yl}-3H-pyrrolo[3,2-c]pyridine-3-carboxylique, en mélange équimoléculaire des isomères Z et E, sous forme d'une huile visqueuse jaune, utilisée telle quelle à l'étape suivante.

Etape 3 : Dans un tricol de 25 mL, on agite pendant 12 heures à température ambiante une solution de 400 mg d'ester tert.butylique de l'acide 2-{9-[1-éthoxycarbonyl-méthylidène]-9H-fluorèn-4-yl}-3H-pyrrolo[3,2-c]pyridine-3-carboxylique, obtenu à l'étape précédente, dans 10 mL de tétrahydrofurane en présence de 2 mL d'une solution aqueuse normale d'hydroxyde de sodium. Après concentration du solvant sous pression réduite, le résidu est par un mélange d'eau et d'acétate d'éthyle. La phase aqueuse est acidifiée à pH = 2, puis extraite 4 fois à l'acétate d'éthyle. Les phases organiques jointes sont lavées à l'eau et concentrées sous pression réduite. Le résidu blanc obtenu est cristallisé dans l'oxyde de diisopropyle, on obtient ainsi 60 mg d'acide [4-(3H-imidazo[4,5-c]pyridin-2-yl)-fluorèn-9-ylidène]-acetique, en mélange 40-60 des isomères Z et E, sous forme d'une poudre blanche, dont les caractéristiques sont les suivantes :
spectre de masse (E/I) : m/z = 339 (M+).

Etape 4 : On opère comme à l'exemple 14 à partir de 45 mg d'acide [4-(3H-imidazo[4,5-c]pyridin-2-yl)-fluorèn-9-ylidène]-acetique, obtenu à l'étape précédente, et de 18,7 mg de 3-aminopyridine en présence de 38,1 mg d'EDCI et de 30,5 mg d'HOBT. Après purification par précipitation dans l'eau, on obtient 26 mg de 2-[4-(3H-imidazo[4,5-c]pyridin-2-yl)-fluorèn-9-ylidène]-N-pyridin-3-yl-acétamide, en mélange 40-60 des isomères Z et E, sous forme d'une poudre beige dont les caractéristiques sont les suivantes :
Spectre de masse (E/I) : m/z = 415 (M+).
Spectre de RMN 1H (400 MHz, □ en ppm, DMSO): 7,15 (d, J = 7,5 Hz, 0,4H) ; 7,27 (t, J = 7,5 Hz, 1H) ; de 7,30 à 7,49 (m, 3,6H) ; 7,53 (t, J = 7,5 Hz, 0,6H) ; 7,60 (t, J = 7,5 Hz, 0,4H) ; de 7,62 à 7,75 (m, 2H) ; 7,92 (d large, J = 7,5 Hz, 0,6H) ; 8,10 (d, J = 7,5 Hz, 0,4H) ; 8,23 (m large, 1H) ; 8,35 (s large, 1H) ; 8,41 (d, J = 5,5 Hz, 1H) ; 8,75 (d, J = 8,5 Hz, 0,4H) ; 8,41 (s large, 1H) ; 8,95 (d, J = 8,5 Hz, 0,6Hz) ; 9,05 (s large, 1H) ; 10,89 (s, 0,4H) ; 10,91 (s, 0,6H) ; de 13,2 à 13,6 (m très étalé, 1H).

### Exemple 18 : Synthèse de la N-[4-(3H-imidazo[4,5-c]pyridin-2-yl)-9H-fluorèn-9(R,S)-yl]-(1H-indol-5-ylméthyl)-amine

Dans un tricol de 5 mL, on dissout 74 mg de 4-(3H-Imidazo[4,5-c]pyridin-2-yl)-fluorène-9(R,S)-amine, isolée sous forme de dichlorhydrate, dans 0,5 mL d'éthanol, on ajoute 45 mg de 5-formyl-indole et 70 µL de diisopropyléthylamine et on agite 2 heures 30 minutes à température ambiante. On ajoute ensuite 28 mg de borohydrure de sodium puis on agite pendant 18 heures à température ambiante. Le milieu réactionnel est versé dans 5 mL d'une solution aqueuse saturée de dihydrogénophosphate de potassium, puis extrait 3 fois par 10 mL de dichlorométhane. Après purification sur gel de silice, en éluant avec des mélanges de dichlorométhane et de méthanol (98-2, puis 96-4 en volumes), on obtient 34 mg de N-[4-(3H-imidazo[4,5-c]pyridin-2-yl)-9H-fluorèn-9(R,S)-yl]-(1H-indol-5-ylméthyl)-amine, sous forme d'un solide blanc dont les caractéristiques sont les suivantes :
Spectre de masse (E/I) : m/z = 427 (M+).

### Exemple 19 : Synthèse de la 4-(3H-Imidazo[4,5-c]pyridin-2-yl)-fluorène-9-one oxime (Z).

103 mg de 4-(3H-imidazo[4,5-c]pyridin-2-yl)-fluorène-9-one oxime (Z,E), obtenue à l'exemple 5, sont injectés sur une colonne chirale préparative contenant 700 g de silice Chiralpak AS (20 µm). On élue avec un mélange de n.heptane, de méthanol, d'isopropanol et de triéthylamine (90-2,5-2,5-0,1 en volumes). En récupérant et concentrant sous pression réduite la seconde fraction éluée, on obtient 23,8 mg d'oxime Z.

### Exemple 20 : Synthèse de l'ester éthylique de l'acide [4-(3H-imidazo[4,5-c]pyridin-2-yl)-9H-fluorèn-9(R,S)-yl]-carbamoïque.

Dans un tricol de 25 mL sous atmosphère d'argon, à une suspension refroidie à 5°C de 225 mg de 4-(3H-imidazo[4,5-c]pyridin-2-yl)-fluorène-9(R,S)-amine, obtenue à l'exemple 6, dans 3 mL de tetrahydrofurane et 113 µL de triéthylamine, on ajoute 72 µL de chloroformiate d'éthyle. Le milieu réactionnel est agité pendant 1 heure en laissant revenir à température ambiante, puis concentré sous pression réduite. Après purification par flash-chromatographie sur gel de silice, en éluant avec un mélange de dichlorométhane et de méthanol (96-4 en volumes), on obtient 138 mg d'ester éthylique de l'acide [4-(3H-imidazo[4,5-c]pyridin-2-yl)-9H-fluorèn-9(R,S)-yl]-carbamoïque, sous forme d'un solide jaune-pâle dont les caractéristiques sont les suivantes :
Spectre de masse (E/I) : m/z = 370 (M+).

### Exemple 21 : Synthèse du N-[4-(3H-imidazo[4,5-c]pyridin-2-yl)-9H-fluorèn-9(R,S)-yl]-2-(pyridin-3-yl)-acétamide.

On opère comme à l'exemple 14 à partir de 85,2 mg 4-(3H-Imidazo[4,5-c]pyridin-2-yl)-fluorène-9(R,S)-amine, obtenue à l'exemple 6, et de 21 mg d'acide (pyridin-3-yl)acétique en présence de 44 mg d'EDCI et de 31 mg d'HOBT dans 1 mL de dichlorométhane et 1 mL de DMF pendant 20 heures. Après dilution avec 25 mL d'eau, le précipité formé est essoré, lavé à l'eau et séché sous pression réduite à 50°C. On obtient ainsi 20 mg de N-[4-(3H-imidazo[4,5-c]pyridin-2-yl)-9H-fluorèn-9(R,S)-yl]-2-(pyridin-3-yl)-acétamide, sous forme d'une poudre beige, dont les caractéristiques sont les suivantes :
Spectre de masse (E/I) : m/z = 417 (M+).

### Exemple 22 : Synthèse de la N-[4-(3H-imidazo[4,5-c]pyridin-2-yl)-9H-fluorèn-9(R,S)-yl]-(1H-indol-6-ylméthyl)-amine

On opère comme à l'exemple 18, à partir de 94 mg de dichlorhydrate de 4-(3H-Imidazo[4,5-c]pyridin-2-yl)-fluorène-9(R,S)-amine, 55 mg de 6-formyl-indole et 90 µL de diisopropyléthylamine dans 0,7 mL d'éthanol pendant 2 heures à température ambiante, puis on ajoute 28 mg de borohydrure de sodium et on agite pendant 1 heure à température ambiante. Après purification sur gel de silice, en éluant avec des mélanges de dichlorométhane et de méthanol (95-5 en volumes), on obtient 58 mg de N-[4-(3H-imidazo[4,5-c]pyridin-2-yl)-9H-fluorèn-9(R,S)-yl]-(1H-indol-6-ylméthyl)-amine, sous forme d'un solide rose-pâle dont les caractéristiques sont les suivantes :
Spectre de masse (E/I) : m/z = 427 (M+).

### Exemple 23 : Synthèse de la 4-(3H-Imidazo[4,5-c]pyridin-2-yl)-fluorène-9-one oxime (E).

103 mg de 4-(3H-imidazo[4,5-c]pyridin-2-yl)-fluorène-9-one oxime (Z,E), obtenue à l'exemple 5, sont injectés sur une colonne chirale préparative contenant 700 g de silice Chiralpak AS (20 µm). On élue avec un mélange de n.heptane, de méthanol, d'isopropanol et de triéthylamine (90-2,5-2,5-0,1 en volumes). En récupérant et concentrant sous pression réduite la première fraction éluée, on obtient 37 mg d'oxime E, dont les carctéristiques sont les suivantes :
Spectre RMN 1H (300 MHz, □ en ppm, DMSO : de 7,18 à 7,28 (m, 2H) ; 7,33 (m, 1H) ; 7,58 (t, J = 7,5 Hz, 1H) ; 7,67 (m étalé, 1H) ; 7,72 (d large, J = 7,5 Hz, 1H) ; 7,76 (d large, J = 7,5 Hz, 1H) ; 8,40 (d, J = 5,5 Hz, 1H) ; 8,62 (d large, J = 7,5 Hz, 1H) ; 9,04 (s large, 1H).

### Exemple 24 : Synthèse du trifluoroacétate de 2-(2-amino-thiazol-4-yl)-N-[4-(3H-imidazo[4,5-c]pyridin-2-yl)-9H-fluorèn-9(R,S)-yl]-acétamide.

Dans un tricol de 10 mL sous atmosphère d'argon, à une suspension de 80 mg de 4-(3H-Imidazo[4,5-c]pyridin-2-yl)-fluorène-9(R,S)-amine, obtenue à l'exemple 6, dans 2 mL de toluène on ajoute 210 µL d'une solution 2M de triméthylaluminium dans le toluène et on chauffe à 80°C pendant 20 minutes. On ajoute de nouveau 90 µL de solution 2M de triméthylaluminium dans le toluène et on chauffe de nouveau 10 minutes à 80°C. On ajoute 45 mg d'ester éthylique de l'acide (2-amino-thiazol-4-yl)acétique et on maintient le chauffage à 80°C pendant 2 heures. Le milieu réactionnel est versé dans 5 mL d'une solution aqueuse saturée de dihydrogénophosphate de potassium, puis extrait 3 fois par 10 mL de dichlorométhane. Après purification par HPLC/MS sur une colonne de silice Symmetry C18 (5 µm), en éluant par un gradient eau 100% (contenant 0,07% de TFA) à acétonitrile 100% (contenant 0,07% de TFA), on obtient 7 mg de trifluoroacétate de 2-(2-amino-thiazol-4-yl)-N-[4-(3H-imidazo[4,5-c]pyridin-2-yl)-9H-fluorèn-9(R,S)-yl]-acétamide, sous forme d'un solide blanc dont les caractéristiques sont les suivantes :
Spectre de masse (E/I) : m/z = 438 (M+).

### Exemple 25 : Synthèse du 2-(6-chloro-pyridin-3-yl)-N-[4-(3H-imidazo[4,5-c]pyridin-2-yl)-9H-fluorèn-9(R,S)-yl]-acétamide.

On opère comme à l'exemple 14 à partir de 101,3 mg 4-(3H-Imidazo[4,5-c]pyridin-2-yl)-fluorène-9(R,S)-amine, obtenue à l'exemple 6, et de 39 mg d'acide (2-chloro-pyridin-5-yl)acétique en présence de 65,4 mg d'EDCI et de 46,1 mg d'HOBT dans 1,8 mL de dichlorométhane et 1,8 mL de DMF pendant 20 heures. Après purification par cristallisation dans l'eau, on obtient 16 mg de 2-(6-chloro-pyridin-3-yl)-N-[4-(3H-imidazo[4,5-c]pyridin-2-yl)-9H-fluorèn-9(R,S)-yl]-acétamide, sous forme d'une poudre jaune, dont les caractéristiques sont les suivantes :
Spectre de masse (E/I) : m/z = 451 (M+).

### Exemple 26 : Synthèse du trifluoroacétate de N-[4-(3H-imidazo[4,5-c]pyridin-2-yl)-9H-fluorèn-9(R,S)-yl]-quinolin-5-ylméthyl-amine

On opère comme à l'exemple 18, à partir de 100 mg de 4-(3H-Imidazo[4,5-c]pyridin-2-yl)-fluorène-9(R,S)-amine, obtenue à l'exemple 6, et 59 mg de quinoléine-5-carboxaldéhyde dans 1 mL d'éthanol pendant 3 heures à température ambiante, puis on ajoute 29 mg de borohydrure de sodium et on agite pendant 2 heures à température ambiante. Après purification par HPLC/MS sur une colonne de silice Symmetry C18 (5 µm), en éluant par un gradient eau 100% (contenant 0,07% de TFA) à acétonitrile 100% (contenant 0,07% de TFA), on obtient 49,5 mg de trifluoroacétate de N-[4-(3H-imidazo[4,5-c]pyridin-2-yl)-9H-fluorèn-9(R,S)-yl]-quinolin-5-ylméthyl-amine, sous forme d'un solide jaune-pâle dont les caractéristiques sont les suivantes :
Spectre de masse (E/I) : m/z = 439 (M+).

### Exemple 27 : Synthèse du N-[4-(3H-imidazo[4,5-c]pyridin-2-yl)-9H-fluorèn-9(R,S)-yl]-2-(4-méthoxy-phényl)-acétamide.

On opère comme à l'exemple 14 à partir de 99,2 mg 4-(3H-Imidazo[4,5-c]pyridin-2-yl)-fluorène-9(R,S)-amine, obtenue à l'exemple 6, et de 37 mg d'acide (4-méthoxy-phényl)acétique en présence de 64 mg d'EDCI et de 45,1 mg d'HOBT dans 1,5 mL de dichlorométhane et 1,5 mL de DMF pendant 20 heures. Après purification par cristallisation dans l'eau, on obtient 35 mg de N-[4-(3H-imidazo[4,5-c]pyridin-2-yl)-9H-fluorèn-9(R,S)-yl]-2-(4-méthoxy-phényl)-acétamide, sous forme d'une poudre beige, dont les caractéristiques sont les suivantes :
Spectre de masse (E/I) : m/z = 446 (M+).

### Exemple 28 : Synthèse du trifluoroacétate de la [4-(3H-imidazo[4,5-c]pyridin-2-yl)-9H-fluorèn-9(R,S)-yl]-urée.

Dans un tricol de 10 mL sous atmosphère d'argon, à une suspension de 80 mg de 4-(3H-imidazo[4,5-c]pyridin-2-yl)-fluorène-9(R,S)-amine, obtenue à l'exemple 6, dans 1 mL de toluène anhydre, on ajoute 210 µL d'une solution 2M de triméthylaluminium. La suspension obtenue est agitée 15 minutes à température ambiante puis 15 mn à 80°C. On ajoute alors 30 mg de carbamate de 2-chloro-éthyle, puis on agite 1 heure à 40°C. Après addition de 0,5 mL d'eau, le précipité formé est essoré. Après purification par HPLC/MS sur une colonne de silice Symmetry C18 (5 µm), en éluant par un gradient eau 100% (contenant 0,07% de TFA) à acétonitrile 100% (contenant 0,07% de TFA), puis lyophilisation des fractions contenant le produit de masse attendue, on obtient 18 mg de trifluoroacétate de la [4-(3H-imidazo[4,5-c]pyridin-2-yl)-9H-fluorèn-9(R,S)-yl]-urée, sous forme d'un solide blanc dont les caractéristiques sont les suivantes :
Spectre de masse (E/I) : m/z = 341 (M+).

### Exemple 29 : Synthèse du chlorhydrate de l'acide 4-(1H-Imidazo[4,5-c]pyridin-2-yl)-9H-fluorène-9(R,S)-carboxylique.

Etape 1 : Dans un tricol de 500 mL sous atmosphère d'argon, on dissout 1,4 g d'ester de tert.butyle de l'acide fluorèn-4-carboxylique, qui peut être obtenu selon Helv. Chim. Acta 1984, 67, 2009-16, dans 50 mL de tetrahydrofurane. Après refroidissement à -20°C, on ajoute 1,18 g de tert.butylate de potassium, puis après 10 minutes d'agitation à -20°C, on ajoute à la solution violette obtenue 0,995 g de chloroformiate de méthyle et on agite 1 heure à -20°C. Le milieu réactionnel est alors dilué avec 200 mL d'acétate d'éthyle, lavé avec une solution saturée de dihydrogénophosphate de sodium. La phase organique est décantée, séchée sur sulfate de magnésium et concentrée sous pression réduite. Après purification sur gel de silice (70-230 Mesh) en éluant avec un mélange de dichlorométhane et de n.heptane (66-34 en volumes), on obtient 1,4 g d'ester 4-tert.butylique 9,9-diméthylique de l'acide fluorène-4,9,9-tricarboxylique, sous forme d'une huile incolore visqueuse utilisée telle quelle à l'étape suivante.

Etape 2 : Dans un tricol de 500 mL, on dissout 1,4 g d'ester 4-tert.butyle 9,9-diméthyle de l'acide fluorène-4,9,9-tricarboxylique, obtenu à l'étape précédente, dans 10 mL de dichlorméthane, puis après refroidissement à 0°C on ajoute 10 mL d'acide trifluoroacétique et on agite 1 heure à 0°C. Après concentration sous pression réduite, on obtient 1,2 g d'ester 9,9-diméthylique de l'acide fluorène-4,9,9-tricarboxylique, sous forme d'une poudre blanche utilisée telle quelle à l'étape suivante.

Etape 3 : Dans un tricol de 500 mL, on agite, pendant une heure et quinze minutes à température ambiante, 1,2 g d'ester 9,9-diméthylique de l'acide fluorène-4,9,9-tricarboxylique, obtenu à l'étape précédente dans 35 mL de chlorure de thionyle. Après concentration sous pression réduite, le chlorure d'acide obtenu (1,33 g) est dissout dans 40 mL de dichlorométhane, puis on ajoute 0,6 g de 3,4-diaminopyridine et on agite une heure et trente minutes à température ambiante. Après purification par flash-chromatographie sur gel de silice (70-230 Mesh) en éluant avec un mélange de dichlorométhane et de méthanol (96-4 en volumes), on obtient 1,3 g d'un mélange équimoléculaire d'ester diméthylique de l'acide 4-(4-amino-pyridin-3-ylcarbamoyl)-fluorène-9,9-dicarboxylique et d'ester diméthylique de l'acide 4-(3-amino-pyridin-4-ylcarbamoyl)-fluorène-9,9-dicarboxylique, sous forme d'une poudre beige utilisée telle quelle à l'étape suivante.

Etape 4: Dans un tricol de 25 mL, on dissout 1,3 g du mélange d'amides obtenu à l'étape précédente dans 35 mL d'acide trifluoroacétique, 3,5 mL d'anhydride trifluoroacétique et 1,5 mL d'acide chlorhydrique à 36%. On agite alors pendant 2 jours à 90°C, puis on ajoute en trois fois 8,5 mL d'acide chlorhydrique à 36% et on chauffe de nouveau pendant 60 heures à 90°C. Après concentration sous pression réduite, le résidu est purifié par flash-chromatographie sur gel de silice (70-230 Mesh) en éluant avec un mélange de dichlorométhane et de méthanol (95-5 puis 90-10 en volumes). On obtient ainsi 1 g de chlorhydrate de l'acide 4-(1H-Imidazo[4,5-c]pyridin-2-yl)-9H-fluorène-9(R,S)-carboxylique, sous forme d'une poudre écrue dont les caractéristiques sont les suivantes :
Spectre de masse (E/I) : m/z = 363 (M+)

### Exemple 30 : Synthèse du trifluoroacétate de 4-(3H-imidazo[4,5-c]pyridin-2-yl)-fluorène-9-one semicarbazone (Z, E)

Dans un tricol de 5 mL, on agite, pendant 6 heures à température ambiante, une suspension contenant 35 mg de 4-(3H-Imidazo[4,5-c]pyridin-2-yl)-fluorène-9-one, obtenue à l'exemple 1, 29 mg d'acétate de sodium et 20 mg de chlorhydrate de semicarbazide dans 0,4 mL d'une solution aqueuse à 50% d'éthanol. On rajoute 29 mg d'acétate de sodium et 20 mg de chlorhydrate de semicarbazide puis on agite de nouveau pendant 18 heures à température ambiante. Après addition de 2 mL d'eau, le précipité formé est essoré et lavé 2 fois avec 1 mL d'eau. Après purification par HPLC/MS sur une colonne de silice Symmetry C18 (5 µm), en éluant par un gradient eau 100% (contenant 0,07% de TFA) à acétonitrile 100% (contenant 0,07% de TFA), on obtient 5,5 mg de trifluoroacétate de 4-(3H-imidazo[4,5-c]pyridin-2-yl)-fluorène-9-one semicarbazone (Z, E), sous forme d'un solideblanc dont les caractéristiques sont les suivantes :
Spectre de masse (E/I) : m/z = 354 (M+).

### Exemple 31 : Synthèse du [4-(3H-imidazo[4,5-c]pyridin-2-yl)-9H-fluorèn-9(R,S)-yl]-amide de l'acide 2-hydroxy-quinoléine-4-carboxylique.

On opère comme à l'exemple 14 à partir de 298 mg de 4-(3H-imidazo[4,5-c]pyridin-2-yl)-fluorène-9(R,S)-amine, obtenue à l'exemple 6, et de 189 mg d'acide 2-hydroxy-quinoléine-4-carboxylique en présence de 211 mg d'EDCI et de 149 mg d'HOBT dans 7 mL de DMF pendant 20 heures. Après purification par précipitation dans l'eau, puis lavages successifs avec une solution saturée d'hydrogénocarbonate de sodium puis avec de l'isopropanol, on obtient 450 mg de [4-(3H-imidazo[4,5-c]pyridin-2-yl)-9H-fluorèn-9(R,S)-yl]-amide de l'acide 2-hydroxy-quinoléine-4-carboxylique, sous forme d'un solide brun, dont les caractéristiques sont les suivantes :
Spectre de masse (E/I) : m/z = 469 (M+).

### Exemple 32 : Synthèse du [4-(3H-imidazo[4,5-c]pyridin-2-yl)-9H-fluoren-9(R,S)-yl]-amide de l'acide tetrahydro-pyran-4-carboxylique

On opère comme à l'exemple 14 à partir de 202 mg de 4-(3H-imidazo[4,5-c]pyridin-2-yl)-fluorène-9(R,S)-amine, obtenue à l'exemple 6, et de 80 mg d'acide tetrahydro-pyran-4-carboxylique en présence de 177 mg d'EDCI et de 125 mg d'HOBT dans 3,4 mL de DMF pendant 20 heures. Après purification par précipitation dans l'eau, puis lavages successifs avec une solution saturée d'hydrogénocarbonate de sodium puis avec de l'éther de pétrole, on obtient 253 mg de [4-(3H-imidazo[4,5-c]pyridin-2-yl)-9H-fluoren-9(R,S)-yl]-amide de l'acide tetrahydro-pyran-4-carboxylique, sous forme d'un solide blanc cristallin, dont les caractéristiques sont les suivantes :
Spectre de masse (E/I) : m/z = 410 (M+).

### Exemple 33 : Synthèse de l'ester de tert-butyle de l'acide 4-[4-(3H-imidazo[4,5-]pyridin-2-yl)-9H-fluorèn-9(R,S)-ylcarbamoyl]-pipéridine-1-carboxylique.

On opère comme à l'exemple 14 à partir de 208 mg de 4-(3H-imidazo[4,5-c]pyridin-2-yl)-fluorène-9(R,S)-amine, obtenue à l'exemple 6, et de 145,4 mg d'acide tert-butoxycarbonyl-nipécotique en présence de 182 mg d'EDCI et de 128,5 mg d'HOBT dans 3,5 mL de DMF pendant 20 heures. Après purification par précipitation dans l'eau, puis lavages successifs avec une solution saturée d'hydrogénocarbonate de sodium puis avec de l'éther de pétrole, on obtient 195 mg d'ester de tert-butyle de l'acide 4-[4-(3H-imidazo[4,5-c]pyridin-2-yl)-9H-fluorèn-9(R,S)-ylcarbamoyl]-pipéridine-1-carboxylique, sous forme d'un solide beige cristallin, dont les caractéristiques sont les suivantes :
Spectre de masse (E/I) : m/z = 509 (M+).

### Exemple 34 : Synthèse du chlorhydrate du [4-(3H-imidazo[4,5-c]pyridin-2-yl)-9H-fluorèn-9(R,S)-yl]-amide de l'acide 6-hydroxy-naphthalène-1-carboxylique.

On opère comme à l'exemple 14 à partir de 208 mg de 4-(3H-imidazo[4,5-c]pyridin-2-yl)-fluorène-9(R,S)-amine, obtenue à l'exemple 6, et de 119 mg 6-hydroxy-naphthalène-1-carboxylique en présence de 182 mg d'EDCI et de 128,5 mg d'HOBT dans 3,5 mL de DMF pendant 20 heures. Après purification par flash-chromatographie sur gel de silice (70-230 M), en éluant par un mélange de dichlorométhane et de méthanol (95-5 en volumes), on obtient 100 mg de chlorhydrate du [4-(3H-imidazo[4,5-c]pyridin-2-yl)-9H-fluorèn-9(R,S)-yl]-amide de l'acide 6-hydroxy-naphthalène-1-carboxylique, sous forme d'un solide blanc cristallin, dont les caractéristiques sont les suivantes :
Spectre de masse (E/I) : m/z = 468 (M+).

### Exemple 35 : Synthèse du [4-(3H-imidazo[4,5-c]pyridin-2-yl)-9H-fluorèn-9(R,S)-yl]-amide de l'acide isoquinoléine-5-carboxylique.

On opère comme à l'exemple 14 à partir de 208 mg de 4-(3H-imidazo[4,5-c]pyridin-2-yl)-fluorène-9(R,S)-amine, obtenue à l'exemple 6, et de 110 mg d'acide isoquinoléine-5-carboxylique en présence de 182 mg d'EDCI et de 128,5 mg d'HOBT dans 10,5 mL de DMF pendant 20 heures. Après purification par flash-chromatographie sur gel de silice (70-230 M), en éluant par un mélange de dichlorométhane et de méthanol (95-5 en volumes), on obtient 106 mg de [4-(3H-imidazo[4,5-c]pyridin-2-yl)-9H-fluorèn-9(R,S)-yl]-amide de l'acide isoquinoléine-5-carboxylique, sous forme d'un solide jaune cristallin, dont les caractéristiques sont les suivantes :
Spectre de masse (E/I) : m/z = 453 (M+).

### Exemple 36 : Synthèse du 2-furan-3-yl-N-[4-(3H-imidazo[4,5-c]pyridin-2-yl)-9H-fluorèn-9(R,S)-yl]-2-oxo-acétamide.

On opère comme à l'exemple 14 à partir de 351 mg de 4-(3H-imidazo[4,5-c]pyridin-2-yl)-fluorène-9(R,S)-amine, obtenue à l'exemple 6, et de 150 mg d'acide 3-furylglyoxylique en présence de 308 mg d'EDCI et de 217 mg d'HOBT dans 5,9 mL de DMF pendant 20 heures. Après purification par flash-chromatographie sur gel de silice (70-230 M), en éluant par un mélange de dichlorométhane et de méthanol (97,5-2,5 en volumes), on obtient 96 mg de 2-furan-3-yl-N-[4-(3H-imidazo[4,5-c]pyridin-2-yl)-9H-fluorèn-9(R,S)-yl]-2-oxo-acétamide, sous forme d'un solide jaune-pâle cristallin, dont les caractéristiques sont les suivantes :
Sspectre de masse (E/I) : m/z = 420 (M+).

### Exemple 37 : Synthèse du trifluoroacétate du N-[4-(3H-imidazo[4,5-c]pyridin-2-yl)-9H-fluorèn-9(RS)-yl]-2-pyridin-4-yl-acétamide

On opère comme à l'exemple 24, mais à partir de 50 mg de 4-(3H-Imidazo[4,5-c]pyridin-2-yl)-fluorène-9(R,S)-amine, obtenue à l'exemple 6, 300 µL d'une solution 2M de triméthylaluminium dans le toluène et 46 µL d'ester éthylique de l'acide (4-pyridyl)acétique dans 2 mL de toluène et 0,5 mL de N-méthylpyrrolidone pendant 10 minutes à température ambiante puis 10 minutes à 60°C dans un réacteur microondes. Après purification par HPLC/MS sur une colonne de silice Symmetry C18 (5 µm), en éluant par un gradient eau 100% (contenant 0,07% de TFA) à acétonitrile 100% (contenant 0,07% de TFA), on obtient 7,8 mg de trifluoroacétate du N-[4-(3H-imidazo[4,5-c]pyridin-2-yl)-9H-fluorèn-9(RS)-yl]-2-pyridin-4-yl-acétamide, sous forme d'un solide blanc dont les caractéristiques sont les suivantes :
Spectre de masse (E/I) : m/z = 417 (M+).

### Exemple 38 : Synthèse du trifluoroacétate du [4-(3H-imidazo[4,5-c]pyridin-2-yl)-9H-fluorèn-9(R,S)-yl]-amide de l'acide 2H-1-benzopyran-5-carboxylique.

On opère comme à l'exemple 14 à partir de 74 mg de 4-(3H-imidazo[4,5-c]pyridin-2-yl)-fluorène-9(R,S)-amine, obtenue à l'exemple 6, et de 39 mg d'acide 2H-1-benzopyran-5-carboxylique, en présence de 57,5 mg d'EDCI, 40,5 mg d'HOBT et 70 µL de diisopropyléthylamine dans 1 mL de DMF pendant 24 heures. Après purification par HPLC/MS sur une colonne de silice Symmetry C18 (5 µm), en éluant par un gradient eau 100% (contenant 0,07% de TFA) à acétonitrile 100% (contenant 0,07% de TFA), on obtient 21 mg de trifluoroacétate du [4-(3H-imidazo[4,5-c]pyridin-2-yl)-9H-fluorèn-9(R,S)-yl]-amide de l'acide 2H-1-benzopyran-5-carboxylique, sous forme d'un solide beige dont les caractéristiques sont les suivantes :
Spectre de masse (E/I) : m/z = 456 (M+).
L'acide 2H-1-benzopyran-5-carboxylique peut être obtenu selon W02005012291.

### Exemple 39 : Synthèse du trifluoroacétate du [4-(3H-imidazo[4,5-c]pyridin-2-yl)-9H-ffuorèn-9(R,S)-yl]-amide de l'acide 1H-pyrazole-3-carboxylique.

On opère comme à l'exemple 14 à partir de 75 mg de 4-(3H-imidazo[4,5-c]pyridin-2-yl)-fluorène-9(R,S)-amine, obtenue à l'exemple 6, et de 42 mg 1H-pyrazole-3-carboxylique, en présence de 72 mg d'EDCI, 51 mg d'HOBT et 131 µL de diisopropyléthylamine dans 1,25 mL de DMFet 1,25 mL de dichlorométhane pendant 24 heures. Après purification par HPLC/MS sur une colonne de silice Symmetry C18 (5 µm), en éluant par un gradient eau 100% (contenant 0,07% de TFA) à acétonitrile 100% (contenant 0,07% de TFA), on obtient 58,5 mg de trifluoroacétate du [4-(3H-imidazo[4,5-c]pyridin-2-yl)-9H-fluorèn-9(R,S)-yl]-amide de l'acide 1H-pyrazole-3-carboxylique, sous forme d'un solide blanc dont les caractéristiques sont les suivantes :
Spectre de masse (E/I) : m/z = 392 (M+).

### Exemple 40 : Synthèse du trifluoroacétate du N-[4-(3H-imidazo[4,5-c]pyridin-2-yl)-9H-fluorèn-9(R,S)-yl]-succinamide.

On opère comme à l'exemple 14 à partir de 74,6 mg de 4-(3H-imidazo[4,5-c]pyridin-2-yl)-fluorène-9(R,S)-amine, obtenue à l'exemple 6, et de 43,9 mg d'acide succinamique, en présence de 71,9 mg d'EDCI de 50,7 mg d'HOBT et 131 µL de diisopropylamine dans 1,25 mL de dichlorométhane et 1,25 mL de DMF pendant 20 heures. Après purification par HPLC/MS sur silice Symmetry C18 (5 µm), en opérant dans les conditions décrites à l'exemple 28, on obtient 63 mg de trifluoroacétate du N-[4-(3H-imidazo[4,5-c]pyridin-2-yl)-9H-fluorèn-9(R,S)-yl]-succinamide, sous forme d'un solide blanc, dont les caractéristiques sont les suivantes :
pectre de masse (E/I) : m/z = 397 (M+).

### Exemple 41 : Synthèse du trifluoroacétate du [4-(3H-imidazo[4,5-c]pyridin-2-yl)-9H-fluorèn-9(R,S)-yl]-amide de l'acide 1H-benzotriazole-5-carboxylique.

On opère comme à l'exemple 14 à partir de 74,62 mg de 4-(3H-imidazo[4,5-c]pyridin-2-yl)-fluorène-9(R,S)-amine, obtenue à l'exemple 6, et de 61,2 mg d'acide 1H-benzotriazole-5-carboxylique, qui peut être obtenu selon Archiv. Pharm. 1989, 322, 457-59, en présence de 71,9 mg d'EDCI, de 50,7 mg d'HOBT et de 131 µL de diisopropylamine dans 1,25 mL de dichlorométhane et 1,25 mL de DMF pendant 20 heures. Après purification par HPLC/MS dans les conditions décrites à l'exemple 28, on obtient 66,5 mg de trifluoroacétate du [4-(3H-imidazo[4,5-c]pyridin-2-yl)-9H-fluorèn-9(R,S)-yl]-amide de l'acide 1H-benzotriazole-5-carboxylique, sous forme d'un solide blanc, dont les caractéristiques sont les suivantes :
Spectre de masse (E/l) : m/z = 443 (M+).
Spectre RMN 1H (300 MHz, □en ppm, DMSO-d6) : 6,41 (d, J = 8,5 Hz, 1H) ; 7,27 (t large, J = 7,5 Hz, 1H) ; 7,38 (t large, J = 7,5 Hz, 1H) ; de 7,54 à 7,67 (m, 3H) ; 7,77 (d large, J = 7,5 Hz, 1H) ; 7,85 (d large, J = 7,5 Hz, 1H) ; de 7,88 à 8,13 (m très étalé, 2H) ; 8,15 (d large, J = 6,5 Hz, 1H); de 8,50 à 8,70 (m très étalé, 1H) ; 8,64 (d, J = 6,5 Hz, 1H) ; 9,37 (d, J = 8,5 Hz, 1H) ; 9,53 (s large, 1H) ; de 14,1 à 15,2 (m très étalé, 1H) ; de 15,7 à 16,3 (m étalé, 1H).

### Exemple 42 : Synthèse du trifluoroacétate du N-[4-(3H-imidazo[4,5-c]pyridin-2-yl)-9H-fluorèn-9(R,S)-yl]-2-(5-mercapto-4H-1,2,4-triazol-3-yl)-acétamide.

On opère comme à l'exemple 14 à partir de 75 mg de 4-(3H-imidazo[4,5-c]pyridin-2-yl)-fluorène-9(R,S)-amine, obtenue à l'exemple 6, et de 44 mg de l'acide (5-mercapto-4H-1,2,4-triazol-3-yl)-acétique, en présence de 72 mg d'EDCI, 51 mg d'HOBT et 131 µL de diisopropyléthylamine dans 1,25 mL de DMFet 1,25 mL de dichlorométhane pendant 24 heures. Après purification par HPLC/MS sur une colonne de silice Symmetry C18 (5 µm), en éluant par un gradient eau 100% (contenant 0,07% de TFA) à acétonitrile 100% (contenant 0,07% de TFA), on obtient 67,5 mg de trifluoroacétate du N-[4-(3H-imidazo[4,5-c]pyridin-2-yl)-9H-fluorèn-9(R,S)-yl]-2-(5-mercapto-4H-1,2,4-triazol-3-yl)-acétamide, sous forme d'un solide jaune-pâle dont les caractéristiques sont les suivantes :
Spectre de masse (E/l) : m/z = 439 (M+).

### Exemple 43: Synthèse du [4-(3H-imidazo[4,5-c]pyridin-2-yl)-9H-fluorèn-9(R,S)-yl]-amide de l'acide 2-oxo-2,3-dihydro-1H-benzimidazole-5-carboxylique.

On opère comme à l'exemple 14 à partir de 202 mg de 4-(3H-imidazo[4,5-c]pyridin-2-yl)-fluorène-9(R,S)-amine, obtenue à l'exemple 6, et de 110 mg d'acide 2-oxo-2,3-dihydro-1H-benzimidazole-5-carboxylique, en présence de 177 mg d'EDCI et de 125 mg d'HOBT dans 5,9 mL de DMF pendant 20 heures. Après purification par flash-chromatographie sur gel de silice (70-230 M), en éluant par un mélange de dichlorométhane et de méthanol (95-5 en volumes), on obtient 139 mg de [4-(3H-imidazo[4,5-c]pyridin-2-yl)-9H-fluorèn-9(R,S)-yl]-amide de l'acide 2-oxo-2,3-dihydro-1H-benzimidazole-5-carboxylique, sous forme d'un solide jaune-pâle cristallin, dont les caractéristiques sont les suivantes :
Spectre de masse (E/l) : m/z = 458 (M+).

### Exemple 44: Synthèse du [4-(3H-imidazo[4,5-c]pyridin-2-yl)-9H-fluorèn-9(R,S)-yl]-amide de l'acide 6-chloro-1H-benzimidazole-4-carboxylique.

On opère comme à l'exemple 14 à partir de 200 mg de 4-(3H-imidazo[4,5-c]pyridin-2-yl)-fluorène-9(R,S)-amine, obtenue à l'exemple 6, et de 120 mg d'acide 6-chloro-1H-benzimidazole-4-carboxylique, en présence de 175 mg d'EDCI et de 124 mg d'HOBT dans 5,8 mL de DMF pendant 20 heures. Après purification par flash-chromatographie sur gel de silice (70-230 M), en éluant par un mélange de dichlorométhane et de méthanol (95-5 en volumes), on obtient 40 mg de [4-(3H-imidazo[4,5-c]pyridin-2-yl)-9H-fluorèn-9(R,S)-yl]-amide de l'acide 6-chloro-1H-benzimidazole-4-carboxylique, sous forme d'un solide jaune-pâle cristallin, dont les caractéristiques sont les suivantes :
Spectre de masse (E/l) : m/z = 476 (M+).

### Exemple 45: Synthèse du [4-(3H-imidazo[4,5-c]pyridin-2-yl)-9H-fluorèn-9(R,S)-yl]-amide de l'acide 1H-indazole-5-carboxylique.

On opère comme à l'exemple 14 à partir de 202 mg de 4-(3H-imidazo[4,5-c]pyridin-2-yl)-fluorène-9(R,S)-amine, obtenue à l'exemple 6, et de 122,5 mg de chlorhydrate de l'acide 1H-indazole-5-carboxylique, en présence de 177 mg d'EDCI de 124 mg d'HOBT et de 122 µL de diisopropylamine dans 5,8 mL de DMF pendant 20 heures. Après purification par flash-chromatographie sur gel de silice (70-230 M), en éluant par un mélange de dichlorométhane et de méthanol (95-5 en volumes), on obtient 87 mg de [4-(3H-imidazo[4,5-c]pyridin-2-yl)-9H-fluorèn-9(R,S)-yl]-amide de l'acide 1H-indazole-5-carboxylique, sous forme d'un solide jaune-pâle cristallin, dont les caractéristiques sont les suivantes :
Spectre de masse (E/l): m/z = 442 (M+).

### Exemple 46 : Synthèse du [4-(3H-imidazo[4,5-c]pyridin-2-yl)-9H-fluorèn-9(R,S)-yl]-amide de l'acide 1H-indole-6-carboxylique.

On opère comme à l'exemple 14 à partir de 204 mg de 4-(3H-imidazo[4,5-c]pyridin-2-yl)-fluorène-9(R,S)-amine, obtenue à l'exemple 6, et de 100 mg d'acide 1H-indole-6-carboxylique, en présence de 179 mg d'EDCI et de 126 mg d'HOBT dans 3 mL de DMF pendant 20 heures. Après purification par flash-chromatographie sur gel de silice (70-230 M), en éluant par un mélange de dichlorométhane et de méthanol (90-10 en volumes), on obtient 54 mg de [4-(3H-imidazo[4,5-c]pyridin-2-yl)-9H-fluorèn-9(R,S)-yl]-amide de l'acide 1H-indole-6-carboxylique, sous forme d'un solide jaune-pâle, dont les caractéristiques sont les suivantes :
Spectre de masse (E/l) : m/z = 441 (M+).

### Exemple 47 : Synthèse du N-[4-(3H-imidazo[4,5-c]pyridin-2-yl)-9H-fluorèn-9(R,S)-yl]-isonicotinamide.

On opère comme à l'exemple 14 à partir de 208 mg de 4-(3H-imidazo[4,5-c]pyridin-2-yl)-fluorène-9(R,S)-amine, obtenue à l'exemple 6, et de 78 mg d'acide isonicotinique, en présence de 182 mg d'EDCI et de 128 mg d'HOBT dans 3,5 mL de DMF pendant 20 heures. Après purification par flash-chromatographie sur gel de silice (70-230 M), en éluant par un mélange de dichlorométhane et de méthanol (95-5 en volumes), on obtient 90 mg de N-[4-(3H-imidazo[4,5-c]pyridin-2-yl)-9H-fluorèn-9(R,S)-yl]-isonicotinamide, sous forme d'un solide blanc cristallin, dont les caractéristiques sont les suivantes :
Spectre de masse (E/l) : m/z = 403 (M+).
Spectre RMN 1H (300 MHz, □ en ppm, DMSO-d6) : 6,36 (d, J = 8,5 Hz, 1H) ; 7,26 (t large, J = 7,5 Hz, 1H) ; 7,34 (t large, J = 7,5 Hz, 1H) ; de 7,48 à 7,60 (m, 3H) ; de 7,65 à 7,77 (m, 3H); 7,87 (d large, J = 6,0 Hz, 2H) ; 8,39 (d, J = 5,5 Hz, 1H); 8,75 (d large, J = 6,0 Hz, 2H) ; 9,04 (s large, 1H) ; 9,47 (d, J = 8,5 Hz, 1H); de 13,0 à 14,0 (m très étalé, 1H).

### Exemple 48 : Synthèse du N-[4-(3H-imidazo[4,5-c]pyridin-2-yl)-9H-fluorèn-9(R,S)-yl]-nicotinamide.

On opère comme à l'exemple 14 à partir de 208 mg de 4-(3H-imidazo[4,5-c]pyridin-2-yl)-fluorène-9(R,S)-amine, obtenue à l'exemple 6, et de 78 mg d'acide nicotinique, en présence de 182 mg d'EDCI et de 128 mg d'HOBT dans 3,5 mL de DMF pendant 20 heures. Après purification par flash-chromatographie sur gel de silice (70-230 M), en éluant par un mélange de dichlorométhane et de méthanol (95-5 en volumes), on obtient 78 mg de N-[4-(3H-imidazo[4,5-c]pyridin-2-yl)-9H-fluorèn-9(R,S)-yl]-nicotinamide, sous forme d'un solide vert-pâle cristallin, dont les caractéristiques sont les suivantes :
Spectre de masse (E/l) : m/z = 403 (M+).

### Exemple 49 : Synthèse du chlorhydrate du [4-(3H-imidazo[4,5-c]pyridin-2-yl)-9H-fluorèn-9(R,S)-yl]-amide de l'acide 4-oxo-pentanoïque.

On opère comme à l'exemple 14 à partir de 208 mg de 4-(3H-imidazo[4,5-c]pyridin-2-yl)-fluorène-9(R,S)-amine, obtenue à l'exemple 6, et de 73,6 mg d'acide lévulinique, en présence de 182 mg d'EDCI et de 128 mg d'HOBT dans 3,5 mL de DMF pendant 20 heures. Après purification par flash-chromatographie sur gel de silice (70-230 M), en éluant par un mélange de dichlorométhane et de méthanol (95-5 en volumes), on obtient 62 mg de chlorhydrate du [4-(3H-imidazo[4,5-c]pyridin-2-yl)-9H-fluorèn-9(R,S)-yl]-amide de l'acide 4-oxo-pentanoique, sous forme d'un solide blanc cristallin, dont les caractéristiques sont les suivantes :
Spectre de masse (E/l) : m/z = 396 (M+).

### Exemple 50 : Synthèse du chlorhydrate de 3-hydroxy-N-[4-(3H-imidazo[4,5-c]pyridin-2-yl)-9H-fluorèn-9(R,S)-yl]-benzamide.

On opère comme à l'exemple 14 à partir de 208 mg de 4-(3H-imidazo[4,5-c]pyridin-2-yl)-fluorène-9(R,S)-amine, obtenue à l'exemple 6, et de 87,5 mg d'acide 3-hydroxy-benzoique, en présence de 182 mg d'EDCI et de 128 mg d'HOBT dans 3,5 mL de DMF pendant 20 heures. Après purification par flash-chromatographie sur gel de silice (70-230 M), en éluant par un mélange de dichlorométhane et de méthanol (95-5 en volumes), on obtient 34 mg chlorhydrate de 3-hydroxy-N-[4-(3H-imidazo[4,5-c]pyridin-2-yl)-9H-fluorèn-9(R,S)-yl]-benzamide, sous forme d'un solide vert-pâle cristallin, dont les caractéristiques sont les suivantes :
Spectre de masse (E/l) : m/z = 418 (M+).

### Exemple 51 : Synthèse du trifluoroacétate du [4-(3H-imidazo[4,5-c]pyridin-2-yl)-9H-fluorèn-9(R,S)-yl]-amide de l'acide morpholine-4-carboxylique.

Dans un ballon de 10 mL sous atmosphère d'argon, on dissout 34 mg de 4-(3H-imidazo[4,5-c]pyridin-2-yl)-fluorène-9(R,S)-amine, obtenue à l'exemple 6, dans 0,5 mL de tetrahydrofurane contenant 9,5 µL de pyridine, puis on ajoute 23 mg de chloroformiate de p.nitrophényle et on agite 2 heures à 20°C. On ajoute alors 2 µL de pyridine et 2,5 mg de chloroformiate de p.nitrophényle supplémentaires et on agite de nouveau 2 heures à température ambiante. On ajoute enfin 70 µL de morpholine en solution dans 0,5 mL de dichlorométhane et on agite 3 jours à température ambiante. Le milieu réactionnel est concentré sous pression réduite. Après purification par HPLC/MS dans les conditions décrites à l'exemple 28, on obtient 4,7 mg de trifluoroacétate du [4-(3H-imidazo[4,5-c]pyridin-2-yl)-9H-fluorèn-9(R,S)-yl]-amide de l'acide morpholine-4-carboxylique, sous forme d'un solide jaune, dont les caractéristiques sont les suivantes :
Spectre de masse (E/l) : m/z = 411 (M+)

### Exemple 52 : Synthèse du trifluoroacétate du [4-(3H-imidazo[4,5-c]pyridin-2-yl)-9H-fluorèn-9(R,S)-yl]-amide de l'acide 5-diméthylamino-naphthalène-1-sulfonique.

Dans un ballon de 10 mL sous atmosphère d'argon, on dissout 72 mg de 4-(3H-imidazo[4,5-c]pyridin-2-yl)-fluorène-9(R,S)-amine, obtenue à l'exemple 6, dans 1,2 mL de tetrahydrofurane contenant 40 µl de triéthylamine, puis on ajoute 72 mg de chlorure de dansyle et on agite pendant 3 jours à température ambiante. Le milieu réactionnel est concentré sous pression réduite. Après purification par HPLC/MS dans les conditions décrites à l'exemple 28, on obtient 18,5 mg de trifluoroacétate du [4-(3H-imidazo[4,5-c]pyridin-2-yl)-9H-fluorèn-9(R,S)-yl]-amide de l'acide 5-diméthylamino-naphthalène-1-sulfonique, sous forme d'un solide jaune, dont les caractéristiques sont les suivantes :
Spectre de masse (E/l) : m/z = 531 (M+).
Spectre RMN 1H (400 MHz, □ en ppm, DMSO-d6) : 2,90 (s, 6H) ; 5,42 (d, J = 9,0 Hz, 1H) ; 6,85 (d large, J = 7,5 Hz, 1H) ; de 7,07 à 7,16 (m, 2H) ; 7,20 (d large, J = 8,0 Hz, 1H); de 7,32 à 7,40 (m, 2H); 7,47 (d large, J = 8,0 Hz, 1H); de 7,63 à 7,72 (m, 3H) ; 8,20 (m étalé, 1H); 8,36 (d large, J = 8,0 Hz, 1H); 8,43 (d large, J= 8,0 Hz, 1H) ; 8,57 (d large, J = 8,5 Hz, 1H); 8,65 (d, J = 6,5 Hz, 1H) ; 8,90 (d, J = 9,0 Hz, 1H) ; de 9,55 à 9,64 (m très étalé, 1H) ; de 14,4 à 14,9 (m très étalé, 1H).

### Exemple 53: Synthèse du [4-(3H-imidazo[4,5-c]pyridin-2-yl)-9H-fluorèn-9(R,S)-yl]-amide de l'acide 1H-indole-4-carboxylique

On opère comme à l'exemple 14 à partir de 75 mg de 4-(3H-imidazo[4,5-c]pyridin-2-yl)-fluorène-9(R,S)-amine, obtenue à l'exemple 6, et de 60 mg de l'acide 1H-indole-4-carboxylique, en présence de 72 mg d'EDCI, 51 mg d'HOBT et 131 µL de diisopropyléthylamine dans 1,25 mL de DMF et 1,25 mL de dichlorométhane pendant 24 heures. Après purification par HPLC/MS sur une colonne de silice Symmetry C18 (5 µm), en éluant par un gradient eau 100% (contenant 0,07% de TFA) à acétonitrile 100% (contenant 0,07% de TFA), puis flash-chromatographie sur une cartouche de silice SCX MegaBond Elut, en éluant avec du méthanol, on obtient 54 ,5 mg du [4-(3H-imidazo[4,5-c]pyridin-2-yl)-9H-fluorèn-9(R,S)-yl]-amide de l'acide 1H-indole-4-carboxylique, sous forme d'un solide jaune-pâle dont les caractéristiques sont les suivantes :
Spectre de masse (E/l) : m/z = 441 (M+)

### Exemple 54 : Synthèse du [4-(3H-imidazo[4,5-c]pyridin-2-yl)-9H-fluorèn-9(R,S)-yl]-amide de l'acide 6-amino-naphthalène-1-carboxylique.

Etape 1 : On opère comme à l'exemple 14 à partir de 414 mg de 4-(3H-imidazo[4,5-c]pyridin-2-yl)-fluorène-9(R,S)-amine, obtenue à l'exemple 6, et de 274 mg d'acide 6-nitro-naphthalène-1-carboxylique, en présence de 363 mg d'EDCI et de 256 mg d'HOBT dans 3,5 mL de DMF pendant 20 heures. Après purification par flash-chromatographie sur gel de silice (70-230 M), en éluant par un mélange de dichlorométhane et de méthanol (95-5 en volumes), on obtient 300 mg de [4-(3H-imidazo[4,5-c]pyridin-2-yl)-9H-fluorèn-9(R,S)-yl]-amide de l'acide 6-nitro-naphthalène-1-carboxylique, sous forme d'un solide jaune cristallin, dont les caractéristiques sont les suivantes :
Spectre de masse (E/l) : m/z = 497 (M+).

Etape 2: 524 mg de [4-(3H-imidazo[4,5-c]pyridin-2-yl)-9H-fluorèn-9(R,S)-yl]-amide de l'acide 6-nitro-naphthalène-1-carboxylique, obtenu comme à l'étape précédente, sont hydrogénés en solution dans 30 mL d'éthanol et 40 mL de diméthylformamide, à température ambiante en autoclave sous 1 bar de pression pendant 20 heures, en présence de 16 mg de Palladium sur charbon à 10%. Après filtration sur Célite, le filtrat est concentré sous pression réduite. Après purification par flash-chromatographie sur gel de silice (70-230 M), en éluant par un mélange de dichlorométhane et de méthanol (95-5 en volumes), on obtient 176 mg de [4-(3H-imidazo[4,5-c]pyridin-2-yl)-9H-fluorèn-9(R,S)-yl]-amide de l'acide 6-amino-naphthalène-1-carboxylique, sous forme d'un solide blanc cristallin, dont les caractéristiques sont les suivantes :
Spectre de masse (E/l) : m/z = 467 (M+).

### Exemple 55 : Synthèse du trifluoroacétate du [4-(3H-imidazo[4,5-c]pyridin-2-yl)-9H-fluorèn-9(R,S)-yl]-amide de l'acide quinoléine-5-carbothioique

157 mg de [4-(3H-imidazo[4,5-c]pyridin-2-yl)-9H-fluorèn-9(R,S)-yl]-amide de l'acide quinoléine-5-carboxylique, obtenu comme à l'exemple 14, sont dissout dans 10 mL de dioxane et chauffés pendant 15 heures à une température voisine de 100°C en présence de 80 mg de réactif de Lawesson. Après refroidissement le milieu réactionnel est concentré sous pression réduite. Après purification par HPLC/MS dans les conditions décrites à l'exemple 28, on obtient 42 mg de trifluoroacétate du [4-(3H-imidazo[4,5-c]pyridin-2-yi)-9H-fluorèn-9(R,S)-yi]-amide de l'acide quinoléine-5-carbothioïque, sous forme d'un solide cristallin jaune, dont les caractéristiques sont les suivantes :
Spectre de masse (E/l) : m/z = 469 (M+)
Spectre RMN 1H (300 MHz, □ en ppm, DMSO-d6) : 7,31 (t large, J = 7,5 Hz, 1H) ; de 7,37 à 7,49 (m, 2H) ; de 7,60 à 7,70 (m, 4H) ; de 7,75 à 7,84 (m, 2H) ; 7,90 (d large, J = 7,5 Hz, 1H) ; 8,06 (d large, J = 8,0 Hz, 1H) ; 8,11 (d large, J = 8,0 Hz, 1H); 8,22 (d large, J = 6,5 Hz, 1H); de 8,64 à 8,73 (m, 2H) ; 8,96 (dd, J = 2,0 et 4,0 Hz, 1H) ; 9,59 (s large, 1H) ; 11,32 (d, J = 9,0 Hz, 1H) ; de 14,2 à 15,0 (m très étalé, 2H).

### Exemple 56 : Synthèse du N-[4-(3H-Imidazo[4,5-c]pyridin-2-yl)-9H-fluorèn-9(R,S)-yl]-1-oxy-nicotinamide.

On opère comme à l'exemple 14 à partir de 212 mg de 4-(3H-imidazo[4,5-c]pyridin-2-yl)-fluorène-9(R,S)-amine, obtenue à l'exemple 6, et de 90 mg de N-oxyde de l'acide nicotinique, en présence de 186 mg d'EDCI et de 131 mg d'HOBT dans 5,2 mL de DMF pendant 20 heures. Après purification par flash-chromatographie sur gel de silice (70-230 M), en éluant par un mélange de dichlorométhane et de méthanol (95-5 en volumes), on obtient 109 mg de N-[4-(3H-imidazo[4,5-c]pyridin-2-yl)-9H-fluorèn-9(R,S)-yl]-1-oxy-nicotinamide, sous forme d'un solide cristallin vert-pâle, dont les caractéristiques sont les suivantes :
Spectre de masse (E/l) : m/z = 419 (M+).

### Exemple 57 : Synthèse de l'ester de tert-butyle de l'acide {(S)-2-carbamoyl-1-[4-(3H-imidazo[4,5-c]pyridin-2-yl)-9H-fluorèn-9(R,S)-ylcarbamoyl]-éthyl}-carbamoïque.

On opère comme à l'exemple 14 à partir de 212 mg de 4-(3H-imidazo[4,5-c]pyridin-2-yl)-fluorène-9(R,S)-amine, obtenue à l'exemple 6, et de 150 mg de N-Boc-asparagine, en présence de 186 mg d'EDCI et de 131 mg d'HOBT dans 5,2 mL de DMF pendant 20 heures. Après purification par précipitation dans l'eau, puis lavages successifs avec une solution saturée d'hydrogénocarbonate de sodium puis avec de l'éther de pétrole, on obtient 220 mg d'ester de tert-butyle de l'acide {(S)-2-carbamoyl-1-[4-(3H-imidazo[4,5-c]pyridin-2-yl)-9H-fluorèn-9(R,S)-ylcarbamoyl]-éthyl}-carbamoïque, sous forme d'un solide jaune cristallin, dont les caractéristiques sont les suivantes :
Spectre de masse (E/l) : m/z = 512 (M+).

### Exemple 58 : Synthèse du N-[4-(3H-imidazo[4,5-c]pyridin-2-yl)-9H-fluorèn-9(R,S)-yl]-2-uréido-acétamide.

On opère comme à l'exemple 14 à partir de 212 mg de 4-(3H-imidazo[4,5-c]pyridin-2-yl)-fluorène-9(R,S)-amine, obtenue à l'exemple 6, et de 76,4 mg d'acide hydantoïque, en présence de 186 mg d'EDCI et de 131 mg d'HOBT dans 5,2 mL de DMF pendant 20 heures. Après purification par précipitation dans l'eau, puis lavages successifs avec une solution saturée d'hydrogénocarbonate de sodium puis avec de l'éther de pétrole, on obtient 161 mg de N-[4-(3H-imidazo[4,5-c]pyridin-2-yl)-9H-fluorèn-9(R,S)-yl]-2-uréido-acétamide, sous forme d'un solide jaune cristallin, dont les caractéristiques sont les suivantes :
Spectre de masse (E/l) : m/z = 398 (M+).

### Exemple 59 : Synthèse de l'ester de tert-butyle de l'acide {(R)-1-carbamoyl-2-[4-(3H-imidazo[4,5-c]pyridin-2-yl)-9H-fluorèn-9(R,S)-ylcarbamoyl]-éthyl}-carbamoïque.

On opère comme à l'exemple 14 à partir de 297 mg de 4-(3H-imidazo[4,5-c]pyridin-2-yl)-fluorène-9(R,S)-amine, obtenue à l'exemple 6, et de 210 mg de N-Boc-isoasparagine, en présence de 260 mg d'EDCI et de 183 mg d'HOBT dans 7,3 mL de DMF pendant 20 heures. Après purification par précipitation dans l'eau, puis lavages successifs avec une solution saturée d'hydrogénocarbonate de sodium puis avec du méthanol puis avec de l'éther de pétrole, on obtient 176 mg d'ester de tert-butyle de l'acide {(R)-1-carbamoyl-2-[4-(3H-imidazo[4,5-c]pyridin-2-yl)-9H-fluorèn-9(R,S)-ylcarbamoyl]-éthyl}-carbamoïque, sous forme d'un solide jaune-pâle cristallin, dont les caractéristiques sont les suivantes :
Spectre de masse (E/l) : m/z =512 (M+).

### Exemple 60 : Synthèse du [4-(3H-imidazo[4,5-c]pyridin-2-yl)-9H-fluorèn-9(R,S)-yl]-amide de l'acide 1-oxo-indan-4-carboxylique.

On opère comme à l'exemple 14 à partir de 183 mg de 4-(3H-imidazo[4,5-c]pyridin-2-yl)-fluorène-9(R,S)-amine, obtenue à l'exemple 6, et de 100 mg d'acide indanone-4-carboxylique, en présence de 163 mg d'EDCI et de 115 mg d'HOBT dans 4,6 mL de DMF pendant 20 heures. Après purification par flash-chromatographie sur gel de silice (70-230 M), en éluant par un mélange de dichlorométhane et de méthanol (95-5 en volumes), on obtient 84 mg de [4-(3H-imidazo[4,5-c]pyridin-2-yl)-9H-fluorèn-9(R,S)-yl]-amide de l'acide 1-oxo-indan-4-carboxylique, sous forme d'un solide cristallin blanc, dont les caractéristiques sont les suivantes :
Spectre de masse (E/l) : m/z = 456 (M+).

### Exemple 61 : Synthèse de l'ester de tert-butyle de l'acide {(R)-2-carbamoyl-1-[4-(3H-imidazo[4,5-c]pyridin-2-yl)-9H-fluorèn-9(R,S)-ylcarbamoyl]-éthyl}-carbamoïque.

On opère comme à l'exemple 14 à partir de 212,3 mg de 4-(3H-imidazo[4,5-c]pyridin-2-yl)-fluorène-9(R,S)-amine, obtenue à l'exemple 6, et de 150 mg de N-Boc-D-asparagine, en présence de 186 mg d'EDCI et de 131 mg d'HOBT dans 5,2 mL de DMF pendant 20 heures. Après purification par flash-chromatographie sur gel de silice (70-230 M), en éluant par un mélange de dichlorométhane et de méthanol (95-5 en volumes), on obtient 280 mg d'ester de tert-butyle de l'acide {(R)-2-carbamoyl-1-[4-(3H-imidazo[4,5-c]pyridin-2-yl)-9H-fluorèn-9(R,S)-ylcarbamoyl]-éthyl}-carbamoïque, sous forme d'un solide cristallin blanc, dont les caractéristiques sont les suivantes :
Spectre de masse (E/l) : m/z = 512 (M+).

### Exemple 62 : Synthèse du (R)-2-amino-N4-[4-(3H-imidazo[4,5-c]pyridin-2-yl)-9H-fluorèn-9(R,S)-yl]-succinamide.

On dissout 144 mg d'ester de tert-butyle de l'acide {(R)-1-carbamoyl-2-[4-(3H-imidazo[4,5-c]pyridin-2-yl)-9H-fluorèn-9(R,S)-ylcarbamoyl]-éthyl}-carbamoïque, obtenu à l'exemple 59, dans 5 mL de dioxane et on ajoute 1 mL d'une solution 4M d'acide chlorhydrique dans le dioxane. Après 5 heures d'agitation à température ambiante, le solide formé est essoré. Après lavages successifs par une solution aqueuse saturée d'hydrogénocarbonate de sodium, puis par du dichlorométhane, on obtient 145 mg de (R)-2-amino-N4-[4-(3H-imidazo[4,5-c]pyridin-2-yl)-9H-fluorèn-9(R,S)-yl]-succinamide, sous forme d'un solide cristallin vert-pâle, dont les caractéristiques sont les suivantes :
Spectre de masse (E/l): m/z = 412 (M+).

### Exemple 63: Synthèse du chlorhydrate du (S)-2-Amino-N1-[4-(3H-imidazo[4,5-c]pyridin-2-yl)-9H-fluorèn-9(R,S)-yl]-succinamide.

On dissout 200 mg d'ester de tert-butyle de l'acide {(S)-2-carbamoyl-1-[4-(3H-imidazo[4,5-c]pyridin-2-yl)-9H-fluorèn-9(R,S)-ylcarbamoyl]-éthyl}-carbamoïque, obtenu à l'exemple 57, dans 5 mL de dioxane et 4 mL de dichlorométhane, puis on ajoute 0,87 mL d'une solution 4M d'acide chlorhydrique dans le dioxane. Après 15 heures d'agitation à température ambiante, le solide formé est essoré. Après lavage successif par un mélange de dichlorométhane et de méthanol (95-5 en volumes), on obtient 54 mg de chlorhydrate du (S)-2-Amino-N1-[4-(3H-imidazo[4,5-c]pyridin-2-yl)-9H-fluorèn-9(R,S)-yl]-succinamide, sous forme d'un solide cristallin jaune-pâle, dont les caractéristiques sont les suivantes :
Spectre de masse (E/l): m/z = 412 (M+).

### Exemple 64: Synthèse du [4-(3H-imidazo[4,5-c]pyridin-2-yl)-9H-fluorèn-9(R,S)-yl]-amide de l'acide isoquinoléine-1-carboxylique.

On opère comme à l'exemple 14 à partir de 202,5 mg de 4-(3H-imidazo[4,5-c]pyridin-2-yl)-fluorène-9(R,S)-amine, obtenue à l'exemple 6, et de 106,8 mg d'acide isoquinoléine-1-carboxylique, en présence de 177,4 mg d'EDCI et de 125 mg d'HOBT dans 5,9 mL de DMF pendant 20 heures. Après purification par flash-chromatographie sur gel de silice (70-230 M), en éluant par un mélange de dichlorométhane et de méthanol (95-5 en volumes), on obtient 80 mg de [4-(3H-imidazo[4,5-c]pyridin-2-yl)-9H-fluorèn-9(R,S)-yl]-amide de l'acide isoquinoléine-1-carboxylique, sous forme d'un solide cristallin jaune-pâle, dont les caractéristiques sont les suivantes :
Spectre de masse (E/l : m/z = 453 (M+).

### Exemple 65: Synthèse du chlorhydrate du (R)-2-Amino-N1-[4-(3H-imidazo[4,5-c]pyridin-2-yl)-9H-fluorèn-9(R,S)-yl]-succinamide.

On dissout 244 mg d'ester de tert-butyle de l'acide {(R)-2-carbamoyl-1-[4-(3H-imidazo[4,5-c]pyridin-2-yl)-9H-fluorèn-9(R,S)-ylcarbamoyl]-éthyl}-carbamoïque, obtenu à l'exemple 61, dans 5 mL de dichlorométhane, puis on ajoute 1 mL d'une solution 4M d'acide chlorhydrique dans le dioxane. Après 5 heures d'agitation à température ambiante, le solide formé est essoré et lavé à l'eau. On obtient ainsi 160 mg de chlorhydrate du (R)-2-Amino-N1-[4-(3H-imidazo[4,5-c]pyridin-2-yl)-9H-fluorèn-9(R,S)-yl]-succinamide, sous forme d'un solide cristallin jaune-pâle, dont les caractéristiques sont les suivantes :
Spectre de masse (E/l): m/z = 412 (M+).

### Exemple 66 : Synthèse du 2-acétylamino-N-[4-(3H-imidazo[4,5-c]pyridin-2-yl)-9H-fluorèn-9(R,S)-yl]-isonicotinamide.

On opère comme à l'exemple 14 à partir de 212,3 mg de 4-(3H-imidazo[4,5-c]pyridin-2-yl)-fluorène-9(R,S)-amine, obtenue à l'exemple 6, et de 116,6 mg d'acide 2-acétylamino-isonicotinique, en présence de 186 mg d'EDCI et de 131 mg d'HOBT dans 5,2 mL de DMF pendant 20 heures. Après purification par flash-chromatographie sur gel de silice (70-230 M), en éluant par un mélange de dichlorométhane et d'éthanol (95-5 en volumes), on obtient 108 mg de 2-acétylamino-N-[4-(3H-imidazo[4,5-c]pyridin-2-yl)-9H-fluorèn-9(R,S)-yl]-isonicotinamide, sous forme d'un solide jaune-pâle, dont les caractéristiques sont les suivantes :
Spectre de masse (E/I) : m/z = 460 (M+).

Spectre RMN 1H (300 MHz, □ en ppm, DMSO-d6) : 2,12 (s, 3H) ; 6,34 (d, J = 8,5 Hz, 1H) ; 7,25 (t large, J = 7,5 Hz, 1H) ; 7,34 (t large, J = 7,5 Hz, 1H); de 7,48 à 7,59 (m, 4H) ; de 7,62 à 7,76 (m, 3H) ; de 8,37 à 8,44 (m, 2H); 8,55 (s large, 1H) ; 9,04 (s large, 1H) ; 8,43 (d, J = 8,5 Hz, 1H) ; 10,6 (s large, 1H) ; 13,4 (m très étalé, 1H).

### Exemple 67: Synthèse du [4-(3H-imidazo[4,5-c]pyridin-2-yl)-9H-fluorèn-9(R,S)-yl]-amide de l'acide quinoléine-4-carboxylique.

On opère comme à l'exemple 14 à partir de 212,3 mg de 4-(3H-imidazo[4,5-c]pyridin-2-yl)-fluorène-9(R,S)-amine, obtenue à l'exemple 6, et de 112 mg d'acide quinoléine-4-carboxylique, en présence de 186 mg d'EDCI et de 131 mg d'HOBT dans 5,2 mL de DMF pendant 20 heures. Après purification par lavages successifs avec une solution aqueuse saturée d'hydrogénocarbonate de sodium puis avec du dichlorométhane, on obtient 213 mg de [4-(3H-imidazo[4,5-c]pyridin-2-yl)-9H-fluorèn-9(R,S)-yl]-amide de l'acide quinoléine-4-carboxylique, sous forme d'un solide jaune-pâle, dont les caractéristiques sont les suivantes :
Spectre de masse (E/l): m/z = 453 (M+).

### Exemple 68 : Synthèse de la 4-(1H-benzimidazol-2-yl)-fluorèn-9-one oxime (Z,E)

Etape 1 : On opère comme à l'étape 1 de l'exemple 1, à partir de 2,01 g d'o-phenylenediamine dans 5 mL de dichlorométhane, 3,4 mL de triéthylamine et 3 g de chlorure de l'acide flùorèn-4-one-9-carboxylique. Après 2 heures et 30 minutes d'agitation à température ambiante, le milieu réactionnel est versé dans l'eau et extrait au dichlorométhane. Les phases organiques sont lavées avec une solution aqueuse saturée d'hydrogénocarbonate de sodium, séchée sur sulfate de magnésiume et concentrée sous pression réduite. Après purification par empatage dans l'oxyde de diéthyle, on obtient ainsi 3,4 g du (2-amino-phényl)amide de l'acide 9-oxo-9H-fluorène-4-carboxylique, utilisé tel quel à l'étape suivante, dont les caractéristiques sont les suivantes :
Spectre de masse (E/l) : m/z = 314 (M+).

Etape 2 : Dans un tricol de 50 mL sous atmosphère d'argon, on chauffe, à 80°C pendant 2 heures, une solution de 3 g de (2-amino-phényl)amide de l'acide 9-oxo-9H-fluorène-4-carboxilique, obtenu à l'étape précédente, dans 150 mL d'acide acétique. Après refroidissement, le précipité formé est essoré. Après purification par flash-chromatographie sur gel de silice, en éluant par des mélanges de dichlorométhane et d'acétate d'éthyle (90-10 puis 80-20 et enfin 70-30 en volumes), on obtient 1,73 g de 4-(1H-benzimidazol-2-yl)-fluorèn-9-one, sous forme d'une poudre jaune utilisée telle quelle à l'étape suivante, dont les caractéristiques sont les suivantes :
Spectre de masse (E/l) : m/z = 296 (M+).

Etape 3 : On opère comme à l'exemple 5, à partir de 1,7 g de 4-(1H-benzimidazol-2-yl)-fluorèn-9-one, obtenue à l'étape précédente, de 1,196 g de chlorhydrate d'hydroxylamine et de 2,53 g d'acétate de sodium chauffés à 60°C pendant 3 heures dans 30 mL d'éthanol. Après concentration du solvant sous pression réduite, le résidu est repris successivement par de l'eau, puis du toluène et enfin empâté dans de l'oxyde diéthyle, on obtient ainsi 1,62 g de 4-(1H-benzimidazol-2-yl)-fluorèn-9-one oxime, en mélange 50-50 des isomères Z et E, sous forme d'une poudre jaune-pâle, dont les caractéristiques sont les suivantes :
Spectre de masse (E/l) : m/z = 311 (M+).

### Exemple 69 : Synthèse de la 4-(6-fluoro-1H-benzimidazol-2-yl)-fluorèn-9-one

Etape 1 : On opère comme à l'étape 1 de l'exemple 1, à partir de 2,34 g de 4-fluoro-benzène-1,2-diamine dans 5 mL de dichlorométhane, 3,445 mL de triéthylamine et 3 g de chlorure de l'acide fluorèn-4-one-9-carboxylique. Après 1 nuit d'agitation à température ambiante, on obtient ainsi 3,37 g (82%) du mélange équimoléculaire d'amides utilisé tel quel à l'étape suivante.

Etape 2: Dans un tricol de 50 mL sous atmosphère d'argon, on chauffe, à 100°C pendant 4 heures, une solution de 3,37 g du mélange d'amides, obtenu à l'étape précédente, dans 168 mL d'acide acétique. Après refroidissement, le précipité formé est essoré puis trituré à l'oyxde de diéthyle et essoré de nouveau. Après lavage à l'oxyde de diéthyle, puis séchage en étuve sous pression réduite à 35°C on obtient, 2,81 g de 4-(1H-benzimidazol-2-yl)-fluorèn-9-one, sous forme d'une poudre jaune dont les caractéristiques sont les suivantes :
Spectre de masse (E/l) : m/z = 314 (M+).

### Exemple 70 : Synthèse de la 4-(6-fluoro-1H-benzimidazol-2-yl)-fluorèn-9-one oxime (Z,E).

On opère comme à l'exemple 5, à partir de 2,6 g de 4-(1H-benzimidazol-2-yl)-fluorèn-9-one, obtenue à l'exemple 69, de 1,744 g de chlorhydrate d'hydroxylamine et de 3,43 g d'acétate de sodium à température ambiante pendant 20 heures dans 45 mL d'éthanol. Après concentration du solvant sous pression réduite, le résidu est repris successivement par de l'eau, puis du toluène et enfin empâté dans de l'oxyde diéthyle, on obtient ainsi 1,94 g de 4-(6-fluoro-1H-benzimidazol-2-yl)-fluorèn-9-one oxime, en mélange 40-60 des isomères Z et E, sous forme d'une poudre jaune-pâle, dont les caractéristiques sont les suivantes :
Spectre de masse (E/l) : m/z = 329 (M+).

### Exemple 71 : Synthèse de la 4-(6-fluoro-1H-benzimidazol-2-yl)-9H-fluorèn-9(R,S)-ylamine.

On opère comme à l'exemple 6, à partir de 1,7 g de 4-(6-fluoro-1H-benzimidazol-2-yl)-fluorèn-9-one oxime(Z,E), obtenue à l'exemple 70, et de 60 mg de Nickel de Raney dans 90 mL d'éthanol et 90 mL de tetrahydrofurane pendant 4 heures à 60°C sous une pression d'un bar d'hydrogène. Après purification par empâtage dans l'oxyde de diisopropyle, on obtient 1,58 g de 4-(6-fluoro-1H-benzimidazol-2-yl)-9H-fluorèn-9(R,S)-ylamine, sous forme d'une poudre beige dont les caractéristiques sont les suivantes :
Spectre de masse (E/l) : m/z = 315 (M+).
Point de fusion (Kofler) = 170-72°C.

### Exemples 72 et 73: Synthèse du N-[4-(3H-imidazo[4,5-c]pyridin-2-yl)-9H-fluorèn-9(R,S)-yl]-acétamide et du 2-amino-N-[4-(3H-imidazo[4,5-c]pyridin-2-yl)-9H-fluorèn-9(RS-yl]-isonicotinamide.

On opère comme à l'exemple 14 à partir de 300 mg de 4-(3H-imidazo[4,5-c]pyridin-2-yl)-fluorène-9(R,S)-amine, obtenue à l'exemple 6, et de 153,3 mg d'acide 2-amino-isonicotinique, en présence de 319 mg d'EDCI et de 225 mg d'HOBT dans 17 mL de DMF pendant 20 heures. Après purification par flash-chromatographie sur gel de silice (70-230 Mesh), en éluant par un mélange de dichlorométhane et de méthanol (90-10 en volumes), on obtient :
a) en récupérant la première fraction chromatographiée, 22 mg de N-[4-(3H-imidazo[4,5-c]pyridin-2-yl)-9H-fluorèn-9(R,S)-yl]-acétamide, sous forme d'une meringue blanche, dont les caractéristiques sont les suivantes :
   Spectre de masse (E/I): m/z = 340 (M+).
b) en récupérant la seconde fraction chromatographiée, 30 mg de 2-amino-N-[4-(3H-imidazo[4,5-c]pyridin-2-yl)-9H-fluorèn-9(RS-yl]-isonicotinamide, sous forme d'une meringue blanche, dont les caractéristiques sont les suivantes :
   Spectre de masse (E/I) : m/z = 418 (M+).

Spectre RMN 1H (300 MHz, □ en ppm, DMSO-d6) : 6,11 (s large, 2H) ; 6,31 (d, J = 8,5 Hz, 1H) ; de 6,90 à 6,95 (m, 2H) ; 7,24 (t large, J = 7,5 Hz, 1H) ; 7,33 (t large , J = 7,5 Hz, 1H) ; de 7,45 à 7,57 (m, 3H) ; 7,62 (m très étalé, 1H) ; de 7,66 à 7,72 (m, 2H) ; 7,99 (d, J = 5,5 Hz, 1H) ; 8,40 (d, J = 5,5 Hz, 1H) ; de 8,93 à 9,10 (m étalé, 1H) ; 9,19 (d, J = 8,5 Hz, 1H) ; de 13,2 à 13,5 (m très étalé, 1H).

### Exemple 74 : Synthèse de la 1-furan-2-ylméthyl-3-[4-(3H-imidazo[4,5-c]pyridin-2-yl)-9H-fluorèn-9(R,S)-yl]-urée

On opère comme à l'exemple 77, à partir de 75 mg de 4-(3H-imidazo[4,5-c]pyridin-2-yl)-fluorène-9(R,S)-amine, obtenue à l'exemple 6, et 29 µL d'isocyanate de furfuryle dans 1,25 mL de tetrahydrofurane contenant 41 µL de triéthylamine pendant 3 heures à température ambiante. Le résidu obtenu est repris avec 5 mL de dichlorométhane, puis essoré et lavé au dichlorométhane. On obtient ainsi 90 mg 1-furan-2-ylméthyl-3-[4-(3H-imidazo[4,5-c]pyridin-2-yl)-9H-fluorèn-9(R,S)-yl]-urée, sous forme d'un solide blanc dont les caractéristiques sont les suivantes :
Spectre de masse (E/I) : m/z = 421 (M+).

### Exemple 75 : Synthèse de la 1-(4-fluoro-benzyl)-3-[4-(3H-imidazo[4,5-c]pyridin-2-yl)-9H-fluorèn-9(R,S)-yl]-urée.

On opère comme à l'exemple 77, à partir de 75 mg de 4-(3H-imidazo[4,5-c]pyridin-2-yl)-fluorène-9(R,S)-amine, obtenue à l'exemple 6, et 33,6 µL d'isocyanate de 4-fluorobenzyle dans 1,25 mL de tetrahydrofurane contenant 41 µL de triéthylamine pendant 3 heures à température ambiante. Le résidu obtenu est repris avec 5 mL de dichlorométhane, puis essoré et lavé au dichlorométhane. On obtient ainsi 96 mg 1-(4-fluoro-benzyl)-3-[4-(3H-imidazo[4,5-c]pyridin-2-yl)-9H-fluorèn-9(R,S)-yl]-urée, sous forme d'un solide jaune-pâle dont les caractéristiques sont les suivantes :
Spectre de masse (E/I) : m/z = 449 (M+).

### Exemple 76 : Synthèse de la 1-(3,5-diméthyl-isoxazol-4-yl)-3-[4-(3H-imidazo[4,5-c]pyridin-2-yl)-9H-fluorèn-9(R,S)-yl]-urée

On opère comme à l'exemple 77, à partir de 75 mg de 4-(3H-imidazo[4,5-c]pyridin-2-yl)-fluorène-9(R,S)-amine, obtenue à l'exemple 6, et 36,5 mg d'isocyanate de 3,5-diméthyl-isoxazol-4-yle dans 1,25 mL de tetrahydrofurane contenant 41 µl de triéthylamine pendant 3 heures à température ambiante. Le résidu obtenu est repris avec 5 mL de dichlorométhane, puis essoré et lavé au dichlorométhane. On obtient ainsi 95 mg de 1-(3,5-diméthyl-isoxazol-4-yl)-3-[4-(3H-imidazo[4,5-c]pyridin-2-yl)-9H-fluorèn-9(R,S)-yl]-urée, sous forme d'un solide blanc dont les caractéristiques sont les suivantes :
Spectre de masse (E/I) : m/z = 436 (M+).

### Exemple 77 : Synthèse de l'ester d'éthyle de l'acide 3-{3-[4-(3H-imidazo[4,5-c]pyridin-2-yl)-9H-fluorèn-9(R,S)-yl]-uréido}-propionique

A une solution de 75 mg de 4-(3H-imidazo[4,5-c]pyridin-2-yl)-fluorène-9(R,S)-amine, obtenue à l'exemple 6, dans 1,25 mL de tetrahydrofurane contenant 41 µL de triéthylamine, on ajoute 35 µL de 3-isocyanato-propionate d'éthyle. La suspension blanche obtenue est agitée 3 heures à température ambiante, puis concentrée sous pression réduite. Le résidu obtenu est repris avec 5 mL de dichlorométhane, puis essoré et lavé au dichlorométhane. On obtient ainsi 80 mg d'ester d'éthyle de l'acide 3-{3-[4-(3H-imidazo[4,5-c]pyridin-2-yl)-9H-fluorèn-9(R,S)-yl]-uréido}-propionique, sous forme d'un solide blanc dont les caractéristiques sont les suivantes :
Spectre de masse (E/I) : m/z = 441 (M+).

### Exemple 78 : Synthèse de l'ester d'éthyle de l'acide {3-[4-(3H-midazo[4,5-c]pyridin-2-yl)-9H-fluorèn-9(R,S)-yl]-uréido}-acétique

On opère comme à l'exemple 77, à partir de 75 mg de 4-(3H-imidazo[4,5-c]pyridin-2-yl)-fluorène-9(R,S)-amine, obtenue à l'exemple 6, et 33 µL d'isocyanatoacétate d'éthyle. On obtient ainsi 81 mg d'ester d'éthyle de l'acide {3-[4-(3H-midazo[4,5-c]pyridin-2-yl)-9H-fluorèn-9(R,S)-yl]-uréido}-acétique, sous forme d'un solide blanc dont les caractéristiques sont les suivantes :
Spectre de masse (E/I) : m/z = 427 (M+).

### Exemple 79 : Synthèse du trifluoroacétate du 3-{3-[4-(3H-imidazo[4,5-c]pyridin-2-yl)-9H-fluorèn-9(R,S)-yl]-uréido)-propionamide.

Etape 1: On opère comme à l'exemple 77, à partir de 75 mg de 4-(3H-imidazo[4,5-c]pyridin-2-yl)-fluorène-9(R,S)-amine, obtenue à l'exemple 6, et 41 µL de 3-isocyanatopropionate d'éthyle. On obtient ainsi 80 mg d'ester d'éthyle de l'acide 3-{3-[4-(3H-midazo[4,5-c]pyridin-2-yl)-9H-ftuorèn-9(R,S)-yl]-uréido}-propionique, sous forme d'un solide blanc dont les caractéristiques sont les suivantes :
Spectre de masse (E/I) : m/z = 441 (M+).

Etape 2 : Une solution de 15 mg d'ester d'éthyle de l'acide 3-{3-[4-(3H-midazo[4,5-c]pyridin-2-yl)-9H-fluorèn-9(R,S)-yl]-uréido}-propionique, obtenu à l'étape précédente, dans 2 mL d'ammoniac 7N dans le méthanol est chauffée en tube scellé dans un réacteur micro-ondes pendant 20 minutes à 120°C puis 35 minutes à 130°C. Après concentration, puis purification par HPLC/MS sur une colonne de silice Symmetry C18 (5 µm), en éluant par un gradient eau 100% (contenant 0,07% de TFA) à acétonitrile 100% (contenant 0,07% de TFA), on obtient 3,5 mg de trifluoroacétate de 3-{3-[4-(3H-imidazo[4,5-c]pyridin-2-yl)-9H-fluorèn-9(R,S)-yl]-uréido}-propionamide, sous forme d'un solide blanc dont les caractéristiques sont les suivantes :
Spectre de masse (E/I) : m/z = 412 (M+).

### Exemple 80 : Synthèse de la 3-[4-(3H-imidazo[4,5-c]pyridin-2-yl)-9H-fluorèn-9(R,S)-yl]-imidazolidine-2,4-dione.

Dans un appareil micro-ondes (EmrgsTM Personal Chemistry), on chauffe, pendant 15 minutes à 120°C, 15 mg d'ester d'éthyle de l'acide {3-[4-(3H-midazo[4,5-c]pyridin-2-yl)-9H-fluorèn-9(R,S)-yl]-uréido}-acétique, obtenu à l'exemple 78, dans 2 mL d'une solution 7M d'ammoniac dans le méthanol. Après concentration sous pression réduite, le résidu est repris dans le minimum d'oxyde de diéthyle et essoré. On obtient ainsi 13 mg de 3-[4-(3H-imidazo[4,5-c]pyridin-2-yl)-9H-fluorèn-9(R,S)-yl]-imidazolidine-2,4-dione, sous forme d'un solide jaune pâle dont les caractéristiques sont les suivantes :
Spectre de masse (E/I) : m/z = 381 (M+).
Spectre RMN 1H (400 MHz, □en ppm, DMSO-d6) : de 3,80 à 4,20 (m très étalé, 2H) ; de 6,02 à 6,23 (m étalé, 1H); de 7,17 à 7,32 (m, 2H); de 7,32 à 7,49 (m, 2H) ; de 7,53 à 7,62 (m, 2H); de 7,67 à 7,78 (m, 2H) ; de 7,80 à 8,61 (m très étalé, 1H); 8,28 (d large, J = 5,5 Hz, 1H) ; 8,96 (s, 1H) ; de 12,5 à 14,0 (m très étalé, 1H).

### Exemple 81: Synthèse du N-[4-(3H-imidazo[4,5-c]pyridin-2-yl)-9H-fluorèn-9(R,S)-yl]-1-oxy-isonicotinamide.

On opère comme à l'exemple 14 à partir de 298 mg de 4-(3H-imidazo[4,5-c]pyridin-2-yl)-fluorène-9(R,S)-amine, obtenue à l'exemple 6, et de 139 mg du N-oxyde de l'acide isonicotinique, en présence de 211 mg d'EDCI et de 148,6 mg d'HOBT dans 7,2 mL de DMF pendant 20 heures. Après purification par lavages successifs avec une solution aqueuse saturée d'hydrogénocarbonate de sodium puis avec de l'eau et de l'isopropanol, on obtient 45 mg de N-[4-(3H-imidazo[4,5-c]pyridin-2-yl)-9H-fluorèn-9(R,S)-yl]-1-oxy-isonicotinamide, sous forme d'un solide beige, dont les caractéristiques sont les suivantes :
Spectre de masse (E/I) : m/z = 419 (M+).

### Exemple 82: Synthèse du 2-éthyl-N-[4-(3H-imidazo[4,5-c]pyridin-2-yl)-9H-fluorèn-9(R,S)-yl]-isonicotinamide.

On opère comme à l'exemple 14 à partir de 298 mg de 4-(3H-imidazo[4,5-c]pyridin-2-yl)-fluorène-9(R,S)-amine, obtenue à l'exemple 6, et de 151,2 mg de l'acide 2-éthyl-isonicotinique, qui peut être obtenu selon J. Biol. Chem. 2002, 277, 12824-29, en présence de 211 mg d'EDCI et de 148,6 mg d'HOBT dans 7,2 mL de DMF pendant 20 heures. Après purification par lavages successifs avec une solution aqueuse saturée d'hydrogénocarbonate de sodium puis avec de l'eau et de l'isopropanol, on obtient 310 mg de 2-éthyl-N-[4-(3H-imidazo[4,5-c]pyridin-2-yl)-9H-fluorèn-9(R,S)-yl]-isonicotinamide, sous forme d'un solide beige, dont les caractéristiques sont les suivantes :
Spectre de masse (E/I) : m/z = 431 (M+).

### Exemple 83 : Synthèse du [4-(1H-benzimidazol-2-yl)-9H-fluorèn-9(R,S)-yl]-amide de l'acide quinoléine-5-carboxylique.

Etape 1 : On opère comme à l'exemple 6, à partir de 1,57 g de 4-(1H-benzimidazol-2-yl)-fluorèn-9-one oxime(Z,E), obtenue à l'exemple 68, et de 56 mg de Nickel de Raney dans 80 mL d'éthanol et 80 mL de tetrahydrofurane pendant 4 heures à 60°C sous une pression d'un bar d'hydrogène. Après purification par empâtage dans l'oxyde de diisopropyle, on obtient 1,31 g de 4-(1H-benzimidazol-2-yl)-9H-fluorèn-9(R,S)-ylamine, sous forme d'une poudre blanche, dont les caractéristiques sont les suivantes :
Spectre de masse (E/I) : m/z = 297 (M+).

Etape 2 : On opère comme à l'exemple 14 à partir de 200 mg de 4-(1H-benzimidazol-2-yl)-9H-fluorèn-9(R,S)-ylamine, obtenue à l'étape précedente, et de 120 mg d'acide quinoléine-5-carboxylique, en présence de 193 mg d'EDCI et de 135 mg d'HOBT dans 2 mL de DMF pendant 20 heures. Après purification par flash-chromatographie sur gel de silice, en éluant avec un mélange de dichlorométhane et de méthanol (95-5 en volumes), on obtient 125 mg de [4-(1H-benzimidazol-2-yl)-9H-fluorèn-9(R,S)-yl]-amide de l'acide quinoléine-5-carboxylique, sous forme d'une poudre jaune-pâle, dont les caractéristiques sont les suivantes :
Spectre de masse (E/I) : m/z = 493 (M+).
Spectre RMN 1H (300 MHz, □ en ppm, DMSO-d6) : 6,43 (d, J = 8,5 Hz, 1H); de 7,21 à 7,33 (m, 3H); 7,37 (t large, J = 7,5 Hz, 1H) ; de 7,52 à 7,61 (m, 3H) ; de 7,65 à 7,90 (m, 7H) ; 8,14 (d large, J = 8,5 Hz, 1H) ; 8,87 (d large, J = 8,5 Hz, 1H) ; 8,99 (dd, J = 2,0 et 4,0 Hz, 1H) ; 9,37 (d, J = 8,5 Hz, 1H) ; 12,9 (s large, 1H).

### Exemple 84 : Synthèse du [4-(6-fluoro-1H-benzimidazol-2-yl)-9H-fluorèn-9(R,S)-yl]-amide de l'acide quinoléine-5-carboxylique.

On opère comme à l'exemple 14 à partir de 200 mg de 4-(6-fluoro-1H-benzimidazol-2-yl)-9H-fluorèn-9(R,S)-ylamine, obtenue à l'exemple 71, et de 113 mg d'acide quinoléine-5-carboxylique, en présence de 182,4 mg d'EDCI et de 128,5 mg d'HOBT dans 2 mL de DMF pendant 20 heures. Après purification par flash-chromatographie sur gel de silice, en éluant avec un mélange de dichlorométhane et de méthanol (95-5 en volumes), on obtient 134 mg de [4-(6-fluoro-1H-benzimidazol-2-yl)-9H-fluorèn-9(R,S)-yl]-amide de l'acide quinoléine-5-carboxylique, sous forme d'une poudre jaune-pâle, dont les caractéristiques sont les suivantes:
Spectre de masse (E/I) : m/z = 470 (M+).
Spectre RMN 1H (400 MHz, □ en ppm, DMSO-d6) : 6,43 (d, J = 8,5 Hz, 1H); 7,14 (t large, J = 10,0 Hz, 1H) ; 7,26 (t large, J = 7,5 Hz, 1H) ; 7,38 (t large, J = 7,5 Hz, 1H); 7,55 (t, J = 7,5 Hz, 2H) ; de 7,35 à 7,61 (m très étalé, 2H) ; de 7,65 à 7,70 (m, 2H) ; 7,73 (d large, J = 7,5 Hz, 1H) ; 7,80 (m, 1H) ; de 7,86 à 7,90 (m, 2H) ; 8,14 (d large, J = 8,5 Hz, 1H); 8,86 (d large, J = 8,5 Hz, 1H) ; 8,99 (dd, J = 2,0 et 4,0 Hz, 1H) ; 9,38 (d, J = 8,5 Hz, 1H) ; 13,1 (s large, 1H).

### Exemple 85 : Synthèse du [4-(3H-imidazo[4,5-c]pyridin-2-yl)-9H-fluoren-9-yl]-amide de l'acide 2-cyclopropyl-quinoléine-4-carboxylique.

On opère comme à l'exemple 14 à partir de 364 mg de 4-(3H-imidazo[4,5-c]pyridin-2-yl)-fluorène-9(R,S)-amine, obtenue à l'exemple 6, et de 286 mg d'acide 2-cyclopropyl-quinoléine-4-carboxylique, en présence de 234 mg d'EDCI et de 165 mg d'HOBT dans 10 mL de DMF pendant 20 heures. Après purification par lavages successifs avec une solution aqueuse saturée d'hydrogénocarbonate de sodium puis avec de l'eau et de l'éther de diisopropyle, on obtient 344 mg de [4-(3H-imidazo[4,5-c]pyridin-2-yl)-9H-fluoren-9-yl]-amide de l'acide 2-cyclopropyl-quinoléine-4-carboxylique, sous forme d'une poudre beige, dont les caractéristiques sont les suivantes :
Spectre de masse (E/I) : m/z = 493 (M+).

### Exemple 86 : Synthèse du [4-(3H-imidazo[4,5-c]pyridin-2-yl)-9H-fluoren-9-yl]-amide de l'acide pyridazine-4-carboxylique

On opère comme à l'exemple 14 à partir de 298 mg de 4-(3H-imidazo[4,5-c]pyridin-2-yl)-fluorène-9(R,S)-amine, obtenue à l'exemple 6, et de 124 mg d'acide pyridazine-4-carboxylique, en présence de 211 mg d'EDCI et de 148,6 mg d'HOBT dans 7,2 mL de DMF pendant 20 heures. Après purification par lavages successifs avec une solution aqueuse saturée d'hydrogénocarbonate de sodium puis avec de l'eau et de l'isopropanol, on obtient 210 mg de [4-(3H-imidazo[4,5-c]pyridin-2-yl)-9H-fluoren-9-yl]-amide de l'acide pyridazine-4-carboxylique, sous forme d'une poudre brune, dont les caractéristiques sont les suivantes :
Spectre de masse (E/I) : m/z = 404 (M+).

### Exemple 87 : Synthèse du 2-chloro-N-[4-(3H-imidazo[4,5-c]pyridin-2-yl)-9H-fluorèn-9(R,S)-yl]-isonicotinamide.

On opère comme à l'exemple 14 à partir de 298 mg de 4-(3H-imidazo[4,5-c]pyridin-2-yl)-fluorène-9(R,S)-amine, obtenue à l'exemple 6, et de 157,6 mg d'acide 2-chloro-isonicotinique, en présence de 211 mg d'EDCI et de 148,6 mg d'HOBT dans 7,2 mL de DMF pendant 20 heures. Après purification par lavages successifs avec une solution aqueuse saturée d'hydrogénocarbonate de sodium puis avec de l'eau et de l'isopropanol, on obtient 405 mg de 2-chloro-N-[4-(3H-imidazo[4,5-c]pyridin-2-yl)-9H-fluorèn-9(R,S)-yl]-isonicotinamide, sous forme d'une poudre brune, dont les caractéristiques sont les suivantes :
Spectre de masse (E/I) : m/z = 437 (M+).

### Exemple 88 : Synthèse du [4-(3H-imidazo[4,5-c]pyridin-2-yl)-9H-fluorèn-9-yl]-amide de l'acide 2,6-dihydroxy-pyrimidine-4-carboxylique.

On opère comme à l'exemple 14 à partir de 298 mg de 4-(3H-imidazo[4,5-c]pyridin-2-yl)-fluorène-9(R,S)-amine, obtenue à l'exemple 6, et de 156 mg d'acide 2,6-dihydroxy-pyrimidine-4-carboxylique, en présence de 211 mg d'EDCI et de 148,6 mg d'HOBT dans 7,2 mL de DMF pendant 20 heures. Après purification par lavages successifs avec une solution aqueuse saturée d'hydrogénocarbonate de sodium puis avec de l'eau et de l'isopropanol, on obtient 365 mg de [4-(3H-imidazo[4,5-c]pyridin-2-yi)-9H-fluorèn-9-yl]-amide de l'acide 2,6-dihydroxy-pyrimidine-4-carboxylique, sous forme d'une poudre brune, dont les caractéristiques sont les suivantes :
Spectre de masse (E/I) : m/z = 436 (M+).

### Exemple 89 : Synthèse du [4-(3H-imidazo[4,5-c]pyridin-2-yl)-9H-fluorèn-9-yl]-amide de l'acide 2-hydroxy-pyrimidine-4-carboxylique.

On opère comme à l'exemple 14 à partir de 298 mg de 4-(3H-imidazo[4,5-c]pyridin-2-yl)-fluorène-9(R,S)-amine, obtenue à l'exemple 6, et de 140 mg d'acide 2-hydroxy-pyrimidine-4-carboxylique, en présence de 211 mg d'EDCI et de 148,6 mg d'HOBT dans 7,2 mL de DMF pendant 20 heures. Après purification par lavages successifs avec une solution aqueuse saturée d'hydrogénocarbonate de sodium puis avec de l'eau et de l'isopropanol, on obtient 195 mg de [4-(3H-imidazo[4,5-c]pyridin-2-yl)-9H-fluorèn-9-yl]-amide de l'acide 2-hydroxy-pyrimidine-4-carboxylique, sous forme d'une poudre brune, dont les caractéristiques sont les suivantes :
Spectre de masse (E/I) : m/z = 420 (M+).

### Exemple 90 : Synthèse du [4-(3H-imidazo[4,5-c]pyridin-2-yl)-9H-fluorèn-9-yl]-amide de l'acide 2-méthyl-quinoléine-5-carboxylique.

On opère comme à l'exemple 14 à partir de 200 mg de 4-(3H-imidazo[4,5-c]pyridin-2-yl)-fluorène-9(R,S)-amine, obtenue à l'exemple 6, et de 138 mg d'acide 2-méthyl-quinoléine-5-carboxylique, qui peut être obtenu selon J. Med. Chem. 2002, 4647, en présence de 128,4 mg d'EDCI et de 90,5 mg d'HOBT dans 5,5 mL de DMF pendant 20 heures. Après purification par lavages successifs avec une solution aqueuse saturée d'hydrogénocarbonate de sodium puis avec de l'eau et de l'oxyde de diisopropyle, on obtient 70 mg de [4-(3H-imidazo[4,5-c]pyridin-2-yl)-9H-fluorèn-9-yl]-amide de l'acide 2-méthyl-quinoléine-5-carboxylique, sous forme d'une poudre jaune-pâle, dont les caractéristiques sont les suivantes :
Spectre de masse (E/I) : m/z = 467 (M+).

### Exemple 91 : Synthèse du [4-(3H-imidazo[4,5-c]pyridin-2-yl)-9H-fluoren-9-yl]-amide de l'acide 2-amino-pyrimidine-4-carboxylique.

On opère comme à l'exemple 14 à partir de 214,5 mg de 4-(3H-imidazo[4,5-c]pyridin-2-yl)-fluorène-9(R,S)-amine, obtenue à l'exemple 6, et 100 mg d'acide 2-amino-pyrimidine-4.-carboxylique, en présence de 151,6 mg d'EDCI et de 107 mg d'HOBT dans 5 mL de DMF pendant 20 heures. Après purification par lavages successifs avec une solution aqueuse saturée d'hydrogénocarbonate de sodium puis avec de l'eau et de l'isopropanol, on obtient 190 mg de [4-(3H-imidazo[4,5-c]pyridin-2-yl)-9H-fluorèn-9-yl]-amide de l'acide 2-amino-pyrimidine-4-carboxylique, sous forme d'une poudre beige, dont les caractéristiques sont les suivantes :
Spectre de masse (E/I) : m/z = 419 (M+).

### Exemple 92 : Synthèse du [4-(3H-imidazo[4,5-c]pyridin-2-yl)-9H-fluorèn-9(R,S)-yl]-amide de l'acide 1H-pyrrolo[2,3-b]pyridine-4-carboxylique.

On opère comme à l'exemple 14 à partir de 300 mg de 4-(3H-imidazo[4,5-c]pyridin-2-yl)-fluorène-9(R,S)-amine, obtenue à l'exemple 6, et de 171 mg d'acide 1H-pyrrolo[2,3-b]pyridine-4-carboxylique, qui peut être obtenu selon WO 2003/000688, en présence de 212 mg d'EDCI et de 150 mg d'HOBT dans 10 mL de DMF pendant 20 heures. Après purification par lavages successifs avec une solution aqueuse saturée d'hydrogénocarbonate de sodium puis avec de l'eau et de l'isopropanol, on obtient 245 mg de [4-(3H-imidazo[4,5-c]pyridin-2-yl)-9H-fluorèn-9(R,S)-yl]-amide de l'acide 1H-pyrrolo[2,3-b]pyridine-4-carboxylique, sous forme d'une poudre blanc cassé, dont les caractéristiques sont les suivantes :
Spectre de masse (E/I) : m/z = 442 (M+).
Point de fusion (Kofler) = 236°C.

### Exemple 93 : Synthèse du [4-(3H-imidazo[4,5-c]pyridin-2-yl)-9H-fluorèn-9-yl]-amide de l'acide 6-hydroxy-pyrimidine-4-carboxylique

On opère comme à l'exemple 14 à partir de 106,55 mg de 4-(3H-imidazo[4,5-c]pyridin-2-yl)-fluorène-9(R,S)-amine, obtenue à l'exemple 6, et de 50 mg d'acide 6-hydroxy-pyrimidine-4-carboxylique, en présence de 75,3 mg d'EDCI et de 53 mg d'HOBT dans 2,5 mL de DMF pendant 20 heures. Après purification par lavages successifs avec une solution aqueuse saturée d'hydrogénocarbonate de sodium puis avec de l'eau et de l'isopropanol, on obtient 65 mg de [4-(3H-imidazo[4,5-c]pyridin-2-yl)-9H-fluorèn-9-yl]-amide de l'acide 6-hydroxy-pyrimidine-4-carboxylique, sous forme d'une poudre brune, dont les caractéristiques sont les suivantes :
Spectre de masse (E/I) : m/z = 420 (M+).

L'acide 6-hydroxy-pyrimidine-4-carboxylique peut être obtenu par hydrolyse de l'ester de méthyle de l'acide 6-hydroxy-pyrimidine-4-carboxylique, qui peut être obtenu selon J. Med. Chem. 2000, 43, 3995-4002.

### Exemple 94 : Synthèse du 2-hydroxyméthyl-N-[4-(3H-imidazo[4,5-c]pyridin-2-yl)-9H-fluorèn-9(R,S)-yl]-isonicotinamide.

On opère comme à l'exemple 14 à partir de 97,4 mg de 4-(3H-imidazo[4,5-c]pyridin-2-yl)-fluorène-9(R,S)-amine, obtenue à l'exemple 6, et de 50 mg d'acide 2-hydroxyméthyl-isonicotinique, en présence de 68,8 mg d'EDCI et de 48,5 mg d'HOBT dans 2,5 mL de DMF pendant 20 heures. Après purification par lavages successifs avec une solution aqueuse saturée d'hydrogénocarbonate de sodium puis avec de l'eau et de l'isopropanol, on obtient 70 mg de 2-hydroxyméthyl-N-[4-(3H-imidazo[4,5-c]pyridin-2-yl)-9H-fluorèn-9(R,S)-yl]-isonicotinamide, sous forme d'une poudre beige, dont les caractéristiques sont les suivantes :
Spectre de masse (E/I) : m/z = 433 (M+).

L'acide 2-hydroxyméthyl-isonicotinique peut être obtenu par saponification de l'ester de méthyle de l'acide 2-hydroxyméthyl-isonicotinique, qui peut être obtenu selon E.P. 533131.

### Exemple 95 : Synthèse du 2-fluoro-N-[4-(3H-imidazo[4,5-c]pyridin-2-yl)-9H-fluorèn-9(R,S)-yl]-isonicotinamide.

On opère comme à l'exemple 14 à partir de 298,4 mg de 4-(3H-imidazo[4,5-c]pyridin-2-yl)-fluorène-9(R,S)-amine, obtenue à l'exemple 6, et de 141 mg d'acide 2-fluoro-isonicotinique, en présence de 211 mg d'EDCI et de 148,65 mg d'HOBT dans 7 mL de DMF pendant 20 heures. Après purification par lavages successifs avec une solution aqueuse saturée d'hydrogénocarbonate de sodium puis avec de l'eau et de l'isopropanol, on obtient 290 mg de 2-fluoro-N-[4-(3H-imidazo[4,5-c]pyridin-2-yl)-9H-fluorèn-9(R,S)-yl]-isonicotinamide, sous forme d'une poudre beige, dont les caractéristiques sont les suivantes :
Spectre de masse (E/I) : m/z = 421 (M+).

### Exemple 96 : Synthèse du N-[4-(3H-imidazo[4,5-c]pyridin-2-yl)-9H-fluorèn-9(R,S)-yl]-3-méthyl-isonicotinamide.

On opère comme à l'exemple 14 à partir de 298,4 mg de 4-(3H-imidazo[4,5-c]pyridin-2-yl)-fluorène-9(R,S)-amine, obtenue à l'exemple 6, et de 137 mg d'acide 3-méthyl-isonicotinique, en présence de 211 mg d'EDCI et de 148,6 mg d'HOBT dans 7 mL de DMF pendant 20 heures. Après purification par lavages successifs avec une solution aqueuse saturée d'hydrogénocarbonate de sodium puis avec de l'eau et de l'isopropanol, on obtient 290 mg de N-[4-(3H-imidazo[4,5-c]pyridin-2-yl)-9H-fluorèn-9(R,S)-yl]-3-méthyl-isonicotinamide, sous forme d'une poudre beige, dont les caractéristiques sont les suivantes :
Spectre de masse (E/I) : m/z = 417 (M+).

### Exemple 97 : Synthèse du chlorhydrate du [4-(3H-imidazo[4,5-c]pyridin-2-yl)-9H-fluorèn(R,S)-9-yl]-amide de l'acide 1,8-naphtyridine-4-carboxylique.

On opère comme à l'exemple 14 à partir de 298,4 mg de 4-(3H-imidazo[4,5-c]pyridin-2-yl)-fluorène-9(R,S)-amine, obtenue à l'exemple 6, et de 174,2 mg de chlorhydrate de l'acide 1,8-naphtyridine-4-carboxylique, en présence de 211 mg d'EDCI et de 148,6 mg d'HOBT dans 7 mL de DMF pendant 20 heures. Après purification par lavages successifs avec une solution aqueuse saturée d'hydrogénocarbonate de sodium puis avec de l'eau et de l'isopropanol, on obtient 435 mg de chlorhydrate du [4-(3H-imidazo[4,5-c]pyridin-2-yl)-9H-fluorèn-9(R,S)-yl]-amide de l'acide 1,8-naphtyridine-4-carboxylique, sous forme d'une poudre brun-clair, dont les caractéristiques sont les suivantes :
Spectre de masse (E/I) : m/z = 4547 (M+).

### Exemple 98 : Synthèse du 3-bromo-N-[4-(3H-imidazo[4,5-c]pyridin-2-yl)-9H-fluorèn-9(R,S)-yl]-isonicotinamide.

On opère comme à l'exemple 14 à partir de 298,4 mg de 4-(3H-imidazo[4,5-c]pyridin-2-yl)-fluorène-9(R,S)-amine, obtenue à l'exemple 6, et de 202 mg d'acide 3-bromo-isonicotinique, en présence de 211 mg d'EDCI et de 148,6 mg d'HOBT dans 7 mL de DMF pendant 20 heures. Après purification par lavages successifs avec une solution aqueuse saturée d'hydrogénocarbonate de sodium puis avec de l'eau et de l'isopropanol, on obtient 435 mg de 3-bromo-N-[4-(3H-imidazo[4,5-c]pyridin-2-yl)-9H-fluorèn-9(R,S)-yl]-isonicotinamide, sous forme d'une poudre brune, dont les caractéristiques sont les suivantes :
Spectre de masse (E/I) : m/z = 482 (M+).

### Exemple 99 : Synthèse du [4-(3H-imidazo[4,5-c]pyridin-2-yl)-9H-fluorèn(R,S)-9-yl]-amide de l'acide 1H-pyrrolo[2,3-b]pyridine-3-carboxylique.

On opère comme à l'exemple 14 à partir de 298 mg 4-(3H-imidazo[4,5-c]pyridin-2-yl)-fluorène-9(R,S)-amine, obtenue à l'exemple 6, et de 170 mg d'acide 1H-pyrrolo[2,3-b]pyridine-3-carboxylique, en présence de 287 mg d'EDCI et de 202 mg d'HOBT dans 5 mL de DMF pendant 20 heures. Après purification par flash-chromatographie sur gel de silice, en éluant avec un mélange de dichlorométhane et de méthanol (90-10 en volumes), on obtient 155 mg de [4-(3H-imidazo[4,5-c]pyridin-2-yl)-9H-fluorèn(R,S)-9-yl]-amide de l'acide 1H-pyrrolo[2,3-b]pyridine-3-carboxylique, sous forme d'une poudre blanche, dont les caractéristiques sont les suivantes :
Spectre de masse (E/I) : m/z = 442 (M+).
Point de fusion (Kofler) = 225°C (décomp.).

L'acide 1H-pyrrolo[2,3-b]pyridine-3-carboxylique peut être préparé selon WO 04111048.

### Exemple 100 : Synthèse du N-[4-(3H-Imidazo[4,5-c]pyridin-2-yl)-9H-fluoren-9(R,S)-yl]-2(R,S)-(pyridin-3-yl)-propionamide.

On opère comme à l'exemple 14 à partir de 200 mg de 4-(3H-imidazo[4,5-c]pyridin-2-yl)-fluorène-9(R,S)-amine, obtenue à l'exemple 6, et de 138,3 mg de chlorhydrate de l'acide 2-(pyridin-3-yl)propanoique, en présence de 128,4 mg d'EDCI, de 45,3 mg d'HOBT et de 104 µL de triéthylamine dans 5 mL de DMF pendant 20 heures. Après purification par flash-chromatographie sur gel de silice, en éluant avec un mélange de dichlorométhane et de méthanol (90-10 en volumes), on obtient 444 mg de N-[4-(3H-Imidazo[4,5-c]pyridin-2-yl)-9H-fluoren-9(R,S)-yl]-2(R,S)-(pyridin-3-yl)-propionamide, sous forme d'une poudre jaune, dont les caractéristiques sont les suivantes :
Spectre de masse (E/I) : m/z = 431 (M+).

Le chlorhydrate de l'acide 2-(pyridin-3-yl)propanoique peut être obtenu selon WO 95 10516.

### Exemple 101 : Synthèse du [4-(3H-imidazo[4,5-c]pyridin-2-yl)-9H-fluorèn-9(R,S)-yl]-amide de l'acide 1-méthyl-1H-pyrrolo[2,3-b]pyridine-3-carboxylique.

On opère comme à l'exemple 14 à partir de 298 mg de 4-(3H-imidazo[4,5-c]pyridin-2-yl)-fluorène-9(R,S)-amine, obtenue à l'exemple 6, et de 185 mg d'acide 1-méthyl-1H-pyrrolo[2,3-b]pyridine-3-carboxylique, en présence de 287 mg d'EDCI et de 202 mg d'HOBT dans 5 mL de DMF pendant 20 heures. Après purification par flash-chromatographie sur gel de silice, en éluant avec un mélange de dichlorométhane et de méthanol (90-10 en volumes), on obtient 165 mg de [4-(3H-imidazo[4,5-c]pyridin-2-yl)-9H-fluorèn-9(R,S)-yl]-amide de l'acide 1-méthyl-1H-pyrrolo[2,3-b]pyridine-3-carboxylique, sous forme d'un solide blanc-cassé, dont les caractéristiques sont les suivantes :
Spectre de masse (E/I) : m/z = 456 (M+).
Point de fusion (Kofler) = 204°C (décomp.).

L'acide 1-méthyl-1H-pyrrolo[2,3-b]pyridine-3-carboxylique peut être obtenu selon WO 96 11929.

### Exemple 102 : Synthèse du 2-bromo-N-[4-(3H-imidazo[4,5-c]pyridin-2-yl)-9H-fluorèn-9(R,S)-yl]-isonicotinamide.

On opère comme à l'exemple 14 à partir de 298,4 mg de 4-(3H-imidazo[4,5-c]pyridin-2-yl)-fluorène-9(R,S)-amine, obtenue à l'exemple 6, et de 202 mg d'acide 2-bromo-isonicotinique, en présence de 211 mg d'EDCI et de 148,6 mg d'HOBT dans 7 mL de DMF pendant 20 heures. Après purification par lavages successifs avec une solution aqueuse saturée d'hydrogénocarbonate de sodium puis avec de l'eau et de l'isopropanol, on obtient 345 mg de 2-bromo-N-[4-(3H-imidazo[4,5-c]pyridin-2-yl)-9H-fluorèn-9(R,S)-yl]-isonicotinamide, sous forme d'une poudre brune, dont les caractéristiques sont les suivantes :
Spectre de masse (E/I) : m/z = 482 (M+).

### Exemple 103 : Synthèse du 3-hydroxy-N-[4-(3H-imidazo[4,5-c]pyridin-2-yl)-9H-fluorèn-9(R,S)-yl]-isonicotinamide.

On opère comme à l'exemple 14 à partir de 400 mg de 4-(3H-imidazo[4,5-c]pyridin-2-yl)-fluorène-9(R,S)-amine, obtenue à l'exemple 6, et de 196 mg d'acide 3-hydroxy-isonicotinique, en présence de 211 mg d'EDCI et de 148,6 mg d'HOBT dans 7 mL de DMF pendant 20 heures. Après purification par flash-chromatographie sur gel de silice, en éluant avec un mélange de dichlorométhane et de méthanol (90-10 en volumes), on obtient 250 mg de 3-hydroxy-N-[4-(3H-imidazo[4,5-c]pyridin-2-yl)-9H-fluorèn-9(R,S)-yl]-isonicotinamide, sous forme d'un solide beige, dont les caractéristiques sont les suivantes :
Spectre de masse (E/I) : m/z = 419 (M+).

### Exemple 104 : Synthèse du 3-amino-N-[4-(3H-imidazo[4,5-c]pyridin-2-yl)-9H-fluorèn-9(R,S)-yl]-isonicotinamide.

On opère comme à l'exemple 14 à partir de 298,4 mg de 4-(3H-imidazo[4,5-c]pyridin-2-yl)-fluorène-9(R,S)-amine, obtenue à l'exemple 6, et de 138 mg d'acide 3-amino-isonicotinique, en présence de 211 mg d'EDCI et de 148,6 mg d'HOBT dans 7 mL de DMF pendant 20 heures. Après purification par flash-chromatographie sur gel de silice, en éluant avec un mélange de dichlorométhane et de méthanol (95-5 en volumes), on obtient 176 mg de 3-amino-N-[4-(3H-imidazo[4,5-c]pyridin-2-yl)-9H-fluorèn-9(R,S)-yl]-isonicotinamide, sous forme d'un solide jaune-pâle, dont les caractéristiques sont les suivantes :
Spectre de masse (E/I) : m/z = 418 (M+).

### Exemple 105 : Synthèse du 2-cyano-N-[4-(3H-imidazo[4,5-c]pyridin-2-yl)-9H-fluorèn-9(R,S)-yl]-isonicotinamide.

On opère comme à l'exemple 14 à partir de 298,4 mg de 4-(3H-imidazo[4,5-c]pyridin-2-yl)-fluorène-9(R,S)-amine, obtenue à l'exemple 6, et de 148 mg d'acide 2-cyano-isonicotinique, en présence de 211 mg d'EDCI et de 148,6 mg d'HOBT dans 7 mL de DMF pendant 20 heures. Après purification par lavages successifs avec une solution aqueuse saturée d'hydrogénocarbonate de sodium puis avec de l'eau et de l'isopropanol, on obtient 388 mg de 2-cyano-N-[4-(3H-imidazo[4,5-c]pyridin-2-yl)-9H-fluorèn-9(R,S)-yl]-isonicotinamide, sous forme d'une poudre brune, dont les caractéristiques sont les suivantes :
Spectre de masse (E/I) : m/z = 428 (M+).

### Exemple 106 : Synthèse du N-[4-(6-méthoxy-1H-benzimidazol-2-yl)-9H-fluorèn-9(R,S)-yl]-isonicotinamide.

Etape 1 : On opère comme à l'étape 1 de l'exemple 1, à partir de 2,562 g de 4-méthoxy-o-phénylènediamine dans 150 mL de dichlorométhane, 3,445 mL de triéthylamine et 3 g de chlorure de l'acide fluorèn-4-one-9-carboxylique pendant 20 heures d'agitation à température ambiante. Après purification par flash-chromatographie sur gel de silice (40-63 µm), en éluant avec un mélange de dichlorométhane et de méthanol (98-2 en volumes), on obtient 2,97 g du mélange des amides régioisomères, utilisé tel quel à l'étape suivante.

Etape 2: Dans un tricol de 500 mL sous atmosphère d'argon, on chauffe, à 100°C pendant 5 heures, une solution de 2,97 g du mélange des amides, obtenu à l'étape précédente, dans 150 mL d'acide acétique. Après refroidissement, le précipité formé est essoré. Après purification par flash-chromatographie sur gel de silice (40-63 µm), en éluant avec un mélange de dichlorométhane et de méthanol 99-1 en volumes, on obtient 1,71 g de 4-(6-méthoxy-1H-benzimidazol-2-yl)-9H-fluorèn-9-one, sous forme d'une poudre brune, utilisée telle quelle à l'étape suivante.

Etape 3 : On opère comme à l'exemple 5, à partir de 1 g de 4-(6-méthoxy-1H-benzimidazol-2-yl)-9H-fluorèn-9-one, obtenue à l'étape précédente, de 0,639 g de chlorhydrate d'hydroxylamine et de 1,257 g d'acétate de sodium agités à température ambiante pendant 20 heures dans 17 mL d'éthanol. Après purification par empâtage dans de l'oxyde de diisopropyle, on obtient 959 mg de 4-(6-méthoxy-1H-benzimidazol-2-yl)-9H-fluorèn-9-one oxime, en mélange 50-50 des isomères Z et E, sous forme d'une poudre marron, dont les caractéristiques sont les suivantes :
Spectre de masse (E/I) : m/z = 341 (M+).
Spectre RMN 1H (300 MHz, □ en ppm, DMSO-d6) : 3,92 (s, 3H) ; 6,78 (d large, J = 8,5 Hz, 0,5 Hz) ; 6,93 (d large, J = 8,5 Hz, 0,5H) ; de 7,25 à 7,52 (m, 4H) ; de 7,59 à 7,72 (m, 1H); de 7,79 à 7,92 (m, 2,5H) ; 8,08 (d large, J = 8,5 Hz, 0,5H) ; 8,47 (d large, J = 8,5 Hz, 0,5H) ; 8,73 (d large, J = 8,5 Hz, 0,5H).

Etape 4 : On opère comme à l'exemple 6, à partir de 900 mg de 4-(6-méthoxy-1H-benzimidazol-2-yl)-9H-fluorèn-9-one oxime(Z,E), obtenue à l'étape précédente, et de 1,55 mg de Nickel de Raney dans 50 mL d'éthanol et 50 mL de tetrahydrofurane pendant 16 heures à 60°C sous une pression d'un bar d'hydrogène. Après purification par empâtage dans l'oxyde de diisopropyle, on obtient 631 mg de 4-(6-méthoxy-1H-benzimidazol-2-yl)-9H-fluorèn-9(R,S)-ylamine, sous forme d'une poudre brune, dont les caractéristiques sont les suivantes :
Spectre de masse (E/I) : m/z = 327 (M+).

Etape 5 : On opère comme à l'exemple 14 à partir de 300 mg de 44-(6-méthoxy-1H-benzimidazol-2-yl)-9H-fluorèn-9(R,S)-ylamine, obtenue à l'étape précedente, et de 118,6 mg d'acide isonicotinique, en présence de 263,4 mg d'EDCI et de 185,7 mg d'HOBT dans 3 mL de DMF pendant 20 heures. Après purification par flash-chromatographie successives sur cartouche de silice Isolute, en éluant avec des mélanges d'acétate d'éthyle et de méthanol (99-1 puis 98-2 en volumes), on obtient 9,1 mg de N-[4-(6-méthoxy-1H-benzimidazol-2-yl)-9H-fluorèn-9(R,S)-yl]-isonicotinamide, sous forme d'une poudre beige, dont les caractéristiques sont les suivantes :
Spectre de masse (E/I) : m/z = 432 (M+).

### Exemple 107 : Synthèse de la 4-(6-hydroxy-1H-benzimidazol-2-yl)-fluorèn-9-one oxime (Z,E).

Etape 1 : Dans un tricol de 50 mL sous atmosphère d'argon, on dissout 700 mg de 4-(6-méthoxy-1H-benzimidazol-2-yl)-9H-fluorèn-9-one, obtenue à l'étape 2 de l'exemple 106, dans 10 mL de dichlorométhane, puis on ajoute goutte à goutte 5,35 mL d'une solution 1M de tribromure de bore dans le dichlorométhane et on agite pendant 20 heures à température ambiante. Le milieu réactionnel est coulé dans 50 mL d'eau. Le précipité formé est essoré et lavé à l'eau puis au toluène et enfin séché. Après purification par flash-chromatographie sur gel de silice, en éluant avec un mélange de dichlorométhane et de méthanol (90-10 en volumes), on obtient 363 mg de 4-(6-hydroxy-1H-benzimidazol-2-yl)-fluorèn-9-one, sous forme d'une poudre brune, dont les caractéristiques sont les suivantes :
Spectre de masse (E/I) : m/z = 312 (M+).

Etape 2 : On opère comme à l'exemple 5, à partir de 571 mg de 4-(6-hydroxy-1H-benzimidazol-2-yl)-fluorèn-9-one, obtenue à l'étape précédente, de 381 mg de chlorhydrate d'hydroxylamine et de 750 mg d'acétate de sodium agités à température ambiante pendant 20 heures dans 11 mL d'éthanol. Après purification par empâtage dans de l'oxyde de diisopropyle, on obtient 539 mg de 4-(6-hydroxy-1H-benzimidazol-2-yl)-fluorèn-9-one oxime, en mélange 50-50 des isomères Z et E, sous forme d'une poudre marron, dont les caractéristiques sont les suivantes :
Spectre de masse (E/I) : m/z = 327(M+).

### Exemple 108 : Synthèse de l'ester de méthyle de l'acide 4-[4-(3H-imidazo[4,5-c]pyridin-2-yl)-9H-fluorèn-9(R,S)-ylcarbamoyl]-pyridine-2-carboxylique.

On opère comme à l'exemple 14 à partir de 597 mg de 4-(3H-imidazo[4,5-c]pyridin-2-yl)-fluorène-9(R,S)-amine, obtenue à l'exemple 6, et de 362 mg du 2-ester de méthyle de l'acide pyridine-2,4-dicarboxylique, en présence de 422 mg d'EDCI et de 297 mg d'HOBT dans 14 mL de DMF pendant 20 heures. Après purification par flash-chromatographie sur gel de silice, en éluant avec un mélange de dichlorométhane et de méthanol (90-10 en volumes), on obtient 610 mg d'ester de méthyle de l'acide 4-[4-(3H-imidazo[4,5-c]pyridin-2-yl)-9H-fluorèn-9(R,S)-ylcarbamoyl]-pyridine-2-carboxylique, sous forme d'un solide beige, dont les caractéristiques sont les suivantes :
Spectre de masse (E/I) : m/z = 461 (M+).

Le 2-ester de méthyle de l'acide pyridine-2,4-dicarboxylique peut être obtenu selon EP 479177.

### Exemple 109 : Synthèse de l'acide 4-[4-(3H-imidazo[4,5-c]pyridin-2-yl)-9H-fluorèn-9(R,S)-ylcarbamoyl]-pyridine-2-carboxylique.

Dans un tricol de 50 mL on dissout dans 20 mL de méthanol 500 mg d'ester de méthyle de l'acide 4-[4-(3H-imidazo[4,5-c]pyridin-2-yl)-9H-fluorèn-9(R,S)-ylcarbamoyl]-pyridine-2-carboxylique, obtenu à l'exemple 108, puis on ajoute une solution de 60,8 mg d'hydroxyde de potassium dans 10 mL d'eau et on agite à température ambiante pendant 20 heures. Le milieu réactionnel est concentré sous pression réduite, re-dissout dans 50 mL d'eau et extrait 2 fois par 50 mL d'oxyde de diéthyle. La phase aqueuse est acidifiée à pH= 4 par addition d'une solution aqueuse normale d'acide chlorhydrique. Le précipité formé est essoré, lavé à l'eau et à l'oxyde de diisopropyle. On obtient ainsi 410 mg d'acide 4-[4-(3H-imidazo[4,5-c]pyridin-2-yl)-9H-fluorèn-9(R,S)-ylcarbamoyl]-pyridine-2-carboxylique, sous forme d'un solide beige dont les caractéristiques sont les suivantes :
Spectre de masse (E/I) : m/z = 447 (M+).

### Exemple 110 : Synthèse du N-[4-(3H-imidazo[4,5-b]pyridin-2-yl)-9H-fluorèn-9(R,S)-yl]-isonicotinamide.

Etape 1 : On opère comme à l'étape 1 de l'exemple 1, à partir de 2,023 g de 2,3-diamino-pyridine dans 150 mL de dichlorométhane, 3,445 mL de triéthylamine et 3 g de chlorure de l'acide fluorèn-4-one-9-carboxylique pendant 20 heures d'agitation à température ambiante. Après purification par flash-chromatographie sur gel de silice (40-63 µm), en éluant avec des mélanges de dichlorométhane et de méthanol (98-2 puis 95-5 en volumes), on obtient 1,495 g du mélange des amides régioisomères, utilisé tel quel à l'étape suivante.

Etape 2 : Dans un tricol de 250 mL sous atmosphère d'argon, on chauffe, à 100°C pendant 2 heures et 30 minutes, une solution de 1,49 g du mélange des amides, obtenu à l'étape précédente, dans 75 mL d'acide acétique. Après refroidissement, le précipité formé est essoré. Après purification par empâtage dans de l'oxyde de diisopropyle, on obtient 1,40 g de 4-(3H-imidazo[4,5-b]pyridin-2-yl)-9H-fluorèn-9-one, sous forme d'une poudre jaune utilisée telle quelle à l'étape suivante.

Etape 3 : On opère comme à l'exemple 5, à partir de 1,4 g de 4-(3H-imidazo[4,5-b]pyridin-2-yl)-9H-fluorèn-9-one, obtenue à l'étape précédente, de 1 g de chlorhydrate d'hydroxylamine et de 1,93 g d'acétate de sodium agités à température ambiante pendant 20 heures dans 25 mL d'éthanol. Après purification par flash-chromatographie sur gel de silice, en éluant avec des mélanges dichlorométhane et d'ammoniac en solution 7M dans le méthanol (90-10 puis 85-15 en volumes), on obtient 1,32 g de 4-(3H-imidazo[4,5-b]pyridin-2-yl)-9H-fluorèn-9-one oxime, en mélange 50-50 des isomères Z et E, sous forme d'une poudre beige, dont les caractéristiques sont les suivantes :
Spectre de masse (E/I) : m/z = 312 (M+).
Spectre RMN 1H (300 MHz, □en ppm, DMSO-d6) : 7,26 (t large, J = 7,5 Hz, 0,5H) ; de 7,30 à 7,48 (m, 2H) ; de 7,57 à 7,69 (m, 1,5H) ; de 7,77 à 7,86 (m, 2,5H) ; 8,04 (d large, J = 8,5 Hz, 0,5H) ; 8,48 (d large, J = 8,5 Hz, 0,5H) ; de 8,67 à 8,75 (m, 1,5H) ; 8,78 (d large, J = 5,5 Hz, 1H).

Etape 4 : On opère comme à l'exemple 6, à partir de 1,30 g de 4-(3H-imidazo[4,5-b]pyridin-2-yl)-9H-fluorèn-9-one oxime(Z,E), obtenue à l'étape précédente, et de 2,45 mg de Nickel de Raney dans 65 mL d'éthanol et 65 mL de tetrahydrofurane pendant 5 heures à 60°C sous une pression d'un bar d'hydrogène. Après purification par empâtage dans l'oxyde de diisopropyle, on obtient 1,06 g de 4-(3H-imidazo[4,5-b]pyridin-2-yl)-9H-fluorèn-9(R,S)-ylamine, sous forme d'une poudre blanche, dont les caractéristiques sont les suivantes :
Spectre de masse (E/I) : m/z = 298 (M+).

Etape 5 : On opère comme à l'exemple 14 à partir de 300 mg de 4-(3H-imidazo[4,5-b]pyridin-2-yl)-9H-fluorèn-9(R,S)-ylamine, obtenue à, l'étape précedente, et de 130 mg d'acide isonicotinique, en présence de 289,3 mg d'EDCI et de 204 mg d'HOBT dans 3 mL de DMF pendant 20 heures. Après purification par lavages successifs avec une solution aqueuse saturée d'hydrogénocarbonate de sodium puis avec de l'eau et empatage dans de l'oxyde de diisopropyle, on obtient 227 mg de N-[4-(3H-imidazo[4,5-b]pyridin-2-yt)-9H-fluorèn-9(R,S)-yl]-isonicotinamide., sous forme d'une poudre beige, dont les caractéristiques sont les suivantes :
Spectre de masse (E/l) : m/z = 403 (M+).

Spectre RMN 1H (300 MHz, □ en ppm, DMSO-d6) : 6,38 (s, 1H) ; 7,29 (t large, J = 7,5 Hz, 1H) ; 7,39 (t large, J = 7,5 Hz, 1H) ; 7,60 (t, J = 7,5 Hz, 1H) ; de 7,63 à 7,70 (m, 2H) ; de 7,74 à 7,85 (m, 2H) ; 7,88 (d large, J = 7,5 Hz, 1H) ; 8,34 (d large, J = 6,0 Hz, 2H) ; 8,67 (d large, J = 8,5 Hz, 1H) ; 8,77 (d large, J = 5,0 Hz, 1H) ; 9,04 (d large, J = 6,0 Hz, 2H).

### Exemple 111 : Synthèse du [4-(3H-imidazo[4,5-c]pyridin-2-yl)-9H-fluorèn-9(R,S)-yl]-amide de l'acide 1H-indazole-4-carboxylique.

On opère comme à l'exemple 14 à partir de 300 mg de 4-(3H-imidazo[4,5-c]pyridin-2-yl)-fluorène-9(R,S)-amine, obtenue à l'exemple 6, et de 171 mg d'acide 1H-indazole-4-carboxylique, en présence de 212 mg d'EDCI et de 150 mg d'HOBT dans 3 mL de DMF pendant 20 heures. Après purification par flash-chromatographie sur gel de silice, en éluant avec un mélange de dichlorométhane et de méthanol (90-10 en volumes), on obtient 154 mg de [4-(3H-imidazo[4,5-c]pyridin-2-yl)-9H-fluorèn-9(R,S)-yl]-amide de l'acide 1H-indazole-4-carboxylique, sous forme d'un solide jaune, dont les caractéristiques sont les suivantes :
Spectre de masse (E/I) : m/z = 442 (M+).
Point de fusion (Kofler) = 230-35°C.

### Exemple 112 : Synthèse du 4-{[4-(3H-imidazo[4,5-c]pyridin-2-yl)-9H-fluorèn-9(R,S)-yl]-amide}-2-[(3-méthoxy-propyl)-amide] de l'acide pyridine-2,4-dicarboxylique.

On opère comme à l'exemple 14 à partir de 62,6 mg de 4-(3H-imidazo[4,5-c]pyridin-2-yl)-fluorène-9(R,S)-amine, obtenue à l'exemple 6, et de 50 mg du 2-[(3-méthoxy-propyl)-amide] de l'acide pyridine-2,4-dicarboxylique, en présence de 44,3 mg d'EDCI et de 31,2 mg d'HOBT dans 2 mL de DMF pendant 20 heures. Après purification par lavages successifs avec une solution aqueuse saturée d'hydrogénocarbonate de sodium puis avec de l'eau et de l'isopropanol, on obtient 70 mg du 4-{[4-(3H-imidazo[4,5-c]pyridin-2-yl)-9H-fluorèn-9(R,S)-yl]-amide) 2-[(3-méthoxy-propyl)-amide] de l'acide pyridine-2,4-dicarboxylique, sous forme d'une poudre beige, dont les caractéristiques sont les suivantes :
Spectre de masse (E/I) : m/z = 518 (M+).

### Exemple 113: Synthèse du [4-(3H-imidazo[4,5-c]pyridin-2-yl)-9H-fluorèn-9(R,S)-yl]-amide de l'acide 2-(furan-2-y)l-quinoléine-4-carboxylique.

On opère comme à l'exemple 14 à partir de 298,4 mg de 4-(3H-imidazo[4,5-c]pyridin-2-yl)-fluorène-9(R,S)-amine, obtenue à l'exemple 6, et de 239 mg d'acide 2-(furan-2-y)l-quinoléine-4-carboxylique, en présence de 211 mg d'EDCI et de 148,6 mg d'HOBT dans 7 mL de DMF pendant 20 heures. Après purification par lavages successifs avec une solution aqueuse saturée d'hydrogénocarbonate de sodium puis avec de l'eau et de l'isopropanol, on obtient 440 mg du [4-(3H-imidazo[4,5-c]pyridin-2-yl)-9H-fluorèn-9(R,S)-yl]-amide de l'acide 2-(furan-2-y)l-quinoléine-4-carboxylique, sous forme d'une poudre brune, dont les caractéristiques sont les suivantes :
Spectre de masse (E/I) : m/z = 519 (M+).

### Exemple 114 : Synthèse du 3-fluoro-N-[4-(3H-imidazo[4,5-c]pyridin-2-yl)-9H-fluorèn-9(R,S)-yl]-isonicotinamide.

On opère comme à l'exemple 14 à partir de 298,4 mg de 4-(3H-imidazo[4,5-c]pyridin-2-yl)-fluorène-9(R,S)-amine, obtenue à l'exemple 6, et de 141 mg d'acide 3-fluoro-isonicotinique, en présence de 211 mg d'EDCI et de 148,6 mg d'HOBT dans 7 mL de DMF pendant 20 heures. Après purification par lavages successifs avec une solution aqueuse saturée d'hydrogénocarbonate de sodium puis avec de l'eau et de l'isopropanol, on obtient 350 mg de 3-fluoro-N-[4-(3H-imidazo[4,5-c]pyridin-2-yl)-9H-fluorèn-9(R,S)-yl]-isonicotinamide, sous forme d'une poudre brune, dont les caractéristiques sont les suivantes :
Spectre de masse (E/I) : m/z = 421 (M+).

### Exemple 115 : Synthèse du 3-amino-N-[4-(3H-imidazo[4,5-c]pyridin-2-yl)-9H-fluorèn-9(R,S)-yl]-benzamide.

On opère comme à l'exemple 14 à partir de 300 mg de 4-(3H-imidazo[4,5-c]pyridin-2-yl)-fluorène-9(R,S)-amine, obtenue à l'exemple 6, et de 137 mg d'acide 3-amino-benzoique, en présence de 211 mg d'EDCI et de 148,6 mg d'HOBT dans 3 mL de DMF pendant 20 heures. Après purification par flash-chromatographie sur gel de silice, en éluant avec un mélange de dichlorométhane et de méthanol (90-10 en volumes), on obtient 230 mg de 3-amino-N-[4-(3H-imidazo[4,5-c]pyridin-2-yl)-9H-fluorèn-9(R,S)-yl]-benzamide, sous forme d'un solide jaune-pâle, dont les caractéristiques sont les suivantes :
Spectre de masse (E/I) : m/z = 417 (M+).

### Exemple 116 : Synthèse du N-[4-(3H-imidazo[4,5-c]pyridin-2-yl)-9H-fluorèn-9(R,S)-yl]-2-méthylamino-isonicotinamide.

Dans un autoclave de 30 mL, on chauffe pendant 20 heures à 90°C, une solution de 230 mg de 2-bromo-N-[4-(3H-imidazo[4,5-c]pyridin-2-yl)-9H-fluorèn-9(R,S)-yl]-isonicotinamide, obtenu à l'exemple 102, dans 6 mL d'éthanol et 6 mL de méthylamine. Le milieu réactionnel est concentré sous pression réduite, puis purifié par flash-chromatographie sur gel de silice, en éluant avec des mélanges de dichlorométhane et de méthanol (97,5-2,5 puis 95-5 en volumes). On obtient ainsi, après lavage avec une aqueuse solution décinormale d'hydroxyde de sodium, 20 mg de N-[4-(3H-imidazo[4,5-c]pyridin-2-yl)-9H-fluorèn-9(R,S)-yl]-2-méthylamino-isonicotinamide, sous forme d'un solide brun dont les caractéristiques sont les suivantes :
Spectre de masse (E/I) : m/z = 432 (M+).

### Exemple 117 : Synthèse du N-[4-(6-hydroxy-1H-benzimidazol-2-yl)-9H-fluorèn-9(R,S)-yl]-isonicotinamide.

Etape 1 : On opère comme à l'exemple 6, à partir de 500 mg de 4-(6-hydroxy-1H-benzimidazol-2-yl)-fluorèn-9-one oxime (Z,E), obtenue à l'exemple 107, et de 5 mg de Nickel de Raney dans 25 mL d'éthanol et 25 mL de tetrahydrofurane pendant 5 heures à 60°C sous une pression d'un bar d'hydrogène. Après purification par empâtage dans l'oxyde de diisopropyle, on obtient 385 mg de 4-(6-hydroxy-1H-benzimidazol-2-yl)-fluorèn-9(R,S)-ylamine, sous forme d'une poudre brune, dont les caractéristiques sont les suivantes :
Spectre de masse (E/I) : m/z = 313 (M+).

Etape 2 : On opère comme à l'exemple 14 à partir de 300 mg de 4-(6-hydroxy-1H-benzimidazol-2-yl)-fluorèn-9(R,S)-ylamine, obtenue à l'étape précedente, et de 130 mg d'acide isonicotinique, en présence de 275 mg d'EDCI et de 194 mg d'HOBT dans 3 mL de DMF pendant 20 heures. Après purification par lavages successifs avec une solution aqueuse saturée d'hydrogénocarbonate de sodium puis avec de l'eau et empâtage dans de l'oxyde de diisopropyle, on obtient 87 mg de N-[4-(6-hydroxy-1H-benzimidazol-2-yl)-9H-fluorèn-9(R,S)-yl]-isonicotinamide, sous forme d'une poudre beige, dont les caractéristiques sont les suivantes :
Spectre de masse (E/I) : m/z = 418 (M+).

### Exemple 118 : Synthèse du [4-(3H-imidazo[4,5-c]pyridin-2-yl)-9H-fluorèn-9(R,S)-yl]-amide de l'acide 6-bromo-3H-imidazo[4,5-b]pyridine-7-carboxylique.

On opère comme à l'exemple 14 à partir de 149 mg de 4-(3H-imidazo[4,5-c]pyridin-2-yl)-fluorène-9(R,S)-amine, obtenue à l'exemple 6, et de 121 mg d'acide 6-bromo-3H-imidazo[4,5-b]pyridine-7-carboxylique, en présence de 105,4 mg d'EDCI et de 74,3 mg d'HOBT dans 3,5 mL de DMF pendant 20 heures. Après purification par flash-chromatographie sur gel de silice, en éluant avec un mélange de dichlorométhane et de méthanol (90-10 en volumes), on obtient 100 mg de [4-(3H-imidazo[4,5-c]pyridin-2-yl)-9H-fluorèn-9(R,S)-yl]-amide de l'acide 6-bromo-3H-imidazo[4,5-b]pyridine-7-carboxylique, sous forme d'un solide vert-pâle, dont les caractéristiques sont les suivantes :
Spectre de masse (E/I) : m/z = 522 (M+).

### Exemple 119: Synthèse du [4-(3H-imidazo[4,5-c]pyridin-2-yl)-9H-fluorèn-9(R,S)-yl]-amide de l'acide 2-pipéridin-1-yl-quinoléine-4.-carboxylique.

On opère comme à l'exemple 14 à partir de 298,4 mg de 4-(3H-imidazo[4,5-c]pyridin-2-yl)-fluorène-9(R,S)-amine, obtenue à l'exemple 6, et de 256,3 mg d'acide 2-pipéridin-1-yl-quinoléine-4-carboxylique (ou 2-pipéridino-cinchoninique), en présence de 211 mg d'EDCI et de 148,6 mg d'HOBT dans 7 mL de DMF pendant 20 heures. Après purification par lavages successifs avec une solution aqueuse saturée d'hydrogénocarbonate de sodium puis avec de l'eau et de l'isopropanol, on obtient 495 mg de [4-(3H-imidazo[4,5-c]pyridin-2-yl)-9H-fluorèn-9(R,S)-yl]-amide de l'acide 2-pipéridin-1-yl-quinoléine-4-carboxylique, sous forme d'une poudre jaune, dont les caractéristiques sont les suivantes :
Spectre de masse (E/I) : m/z = 536 (M+).

### Exemple 120 : Synthèse du N-[4-(3H-imidazo[4,5-c]pyridin-2-yl)-9H-fluorèn-9(R,S)-yl]-2-pyrrolidin-1-yl-isonicotinamide.

On opère comme à l'exemple 14 à partir de 298,4 mg de 4-(3H-imidazo[4,5-c]pyridin-2-yl)-fluorène-9(R,S)-amine, obtenue à l'exemple 6, et de 199,2 mg d'acide 2-pyrrolidin-1-yl-isonicotinique, en présence de 211 mg d'EDCI et de 148,6 mg d'HOBT dans 7 mL de DMF pendant 20 heures. Après purification par lavages successifs avec une solution aqueuse saturée d'hydrogénocarbonate de sodium puis avec de l'eau et de l'isopropanol, on obtient 260 mg de N-[4-(3H-imidazo[4,5-c]pyridin-2-yl)-9H-fluorèn-9(R,S)-yl]-2-pyrrolidin-1-yl-isonicotinamide, sous forme d'une poudre jaune, dont les caractéristiques sont les suivantes :
Spectre de masse (E/I) : m/z = 472 (M+).

### Exemple 121 : Synthèse du 2-hydroxy-N-[4-(3H-imidazo[4,5-c]pyridin-2-yl)-9H-fluorèn-9(R,S)-yl]-6-méthyl-isonicotinamide.

On opère comme à l'exemple 14 à partir de 298,4 mg de 4-(3H-imidazo[4,5-c]pyridin-2-yl)-fluorène-9(R,S)-amine, obtenue à l'exemple 6, et de 153 mg d'acide 2-hydroxy-6-méthyl-isonicotinique, en présence de 211 mg d'EDCI et de 148,6 mg d'HOBT dans 7 mL de DMF pendant 20 heures. Après purification par flash-chromatographie sur gel de silice, en éluant avec un mélange de dichlorométhane et de méthanol (90-10 en volumes), on obtient 340 mg de 2-hydroxy-N-[4-(3H-imidazo[4,5-c]pyridin-2-yl)-9H-fluorèn-9(R,S)-yl]-6-méthyl-isonicotinamide, sous forme d'un solide beige, dont les caractéristiques sont les suivantes :
Spectre de masse (E/I) : m/z = 433 (M+).

### Exemple 122 : Synthèse de l'ester de 4-(3H-imidazo[4,5-c]pyridin-2-yl)-9H-fluoren-9(R,S)-yle de l'acide isonicotinique.

Dans un ballon de 25 mL sous atmosphere d'argon, on dissout 299,3 mg de 4-(3H-Imidazo[4,5-c]pyridin-2-yl)-fluorène-9(R,S)-ol, obtenu à l'exemple 3, dans 5 mL de pyridine. A cette solution refroidie à 0°C, on ajoute 356 mg de chlorhydrate du chlorure de l'acide isonicotinique ; puis on agite 18 heures à température ambiante. Le milieu réactionnel est concentré puis coulé dans 10 mL d'eau. Le précipité formé est essoré et lavé successivement par 10 mL d'une solution aqueuse saturée d'hydrogénocarbonate de sodium, 2 fois 10 mL d'eau et 2 fois 10 mL d'oxyde de diisopropyle. On obtient ainsi, après séchage sous pression réduite à 50°C, 335 mg d'ester de 4-(3H-imidazo[4,5-c]pyridin-2-yl)-9H-fluoren-9(R,S)-yle de l'acide isonicotinique, sous forme d'une poudre jaune-pâle dont les caractéristiques sont les suivantes :
Spectre de masse (E/I) : m/z = 404 (M+).

### Exemple 123 : Synthèse du 2-chloro-N-[4-(3H-imidazo[4,5-c]pyridin-2-yl)-9H-fluorèn-9(R,S)-yl]-6-méthyl-isonicotinamide.

On opère comme à l'exemple 14 à partir de 400 mg de 4-(3H-imidazo[4,5-c]pyridin-2-yl)-fluorène-9(R,S)-amine, obtenue à l'exemple 6, et de 241,6 mg d'acide 2-chloro-6-méthyl-isonicotinique, en présence de 270 mg d'EDCI et de 91 mg d'HOBT dans 7 mL de DMF pendant 20 heures. Après purification par lavages successifs avec une solution aqueuse saturée d'hydrogénocarbonate de sodium puis avec de l'eau et de l'isopropanol, on obtient 332 mg de 2-chloro-N-[4-(3H-imidazo[4,5-c]pyridin-2-yl)-9H-fluorèn-9(R,S)-yl]-6-méthyl-isonicotinamide, sous forme d'un solide beige, dont les caractéristiques sont les suivantes :
Spectre de masse (E/I) : m/z = 433 (M+).
Point de fusion (Kofler) = 250°C.

### Exemple 124 : Synthèse de l'ester de 4-(3H-imidazo[4,5-c]pyridin-2-yl)-9H-fluorèn-9(R,S)-yle de l'acide 5-quinoléine-carboxylique.

Dans un ballon de 50 mL, on introduit successivement 347 mg de l'acide 5-quinoléine-carboxylique, deux gouttes de diméthylformamide et 15 mL de dichlorométhane. Après refroidissement à 0°C, on ajoute 206 □1 de chlorure d'oxalyle et on agite 1 heure à température ambiante. On évapore le solvant, puis on ajoute 300mg de 4-(3H-imidazo[4,5-c]pyridine-2-yl)-fluorène-9(R,S)-ol, synthétisé comme à l'exemple 3, en solution dans un mélange de 5 mL de pyridine et 2 mL de dichlorométhane. Au bout de 5 minutes à température ambiante, on ajoute 1 mL d'eau, puis le solvant est évaporé. Le milieu réactionnel pâteux est jeté dans 20 mL de solution aqueuse saturée d'hydrogénocarbonate de sodium. Le solide obtenu est filtré puis successivement lavé avec de l'eau (2x10 mL) et de l'ether diisopropylique (2 x 10mL). Après purification par flash-chromatographie sur gel de silice (40-63 µm), en éluant avec un mélange de dichlorométhane et de méthanol (95-05 en volumes), on obtient 120 mg de l'ester de 4-(3H-imidazo[4,5-c]pyridin-2-yl)-9H-fluorèn-9(R,S)-yle de l'acide 5-quinoléine-carboxylique, sous forme d'une meringue blanc-cassé dont les caractéristiques sont les suivantes :
Spectre de masse (LCMS) : m = 455 (MH+).
Spectre RMN 1H (300 MHz, □en ppm, DMSO-d6) :

### Exemple 125: Synthèse du N-[4-(3H-imidazo[4,5-c]pyridin-2-yl)-9H-fluorèn-9(R,S)-yl]-amide de l'acide 2-tert-butoxycarbonylamino-isonicotinique.

On opère comme à l'exemple 14 à partir de 3,2 g de 4-(3H-imidazo[4,5-c]pyridin-2-yl)-fluorène-9(R,S)-amine, obtenue à l'exemple 6, et de 2,6 g d'acide 2-tert-butoxycarbonylamino-isonicotinique, en présence de 2,3 g d'EDCI et de 1,6 g d'HOBT dans 80 mL de DMF pendant 20 heures. Après lavages successifs avec une solution aqueuse saturée d'hydrogénocarbonate de sodium, avec de l'eau puis avec de l'éther isopropylique, on obtient 5,4 g de N-[4-(3H-imidazo[4,5-c]pyridin-2-yl)-9H-fluorèn-9(R,S)-yl]-amide de l'acide 2-tert-butoxycarbonylamino-isonicotinique, sous forme d'un solide bleige, dont les caractéristiques sont les suivantes :
Spectre de masse (E/I) : m/z = 518 (M+).

L'acide 2-tert-butoxycarbonylamino-isonicotinique peut être obtenu selon WO 2001074788.

### Exemple 126 : Synthèse du N-[4-(3H-imidazo[4,5-c]pyridin-2-yl)-9H-fluorèn-9(R,S)-yl]-isophthalamide.

On opère comme à l'exemple 14 à partir de 325 mg de 4-(3H-imidazo[4,5-c]pyridin-2-yl)-fluorène-9(R,S)-amine, obtenue à l'exemple 6, et de 180 mg d'acide isophthalamique, en présence de 230 mg d'EDCI et de 162 mg d'HOBT dans 8 mL de DMF pendant 20 heures. Après lavages successifs avec une solution aqueuse saturée d'hydrogénocarbonate de sodium, avec de l'eau puis avec de l'éther isopropylique, on obtient 380 mg de N-[4-(3H-imidazo[4,5-c]pyridin-2-yl)-9H-fluorèn-9(R,S)-yl]-isophthalamide, sous forme d'un solide bleige, dont les caractéristiques sont les suivantes :
Spectre de masse (E/I) : m/z = 445 (M+).
L' acide isophthalamique peut être obtenu selon WO 2000021920.

### Exemple 127: Synthèse du N-[4-(3H-imidazo[4,5-c]pyridin-2-yl)-9H-fluorèn-9(R,S)-yl]-4-méthoxy-benzamide.

On opère comme à l'exemple 14 à partir de 400 mg de 4-(3H-imidazo[4,5-c]pyridin-2-yl)-fluorène-9(R,S)-amine, obtenue à l'exemple 6, et de 200 mg d'acide 4-méthoxy benzoïque, en présence de 270 mg d'EDCI et de 91 mg d'HOBT dans 14 mL de DMF pendant 20 heures. Après lavages successifs avec une solution aqueuse saturée d'hydrogénocarbonate de sodium puis avec de l'eau le mélange brut est purifié par flash-chromatographie sur gel de silice, en éluant avec un mélange de dichlorométhane et de méthanol (95-05 en volumes), on obtient 78 mg de N-[4-(3H-imidazo[4,5-c]pyridin-2-yl)-9H-fluorèn-9(R,S)-yl]-4-méthoxy-benzamide, sous forme d'un solide blanc, dont les caractéristiques sont les suivantes :
Spectre de masse (LC/MS) : m/z = 432 (M+).
Point de fusion (Kofler) = 194°C.

### Exemple 128: Synthèse du N-[4-(3H-imidazo[4,5-c]pyridin-2-yl)-9H-fluorèn-9(R,S)-yl]-amide de l'acide 3-chloro-6-méthoxy-quinoléine-4-carboxylique.

On opère comme à l'exemple 14 à partir de 300 mg de 4-(3H-imidazo[4,5-c]pyridin-2-yl)-fluorène-9(R,S)-amine, obtenue à l'exemple 6, et de 238 mg d'acide 3-chloro-6-méthoxy-quinoléine-4-carboxylique, en présence de 211 mg d'EDCI et de 148 mg d'HOBT dans 7 mL de DMF pendant 20 heures. Après lavages successifs avec une solution aqueuse saturée d'hydrogénocarbonate de sodium puis avec de l'eau le mélange brut est purifié par flash-chromatographie sur gel de silice, en éluant avec un mélange de dichlorométhane et d'une solution 7N de méthanol ammoniacal (95-05 en volumes), on obtient 310 mg de N-[4-(3H-imidazo[4,5-c]pyridin-2-yl)-9H-fluorèn-9(R,S)-yl]-amide de l'acide 3-chloro-6-méthoxy-quinoléine-4-carboxylique, sous forme d'une poudre blanche, dont les caractéristiques sont les suivantes :
Spectre de masse (E/I) : m/z = 517 (M+).

### Exemple 129 : Synthèse du chlorhydrate du 4-amino-N-[4-(3H-imidazo[4,5-c]pyridin-2-yl)-9H-fluorèn-9(R,S)-yl]-benzamide.

Etape 1 : On opère comme à l'exemple 14 à partir de 800 mg de 4-(3H-imidazo[4,5-c]pyridin-2-yl)-fluorène-9(R,S)-amine, obtenue à l'exemple 6, et de 640 mg d'acide 4-tert-butoxycarbonylamino-benzoïque, en présence de 540 mg d'EDCI et de 180 mg d'HOBT dans 14 mL de DMF pendant 20 heures. Après lavages successifs avec une solution aqueuse saturée d'hydrogénocarbonate de sodium puis avec de l'eau le mélange brut est purifié par flash-chromatographie sur gel de silice, en éluant avec un mélange de dichlorométhane et de méthanol (95-05 en volumes), on obtient 168 mg de l'ester de tert-butyle de l'acide 4-[4-(3H-Imidazo[4,5-c]pyridin-2-yl)-9H-fluorèn-9(R,S)-ylcarbamoyl]-phényl}-carbamoïque, sous forme d'un solide blanc, dont les caractéristiques sont les suivantes :
) Spectre de masse (LC/MS) : m/z = 517 (M+).

Etape 2 : On dissout 88 mg d'ester de tert-butyle de l'acide 4-[4-(3H-imidazo[4,5-c]pyridin-2-yl)-9H-fluorèn-9(R,S)-ylcarbamoyl]-phényl}-carbamoïque, obtenu à l'étape 1, dans 5 mL de dichlorométhane et on ajoute 6 mL d'une solution 4M d'acide chlorhydrique dans le dioxane. Après 20 heures d'agitation à température ambiante, le solide formé est essoré. Après lavages par deux fois 3 mL d'éther isopropylique et séchage, on obtient 89 mg de chlorhydrate de 4-Amino-N-[4-(3H-imidazo[4,5-c]pyridin-2-yl)-9H-fluorèn-9(R,S)-yl]-benzamide sous forme d'une poudre blanche, dont les caractéristiques sont les suivantes :
Spectre de masse (LC/MS) : m/z = 417 (M+).

### Exemple 130 : Synthèse du N-[4-(3H-imidazo[4,5-c]pyridin-2-yl)-9H-fluorèn-9(R,S)-yl]-amide de l'acide 3-hydroxy-quinoléine-4-carboxylique.

On opère comme à l'exemple 14 à partir de 370 mg de 4-(3H-imidazo[4,5-c]pyridin-2-yl)-fluorène-9(R,S)-amine, obtenue à l'exemple 6, et de 300 mg d'acide 3-hydroxy-quinoléine-4-carboxylique, en présence de 240 mg d'EDCI et de 84 mg d'HOBT dans 5 mL de DMF pendant 15 heures. Après lavages successifs avec une solution aqueuse saturée d'hydrogénocarbonate de sodium puis avec de l'eau le mélange brut est purifié par flash-chromatographie sur gel de silice, en éluant avec un mélange de dichlorométhane et d'une solution 7N de méthanol ammoniacal (90-10 en volumes), on obtient 73 mg de [4-(3H-imidazo[4,5-c]pyridin-2-yl)-9H-fluorèn-9(R,S)-yl]-amide de l'acide 3-hydroxy-quinoléine-4-carboxylique sous forme d'un solide jaune, dont les caractéristiques sont les suivantes :
Spectre de masse (LC/MS) : m/z = 511 (M+).
Point de fusion (Kofler) = 250°C.

L'acide 3-hydroxy-quinoléine-4-carboxylique peut être obtenu selon Organic Syntheses, 40, 54-60 ; 1960.

### Exemple 131 : Synthèse du N-[4-(3H-imidazo[4,5-c]pyridin-2-yl)-9H-fluorèn-9(R,S)-yl]-amide de l'acide 2-amino-5-chloro-pyrimidine-4-carboxylique.

On opère comme à l'exemple 14 à partir de 8,6 g de 4-(3H-imidazo[4,5-c]pyridin-2-yl)-fluorène-9(R,S)-amine, obtenue à l'exemple 6, et de 5 g d'acide 2-amino-5-chloro-pyrimidine-4-carboxylique, en présence de 5,6 g d'EDCI et de 1,9 g d'HOBT dans 115 mL de DMF pendant 15 heures. Après lavages successifs avec une solution aqueuse saturée d'hydrogénocarbonate de sodium puis avec de l'eau le mélange brut est purifié par flash-chromatographie sur gel de silice, en éluant avec un mélange de dichlorométhane et d'une solution 7N de méthanol ammoniacal (95-05 en volumes), on obtient 6,1 g de N-[4-(3H-imidazo[4,5-c]pyridin-2-yl)-9H-fluorèn-9(R,S)-yl]-amide de l'acide 2-amino-5-chloro-pyrimidine-4-carboxylique sous forme d'une poudre blanche, dont les caractéristiques sont les suivantes :
Spectre de masse (E/I) : m/z = 453 (MH+).
Point de fusion (Kofler) = 216°C.

### Exemple 132 : Synthèse du [4-(3H-imidazo[4,5-c]pyridin-2-yl)-9H-fluorèn-9(R,S)-yl]-amide de l'acide 3-bromo-1H-pyrrolo[2,3-b]pyridine-4-carboxylique.

Dans un ballon de 100 mL, on dissout 700 mg de [4-(3H-imidazo[4,5-c]pyridin-2-yl)-9H-fluorèn-9(R,S)-yl]-amide de l'acide 1H-pyrrolo[2,3-b]pyridine-4-carboxylique, obtenu comme à l'exemple 92, dans 15 mL d'acide acétique glacial. Après refroidissement à 10°C, on ajoute 89,4 □l de brome et on agite 10 minutes à température ambiante. De l'eau est additionnée (1ml) puis le solvant est évaporé. Le solide obtenu est lavé successivement avec de l'eau, une solution aqueuse saturée d'hydrogénocarbonate de sodium puis de l'eau. Après purification par flash-chromatographie sur gel de silice (40-63 µm), en éluant avec un mélange de dichlorométhane et de méthanol (90-10 en volumes), on obtient 670 mg du [4-(3H-imidazo[4,5-c]pyridin-2-yl)-9H-fluorèn-9(R,S)-yl]-amide de l'acide 3-bromo-1H-pyrrolo[2,3-b]pyridine-4-carboxylique, sous forme d'une meringue jaune dont les caractéristiques sont les suivantes :
Spectre de masse (LCMS) : m = 521 (MH+).

### Exemples 133 à 150 :

Les produits des exemples 133 à 150 ont été préparés en parallèle selon le mode opératoire suivant :
A 100 mg (0.335 mmol) d'amine et x mg (0.335 mmol, 1.0 eq) d'acide dans 2.5 ml de DMF sont ajoutés 71 mg (0.370 mmol, 1.1 eq) de chlorhydrate d'EDCI et 50 mg (0.370 mmol, 1.1 eq) de HOBT. Dans le cas où l'acide est sous forme de chlorhydrate, 47 µl (0.335 mmol, 1.0 eq) de TEA sont ajoutés. Le milieu réactionnel est agité 20 h à température ambiante (25 °C) puis concentré.

Le produit brut solide ou pâteux est repris dans 4 ml d'eau et filtré, lavé successivement par 4 ml d'eau, 2 fois 4 ml de solution aqueuse saturée d'hydrogénocarbonate de sodium, 4 ml d'eau puis 2 fois 5 ml d'éther isopropylique.

Les produits isolés dans les conditions ci-dessus présentent une pureté LC/MS supérieure à 90% dans les conditions suivantes :
Colonne Hypersil Gold C18 3x50 mm (diamètre particules : 3Mm)
Détection U.V. DAD (200<λ<400 nm)
Gradient de solvants A (eau contenant 0,1% d'acide formique) et (acétonitrile) à un débit de 0,8 mL/mn

| Temps (mn) | %A | %B |
|---|---|---|
| 0 | 95 | 5 |
| 5 | 5 | 95 |
| 5.5 | 5 | 95 |
| 6.5 | 95 | 5 |
| 7 | 95 | 5 |

Lorqu'une purification complémentaire a été nécessaire les conditions de cette purification sont décrites dans le tableau ci-dessous qui contient également les masses obtenues et les caractéristiques des composés des exemples 133 à 150 (Spectre de masse et temps de rétention par analyse LC/MS)

| Exemple | Masse obtenue (mg) | Spectre de masse (MH+) | Temps de rétention LC/MS (mn) | Conditions de purification complémentaire |
|---|---|---|---|---|
| 133 | 113 | 499 | 4.18 | |
| 134 | 72 | 435 | 3.69 | |
| 135 | 68 | 423 | 3.64 | |
| 136 | 112 | 504 | 3.33 | |
| 137 | 76 | 474 | 2.73 | |
| 138 | 75 | 439 | 2.51 | |
| 139 | 91 | 423 | 3 | |
| 140 | 104 | 475 | 3.36 | |
| 141 | 101 | 434 | 2.6 | |
| 142 | 40 | 447 | 3.43 | Plaque silice prép 0.5 mm CH2Cl2/NH3 7N dans MeOH 96/4 |
| 143 | 53 | 473 | 3.14 | Plaque silice prép 0.5 mm CH2Cl2/NH3 7N dans MeOH 96/4 |
| 144 | 45 | 495 | 2.6 | Cristallisation dans CH2Cl2/MeOH 80/20 |
| 145 | 27 | 465 | 3.26 | Plaque silice prép 0.5 mm CH2Cl2/NH3 7N dans MeOH 96/4 |
| 146 | 74 | 512 | 2.78 | Cristallisation dans CH2Cl2/MeOH 80/20 |
| 147 | 77 | 451 | 2.5 | Plaque silice prép 0.5 mm CH2Cl2/NH3 7N dans MeOH 95/5 |
| 148 | 100 | 528 | 2.59 | Plaque silice prép 0.5 mm CH2Cl2/NH3 7N dans |
| | | | | MeOH 95/5 |
| 149 | 81 | 507 | 2.65 | |
| 150 | 95 | 475 | 2.67 | |

Le composé de l'exemple 145 peut être également caractérisé par le spectre de RMN suivant (400 MHz - δ en ppm - DMSO-d6) : 0,80 (s, 3H); 1,33 (s, 3H) ; 1,80 (m, 1H) ; 1,89 (m, 1H) ; 2,00 (s, 3H) ; 2,17 (dd, J = 8,5 et 14,0 Hz, 1H) ; 2,25 (dd, J = 7,5 et 14,0 Hz, 1H) ; 2,40 (m, 1H); 2,96 (m, 1H); 6,10 (d, J = 8,5 Hz, 1H) ; 7,22 (t, J = 7,5 Hz, 1H) ; 7,32 (t, J = 7,5 Hz, 1H) ; de 7,44 à 7,53 (m, 3H) ; de 7,59 à 7,69 (m, 3H) ; 8,37 (d, J = 5,5 Hz, 1H) ; 8,52 (d, J = 8,5 Hz, 1H) ; 9,02 (s, 1H) ; 13,4 (m très étalé, 1H).

### Exemple 151 : [4-(3H-imidazo[4,5-c]pyridin-2-yl)-9H-fluorèn-9(R,S)-yl]-amide de l'acide indole-3-carboxylique.

On opère comme à l'exemple 14 à partir de 298 mg de 4-(3H-imidazo[4,5-c]pyridin-2-yl)-fluorène-9(R,S)-amine, obtenue à l'exemple 6, et de 169 mg d'acide indole-3-carboxylique, en présence de 211 mg d'EDCI et de 149 mg d'HOBT dans 5 mL de DMF pendant 72 heures. Après purification par flash-chromatographie sur gel de silice (15-40µm), en éluant avec un mélange de méthanol-dichlorométhane (10/90 en volumes), on récupère 112 mg de [4-(3H-imidazo[4,5-c]pyridin-2-yi)-9H-fluorèn-9(R,S)-yl]-amide de l'acide indole-3-carboxylique sous forme d'une poudre beige dont les caractéristiques sont les suivantes :
Point de fusion (Kofler) = 210°C décomp.
Spectre RMN 1H (300 MHz, DMSO-d6) D en ppm : 6,43 (d, J = 8,5 Hz, 1H) ; de 7,12 à 7,27 (m, 3H) ; 7,33 (dt, J = 1,5 et 7,5 Hz, 1H) ; de 7,41 à 7,72 (m, 6H) ; 7,75 (d large, J = 7,5 Hz, 1H); 8,11 (d, J = 3,0 Hz, 1H) ; 8,31 (m, 1H) ; 8,40 (d, J = 5,5 Hz, 1H) ; 8,55 (d, J = 8,5 Hz, 1H) ; 9,05 (s large, 1H) ; 11,55 (m large, 1H) ; 13,35 (m large, 1H).
Spectre de masse (ES) : m/z =442 (MH+).

### Exemple 152 : Synthèse de l'énantiomère dextrogyre du [4-(3H-imidazo[4,5-c]pyridin-2-yl)-9H-fluorèn-9(R,S)-yl]-amide de l'acide 1H-pyrrolo[2,3-b]pyridine-4-carboxylique.

8 g de [4-(3H-imidazo[4,5-c]pyridin-2-yl)-9H-fluorèn-9(R,S)-yl]-amide de l'acide 1H-pyrrolo[2,3-b]pyridine-4-carboxylique, obtenue à l'exemple 92, sont purifiés, en phase supercritique, sur une colonne chiraie préparative de 5 cm de diamètre, contenant 350 g de silice Chiralcel 20 µm, en 20 injections successives. On élue, à un débit de 250 mL/mn, avec 100 bars de gaz carbonique en présence 30% de méthanol et 0,1% de triéthylamine comme co-solvant. En récupérant et concentrant sous pression réduite les premières fractions éluées, on obtient 3,59 g d'énantiomère dextrogyre, sous forme d'une poudre blanche dont les caractéristiques sont les suivantes :
Spectre RMN 1H (300 MHz ; DMSO-d6 ; □ en ppm) : 6,42 (d, J = 8,5 Hz, 1H) ; 6,90 (m, 1H) ; 7,25 (t, J = 7,5 Hz, 1H) ; 7,35 (t, J = 7,5 Hz, 1H) ; 7,47 (d, J = 5,0 Hz, 1H) ; de 7,50 à 7,57 (m, 2H) ; de 7,60 à 7,72 (m, 4H) ; 7,79 (d, J = 7,5 Hz, 1H) ; 8,29 (d, J = 5,0 Hz, 1H) ; 8,37 (d, J = 5,5 Hz, 1H) ; 9,04 (s large, 1H) ; 9,28 (d, J = 8,5 Hz, 1H) ; 11,85 (s large, 1H) ; 13,35 (m étalé, 1H).
□D20 = +136,5 +/- 2° (c = 0,507 ; DMSO).

### Exemple 153 : Synthèse de l'énantiomère lévogyre du [4-(3H-imidazo[4,5-c]pyridin-2-yl)-9H-fluorèn-9(R,S)-yl]-amide de l'acide 1H-pyrrolo[2,3-b]pyridine-4-carboxylique.

En opérant comme à l'exemple 153, mais en récupérant et concentrant sous pression réduite les secondes fractions éluées, on obtient 3,53 g d'énantiomère lévogyre dont les caractéristiques sont les suivantes :
□D20 = -117,5 +/- 2 ° (c = 0,5 ; DMSO).

### Exemple 154: Synthèse du N-[4-(3H-imidazo[4,5-c]pyridin-2-yl)-9H-fluorèn-9(R,S)-yl]-amide de l'acide 3-hydroxy-2-méthyl-quinoléïne-4 carboxylique.

On opère comme à l'exemple 14 à partir de 200 mg de 4-(3H-imidazo[4,5-c]pyridin-2-yl)-fluorène-9(R,S)-amine, obtenue à l'exemple 6, et de 143 mg d'acide 3-hydroxy-2-méthyl-quinoléïne-4-carboxylique, en présence de 135 mg d'EDCl et de 45 mg d'HOBT dans 2 mL de DMF pendant 20 heures. Après lavages successifs avec une solution aqueuse saturée d'hydrogénocarbonate de sodium puis avec de l'eau le mélange brut est purifié par flash-chromatographie sur gel de silice, en éluant avec un mélange de dichlorométhane et de méthanol (95-05 en volumes), on obtient 16 mg de N-[4-(3H-imidazo[4,5-c]pyridin-2-yl)-9H-fluorèn-9(R,S)-yl]-amide de l'acide 3-hydroxy-2-méthyl-quinoléïne-4 carboxylique, sous forme d'un solide jaune, dont les caractéristiques sont les suivantes :
Spectre de masse (LC/MS) : m/z = 483 (M+).
Point de fusion (Kofler) = 240°C.

### Exemple 155 : Synthèse du N-[4-(3H-Imidazo[4,5-c]pyridin-2-yl)-9H-fluorèn-9(R,S)-yl]-3,5-diméthoxy-benzamide.

On opère comme à l'exemple 14 à partir de 150 mg de 4-(3H-imidazo[4,5-c]pyridin-2-yl)-fluorène-9(R,S)-amine, obtenue à l'exemple 6, et de 92 mg d'acide 3,5-diméthoxy-benzoïque, en présence de 98 mg d'EDCl et de 34 mg d'HOBT dans 2 mL de DMF pendant 20 heures. Après lavages successifs avec une solution aqueuse saturée d'hydrogénocarbonate de sodium puis avec de l'eau le mélange brut est purifié par flash-chromatographie sur gel de silice, en éluant avec un mélange de dichlorométhane et de méthanol (95-05 en volumes), on obtient 18 mg de N-[4-(3H-Imidazo[4,5-c]pyridin-2-yl)-9H-fluorèn-9(R,S)-yl]-3,5-diméthoxy-benzamide, sous forme d'une meringue blanche, dont la caractéristique est la suivante :
Spectre de masse (E/I) : m/z = 462 (M+).

### Exemple 156: [4-(3H-imidazo[4,5-c]pyridin-2-yl)-9H-fluorèn-9(R,S)-yl]-amide de l'acide bromo-6-hydroxy-2-quinoléine-4-carboxylique.

On opère comme à l'exemple 14 à partir de 298 mg de 4-(3H-Imidazo[4,5-c]pyridin-2-yl)-fluorène-9(R,S)-amine, obtenue à l'exemple 6, et de 282 mg d'acide bromo-6-hydroxy-2-quinoleine-4-carboxylique en présence de 211 mg d'EDCl et de 149 mg d'HOBT dans 5 mL de DMF pendant 20 heures. Après évaporation à sec sous vide, on reprend le résidu avec 100 ml d'acétate d'éthyle et 50 ml d'une solution de bicarbonate de sodium saturée. Le mélange est filtré sur verre fritté et le solide sèché sous vide à 40°C. On obtient 513 mg du [4-(3H-imidazo[4,5-c]pyridin-2-yl)-9H-fluorèn-9(R,S)-yl]-amide de l'acide bromo-6-hydroxy-2-quinoléine-4-carboxylique, sous forme d'un solide beige dont les caractéristiques sont les suivantes :
Spectre de 1H - RMN (400 MHz, DMSO-d6) □ en ppm : 6,29 (d, J = 8,5 Hz, 1H) ; 6,72 (s, 1H) ; 7,22 (t large, J = 7,5 Hz, 1H) ; de 7,26 à 7,34 (m, 2H) ; 7,42 (m, 2H) ; 7,64 (d large, J = 8,0 Hz, 1H) ; 7,68 (m large, 1H) ; 7,73 (dd, J = 2,0 et 8,5 Hz, 1H) ; 7,84 (m large, 1H) ; 8,02 (m large, 1H) ; 8,09 (d, J = 2,0 Hz, 1H) ; 8,27 (m large, 1H) ; 8,79 (s large, 1H) ; 9,51 (d, J = 8,5 Hz, 1H) ; 12,2 (m étalé, 2H).
Spectre de masse (ES) : m/z = 548 (MH+).

### Exemple 157 : Synthèse du N-[4-(3H-imidazo[4,5-c]pyridin-2-yl)-9H-fluorèn-9(R,S)-yl]-amide de l'acide 9H-purine-6-carboxylique.

On opère comme à l'exemple 14 à partir de 300 mg de 4-(3H-imidazo[4,5-c]pyridin-2-yl)-fluorène-9(R,S)-amine, obtenue à l'exemple 6, et de 182 mg d'acide 9H-purine-6-carboxylique, en présence de 212 mg d'EDCl et de 70 mg d'HOBT dans 4 mL de DMF pendant 15 heures. Après lavages successifs avec une solution aqueuse saturée d'hydrogénocarbonate de sodium, avec de l'eau puis avec de l'éther isopropylique, on obtient 400 mg de N-[4-(3H-imidazo[4,5-c]pyridin-2-yl)-9H-fluorèn-9(R,S)-yl]-amide de l'acide 9H-purine-6-carboxylique, sous forme d'un solide blanc, dont les caractéristiques sont les suivantes :
Spectre de masse (E/I) : m/z = 444 (M+).

### Exemple 158 : Synthèse du N-[4-(3H-imidazo[4,5-c]pyridin-2-yl)-9H-fluorèn-9(R,S)-yl]-amide de l'acide 2-amino-6-méthyl-pyrimidine-4-carboxylique.

On opère comme à l'exemple 14 à partir de 300 mg de 4-(3H-imidazo[4,5-c]pyridin-2-yl)-fluorène-9(R,S)-amine, obtenue à l'exemple 6, et de 170 mg d'acide 2-amino-6-méthyl-pyrimidine-4-carboxylique, en présence de 212 mg d'EDCl et de 68 mg d'HOBT dans 4 mL de DMF pendant 4 heures. Après lavages successifs avec une solution aqueuse saturée d'hydrogénocarbonate de sodium puis avec de l'eau, le mélange brut est purifié par flash-chromatographie sur gel de silice, en éluant avec un mélange de dichlorométhane et d'une solution 7N de méthanol ammoniacal (95-05 en volumes), on obtient 50 mg de N-[4-(3H-imidazo[4,5-c]pyridin-2-yl)-9H-fluorèn-9(R,S)-yl]-amide de l'acide 2-amino-6-méthyl-pyrimidine-4-carboxylique sous forme d'un solide jaune pâle, dont les caractéristiques sont les suivantes :
Spectre de masse (E/I) : m/z = 433 (M+).
Point de fusion (Kofler) = 210°C.

### Exemple 159 Synthèse du 2-éthyl-6-hydroxy-N-[4-(3H-imidazo[4,5-c]pyridin-2-yl)-9H-fluorèn-9(R,S)-yl]-isonicotinamide.

On opère comme à l'exemple 14 à partir de 300 mg de 4-(3H-imidazo[4,5-c]pyridin-2-yl)-fluorène-9(R,S)-amine, obtenue à l'exemple 6, et de 185 mg d'acide 2-éthyl-6-hydroxy-isonicotinique, en présence de 212 mg d'EDCI et de 68 mg d'HOBT dans 7 mL de DMF pendant 20 heures. Après lavages successifs avec une solution aqueuse saturée d'hydrogénocarbonate de sodium, avec de l'eau puis avec de l'éther isopropylique, on obtient 330 mg de 2-éthyl-6-hydroxy-N-[4-(3H-imidazo[4,5-c]pyridin-2-yl)-9H-fluorèn-9(R,S)-yl]-isonicotinamide sous forme d'une poudre jaune pâle, dont les caractéristiques sont les suivantes :
Spectre de masse (LC/MS) : m/z = 447 (M+).
L'acide 2-éthyl-6-hydroxy-isonicotinique peut être obtenu selon EP542059.

### Exemple 160 : Synthèse du 3,5-dichloro-N-[4-(3H-imidazo[4,5-c]pyridin-2-yl)-9H-fluorèn-9(R,S)-yl]-isonicotinamide.

On opère comme à l'exemple 14 à partir de 300 mg de 4-(3H-imidazo[4,5-c]pyridin-2-yl)-fluorène-9(R,S)-amine, obtenue à l'exemple 6, et de 193 mg d'acide 3,5-dichloro-isonicotinique, en présence de 212 mg d'EDCl et de 68 mg d'HOBT dans 10 mL de DMF pendant 15 heures. Après lavages successifs avec une solution aqueuse saturée d'hydrogénocarbonate de sodium puis avec de l'eau, le mélange brut est purifié par flash-chromatographie sur gel de silice, en éluant avec un mélange de dichlorométhane et de méthanol (90-10 en volumes), on obtient 250 mg de 3,5-dichloro-N-[4-(3H-imidazo[4,5-c]pyridin-2-yl)-9H-fluorèn-9(R,S)-yl]-isonicotinamide sous forme d'un solide blanc, dont les caractéristiques sont les suivantes :
Spectre de masse (LC/MS) : m/z = 472 (M+).
Point de fusion (Kofler) = 240°C.

### Exemple 161 : Synthèse du [4-(3H-imidazo[4,5-c]pyridin-2-yl)-9H-fluorène-9(R,S)-yl]-amide de l'acide 5-amino-3H-1,2,3-triazole-4-carboxylique.

On opère comme dans l'exemple 14, mais à partir de 300 mg de 4-(3H-imidazo[4,5-c]pyridin-2-yl)-fluorène-9(R,S)-amine, obtenue à l'exemple 6, et de 135 mg d'acide 5-amino-3H-1,2,3-triazole-4-carboxylique dans 3 mL de diméthylformamide, en présence de 289 mg de chlorhydrate de 1-(3-diméthylaminopropyl)-3-éthylcarbodiimide (EDCI) et 204 mg de 1-hydroxybenzotriazole (HOBT), pendant 20 heures à température ambiante. Après purification par flash-chromatographie sur gel de silice (40-63µm), en éluant avec un mélange de dichlorométhane et de méthanol (9/1 puis 85/15 en volumes), on obtient 100 mg de [4-(3H-imidazo[4,5-c]pyridin-2-yl)-9H-fluorène-9(R,S)-yl]-amide de l'acide 5-amino-3H-1,2,3-triazole-4-carboxylique, sous forme d'une poudre jaune pâle dont les caractéristiques sont les suivantes :
Spectre de masse (LCMS) : m/z = 408 (M+).

### Exemple 162 : Synthèse du N-[4-(3H-imidazo[4,5-c]pyridin-2-yl)-9H-fluorèn-9(R,S)-yl]-amide de l'acide 6-méthyl-2-méthylamino-pyrimidine-4-carboxylique.

On opère comme à l'exemple 14 à partir de 90 mg de 4-(3H-imidazo[4,5-c]pyridin-2-yl)-fluorène-9(R,S)-amine, obtenue à l'exemple 6, et de 50 mg d'acide 6-méthyl-2-méthylamino-pyrimidine-4-carboxylique, en présence de 64 mg d'EDCl et de 20 mg d'HOBT dans 2 mL de DMF pendant 6 heures. Après lavages successifs avec une solution aqueuse saturée d'hydrogénocarbonate de sodium puis avec de l'eau le mélange brut est purifié par flash-chromatographie sur gel de silice, en éluant avec un mélange de dichlorométhane et de méthanol (90-10 en volumes), on obtient 35 mg de N-[4-(3H-imidazo[4,5-c]pyridin-2-yl)-9H-fluorèn-9(R,S)-yl]-amide de l'acide 6-méthyl-2-méthylamino-pyrimidine-4-carboxylique, sous forme d'une poudre blanche, dont les caractéristiques sont les suivantes :
Spectre de masse (LC/MS) : m/z = 447 (M+).
Point de fusion (Kofler) = 196°C.
L'acide 6-méthyl-2-méthylamino-pyrimidine-4-carboxylique, peut être obtenu par hydrolyse de l'ester méthylique correspondant selon Helvetica Chimica Acta, 81(2), 231-235 ; 1998.

### Exemple 163 Synthèse du N-[4-(3H-imidazo[4,5-c]pyridin-2-yl)-9H-fluorèn-9(R,S)-yl]-amide de l'acide 5-amino-2,6-dihydroxy-pyrimidine-4-carboxylique.

On opère comme à l'exemple 14 à partir de 300 mg de 4-(3H-imidazo[4,5-c]pyridin-2-yl)-fluorène-9(R,S)-amine, obtenue à l'exemple 6, et de 172 mg d'acide 5-amino-2,6-dihydroxy-pyrimidine-4-carboxylique, en présence de 212 mg d'EDCl et de 68 mg d'HOBT dans 15 mL de DMF pendant 15 heures. Après lavages successifs avec une solution aqueuse saturée d'hydrogénocarbonate de sodium puis avec de l'eau le mélange brut est purifié par flash-chromatographie sur gel de silice, en éluant avec un mélange de dichlorométhane et de méthanol (90-10 en volumes), on obtient 210 mg de N-[4-(3H-imidazo[4,5-c]pyridin-2-yl)-9H-fluorèn-9(R,S)-yl]-amide de l'acide 5-amino-2,6-dihydroxy-pyrimidine-4-carboxylique sous forme d'une poudre blanche, dont les caractéristiques sont les suivantes :
Spectre de masse (LC/MS) : m/z = 451 (M+).

### Exemple 164 : Synthèse du N-[4-(9H-Purin-8-yl)-9H-fluorène-9(R,S)-yl]-amide de l'acide isonicotinique.

Etape 1 : On opère comme à l'étape 1 de l'exemple 1, à partir de 2 g de 4,5-diaminopyrimidine dans 150 mL de dichlorométhane, 3,4 mL de triéthylamine et 3 g de chlorure de l'acide fluorène-4-one-9-carboxylique. Après 20 heures d'agitation à température ambiante, le milieu réactionnel est versé sur une solution aqueuse saturée d'hydrogénocarbonate de sodium et extrait au dichlorométhane. Les phases organiques sont lavées avec de l'eau, séchées sur sulfate de magnésium et concentrées sous pression réduite. Après purification par flash-chromatographie sur gel de silice (40-63µm), en éluant par un mélange de dichlorométhane et de méthanol (98/2 puis 95/5 en volumes), on obtient 1,1 g du (5-amino-pyrimidin-4-yl)amide de l'acide 9-oxo-9H-fluorène-4-carboxylique, utilisé tel quel à l'étape suivante, dont les caractéristiques sont les suivantes :
Spectre de masse (E/I) : m/z = 316 (M+)

Etape 2 : Dans un ballon de 250 mL sous atmosphère d'argon, on chauffe, à 95°C pendant 5 heures, une solution de 1,1 g de (5-amino-pyrimidin-4-yl)amide de l'acide 9-oxo-9H-fluorène-4-carboxylique, obtenu à l'étape précédente, dans 55 mL d'acide acétique. Après refroidissement, on amène à sec sous pression réduite. On purifie par flash-chromatographie sur gel de silice (40-63µm), en éluant par un mélange de dichlorométhane et de méthanol (98/2 en volumes), puis empâtage dans l'éther isopropylique, on obtient ainsi 565 mg de 4-(9H-purin-8-yl)-fluorène-9-one, sous forme d'une poudre jaune pâle utilisée telle quelle à l'étape suivante, dont les caractéristiques sont les suivantes :
Spectre de masse (E/I) : m/z = 298 (M+).
Spectre RMN 1H (400 MHz, □ en ppm, DMSO-d6) : 7,42 (t, J = 7,5 Hz, 1H) ; 7,50 (dt, J = 1,0 et 7,5 Hz, 1H) ; 7,61 (t, J = 7,5 Hz, 1H) ; 7,67 (d, J = 7,5 Hz, 1H) ; 7,70 (d, J = 7,5 Hz, 1H) ; 7,86 (d, J = 8,0 Hz, 1H) ; 7,92 (d, J = 7,5 Hz, 1H) ; 9,00 (s, 1H) ; 9,25 (s, 1H) ; 14,5 (s large, 1H).

Etape 3 : On opère comme à l'exemple 5, à partir de 564 mg de 4-(9H-purin-8-yl)-fluorène-9-one, obtenue à l'étape précédente, de 394 mg de chlorhydrate d'hydroxylamine et de 776 mg d'acétate de sodium dans 12 mL d'éthanol pendant 20h à température ambiante. Après concentration du solvant sous pression réduite, le résidu est repris successivement par de l'eau, puis du toluène et enfin empâté dans de l'oxyde diisopropyle, on obtient ainsi 502 mg de 4-(9H-purin-8-yl)-fluorène-9-one oxime (Z,E), en mélange 50-50 des isomères Z et E, sous forme d'une poudre blanc cassé, dont les caractéristiques sont les suivantes :
Spectre de masse (LCMS) : m/z = 313 (M+).

Etape 4 : On opère, comme dans l'exemple 6, en autoclave, à partir de 450 mg de 4-(9H-purin-8-yl)-fluorène-9-one oxime (Z,E), obtenue à l'étape précédente, en solution dans un mélange de 25 mL d'éthanol et 25 mL de tétrahydrofurane, en présence de Nickel activé selon Raney, sous une pression initiale d'hydrogène de 1 bar, à 60° pendant 20 heures. Après filtration du catalyseur sur Célite, concentration du filtrat sous pression réduite on obtient ainsi 384 mg de 4-(9H-purin-8-yl)-9H-fluorène-9(R,S)-amine, sous forme d'une poudre beige, dont les caractéristiques sont les suivantes :
Spectre de masse (El) : m/z = 299 (M+).
Spectre RMN 1H (400 MHz, □en ppm, DMSO-d6) : 4,83 (s, 1H) ; 7,17 (t, J = 7,5 Hz, 1H) ; 7,31 (t, J = 7,5 Hz, 1H) ; 7,48 (t, J = 7,5 Hz, 1H) ; 7,57 (d large, J = 8,0 Hz, 1H) ; 7,65 (d, J = 7,5 Hz, 1H) ; 7,69 (d, J = 7,5 Hz, 1H) ; 7,86 (d, J = 7,5 Hz, 1H) ; 8,90 (s, 1H) ; 9,11 (s, 1H).

Etape 5 : On opère comme dans l'exemple 14, à partir de 300 mg de 4-(9H-purin-8-yl)-9H-fluorène-9(R,S)-amine, obtenue à l'étape précédente, et de 129 mg d'acide isonicotinique dans 3 mL de diméthylformamide, en présence de 288 mg de chlorhydrate de 1-(3-diméthylaminopropyl)-3-éthylcarbodiimide (EDCI) et 204 mg de 1-hydroxybenzotriazole (HOBT) pendant 20 heures à température ambiante. Après purification par flash-chromatographie sur gel de silice (40-63µm) en éluant avec un mélange de dichlorométhane et de méthanol (95/5 puis 9/1 en volumes), on obtient 230 mg de N-[4-(9H-purin-8-yl)-9H-fluorène-9(R,S)-yl]-amide de l'acide isonicotinique, sous forme d'une poudre beige dont les caractéristiques sont les suivantes :
Spectre de masse (EI/LCMS) : m/z = 404 (M+).
Spectre RMN 1H (400 MHz, □ en ppm, DMSO-d6) : 6,37 (d, J = 8,5 Hz, 1H) ; 7,28 (t, J = 7,5 Hz, 1H) ; 7,36 (t, J = 7,5 Hz, 1H) ; 7,54 (t, J = 7,5 Hz, 1H) ; 7,60 (m, 2H) ; 7,72 (d, J = 7,5 Hz, 1H) ; 7,77 (d, J = 7,5 Hz, 1H) ; 7,88 (d large, J = 6,0 Hz, 2H) ; 8,75 (d large, J = 6,0 Hz, 2H) ; 9,00 (s, 1H) ; 9,22 (s large, 1H) ; 9,48 (d, J = 8,5 Hz, 1H) ; 13,95 (m étalé, 1H).

### Exemple 165: Synthèse du N-[4-(3H-imidazo[4,5-c]pyridin-2-yl)-9H-fluorèn-9(R,S)-yl]-amide de l'acide 3-amino-quinoléine-4-carboxylique.

On opère comme à l'exemple 14 à partir de 300 mg de 4-(3H-imidazo[4,5-c]pyridin-2-yl)-fluorène-9(R,S)-amine, obtenue à l'exemple 6, et de 189 mg d'acide 3-amino-quinoléine-4-carboxylique, en présence de 212 mg d'EDCl et de 68 mg d'HOBT dans 7 mL de DMF pendant 15 heures. Après lavages successifs avec une solution aqueuse saturée d'hydrogénocarbonate de sodium puis avec de l'eau le mélange brut est purifié par flash-chromatographie sur gel de silice, en éluant avec un mélange de dichlorométhane et d'une solution 7N de méthanol ammoniacal (95-05 en volumes), on obtient 230 mg de N-[4-(3H-imidazo[4,5-c]pyridin-2-yl)-9H-fluorèn-9(R,S)-yl]-amide de l'acide 3-amino-quinoléine-4-carboxylique sous forme d'une poudre blanche, dont les caractéristiques sont les suivantes :
Spectre de masse (E/I) : m/z = 468 (M+).
Point de fusion (Kofler) = 234°C.

L'acide 3-amino-quinoléine-4-carboxylique peut être obtenu selon Journal of Heterocyclic Chemistry, 17 (3), 465-73 ; 1980.

### Exemple 166: Synthèse du N-[4-(3H-imidazo[4,5-c]pyridin-2-yl)-9H-fluorèn-9(R,S)-yl]-amide de l'acide 6-méthoxy-quinoléine-4-carboxylique.

On opère comme à l'exemple 14 à partir de 300 mg de 4-(3H-imidazo[4,5-c]pyridin-2-yl)-fluorène-9(R,S)-amine, obtenue à l'exemple 6, et de 300 mg d'acide 6-méthoxy-quinoléine-4-carboxylique, en présence de 422 mg d'EDCI et de 297 mg d'HOBT dans 7 mL de DMF pendant 15 heures. Après lavages successifs avec une solution aqueuse saturée d'hydrogénocarbonate de sodium puis avec de l'eau le mélange brut est purifié par flash-chromatographie sur gel de silice, en éluant avec un mélange de dichlorométhane et d'une solution 7N de méthanol ammoniacal (95-05 en volumes), on obtient 180 mg de N-[4-(3H-imidazo[4,5-c]pyridin-2-yl)-9H-fluorèn-9(R,S)-yl]-amide de l'acide 6-méthoxy-quinoléine-4-carboxylique sous forme d'un solide jaune, dont les caractéristiques sont les suivantes :
Spectre de masse (E/I) : m/z = 483 (M+).

### Exemple 167 et 172: Synthèse du N-[4-(3H-imidazo[4,5-c]pyridin-2-yl)-9H-fiuorèn-9(R,S)-yl]-amide de l'acide 3,4-diamino-benzoïque et du N-[4-(3H-imidazo[4,5-c]pyridin-2-yl)-9H-fluorèn-9(R,S)-yl]-amide de l'acide 4-amino-3-hydroxy-benzoïque.

Etape 1 : On opère comme à l'exemple 14 à partir de 600 mg de 4-(3H-imidazo[4,5-c]pyridin-2-yl)-fluorène-9(R,S)-amine, obtenue à l'exemple 6, et de 424 mg d'acide 3,4-dinitro-benzoïque, en présence de 422 mg d'EDCl et de 300 mg d'HOBT dans 14 mL de DMF pendant 15 heures. Après lavages successifs avec une solution aqueuse saturée d'hydrogénocarbonate de sodium puis avec de l'eau le mélange brut est purifié par flash-chromatographie sur gel de silice, en éluant avec un mélange de dichlorométhane et d'une solution 7N de méthanol ammoniacal (95-05 en volumes), on obtient 215 mg de N-[4-(3H-imidazo[4,5-c]pyridin-2-yl)-9H-fluorèn-9(R,S)-yl]-amide de l'acide 3,4-dinitro-benzoïque sous forme d'un solide jaune, dont les caractéristiques sont les suivantes :
Spectre de masse (E/I) : m/z = 492 (M+).

Etape 2 : Dans un autoclave de 45 mL, on dissout 365 mg de N-[4-(3H-imidazo[4,5-c]pyridin-2-yl)-9H-fluorèn-9(R,S)-yl]-amide de l'acide 3,4-dinitro-benzoïque obtenue à l'étape 1, dans un mélange de 60 mL d'éthanol, on ajoute 78 mg de Palladium sur charbon à 10% puis on soumet à une pression initiale d'hydrogène de 1 bar pendant 20 heures. Après refroidissement, le volume d'hydrogène absorbé est de 100 mL. Après des purges à l'argon, l'autoclave est ouvert, on filtre sur célite et rince à l'éthanol. Le filtrat est concentré sous pression réduite et purifié par flash-chromatographie sur gel de silice, en éluant avec un mélange de dichlorométhane et d'une solution 7N de méthanol ammoniacal (95-05 en volumes), on obtient :
- d'une part 70 mg de N-[4-(3H-imidazo[4,5-c]pyridin-2-yl)-9H-fluorèn-9(R,S)-yl]-amide de l'acide 3,4-diamino-benzoïque sous forme d'une meringue jaune, dont les caractéristiques sont les suivantes :
   Spectre de masse (E/I) : m/z = 432 (M+).
- et d'autre part 150 mg de N-[4-(3H-imidazo[4,5-c]pyridin-2-yl)-9H-fluorèn-9(R,S)-yl]-amide de l'acide 4-amino-3-hydroxy-benzoïque sous forme d'une meringue jaune, dont les caractéristiques sont les suivantes :
   Spectre de masse (E/I) : m/z = 433 (M+).

### Exemple 168: Synthèse du 3,5-dihydroxy-N-[4-(3H-imidazo[4,5-c]pyridin-2-yl)-9H-fluorèn-9(R,S)-yl]-benzamide.

On opère comme à l'exemple 14 à partir de 300 mg de 4-(3H-imidazo[4,5-c]pyridin-2-yl)-fluorène-9(R,S)-amine, obtenue à l'exemple 6, et de 308 mg d'acide 3,5-dihydroxy-benzoïque, en présence de 422 mg d'EDCl et de 297 mg d'HOBT dans 7 mL de DMF pendant 20 heures. Après lavages successifs avec une solution aqueuse saturée d'hydrogénocarbonate de sodium puis avec de l'eau le mélange brut est purifié par HPLC préparative sur colonne Kromasil C8, en éluant avec un mélange de d'eau additionné de 0,1% d'acide trifluoroacétique et de méthanol (80-20 en volumes), on obtient 170 mg de 3,5-dihydroxy-N-[4-(3H-imidazo[4,5-c]pyridin-2-yl)-9H-fluorèn-9(R,S)-yl]-benzamide sous forme d'une meringue jaune pâle, dont les caractéristiques sont les suivantes :
Spectre de masse (E/I) : m/z = 483 (M+).

### Exemple 169 : Synthèse du [4-(3H-imidazo[4,5-c]pyridin-2-yl)-9H-fluorène-9(R,S)-yl]-amide de l'acide 1H-Imidazole-4-carboxylique.

On opère comme dans l'exemple 14 , à partir de 300 mg de 4-(3H-imidazo[4,5-c]pyridin-2-yl)-fluorène-9(R,S)-amine, obtenue à l'exemple 6, et de 118 mg d'acide 1H-Imidazole-4-carboxylique dans 3 mL de diméthylformamide, en présence de 289 mg de chlorhydrate de 1-(3-diméthylaminopropyl)-3-éthylcarbodiimide (EDCI) et 204 mg de 1-hydroxybenzotriazole (HOBT), pendant 20 heures à température ambiante. Après purification par flash-chromatographie sur gel de silice (40-63µm), en éluant avec un mélange de dichlorométhane et d'ammoniac 7N dans le méthanol (95/5 puis 9/1 en volumes), on obtient 52 mg de [4-(3H-imidazo[4,5-c]pyridin-2-yl)-9H-fluoren-9(R,S)-yl]-amide de l'acide 1H-imidazole-4-carboxylique, sous forme d'une poudre beige dont les caractéristiques sont les suivantes :
Spectre de masse (EI/LCMS) : m/z = 392 (M+).

### Exemple 170 : Synthèse du [4-(3H-imidazo[4,5-c]pyridin-2-yl)-9H-fluorène-9(R,S)-yl]-amide de l'acide 5-amino-2H-1,2,4-triazole-3-carboxylique.

On opère comme dans l'exemple 14, à partir de 300 mg de 4-(3H-imidazo[4,5-c]pyridin-2-yl)-fluorène-9(R,S)-amine, obtenue à l'exemple 6, et de 144 mg d'acide 5-amino-2H-1,2,4-triazole-3-carboxylique dans 5 mL de diméthylformamide, en présence de 289 mg de chlorhydrate de 1-(3-diméthylaminopropyl)-3-éthylcarbodiimide (EDCI) et 204 mg de 1-hydroxybenzotriazole (HOBT), pendant 20 heures à température ambiante. Après purification par flash-chromatographie sur gel de silice (40-63µm), en éluant avec un mélange de dichlorométhane et de méthanol (9/1 puis 8/2 en volumes), on obtient 124 mg de [4-(3H-imidazo[4,5-c]pyridin-2-yl)-9H-fluorène-9(R,S)-yl]-amide de l'acide 5-amino-2H-1,2,4-triazole-3-carboxylique, sous forme d'une poudre blanc cassé dont les caractéristiques sont les suivantes :
Spectre de masse (El/Cl): m/z = 408 (M+).

### Exemple 171 : Synthèse du N-[4-(3H-imidazo[4,5-c]pyridin-2-yl)-9H-fluorèn-9(R,S)-yl]-amide de l'acide 2-méthyl-benzoxazole-4-carboxylique.

On opère comme à l'exemple 14 à partir de 300 mg de 4-(3H-imidazo[4,5-c]pyridin-2-yl)-fluorène-9(R,S)-amine, obtenue à l'exemple 6, et de 178 mg d'acide 2-méthyl-benzoxazole-4-carboxylique, en présence de 212 mg d'EDCI et de 68 mg d'HOBT dans 7 mL de DMF pendant 20 heures. Après lavages successifs avec une solution aqueuse saturée d'hydrogénocarbonate de sodium, avec de l'eau puis avec de l'éther isopropylique, on obtient 310 mg de N-[4-(3H-imidazo[4,5-c]pyridin-2-yl)-9H-fluorèn-9(R,S)-yl]-amide de l'acide 2-méthyl-benzoxazole-4-carboxylique sous forme d'une poudre grise, dont les caractéristiques sont les suivantes :
Spectre de masse (LC/MS) : m/z = 457 (M+).
Point de fusion (Kofler) = 200°C.

### Exemple 172 : Synthèse du [4-(3H-imidazo[4,5-c]pyridin-2-yl)-9H-fluorène-9(R,S)-yl]-amide de l'acide 4-amino-3-hydroxy-benzoique.

On opère comme dans l'exemple 14, à partir de 300 mg de 4-(3H-imidazo[4,5-c]pyridin-2-yl)-fluorène-9(R,S)-amine, obtenue à l'exemple 6, et de 153 mg d'acide 4-amino-3-hydroxy-benzoique dans 5 mL de diméthylformamide, en présence de 289 mg de chlorhydrate de 1-(3-diméthylaminopropyl)-3-éthylcarbodümide (EDCl) et 204 mg de 1-hydroxybenzotriazole (HOBT), pendant 20 heures à température ambiante. Après purification par flash-chromatographie sur gel de silice (40-63µm), en éluant avec un mélange de dichlorométhane et de méthanol (8/2 puis 5/5 en volumes), on obtient 100 mg de [4-(3H-imidazo[4,5-c]pyridin-2-yl)-9H-fluorène-9(R,S)-yl]-amide de l'acide 4-amino-3-hydroxy-benzoique, sous forme d'une poudre blanc cassé dont les caractéristiques sont les suivantes :
Spectre de masse (El/Cl) : m/z = 433 (M+).

### Exemple 173 Synthèse du 2-carbamoyl-N-[4-(3H-imidazo[4,5-c]pyridin-2-yl)-9H-fluorèn-9(R,S)-yl]-isonicotinamide.

On opère comme à l'exemple 14 à partir de 300 mg de 4-(3H-imidazo[4,5-c]pyridin-2-yl)-fluorène-9(R,S)-amine, obtenue à l'exemple 6, et de 175 mg d'acide carbamoyl-2-isonicotinique, en présence de 210 mg d'EDCI et de 148 mg d'HOBT dans 7 mL de DMF pendant 20 heures. Après lavages successifs avec une solution aqueuse saturée d'hydrogénocarbonate de sodium, avec de l'eau, puis avec de l'isopropanol, on obtient 160 mg de 2-carbamoyl-N-[4-(3H-imidazo[4,5-c]pyridin-2-yl)-9H-fluorèn-9(R,S)-yl]-isonicotinamide sous forme d'un solide marron, dont les caractéristiques sont les suivantes :
Spectre de masse (E/I) : m/z = 446 (M+).

### Exemple 174 : Synthèse du N-[4-(3H-imidazo[4,5-c]pyridin-2-yl)-9H-fluorèn-9(R,S)-yl]-amide de l'acide pyridine-3-méthyl ester 3,4-dicarboxylique.

On opère comme à l'exemple 14 à partir de 300 mg de 4-(3H-imidazo[4,5-c]pyridin-2-yl)-fluorène-9(R,S)-amine, obtenue à l'exemple 6, et de 183 mg d'acide pyridine-3-méthyl ester 3,4-dicarboxylique, en présence de 212 mg d'EDCI et de 68 mg d'HOBT dans 7 mL de DMF pendant 20 heures. Après lavages successifs avec une solution aqueuse saturée d'hydrogénocarbonate de sodium, avec de l'eau, de l'acétate d'éthyle puis avec de l'éther isopropylique, on obtient 110 mg de N-[4-(3H-imidazo[4,5-c]pyridin-2-yl)-9H-fluorèn-9(R,S)-yl]-amide de l'acide pyridine-3-méthyl ester 3,4-dicarboxylique sous forme d'un solide blanc, dont les caractéristiques sont les suivantes :
Spectre de masse (E/I) : m/z = 461 (M+).
Point de fusion (Kofler) = 196°C.

### Exemple 175 : Synthèse du [4-(3H-imidazo[4,5-c]pyridin-2-yl)-9H-fluorène-9(R,S)-yl]-amide de l'acide 5-hydroxy-4H-1,2,4-triazole-3-carboxylique.

On opère comme dans l'exemple 14, à partir de 330 mg de 4-(3H-imidazo[4,5-c]pyridin-2-yl)-fluorène-9(R,S)-amine, obtenue à l'exemple 6, et 150 mg d'acide 5-hydroxy-4H-1,2,4-triazole-3- carboxylique dans 3,3 mL de diméthylformamide, en présence de 318 mg de chlorhydrate de 1-(3-diméthylaminopropyl)-3-éthylcarbodiimide (EDCI) et 224 mg de 1-hydroxybenzotriazole (HOBT), pendant 20 heures à température ambiante. Après purification par flash-chromatographie sur gel de silice (40-63µm), en éluant avec un mélange de dichlorométhane et de méthanol (9/1 en volumes), on obtient 164 mg de [4-(3H-imidazo[4,5-c]pyridin-2-yl)-9H-fluorène-9(R,S)-yl]-amide de l'acide 5-hydroxy-4H-1,2,4-triazole-3-carboxylique, sous forme d'une poudre blanche dont les caractéristiques sont les suivantes :
Spectre de masse (EI) : m/z = 409 (M+).

### Exemple 176 Synthèse du N-[4-(3H-imidazo[4,5-c]pyridin-2-yl)-9H-fluorèn-9(R,S)-yl]-amide de l'acide pyrimidine-4-carboxylique.

On opère comme à l'exemple 14 à partir de 300 mg de 4-(3H-imidazo[4,5-c]pyridin-2-yl)-fluorène-9(R,S)-amine, obtenue à l'exemple 6, et de 126 mg d'acide pyrimidine-4-carboxylique, en présence de 212 mg d'EDCl et de 68 mg d'HOBT dans 7 mL de DMF pendant 6 heures. Après lavages successifs avec une solution aqueuse saturée d'hydrogénocarbonate de sodium, avec de l'eau, puis avec de l'éther isopropylique, on obtient 90 mg de N-[4-(3H-imidazo[4,5-c]pyridin-2-yl)-9H-fluorèn-9(R,S)-yl]-amide de l'acide pyrimidine-4-carboxylique sous forme d'un solide marron, dont les caractéristiques sont les suivantes :
Spectre de masse (E/I) : m/z = 404 (M+).
Point de fusion (Kofler) = 192°C.

### Exemple 177: Synthèse du N-[4-(3H-imidazo[4,5-c]pyridin-2-yl)-9H-fluorèn-9(R,S)-yl]-amide de l'acide 1,3-dioxo-2,3-dihydro-1H-isoindole-5-carboxylique.

On opère comme à l'exemple 14 à partir de 300 mg de 4-(3H-imidazo[4,5-c]pyridin-2-yl)-fluorène-9(R,S)-amine, obtenue à l'exemple 6, et de 192 mg d'acide 1,3-dioxo-2,3-dihydro-1H-isoindole-5-carboxylique, en présence de 212 mg d'EDCI et de 150 mg d'HOBT dans 7 mL de DMF pendant 20 heures. Après lavages successifs avec une solution aqueuse saturée d'hydrogénocarbonate de sodium, avec de l'eau, avec du méthanol puis avec de l'éther isopropylique, on obtient 122 mg de N-[4-(3H-imidazo[4,5-c]pyridin-2-yl)-9H-fluorèn-9(R,S)-yl]-amide de l'acide 1,3-dioxo-2,3-dihydro-1H-isoindole-5-carboxylique sous forme d'une poudre jaune, dont les caractéristiques sont les suivantes :
Spectre de masse (E/I) : m/z = 471 (M+).
Point de fusion (Kofler) = 258°C.

### Exemple 178: Synthèse du 4-hydroxy-N-[4-(3H-imidazo[4,5-c]pyridin-2-yl)-9H-fluorèn-9(R,S)-yl]-benzamide.

On opère comme à l'exemple 14 à partir de 300 mg de 4-(3H-imidazo[4,5-c]pyridin-2-yl)-fluorène-9(R,S)-amine, obtenue à l'exemple 6, et de 139 mg d'acide 4-hydroxy-benzoïque, en présence de 212 mg d'EDCI et de 150 mg d'HOBT dans 7 mL de DMF pendant 20 heures. Après lavages successifs avec une solution aqueuse saturée d'hydrogénocarbonate de sodium puis avec de l'eau le mélange brut est purifié par flash-chromatographie sur gel de silice, en éluant avec un mélange de dichlorométhane et d'une solution 7N de méthanol ammoniacal (90-10 en volumes), on obtient 283 mg de 4-hydroxy-N-[4-(3H-imidazo[4,5-c]pyridin-2-yl)-9H-fluorèn-9(R,S)-yl]-benzamide sous forme d'un solide jaune, dont les caractéristiques sont les suivantes :
Spectre de masse (E/I) : m/z = 418 (M+)

### Exemple 179: Synthèse du 2,4-dihydroxy-N-[4-(3H-imidazo[4,5-c]pyridin-2-yl)-9H-fluorèn-9(R,S)-yl]-benzamide.

On opère comme à l'exemple 14 à partir de 300 mg de 4-(3H-imidazo[4,5-c]pyridin-2-yl)-fluorène-9(R,S)-amine, obtenue à l'exemple 6, et de 155 mg d'acide 2,4-dihydroxy-benzoïque, en présence de 212 mg d'EDCI et de 150 mg d'HOBT dans 7 mL de DMF pendant 20 heures. Après lavages successifs avec une solution aqueuse saturée d'hydrogénocarbonate de sodium puis avec de l'eau le mélange brut est purifié par flash-chromatographie sur gel de silice, en éluant avec un mélange de dichlorométhane et d'une solution 7N de méthanol ammoniacal (90-10 en volumes), on obtient 168 mg de 2,4-dihydroxy-N-[4-(3H-imidazo[4,5-c]pyridin-2-yl)-9H-fluorèn-9(R,S)-yl]-benzamide sous forme d'un solide jaune, dont les caractéristiques sont les suivantes :
Spectre de masse (E/I) : m/z = 434 (M+).

### Exemple 180: [4-(3H-imidazo[4,5-c]pyridin-2-yl)-9H-fluorèn-9(R,S)-yl]-amide de l'acide 6-chloro-1H-pyrrolo[2,3-b]pyridine-3-carboxylique.

Etape 1 : A une suspension de 5.2 g de chlorure d'aluminium dans 4.5 mL de dichlorométhane anhydre, on additionne 2.9 mL de chlorure de trichloroacétyle. Le mélange est agité pendant 30 minutes à température ambiante sous argon, puis on ajoute 2.0 g de 6-chloro-1H-pyrrolo[2,3-b]pyridine, qui peut être préparée selon Synthesis 1992, 661, par petites fractions puis on chauffe au reflux pendant 3 heures. Après refroidissement à température ambiante, le mélange réactionnel est coulé sur 80 g de glace. Le solide pâteux est récupéré par décantation, repris par 15 mL d'eau et 8 mL de soude à 35% puis chauffé à 90°C pendant 15 minutes. Après refroidissement le milieu réactionnel est filtré, le filtrat acidifié par de l'acide chlorhydrique concentré, le solide filtré et sèché à l'air. On obtient ainsi un solide beige contenant 50% d'acide 6-chloro-1H-pyrrolo[2,3-b]pyridine-3-carboxylique utilisé tel quel à l'étape suivante et dont les caractéristiques sont les suivantes :
Spectre de RMN 1H (400 MHz - DMSO-d6) □ en ppm : 7,28 (d, J = 8,5 Hz, 1H) ; 8,16 (d, J = 3,0 Hz, 1H) ; 8,31 (d, J = 8,5 Hz, 1H) ; 12,35 (m étalé, 1H) ; 12,6 (s large, 1H.
Spectre de masse (ES) : m/z = 197 (MH+).

Etape 2 : On opère comme à l'exemple 14 à partir de 298 mg de 4-(3H-Imidazo[4,5-c]pyridin-2-yl)-fluorène-9(R,S)-amine, obtenue à l'exemple 6, et de 216 mg d'acide 6-chloro-1H-pyrrolo[2,3-b]pyridine-3-carboxylique brut préparé précédemment, en présence de 211 mg d'EDCl et de 149 mg d'HOBT, dans 15 mL de DMF pendant 42 heures. Le milieu réactionnel est versé sur 75 mL d'eau puis extrait avec 3 fois 35 mL d'un mélange de dichlorométhane et de méthanol (90/10 en volumes). Les phases organiques sont lavées avec 35 mL d'eau distillée, 35 mL d'une solution saturée de bicarbonate de sodium enfin 35 mL d'eau, sèchées sur sulfate de sodium et évaporées à sec sous vide. Le résidu est chromatographié sur gel de silice en éluant avec un mélange de dichlorométhane et de méthanol (90/10 en volumes). Après évaporation à sec sous vide, on triture le résidu avec 3.5 ml d'éther éthylique et le solide est filtré et sèché sous vide à 40°C. On obtient 200 mg de [4-(3H-imidazo[4,5-c]pyridin-2-yl)-9H-fluorèn-9(R,S)-yl]-amide de l'acide 6-chloro-1H-pyrrolo[2,3-b]pyridine-3-carboxylique, sous forme d'un solide beige dont les caractéristiques sont les suivantes :
Spectre de RMN 1H (400 MHz - DMSO-d6) □ en ppm : 6,41 (d, J = 8,5 Hz, 1H) ; 7,25 (t, J = 7,5 Hz, 1H) ; 7,31 (d, J = 8,5 Hz, 1H) ; 7,33 (t partiellement masqué, J = 7,5 Hz, 1H) ; 7,50 (d partiellement masqué, J = 7,5 Hz, 1H) ; 7,52 (t, J = 7,5 Hz, 1H) ; 7,59 (d, J = 7,5 Hz, 1H) ; 7,63 (m étalé, 1H) ; 7,69 (d, J = 7,5 Hz, 1H) ; 7,75 (d, J = 7,5 Hz, 1H) ; 8,26 (d, J = 3,0 Hz, 1H) ; 8,40 (d large, J = 5,5 Hz, 1H) ; 8,61 (d, J = 8,5 Hz, 1H) ; 8,78 (d, J = 8,5 Hz, 1H) ; 9,07 (m étalé, 1H) ; 12,35 (d, J = 3,0 Hz, 1H) ; 13,45 (m étalé, 1H).
Spectre de masse (ES) : m/z = 477 (MH+).

### Exemple 181 : Synthèse du [4-(3H-imidazo[4,5-c]pyridin-2-yl)-9H-fluorène-9(R,S)-yl]-amide de l'acide 3-benzyl-isoxazole-5-carboxylique.

On opère comme dans l'exemple 14, à partir de 280 mg de 4-(3H-imidazo[4,5-c]pyridin-2-yl)-fluorène-9(R,S)-amine, obtenue à l'exemple 6, et 200 mg d'acide 3-benzyl-isoxazole-5-carboxylique dans 3 mL de diméthylformamide, en présence de 270 mg de chlorhydrate de 1-(3-diméthylaminopropyl)-3-éthylcarbodümide (EDCl) et 190 mg de 1-hydroxybenzotriazole (HOBT), pendant 20 heures à température ambiante. Après purification par flash-chromatographie sur gel de silice (40-63µm), en éluant avec un mélange de dichlorométhane et de méthanol (95/5 en volumes), on obtient 235 mg de [4-(3H-imidazo[4,5-c]pyridin-2-yl)-9H-fluorène-9(R,S)-yl]-amide de l'acide 3-benzyl-isoxazole-5-carboxylique, sous forme d'une poudre blanche dont les caractéristiques sont les suivantes :
Point de fusion (Kofler) = 192-194°C.
Spectre de masse (LCMS) : m/z = 485 (M+).

### Exemple 182 : Synthèse du [4-(3H-imidazo[4,5-c]pyridin-2-yl)-9H-fluorène-9(R,S)-yl]-amide de l'acide 5-(4-hydroxy-phenyl)-3H-1,2,3-triazole-4-carboxylique.

On opère comme dans l'exemple 14, à partir 280 mg de 4-(3H-lmidazo[4,5-c]pyridin-2-yl)-fluorène-9(R,S)-amine, obtenue à l'exemple 6, et 200 mg d'acide 5-(4-hydroxy-phenyl)-3H-1,2,3-triazole-4-carboxylique dans 3 mL de diméthylformamide, en présence de 270 mg de chlorhydrate de 1-(3-diméthylaminopropyl)-3-éthylcarbodümide (EDCI) et 190 mg de 1-hydroxybenzotriazole (HOBT), pendant 20 heures à température ambiante. Après purification par flash-chromatographie sur gel de silice (40-63µm), en éluant avec un mélange de dichlorométhane et de méthanol (95/5 puis 9/1 en volumes) puis un mélange de dichlorométhane et d'ammoniac à 7N dans le méthanol (9/1en volumes) et enfin un mélange de dichlorométhane et de méthanol (8/2 en volumes), on obtient 163 mg de [4-(3H-imidazo[4,5-c]pyridin-2-yl)-9H-fluorène-9(R,S)-yl]-amide de l'acide 5-(4-hydroxy-phenyl)-3H-1,2,3-triazole-4-carboxylique, sous forme d'une poudre blanche dont les caractéristiques sont les suivantes :
Spectre de masse (LCMS) : m/z = 485 (M+)

### Exemple 183 : Synthèse du [4-(5-cyano-6-fluoro-1H-benzimidazol-2-yl)-9H-fluorén-9(R,S)-yl]-amide de l'acide 1H-pyrrolo[2,3-b]pyridine-4-carboxylique.

On agite à température ambiante 20 mg de 4,5-diamino-2-fluoro-benzonitrile, qui peut être préparé selon WO9926941A1, 40 mg de [4-formyl-9H-fluorène-9(R,S)-yl]-amide de l'acide 1H-pyrrolo[2,3-b]pyridine-4-carboxylique, qui peut être obtenu comme à l'étape 6 de l'exemple 234, et 18 mg de chlorure ferrique en solution dans 2 mL de diméthylformamide. L'agitation est stoppée après 60 heures et le solvant est évaporé sous pression réduite. L'huile sombre obtenue est purifiée par flash-chromatographie sur gel de silice, en éluant avec un gradient de mélanges de dichlorométhane et de méthanol (de 100/0 à 90/10 en volumes) pour conduire à 22 mg du [4-(5-cyano-6-fluoro-1H-benzimidazol-2-yl)-9H-fluorèn-9(R,S)-yl]-amide de l'acide 1H-pyrrolo[2,3-b]pyridine-4-carboxylique sous la forme d'une poudre beige, dont la caractéristique est la suivante :
Spectre de masse (ESI+) : m/z = 485 (MH+)

### Exemple 184 : Synthèse du [4-(3H-imidazo[4,5-c]pyridin-2-yl)-9H-fluorène-9(R,S)-yl]-amide de l'acide 2-amino-4-méthyl-thiazole-5-carboxylique.

On opère comme dans l'exemple 14, à partir de 300 mg de 4-(3H-imidazo[4,5-c]pyridin-2-yl)-fluorène-9(R,S)-amine, obtenue à l'exemple 6, et de 167 mg d'acide 2-amino-4-méthyl-thiazole-5-carboxylique, qui peut être obtenu selon WO2004/099156, dans 3 mL de diméthylformamide, en présence de 289 mg de chlorhydrate de 1-(3-diméthylaminopropyl)-3-éthylcarbodiimide (EDCl) et 204 mg de 1-hydroxybenzotriazole (HOBT), pendant 20 heures à température ambiante. Après purification par flash-chromatographie sur gel de silice (40-63µm), en éluant avec un mélange de dichlorométhane et de méthanol (95/5 puis 9/1en volumes), on obtient 85,2 mg de [4-(3H-imidazo[4,5-c]pyridin-2-yl)-9H-fluorène-9(R,S)-yl]-amide de l'acide 2-amino-4-méthyl-thiazole-5-carboxylique, sous forme d'une poudre beige dont les caractéristiques sont les suivantes :
Point de fusion (Kofler) = 224-226°C .
Spectre de masse (EI) : m/z = 438 (M+).

### Exemple 185 : Synthèse du [4-(1H-benzimidazol-2-yl)-9H-fluorène-9(R,S)-yl]-amide de l'acide 2-amino-5-chloro-pyrimidine-4-carboxylique.

On opère comme dans l'exemple 14, à partir de 300 mg de 4-(1H-benzimidazol-2-yl)-9H-fluorèn-9(R,S)-ylamine, obtenue à l'exemple 83, et 193 mg d'acide 2-amino-5-chloro-pyrimidine-4-carboxylique dans 3 mL de diméthylformamide, en présence de 213 mg de chlorhydrate de 1-(3-diméthylaminopropyl)-3-éthylcarbodiimide (EDCI) et 68 mg de 1-hydroxybenzotriazole (HOBT), pendant 20 heures à température ambiante. Après purification par flash-chromatographie sur gel de silice (40-63µm), en éluant avec un mélange de dichlorométhane et de méthanol (98/2 en volumes), on obtient 270 mg de [4-(1H-benzimidazol-2-yl)-9H-fluorène-9(R,S)-yl]-amide de l'acide 2-amino-5-chloro-pyrimidine-4-carboxylique, sous forme d'une poudre jaune pâle dont les caractéristiques sont les suivantes :
Spectre de masse (EI/LCMS) : m/z = 452 (M+).
Spectre RMN 1H (400 MHz, □ en ppm, DMSO-d6) : 6,25 (d, J = 8,5 Hz, 1H) ; 7,12 (s large, 2H) ; de 7,21 à 7,33 (m, 3H) ; 7,36 (t, J = 7,5 Hz, 1H) ; de 7,51 à 7,60 (m, 4H) ; 7,66 (d, J = 8,5 Hz, 1H) ; 7,70 (d, J = 7,5 Hz, 1H) ; 7,76 (d, J = 7,5 Hz, 1H) ; 8,39 (s, 1H) ; 9,31 (d, J = 8,5 Hz, 1H) ; 12,95 (s, 1H).

### Exemple 186 : Synthèse du [4-(5-fluoro-1H-benzimidazol-2-yl)-9H-fluorène-9(R,S)-yl]-amide de l'acide 2-amino-5-chloro-pyrimidine-4-carboxylique.

On opère comme dans l'exemple 14, à partir de 300 mg de 4-(6-fluoro-1H-benzimidazol-2-yl)-9H-fluorèn-9(R,S)-ylamine, obtenue à l'exemple 71, et de 181 mg d'acide 2-amino-5-chloro-pyrimidine-4-carboxylique dans 3 mL de diméthylformamide, en présence de 200 mg de chlorhydrate de 1-(3-diméthylaminopropyl)-3-éthylcarbodümide (EDCl) et 64 mg de 1-hydroxybenzotriazole (HOBT), pendant 20 heures à température ambiante. Après purification par flash-chromatographies successives sur gel de silice (40-63µm), en éluant avec un mélange de dichlorométhane et de méthanol (98/2 en volumes), puis sur gel d'alumine 90, en éluant avec un mélange de dichlorométhane et de méthanol (98/2 en volumes), on obtient 186 mg de [4-(5-fluoro-1H-benzimidazol-2-yl)-9H-fluorène-9(R,S)-yl]-amide de l'acide 2-amino-5-chloro-pyrimidine-4-carboxylique, sous forme d'une poudre beige dont les caractéristiques sont les suivantes :
Spectre de masse (EI/LCMS) : m/z = 470 (M+).
Spectre RMN 1H (400 MHz, □ en ppm, DMSO-d6) : 6,24 (d, J = 8,5 Hz, 1H) ; 7,11 (s large, 2H) ; 7,14 (m partiellement masqué, 1H) ; 7,25 (t, J = 7,5 Hz, 1H) ; 7,36 (t large, J = 7,5 Hz, 1H) ; 7,46 (m étalé, 1H) ; de 7,50 à 7,58 (m, 3H) ; 7,63 (m étalé partiellement masqué, 1H) ; 7,66 (d, J = 7,5 Hz, 1H) ; 7,70 (d, J = 7,5 Hz, 1H) ; 8,39 (s, 1H) ; 9,31 (d, J = 8,5 Hz, 1H) ; 13,05 (m étalé, 1H).

### Exemple 187: Synthèse du [4-(3H-imidazo[4,5-c]pyridin-2-yl)-9H-fluorèn-9(R,S)-yl]-amide de l'acide pyrimidine-5-carboxylique.

On opère comme à l'exemple 14 à partir de 300 mg de 4-(3H-imidazo[4,5-c]pyridin-2-yl)-fluorène-9(R,S)-amine, obtenue à l'exemple 6, et de 127 mg d'acide pyrimidine-5-carboxylique, en présence de 212 mg d'EDCI et de 68 mg d'HOBT dans 7 mL de DMF pendant 15heures. Après lavages successifs avec une solution aqueuse saturée d'hydrogénocarbonate de sodium, avec de l'eau, puis avec de l'éther isopropylique, le mélange brut est purifié par HPLC préparative sur colonne Xterra RP 18, 5 µm, en éluant avec un mélange d'acétonitrile 35% et de tampon NH4HCO3 (20 mMol, pH = 9), on obtient 67 mg de [4-(3H-imidazo[4,5-c]pyridin-2-yl)-9H-fluorèn-9(R,S)-yl]-amide de l'acide pyrimidine-5-carboxylique sous forme d'un solide beige, dont les caractéristiques sont les suivantes :
Spectre de masse (E/I) : m/z = 404 (M+).
Point de fusion (Kofler) = 192°C.

### Exemple 188 : Synthèse du [4-(5-fluoro-1H-benzimidazol-2-yl)-9H-fluorène-9(R,S)-yl]-amide de l'acide 1H-pyrrolo[2,3-b]pyridine-4-carboxylique.

On opère comme dans l'exemple 14, à partir de 300 mg de 4-(6-fluoro-1H-benzimidazol-2-yl)-9H-fluorène-9(R,S)-ylamine, obtenue à l'exemple 71, et 170 mg d'acide 1H-pyrrolo[2,3-b]pyridine-4-carboxylique, qui peut être obtenu selon WO 2003/000688, dans 3 mL de diméthylformamide, en présence de 200 mg de chlorhydrate de 1-(3-diméthylaminopropyl)-3-éthylcarbodiimide (EDCI) et 64 mg de 1-hydroxybenzotriazole (HOBT), pendant 20 heures à température ambiante. Après purification par flash-chromatographie sur gel de silice (40-63µm), en éluant avec un mélange de dichlorométhane et de méthanol (98/2 en volumes), on obtient 243 mg de [4-(5-fluoro-1H-benzimidazol-2-yl)-9H-fluorèn-9(R,S)-yl]-amide de l'acide 1H-pyrrolo[2,3-b]pyridine-4-carboxylique, sous forme d'une poudre blanc cassé dont les caractéristiques sont les suivantes :
Spectre de masse (LCMS) : m/z = 459 (M+).
Spectre RMN 1H (400 MHz, □ en ppm, DMSO-d6) : 6,41 (d, J = 8,5 Hz, 1H) ; 6,91 (dd, J = 2,0 et 3,5 Hz, 1H) ; 7,14 (m, 1H) ; 7,25 (t, J = 7,5 Hz, 1H) ; 7,34 (t, J = 7,5 Hz, 1H) ; 7,38 (m partiellement masqué, 1H) ; 7,46 (d, J = 5,0 Hz, 1H) ; 7,52 (t, J = 7,5 Hz, 1H) ; de 7,55 à 7,67 (m, 4H) ; 7,75 (d, J = 7,5 Hz, 1H) ; 7,79 (m partiellement masqué, 1H) ; 8,30 (d, J = 5,0 Hz, 1H) ; 9,28 (d, J = 8,5 Hz, 1H) ; 11,85 (s large, 1H) ; 13,05 (s large, 1H).

### Exemple 189: [4-(3H-imidazo[4,5-c]pyridin-2-yl)-9H-fluorèn-9(R,S)-yl]-amide de l'acide isoquinoléine-4-carboxylique

On opère comme à l'exemple 14 à partir de 298 mg de 4-(3H-Imidazo[4,5-c]pyridin-2-yl)-fluorène-9(R,S)-amine, obtenue à l'exemple 6, et de 173 mg d'acide isoquinoleine-4-carboxylique, en présence de 211 mg d'EDCl et de 68 mg d'HOBT, dans 5 mL de DMF pendant 20 heures. Après purification par flash-chromatographie sur alumine en éluant avec un mélange de méthanol et de dichlorométhane (5/95 en volumes), puis cristallisation dans 20 mL d'oxide d'isopropyle, on obtient 40 mg de [4-(3H-imidazo[4,5-c]pyridin-2-yl)-9H-fluorèn-9(R,S)-yl]-amide de l'acide isoquinoléine-4-carboxylique, sous forme d'un solide beige dont les caractéristiques sont les suivantes :
Point de fusion (Kofler) = 200°C.
Spectre de RMN 1H (400 MHz, DMSO-d6) □en ppm : 6,44 (d, J = 8,5 Hz, 1H) ; 7,27 (t, J = 7,5 Hz, 1H) ; 7,39 (t, J = 7,5 Hz, 1H) ; 7,51 (d, J = 7,5 Hz, 1H) ; 7,57 (t, J = 7,5 Hz, 1H) ; 7,67 (d large, J = 5,5 Hz, 1H) ; 7,71 (d, J = 7,5 Hz, 1H) ; 7,78 (m, 2H) ; 7,95 (m, 2H) ; 8,23 (d, J = 8,5 Hz, 1H) ; 8,39 (d, J = 5,5 Hz, 1H) ; 8,46 (d, J = 8,5 Hz, 1H) ; 8,75 (s, 1H) ; 9,04 (s, 1H) ; 9,42 (s, 1H) ; 9,47 (d, J = 8,5 Hz, 1H) ; 13,4 (m étalé, 1H).
Spectre de masse (ES) :m/z=454 (MH+).

### Exemple 190 : Synthèse du [4-(3H-imidazo[4,5-c]pyridin-2-yl)-9H-fluorène-9(R,S)-yl]-amide de l'acide 2,3-diméthyl-quinoxaline-5-carboxylique.

On opère comme dans l'exemple 14, à partir de 300 mg de 4-(3H-imidazo[4,5-c]pyridin-2-yl)-fluorène-9(R,S)-amine, obtenue à l'exemple 6, et 224 mg d'acide 2,3-diméthyl-quinoxaline-5-carboxylique dans 3 mL de diméthylformamide, en présence de 212 mg de chlorhydrate de 1-(3-diméthylaminopropyl)-3-éthylcarbodümide (EDCl) et 68 mg de 1-hydroxybenzotriazole (HOBT), pendant 20 heures à température ambiante. Après purification par flash-chromatographie sur gel de silice (40-63µm), en éluant avec un mélange de dichlorométhane et de méthanol (95/5 en volumes), on obtient 295 mg de [4-(3H-imidazo[4,5-c]pyridin-2-yl)-9H-fluorène-9(R,S)-yl]-amide de l'acide 2,3-diméthyl-quinoxaline-5-carboxylique, sous forme d'une poudre jaune pâle dont les caractéristiques sont les suivantes :
Spectre de masse (El) : m/z = 482 (M+).

### Exemple 191 : Synthèse du [4-(3H-imidazo[4,5-c]pyridin-2-yl)-9H-fluorène-9(R,S)-yl]-amide de l'acide 3-amino-1H-pyrazole-4-carboxylique.

On opère comme dans l'exemple 14, à partir de 300 mg de 4-(3H-imidazo[4,5-c]pyridin-2-yl)-fluorène-9(R,S)-amine, obtenue à l'exemple 6, et 141 mg d'acide 3-amino-1H-pyrazole-4-carboxylique dans 3 mL de diméthylformamide, en présence de 289 mg de chlorhydrate de 1-(3-diméthylaminopropyl)-3-éthylcarbodiimide (EDCI) et 204 mg de 1-hydroxybenzotriazole (HOBT), pendant 20 heures à température ambiante. Après purification par CLHP une colonne XTERRA de silice RP18 5µm (30x150 mm), en éluant avec un mélange (75/25) d'acétonitrile et de tampon pH=9 (ammoniaque/formiate d'ammonium 20 mmol), on obtient 8 mg de [4-(3H-imidazo[4,5-c]pyridin-2-yl)-9H-fluorène-9(R,S)-yl]-amide de l'acide 3-amino-1H-pyrazole-4-carboxylique, sous forme d'un solide blanc dont les caractéristiques sont les suivantes :
Spectre de masse (EI/LCMS) : m/z = 407 (M+).
Spectre RMN 1H (300 MHz ; DMSO-d6 ; □ en ppm) : 6,25 (d, J = 8,5 Hz, 1H) ; 7,10 (s large, 2H) ; 7,25 (t, J = 7,5 Hz, 1H) ; 7,36 (t, J = 7,5 Hz, 1H) ; de 7,48 à 7,59 (m, 3H) ; 7,65 (d, J = 5,5 Hz, 1H) ; de 7,68 à 7,74 (m, 2H) ; 8,37 (d, J = 5,5 Hz, 1H) ; 8,39 (s, 1H) ; 9,01 (s, 1H) ; 9,31 (d, J = 8,5 Hz, 1H).

### Exemple 192 : Synthèse de l'énantiomère dextrogyre du N-[4-(3H-imidazo[4,5-c]pyridin-2-yl)-9H-fluorèn-9(R,S)-yl]-amide de l'acide 2-amino-5-chloro-pyrimidine-4-carboxylique.

150 mg de N-[4-(3H-imidazo[4,5-c]pyridin-2-yl)-9H-fluorèn-9(R,S)-yl]-amide de l'acide 2-amino-5-chloro-pyrimidine-4.-carboxylique, obtenu à l'exemple 131, sont injectés sur une colonne chirale préparative contenant 300 g de silice Whelk 01 RR 10 µm. On élue avec un mélange de n.heptane, de méthanol, d'éthanol et de triéthylamine (60-10-30-0,2 en volumes). En récupérant et concentrant sous pression réduite la première fraction éluée, on obtient 70,9 mg d'énantiomère dextrogyre dont les caractéristiques sont les suivantes :
□D20 = +117,3 +/- 2 ° (c = 0,5 ; MeOH).

### Exemple 193 : Synthèse de l'énantiomère lévogyre du N-[4-(3H-imidazo[4,5-c]pyridin-2-yl)-9H-fluorèn-9(R,S)-yl]-amide de l'acide 2-amino-5-chloro-pyrimidine-4-carboxylique.

En opérant comme à l'exemple 192, mais en récupérant et concentrant sous pression réduite la seconde fraction éluée, on obtient 68,7 mg d'énantiomère lévogyre dont les caractéristiques sont les suivantes :
□D20 = -77 +/- 2 ° (c = 0,3 ; MeOH).

### Exemple 194 : Synthèse du [4-(3H-imidazo[4,5-c]pyridin-2-yl)-9H-fluorène-9(R,S)-yl]-amide de l'acide 1H-benzimidazole -4-carboxylique.

On opère comme dans l'exemple 14, mais à partir de 300 mg de 4-(3H-imidazo[4,5-c]pyridin-2-yl)-fluorène-9(R,S)-amine, obtenue à l'exemple 6, et 179 mg d'acide 1H-benzimidazole-4-carboxylique dans 3 mL de diméthylformamide, en présence de 212 mg de chlorhydrate de 1-(3-diméthylaminopropyl)-3-éthylcarbodümide (EDCI) et 68 mg de 1-hydroxybenzotriazole (HOBT), pendant 20 heures à température ambiante. Après purification par flash-chromatographie sur gel de silice (40-63µm), en éluant avec un mélange de dichlorométhane et de méthanol (95/5 puis 9/1 en volumes), on obtient ainsi 144 mg de [4-(3H-imidazo[4,5-c]pyridin-2-yl)-9H-fluorène-9(R,S)-yl]-amide de l'acide 1H-benzimidazole-4-carboxylique, sous forme d'une poudre blanc cassé dont les caractéristiques sont les suivantes :
Spectre de masse (EI) : m/z = 442 (M+)

### Exemple 195 : Synthèse du [4-(3H-imidazo[4,5-c]pyridin-2-yl)-9H-fluorène-9(R,S)-yl]-amide de l'acide 3-méthyl-quinoxaline-5-carboxylique.

On opère comme dans l'exemple 14, mais à partir de 300 mg de 4-(3H-imidazo[4,5-c]pyridin-2-yl)-fluorène-9(R,S)-amine, obtenue à l'exemple 6, 200 mg d'acide 3-methyl-quinoxaline-5-carboxylique, 212 mg de chlorhydrate de 1-(3-diméthylaminopropyl)-3-éthylcarbodiimide (EDCI), et 68 mg de 1-hydroxybenzotriazole (HOBT) dans 3 mL de diméthylformamide, pendant 20 heures à température ambiante. On amène à sec le diméthylformamide, on ajoute de l'eau, et on essore le précipité formé qui est ensuite lavé à l'eau puis avec une solution saturée d'hydrogénocarbonate de sodium à 10% et de nouveau à l'eau. Le solide brut obtenu est séché puis purifié par flash-chromatographie sur gel de silice (40-63µm) en éluant avec un mélange de dichlorométhane et de méthanol (95/5 en volumes). Après empâtage à l'éther diisopropylique, on obtient ainsi 308 mg (65%) de [4-(3H-imidazo[4,5-c]pyridin-2-yl)-9H-fluorène-9(R,S)-yl]-amide de l'acide 3-méthyl-quinoxaline-5-carboxylique, sous forme d'une poudre beige dont les caractéristiques sont les suivantes :
Point de fusion (Kofler) = >260°C.
Spectre de masse (LCMS) : m/z = 468 (M+)
Spectre RMN 1H (400 MHz, □ en ppm, DMSO-d6) : 2,59 (s, 3H) ; 6,44 (d, J = 7,5 Hz, 1H) ; 7,28 (t, J = 7,5 Hz, 1H) ; 7,38 (t, J = 7,5 Hz, 1H) ; 7,47 (d large, J = 8,0 Hz, 1H) ; 7,57 (t, J = 7,5 Hz, 1H) ; 7,67 (m large, 1H) ; 7,71 (d, J = 7,5 Hz, 1H) ; 7,81 (d, J = 7,5 Hz, 1H) ; 7,94 (t, J = 7,5 Hz, 1H) ; 7,98 (d, J = 7,5 Hz, 1H) ; 8,25 (d large, J = 7,5 Hz, 1H) ; 8,39 (d, J = 5,5 Hz, 1H) ; 8,55 (d large, J = 7,5 Hz, 1H) ; 8,95 (s, 1H) ; 9,04 (s large, 1H) ; 10,6 (d, J = 7,5 Hz, 1H) ; 13,4 (m étalé, 1H).

### Exemple 196 : Synthèse du [4-(3H-imidazo[4,5-c]pyridin-2-yl)-9H-fluorène-9(R,S)-yl]-amide de l'acide quinoxaline-5-carboxylique.

On opère comme dans l'exemple 14, mais à partir de 300 mg de 4-(3H-imidazo[4,5-c]pyridin-2-yl)-fluorène-9(R,S)-amine, obtenue à l'exemple 6, 192 mg d'acide quinoxaline-5-carboxylique, 212 mg de chlorhydrate de 1-(3-diméthylaminopropyl)-3-éthylcarbodiimide (EDCI) et 68 mg de 1-hydroxybenzotriazole (HOBT) dans 3 mL de diméthylformamide, pendant 20 heures à température ambiante. On amène à sec le diméthylformamide, on ajoute de l'eau, puis on essore le précipité formé qui est ensuite lavé à l'eau puis avec une solution saturée d'hydrogénocarbonate de sodium à 10% et de nouveau à l'eau. Le solide brut obtenu est séché puis purifié par flash-chromatographie sur gel de silice (40-63µm) en éluant avec un mélange de dichlorométhane et de méthanol (95/5 en volumes). Après empâtage à l'éther diisopropylique, on obtient ainsi 294 mg de [4-(3H-imidazo[4,5-c]pyridin-2-yl)-9H-fluorène-9(R,S)-yl]-amide de l'acide quinoxaline-5-carboxylique, sous forme d'une poudre jaune pâle dont les caractéristiques sont les suivantes : Point de fusion (Kofler) = >260°C.
Spectre de masse (EI/LC/MS) : m/z = 454 (M+)
Spectre RMN 1H (400 MHz, □ en ppm, DMSO-d6) : 6,50 (d, J = 8,0 Hz, 1H) ; 7,27 (t, J = 7,5 Hz, 1H) ; 7,36 (t, J = 7,5 Hz, 1H) ; 7,46 (d large, J = 8,0 Hz, 1H) ; 7,55 (t, J = 7,5 Hz, 1H) ; 7,67 (d large, J = 5,5 Hz, 1H) ; 7,71 (m, 2H) ; 7,89 (d, J = 7,5 Hz, 1H); 8,05 (t, J = 8,0 Hz, 1H) ; 8,32 (d large, J = 8,0 Hz, 1H) ; 8,40 (d, J = 5,5 Hz, 1H) ; 8,62 (d large, J = 7,5 Hz, 1H) ; 8,97 (d, J = 2,0 Hz, 1H) ; 9,04 (m large, 2H) ; 10,35 (d, J = 8,0 Hz, 1H) ; 13,4 (m étalé, 1H).

### Exemple 197 : Synthèse du [4-(3H-imidazo[4,5-c]pyridin-2-yl)-9H-fluorène-9(R,S)-yl]-amide de l'acide 2-furan-2-yl-3H-benzimidazole-4-carboxylique.

On opère comme dans l'exemple 14, mais à partir de 300 mg de 4-(3H-imidazo[4,5-c]pyridin-2-yl)-fluorène-9(R,S)-amine, obtenue à l'exemple 6, 253 mg d'acide 2-furan-2-yl-3H-benzimidazole-4.-carboxylique, 212 mg de chlorhydrate de 1-(3-diméthylaminopropyl)-3-éthylcarbodümide (EDCl), et 68 mg de 1-hydroxybenzotriazole (HOBT) dans 3 mL de diméthylformamide pendant 20 heures à température ambiante. On amène à sec le diméthylformamide, on ajoute de l'eau, et on essore le précipité formé qui est ensuite lavé à l'eau puis avec une solution saturée d'hydrogénocarbonate de sodium à 10% et de nouveau à l'eau. Le solide brut obtenu est séché puis purifié par flash-chromatographie sur gel de silice (40-63µm) en éluant avec un mélange de dichlorométhane et d'ammoniac en solution 7N dans le méthanol (9/1 en volumes). Après empâtage à l'éther diisopropylique, on obtient ainsi 195 mg de [4-(3H-imidazo[4,5-c]pyridin-2-yl)-9H-fluorène-9(R,S)-yl]-amide de l'acide 2-furan-2-yl-3H-benzimidazole-4- carboxylique, sous forme d'une poudre beige dont les caractéristiques sont les suivantes :
Point de fusion (Kofler) = 252°C.
Spectre de masse (LC/MS) : m/z = 508 (M+)

### Exemple 198: Synthèse du [4-(3H-imidazo[4,5-c]pyridin-2-yl)-9H-fluorène-9(R,S)-yl]-amide de l'acide 2-thiophèn-2-yl-3H-benzimidazole-4-carboxylique

On opère comme dans l'exemple 14, mais à partir de 300 mg de 4-(3H-imidazo[4,5-c]pyridin-2-yl)-fluorène-9(R,S)-amine, obtenue à l'exemple 6, 270 mg d'acide 2-thiophèn-2-yl-3H-benzimidazole-4-carboxylique, 212 mg de chlorhydrate de 1-(3-diméthylaminopropyl)-3-éthylcarbodiimide (EDCl) et 68 mg de 1-hydroxybenzotriazole (HOBT) dans 3 mL de diméthylformamide pendant 20 heures à température ambiante. On amène à sec le diméthylformamide, on ajoute de l'eau, et on essore le précipité formé qui est ensuite lavé à l'eau puis avec une solution saturée d'hydrogénocarbonate de sodium à 10% et de nouveau à l'eau. Le solide brut obtenu est séché puis purifié par flash-chromatographie sur gel de silice (40-63µm) en éluant avec un mélange de dichlorométhane et d'ammoniac en solution 7N dans le méthanol (95/5 puis 9/1 en volumes). Après empâtage à l'éther diisopropylique, on obtient ainsi 347 mg de [4-(3H-imidazo[4,5-c]pyridin-2-yl)-9H-fluorène-9(R,S)-yl]-amide de l'acide 2-thiophèn-2-yl-3H-benzimidazole-4-carboxylique, sous forme d'une poudre blanc cassé dont les caractéristiques sont les suivantes :
Point de fusion (Kofler) = >260°C.
Spectre de masse (LCMS) : m/z = 524 (M+)

### Exemple 199: Synthèse du [4-(3H-imidazo[4,5-c]pyridin-2-yl)-9H-fluorène-9(R,S)-yl]-amide de l'acide quinoxaline-6-carboxylique

On opère comme dans l'exemple 14, mais à partir de 300 mg de 4-(3H-imidazo[4,5-c]pyridin-2-yl)-9H-fluorène-9(R,S)-amine, obtenue à l'exemple 6, 193 mg d'acide quinoxaline-6-carboxylique, 212 mg de chlorhydrate de 1-(3-diméthylaminopropyl)-3-éthylcarbodiimide (EDCI) et 68 mg de 1-hydroxybenzotriazole (HOBT) dans 3 mL de diméthylformamide, pendant 20 heures à température ambiante. On amène à sec le diméthylformamide, on ajoute de l'eau, et on essore le précipité formé qui est ensuite lavé à l'eau puis avec une solution saturée d'hydrogénocarbonate de sodium à 10% et de nouveau à l'eau. Le solide brut obtenu est séché puis purifié par flash-chromatographie sur gel de silice (40-63µm) en éluant avec un mélange de dichlorométhane et de méthanol (95/5 puis 9/1 en volumes). Après empâtage à l'éther diisopropylique, on obtient ainsi 306 mg de [4-(3H-imidazo[4,5-c]pyridin-2-yl)-9H-fluorène-9(R,S)-yl]-amide de l'acide quinoxaline-6-carboxylique, sous forme d'une poudre blanc cassé dont les caractéristiques sont les suivantes :
Point de fusion (Kofler) = >260°C.
Spectre de masse (LC/MS) : m/z = 454 (M+)

### Exemple 200: Synthèse du [4-(9H-purine-8-yl)-9H-fluorène-9(R,S)-yl]-amide de l'acide 1H-pyrrolo[2,3-b]pyridine-4-carboxylique

On opère comme dans l'exemple 14, mais à partir de 100 mg de 4-(9H-purine-8-yl)-fluorène-9(R,S)-amine, obtenue à l'exemple 164, 60mg d'acide 1H-pyrrolo-[2,3-b]pyridine-4-carboxylique, 70mg de chlorhydrate de 1-(3-diméthylaminopropyl)-3-éthylcarbodümide (EDCl) et 23 mg de 1-hydroxybenzotriazole (HOBT) dans 1 mL de diméthylformamide, pendant 20 heures à température ambiante. On amène à sec le diméthylformamide, on ajoute de l'eau, et on essore le précipité formé qui est ensuite lavé à l'eau puis avec une solution saturée d'hydrogénocarbonate de sodium à 10% et de nouveau à l'eau. Le solide brut obtenu est séché puis purifié par flash-chromatographie sur gel de silice (40-63µm) en éluant avec un mélange de dichlorométhane et de méthanol (98/2 puis 95/5 en volumes). Après empâtage à l'éther diisopropylique, on obtient ainsi 53,3 mg de [4-(9H-purine-8-yl)-9H-fluorène-9(R,S)-yl]-amide de l'acide 1H-pyrrolo-[2,3-b]pyridine-4-carboxylique, sous forme d'une poudre beige dont les caractéristiques sont les suivantes :
Point de fusion (Kofler) = >260°C.
Spectre de masse (LCMS) : m/z = 443 (M+)
Spectre RMN 1H (300 MHz, □ en ppm, DMSO-d6) : 6,41 (d, J = 8,5 Hz, 1H) ; 6,91 (dd, J = 2,0 et 3,5 Hz, 1H) ; 7,27 (t, J = 7,5 Hz, 1H) ; 7,36 (t, J = 7,5 Hz, 1H) ; 7,47 (d, J = 5,0 Hz, 1H) ; 7,55 (t, J = 7,5 Hz, 1H) ; de 7,58 à 7,66 (m, 3H) ; 7,73 (d, J = 7,5 Hz, 1H) ; 7,82 (d, J = 7,5 Hz, 1H) ; 8,30 (d, J = 5,0 Hz, 1H) ; 9,00 (s, 1H) ; 9,22 (s, 1H) ; 9,29 (d, J = 8,5 Hz, 1H) ; 11,85 (m large, 1H) ; 13,95 (m étalé, 1H) .

### Exemple 201 : Synthèse du [4-(3H-imidazo[4,5-c]pyridin-2-yl)-9H-fluorèn-9(R,S)-yl]-amide de l'acide 2-hydroxy-quinazoline-4-carboxylique.

On opère comme à l'exemple 14, mais à partir de 400 mg de 4-(3H-imidazo[4,5-c]pyridin-2-yl)-fluorène-9(R,S)-amine, obtenue à l'exemple 6, 283 mg de chlorhydrate de 1-(3-diméthylaminopropyl)-3-éthylcarbodiimide (EDCI), 91 mg de 1-hydroxybenzotriazole (HOBT) et 255 mg d'acide 2-hydroxy-quinazoline-4-carboxylique dans 10 mL de diméthylformamide, pendant 20 heures à température ambiante. On ajoute alors 100 mL d'eau, et on essore le précipité formé qui est ensuite lavé à l'eau puis avec une solution saturée d'hydrogénocarbonate de sodium. Le solide brut obtenu est purifié par flash-chromatographie sur gel de silice (70-230 Mesh) en éluant avec un mélange de dichlorométhane et d'ammoniac à 7N dans le méthanol (90/10 en volumes). On obtient ainsi 50 mg de [4-(3H-imidazo[4,5-c]pyridin-2-yl)-9H-fluorèn-9(R,S)-yl]-amide de l'acide 2-hydroxy-quinazoline-4-carboxylique, sous forme d'une poudre jaune pâle dont les caractéristiques sont les suivantes :
Point de fusion (Kofler) > 260°C
Spectre de masse (LCMS) : m/z = 470 (M+)

### Exemple 202: Synthèse du chlorhydrate du [4-(3H-imidazo[4,5-c]pyridin-2-yl)-9H-fluorèn-9(R,S)-yl]-amide de l'acide 6-(2-amino-éthylamino)-nicotinique.

Etape 1 : On opère comme à l'exemple 14, mais à partir de 645 mg de 4-(3H-imidazo[4,5-c]pyridin-2-yl)-fluorène-9(R,S)-amine, obtenue à l'exemple 6, 456 mg de chlorhydrate de 1-(3-diméthylaminopropyl)-3-éthylcarbodiimide (EDCI), 146 mg de 1-hydroxybenzotriazole (HOBT) et 608 mg d'acide 6-(2-tert-butoxycarbonylamino-éthylamino)-nicotinique dans 15 mL de diméthylformamide, pendant 20 heures à température ambiante. On ajoute alors 50 mL d'eau, et on essore le précipité formé qui est ensuite lavé à l'eau puis avec une solution saturée d'hydrogénocarbonate de sodium. Le solide brut obtenu est purifié par flash-chromatographie sur gel de silice (70-230 Mesh) en éluant avec un mélange de dichlorométhane et de méthanol (90/10 en volumes). On obtient ainsi 540 mg de [4-(3H-imidazo[4,5-c]pyridin-2-yl)-9H-fluorèn-9(R,S)-yl]-amide de l'acide 6-(2-tert-butoxycarbonylamino-éthylamino)-nicotinique, sous forme d'une poudre beige dont la caractéristique est la suivante :
Spectre de masse (LCMS) : m/z = 561 (M+)

Etape 2 : On dissout 320 mg de [4-(3H-imidazo[4,5-c]pyridin-2-yl)-9H-fluorèn-9(R,S)-yl]-amide de l'acide 6-(2-tert-butoxycarbonylamino-éthylamino)-nicotinique, obtenu ci -dessus, dans 10 mL de dioxane, puis on ajoute 2 mL d'une solution 4M d'acide chlorhydrique dans le dioxane. Après 20 heures d'agitation à température ambiante, le solide formé est essoré et lavé à l'éthanol puis à l'éther éthylique. On obtient ainsi 280 mg de chlorhydrate du [4-(3H-imidazo[4,5-c]pyridin-2-yl)-9H-fluorèn-9(R,S)-yl]-amide de l'acide 6-(2-amino-éthylamino)-nicotinique, sous forme d'une poudre jaune-pâle, dont les caractéristiques sont les suivantes :
Point de fusion (Kofler) > 260°C
Spectre de masse (LCMS) : m/z = 571 (M+)

### Exemple 203: Synthèse du [4-(1H-benzimidazol-2-yl)-9H-fluorène-9(R,S)-yl]-amide de l'acide 1H-pyrrolo[2,3-b]pyridine-4-carboxylique

On opère comme dans l'exemple 14, mais à partir de 300 mg de 4-(1H-benzimidazol-2-yl)-9H-fluorèn-9(R,S)-ylamine, obtenue à l'exemple 83, 180 mg d'acide 1H-pyrrolo-[2,3-b]pyridine-4-carboxylique, 213 mg de chlorhydrate de 1-(3-diméthylaminopropyl)-3-éthylcarbodümide (EDCI) et 68 mg de 1-hydroxybenzotriazole (HOBT) dans 3 mL de diméthylformamide, pendant 20 heures à température ambiante. On amène à sec le diméthylformamide, on ajoute de l'eau, et on essore le précipité formé qui est ensuite lavé à l'eau puis avec une solution saturée d'hydrogénocarbonate de sodium à 10% et de nouveau à l'eau. Le solide brut obtenu est séché puis purifié par flash-chromatographie sur gel de silice (40-63µm) en éluant avec un mélange de dichlorométhane et de méthanol (98/2 en volumes) puis avec un mélange de dichlorométhane et d' ammoniac en solution 7N dans le méthanol (95/5 en volumes). Après empâge à l'éther diisopropylique, on obtient ainsi 202 mg de 4-(1H-benzimidazol-2-yl)-9H-fluorène-9(R,S)-yl]-amide de l'acide 1H-pyrrolo-[2,3-b]pyridine-4-carboxylique, sous forme d'une poudre blanc-cassé dont les caractéristiques sont les suivantes :
Point de fusion (Kofler) = 220°C.
Spectre de masse (El) : m/z = 441 (M+)
Spectre de RMN (300 MHz, □ en ppm, DMSO-d6) : 6,41 (d, J = 8,5 Hz, 1H) ; 6,91 (m large, 1H) ; 7,24 (t, J = 7,5 Hz, 1H) ; 7,28 (m large, 2H) ; 7,34 (t, J = 7,5 Hz, 1H) ; 7,47 (d, J = 5,5 Hz, 1H) ; 7,52 (t, J = 7,5 Hz, 1H) ; de 7,58 à 7,64 (m, 4H) ; 7,66 (d, J = 7,5 Hz, 1H) ; 7,76 (d, J = 7,5 Hz, 1H) ; 7,76 (m étalé partiellement masqué, 1H) ; 8,30 (d, J = 5,5 Hz, 1H) ; 9,28 (d, J = 8,5 Hz, 1H) ; 11,85 (m large, 1H) ; 12,95 (m large, 1H).

### Exemple 204 : Synthèse du [4-(5-trifluorométhyl-1H-benzimidazol-2-yl)-9H-fluorène-9(R,S)-yl]-amide de l'acide 1H-pyrrolo[2,3-b]pyridine-4-carboxylique

Etape 1 : On opère comme à l'étape 1 de l'exemple 1, mais à partir de 2,6 g de 3,4-diaminobenzotrifluorométhyle dans 85 mL de dichlorométhane, 3,4 mL de triéthylamine et 3 g de chlorure de l'acide fluorène-4-one-9-carboxylique. Après 20 heures d'agitation à température ambiante, le milieu réactionnel est versé sur de l'eau et extrait au dichlorométhane. Les phases organiques sont lavées avec une solution aqueuse saturée d'hydrogénocarbonate de sodium à 10%, séchées sur sulfate de magnésium et concentrées sous pression réduite. Après purification par flash-chromatographie sur gel de silice (40-63µm), en éluant par du dichlorométhane puis par un mélange de dichlorométhane et de méthanol (98/2 en volumes) On obtient ainsi 2,6 g du (2-amino-5-trifluorométhyl-phenyl)amide de l'acide 9-oxo-9H-fluorène-4-carboxylique sous forme d'une poudre jaune utilisée telle quelle à l'étape suivante, dont les caractéristiques sont les suivantes :
Spectre de masse (LC/MS) : m/z = 382 (M+)

Etape 2 : On opère comme à l'exemple 68. Dans un ballon de 500 mL sous atmosphère d'argon, on chauffe, à 120°C pendant 1h15, une solution de 2,6 g de (2-amino-5-trifluorométhyl-phenyl)amide de l'acide 9-oxo-9H-fluorène-4-carboxylique, obtenu à l'étape précédente, dans 130 mL d'acide acétique. Après refroidissement, on amène à sec sous pression réduite. On purifie par flash-chromatographie sur gel de silice (40-63µm), en éluant par un mélange de dichlorométhane et de méthanol (98/2 en volumes), puis par un mélange de dichlorométhane et d'ammoniac en solution 7N dans le méthanol. Après empâtage à l'éther diisopropylique, on obtient 2,1 g de 4-(5-trifluorométhyl-1H-benzimidazol-2-yl)-9H-fluorène-9-one, sous forme d'une résine orangée utilisée telle quelle à l'étape suivante, dont les caractéristiques sont les suivantes :
Spectre de masse (EI/LC/MS) : m/z = 364 (M+)
Spectre RMN 1H (300 MHz, □ en ppm, DMSO-d6) : 7,40 (t large, J = 7,5 Hz, 1H) ; 7,50 (dt, J = 1,5 et 7,5 Hz, 1H) ; 7,60 (t, J =7,5 Hz, 1H) ; de 7,61 à 7,71 (m, 3H) ; 7,83 (d large, J = 7,5 Hz, 1H) ; 7,88 (m, 2H) ; 8,08 (s large, 1H) ; 13,5 (m très étalé, 1H) .

Etape 3 : On opère comme à l'exemple 5, mais à partir de 2,1 g de 4-(5-trifluorométhyl-1H-benzimidazol-2-yl)-9H-fluorène-9-one, obtenue à l'étape précédente, 1,2 g de chlorhydrate d'hydroxylamine et 2,4 g d'acétate de sodium dans 100 mL d'éthanol pendant 20h à température ambiante. Après concentration du solvant sous pression réduite, le résidu est repris successivement par de l'eau, puis du toluène, amené à sec et enfin empâté dans de l'oxyde diisopropyle, filtré et lavé deux fois. On obtient ainsi 2,3 g de 4-(5-triftuorométhyl-1H-benzimidazol-2-yl)-9H-fluorène-9-one oxime (Z,E), en mélange 50-50 des isomères Z et E, sous forme d'une poudre beige, dont les caractéristiques sont les suivantes :
Spectre de masse (LCMS) : m/z = 379 (M+).

Etape 4 : On opère comme à l'exemple 6. Dans un autoclave de 528 mL, on dissout 2,2 g de 4-(5-trifluorométhyl-1H-benzimidazol-2-yl)-9H-fluorène-9-one oxime (Z,E), obtenue à l'étape précédente, dans un mélange de 80 mL d'éthanol et 80 mL de tétrahydrofurane, on ajoute une spatule de Nickel activé selon Raney puis on soumet à une pression initiale d'hydrogène de 1 bar et on chauffe l'autoclave à 60° pendant 4 heures. Après refroidissement, le volume d'hydrogène absorbé est de 412 mL. Après filtration du catalyseur sur Célite, on concentre sous pression réduite. On obtient ainsi 1,73g g de 4-(5-trifluorométhyl-1H-benzimidazol-2-yl)-9H-fluorène-9(R,S)-amine, sous forme d'une mousse grise, dont les caractéristiques sont les suivantes :
Spectre de masse (LC/MS) : m/z = 365 (M+)

Etape 5 : On opère comme à l'exemple 14, mais à partir de 365 mg de 4-(5-trifluorométhyl-1H-benzimidazol-2-yl)-9H-fluorène-9(R,S)-amine, obtenue à l'étape précédente, 178 mg d'acide 1H-pyrrolo-[2,3-b]pyridine-4-carboxylique, 210 mg de chlorhydrate de 1-(3-diméthylaminopropyl)-3-éthylcarbodiimide (EDCI) et 68 mg de 1-hydroxybenzotriazole (HOBT) dans 3,6 mL de diméthylformamide, pendant 20 heures à température ambiante. On amène à sec le diméthylformamide, on ajoute de l'eau, et on essore le précipité formé qui est ensuite lavé à l'eau puis avec une solution saturée d'hydrogénocarbonate de sodium à 10% et de nouveau à l'eau. Le solide brut obtenu est séché puis purifié par flash-chromatographie sur gel de silice (40-63µm) en éluant avec un mélange de dichlorométhane et de méthanol (98/2 puis 95/5 en volumes). Après empâtage à l'éther diisopropylique contenant un peu de dichlorométhane, on obtient ainsi 221 mg de 4-(5-trifluoromethyl-1H-benzimidazol-2-yl)-9H-fluorène-9(R,S)-yl]-amide de l'acide 1H-pyrrolo[2,3-b]pyridine-4-carboxylique, sous forme d'une poudre beige dont les caractéristiques sont les suivantes :
Point de fusion (Kofler) = 220°C .
Spectre de masse (LC/MS) : m/z = 509 (M+)
Spectre RMN 1H (400 MHz, □en ppm, DMSO-d6) : 6,42 (d, J = 8,5 Hz, 1H) ; 6,91 (dd, J = 2,0 et 3,5 Hz, 1H) ; 7,26 (t, J = 7,5 Hz, 1H) ; 7,35 (t, J = 7,5 Hz, 1H) ; 7,47 (d, J = 5,0 Hz, 1H) ; 7,54 (t, J = 7,5 Hz, 1H) ; de 7,55 à 7,64 (m, 4H) ; 7,70 (d, J = 7,5 Hz, 1H) ; 7,79 (d, J = 7,5 Hz, 1H) ; 7,86 (m étalé, 1H) ; 8,05 (m étalé, 1H) ; 8,30 (d, J = 5,0 Hz, 1H) ; 9,28 (d, J = 8,5 Hz, 1H) ; 11,85 (m large, 1H) ; 13,4 (m étalé, 1H) .

### Exemple 205 : Synthèse du [4-(5-méthoxycarbonyl-1H-benzimidazol-2-yl)-9H-fluorène-9(R,S)-yl]-amide de l'acide 1H-pyrrolo[2,3-b]pyridine-4-carboxylique

Etape 1 : On opère comme à l'étape 1 de l'exemple 1, mais à partir de 4,9 g de 3,4-diaminobenzoate de méthyle dans 170 mL de dichlorométhane, 6,9 mL de triéthylamine et 6 g de chlorure de l'acide fluorène-4-one-9-carboxylique. Après 20 heures d'agitation à température ambiante, le précipité formé est filtré, lavé à l'eau, puis avec une solution aqueuse saturée d'hydrogénocarbonate de sodium à 10%, et de nouveau à l'eau. Après empâtage à l'éther diisopropylique, on obtient ainsi 6,5 g du (2-amino-5-méthoxycarbonyl-phényl)amide de l'acide 9-oxo-9H-fluorène-4-carboxylique, sous forme d'une poudre jaune utilisée telle quelle à l'étape suivante, dont les caractéristiques sont les suivantes :
Spectre de masse (LC/MS) : m/z = 372 (M+)

Etape 2 : On opère comme dans l'exemple 68. Dans un ballon de 1 L sous atmosphère d'argon, on chauffe, à 135°C pendant 2 heures, une solution de 6,5 g de (2-amino-5-méthoxycarbonyl-phényl)amide de l'acide 9-oxo-9H-fluorène-4-carboxylique, obtenu à l'étape précédente, dans 320 mL d'acide acétique. Après refroidissement, on amène à sec sous pression réduite. Après empâtge à l'éther diisopropylique, on obtient 6,1 g d'ester de méthyle de l'acide 2-(9H-fluorèn-9-one-4-yl)benzimidazole-4-carboxylique, sous forme d'une poudre jaune utilisée telle quelle à l'étape suivante, dont les caractéristiques sont les suivantes :
Spectre de masse (EI) : m/z = 354 (M+)
Spectre de RMN (400 MHz, □en ppm, DMSO-d6) : 3,90 (s, 3H) ; 7,40 (t large, J = 7,5 Hz, 1H) ; 7,50 (dt, J = 1,5 et 7,5 Hz, 1H) ; 7,59 (t, J = 7,5 Hz, 1H) ; 7,69 (d, J = 7,5 Hz, 1H) ; 7,73 (d, J = 7,5 Hz, 1H) ; 7,77 (d, J = 8,5 Hz, 1H) ; 7,82 (dd, J = 1,0 et 7,5 Hz, 1H) ; 7,90 (dd, J = 1,0 et 8,0 Hz, 1H) ; 7,94 (dd, J = 1,5 et 8,5 Hz, 1H) ; 8,31 (s large, 1H) ; 13,1 (m très étalé, 1H) .

Etape 3 : On opère comme à l'exemple 5, mais à partir de 6,1 g d'ester de méthyle de l'acide 2-(9H-fluorèn-9-one-4-yl)benzimidazole-4-carboxylique, obtenu à l'étape précédente, de 3,6 g de chlorhydrate d'hydroxylamine et de 7 g d'acétate de sodium dans 300 mL d'éthanol agités pendant 4h à température ambiante puis à 80°C pendant 2 heures. Après concentration du solvant sous pression réduite, le résidu est repris successivement par de l'eau, puis du toluène, amené à sec et enfin empâté dans de l'oxyde diisopropyle. On obtient ainsi 6,1 g d'ester de méthyle de l'acide 2-(9H-fluorèn-9-oximino-4-yl)benzimidazole-4-carboxylique, en mélange 50-50 des isomères Z et E, sous forme d'une poudre jaune pâle utilisée telle quelle à l'étape suivante, dont les caractéristiques sont les suivantes :
Spectre de masse (El/Cl) : m/z = 369 (M+).

Etape 4 : On opère comme dans l'exemple 6. Dans un autoclave de 955 mL, on dissout 6,1 g d'ester de méthyle de l'acide 2-(9H-fluorèn-9-oximino-4-yl)benzimidazole-4-carboxylique, obtenue à l'étape précédente, dans un mélange de 230 mL d'éthanol et 230 mL de tétrahydrofurane, on ajoute une spatule de Nickel activé selon Raney puis on soumet à une pression initiale d'hydrogène de 1 bar et on chauffe l'autoclave à 60° pendant 6 heures. Après refroidissement, le volume d'hydrogène absorbé est de 979 mL. Après filtration du catalyseur sur Célite, on concentre le filtrat sous pression réduite. Le solide brut obtenu est empâté à l'éther diisopropylique, on obtient ainsi 5,7 g d'ester de méthyle de l'acide 2-(9H-fluorèn-9(R,S)-amino-4-yl)benzimidazole-4-carboxylique, sous forme d'une poudre beige utilisée telle quelle à l'étape suivante, dont les caractéristiques sont les suivantes :
Spectre de masse (LC/MS) : m/z = 355 (M+)

Etape 5 : On opère comme à l'exemple 14, mais à partir de 2,5 g d'ester de méthyle de l'acide 2-(9H-fluorèn-9(R,S)-amino-4-yl)benzimidazol-4-carboxylique, obtenue à l'étape précédente, 1,25 g d'acide 1H-pyrrolo-[2,3-b]pyridine-4-carboxylique, 1,48 g de chlorhydrate de 1-(3-diméthylaminopropyl)-3-éthylcarbodümide (EDCI) et 475 mg de 1-hydroxybenzotriazole (HOBT) dans 25 mL de diméthylformamide, pendant 20 heures à température ambiante. On amène à sec le diméthylformamide, on ajoute de l'eau, et on essore le précipité formé qui est ensuite lavé à l'eau puis avec une solution saturée d'hydrogénocarbonate de sodium à 10% et de nouveau à l'eau. Le solide brut obtenu est séché puis purifié par flash-chromatographie sur gel de silice (40-63µm) en éluant avec un mélange de dichlorométhane et de méthanol (98/2 puis 95/5 en volumes). Après empâtage à l'éther diisopropylique, on obtient ainsi 2,35 g de [4-(5-méthoxycarbonyl-1H-benzimidazol-2-yl)-9H-fluorène-9(R,S)-yl]-amide de l'acide 1H-pyrrolo[2,3-b]pyridine-4-carboxylique, sous forme d'une poudre beige dont les caractéristiques sont les suivantes :
Point de fusion (Kofler) = 222-226°C .
Spectre de masse (LC/MS) : m/z = 499 (M+)
Spectre RMN 1H (400 MHz, □en ppm, DMSO-d6) : pour ce composé on observe un mélange 50% - 50% de tautomères avec : 3,91 (s, 3H) ; 6,42 (d, J = 8,5 Hz, 1H) ; 6,91 (dd, J = 2,0 et 3,5 Hz, 1H) ; 7,26 (t, J = 7,5 Hz, 1H) ; 7,35 (t, J = 7,5 Hz, 1H) ; 7,47 (d, J = 5,0 Hz, 1H) ; 7,54 (m, 1,5H) ; 7,63 (m, 2,5H) ; 7,70 (m, 1,5H) ; 7,78 (d, J = 7,5 Hz, 1H) ; de 7,84 à 7,99 (m, 1,5H) ; 8,19 (s large, 0,5H) ; 8,30 (d, J = 5,0 Hz, 1H) ; 8,38 (s large, 0,5H) ; 9,29 (d, J = 8,5 Hz, 1H) ; 11,85 (m large, 1H) ; 13,35 (m large, 1H).

### Exemple 206 : Synthèse du [4-(5-carboxy-1H-benzimidazol-2-yl)-9H-fluorène-9(R,S)-yl]-amide de l'acide 1H-pyrrolo[2,3-b]pyridine-4-carboxylique

Dans un ballon de 30 mL sous argon, on dissout 500 mg [4-(5-méthoxycarbonyl-1H-benzimidazole-2-yl)-9H-fluorène-9(R,S)-yl]-amide de l'acide 1H-pyrrolo[2,3-b]pyridine-4-carboxylique, obtenue dans l'exemple 205, dans 10 mL de méthanol, puis on ajoute 2,4 mL d'une solution aqueuse 1,25 M d'hydroxyde de lithium puis on porte au reflux 20 heures. Le milieu réactionnel est refroidi et acidifié à pH 4-5 par une solution aqueuse 1 M d'acide chlorhydrique. Le solide brut est séché puis purifié par flash-chromatographie sur gel de silice (40-63µm) en éluant avec un mélange de dichlorométhane et de méthanol (9/1 puis 8/2 en volumes). Après empâtage à l'éther diisopropylique, on obtient ainsi 210 mg de [4-(5-carboxy-1H-benzimidazol-2-yl)-9H-fluorène-9(R,S)-yl]-amide de l'acide 1H-pyrrolo[2,3-b]pyridine-4-carboxylique, sous forme d'une poudre blanche dont les caractéristiques sont les suivantes :
Point de fusion (Kofler) = >260°C.
Spectre de masse (LC/MS) : m/z = 485 (M+)
Spectre RMN 1H (400 MHz, □ en ppm, DMSO-d6) : pour ce composé on observe un mélange 50% - 50% de tautomères avec : 6,42 (d, J = 8,5 Hz, 1H) ; 6,91 (dd, J = 2,0 et 3,5 Hz, 1H) ; 7,26 (t, J = 7,5 Hz, 1H) ; 7,35 (t, J = 7,5 Hz, 1H) ; 7,46 (d, J = 5,0 Hz, 1H) ; 7,53 (t, J = 7,5 Hz, 1H) ; de 7,56 à 7,67 (m, 3,5H) ; 7,70 (d, J = 8,0 Hz, 1H) ; 7,78 (d, J = 8,0 Hz, 1H) ; 7,82 (m, 0,5H) ; de 7,87 à 7,97 (m, 1H) ; 8,17 (s large, 0,5H) ; 8,30 (d, J = 5,0 Hz, 1H) ; 8,35 (s large, 0,5H) ; 9,29 (d, J = 8,5 Hz, 1H) ; 11,85 (m large, 1H) ; 12,7 (m très étalé, 1H) ; 13,25 (m large, 1H) .

Exemple 207 : Synthèse du [4-(4-méthyl-1H-benzimidazol-2-yl)-9H-fluorène-9(R,S)-yl]-amide de l'acide 1H-pyrrolo[2,3-b]pyridine-4-carboxylique

Etape 1 : On opère comme à l'étape 1 de l'exemple 1, mais à partir de 2,932 g de 2,3-diaminotoluène, 5,622 mL de triéthylamine et 4,85 g de chlorure de l'acide fluorène-4-one-9-carboxylique dans 100 mL de dichlorométhane. Après 20 heures d'agitation à température ambiante, le précipité formé est filtré, lavé au dichlorométhane, à l'eau, puis avec une solution aqueuse saturée d'hydrogénocarbonate de sodium à 10% et enfin à l'eau. Après empâtage à l'éther diisopropylique, on obtient ainsi 5 g du (2-amino-6-méthyl-phényl)amide de l'acide 9-oxo-9H-fluorène-4-carboxylique sous forme d'une poudre orange utilisée telle quelle à l'étape suivante, dont les caractéristiques sont les suivantes :
Spectre de masse (LC/MS/ES/+/-): m/z = 328 (M+)

Etape 2 : On opère comme dans l'exemple 68. Dans un ballon de 250 mL sous atmosphère d'argon, on chauffe à reflux pendant 6 heures, une suspension de 5 g de (2-amino-6-méthyl-phényl)amide de l'acide 9-oxo-9H-fluorène-4-carboxylique, obtenu à l'étape précédente, dans 100 mL d'acide acétique. Après refroidissement, on amène à sec sous pression réduite. On reprend le résidu dans 25 ml d'eau puis on amène à pH = 8 par addition d'une solution aqueuse saturée d'hydrogénocarbonate de sodium. Le précipité formé est filtré, lavé à l'eau, et empâté à l'éther diisopropylique, on obtient ainsi 4,7 g de 4-(4-méthyl-1H-benzimidazol-2-yl)-9H-fluorène-9-one, sous forme d'une poudre orange utilisée telle quelle à l'étape suivante, dont les caractéristiques sont les suivantes :
Spectre de masse (El) : m/z = 310 (M+)

Etape 3 : On opère comme à l'exemple 5, mais à partir de 4,7 g de 4-(4-méthyl-1H-benzimidazole-2-yl)-9H-fluorène-9-one, obtenue à l'étape précédente, 3,16 g de chlorhydrate d'hydroxylamine et 6,21 g d'acétate de sodium dans 65 mL d'éthanol agités pendant 20h à température ambiante. Après concentration du solvant sous pression réduite, le résidu est repris successivement par de l'eau, filtre, lavé à l'eau, empâté à l'éther diisopropylique. On obtient ainsi 4 g de 4-(4-méthyl-1H-benzimidazol-2-yl)-9H-fluorène-9-one oxime (Z,E), en mélange 60-40 des isomères Z et E, sous forme d'une poudre beige utilisée telle quelle à l'étape suivante.

Etape 4 : On opère comme à l'exemple 6, mais à partir de 4 g de 4-(4-méthyl-1H-benzimidazol-2-yl)-9H-fluorène-9-one oxime (Z,E), obtenue à l'étape précédente, et 0,7g de Nickel de Raney dans 100 mL d'éthanol et 100 mL de tétrahydrofurane pendant 10 heures à 60°C sous une pression initiale d'un bar d'hydrogène. Après filtration du catalyseur, puis purification par empâtage dans l'oxyde de diisopropyle, on obtient ainsi 2.7g de 4-(4-méthyl-1H-benzimidazol-2-yl)-9H-fluorèn-9(R,S)-yl-amine, sous forme d'une poudre beige utilisée telle quelle à l'étape suivante.

Etape 5 : On opère comme dans l'exemple 14, mais à partir de 311 mg de 4-(4-méthyl-1H-benzimidazo-2-yl)-9H-fluorène-9(R,S)-ylamine, obtenue à l'étape précédente, 162 mg d'acide 1H-pyrrolo-[2,3-b]pyridine-4-carboxylique, 211 mg de chlorhydrate de 1-(3-diméthylaminopropyl)-3-éthylcarbodiimide (EDCI) et 148 mg de 1-hydroxybenzotriazole (HOBT) dans 7 mL de diméthylformamide, pendant 20 heures à température ambiante. Après purification par flash-chromatographie sur gel de silice (40-63µm), en éluant avec un mélange de dichlorométhane et de méthanol (95/5 en volumes), puis empâtage au dichlorométhane, on obtient ainsi 255 mg de [4-(4-méthyl-1H-benzimidazol-2-yl)-9H-fluorène-9(R,S)-yl]-amide de l'acide 1H-pyrrolo[2,3-b]pyridine-4-carboxylique, sous forme d'une poudre blanc-cassé dont les caractéristiques sont les suivantes :
Spectre de masse (LC/MS/ES+/-) : m/z = 455 (M+)
Spectre RMN 1H (400 MHz, □en ppm, DMSO-d6) : pour ce composé, on observe un mélange 50% - 50% de tautomères avec : 2,55 (s, 1,5H) ; 2,62 (s, 1,5H) ; 6,41 (d, J = 8,5 Hz, 1H) ; 6,90 (d large, J = 3,5 Hz, 1H) ; 7,07 (d, J = 7,5 Hz, 1H) ; 7,17 (t, J = 7,5 Hz, 1H) ; 7,24 (m large, 1H) ; 7,33 (t, J = 7,5 Hz, 1H) ; 7,39 (m large, 0,5 H) ; 7,47 (d, J = 5,0 Hz, 1H) ; 7,51 (t, J = 7,5 Hz, 1H) ; 7,51 (m masqué, 0,5H) ; de 7,55 à 7,64 (m, 2,5H) ; 7,67 (d, J = 7,5 Hz, 1H) ; de 7,70 à 7,78 (m, 1,5H) ; 8,30 (d, J = 5,0 Hz, 1H) ; 9,28 (d large, J = 8,5 Hz, 1H) ; 11,85 (m large, 1H) ; 13,4 (m large, 1H) .

### Exemple 208 : Synthèse du [4-(5-carboxamido-1H-benzimidazol-2-yl)-9H-fluorène-9(R,S)-yl]-amide de l'acide 1H-pyrrolo[2,3-b]pyridine-4-carboxylique

Dans un ballon de 10 mL, on dissout 300 mg de [4-(5-carboxy-1H-benzimidazol-2-yl)-9H-fluorène-9(R,S)-yl]-amide de l'acide 1H-pyrrolo[2,3-b]pyridine-4-carboxylique, obtenu dans l'exemple 206, dans 3,5 mL de diméthylformamide, puis on ajoute successivement 66 mg de chlorure d'ammonium, 480 mg de hexafluorophosphate de benzotriazol-1-yl-oxy-trispyrrolidinophosphonium (PYBOP), 125 mg de 1-hydroxybenzotriazole (HOBT), 0,41 mL de N,N diisopropylethylamine (DIPEA) puis on agite 20 heures à température ambiante. On amène à sec le diméthylformamide, on ajoute de l'eau, et de l'acétate d'éthyle, il y a formation d'un précipité que l'on filtre et qui est ensuite lavé à l'eau puis avec une solution saturée d'hydrogénocarbonate de sodium à 10% et de nouveau à l'eau. Après empâtage à l'éther diisopropylique, on obtient ainsi 100 mg de [4-(5-carboxamido-1H-benzimidazol-2-yl)-9H-fluorène-9(R,S)-yl]-amide de l'acide 1H-pyrrolo[2,3-b]pyridine-4-carboxylique, sous forme d'une poudre blanc-cassé dont les caractéristiques sont les suivantes :
Point de fusion (Kofler) = >260°C.
Spectre de masse (LC/MS) : m/z = 484 (M+)

### Exemple 209: Synthèse du N-[4-(1H-imidazo[4,5c]pyridin-2-yl)-9H-fluorène-9(R,S)-yl]-amide de l'acide cyclohexan-3-one-4(R,S)-carboxylique

On opère comme dans l'exemple 14, mais à partir de 100 mg de (1H-imidazo[4,5c]pyridin-2-yl)-9H-fluorène-9(R,S)-yl-amine, obtenue à l'exemple 6, 47 mg d'acide cyclohexan-3-one-4(R,S)-carboxylique, 71 mg de chlorhydrate de 1-(3-diméthylaminopropyl)-3-éthylcarbodiimide (EDCI) et 50 mg de 1-hydroxybenzotriazole (HOBT) dans 2,5 mL de diméthylformamide, pendant 20 heures à température ambiante. Après purification par flash-chromatographie sur gel de silice (40-63µm) en éluant avec un mélange de dichlorométhane et de méthanol (98/2 en volumes), on obtient ainsi 123 mg de N-[4-(1H-imidazo[4,5c]pyridin-2-yl)-9H-fluorène-9(R,S)-yl]-amide de l'acide cyclohexan-3-one-4(R,S)-carboxylique, sous forme d'une poudre blanche dont les caractéristiques sont les suivantes :
Spectre de masse (EI) : m/z = 422 (M+)

Exemple 210 : Synthèse du [4-(5,7-difluoro-1H-benzimidazol-2-yl)]-9H-fluorène-9(R,S)-yl]-amide de l'acide 1H-pyrrolo[2,3-b]pyridine-4-carboxylique Etape 1 : On opère comme à l'étape 1 de l'exemple 1, mais à partir de 2,1 g de 1,2-diamino-3,5-difluorobenzène, 3,4 mL de triéthylamine et 3 g de chlorure de l'acide fluorène-4-one-9-carboxylique dans 85 mL de dichlorométhane. Après 20 heures d'agitation à température ambiante, le précipité est filtré, lavé au dichlorométhane, à l'eau, puis avec une solution aqueuse saturée d'hydrogénocarbonate de sodium à 10%, et de nouveau à l'eau. Après séchage puis empâtage à l'éther diisopropylique, on obtient ainsi 2,8g du (2-amino-3,5-difluoro-phényl)amide de l'acide 9-oxo-9H-fluorène-4-carboxylique, sous forme d'une poudre jaune pâle utilisée telle quelle à l'étape suivante, dont les caractéristiques sont les suivantes :
Spectre de masse (EI) : m/z = 350 (M+)

Etape 2 : On opère comme dans l'exemple 68. Dans un ballon de 500 mL sous atmosphère d'argon, on chauffe, à 135°C pendant 10h heures, une solution de 2,8 g de (2-amino-3,5-difluoro-phényl)amide de l'acide 9-oxo-9H-fluorène-4-carboxylique, obtenu à l'étape précédente, dans 140 mL d'acide acétique. Après refroidissement, on amène à sec sous pression réduite. Après empâtage à l'éther diisopropylique, on obtient 2,6 g de 4-(5,7-difluoro-1H-benzimidazol-2-yl)-9H-fluorène-9-one, sous forme d'une poudre vert-pâle utilisée telle quelle à l'étape suivante, dont les caractéristiques sont les suivantes :
Spectre de masse (EI) : m/z = 332 (M+)

Etape 3 : On opère comme à l'exemple 5, mais à partir de 2,6 g de 4-(5,7-difluoro-1H-benzimidazol-2-yl)-9H-fluorène-9-one, obtenue à l'étape précédente, de 1,6 g de chlorhydrate d'hydroxylamine et de 3,2 g d'acétate de sodium dans 130 mL d'éthanol agités pendant 20h à température ambiante. Après concentration du solvant sous pression réduite, le résidu est repris successivement par de l'eau, puis du toluène, amené à sec et enfin empâté dans de l'oxyde diisopropyle. On obtient ainsi 1,85 g de 4-(5,7-difluoro-1H-benzimidazol-2-yl)-9H-fluorène-9-one oxime (Z,E) en mélange 50-50 des isomères Z et E, sous forme d'une poudre beige utilisée telle quelle à l'étape suivante, dont les caractéristiques sont les suivantes :
Spectre de masse (EI/CI/LC/MS) : m/z = 347 (M+).

Etape 4 : On opère comme dans l'exemple 6. Dans un autoclave de 211 mL, on dissout 1,85 g de 4-(5,7-difluoro-1H-benzimidazol-2-yl)-9H-fluorène-9-one oxime (Z,E), obtenue à l'étape précédente, dans un mélange de 70 mL d'éthanol et 70 mL de tétrahydrofurane, on ajoute une spatule de Nickel activé selon Raney puis on soumet à une pression initiale d'hydrogène de 1 bar et on chauffe l'autoclave à 60° pendant 6 heures. Après refroidissement, le volume d'hydrogène absorbé est de 342 mL. Après filtration du catalyseur sur, le filtrat est concentré sous pression réduite puis repris dans un mélange de dichlorométhane avec un peu de méthanol et séché sur sulfate de magnésium, le solide brut obtenu est empâté à l'éther diisopropylique. On obtient ainsi 1,32 g de 4-(5,7-difluoro-1H-benzimidazol-2-yl)-9H-fluorène-9(R,S)-amine, sous forme d'une poudre grise utilisée telle quelle à l'étape suivante, dont les caractéristiques sont les suivantes :
Spectre de masse (EI) : m/z = 333 (M+)

Etape 5 : On opère comme dans l'exemple 14, mais à partir de 330 mg de 4-(5,7-difluoro-1H-benzimidazol-2-yl)-9H-fluorène-9(R,S)-amine, obtenue à l'étape précédente, 177 mg d'acide 1H-pyrrolo-[2,3-b]pyridine-4-carboxylique, 209 mg de chlorhydrate de 1-(3-diméthylaminopropyl)-3-éthylcarbodiimide (EDCI) et 67 mg de 1-hydroxybenzotriazole (HOBT) dans 3,3 mL de diméthylformamide pendant 20 heures à température ambiante. On amène à sec le diméthylformamide, on ajoute de l'eau, puis on essore le précipité formé qui est ensuite lavé à l'eau puis avec une solution saturée d'hydrogénocarbonate de sodium à 10% et de nouveau à l'eau. Le solide brut obtenu est séché puis purifié par flash-chromatographie sur gel de silice (40-63µm) en éluant avec un mélange de dichlorométhane et de méthanol (98/2 puis 95/5 en volumes). Après empâtage à l'éther diisopropylique, on obtient ainsi 280 mg de 4-(5,7-difluoro-1H-benzimidazol-2-yl)]-9H-fluorène-9(R,S)-yl]-amide de l'acide 1H-pyrrolo[2,3-b]pyridine-4-carboxylique, sous forme d'une poudre blanc-cassé dont les caractéristiques sont les suivantes :
Point de fusion (Kofier) = 238-240°C.
Spectre de masse (El) : m/z = 477 (M+)
Spectre RMN 1H (400 MHz, □en ppm, DMSO-d6) : 6,41 (d, J = 8,5 Hz, 1H) ; 6,91 (m large, 1H) ; 7,16 (t large, J = 10,5 Hz, 1H) ; de 7,23 à 7,41 (m masqué, 1H) ; 7,27 (t, J = 7,5 Hz, 1H) ; 7,36 (t, J = 7,5 Hz, 1H) ; 7,47 (d, J = 5,0 Hz, 1H) ; 7,52 (m, 2H) ; 7,62 (m, 2H) ; 7,67 (d, J = 7,5 Hz, 1H) ; 7,78 (d, J = 7,5 Hz, 1H) ; 8,30 (d, J = 5,0 Hz, 1H) ; 9,29 (d, J = 8,5 Hz, 1H) ; 11,85 (m large, 1H) ; 13,4 (m étalé, 1H).

### Exemple 211 : [4-(6-sulfamoyl-1H-benzimidazol-2-yl)-9H-fluorèn-9(R,S)-yl]-amide de l'acide 1H-pyrrolo[2,3-b]pyridine-4-carboxylique

Etape 1 : Dans un ballon de 250 mL, on dissout 0.94 g de 4-sulfonamido-benzène-1,2-diamine dans 15 mL de tétrahydrofurane, puis on ajoute, successivement à 0°C, 1.21 g de chlorure de l'acide fluorèn-4-one-9-carboxylique et 0.42 g d'hydrogénocarbonate de sodium. Après 1 heure d'agitation à température ambiante, le mélange est porté à 40°C pendant 2 heures. On ajouter 10 mL d'eau puis 10 mL d'une solution aqueuse 1 M d'acide chlorhydrique puis on extrait à l'oxyde de diéthyle. La phase aqueuse est décantée, puis amenéer à pH = 10 avec par addition d'une solution aqueuse 1M d'hydroxyde de sodium et extraite avec 3 fois avec 20 mL d'acétate d'éthyle. Après concentration à sec de l'acétate d'éthyle, on obtient ainsi 80 mg d'un mélange brut contenant majoritairement du (2-amino-4-sulfamoyl-phényl)-amide de l'acide 9-oxo-9H-fluorène-4-carboxylique.

Etape 2 : Dans un ballon de 10 mL, le mélange obtenu à l'étape précédente dans 15 mL d'acide acétique et on chauffe à 110°C pendant 2 heures. Après refroidissement et concentration du solvant, on ajoute 5 mL d'eau et la phase aqueuse est amenée à pH = 8-9 par addition d'une solution saturée d'hydrogénocarbonate de potassium. Le solide brut obtenu est purifié par flash-chromatographie sur gel de silice (40-63µm) en éluant avec un mélange de dichlorométhane et d'ammoniac à 7N dans le méthanol (90/10 en volumes). On obtient ainsi 58 mg de 2-(9-oxo-9H-fluorèn-4-yl)-1H-benzimidazole-5-sulfonamide sous forme d'une poudre jaune dont les caractéristiques sont les suivantes :
Spectre de masse (E/I) : m/z = 376(M+)

Etape 3 : On opère comme à l'exemple 5, mais à partir de 56 mg 2-(9-oxo-9H-fluorèn-4-yl)-1H-benzimidazole-5-sulfonamide, obtenu à l'étape précédente, de 31 mg de chlorhydrate d'hydroxylamine et de 62 mg d'acétate de sodium dans 5 mL d'éthanol agités pendant 1h à température ambiante puis à 80°C pendant 3 heures. Après concentration du solvant sous pression réduite, le résidu est repris successivement par de l'eau, puis du toluène, amené à sec et enfin empâté dans de l'oxyde diisopropyle. On obtient ainsi 50 mg de 2-[9(Z,E)-hydroxyimino-9H-fluorèn-4-yl]-1H-benzimidazole-5-sulfonamide, en mélange 50-50 des isomères Z et E, sous forme d'une poudre ambre, utilisée telle quelle à l'étape suivante, dont les caractéristiques sont les suivantes :
Spectre de masse (EI/CI) : m/z = 391 (M+).

Etape 4 : On opère comme dans l'exemple 6. Dans un autoclave de 25 mL, on dissout 50 mg 2-[9(Z,E)-hydroxyimino-9H-fluorèn-4-yl]-1H-benzimidazole-5-sulfonamide, obtenu à l'étape précédente, dans un mélange de 5 mL d'éthanol et 5 mL de tétrahydrofurane, on ajoute une spatule de Nickel activé selon Raney puis on soumet à une pression initiale d'hydrogène de 1 bar et on chauffe l'autoclave à 60° pendant 12 heures. Après refroidissement, le volume d'hydrogène absorbé est de 20 mL. Après filtration du catalyseur, concentration du solvant sous pression réduite et purification par empâtage à l'oxyde diisopropyle, on obtient ainsi 45 mg 2-[9(R,S)-amino-9H-fluorèn-4-yl]-1H-benzimidazole-5-sulfonamide sous forme d'une poudre beige, utilisée telle quelle à l'étape suivante, dont les caractéristiques sont les suivantes :
Spectre de masse (LC/MS) : m/z = 377 (M+)

Etape 5 : On opère comme à l'exemple 14, mais à partir de 35 mg 2-[9(R,S)-amino-9H-fluorèn-4-yl]-1H-benzimidazole-5-sulfonamide, obtenu à l'étape précédente, 16 mg d'acide 1H-pyrrolo-[2,3-b]pyridine-4-carboxylique, 20 mg de chlorhydrate de 1-(3-diméthylaminopropyl)-3-éthylcarbodiimide (EDCI) et 14 mg de 1-hydroxybenzotriazole (HOBT) dans 5 mL de diméthylformamide, pendant 20 heures à température ambiante. Après purification par flash-chromatographie sur gel de silice (40-63µm), en éluant avec un mélange de dichlorométhane et d'ammoniac à 7N dans le méthanol (90/10 en volumes), on obtient ainsi 19 mg de [4-(6-sulfamoyl-1H-benzoimidazol-2-yl)-9H-fluorèn-9(R,S)-yl]-amide de l'acide 1H-pyrrolo[2,3-b]pyridine-4-carboxylique, sous forme d'une poudre ambre dont les caractéristiques sont les suivantes :
Spectre de masse (LC/MS) : m/z = 521 (M+)
Spectre RMN 1H (400 MHz, □ en ppm, DMSO-d6) : pour ce composé, on observe un mélange 50/50 de tautomères avec: 6,41 (d, J = 8,5 Hz, 1H); 6,91 (m large, 1H) ; de 7,23 à 7,38 (m, 4H) ; 7,47 (d, J = 5,0 Hz, 1H) ; de 7,49 à 7,66 (m, 4H) ; 7,70 (d, J = 7,5 Hz, 1H) ; 7,73 à 7,86 (m, 4H) ; 8,15 (m étalé, 1H) ; 8,29 (d, J = 5,0 Hz, 1H) ; 9,29 (d, J = 8,5 Hz, 1H) ; 11,85 (m large, 1H) ; 13,4 (m étalé, 1H).

### Exemple 212 : Synthèse du 4-(4,5-difluoro-1H-benzimidazole-2-yl)-9H-fluorène-9(R,S)-yl]-amide de l'acide 1H-pyrrolo[2,3-b]pyridine-4-carboxylique

Etape 1 : On opère comme à l'étape 1 de l'exemple 1 ,mais à partir de 0,793 g de 1,2-diamino-3,4-difluorobenzène, 1,406 mL de triéthylamine et 1,213g de chlorure de l'acide fluorène-4-one-9-carboxylique dans 25 mL de dichlorométhane. Après 20 heures d'agitation à température ambiante, le précipité formé est filtré, lavé, à l'eau, puis avec une solution aqueuse saturée d'hydrogénocarbonate de sodium à 10% et enfin à l'eau. Après empâtage à l'éther diisopropylique, on obtient ainsi 1.7 g du (6-amino-2,3-difluoro-phényl)amide de l'acide 9-oxo-9H-fluorène-4-carboxylique sous forme d'une poudre orange utilisée telle quelle à l'étape suivante, dont les caractéristiques sont les suivantes :
Spectre de masse (LC/MS/ES/+/-) : m/z = 350 (M+)

Etape 2 : On opère comme dans l'exemple 68. Dans un ballon de 100 mL sous atmosphère d'argon, on chauffe à 115°C pendant 6 heures, une suspension de 1,7 g de (6-amino-2,3-difluoro-phényl)amide de l'acide 9-oxo-9H-fluorène-4-carboxylique, obtenu à l'étape précédente, dans 35 mL d'acide acétique. Après refroidissement, on amène à sec sous pression réduite. On reprend le résidu dans l'eau et on amène à pH = 8 par addition d'une solution aqueuse saturée d'hydrogénocarbonate de sodium à 10%, à l'eau. Le précipité formé est filtré, lave à l'eau, on rince à l'éther diisopropylique, on obtient ainsi 1,5 g de 4-(4,5-difluoro-1H-benzimidazol-2-yl)-9H-fluorène-9-one, sous forme d'une poudre orange utilisée telle quelle à l'étape suivante, dont les caractéristiques sont les suivantes :
Spectre de masse (EI) : m/z = 332 (M+)

Etape 3 : On opère comme à l'exemple 5, mais à partir de 1,5 g de 4-(4,5-difluoro-1H-benzimidazole-2-yl)-9H-fluorène-9-one, obtenue à l'étape précédente, de 0,941g de chlorhydrate d'hydroxylamine et de 1,85 g d'acétate de sodium dans 25 mL d'éthanol agités pendant 20h à température ambiante. Après concentration du solvant sous pression réduite, le résidu est repris par de l'eau, filtré, lavé à l'eau, et empâté à l'éther diisopropylique. On obtient ainsi 1,5 g de 4-(4,5-difluoro-1H-benzimidazol-2-yl)-9H-fluorène-9-one oxime (Z,E) en mélange 50-50 des isomères Z et E, sous forme d'une poudre beige utilisée telle quelle à l'étape suivante.

Etape 4 : On opère comme à l'exemple 6, mais à partir de 4 g de 4-(4,5-difluoro-1H-benzimidazol-2-yl)-9H-fluorène-9-one oxime (Z,E), obtenue à l'étape précédente, et 0,27g de Nickel de Raney dans 35 mL d'éthanol et 35 ml de tétrahydrofurane pendant 10 heures à 60°C sous une pression initiale d'un bar d'hydrogène. Après filtration du catalyseur, et purification par empâtage dans l'oxyde de diisopropyle, on obtient ainsi 1g de 4-(4,5-difluoro-1H-benzimidazo-2-yl)-9H-fluorèn-9(R,S)-yl-amine, sous forme d'une poudre beige utilisée telle quelle à l'étape suivante.

Etape 5 : On opère comme dans l'exemple 14, mais à partir de 333 mg de 4-(4,5-difluoro-1H-benzimidazol-2-yl)-9H-fluorène-9(R,S)-yl-amine, obtenue à l'étape précédente, 162 mg d'acide 1H-pyrrolo-[2,3-b]pyridine-4-carboxylique, 211 mg de chlorhydrate de 1-(3-diméthylaminopropyl)-3-éthylcarbodiimide (EDCI) et 148 mg de 1-hydroxybenzotriazole (HOBT) dans 7 mL de diméthylformamide, pendant 20 heures à température ambiante. Après purification par flash-chromatographie sur gel de silice (40-63µm), en éluant avec un mélange de dichlorométhane et de méthanol ammoniacal 7N (97,5/2,5 en volumes), puis empâtage au dichlorométhane, on obtient ainsi 296 mg de [4-(4,5-difluoro-1H-benzimidazol-2-yl)-9H-fluorène-9(R,S)-yl]-amide de l'acide 1H-pyrrolo[2,3-b]pyridine-4-carboxylique, sous forme d'une poudre blanc-cassé dont les caractéristiques sont les suivantes :
Spectre de masse (LC/MS/ES+/-) : m/z = 477 (M+)
Spectre RMN 1H (400 MHz, □ en ppm, DMSO-d6) : 6,41 (d, J = 8,5 Hz, 1H) ; 6,91 (dd, J = 2,0 et 3,5 Hz, 1H) ; 7,27 (t, J = 7,5 Hz, 1H) ; 7,33 (m partiellement masqué, 1H) ; 7,36 (t, J = 7,5 Hz, 1H) ; 7,44 (m étalé, 1H) ; 7,47 (d, J = 5,0 Hz, 1H) ; 7,50 (d partiellement masqué, J = 8,0 Hz, 1H) ; 7,53 (t, J = 7,5 Hz, 1H) ; 7,63 (m, 2H) ; 7,68 (d, J = 7,5 Hz, 1H) ; 7,79 (d, J = 7,5 Hz, 1H) ; 8,30 (d, J = 5,0 Hz, 1H) ; 9,29 (d, J = 8,5 Hz, 1H) ; 11,85 (m large, 1H) ; 13,4 (m étalé, 1H).

Exemple 213 : Synthèse du [4-(5-trifluorométhoxy-1H-benzimidazol-2-yl) -9H-fluorène-9(R,S)-yl]-amide de l'acide 1H-pyrrolo[2,3-b]pyridine-4-carboxylique Etape 1 : On opère comme à l'étape 1 de l'exemple 1, mais à partir de 1g de 1,2-diamino-4-(trifluorométhoxy)benzène, 1,3 mL de triéthylamine et 1.15 g de chlorure de l'acide fluorène-4-one-9-carboxylique dans 30 mL de dichlorométhane. Après 20 heures d'agitation à température ambiante, le précipité formé est filtré, lavé au dichlorométhane, à l'eau, puis avec une solution aqueuse saturée d'hydrogénocarbonate de sodium à 10% et enfin à l'eau. Après séchage et empâtage à l'éther diisopropylique, on obtient ainsi 485 mg du (2-amino-4-trifluorométhoxy-phényl) amide de l'acide 9-oxo-9H-fluorène-4-carboxylique, sous forme d'une poudre jaune utilisée telle quelle à l'étape suivante, dont les caractéristiques sont les suivantes :
Spectre de masse (EI) : m/z = 350 (M+)

Etape 2 : On opère comme dans l'exemple 68. Dans un ballon de 250 mL sous atmosphère d'argon, on chauffe, à 135°C pendant 2 heures, une solution de 1,12 g de (2-amino-4-trifluorométhoxy-phényl) amide de l'acide 9-oxo-9H-fluorène-4-carboxylique, obtenu à l'étape précédente, dans 50 mL d'acide acétique. Après refroidissement, on amène à sec sous pression réduite. Le produit brut est purifié par flash-chromatographie sur gel de silice (40-63µm) en éluant avec un mélange de dichlorométhane et de méthanol (95/5 en volumes). Après empâtage à l'éther diisopropylique, on obtient 550 mg de 4-(5-trifluorométhoxy-1H-benzimidazol-2-yl)-9H-fluorène-9-one, sous forme d'une poudre jaune pâle utilisée telle quelle à l'étape suivante, dont les caractéristiques sont les suivantes :
Spectre de masse (EI) : m/z = 380 (M+)

Etape 3: On opère comme à l'exemple 5, mais à partir de 1 g de 4-(5-trifluorométhoxy-1H-benzimidazol-2-yl)-9H-fluorène-9-one, obtenue à l'étape précédente, de 580 mg de chlorhydrate d'hydroxylamine et de 1,1 g d'acétate de sodium dans 50 mL d'éthanol agités pendant 20h à température ambiante. Après concentration du solvant sous pression réduite, le résidu est repris successivement par de l'eau, puis du toluène, amené à sec. Le produit brut est purifié par flash-chromatographie sur gel de silice (40-63µm) en éluant avec un mélange de dichlorométhane et de méthanol (98/2 en volumes). On obtient ainsi 730 mg de 4-(5-trifluorométhoxy-1H-benzimidazol-2-yl)-9H-fluorène-9-one oxime (Z,E) en mélange 50-50 des isomères Z et E, sous forme d'une mousse jaune pâle utilisée telle quelle à l'étape suivante, dont les caractéristiques sont les suivantes :
Spectre de masse (EI/LC/MS) : m/z = 395 (M+).

Etape 4 : On opère comme dans l'exemple 6. Dans un autoclave de 111 mL, on dissout 730 mg de 4-(5-trifluorométhoxy-1H-benzimidazol-2-yl)-9H-fluorène-9-one oxime (Z,E), obtenue à l'étape précédente, dans un mélange de 25 mL d'éthanol et 25 mL de tétrahydrofurane, on ajoute une spatule de Nickel activé selon Raney puis on soumet à une pression initiale d'hydrogène de 1 bar et on chauffe l'autoclave à 60° pendant 4 heures. Après refroidissement, le volume d'hydrogène absorbé est de 122 mL. Après filtration du catalyseur sur Célite, le filtrat est concentré sous pression réduite puis repris dans du dichlorométhane et séché sur sulfate de magnésium. On obtient ainsi 640 mg de 4-(5-trifluorométhoxy-1H-benzimidazol-2-yl)-9H-fluorène-9(R,S)-amine, sous forme d'une mousse beige utilisée telle quelle à l'étape suivante, dont les caractéristiques sont les suivantes :
Spectre de masse (LC/MS) : m/z = 381 (M+)

Etape 5 : On opère comme dans l'exemple 14, mais à partir de 300 mg de 4-(5-trifluorométhoxy-1H-benzimidazol-2-yl)-9H-fluorène-9(R,S)-amine, obtenue à l'étape précédente, 140 mg d'acide 1H-pyrrolo-[2,3-b]pyridine-4-carboxylique, 166 mg de chlorhydrate de 1-(3-diméthylaminopropyl)-3-éthylcarbodiimide (EDCI) et 53 mg de 1-hydroxybenzotriazole (HOBT) dans 3 mL de diméthylformamide, pendant 20 heures à température ambiante. On amène à sec le diméthylformamide, on ajoute de l'eau et on essore le précipité formé qui est ensuite lavé à l'eau puis avec une solution saturée d'hydrogénocarbonate de sodium à 10% et de nouveau à l'eau. Le solide brut obtenu est séché puis purifié par flash-chromatographie sur gel de silice (40-63µm) en éluant avec un mélange de dichlorométhane et de méthanol (98/2 puis 95/5 en volumes). Après empâtage à l'éther diisopropylique, on obtient ainsi 213 mg de [4-(5-trifluorométhoxy-1H-benzimidazol-2-yl)-9H-fluorène-9(R,S)-yl]-amide de l'acide 1H-pyrrolo[2,3-b]pyridine-4-carboxylique, sous forme d'une poudre blanche dont les caractéristiques sont les suivantes :
Point de fusion (Kofler) = 204-206°C.
Spectre de masse (LC/MS) : m/z = 525 (M+)
Spectre RMN 1H (400 MHz, □ en ppm, DMSO-d6) : pour ce lot, on observe un mélange de tautomères avec : 6,41 (d, J = 8,5 Hz, 1H) ; 6,91 (dd, J = 2,0 et 3,5 Hz, 1H) ; 7,26 (m, 2H) ; 7,35 (dt, J = 1,5 et 7,5 Hz, 1H) ; 7,47 (d, J = 5,0 Hz, 1H) ; 7,52 (t, J = 7,5 Hz, 1H) ; de 7,57 à 7,80 (m, 7H) ; 8,30 (d, J = 5,0 Hz, 1H) ; 9,28 (d, J = 8,5 Hz, 1H) ; 11,86 (m large, 1H) ; 13,3 (m étalé, 1H).

### Exemple 214, 215, 217 et 218 : séparation des 4 énantiomères du [4-(3H-imidazo[4,5-c]pyridin-2-yl)-9H-fluorèn-9(R,S)-yl]-amide de l'acide 2-(3-acétyl-2,2-diméthyl-cyclobutan-1-yl) acétique

### Etape 1 : Séparation des couples diastéréosiomères

180 mg de [4-(3H-imidazo[4,5-c]pyridin-2-yl)-9H-fluorèn-9(R,S)-yl]-amide de l'acide 2-(3-acétyl-2,2-diméthyl-cyclobutan-1-yl) acétique, obtenu comme à l'exemple 145 sont chromatographiés sur une colonne de silice Chiralpak AD 20µM, de longueur 35 cm et de diamètre 8 cm, en éluant à un débit de 150 mL/mn avec un mélange de n-heptane d'éthanol et de méthanol (85/10/5 en volumes). En récupérant la première fraction éluée, on obtient ainsi 82,6 mg du couple des diastéréoisomères A et C. En récupérant la seconde fraction éluée, on obtient ainsi 85 mg du couple des diastéréoisomères B et D.

### Etape 2 : Dédoublement des couples de diastéréoisomères

On chromatographie, séparément 82,6 mg du couple des diastéréoisomères A et C et 85 mg du couple des diastéréoisomères B et D, sur une colonne de silice Chiralpak OJ-H, de longueur 25 cm et de diamètre 2 cm, en éluant par une phase mobile supercritique constituée de dioxyde de carbone, d'éthanol et d'acide trifluoroacétique (70/30/0,1 en volumes), à un débit de 100 mL/mn sous une pression de 126 bars. On obtient ainsi :
42,3 mg de diastéréosiomère A (exemple 217) : □D20 = +104 +/- 2 ° (c = 0,5 ; DMSO).
37,6 mg de diastéréosiomère C (exemple 215) : □D20 = -81,2 +/- 1,8 ° (c = 0,5 ; DMSO).
42,3 mg de diastéréosiomère B (exemple 214) : □D20 = +102,3 +/- 2 ° (c = 0,5 ; DMSO).
36,4 mg de diastéréosiomère D (exemple 218) : □D20 = -91 +/- 2,1 ° (c = 0,5 ; DMSO).

### Exemple 216 : Synthèse du [4-(5-cyano-1H-benzimidazol-2-yl)-9H-fluorène-9(R,S)-yl]-amide de l'acide 1H-pyrrolo[2,3-b]pyridine-4-carboxylique

Etape 1 : On opère comme à l'étape 1 de l'exemple 1, mais à partir de 2,4 g de 3,4-diaminobenzonitrile et 30 mL de tétrahydrofuranne, 4,6 mL de triéthylamine et 4 g de chlorure de l'acide fluorène-4-one-9-carboxylique dans 115 mL de dichlorométhane. Après 20 heures d'agitation à température ambiante, le précipité formé est filtré, lavé au dichlorométhane, à l'eau, puis avec une solution aqueuse saturée d'hydrogénocarbonate de sodium à 10% et enfin à l'eau. Après empâtage à l'éther diisopropylique, on obtient ainsi 3,8 g du (2-amino-5-cyano-phényl)amide de l'acide 9-oxo-9H-fluorène-4-carboxylique sous forme d'une poudre beige utilisée telle quelle à l'étape suivante, dont les caractéristiques sont les suivantes :
Spectre de masse (EI) : m/z = 339 (M+)

Etape 2 : On opère comme dans l'exemple 68. Dans un ballon de 500 mL sous atmosphère d'argon, on chauffe à 135°C pendant 4 heures, une solution de 3,8 g de (2-amino-5-cyano-phényl)amide de l'acide 9-oxo-9H-fluorène-4-carboxylique, obtenu à l'étape précédente, dans 190 mL d'acide acétique. Après refroidissement, on amène à sec sous pression réduite. Après empâtage à l'éther diisopropylique, on obtient 3,7 g de 4-(5-cyano-1H-benzimidazol-2-yl)-9H-fluorène-9-one, sous forme d'une poudre beige utilisée telle quelle à l'étape suivante, dont les caractéristiques sont les suivantes :
Spectre de masse (EI/LC/MS) : m/z = 321 (M+)
Spectre RMN 1H (400 MHz, □ en ppm, DMSO-d6) : 7,40 (t, J = 7,5 Hz, 1H) ; 7,49 (dt, J = 1,5 et 7,5 Hz, 1H) ; 7,58 (t, J = 7,5 Hz, 1H) ; de 7,65 à 7,72 (m, 3H) ; 7,83 (m, 2H) ; 7,90 (d large, J = 8,0 Hz, 1H) ; 8,26 (s large, 1H) ; 13,0 (m très étalé, 1H).

Etape 3 : On opère comme à l'exemple 5, mais à partir de 3,7 g de 4-(5-cyano-1H-benzimidazol-2-yl)-9H-fluorène-9-one, obtenue à l'étape précédente, de 2,4 g de chlorhydrate d'hydroxylamine et de 4,7 g d'acétate de sodium dans 185 mL d'éthanol agités pendant 20h à température ambiante. Après concentration du solvant sous pression réduite, le résidu est repris successivement par de l'eau, puis du toluène, amené à sec. Le produit brut est purifié par flash-chromatographie sur gel de silice (40-63µm) en éluant avec un mélange de dichlorométhane et d'ammoniac en solution 7N dans le méthanol (98/2 puis 95/5 en volumes). On obtient ainsi 2,11 g de 4-(5-cyano-1H-benzimidazol-2-yl)-9H-fluorène-9-one oxime (Z,E) en mélange 50-50 des isomères Z et E, sous forme d'une poudre beige utilisée telle quelle à l'étape suivante, dont les caractéristiques sont les suivantes :
Spectre de masse (LC/MS/ES+/-) : m/z = 336 (M+).

Etape 4 : Dans un ballon de 100 mL sous atmosphère d'argon, on dissout 1,58 g de mélange équimoléculaire de 4-(5-cyano-1H-benzimidazol-2-yl)-9H-fluorène-9-one oxime (Z,E), obtenue à l'étape précédente, dans un mélange de 10 mL d'éthanol et 10 mL de d'eau et 10mL d'acide acétique, à température ambiante on ajoute en trois fois 1,2 g de zinc, entre chaque ajout, on agite environ une heure à deux heures. On ajoute de la Célite et on filtre. Le filtrat est concentré sous pression. Le produit brut est purifié par flash-chromatographie sur gel de silice (40-63µm) en éluant avec un mélange de dichlorométhane et de méthanol (9/1 en volumes) puis un mélange de dichlorométhane et d'ammoniac en solution 7N dans le méthanol (95/5 en volumes). On obtient ainsi 1,4 g de 4-(5-cyano-1H-benzimidazole-2-yl)-9H-fluorène-9(R,S)-amine sous forme d'une poudre blanche, utilisée telle quelle à l'étape suivante.

Etape 5 : On opère comme dans l'exemple 14, mais à partir de 1,4 g de 4-(5-cyano-1H-benzimidazole-2-yl)-9H-fluorène-9(R,S)-amine, obtenue à l'étape précédente, 720 mg d'acide 1H-pyrrolo-[2,3-b]pyridine-4-carboxylique, 850 mg de chlorhydrate de 1-(3-diméthylaminopropyl)-3-éthylcarbodiimide (EDCI) et 300 mg de 1-hydroxybenzotriazole (HOBT) dans 15 mL de diméthylformamide pendant 20 heures à température ambiante. On amène à sec le diméthylformamide, on ajoute de l'eau, et on essore le précipité formé qui est ensuite lavé à l'eau puis avec une solution saturée d'hydrogénocarbonate de sodium à 10% et de nouveau à l'eau. Le solide brut obtenu est séché puis purifié par flash-chromatographie sur gel de silice (40-63µm) en éluant avec un mélange de dichlorométhane et de méthanol (98/2 puis 95/5 en volumes). Après empâtage à l'éther diisopropylique, on obtient ainsi 1,6 g de [4-(5-cyano-1H-benzimidazol-2-yl)-9H-fluorène-9(R,S)-yl]-amide de l'acide 1H-pyrrolo[2,3-b]pyridine-4-carboxylique, sous forme d'une poudre rosée, dont les caractéristiques sont les suivantes Point de fusion (Kofler) = >260°C.
Spectre de masse (LC/MS/ES+/-) : m/z = 466 (M+)
Spectre RMN 1H (400 MHz, □ en ppm, DMSO-d6) : 6,41 (d, J = 8,5 Hz, 1H) ; 6,91 (dd, J = 2,0 et 3,5 Hz, 1H) ; 7,25 (t, J = 7,5 Hz, 1H) ; 7,35 (t, J = 7,5 Hz, 1H) ; 7,46 (d, J = 5,0 Hz, 1H) ; 7,53 (t, J =7,5 Hz, 1H) ; de 7,57 à 7,67 (m, 4H) ; 7,71 (d, J = 7,5 Hz, 1H) ; 7,77 (d, J = 7,5 Hz, 1H) ; 7,81 (d large, J = 8,5 Hz, 1H) ; 8,22 (s large, 1H) ; 8,30 (d, J = 5,0 Hz, 1H) ; 9,28 (d, J = 8,5 Hz, 1H) ; 11,85 (m large, 1H) ; 13,5 (m très étalé, 1H).

### Exemple 219 : (6-chloro-1H-benzimidazol-2-yl)-9H-fluorèn-9(R,S)-yl]-amide de l'acide 1H-pyrrolo[2,3-b]pyridine-4-carboxylique :

Etape 1 : A un mélange de 2,84 g de chloro-4-diamino-1,2-benzene et de 5,6 mL de triethylamine dans 80 mLde dichlorométhane on additionne sous argon et à température ambiante 4,35 g de chlorure de l'acide 9-fluorénone-4-carboxylique par petites portions en l'espace de 30 minutes. Le milieu réactionnel est agité à température ambiante sous argon durant la nuit. Après ½ heure d'agitation on note l'apparition d'un précipité. Le lendemain le milieu réactionnel est filtré et le solide lavé successivement avec 50 mLde dichlorométhane, 50 mL d'eau, 2 fois 50 mL d'une solution saturée de bicarbonate de sodium et enfin 50 mL d'eau. Le solide jaune est repris avec 100 mL de toluène et l'ensemble évaporé à sec sous vide. Le résidu est trituré dans 150 mL d'éther diisopropylique, filtré et séché sous vide à 40°C pendant 1 nuit. On obtient ainsi 3,05 g de (2-amino-4-chloro-phényl)amide de l'acide 9-oxo-9H-fluorène-4-carboxylique sous forme d'un solide beige, dont les caractéristiques sont les suivantes :
Point de fusion (Kofler) =226°C
CCM (SiO2) Rf=0,6 (méthanol-dichlorométhane 10/90 en volumes)

Etape 2 : Un mélange de 3,0 g de (2-amino-4-chloro-phényl)amide de l'acide 9-oxo-9H-fluorène-4-carboxylique dans 120 mL d'acide acétique glacial est chauffé sous reflux durant 2,5 heures. On laisse revenir à température ambiante et évapore le milieu réactionnel à sec sous vide. Le résidu est repris par 75 mL de dichlorométhane, la phase organique est lavée avec 2 fois 50 mL d'une solution saturée de bicarbonate de sodium, 50 mL d'une solution saturée de chlorure de sodium. Après séchage sur sulfate de magnésium, la phase organique est évaporée à sec sous vide. On obtient ainsi 3,12 g de 4-(6-chloro-1H-benzimidazol-2-yl)-9H-fluorén-9-one sous forme d'une mousse beige, dont les caractéristiques sont les suivantes :
CCM(SiO2) Rf=0,6 (méthanol-dichlorométhane 10/90 en volumes)

Etape 3 : On agite à température ambiante sous argon un mélange de 2,0 g de 4-(6-chloro-1H-benzimidazol-2-yl)-9H-fluorèn-9-one, de 1,26 g de chlorhydrate d'hydroxylamine et de 2,48 g d'acétate de sodium dans 100 ml d'éthanol. Le lendemain on évapore à sec sous vide le milieu réactionnel, reprend le résidu par 100 ml de dichlorométhane et lave avec 100 ml d'eau. La phase organique est séchée sur sulfate de magnésium et évaporée à sec sous vide. Après séchage à 40°C sous vide on obtient 1,88 g de 4-(6-chloro-1H-benzimidazol-2-yl)-9H-fluorèn-9-one oxime (Z, E) sous forme d'un solide beige, dont les caractéristiques sont les suivantes :
Point de fusion (Kofler) = 200°C (décomp).
CCM(SiO2) Rf=0,4 (méthanol-dichlorométhane 10/90 en volumes)
Spectre de RMN 1H (400 MHz - □ en ppm - DMSO-d6) : pour ce lot, nous observons un mélange d'isomères 50 % 50% avec : De 7,21 à 7,41 (m, 3,5H) ; 7,51 (t, J = 7,5 Hz, 0,5H) ; 7,52 (d large, J = 8,0 Hz, 0,5H) ; 7,56 (t, J = 7,5 Hz, 0,5H) ; de 7,63 à 7,77 (m, 3,5H) ; 7,91 (d large, J = 7,5 Hz, 0,5H) ; 8,43 (d large, J = 7,5 Hz, 0,5 H) ; 8,60 (d large, J = 8,0 Hz, 0,5H) ; de 12,5 à 13,5 (m étalé, 2H).

Etape 4 : On agite à température ambiante un mélange de 345 mg de 4-(6-chloro-1H-benzimidazol-2-yl)-9H-fluorèn-9-one oxime (Z, E) dans un mélange de 2 mL d'acide acétique, de 2 mL d'éthanol et de 2 mL d'eau en présence de 65 mg de zinc en poudre. Après 1 heure et 3 heures on rajoute encore chaque fois 65 mg de zinc en poudre et maintient l'agitation environ 4 heures. On rajoute de la cellite, filtre sur verre fritté et lave le précipité avec 200 mL d'un mélange de méthanol et de dichlorométhane (20/80 en volumes). Le filtrat est évaporé à sec sous vide, on rajoute 2 fois 50 mL de toluène et réévapore à chaque fois à sec sous vide. Le résidu est chromatographié sur gel de silice en éluant avec un mélange de dichlorométhane-solution 7N d'ammoniac dans le méthanol (90/10 en volumes). On obtient ainsi 275 mg de 4-(6-chloro-1H-benzimidazol-2-yl)-9H-fluorèn-9(R,S)-amine sous forme d'une mousse beige, dont les caractéristiques sont les suivantes :
CCM(SiO2) Rf=0,5 (méthanol-dichlorométhane 20/80 en volumes).

Etape 5 : On agite à température ambiante un mélange de 238 mg de 4-(6-chloro-1H-benzimidazol-2-yl)-9H-fluorèn-9(R,S)-amine, de 152 mg de chlorhydrate de 1-(3-diméthylaminopropyl)-3-éthylcarbodiimide (EDCI), de 106 mg de de 1-hydroxybenzotriazole (HOBT) et de 128 mg d'acide 1H-pyrrolo[2,3-b]pyridine-4-carboxylique dans 5 mL de diméthylformamide sec. Après 2 heures on évapore à sec sous vide le milieu réactionnel, reprend le résidu par 50 mL d'une solution saturée de bicarbonate de sodium, triture la suspension et filtre le précipité sur verre fritté. Le solide est lavé avec 80 mL de solution saturée de bicarbonate de sodium puis 80 mL d'eau. Le solide est repris dans 75 mL de toluène et l'ensemble évaporé à sec sous vide. Le solide est chromatographié sur gel de silice en éluant avec un mélange de méthanol et de dichlorométhane (7,5/92,5 en volumes). On obtient ainsi 180 mg de (6-chloro-1H-benzimidazol-2-yl)-9H-fluorèn-9(R,S)-yl]-amide de l'acide 1H-pyrrolo[2,3-b]pyridine-4-carboxylique, sous forme d'une mousse beige dont les caractéristiques sont les suivantes :
Point de fusion (Kofler) = 212°C (décomp).
CCM(Si02) Rf=0,3(méthanol-dichlorométhane 10/90 en volumes)

Spectre RMN (400 MHz - □ en ppm - DMSO-d6) 6,41 (d, J = 8,5 Hz, 1H) ; 6,91 (dd, J = 2,0 et 3,5 Hz, 1H) ; 7,26 (t, J = 7,5 Hz, 1H) ; 7,31 (dd, J = 2,0 et 8,5 Hz, 1H) ; 7,35 (t, J = 7,5 Hz, 1H) ; 7,47 (d, J = 5,0 Hz, 1H) ; 7,52 (t, J = 7,5 Hz, 1H) ; 7,55 (d, J = 8,0 Hz, 1H) ; 7,62 (m, 2H) ; de 7,64 à 7,78 (m étalé, 2H) ; 7,66 (d, J = 7,5 Hz, 1H) ; 7,77 (d, J = 7,5 Hz, 1H) ; 8,29 (d, J = 5,0 Hz, 1H) ; 9,28 (d, J = 8,5 Hz, 1H) ; 11,85 (m large, 1H) ; 13,15 (m étalé, 1H).

### Exemple 220: (6-bromo-1H-benzimidazol-2-yl)-9H-fluorèn-9(R,S)-yl]-amide de l'acide 1H-pyrrolo[2,3-b]pyridine-4-carboxylique :

Etape 1 : A un mélange de 2 g de bromo-4-diamino-1,2-benzene et de 3,0 mL de triéthylamine dans 60 mL de dichlorométhane on additionne sous argon et à température ambiante 2,36 g de chlorure de l'acide 9-fluorénone-4-carboxylique par petites portions en l'espace de 20 minutes. Le milieu réactionnel est agité à température ambiante sous argon durant la nuit. Après ½ heure d'agitation on note l'apparition d'un précipité. Le lendemain le milieu réactionnel est filtré et le solide lavé successivement avec 50 mL de dichlorométhane, 50 mL d'eau, 2 fois 50 mL d'une solution saturée de bicarbonate de sodium et enfin 50 mL d'eau. Le solide jaune est trituré dans 100 mL d'éther diisopropylique, filtré et séché sous vide à 40°C pendant 1 nuit. On obtient ainsi 1.61 g de (2-amino-4-bromo-phényl)amide de l'acide 9-oxo-9H-fluorène-4-carboxylique sous forme d'un solide jaune pâle dont les caractéristiques sont les suivantes :
Point de fusion (Kofler) = 252°C
CCM (SiO2) Rf=0.5 (méthanol-dichlorométhane 10/90 en volumes)

Etape 2: Un mélange de 1.61 g de (2-amino-4-bromo-phényl)amide de l'acide 9-oxo-9H-fluorène-4-carboxylique dans 80 mL d'acide acétique glacial est chauffé sous reflux durant 2,5 heures. On laisse revenir à température ambiante et évapore le milieu réactionnel à sec sous vide. Le résidu est repris par 50 mL de dichlorométhane, la phase organique est lavée avec 2 fois 50 mL d'une solution saturée de bicarbonate de sodium, 50 mL d'une solution saturée de chlorure de sodium. Après séchage sur sulfate de magnésium, la phase organique est évaporée à sec sous vide. Le résidu est chromatographié sur gel de silice en éluant avec un mélange de méthanol et de dichlorométhane (5/95 en volumes). On obtient ainsi 1,5 g de 4-(6-bromo-1H-benzimidazol-2-yl)-9H-fluorèn-9-one, dont les caractéristiques sont les suivantes :
CCM(Si02) Rf=0,6 (méthanol-dichlorométhane 10: 90 en volumes)
Spectre de RMN 1H (400 MHz - □ en ppm - DMSO-d6) : 7,39 (dt, J = 1,0 et 7,5 Hz, 1H) ; 7,44 (dd, J = 2,0 et 8,5 Hz, 1H) ; 7,49 (dt, J = 1,0 et 7,5 Hz, 1H) ; 7,57 (t, J = 7,5 Hz, 1H) ; de 7,63 à 7,70 (m, 3H) ; 7,81 (dd, J = 1,0 et 7,5 Hz, 1H) ; 7,86 (dd, J = 1,0 et 7,5 Hz, 1H) ; 7,90 (d large, J = 2,0 Hz, 1H) ; 13,2 (m étalé, 1H).

Etape 3 : On agite à température ambiante sous argon un mélange de 1.13 g de 4-(6-bromo-1H-benzimidazol-2-yl)-9H-fluorèn-9-one, de 0,628 g de chlorhydrate d'hydroxylamine et de 1.24 g d'acétate de sodium dans 50 mL d'éthanol. Le lendemain on évapore à sec sous vide le milieu réactionnel, reprend le résidu par 100 mL d'eau, filtre le solide puis le lave avec 100 mLd'eau. Après séchage à 40°C sous vide on obtient 1.17 g de 4-(6-bromo-1H-benzimidazol-2-yl)-9H-fluorèn-9-one oxime (Z, E), dont les caractéristiques sont les suivantes :
CCM(SiO2) Rf=0,4 (méthanol-dichlorométhane 10/90 en volumes)

Etape 4 : On agite à température ambiante un mélange de 390 mg de 4-(6-bromo-1H-benzimidazol-2-yl)-9H-fluorèn-9-one oxime (Z, E) dans un mélange de 2 mL d'acide acétique, 2 mL d'éthanol et 2 mL d'eau en présence de 65 mg de zinc en poudre. Après ½ heure on rajoute encore 65 mg de zinc en poudre et maintient l'agitation environ 3 heures. On rajoute de la cellite, filtre sur verre fritté et lave le précipité avec 200 mL d'un mélange de méthanol et de dichlorométhane (20/80 en volumes). Le filtrat est évaporé à sec sous vide, on rajoute 2 fois 50 mL de toluène et réévapore à chaque fois à sec sous vide. Le résidu est chromatographié sur gel de silice en éluant avec un mélange de dichlorométhane et d'une solution 7N d'ammoniac dans le méthanol (90/10 en volumes). On obtient ainsi 69 mg de 4-(6-bromo-1H-benzimidazol-2-yl)-9H-fluorèn-9(R,S)-amine sous forme d'une mousse beige, dont les caractéristiques sont les suivantes :
CCM(Si02) Rf=0,5 (méthanol-dichlorométhane 20/80 en volumes).

Etape 5 : On agite à température ambiante un mélange de 220 mg de 4-(6-bromo-1H-benzimidazol-2-yl)-9H-fluorèn-9(R,S)-amine, 123 mg chlorhydrate de 1-(3-diméthylaminopropyl)-3-éthylcarbodiimide (EDCI), 87 mg de 1-hydroxybenzotriazole (HOBT) et de 105 mg d'acide 1H-pyrrolo[2,3-b]pyridine-4-carboxylique dans 5 mL de diméthylformamide sec. Après 1,5 heures on évapore à sec sous vide le milieu réactionnel, reprend le résidu par 50 mL d'une solution saturée de bicarbonate de sodium, triture la suspension et filtre le précipité sur verre fritté. Le solide est lavé avec 75 mL de solution saturée de bicarbonate de sodium puis 100 mL d'eau. Le solide est repris dans du toluène et l'ensemble évaporé à sec sous vide. Le solide est chromatographié sur gel de silice en éluant avec un mélange de méthanol et de dichlorométhane (5/95 en volumes). On obtient ainsi 214 mg de (6-bromo-1H-benzimidazol-2-yl)-9H-fluorèn-9(R,S)-yl]-amide de l'acide 1H-pyrrolo[2,3-b]pyridine-4-carboxylique sous forme d'un solide blanc cassé, dont les caractéristiques sont les suivantes :
Point de fusion (Kofler) = 206°C (décomp).
CCM(Si02) Rf=0,4 (méthanol-dichlorométhane 10/90 en volumes)
Spectre RMN (400 MHz - □en ppm - DMSO-d6) : 6,41 (d, J = 8,5 Hz, 1H) ; 6,91 (dd, J = 2,0 et 3,5 Hz, 1H) ; 7,26 (t large, J = 7,5 Hz, 1H) ; 7,35 (d large, J = 7,5 Hz, 1H) ; 7,42 (dd, J = 2,0 et 8,5 Hz, 1H) ; 7,47 (d, J = 5,0 Hz, 1H) ; 7,51 (t, J = 7,5 Hz, 1H) ; 7,56 (d large, J = 7,5 Hz, 1H) ; de 7,60 à 7,69 (m, 4H) ; 7,76 (d large, J = 7,5 Hz, 1H) ; 7,87 (m étalé, 1H) ; 8,30 (d, J = 5,0 Hz, 1H) ; 9,28 (d, J = 8,5 Hz, 1H) ; 10,85 (m large, 1H) ; 13,15 (m étalé, 1H) .

### Exemple 221 : Synthèse du 4-(5-fluoro-6-morpholino-1H-benzimidazole-2-yl)-9H-fluorène-9(R,S)-yl]-amide de l'acide 1H-pyrrolo[2,3-b]pyridine-4-carboxylique

Etape 1 : On opère comme à l'étape 1 de l'exemple 1, mais à partir de 1,5 g de 1,2-diamino-4-fluoro-5-morpholino-benzène dans 50 mL de dichlorométhane et 15 mL de tétrahydrofurane, 1,95 mL de triéthylamine et 1,7 g de chlorure de l'acide fluorène-4-one-9-carboxylique. Après 20 heures d'agitation à température ambiante, le précipité formé est filtré, lavé au dichlorométhane, à l'eau, puis avec une solution aqueuse saturée d'hydrogénocarbonate de sodium à 10% et enfin à l'eau. Après empâtage à l'éther diisopropylique, on obtient ainsi 2,2 g du (2-amino-5-fluoro-4-morpholino-phényl)amide de l'acide 9-oxo-9H-fluorène-4-carboxylique sous forme d'une poudre orange utilisée telle quelle à l'étape suivante, dont les caractéristiques sont les suivantes :
Spectre de masse (LC/MS/ES/+/-) : m/z = 417 (M+)

Etape 2 : On opère comme dans l'exemple 68. Dans un ballon de 500 mL sous atmosphère d'argon, on chauffe à 135°C pendant 4 heures, une solution de 2,2 g de(2-amino-5-fluoro-4-morpholino-phényl)amide de l'acide 9-oxo-9H-fluorène-4-carboxylique, obtenu à l'étape précédente, dans 110 mL d'acide acétique. Après refroidissement, on amène à sec sous pression réduite. Le solide brut obtenu est purifié par flash-chromatographie sur gel de silice (40-63µm) en éluant avec un mélange de dichlorométhane et d'ammoniac en solution 7N dans le méthanol (98/2 en volumes). Après empâtage à l'éther diisopropylique, on obtient 1,8 g de 4-(5-fluoro-6-morpholino-1H-benzimidazol-2-yl)-9H-fluorène-9-one, sous forme d'une poudre jaune utilisée telle quelle à l'étape suivante, dont les caractéristiques sont les suivantes :
Spectre de masse (LC/MS/ES/+/-) : m/z = 399 (M+)

Etape 3 : On opère comme à l'exemple 5, mais à partir de 1,8 g de 4-(5-fluoro-6-morpholino-1H-benzimidazol-2-yl)-9H-fluorène-9-one, obtenue à l'étape précédente, de 956 mg de chlorhydrate d'hydroxylamine et de 1,9 g d'acétate de sodium dans 95 mL d'éthanol agités pendant 20h à température ambiante puis chauffés à 60°C pendant 2 heures. Après concentration du solvant sous pression réduite, le résidu est repris successivement par de l'eau, puis du toluène, amené à sec. Le produit brut est purifié par flash-chromatographie sur gel de silice (40-63µm) en éluant avec un mélange de dichlorométhane et de méthanol (98/2 en volumes) puis avec un mélange de dichlorométhane et d'ammoniac en solution 7N dans le méthanol (98/2 puis 95/5 en volumes. Après empâtage à l'éther diisopropylique, on obtient ainsi 1,7 g de 4-(5-fluoro-6-morpholino-1H-benzimidazol-2-yl)-9H-fluorène-9-one oxime (Z,E) en mélange 50-50 des isomères Z et E, sous forme d'une poudre jaune pâle utilisée telle quelle à l'étape suivante, dont les caractéristiques sont les suivantes :
Spectre de masse (LC/MS/ES+/-) : m/z = 414 (M+).

Etape 4 : On opère comme dans l'exemple 6. Dans un autoclave de 211 mL, on dissout 1,7 g de 4-(5-fluoro-6-morpholino-1H-benzimidazol-2-yl)-9H-fluorène-9-one oxime (Z,E), obtenue à l'étape précédente, dans un mélange de 58 mL d'éthanol et 58 mL de tétrahydrofurane, on ajoute une spatule de Nickel activé selon Raney puis on soumet à une pression initiale d'hydrogène de 1 bar et on chauffe l'autoclave à 60° pendant 5 heures. Après refroidissement, le volume d'hydrogène absorbé est de 250 mL. Après filtration sur Célite, le filtrat est concentré sous pression réduite puis repris dans du dichlorométhane et séché sur sulfate de magnésium. Le produit brut est purifié par flash-chromatographie sur gel de silice (40-63µm) en éluant avec un mélange de dichlorométhane et d'ammoniac en solution 7N dans le méthanol (9/1 en volumes). Après empâtage à l'éther diisopropylique, on obtient ainsi 732 mg de 4-(5-fluoro-6-morpholino-1H-benzimidazol-2-yl)-9H-fluorène-9(R,S)-amine sous forme d'une poudre blanche utilisée telle quelle à l'étape suivante, dont les caractéristiques sont les suivantes :
Spectre de masse (EI/CI) : m/z = 400 (M+)

Etape 5 : On opère comme dans l'exemple 14, mais à partir de 350 mg de 4-(5-fluoro-6-morpholino-1H-benzimidazol-2-yl)-9H-fluorène-9(R,S)-amine, obtenue à l'étape précédente, 156 mg d'acide 1H-pyrrolo-[2,3-b]pyridine-4-carboxylique, 184 mg de chlorhydrate de 1-(3-diméthylaminopropyl)-3-éthylcarbodiimide (EDCI) et 59 mg de 1-hydroxybenzotriazole (HOBT) dans 3,5 mL de diméthylformamide, pendant 20 heures à température ambiante. On amène à sec le diméthylformamide, on ajoute de l'eau, et on essore le précipité formé qui est ensuite lavé à l'eau puis avec une solution saturée d'hydrogénocarbonate de sodium à 10% et de nouveau à l'eau. Le solide brut obtenu est séché puis purifié par flash-chromatographie sur gel de silice (40-63µm) en éluant avec un mélange de dichlorométhane et de méthanol (95/5 puis 9/1 en volumes) puis de dichlorométhane et d'ammoniac en solution 7N dans le méthanol (9/1 puis 85/15 en volumes). Après empâtage à l'éther diisopropylique, on obtient ainsi 127 mg de [4-(5-fluoro-6-morpholino-1H-benzimidazol-2-yl)-9H-fluorène-9(R,S)-yl]amide de l'acide 1H-pyrrolo[2,3-b]pyridine-4-carboxylique, sous forme d'une poudre blanche dont les caractéristiques sont les suivantes :
Spectre de masse (LC/MSIES+/-) : m/z = 544 (M+)
Spectre RMN 1H (400 MHz, d en ppm, DMSO-d6) : pour ce composé, on observe un mélange de tautomères avec : 3,05 (m, 4H) ; 3,81 (m, 4H) ; 6,40 (d, J = 8,5 Hz, 1H) ; 6,91 (m large, 1H) ; de 7,10 à 7,66 (m, 9H) ; 7,46 (d, J = 5,0 Hz, 1H) ; 7,74 (d large, J = 7,5 Hz, 1H) ; 8,29 (d, J = 5,0 Hz, 1H) ; 9,27 (d, J = 8,5 Hz, 1H) ; 11,85 (m large, 1H) ; 12,85 (m large, 1 H).

### Exemple 222 : Synthèse du 4-(6-chloro-5-fluoro-1H-benzimidazole-2-yl)-9H-fluorène-9(R,S)-yl]-amide de l'acide 1H-pyrrolo[2,3-b]pyridine-4-carboxylique

Etape 1 : On opère comme à l'étape 1 de l'exemple 1 ,mais à partir de 1 g de 1,2-diamino-4-chloro-5-fluoro-benzène, 1,75 mL de triéthylamine et 1,5 g de chlorure de l'acide fluorène-4-one-9-carboxylique dans 45 mL de dichlorométhane. Après 20 heures d'agitation à température ambiante, le précipité formé est filtré, lavé au dichlorométhane, à l'eau, puis avec une solution aqueuse saturée d'hydrogénocarbonate de sodium à 10% et enfin à l'eau. Après empâtage à l'éther diisopropylique, on obtient ainsi 1,5 g du (2-amino-5-chloro-4-fluoro-phényl)amide de l'acide 9-oxo-9H-fluorène-4-carboxylique sous forme d'une poudre jaune utilisée telle quelle à l'étape suivante, dont les caractéristiques sont les suivantes :
Spectre de masse (LC/MS/ES/+/-) : m/z = 366 (M+)

Etape 2 : On opère comme dans l'exemple 68. Dans un ballon de 500 mL sous atmosphère d'argon, on chauffe à 135°C pendant 4 heures, une solution de 1,5 g de(2-amino-5-chloro-4-fluoro-phényl)amide de l'acide 9-oxo-9H-fluorène-4-carboxylique, obtenu à l'étape précédente, dans 75 mL d'acide acétique. Après refroidissement, on amène à sec sous pression réduite. Après empâtage à l'éther diisopropylique, on obtient 1,3 g de 4-(6-chloro-5-fluoro-1H-benzimidazole-2-yl)-9H-fluorène-9-one, sous forme d'une poudre jaune utilisée telle quelle à l'étape suivante, dont les caractéristiques sont les suivantes :
Spectre de masse (EI) : m/z = 348 (M+)
Spectre RMN 1H (400 MHz, □ en ppm, DMSO-d6) : 7,40 (t, J = 7,5 Hz, 1H) ; 7,49 (t, J = 7,5 Hz, 1H) ; 7,57 (t, J = 7,5 Hz, 1H) ; 7,67 (m, 2H) ; 7,74 (d, J = 9,5 Hz, 1H) ; 7,81 (d, J = 8,0 Hz, 1H) ; 7,86 (d, J = 8,0 Hz, 1H) ; 7,91 (d, J = 7,0 Hz, 1H) ; 13,0 (m très étalé, 1H).

Etape 3 : On opère comme à l'exemple 5, mais à partir de 1,3 g de 4-(6-chloro-5-fluoro-1H-benzimidazole-2-yl)-9H-fluorène-9-one, obtenue à l'étape précédente, de 800 mg de chlorhydrate d'hydroxylamine et de 1,6 g d'acétate de sodium dans 70 mL d'éthanol agités pendant 20h à température ambiante. Après concentration du solvant sous pression réduite, le résidu est repris successivement par de l'eau, puis du toluène, amené à sec. Après empâtage à l'éther diisopropylique, on obtient ainsi 1,1 g de 4-(6-chloro-5-fluoro-1H-benzimidazole-2-yl)-9H-fluorène-9-one oxime (Z,E) en mélange 50-50 des isomères Z et E, sous forme d'une poudre blanc_cassé utilisée telle quelle à l'étape suivante, dont les caractéristiques sont les suivantes :
Spectre de masse (LC/MS/EI) : m/z = 363 (M+).

Etape 4 : On opère comme dans l'exemple 216. Dans un ballon de 100 mL sous atmosphère d'argon, on dissout 1,1 g de 4-(6-chloro-5-fluoro-1H-benzimidazol-2-yl)-9H-fluorène-9-one oxime (Z,E), obtenue à l'étape précédente, dans un mélange de 6,7 mL d'éthanol et 6,7 mL de d'eau et 6,7mL d'acide acétique, à température ambiante on ajoute en trois fois 782 mg de zinc, entre chaque ajout, on agite environ une heure à deux heures. On ajoute de la Célite et on filtre. Le filtrat est concentré sous pression. Le produit brut est purifié par flash-chromatographie sur gel de silice (40-63µm) en éluant avec un mélange de dichlorométhane et d'ammoniac en solution 7N dans le méthanol (9/1 en volumes). Après empâtage à l'éther diisopropylique, on obtient ainsi 860 mg de 4-(6-chloro-5-fluoro-1H-benzimidazole-2-yl)-9H-fluorène-9(R,S)-amine sous forme d'une poudre blanc cassé, utilisée telle quelle à l'étape suivante, dont les caractéristiques sont les suivantes :
Spectre de masse (LC/MS/ES/+/-) : m/z = 349 (M+)

Etape 5 : On opère comme dans l'exemple 14, mais à partir de 350 mg de 4-(6-chloro-5-fluoro-1H-benzimidazol-2-yl)-9H-fluorène-9(R,S)-amine, obtenue à l'étape précédente, 178 mg d'acide 1H-pyrrolo-[2,3-b]pyridine-4-carboxylique, 211 mg de chlorhydrate de 1-(3-diméthylaminopropyl)-3-éthylcarbodiimide (EDCI) et 67 mg de 1-hydroxybenzotriazole (HOBT) dans 3,5 mL de diméthylsulfoxyde, pendant 20 heures à température ambiante. On ajoute de l'eau, et on essore le précipité formé qui est ensuite lavé à l'eau puis avec une solution saturée d'hydrogénocarbonate de sodium à 10% et de nouveau à l'eau. Le solide brut obtenu est séché puis purifié par flash-chromatographie sur gel de silice (40-63µm) en éluant avec un mélange de dichlorométhane et de méthanol (98/2 puis 95/5 en volumes). Après empâtage à l'éther diisopropylique, on obtient ainsi 296 mg de 4-(6-chloro-5-fluoro-1H-benzimidazole-2-yl)-9H-fluorène-9(R,S)-yl]-amide de l'acide 1H-pyrrolo[2,3-b]pyridine-4-carboxylique, sous forme d'une poudre blanc cassé dont les caractéristiques sont les suivantes :
Spectre de masse (LC/MS/ES+/-) : m/z = 493 (M+)
Spectre RMN 1H (400 MHz, □ en ppm, DMSO-d6) : 6,40 (d, J = 8,5 Hz, 1H) ; 6,91 (dd, J = 2,0 et 3,5 Hz, 1H) ; 7,26 (t large, J = 7,5 Hz, 1H) ; 7,35 (t large, J = 7,5 Hz, 1H) ; 7,46 (d, J = 5,0 Hz, 1H) ; 7,52 (t, J = 7,5 Hz, 1H) ; 7,57 (d large, J = 8,0 Hz, 1H) ; 7,62 (m, 2H) ; 7,66 (d large, J = 7,5 Hz, 1H) ; 7,72 (d large, J = 9,5 Hz, 1H) ; 7,77 (d, J = 8,0 Hz, 1H) ; 7,88 (m étalé, 1H) ; 8,30 (d, J = 5,0 Hz, 1H) ; 9,28 (d, J = 8,5 Hz, 1H) ; 11,85 (m large, 1H) ; 13,25 (m très étalé, 1H).

### Exemple 223 : [4-(4,5,6,7-tétrahydro-1H-benzimidazol-2-yl)-9H-fluorèn-9-yl]-amide de l'acide 1H-pyrrolo[2,3-b]pyridine-4-carboxylique

Etape 1 : On opère comme à l'étape 1 de l'exemple 1, mais à partir de 1,29 g de cis-cyclohexane-1,2-diamine dans 100 mL de dichlorométhane, 1,52 g de triéthylamine et 1,82 g de chlorure de l'acide fluorène-4-one-9-carboxylique pendant 20 heures à température ambiante. Après traitement comme à l'étape 1 de l'exemple 1, on obtient 700 mg de (2-amino-cyclohexyl)-amide de l'acide 9-oxo-9H-fluorène-4-carboxylique, sous forme d'une poudre jaune utilisée telle quelle à l'étape suivante, dont les caractéristiques sont les suivantes :
Spectre de masse (LC/MS/ES/+/-) : m/z = 321 (M+)

Etape 2 : Dans un ballon de 25 mL, on chauffe à 150°C pendant 30 minutes, 0,68 g de (2-amino-cyclohexyl)-amide de l'acide 9-oxo-9H-fluorène-4-carboxylique, obtenu à l'étape précédente, dans 5 mL d'acide polyphosphorique (PPA). Après refroidissement, on neutralise à pH = 6 par addition d'une solution aqueuse à 38% d'hydroxyde de sodium. Le précipité formé est filtré, puis purifié par flash-chromatographie sur gel de silice (40-63µm), en éluant avec un mélange de dichlorométhane et de méthanol (90/10 en volumes). On obtient ainsi 0,36 g de 4-(3a,4,5,6,7,7a-hexahydro-1H-benzimidazol-2-yl)-9H-fluorèn-9-one, sous forme d'une poudre jaune utilisée telle quelle à l'étape suivante, dont les caractéristiques sont les suivantes :
Spectre de masse (LC/MS/ES/+/-) : m/z = 303 (M+)

Etape 3: Dans un tube scellé de 20 mL, on dissout 330 mg de 4-(3a,4,5,6,7,7a-hexahydro-1H-benzimidazol-2-yl)-9H-fluorèn-9-one, obtenue à l'étape précédente, dans 12 mL de diméthylsulfoxyde puis on chauffe à 200°C pendant une heure. Après refroidissement, on ajoute 30 mL d'eau puis on extrait 3 fois par 100 mL d'acétate d'éthyle. Le solide brut obtenu est purifié par flash-chromatographie sur gel de silice (40-63µm), en éluant avec un mélange de dichlorométhane et de méthanol (97/3 en volumes). On obtient ainsi 210 mg de 4-(4,5,6,7-tétrahydro-1H-benzimidazol-2-yl)-9H-fluorèn-9-one, sous forme d'une poudre jaune utilisée telle quelle à l'étape suivante, dont les caractéristiques sont les suivantes :
Spectre de masse (LC/MS/ES/+/-) : m/z = 301 (M+)

Etape 4 : On opère comme à l'exemple 5, mais à partir de 200 mg de 4-(4,5,6,7-tétrahydro-1H-benzimidazol-2-yl)-9H-fluorèn-9-one, obtenue à l'étape précédente, de 139 mg de chlorhydrate d'hydroxylamine et de 273 mg d'acétate de sodium dans 22 mL d'éthanol agités pendant 1h à température ambiante puis pendant 3 heures à 80°C. Après concentration du solvant sous pression réduite, le résidu est repris successivement par de l'eau, puis du toluène, amené à sec et enfin empâté à l'oxyde de diisopropyle. On obtient ainsi 44 mg de 4-(4,5,6,7-tétrahydro-1H-benzimidazol-2-yl)-9H-fluorèn-9-one oxime(Z,E), en mélange 50-50 des isomères Z et E, sous forme d'une poudre ambre utilisée telle quelle à l'étape suivante, dont les caractéristiques sont les suivantes :
Spectre de masse (EI/CI) : m/z = 316 (M+).

Etape 5 : On opère comme dans l'exemple 6. Dans un autoclave de 25 mL, on dissout 43 mg 4-(4,5,6,7-tétrahydro-1H-benzimidazol-2-yl)-9H-fluorèn-9-one oxime(Z,E), obtenue à l'étape précédente, dans un mélange de 4 mL d'éthanol et 4 mL de tétrahydrofurane, on ajoute une spatule de Nickel activé selon Raney puis on soumet à une pression initiale d'hydrogène de 1 bar et on chauffe l'autoclave à 60° pendant 12 heures. Après refroidissement, le volume d'hydrogène absorbé est de 12 mL. Après filtration du catalyseur, concentration à sec sous pression réduite et purification par empâtage à l'oxyde diisopropyle, on obtient ainsi 40 mg de 4-(4,5,6,7-tétrahydro-1H-benzimidazol-2-yl)-9H-fluorèn-9(R,S)-yl-amine, sous forme d'une poudre beige utilisée telle quelle à l'étape suivante, dont les caractéristiques sont les suivantes :
Spectre de masse (LC/MS) : m/z = 302 (M+)

Etape 6 : On opère comme à l'exemple 14, mais à partir de 35 mg de 4-(4,5,6,7-tétrahydro-1H-benzimidazol-2-yl)-9H-fluorèn-9(RS)-yl-amine, obtenue à l'étape précédente, 20 mg d'acide 1H-pyrrolo-[2,3-b]pyridine-4-carboxylique, 26 mg de chlorhydrate de 1-(3-diméthylaminopropyl)-3-éthylcarbodiimide (EDCI) et 18 mg de 1-hydroxybenzotriazole (HOBT) dans 4 mL de diméthylformamide, pendant 20 heures à température ambiante. Après purification par flash-chromatographie sur gel de silice (70-230 Mesh), en éluant avec un mélange de dichlorométhane et d'ammoniac à 7N dans le méthanol (90/10 en volumes), on obtient ainsi 19 mg de [4-(4,5,6,7-tétrahydro-1H-benzimidazol-2-yl)-9H-fluorèn-9-yl]-amide de l'acide 1H-pyrrolo[2,3-b]pyridine-4-carboxylique, sous forme d'une poudre ambre dont les caractéristiques sont les suivantes :
Spectre de masse (LC/MS) : m/z = 446 (M+)
Spectre RMN 1H (400 MHz, □ en ppm, DMSO-d6) : 1,83 (m, 4H) ; de 2,55 à 2,67 (m large, 4H) ; 6,34 (d, J = 8,5 Hz, 1H) ; 6,90 (dd, J = 1,5 et 3,5 Hz, 1H) ; de 7,26 à 7,35 (m, 2H) ; 7,39 (t, J = 7,5 Hz, 1H) ; de 7,44 à 7,49 (m, 2H) ; de 7,50 à 7,60 (m, 2H) ; 7,61 (dd, J = 2,0 et 3,5 Hz, 1H) ; 8,10 (d large, J = 8,0 Hz, 1H); 8,28 (d, J = 5,0 Hz, 1H); 9,22 (d, J = 8,5 Hz, 1H) ; 11,85 (m large, 1H) ; 12,0 (s, 1H).

### Exemple 224 : Synthèse du 4-(5,6-difluoro-1H-benzimidazole-2-yl)-9H-fluorène-9(R,S)-yl]-amide de l'acide 1H-pyrrolo[2,3-b]pyridine-4-carboxylique

Etape 1 : On opère comme à l'étape 1 de l'exemple 1, mais à partir de 1 g de 1,2-diamino-4,5-difluoro-benzène, 1,9 mL de triéthylamine et 1,7 g de chlorure de l'acide fluorène-4-one-9-carboxylique dans 50 mL de dichlorométhane. Après 20 heures d'agitation à température ambiante, le précipité formé est filtré, lavé au dichlorométhane, à l'eau, puis avec une solution aqueuse saturée d'hydrogénocarbonate de sodium à 10% et enfin à l'eau. Après empâtage à l'éther diisopropylique, on obtient ainsi 1,4 g du (2-amino-4,5-difluoro-phényl)amide de l'acide 9-oxo-9H-fluorène-4-carboxylique sous forme d'une poudre jaune utilisée telle quelle à l'étape suivante, dont les caractéristiques sont les suivantes :
Spectre de masse (LC/MS/ES/+/-) : m/z = 350 (M+)

Etape 2 : On opère comme dans l'exemple 68. Dans un ballon de 250 mL sous atmosphère d'argon, on chauffe à 135°C pendant 4 heures, une solution de 1,4 g de (2-amino-4,5-difluoro-phényl)amide de l'acide 9-oxo-9H-fluorène-4-carboxylique, obtenu à l'étape précédente, dans 70 mL d'acide acétique. Après refroidissement, on amène à sec sous pression réduite. Le solide brut obtenu est purifié par flash-chromatographie sur gel de silice (40-63µm) en éluant avec un mélange de dichlorométhane et d'ammoniac en solution 7N dans le méthanol (98/2 en volumes). Après empâtage à l'éther diisopropylique, on obtient 1,1 g de 4-(5,6-difluoro-1H-benzimidazole-2-yl)-9H-fluorène-9-one, sous forme d'une poudre jaune utilisée telle quelle à l'étape suivante, dont les caractéristiques sont les suivantes :
Spectre de masse (LC/MS/ES/+/-) : m/z = 332 (M+)

Etape 3 : On opère comme à l'exemple 5, mais à partir de 1,1 g de 4-(5,6-difluoro-1H-benzimidazole-2-yl)-9H-fluorène-9-one, obtenue à l'étape précédente, de 690 mg de chlorhydrate d'hydroxylamine et de 1,4 g d'acétate de sodium dans 60 mL d'éthanol agités pendant 20h à température ambiante. Après concentration du solvant sous pression réduite, le résidu est repris successivement par de l'eau, puis du toluène, amené à sec. Après empâtage à l'éther diisopropylique, on obtient ainsi 830 mg de 4-(5,6-difluoro-1H-benzimidazole-2-yl)-9H-fluorène-9-one oxime (Z,E) en mélange 50-50 des isomères Z et E, sous forme d'une poudre beige utilisée telle quelle à l'étape suivante, dont les caractéristiques sont les suivantes :
Spectre de masse (LCIMS/ESI+/-) : m/z = 347 (M+).

Etape 4 : On opère comme dans l'exemple 216. Dans un ballon de 100 mL sous atmosphère d'argon, on dissout 830 mg de 4-(5,6-difluoro-1H-benzimidazol-2-yl)-9H-fluorène-9-one oxime (Z,E), obtenue à l'étape précédente, dans un mélange de 5,5 mL d'éthanol et 5,5 mL de d'eau et 5,5 mL d'acide acétique, à température ambiante on ajoute en trois fois 624 mg de zinc, entre chaque ajout, on agite environ une heure à deux heures. On ajoute de la Célite et on filtre. Le filtrat est concentré sous pression. Le produit brut est purifié par flash-chromatographie sur gel de silice (40-63µm) en éluant avec un mélange de dichlorométhane et d'ammoniac en solution 7N dans le méthanol (98/2 en volumes). Après empâtage à l'éther diisopropylique, on obtient ainsi 653 mg de 4-(5,6-difluoro-1H-benzimidazole-2-yl)-9H-fluorène-9(R,S)-amine sous forme d'une poudre blanche, utilisée telle quelle à l'étape suivante, dont les caractéristiques sont les suivantes :
Spectre de masse (LC/MS/ES/+/-) : m/z = 333 (M+)

Etape 5 : On opère comme dans l'exemple 14, mais à partir de 330 mg de 4-(5,6-difluoro-1H-benzimidazol-2-yl)-9H-fluorène-9(R,S)-amine, obtenue à l'étape précédente, 178 mg d'acide 1H-pyrrolo-[2,3-b]pyridine-4-carboxylique, 211 mg de chlorhydrate de 1-(3-diméthylaminopropyl)-3-éthylcarbodiimide (EDCI) et 67 mg de 1-hydroxybenzotriazole (HOBT) dans 3,5 mL de diméthylformamide pendant 20 heures à température ambiante. On amène à sec le diméthylformamide, on ajoute de l'eau et on essore le précipité formé qui est ensuite lavé à l'eau puis avec une solution saturée d'hydrogénocarbonate de sodium à 10% et enfin à l'eau. Le solide brut obtenu est séché puis purifié par flash-chromatographie sur gel de silice (40-63µm) en éluant avec un mélange de dichlorométhane et de méthanol (98/2 puis 95/5 en volumes). Après empâtage à l'éther diisopropylique, on obtient ainsi 259 mg de 4-(5,6-difluoro-1H-benzimidazole-2-yl)-9H-fluorène-9(R,S)-yl]-amide de l'acide 1H-pyrrolo[2,3-b]pyridine-4-carboxylique, sous forme d'une poudre blanche dont les caractéristiques sont les suivantes :
Spectre de masse (LC/MS/ES+/-) : m/z = 477 (M+)
Spectre RMN 1H (400 MHz, □ en ppm, DMSO-d6) : 6,40 (d, J = 8,5 Hz, 1H) ; 6,91 (d large, J = 3,5 Hz, 1H) ; 7,25 (t large, J = 7,5 Hz, 1H) ; 7,35 (t large, J = 7,5 Hz, 1H) ; 7,46 (d, J = 5,0 Hz, 1H) ; 7,51 (t, J = 7,5 Hz, 1H) ; de 7,54 à 7,79 (m, 7H) ; 8,29 (d, J = 5,0 Hz, 1H) ; 9,27 (d, J = 8,5 Hz, 1H) ; 11,85 (m large, 1H) ; 13,2 (m étalé, 1H).

### Exemple 225: Synthèse du [4-(5-méthyl-5H-imidazo[4,5-c]pyridin-2-yl)-9H-fluorèn-9-yl]-amide de l'acide 1H-pyrrolo[2,3-b]pyridine-4-carboxylique.

Dans un ballon monocol de 5 mL, on dissout 442 mg de [4-(3H-imidazo[4,5-c]pyridin-2-yl)-9H-fluorèn-9-yl]-amide de l'acide 1H-pyrrolo[2,3-b]pyridine-4-carboxylique, obtenu à l'exemple 92, dans 4 mL de diméthylformamide. La solution jaune obtenue est additionnée goutte à goutte d'une solution de 62 µL d'iodure de méthyle dans 1 mL de diméthylformamide. Après une nuit d'agitation à température ambiante, l'analyse par CCM et LC/MS du milieu réactionnel révèle la consommation totale du produit de départ. L'huile brune, obtenue après évaporation du solvant sous pression réduite, est purifiée par chromatographie flash sur colonne de silice (gradient CH2CI2/MeOH : de 100/0 à 80/20). On obtient ainsi 343 mg de [4-(5-méthyl-5H-imidazo[4,5-c]pyridin-2-yl)-9H-fluorèn-9-yl]-amide de l'acide 1H-pyrrolo[2,3-b]pyridine-4-carboxylique, sous la forme d'une poudre blanc-cassé dont les caractéristiques sont les suivantes :
Spectre de masse (El) : m/z = 456 (M+.)
Spectre de RMN (400 MHz, □en ppm, DMSO-d6) : 4,29 (s, 3H) ; 6,39 (d, J = 8,5 Hz, 1H) ; 6,91 (dd, J = 2,0 et 3,5 Hz, 1H) ; 7,21 (t large, J = 7,5 Hz, 1H) ; 7,31 (t large, J = 7,5 Hz, 1H) ; 7,44 (t partiellement masqué, J = 7,5 Hz, 1H) ; 7,47 (d, J = 5,0 Hz, 1H) ; 7,58 (d large, J = 8,0 Hz, 1H) ; 7,62 (t large, J = 3,5 Hz, 1H); 7,67 (d large, J = 8,0 Hz, 1H) ; 7,85 (d, J = 7,0 Hz, 1H); 7,91 (d large, J = 8,0 Hz, 1H) ; 8,15 (d large, J = 7,0 Hz, 1H) ; 8,25 (d large, J = 8,0 Hz, 1H) ; 8,29 (d, J = 5,0 Hz, 1H) ; 9,09 (s large, 1H) ; 9,25 (d, J = 8,5 Hz, 1H) ; 11,85 (m large, 1H).

### Exemple 226 : Synthèse du [2-amino-5-(1H-benzimidazol-2-yl)-9H-fluorène-9(R,S)-yl]-amide de l'acide 1H-pyrrolo[2,3-b]pyridine-4-carboxylique

Etape 1 : Dans un tricol de 50 mL, on met en suspension 1,615 g d'acide 7-nitro-9-oxo-9H-fluorène-4-carboxylique, qui peut être obtenu selon WO2003057145, dans 30 mL dichlorométhane, puis on ajoute successivement 567 µL de chlorure d'oxalyle et 1 goutte de diméthylformamide. Après 4 heures d'agitation à température ambiante, on ajoute 0,779 g de 1,2-diamino-benzène et 1,686 mL de triéthylamine. Après 20 heures d'agitation à température ambiante, le précipité formé est filtré, lavé, à l'eau, puis avec une solution aqueuse saturée d'hydrogénocarbonate de sodium à 10% et enfin à l'eau. Après empâtage à l'éther diisopropylique, on obtient ainsi 1,6 g du (2-amino-phényl)amide de l'acide 7-nitro-9-oxo-9H-fluorène-4-carboxylique, sous forme d'une poudre orange utilisée telle quelle à l'étape suivante, dont les caractéristiques sont les suivantes :
Spectre de masse (LC/MS/ES/+/-) : m/z = 359 (M+)

Etape 2 : On opère comme dans l'exemple 68. Dans un ballon de 100 mL sous atmosphère d'argon, on chauffe à 130°C pendant 4 heures, une suspension de 1,6 g de (2-amino-phényl)amide de l'acide 7-nitro-9-oxo-9H-fluorène-4-carboxylique, obtenu à l'étape précédente, dans 30 mL d'acide acétique. Après refroidissement, l'insoluble formé est filtré, lavé successivement à l'eau, avec une solution aqueuse saturée d'hydrogénocarbonate de sodium puis à l'eau. Après purification par flash-chromatographie sur gel de silice (40-63µm), en éluant avec un mélange de dichlorométhane et de méthanol ammoniacal 7N (9/1 en volumes), puis empâtage dans l'éther de diisopropylique, on obtient ainsi 550 mg de 5-(1H-benzimidazol-2-yl)-2-nitro-9H-fluorène-9-one, sous forme d'une poudre jaune utilisée telle quelle à l'étape suivante, dont les caractéristiques sont les suivantes :
Spectre de masse (EI) : m/z = 341 (M+)
Spectre de RMN (400 MHz, □ en ppm, DMSO-d6 + TFAd) 7,44 (d, J = 8,0 Hz, 1H) ; 7,67 (m, 2H) ; 7,84 (t, J = 7,5 Hz, 1H) ; 7,96 (m, 2H) ; 8,10 (d large, J = 7,5 Hz, 2H) ; de 7,33 à 7,39 (m, 2H).

Etape 3: Dans un ballon de 100 ml sous argon, 550 mg de 5-(1H-benzimidazol-2-yl)-2-nitro-9H-fluorène-9-one, obtenue à l'étape précédente, sont agités pendant 2 heures à reflux, en présence de 2,513 g de fer et de 313 µl d'une solution aqueuse à 37% d'acide chlorhydrique, dans 3,2ml d'eau et 25 mL d'éthanol. Après refroidisement, on filtre l'insoluble sur célite, puis on lave à l'eau et avec une solution aqueuse saturée d'hydrogénocarbonate de sodium. Cet insoluble est ensuite repris dans du diméthylformamide. Après concentration du diméthylformamide sous pression réduite, le produit brut obtenu est purifié par flash-chromatographie sur gel de silice (40-63µm), en éluant avec un mélange de dichlorométhane et de méthanol ammoniacal 7N (95/5 en volumes). On obtient ainsi 0,45 g 2-amino 5-(1H-benzimidazol-2-yl)-9H-fluorène-9-one, sous forme d'une poudre brune, utilisée telle quelle à l'étape suivante.
Spectre de masse (EI) : m/z = 311 (M+)

Etape 4 : Dans un ballon de 100 ml sous argon, 0,3 g de 2-amino 5-(1H-benzimidazol-2-yl)-9H-fluorène-9-one, obtenue à l'étape précédente, sont agités dans 10 mL d'acétonitrile, pendant 20 heures à température ambiante, en présence de 0,631 g de dicarbonate de tert-butyle et de 11,8 mg de 4-diméthylamino-pyridine. Après concentration du solvant sous pression réduite, le résidu est dissout dans 50 mL d'acétate d'éthyle, puis la phase organique est lavée à l'eau séchée sur sulfate de magnésium et concentrée à sec sous pression réduite. Le solide brut obtenu est purifié par flash-chromatographie sur gel de silice (40-63µm), en éluant avec un mélange d'acétate d'éthyle et de dichlorométhane (5/95 en volumes). On obtient ainsi 0,3 g de 5-[1-(tert-butoxycarbonyl)-benzimidazo!-2-yl-]-7-(tert-butoxycarbonylamino)-9H-fluorène-9-one, sous forme d'une meringue jaune utilisée telle quelle à l'étape suivante.
Spectre de masse (EI) : m/z = 511 (M+)

Etape 5 : On opère comme à l'exemple 5, mais à partir de 300 mg de 5-[1-(tert-butoxycarbonyl)-benzimidazol-2-yl-]-7-(tert-butoxycarbonylamino)-9H-fluorène-9-one, obtenue à l'étape précédente, de 122 mg de chlorhydrate d'hydroxylamine et de 240 mg d'acétate de sodium dans 4 mL d'éthanol agités pendant 20h à température ambiante. Après empâtage à l'éther diisopropylique, on obtient ainsi 250 mg de 5-[1-(tert-butoxycarbonyl)-benzimidazol-2-yl-]-7-(tert-butoxycarbonylamino)-9H-fluorène-9-one oxime (Z,E), en mélange 50/50 des isomères Z et E, sous forme d'une poudre orange utilisée telle quelle à l'étape suivante, dont les caractéristiques sont les suivantes :
Spectre de masse (EI) : m/z = 526 (M+)

Etape 6 : On opère comme à l'exemple 6, mais à partir de 250 mg de 5-[1-(tert-butoxycarbonyl)-benzimidazol-2-yl-]-7-(tert-butoxycarbonylamino)-9H-fluorène-9-one oxime (Z,E), obtenue à l'étape précédente, et de 29 mg de Nickel de Raney dans 4 mL d'éthanol et 4 ml de tétrahydrofurane pendant 10 heures à 60°C sous une pression initiale d'un bar d'hydrogène. Après filtration du catalyseur, et purification par empâtage dans l'oxyde de diisopropyle, on obtient ainsi 250 mg de 5-[1-(tert-butoxycarbonyl)-benzimidazol-2-yl-]-7-(tert-butoxycarbonylamino)-9H-fluorèn-9(R,S)-yl-amine, sous forme d'une poudre orange utilisée telle quelle à l'étape suivante. Etape 7 : On opère comme dans l'exemple 14, mais à partir 250 mg de 5-[1-(tert-butoxycarbonyl)-benzimidazol-2-yl-]-7-(tert-butoxycarbonylamino)-9H-fluorèn-9(R,S)-yl-amine, obtenue à l'étape précédente, 79 mg d'acide 1H-pyrrolo-[2,3-b]pyridine-4-carboxylique, 103 mg de chlorhydrate de 1-(3d-iméthylaminopropyl)-3-éthylcarbodiimide (EDCI) et 72 mg de 1-hydroxybenzotriazole (HOBT) dans 3,5 mL de diméthylformamide, pendant 72 heures à température ambiante. Après purification par empâtage à l'éther diisopropylique, on obtient ainsi 300 mg de {5-[1-(tert-butoxycarbonyl)-benzimidazol-2-yl-]-7-(tert-butoxycarbonylamino)-9H-fluorèn-9(R,S)-yl}-amide de l'acide 1H-pyrrolo[2,3-b]pyridine-4-carboxylique, sous forme d'une poudre orange dont les caractéristiques sont les suivantes :
Spectre de masse (LC/MS/ES+/-) : m/z = 656 (M+)

Etape 8 : Dans un ballon de 100 ml sous argon, on dissout 300 mg de {5-[1-(tert-butoxycarbonyl)-benzimidazol-2-yl-]-7-(tert-butoxycarbonylamino)-9H-fluorèn-9(R,S)-yl}-amide de l'acide 1H-pyrrolo[2,3-b]pyridine-4-carboxylique, obtenu à l'étape précédente, dans 1,5 mL de dioxane, puis on coule, goutte à goutte, 1,45 mL d'une solution 4N d'acide chlorhydrique dans le dioxane et on agite pendant 20 heure à température ambiante. Après concentration du solvant sous pression réduite, le milieu réactionnel est repris dans l'eau, amené à pH = 8 par addition d'une solution aqueuse saturée d'hydrogénocarbonate de sodium. Le précipité formé est essoré, puis purifié par flash-chromatographie sur gel de silice (40-63µm), en éluant avec un mélange de dichlorométhane et de méthanol (95/5 en volumes). On obtient ainsi 15mg [2-amino-5-(1H-benzimidazole-2-yl)-9H-fluorèn-9(R,S)-yl]-amide de l'acide 1H-pyrrolo[2,3-b]pyridine-4-carboxylique, sous forme d'une poudre jaune, don't les caractéristiques sont les suivantes :
Spectre de masse (LC/MS/ES+/-) : m/z = 456 (M+)
Spectre RMN 1H (400 MHz, □ en ppm, DMSO-d6) : 5,39 (s large, 2H) ; 6,25 (d, J = 8,5 Hz, 1H) ; 6,39 (dd, J = 2,0 et 8,5 Hz, 1H) ; 6,81 (s large, 1H) ; 6,93 (dd, J = 2,0 et 3,5 Hz, 1H) ; de 7,20 à 7,35 (m, 4H) ; 7,48 (d, J = 5,0 Hz, 1H) ; de 7,51 à 7,64 (m, 4H) ; 7,75 (m large, 1H) ; 8,29 (d, J = 5,0 Hz, 1H) ; 9,20 (d, J = 8,5 Hz, 1H) ; 11,85 (m large, 1H) ; 12,8 (m large, 1H).

### Exemple 227 : Synthèse du [4-(5-méthyl-4,5,6,7-tetrahydro-3H-imidazo[4,5-c]pyridin-2-yl)-9H-fluorèn-9-yl]-amide de l'acide 1H-pyrrolo[2,3-b]pyridine-4-carboxylique.

On ajoute à température ambiante et sous argon 174 mg de borohydrure de sodium à la suspension de 70 mg de [4-(5-méthyl-5H-imidazo[4,5-c]pyridin-2-yl)-9H-fluorèn-9-yl]-amide de l'acide 1H-pyrrolo[2,3-b]pyridine-4-carboxylique, obtenu comme à l'exemple 225, dans 7 mL de méthanol anhydre. La disparition du produit de départ est constatée par CCM après 1 h d'agitation à température ambiante. L'addition de 2 mL d'une solution de chlorure d'ammonium saturée permet de neutraliser l'excès d'hydrure. Après évaporation du solvant sous pression réduite, le résidu solide est purifié par chromatographie flash sur colonne de silice (gradient CH2Cl2/MeOH : de 100/0 à 70/30). On obtient 70 mg [4-(5-méthyl-4,5,6,7-tetrahydro-3H-imidazo[4,5-c]pyridin-2-yl)-9H-fluorèn-9-yl]-amide de l'acide 1H-pyrrolo[2,3-b]pyridine-4-carboxylique, sous la forme d'une poudre blanche dont les caractéristiques sont les suivantes :
Spectre de masse (ESI+) : m/z = 461 (MH+)
Spectre RMN 1H (400 MHz, □en ppm, DMSO-d6) : 2,90 (s, 3H) ; 3,04 (m large, 2H) ; 3,50 (m large, 2H) ; 4,24 (m large, 2H) ; 6,35 (d, J = 8,5 Hz, 1H) ; 6,89 (m large, 1H) ; 7,28 (t, J = 7,5 Hz, 1H) ; 7,34 (t, J = 7,5 Hz, 1H) ; 7,43 (t, J = 7,5 Hz, 1H) ; 7,46 (d, J = 5,0 Hz, 1H) ; 7,53 (d, J = 7,5 Hz, 1H) ; 7,58 (d, J = 7,5 Hz, 1H) ; 7,61 (t, J = 3,5 Hz, 1H) ; 7,64 (d, J = 7,5 Hz, 1H) ; 7,92 (d large, J = 7,5 Hz, 1H) ; 8,28 (d, J = 5,0 Hz, 1H) ; 9,28 (d, J = 8,5 Hz, 1H) ; 11,95 (m large, 1H) ; 12,8 (m étalé, 1H).

### Exemple 228 : Synthèse du 4-(5-méthoxy-1H-benzimidazole-2-yl)-9H-fluorène-9(R,S)-yl]-amide de l'acide 1H-pyrrolo[2,3-b]pyridine-4-carboxylique

Etape 1 : On opère comme à l'étape 1 de l'exemple 1, mais à partir de 10 g de 1,2-diamino-4-methoxybenzène, 20 mL de triéthylamine et 17 g de chlorure de l'acide fluorène-4-one-9-carboxylique dans 600 mL de dichlorométhane. Après 20 heures d'agitation à température ambiante, le précipité formé est filtré, lavé au dichlorométhane, à l'eau, puis avec une solution aqueuse saturée d'hydrogénocarbonate de sodium à 10% et enfin à l'eau. Après empâtage à l'éther diisopropylique, on obtient ainsi 6,2 g du (2-amino-4-méthoxy-phényl)amide de l'acide 9-oxo-9H-fluorène-4-carboxylique sous forme d'une poudre marron utilisée telle quelle à l'étape suivante, dont les caractéristiques sont les suivantes :
Spectre de masse (El) : m/z = 344 (M+)

Etape 2 : On opère comme dans l'exemple 68. Dans un ballon de 1 L sous atmosphère d'argon, on chauffe à 110°C pendant 5 heures, une solution de 6,2 g de (2-amino-4-méthoxy-phényl) amide de l'acide 9-oxo-9H-fluorène-4-carboxylique, obtenu à l'étape précédente, dans 300 mL d'acide acétique. Après refroidissement, on amène à sec sous pression réduite. Le solide brut obtenu est purifié par flash-chromatographie sur gel de silice (40-63µm) en éluant avec un mélange de dichlorométhane et de méthanol (99/1 puis 98/2 en volumes). Après empâtage à l'éther diisopropylique, on obtient 5,1 g de 4-(5-méthoxy-1H-benzimidazole-2-yl)-9H-fluorène-9-one, sous forme d'une poudre jaune utilisée telle quelle à l'étape suivante, dont les caractéristiques sont les suivantes :
Spectre de masse (EI) : m/z = 326 (M+)
Spectre RMN 1H (400 MHz, □ en ppm, DMSO-d6) : 3,84 (s, 3H) ; 6,92 (dd, J = 2,0 et 8,5 Hz, 1H) ; 7,15 (m large, 1H) ; 7,39 (t, J = 7,5 Hz, 1H) ; 7,49 (t, J = 7,5 Hz, 1H) ; 7,56 (t, J = 7,5 Hz, 1H) ; 7,59 (m large, 1H) ; 7,67 (d, J = 8,0 Hz, 1H); de 7,74 à 7,79 (m, 2H) ; 7,83 (d, J = 7,5 Hz, 1H) ; 12,9 (m étalé, 1H).

Etape 3 : On opère comme à l'exemple 5, mais à partir de 1 g de 4-(5-méthoxy-1H-benzimidazole-2-yl)-9H-fluorène-9-one, obtenue à l'étape précédente, de 640 mg de chlorhydrate d'hydroxylamine et de 1,3 g d'acétate de sodium dans 17 mL d'éthanol agités pendant 20h à température ambiante. Après concentration du solvant sous pression réduite, le résidu est repris successivement par de l'eau, puis du toluène, amené à sec. Après empâtage à l'éther diisopropylique, on obtient ainsi 890 mg de 4-(5-méthoxy-1H-benzimidazole-2-yl)-9H-fluorène-9-one oxime (Z,E) en mélange 50-50 des isomères Z et E, sous forme d'une poudre beige utilisée telle quelle à l'étape suivante, dont les caractéristiques sont les suivantes :
Spectre de masse (El) : m/z = 341 (M+).

Etape 4 : On opère comme dans l'exemple 216. Dans un ballon de 100 mL sous atmosphère d'argon, on dissout 890 mg de 4-(5-méthoxy-1H-benzimidazol-2-yl)-9H-fluorène-9-one oxime (Z,E), obtenue à l'étape précédente, dans un mélange de 5,9 mL d'éthanol et 5,9 mL de d'eau et 5,9 mL d'acide acétique, à température ambiante on ajoute en trois fois 682 mg de zinc, entre chaque ajout, on agite environ une heure à deux heures. On ajoute de la Célite et on filtre. Le filtrat est concentré sous pression. Le produit brut est purifié par flash-chromatographie sur gel de silice (40-63µm) en éluant avec un mélange de dichlorométhane et d'ammoniac en solution 7N dans le méthanol (95/5 en volumes). Après empâtage à l'éther diisopropylique, on obtient ainsi 716 mg de 4-(5-méthoxy-1H-benzimidazole-2-yl)-9H-fluorène-9(R,S)-amine sous forme d'une poudre beige, utilisée telle quelle à l'étape suivante, dont les caractéristiques sont les suivantes :
Spectre de masse (LC/MS/ES/+/-) : m/z = 327 (M+)

Etape 5 : On opère comme dans l'exemple 14, mais à partir de 330 mg de 4-(5-méthoxy-1H-benzimidazol-2-yl)-9H-fluorène-9(R,S)-amine, obtenue à l'étape précédente, 180 mg d'acide 1H-pyrrolo-[2,3-b]pyridine-4-carboxylique, 213 mg de chlorhydrate de 1-(3-diméthylaminopropyl)-3-éthylcarbodiimide (EDCI) et 68 mg de 1-hydroxybenzotriazole (HOBT) dans 3,5 mL de diméthylformamide, pendant 20 heures à température ambiante. On amène à sec le diméthylformamide, on ajoute de l'eau et on essore le précipité formé qui est ensuite lavé à l'eau puis avec une solution saturée d'hydrogénocarbonate de sodium à 10% et enfin à l'eau. Le solide brut obtenu est séché puis purifié par flash-chromatographie sur gel de silice (40-63µm) en éluant avec un mélange de dichlorométhane et de méthanol (95/5 en volumes). Après empâtage à l'éther diisopropylique, on obtient ainsi 278 mg de 4-(5-méthoxy-1H-benzimidazole-2-yl)-9H-fluorène-9(R,S)-yl]-amide de l'acide 1H-pyrrolo[2,3-b]pyridine-4-carboxylique, sous forme d'une poudre blanc cassé dont les caractéristiques sont les suivantes :
Spectre de masse (LC/MS/ES+/-) : m/z = 471 (M+)
Spectre RMN 1H (400 MHz, □ en ppm, DMSO-d6) : pour ce composé, on observe un mélange 50% -50% de tautomères avec : 3,84 (s, 3H) ; 6,40 (d, J = 8,5 Hz, 1H) ; de 6,85 à 6,95 (m, 2H) ; 7,05 (d, J = 2,5 Hz, 0,5H) ; 7,25 (t, J = 7,5 Hz, 1H) ; 7,30 (d, J = 2,5 Hz, 0,5H) ; 7,34 (t, J = 7,5 Hz, 1H) ; de 7,44 à 7,53 (m, 2,5H) ; de 7,59 à 7,67 (m, 4,5H) ; 7,74 (d large, J = 8,0 Hz, 1H) ; 8,29 (d, J = 5,0 Hz, 1H) ; 9,28 (d, J = 8,5 Hz, 1H) ; 11,85 (m large, 1H) ; 12,75 (s, 0,5H) ; 12,8 (s, 0,5H).

### Exemple 229 : Synthèse du [4-(5-hydroxy-5H-imidazo[4,5-c]pyridin-2-yl)-9H-fluorèn-9-yl]-amide de l'acide 1H-pyrrolo[2,3-b]pyridine-4-carboxylique

Dans un ballon monocol de 5 mL, on dissout 2 mg de triméthyloxorhénium dans 1 mL de diméthylformamide. On ajoute 147 µL de péroxyde d'hydrogène 30 % et on agite 15 minutes à température ambiante. La solution jaune obtenue est additionnée goutte à goutte d'une solution de 160 mg de [4-(3H-imidazo[4,5-c]pyridin-2-yl)-9H-fluorèn-9-yl]-amide de l'acide 1H-pyrrolo[2,3-b]pyridine-4-carboxylique, obtenu à l'exemple 92, dans 1 mL de diméthylformamide. Après une heure d'agitation à température ambiante, le taux de conversion atteint 50 % et n'évolue plus. L'évaporation du solvant sous pression réduite conduit à une huile orange qui est purifiée par chromatographie flash sur colonne de silice (gradient CH2Cl2/MeOH : de 100/0 à 70/30). On obtient 83 mg de [4-(5-hydroxy-5H-imidazo[4,5-c]pyridin-2-yl)-9H-fluorèn-9-yl]-amide de l'acide 1H-pyrrolo[2,3-b]pyridine-4-carboxylique, sous la forme d'une poudre jaune pâle dont les caractéristiques sont les suivantes :
Spectre de masse (ESI+) : m/z = 459 (MH+)
Spectre de RMN (400 MHz, □ en ppm, DMSO-d6) : 6,41 (d, J = 8,5 Hz, 1H) ; 6,91 (m large, 1H) ; 7,28 (t, J = 7,5 Hz, 1H) ; 7,36 (t, J = 7,5 Hz, 1H) ; 7,48 (t, J = 5,0 Hz, 1H) ; 7,53 (t, J = 7,5 Hz, 1H) ; 7,56 (d, J = 8,0 Hz, 1H) ; 7,63 (m, 2H) ; 7,68 (m, 2H) ; 7,79 (d, J = 7,5 Hz, 1H) ; 8,10 (dd, J = 2,0 et 7,0 Hz, 1H) ; 8,29 (d, J = 5,0 Hz, 1H) ; 8,70 (m large, 1H) ; 9,33 (d, J = 8,5 Hz, 1H) ; 11,9 (m large, 1H) ; 13,5 (m très étalé, 1H).

Exemple 230 : Synthèse du 4-(5-hydroxy-1H-benzimidazole-2-yl)-9H-fluorène-9(R,S)-yl]-amide de l'acide 1H-pyrrolo[2,3-b]pyridine-4-carboxylique Etape 1 : Dans un ballon de 50 mL sous argon, on dissout 1 g de 4-(5-méthoxy-1H-benzimidazole-2-yl)-9H-fluorène-9-one, obtenue à l'étape 2 de l'exemple 228, dans 15 mL de dichlorométhane, on refroidit à 0°C et l'on ajoute de 7,7 mL de tribromure de bore en solution 1 M dans le dichlorométhane. Après 20 heures d'agitation, le milieu réactionnel est versé sur de l'eau, l'insoluble est essoré, lavé à l'eau puis séché. Le produit brut est purifié par flash-chromatographie sur gel de silice (40-63µm) en éluant avec un mélange de dichlorométhane et de méthanol (98/2 puis 95/5 en volumes). Après empâtage avec l'éther diisopropylique, on obtient ainsi 360 mg de 4-(5-hydroxy-1H-benzimidazole-2-yl)-9H-fluorène-9-one sous forme d'une poudre jaune, utilisée telle quelle à l'étape suivante, dont les caractéristiques sont les suivantes
Spectre de masse (LC/MS/ES/+/-) : m/z = 312 (M+).
Spectre RMN 1H (400 MHz, □en ppm, DMSO-d6) : pour ce composé, on observe un mélange de tautomères 70 % - 30 % avec : 6,77 (d large, J = 8,5 Hz, 1H) ; 6,90 (s large, 0,7H) ; 7,07 (s large, 0,3H) ; 7,39 (t, J = 7,5 Hz, 1H) ; de 7,45 à 7,61 (m masqué, 1H) ; 7,50 (t, J = 7,5 Hz, 1H) ; 7,55 (t, J = 7,5 Hz, 1H) ; 7,67 (d, J = 8,0 Hz, 1H) ; 7,75 (m masqué, 0,3H) ; 7,77 (d, J = 8,0 Hz, 1H) ; 7,82 (d, J = 8,0 Hz, 1H) ; 7,85 (m large partiellement masqué, 0,7H) ; 9,10 (s large, 0,3H) ; 9,32 (s large, 0,7H) ; 12,65 (s large, 0,7H) ; 12,75 (s large, 0,3H).

Etape 2 : On opère comme à l'exemple 5, mais à partir de 360 mg 4-(5-hydroxy-1H-benzimidazole-2-yl)-9H-fluorène-9-one, obtenue à l'étape précédente, de 240 mg de chlorhydrate d'hydroxylamine et de 470 mg d'acétate de sodium dans 18 mL d'éthanol agités pendant 20 heures à température ambiante. Après concentration du solvant sous pression réduite, le résidu est repris successivement par de l'eau, puis du toluène, amené à sec. Après empâtage à l'éther diisopropylique, on obtient ainsi 358 mg de 4-(5-hydroxy-1H-benzimidazole-2-yl)-9H-fluorène-9-one oxime (Z,E) en mélange 50-50 des isomères Z et E, sous forme d'une poudre marron utilisée telle quelle à l'étape suivante, dont les caractéristiques sont les suivantes :
Spectre de masse (LC/MS/ES/+/-) : m/z = 327 (M+).

Etape 3 : On opère comme dans l'exemple 216. Dans un ballon de 50 mL sous atmosphère d'argon, on dissout 355 mg de mélange équimoléculaire de 4-(5-hydroxy-1H-benzimidazol-2-yl)-9H-fluorène-9-one oxime (Z,E), obtenue à l'étape précédente, dans un mélange de 2,5 mL d'éthanol et 2,5 mL de d'eau et 2,5 mL d'acide acétique, à température ambiante on ajoute en trois fois 283 mg de zinc, entre chaque ajout, on agite environ une heure à deux heures. On ajoute de la Célite et on filtre. Le filtrat est concentré sous pression. Le produit brut est purifié par flash-chromatographie sur gel de silice (40-63µm) en éluant avec un mélange de dichlorométhane et d'ammoniac en solution 7N dans le méthanol (9/1 en volumes). Après empâtage à l'éther diisopropylique, on obtient ainsi 232 mg de 4-(5-hydroxy-1H-benzimidazole-2-yl)-9H-fluorène-9(R,S)-amine sous forme d'une poudre blanc cassé, utilisée telle quelle à l'étape suivante, dont les caractéristiques sont les suivantes :
Spectre de masse (LC/MS/ES/+/-) : m/z = 313 (M+)

Etape 4 : On opère comme dans l'exemple 14, mais à partir de 230 mg de 4-(5-hydroxy-1H-benzimidazol-2-yl)-9H-fluorène-9(R,S)-amine, obtenue à l'étape précédente, 131 mg d'acide 1H-pyrrolo-[2,3-b]pyridine-4-carboxylique, 155 mg de chlorhydrate de 1-(3-diméthylaminopropyl)-3-éthylcarbodiimide (EDCI) et 49 mg de 1-hydroxybenzotriazole (HOBT) dans 5 mL de diméthylformamide, pendant 20 heures à température ambiante. On amène à sec le diméthylformamide, on ajoute de l'eau et on essore le précipité formé qui est ensuite lavé à l'eau. Le solide brut obtenu est séché puis purifié par flash-chromatographie sur gel de silice (40-63µm) en éluant avec un mélange de dichlorométhane et de méthanol (95/5 puis 9/1 en volumes). Après empâtage à l'éther diisopropylique, on obtient ainsi 220 mg de 4-(5-hydroxy-1H-benzimidazole-2-yl)-9H-fluorène-9(R,S)-yl]-amide de l'acide 1H-pyrrolo[2,3-b]pyridine-4-carboxylique, sous forme d'une poudre blanc cassé dont les caractéristiques sont les suivantes :
Spectre de masse (LC/MS/ES+/-) : m/z = 457 (M+)
Spectre RMN 1H (400 MHz, □ en ppm, DMSO-d6) : pour ce composé, on observe un mélange de tautomères 70 % - 30 % avec : 6,39 (d, J = 8,5 Hz, 1H) ; 6,75 (d large, J = 9,0 Hz, 0,7H) ; 6,80 (d large, J = 9,0 Hz, 0,3H) ; 6,90 (m large partiellement masqué, 0,7H) ; 6,91 (dd, J = 2,0 et 3,5 Hz, 1H) ;7,07 (m large, 0,3H) ; 7,25 (t, J = 7,5 Hz, 1H) ; 7,33 (t, J = 7,5 Hz, 1H) ; 7,35 (m large partiellement masqué, 0,7H) ; 7,44 (m large partiellement masqué, 0,3H) ; 7,46 (d, J = 5,0 Hz, 1H) ; 7,49 (t, J = 7,5 Hz, 1H) ; 7,54 (d, J = 8,0 Hz, 0,6H) ; de 7,58 à 7,66 (m, 3H) ; 7,72 (d large, J = 8,0 Hz, 1,4H) ; 8,29 (d, J = 5,0 Hz, 1H); 9,05 (m large, 0,3H); 9,26 (m large partiellement masqué, 0,7H) ; 9,27 (d, J = 8,5 Hz, 1H) ; 11,85 (m large, 1H); 12,5 (s large, 0,7H) ; 12,6 (s large, 0,3H).

### Exemple 231 : Synthèse du [4-(5-méthylaminocarbonyl-1H-benzimidazol-2-yl)-9H-fluorène-9(R,S)-yl]-amide de l'acide 1H-pyrrolo[2,3-b]pyridine-4-carboxylique

On opère comme dans l'exemple 208 mais à partir de 200 mg de [4-(5-carboxy-1H-benzimidazol-2-yl)-9H-fluorène-9(R,S)-yl]-amide de l'acide 1H-pyrrolo[2,3-b]pyridine-4-carboxylique, obtenue dans l'exemple 206, 55 mg de chlorhydrate de méthylamine, 321 mg de hexafluorophosphate de benzotriazole-1-yl-oxy-trispyrrolidinophosphonium (PYBOP), 83 mg de 1-hydroxybenzotriazole (HOBT) et 272 µL de N,N diisopropyléthylamine (DIPEA) dans 2 mL de diméthylformamide, pendant 20 heures à température ambiante. On amène à sec le diméthylformamide, on ajoute de l'eau, on essore le précipité qui est ensuite lavé à l'eau puis avec une solution saturée d'hydrogénocarbonate de sodium à 10% et enfin à l'eau. Le solide brut obtenu est séché puis purifié par flash-chromatographie sur gel de silice (40-63µm) en éluant avec un mélange de dichlorométhane et de méthanol (95/5 puis 9/1 en volumes). Après empâtage à l'éther diisopropylique, on obtient ainsi 129 mg de [4-(5-méthylaminocarbonyl-1H-benzimidazol-2-yl)-9H-fluorène-9(R,S)-yl]-amide de l'acide 1H-pyrrolo[2,3-b]pyridine-4-carboxylique, sous forme d'une poudre blanc cassé dont les caractéristiques sont les suivantes :
Spectre de masse (LC/MS/ES/+/-) : m/z = 498 (M+)
Spectre RMN 1H (300 MHz, □ en ppm, DMSO-d6) : 2,84 (d, J = 5,0 Hz, 3H) ; 6,41 (d, J = 8,5 Hz, 1H) ; 6,91 (dd, J = 2,0 et 3,5 Hz, 1H) ; 7,25 (t, J = 7,5 Hz, 1H) ; 7,35 (t, J = 7,5 Hz, 1H) ; 7,47 (d, J = 5,0 Hz, 1H) ; 7,53 (t, J = 7,5 Hz, 1H) ; de 7,57 à 7,77 (m, 5H) ; 7,77 (d, J = 7,5 Hz, 1H) ; 7,82 (d large, J = 8,0 Hz, 1H) ; 8,21 (m étalé, 1H) ; 8,30 (d, J = 5,0 Hz, 1H) ; 8,46 (q large, J = 5,0 Hz, 1H) ; 9,28 (d, J = 8,5 Hz, 1H) ; 11,85 (m large, 1H) ; 13,2 (m étalé, 1H).

### Exemple 232 : Synthèse du [4-(5-diméthylaminocarbonyl-1H-benzimidazol-2-yl)-9H-fluorène-9(R,S)-yl]-amide de l'acide 1H-pyrrolo[2,3-b]pyridine-4-carboxylique

On opère comme dans l'exemple 208, mais à partir de 200 mg de [4-(5-carboxy-1H-benzimidazol-2-yl)-9H-fluorène-9(R,S)-yl]-amide de l'acide 1H-pyrrolo[2,3-b]pyridine-4-carboxylique, obtenu dans l'exemple 206, 67 mg de chlorhydrate de diméthylamine, 321 mg de hexafluorophosphate de benzotriazole-1-yl-oxy-trispyrrolidinophosphonium (PYBOP), 83 mg de 1-hydroxybenzotriazole (HOBT) et 272 µL de N,N diisopropyléthylamine (DIPEA) dans 2,5 mL de diméthylformamide, pendant 20 heures à température ambiante. On amène à sec le diméthylformamide, on ajoute de l'eau, on essore le précipité qui est ensuite lavé à l'eau puis avec une solution saturée d'hydrogénocarbonate de sodium à 10% et enfin à l'eau. Le solide brut obtenu est séché puis purifié par flash-chromatographie sur gel de silice (40-63µm) en éluant avec un mélange de dichlorométhane et de méthanol (95/5 puis 9/1 en volumes). Après empâtage à l'éther diisopropylique, on obtient ainsi 126 mg de [4-(5-diméthylaminocarbonyl-1H-benzimidazol-2-yl)-9H-fluorène-9(R,S)-yl]-amide de l'acide 1H-pyrrolo[2,3-b]pyridine-4-carboxylique, sous forme d'une poudre blanc cassé dont les caractéristiques sont les suivantes :
Point de fusion (Köfler) = 220°C.
Spectre de masse (EI/CI) : m/z = 512 (M+)
Spectre RMN 1H (400 MHz, □ en ppm, DMSO-d6) : 3,03 (s, 3H) ; 3,04 (s, 3H) ; 6,41 (d, J = 8,5 Hz, 1H) ; 6,91 (dd, J = 2,0 et 3,5 Hz, 1H) ; de 7,20 à 7,35 (m, 3H) ; 7,47 (d, J = 5,0 Hz, 1H) ; 7,53 (t, J = 7,5 Hz, 1H) ; de 7,56 à 7,67 (m, 4H) ; 7,68 (d large, J = 8,0 Hz, 1H) ; de 7,77 à 7,82 (m, 2H) ; 8,30 (d, J = 5,0 Hz, 1H); 9,28 (d, J = 8,5 Hz, 1H); 11,85 (m large, 1H) ; 13,15 (m large, 1H).

### Exemple 233 : Synthèse du 4-[5-(2-diméthylamino-éthyl)aminocarbonyl-1H-benzimidazol-2-yl)-9H-fluorène-9(R,S)-yl]-amide de l'acide 1H-pyrrolo[2,3-b]pyridine-4-carboxylique

Dans un ballon de 10 mL, on dissout 200 mg de [4-(5-carboxy-1H-benzimidazole-2-yl)-9H-fluorène-9(R,S)-yl]-amide de l'acide 1H-pyrrolo[2,3-b]pyridine-4-carboxylique, obtenu dans l'exemple 206, dans 2,5 mL de diméthylformamide, puis on ajoute successivement 100 µl de N,N-diméthyléthylènediamine, 79 mg de chlorhydrate de 1-(3-diméthylaminopropyl)-3-éthylcarbodiimide (EDCI) et 28 mg de 1-hydroxybenzotriazole (HOBT) puis on agite 20 heures à température ambiante. On amène à sec le diméthylformamide, on ajoute de l'eau et on essore le précipité formé qui est ensuite lavé à l'eau puis avec une solution saturée d'hydrogénocarbonate de sodium à 10% et enfin à l'eau. Le solide brut obtenu est séché puis purifié par flash-chromatographie sur gel de silice (40-63µm) en éluant avec un mélange de dichlorométhane et d'ammoniac en solution 7N dans le méthanol (95/5 puis 9/1 en volumes). Après empâtage à l'éther diisopropylique, on obtient ainsi 103 mg de 4-[5-(2-diméthylamino-éthyl)aminocarbonyl-1H-benzimidazol-2-yl)-9H-fluorène-9(R,S)-yl]-amide de l'acide 1H-pyrrolo[2,3-b]pyridine-4-carboxylique , sous forme d'une poudre blanc cassé dont les caractéristiques sont les suivantes :
Point de fusion (Köfler) = >260°C.
Spectre de masse (LC/MS/ES+/-) : m/z = 555 (M+)
Spectre RMN 1H (400 MHz, □ en ppm, DMSO-d6) : 2,21 (s, 6H) ; 2,45 (t, J = 6,5 Hz, 2H) ; 3,41 (q, J = 6,5 Hz, 2H) ; 6,41 (d, J = 8,5 Hz, 1H) ; 6,91 (dd, J = 2,0 et 3,5 Hz, 1H) ; 7,25 (t, J = 7,5 Hz, 1H) ; 7,35 (t, J = 7,5 Hz, 1H) ; de 7,43 à 7,86 (m, 2H) ; 7,47 (d, J = 5,0 Hz, 1H) ; 7,53 (t, J = 7,5 Hz, 1H) ; 7,62 (m, 2H) ; 7,69 (d large, J = 8,0 Hz, 1H) ; 7,77 (d, J = 7,5 Hz, 1H) ; 7,82 (d large, J = 8,0 Hz, 1H) ; 8,20 (m étalé, 1H) ; 8,30 (d, J = 5,0 Hz, 1H) ; 8,42 (t large, J = 6,5 Hz, 1H) ; 9,28 (d, J = 8,5 Hz, 1H) ; 11,85 (m large, 1H) ; 13,2 (m étalé, 1H).

### Exemple 234 : Synthèse du [4-(4,5,6,7-tétrafluoro-1H-benzimidazol-2-yl)-9H-fluorèn-9(R,S)-yl]-amide de l'acide 1H-pyrrolo[2,3-b]pyridine-4-carboxylique

Etape 1 : On opère comme à l'étape 1 de l'exemple 1, mais à partir de 16,7 ml de méthanol 22,98 mL de triéthylamine et 20 g de chlorure de l'acide 9H-fluorène-9-one-4-carboxylique dans 750 mL de dichlorométhane. Après 20 heures d'agitation à température ambiante, on ajoute 150 ml d'eau. La phase organique est décantée, lavéee à l'eau puis avec une solution aqueuse saturée d'hydrogénocarbonate de sodium, séchée sur sulfate de magnésium et concentrée sous pression réduite Après empâtage à l'éther diisopropylique, on obtient ainsi 18 g d'ester méthylique de l'acide fluorèn-9-one-4-carboxylique, sous forme d'une poudre jaune utilisée telle quelle à l'étape suivante, dont les caractéristiques sont les suivantes :
Spectre de masse (LC/MS/ES/+/-): m/z = 238 (M+)

Etape 2 : On opère comme à l'exemple 5, mais à partir de 20 g d'ester méthylique de l'acide 9H-fluorèn-9-one-4-carboxylique, obtenu à l'étape précédente, de 17,5 g de chlorhydrate d'hydroxylamine et de 34,45 g d'acétate de sodium, agités pendant 20 heures à température ambiante dans 350 mL d'éthanol. Après concentration du solvant sous pression réduite, le milieu réactionnel est repris dans 1000 ml d'acétate d'éthyle, lavé à l'eau puis avec une solution aqueuse saturée de chlorure de sodium, séché sur sulfate de magnésium et concentré sous pression réduite Après empâtage à l'éther diisopropylique, on obtient ainsi 22 g de 4-(méthoxycarbonyl)-9H-fluorène-9-one oxime (Z,E), en mélange 50/50 des isomères Z et E, sous forme d'une poudre jaune utilisée telle quelle à l'étape suivante.

Etape 3 : On opère comme à l'exemple 6, mais à partir de 22 g de 4-(méthoxycarbonyl)-9H-fluorène-9-one oxime (Z,E), obtenue à l'étape précédente, et de 5 g de Nickel de Raney dans 900 mL d'éthanol et 900 mL de tétrahydrofurane, pendant 6 heures à 60°C, sous une pression initiale d'un bar d'hydrogène. Après filtration du catalyseur, concentration sous pression réduite et empâtage à l'éther diisopropylique, on obtient 20 g de l'ester méthylique de l'acide 9(R,S)-amino-9H-fluorène-4-carboxylique, sous forme d'un solide vert utilisé telle quelle à l'étape suivante.

Etape 4 : On opère comme dans l'exemple 14, mais à partir de 10 g d'ester méthylique de l'acide 9(R,S)-amino-9H-fluorène-4-carboxylique, obtenu à l'étape précédente, 6,44 g d'acide 1H-pyrrolo-[2,3-b]pyridine-4-carboxylique, 8,37g de chlorhydrate de 1-(3-diméthylaminopropyl)-3-éthylcarbodiimide (EDCI) et 5,9 g de 1-hydroxybenzotriazole (HOBT) dans 150 mL de diméthylformamide, pendant 20 heures à température ambiante. Après purification par empâtage dans l'éther diisopropylique, on obtient 12,4 g d'ester méthylique de l'acide 9(R,S)-[(1H-pyrrolo[2,3-b]pyridine-4-carbonyl)-amino]-9H-fluorène-4-carboxylique, sous forme d'une poudre beige dont les caractéristiques sont les suivantes :
Spectre de masse (LC/MS/ES+/-) : m/z = 383 (M+)
Spectre de RMN (400 MHz, □ en ppm, DMSO-d6) : 3,99 (s, 3H) ; 6,33 (d, J = 8,5 Hz, 1H) ; 6,88 (m large, 1H) ; de 7,38 à 7,49 (m, 4H) ; 7,62 (m, 2H) ; 7,78 (m, 2H) ; 8,14 (d, J = 7,5 Hz, 1H) ; 8,28 (d, J = 5,0 Hz, 1H) ; 9,22 (d, J = 8,5 Hz, 1H) ; 11,85 (m étalé, 1H).

Etape 5 : Dans un tricol de 100 mL sous argon, on porte à reflux une suspension de 1 g d'ester méthylique de l'acide 9(R,S)-[(1H-pyrroto[2,3-b]pyridine-4-carbonyl)-amino]-9H-fluorène-4-carboxylique, obtenu à l'étape précédente, dans 50 mL de tétrahydrofurane anhydre, puis on ajoute, goutte à goutte, 7,825 mL d'une solution d'hydrure de diisopropylaluminium (DIBAL-H) à 20% dans le toluène. Après 15 minutes d'agitation au reflux, on ajoute de nouveau 2,6 mL de la solution précédente de DIBAL-H et on maintient le reflux pendant 15 minutes supplémentaires. Après refroidissement à température ambiante, le milieu réactionnel est coulé, goutte à goutte, dans 25 mL d'une solution aqueuse saturée de chlorure d'ammonium, puis extrait 3 fois par 25 mL d'acétate d'éthyle. Les phases organiques jointes sont séchées sur sulfate de magnésium et concentrées à sec sous pression réduite. Après purification par flash-chromatographie sur gel de silice (40-63µm), en éluant avec un mélange de dichlorométhane et de méthanol (97,5/2,5 en volumes), puis empâtage à l'éther diisopropylique, on obtient ainsi 200 mg de 9(R,S)-[(1H-pyrrolo[2,3-b]pyridine-4-carbonyl)-amino]-9H-fluorène-4-méthanol, sous forme d'un solide beige utilisé, don't les caractéristiques sont les suivantes :
Spectre de masse (LC/MS/ES+/-) : m/z = 355 (M+)

Etape 6 : Dans un tricol de 100 mL, à une solution de 500 mg de 9(R,S)-[(1H-pyrrolo[2,3-b]pyridine-4-carbonyl)-amino]-9H-fluorène-4-méthanol, préparé à l'étape précédente, dans 40 mL de diméthylformamide, on ajoute 440 mg de N-oxyde de 2,2,6,6-tétraméthyl-pipéridine (TEMPO) et 278,6 mg de chlorure ferreux et on agite pendant 6 heures à température ambiante en faisant buller lentement de l'air dans le milieu réactionnel. On ajoute alors de 220 mg de TEMPO et 139,8 mg de chlorure ferreux supplémentaires et on maintient l'agitation pendant 20 heures de plus sous bullage d'air. Après dilution avec 50 mL d'eau, on amène à pH = 2 par addition d'une solution aqueuse 5M d'acide chlorhydrique et on extrait 3 fois à l'acétate d'éthyle. Les phases organiques jointes sont lavées à l'eau, séchées sur sulfate de magnésium et concentrées à sec sous pression réduite. Après purification par empâtage dans l'oxyde de diisopropyle, on obtient ainsi 380 mg de [4-formyl-9H-fluorène-9(R,S)-yl]-amide de l'acide 1H-pyrrolo[2,3-b]pyridine-4-carboxylique, sous forme d'un solide brun-clair, don't les caractéristiques sont les suivantes :
Spectre de masse (LC/MS/ES+/-) : m/z = 353 (M+)
Spectre de RMN (300 MHz, □ en ppm, DMSO-d6) : 6,36 (d, J = 8,5 Hz, 1H) ; 6,89 (dd, J = 2,0 et 3,5 Hz, 1H) ; 7,44 (d, J = 5,0 Hz, 1H) ; de 7,45 à 7,53 (m, 2H) ; de 7,56 à 7.70 (m, 3H) ; 7.89 (d large, J = 8,0 Hz, 1H) ; 8,02 (d,J = 7,5 Hz, 1H) ; 8,29 (d large, J = 5,0 Hz, 1H) ; 8,61 (m, 1H) ; 9,23 (d, J = 8,5 Hz, 1H) ; 10,6 (s, 1H) ; 11,85 (m étalé, 1H).

Etape 7 : Dans un ballon de 100 mL sous argon, on agite, pendant 20 heures à température ambiante, une suspension de 100 mg de [4-formyl-9H-fluorène-9(R,S)-yl]-amide de l'acide 1H-pyrrolo[2,3-b]pyridine-4-carboxylique, obtenu à l'étape précédente, 51 mg de 1,2-diamino-3,4,5,6-tétrafluorobenzène et 2.35 mg de chlorure ferrique dans 6 mL de méthanol et 2 mL de diméthylformamide. Après concentration du solvant sous pression réduite, le milieu réactionnel est repris un mélange d'eau et de dichlorométhane. La phase organique est lavée à l'eau puis avec une solution saturée de chlorure de sodium, séchée sur sulfate de magnésium et concentrée à sec sous pression réduite. Après purification par flash-chromatographie sur gel de silice (40-63µm), en éluant avec un mélange de dichlorométhane et de méthanol (95/5 en volumes), puis empâtage à l'éther diisopropylique, on obtient ainsi 15 mg de [4-(4,5,6,7-tétrafluoro-1H-benzimidazol-2-yl)-9H-fluorène-9(R,S)-yl]-amide de l'acide 1H-pyrrolo[2,3-b]pyridine-4-carboxylique, sous forme d'une poudre brune dont les caractéristiques sont les suivantes :
Spectre de masse (LC/MS/ES+/-) : m/z = 513 (M+)
Spectre RMN 1H (400 MHz, □ en ppm, DMSO-d6) : 6,41 (d, J = 8,5 Hz, 1H) ; 6,91 (m large, 1H) ; 7,27 (t, J = 7,5 Hz, 1H) ; 7,36 (t, J = 7,5 Hz, 1H) ; 7,46 (d, J = 5,0 Hz, 1H) ; 7,51 (m large partiellement masqué, 1H) ; 7,53 (t, J = 7,5 Hz, 1H) ; 7,62 (m, 2H) ; 7,70 (d large, J = 8,0 Hz, 1H) 7,79 (d, J = 7,5 Hz, 1H) ; 8,30 (d, J = 5,0 Hz, 1H) ; 9,29 (d, J = 8,5 Hz, 1H) ; 11,85 (m large, 1H); 14,2 (m étalé, 1H).

### Exemple 235 : Synthèse du 4-[5-(3-méthoxypropyl)aminocarbonyl-1H-benzimidazol-2-yl)-9H-fluorène-9(R,S)-yl]-amide de l'acide 1H-pyrrolo[2,3-b]pyridine-4-carboxylique

On opère comme dans l'exemple 233, mais à partir de 200 mg de [4-(5-carboxy-1H-benzimidazole-2-yl)-9H-fluorène-9(R,S)-yl]-amide de l'acide 1H-pyrrolo[2,3-b]pyridine-4-carboxylique, obtenu dans l'exemple 206, 84 µl de 3-méthoxypropylamine, 79 mg de chlorhydrate de 1-(3-diméthylaminopropyl)-3-éthylcarbodiimide (EDCI) et 28 mg de 1-hydroxybenzotriazole (HOBT) dans 2,5 mL de diméthylformamide pendant 20 heures à température ambiante. On amène à sec le diméthylformamide, on ajoute de l'eau et on essore le précipité formé qui est ensuite lavé à l'eau puis avec une solution saturée d'hydrogénocarbonate de sodium à 10% et enfin à l'eau. Le solide brut obtenu est séché puis purifié par flash-chromatographie sur gel de silice (40-63µm) en éluant avec un mélange de dichlorométhane et de méthanol (95/5 puis 9/1 en volumes). Après empâtage à l'éther diisopropylique, on obtient ainsi 153 mg de 4-[5-(3-méthoxypropyl)aminocarbonyl-1H-benzimidazol-2-yl)-9H-fluorène-9(R,S)-yl]-amide de l'acide 1H-pyrrolo[2,3-b]pyridine-4-carboxylique, sous forme d'une poudre blanc cassé dont les caractéristiques sont les suivantes :
Point de fusion (Köfler) = 234-236°C.
Spectre de masse (LC/MS/ES+/-) : m/z = 556 (M+)
Spectre RMN 1H (400 MHz, □ en ppm, DMSO-d6) : 1,81 (m, 2H); 3,26 (s, 3H) ; 3,36 (q, J = 6,5 Hz, 2H) ; 3,42 (t, J = 6,5 Hz, 2H) ; 6,42 (d, J = 8,5 Hz, 1H) ; 6,91 (dd, J = 2,0 et 3,5 Hz, 1H) ; 7,23 (m étalé partiellement masqué, 1H) ; 7,25 (t, J = 7,5 Hz, 1H) ; 7,35 (t, J = 7,5 Hz, 1H) ; de 7,40 à 7,90 (m masqué, 1H) ; 7,47 (d, J = 5,0 Hz, 1H) ; 7,53 (t, J = 7,5 Hz, 1H) ; 7,62 (m, 2H) ; 7,69 (d, J = 7,5 Hz, 1H) ; 7,77 (d, J = 7,5 Hz, 1H) ; 7,83 (d large, J = 7,5 Hz, 1H) ; 8,15 (m étalé, 1H) ; 8,30 (d, J = 5,0 Hz, 1H) ; 8,51 (t large, J = 6,5 Hz, 1H) ; 9,28 (d, J = 8,5 Hz, 1H) ; 11,85 (m large, 1H) ; 13,2 (m étalé, 1H).

### Exemple 236: Synthèse du 4-[5-(4-méthyl-pipérazine-1-yl)carbonyl-1H-benzimidazol-2-yl)-9H-fluorène-9(R,S)-yl]-amide de l'acide 1H-pyrrolo[2,3-b]pyridine-4-carboxylique

On opère comme dans l'exemple 233, mais à partir de 200 mg de [4-(5-carboxy-1H-benzimidazole-2-yl)-9H-fluorène-9(R,S)-yl]-amide de l'acide 1H-pyrrolo[2,3-b]pyridine-4-carboxylique, obtenu dans l'exemple 206, 50 µl de 1-méthylpipérazine, 79 mg de chlorhydrate de 1-(3-diméthylaminopropyl)-3-éthylcarbodiimide (EDCI) et 28 mg de 1-hydroxybenzotriazole (HOBT) dans 2,5 mL de diméthylformamide pendant 20 heures à température ambiante. On amène à sec le diméthylformamide, on ajoute de l'eau et on essore le précipité formé qui est ensuite lavé à l'eau puis avec une solution saturée d'hydrogénocarbonate de sodium à 10% et enfin à l'eau. Le solide brut obtenu est séché puis purifié par flash-chromatographie sur gel de silice (40-63µm) en éluant avec un mélange de dichlorométhane et d'ammoniac en solution dans le méthanol (95/5 puis 9/1 en volumes). Après empâtage à l'éther diisopropylique, on obtient ainsi 129 mg de 4-[5-(4-méthyl-pipérazine-1-yl)carbonyl-1H-benzimidazol-2-yl)-9H-fluorène-9(R,S)-yl]-amide de l'acide 1H-pyrrolo[2,3-b]pyridine-4-carboxylique, sous forme d'une poudre blanc-cassé dont les caractéristiques sont les suivantes:
Point de fusion (Köfler) = >260°C.
Spectre de masse (LC/MS/ES+/-) : m/z = 567 (M+)
Spectre RMN 1H (400 MHz, □ en ppm, DMSO-d6) : 2,22 (s, 3H) ; 2,36 (m large, 4H) ; 3,56 (m large, 4H) ; 6,41 (d, J = 8,5 Hz, 1H) ; 6,91 (dd, J = 2,0 et 3,5 Hz, 1H) ; 7,27 (t, J = 7,5 Hz, 1H) ; 7,31 (m étalé, partiellement masqué, 1H) ; 7,35 (t, J = 7,5 Hz, 1H) ; 7,47 (d, J = 5,0 Hz, 1H) ; 7,53 (t, J = 7,5 Hz, 1H) ; de 7,57 à 7,83 (m, 5H) ; 7,67 (d, J = 8,0 Hz, 1H) ; 7,77 (d, J = 7,5 Hz, 1H) ; 8,30 (d, J = 5,0 Hz, 1H) ; 9,28 (d, J = 8,5 Hz, 1H) ; 11,85 (m large, 1H) ; 13,15 (m étalé, 1H) .

### Exemple 237 : Synthèse du [4-(6-diméthylsulfamoyl-1H-benzimidazol-2-yl)-9H-fluorèn-9(R,S)-yl]-amide de l'acide 1H-pyrrolo[2,3-b]pyridine-4-carboxylique

Dans un appareil micro-ondes (Biotage), on chauffe, pendant 1 heure à 220°C, 20 mg de [4-formyl-9H-fluorèn-9(R,S)-yl]-amide de l'acide 1H-pyrrolo[2,3-b]pyridine-4-carboxylique, obtenu à l'étape 6 de l'exemple 234, et 13,4 mg de 4-N,N-diméthylsulfonamido-benzène-1,2-diamine dans 1,5 mL de nitrobenzène. Après retour à température ambiante, on ajoute 2 mL d'eau et extrait avec 3 fois avec 15 mL d'acétate d'éthyle. Les phases organiques jointes sont concentrées à sec sous pression réduite et le résidu est purifié par flash-chromatographie sur gel de silice (40-63µm), en éluant avec un mélange de dichlorométhane et d'ammoniac à 7N dans le méthanol (90/10 en volumes). On obtient ainsi 2 mg de [4-(6-diméthylsulfamoyl-1H-benzimidazol-2-yl)-9H-fluorèn-9(R,S)-yl]-amide de l'acide 1H-pyrrolo[2,3-b]pyridine-4-carboxylique, sous forme d'une poudre ambre dont les caractéristiques sont les suivantes :
Spectre de masse (LC/MS): m/z = 549 (M+)

### Exemple 238 : Synthèse du 4-[5-(pyrrolidin-1-yl)carbonyl)-1H-benzimidazol-2-yl)-9H-fluorène-9(R,S)-yl]-amide de l'acide 1H-pyrrolo[2,3-b]pyridine-4-carboxylique

On opère comme dans l'exemple 233, mais à partir de 143 mg de [4-(5-carboxy-1H-benzimidazol-2-yl)-9H-fluorène-9(R,S)-yl]-amide de l'acide 1H-pyrrolo[2,3-b]pyridine-4-carboxylique, obtenu dans l'exemple 206, 27 µl de pyrrolidine, 56 mg de chlorhydrate de 1-(3-diméthylaminopropyl)-3-éthylcarbodiimide (EDCI) et 20 mg de 1-hydroxybenzotriazole (HOBT) dans 1,5 mL de diméthylformamide pendant 20 heures à température ambiante. On amène à sec le diméthylformamide, on ajoute de l'eau et on essore le précipité formé qui est ensuite lavé à l'eau puis avec une solution saturée d'hydrogénocarbonate de sodium à 10% et de nouveau à l'eau. Le solide brut obtenu est séché puis purifié par flash-chromatographie sur gel de silice (40-63µm) en éluant avec un mélange de dichlorométhane et de méthanol (95/5 puis 9/1 en volumes). Après empâtage à l'éther diisopropylique, on obtient ainsi 89,5 mg de 4-[5-(pyrrolidin-1-yl)carbonyl)-1H-benzimidazol-2-yl)-9H-fluorène-9(R,S)-yl]-amide de l'acide 1H-pyrrolo[2,3-b]pyridine-4-carboxylique, sous forme d'une poudre beige dont les caractéristiques sont les suivantes: Point de fusion (Kofler) = >260°C.
Spectre de masse (LC/MS/ES+/-) : m/z = 538 (M+)
Spectre RMN 1H (400 MHz, □ en ppm, DMSO-d6) : 1,88 (m large, 4H) ; 3,52 (t large, J = 6,5 Hz, 4H) ; 6,41 (d, J = 8,5 Hz, 1H) ; 6,91 (dd, J = 2,0 et 3,5 Hz, 1H);7,26(t,J=7,5 Hz, 1H) ; 7,35 (t, J =8,0 Hz, 1H) ; 7,47 (d, J = 5,0 Hz, 1H) ; 7,47 (m partiellement masqué, 1H) ; 7,53 (t, J = 7,5 Hz, 1H) ; de 7,58 à 7,95 (m, 5H) ; 7,68 (d large, J = 8,0 Hz, 1H) ; 7,77 (d, J = 7,5 Hz, 1H) ; 8,30 (d, J = 5,0 Hz, 1H) ; 9,28 (d, J = 8,5 Hz, 1H) ; 11,85 (m large, 1H) ; 13,1 (m étalé, 1H).

### Exemples 239 et 240 : Dédoublement des énantiomères de l'ester de méthyle de l'acide 2-{9(R,S)-[(1H-pyrrolo[2,3-b]pyridine-4-carbonyl)-amino]-9H-fluorèn-4-yl}-1H-benzimidazole-5-carboxylique.

En opérant comme à l'exemple 153, à partir de 200 mg d'ester de méthyle de l'acide 2-{9(R,S)-[(1H-pyrrolo[2,3-b]pyridine-4-carbonyl)-amino]-9H-fluorèn-4-yl}-1H-benzimidazole-5-carboxylique, obtenu comme à l'exemple 205, mais en utilisant une colonne chirale de type Pirkle Whelk 01 SS, en éluant avec un mélange de n-heptane 50 % EtOH 50 % TEA 0. 1 % et en détectant les produits élués par spectroscopie UV à 254 nM, on obtient en récupérant et en concentrant sous pression réduite les premières fractions éluées 63,4 mg d'énantiomère dextrogyre de l'acide 2-{9(R,S)-[(1H-pyrrolo[2,3-b]pyridine-4-carbonyl)-amino]-9H-fluorèn-4-yl}-1H-benzimidazole-5-carboxylique, sous la forme d'un solide amorphe jaune pâle dont les caractéristiques sont les suivantes :
□D20 = +168,8 +/- 2,2 ° (c = 0,5 ; DMSO).

En récupérant et concentrant sous pression réduite les secondes fractions éluées, on obtient 78,9 mg d'énantiomère lévogyre de l'acide 2-{9(R,S)-[(1H-pyrrolo[2,3-b]pyridine-4-carbonyl)-amino]-9H-fluorèn-4-yl)-1H-benzimidazole-5-carboxylique, contenant environ 1% d'énantiomère dextrogyre, sous la forme d'un solide amorphe jaune pâle dont les caractéristiques sont les suivantes :
□D20 = -155,75 +/- 2,1 ° (c = 0,5 ; DMSO).

### Exemple 241 : Synthèse du {{4-{5-[2-(pyrrolidin-1-yl)éthylaminocarbonyl]-1H-benzimidazole-2-yl}-9H-fluorène-9(R,S)-yl}}amide de l'acide 1H-pyrrolo[2,3-b]pyridine-4-carboxylique

Etape 1 : Dans un ballon de 50 mL sous argon, on dissout 1 g de [4-(5-methoxycarbonyl-1H-benzimidazol-2-yl)-9H-fluorène-9-one, obtenue comme à l'étape 2 de l'exemple 205, dans 15 mL de méthanol, puis on ajoute 5,9 mL d'une solution aqueuse 1,2 M d'hydroxyde de lithium et on porte au reflux pendant 24 heures. On amène à sec sous pression réduite, on ajoute de l'eau, on refroidit à 10°C, puis on acidifie à pH 4-5 par une solution aqueuse 1 M d'acide chlorhydrique. Le solide brut est filtré, lavé à l'eau puis séché. On empâte le solide obtenu à l'éther diisopropylique, on filtre et rince. On obtient ainsi 919 mg de 4-(5-carboxy-1H-benzimidazol-2-yl)-9H-fluorène-9-one, sous forme d'une poudre jaune, utilisée telle quelle à l'étape suivante dont les caractéristiques sont les suivantes:
Spectre de masse (LC/MS/ES/+/-) : m/z = 340 (M+)

Etape 2 : On opère comme dans l'exemple 234, mais à partir de 380 mg de [4-(5-carboxy-1H-benzimidazol-2-yl)-9H-fluorène-9-one, obtenue à l'étape précédente, 140 mg de N-(2-aminoethyl)pyrrolidine, 214 mg de chlorhydrate de 1-(3-diméthylaminopropyl)-3-éthylcarbodiimide (EDCI) et 75 mg de 1-hydroxybenzotriazole (HOBT) dans 4 mL de diméthylformamide pendant 20 heures à température ambiante. Après empâtage du produit brut à l'éther diisopropylique, filtré et rincé, on obtient ainsi 472 mg de 4-{5-[2-(pyrrolidin-1 yl)-éthylaminocarbonyl]-1H-benzimidazol-2-yl}-9H-fluorène-9-one, sous forme d'une poudre jaune utilisée telle quelle à l'étape suivante dont les caractéristiques sont les suivantes :
Spectre de masse (LC/MS/ES+/-) : m/z = 436 (M+)

Etape 3 : On opère comme dans l'exemple 5, mais à partir de 380 mg de 4-{5-[2-(pyrrolidin-1yl)-éthylaminocarbonyl]-1H-benzimidazol-2-yl}-9H-fluorène-9-one, obtenue à l'étape précédente, de 225 mg de chlorhydrate d'hydroxylamine et de 442 mg d'acétate de sodium dans 6 mL d'éthanol agités pendant 20h à température ambiante. Après purification par flash-chromatographie sur gel de silice (40-63µm) en éluant avec un mélange de dichlorométhane et d'ammoniac en solution 7N dans le méthanol (95/5 puis 9/1 en volumes), puis empâtage à l'éther diisopropylique, on filtre et rince. On obtient ainsi 303 mg de 4-{5-[2-(pyrrolidin-1-yl)-éthylaminocarbonyl]-1H-benzimidazol-2-yl}-9H-fluorène-9-one oxime (Z,E), sous forme d'un solide blanc cassé utilisé tel quel à l'étape suivante dont les caractéristiques sont les suivantes:
Spectre de masse (LC/MS/ES/+/-) : m/z = 451 (M+).

Etape 4 : On opère comme dans l'exemple 216. Dans un ballon de 20 mL sous atmosphère d'argon, on dissout 303 mg de 4-{5-[2-(pyrrolidin-1-yl)-éthylaminocarbonyl]-1H-benzimidazol-2-yl}9H-fluorène-9-one oxime (Z,E), obtenue à l'étape précédente, dans un mélange de 1,5 mL d'éthanol et 1,5 mL de d'eau et 1,5 mL d'acide acétique, à température ambiante. On ajoute en trois fois 175 mg de zinc, entre chaque ajout, on agite environ une heure à deux heures. On ajoute de la Célite et on filtre. Le filtrat est concentré sous pression réduite. Après purification par flash-chromatographie sur gel de silice (40-63µm) en éluant avec un mélange de dichlorométhane et d'ammoniac en solution 7N dans le méthanol (9/1 en volumes) puis empâtage à l'éther diisopropylique, on obtient ainsi 257 mg de 4-{5-[2-(pyrrolidin-1-yl)-éthylaminocarbonyl]-1H-benzimidazol-2-yl}-9H-fluorène-9(R,S)-amine sous forme d'un solide beige, utilisé tel quel à l'étape suivante, dont les caractéristiques sont les suivantes:
Spectre de masse (EI/CI) : m/z = 437 (M+)

Etape 5 : On opère comme dans l'exemple 14, mais à partir de 257 mg de 4-{5-[2-(pyrrolidin-1-yl)-éthylaminocarbonyl]-1H-benzimidazol-2-yl}-9H-fluorène-9(R,S)-amine, obtenue à l'étape précédente, 95 mg d'acide 1H-pyrrolo-[2,3-b]pyridine-4-carboxylique, 112 mg de chlorhydrate de 1-(3-diméthylaminopropyl)-3-éthylcarbodiimide (EDCI) et 40 mg de 1-hydroxybenzotriazole (HOBT) dans 2,5 mL de diméthylformamide, pendant 20 heures à température ambiante. Après purification par flash-chromatographie sur gel de silice (40-63µm), en éluant avec un mélange de dichlorométhane et d'ammoniac en solution 7N dans le méthanol (95/5 puis 9/1 en volumes), puis empâtage à l'éther diisopropylique, on obtient ainsi 196 mg de {{4-{5-[2-(pyrrolidin-1-yl)éthylaminocarbonyl]-1H-benzimidazol-2-yl}-9H-fluorène-9(R,S)-yl}}amide de l'acide 1H-pyrrolo[2,3-b]pyridine-4-carboxylique, sous forme d'un solide jaune pâle dont les caractéristiques sont les suivantes:
Spectre de masse (LC/MS/ES+/-) : m/z = 581 (M+)
Spectre RMN 1H (400 MHz, □ en ppm, DMSO-d6) : 1,69 (m, 4H) ; 2,61 (t, J = 7,0 Hz, 2H); 3,31 (m masqué, 4H) ; 3,43 (m, 2H) ; 6,41 (d, J = 8,5 Hz, 1H); 6,91 (dd, J = 2,0 et 3,5 Hz, 1H) ; 7,25 (t, J = 7,5 Hz, 1H) ; 7,35 (t, J = 7,5 Hz, 1H) ; 7,47 (d, J = 5,0 Hz, 1H) ; de 7,50 à 7,89 (m, 4H) ; 7,53 (t, J = 7,5 Hz, 1H) ; 7,69 (d, J = 8,0 Hz, 1H) ; 7,78 (d, J = 7,5 Hz, 1H) ; 7,83 (d large, J = 8,0 Hz, 1H) ; 8,19 (m étalé, 1H) ; 8,30 (d, J = 5,0 Hz, 1H) ; 8,47 (t large, J = 6,0 Hz, 1H) ; 9,28 (d, J = 8,5 Hz, 1H) ; 11,85 (m large, 1H) ; 13,2 (m étalé, 1H).

### Exemple 242 : Synthèse du {4-[5-fluoro-6-(4-méthyl-perhydro-1,4-diazepin-1-yl)-1H-benzimidazol-2-yl]-9H-fluorèn-9(R,S)-yl}-amide de l'acide1H-pyrrolo[2,3-b]pyridine-4-carboxylique

Etape 1 : On opère comme à l'étape 1 de l'exemple 1, mais à partir de 2,86 g de 4-fluoro-5-(4-méthyl-perhydro-1,4-diazépin-1-yl)-benzène-1,2-diamine, qui peut être préparée selon J. Het. Chem. 2005, 42(1), 67-71, 2,81 mL de triéthylamine et 2,427 g de chlorure de l'acide 9H-fluorène-9-one-4-carboxylique dans 50 mL de dichlorométhane pendant 20 heures à température ambiante. Après purification par flash-chromatographie sur gel de silice (40-63µm), en éluant avec un mélange de dichlorométhane et de méthanol (95/5 en volumes), puis empâtage à l'éther diisopropylique, on obtient ainsi 950 mg du [2-amino-5-fluoro-4-(4-méthyl-perhydro-1,4-diazepin-1-yl)-phényl]-amide de l'acide 9-oxo-9H-fluorène-4-carboxylique, sous forme d'une poudre orange utilisée telle quelle à l'étape suivante, dont les caractéristiques sont les suivantes :
Spectre de masse (LC/MS/ES/+/-): m/z = 444 (M+)

Etape 2 : On opère comme dans l'exemple 68. Dans un ballon de 100 mL sous atmosphère d'argon, on chauffe à reflux pendant 6 heures, une suspension de 950 mg du [2-amino-5-fluoro-4-(4-méthyl-perhydro-1,4-diazepin-1-yl)-phényl]-amide de l'acide 9-oxo-9H-fluorène-4-carboxylique, obtenu à l'étape précédente, dans 15 mL d'acide acétique. Après purification par empâtage à l'éther diisopropylique, on obtient ainsi, 950 mg de 4-[5-fluoro-6-(4-méthyl-perhydro-1,4-diazepin-1-yl)-1H-benzimidazol-2-yl]-9H-fluorène-9-one, sous forme d'une poudre orange utilisée telle quelle à l'étape suivante, dont les caractéristiques sont les suivantes :
Spectre de masse (LCMS) : m/z = 426 (M+)

Etape 3 : On opère comme à l'exemple 5, mais à partir de 950 mg de 4-[5-fluoro-6-(4-méthyl-perhydro-1,4-diazepin-1-yl)-1H-benzimidazol-2-yl]-9H-fluorène-9-one, obtenue à l'étape précédente, de 464 mg de chlorhydrate d'hydroxylamine et de 914 mg d'acétate de sodium dans 45 mL d'éthanol agités pendant 20h à température ambiante. Après concentration du solvant sous pression réduite, le résidu est repris successivement par de l'eau, filtré, lavé à l'eau, rincé à l'éther diisopropylique et redissout dans du toluène. Après concentration à sec sous pression réduite, on obtient ainsi 950 mg de 4-[5-fluoro-6-(4-méthyl-perhydro-1,4-diazepin-1-yl)-1H-benzimidazol-2-yl]-9H-fluorène-9-one oxime (Z,E) en mélange 50-50 des isomères Z et E, sous forme d'une poudre brune utilisée telle quelle à l'étape suivante.
Spectre de masse (LCMS) : m/z = 441 (M+)

Etape 4 : On opère comme à l'exemple 6, à partir de 950 mg de 4-[5-fluoro-6-(4-méthyl-perhydro-1,4-diazepin-1-yl)-1H-benzimidazol-2-yl]-9H-fluorène-9-one oxime (Z,E) obtenue à l'étape précédente, et de 135 mg de Nickel de Raney dans 20 mL d'éthanol et 20 mL de tétrahydrofurane pendant 16 heures à 60°C sous une pression initiale d'un bar d'hydrogène. Après filtration du catalyseur, concentration à sec sous pression réduite, puis purification par empâtage à l'oxyde de diisopropyle, on obtient ainsi 700 mg de 4-[5-fluoro-6-(4-méthyl-perhydro-1,4-diazepin-1-yl)-1H-benzimidazol-2-yl]-9H-fluorèn-9(R,S)-yl-amine, sous forme d'une poudre orange utilisée telle quelle à l'étape suivante.
Spectre de masse (LCMS) : m/z = 427 (M+)

Etape 5 : On opère comme dans l'exemple 14, mais à partir de 700mg de 4-[5-fluoro-6-(4-méthyl-perhydro-1,4-diazepin-1-yl)-1H-benzimidazol-2-yl]-9H-fluorèn-9(R,S)-yl-amine, obtenue à l'étape précédente, 162 mg d'acide 1H-pyrrolo-[2,3-b]pyridine-4-carboxylique, 211 mg de chlorhydrate de 1-(3-diméthylaminopropyl)-3-éthylcarbodiimide (EDCI) et 148 mg de 1-hydroxybenzotriazole (HOBT) dans 7 mL de diméthylformamide, pendant 20 heures à température ambiante. Après purification par flash-chromatographie sur gel de silice (40-63µm), en éluant avec un mélange de dichlorométhane et de méthanol ammoniacal 7N (95/5 en volumes), puis empâtage au méthanol, on obtient ainsi 255 mg de {4-[5-fluoro-6-(4-méthyl-perhydro-1,4-diazepin-1-yl)-1H-benzimidazol-2-yl]-9H-fluorèn-9(R,S)-yl}-amide de l'acide 1H-pyrrolo[2,3-b]pyridine-4-carboxylique, sous forme d'une poudre beige dont les caractéristiques sont les suivantes :
Spectre de masse (LC/MS/ES+/-) : m/z = 571 (M+)
Spectre RMN 1H (300 MHz, □ en ppm, DMSO-d6) : 1,96 (m, 2H) ; 2,32 (s, 3H) ; 2,63 (m, 2H) ; 2,73 (m, 2H) ; 3,36 (t large, J = 5,5 Hz, 4H) ; 6,40 (d, J = 8,5 Hz, 1H) ; 6,90 (d large, J = 3,5 Hz, 1H) ; 7,05 (m étalé, 1H) ; de 7,20 à 7,80 (m masqué, 1H) ; 7,26 (t, J = 7,5 Hz, 1H) ; 7,34 (t, J = 7,5 Hz, 1H) ; 7,46 (d, J = 5,0 Hz, 1H) ; 7,49 (t, J = 7,5 Hz, 1H) ; de 7,57 à 7,68 (m, 4H) ; 7,72 (d large, J = 8,0 Hz, 1H) ; 8,29 (d, J = 5,0 Hz, 1H) ; 9,26 (d, J = 8,5 Hz, 1H) ; 11,85 (m large, 1H) ; 12,7 (m étalé, 1H).

### Exemples 243 et 244 : Synthèse de la 2-bromo-5-(3H-imidazo[4,5-c]pyridin-2-yl)-9H-fluorèn-9-one et de la 2,7-dibromo-5-(3H-imidazo[4,5-c]pyridin-2-yl)-fluorèn-9-one:

Etape 1 : Dans un ballon tricol de 100 mL, équipé d'une agitation mécanique, on agite 4.48 g d'acide 9-fluorènone-4-carboxylique en suspension dans 40 mL d'acide trifluoroacétique et de 12 mL d'acide sulfurique concentré. À ce mélange réactionnel sombre, on ajoute 5.33 g de N-bromosuccinimide sur une période de 9 h. Le taux de conversion du produit de départ est suivi par LC/MS, et la réaction est stoppée lorsqu'il plafonne à 75 %. Le précipité jaune formé est filtré sur fritté, lavé par 3 fois 20 mL d'acide trifluoroacétique et 6 fois 50 mL d'eau puis séché à l'étuve 50°C sous vide poussé. On obtient 6.6 g d'une poudre jaune, composée d'un mélange 55/45 d'acide 2-bromo-9H-fluorèn-9-one-4-carboxylique et d'acide 2,7-dibromo-9H-fluorén-9-one-4-carboxylique, utilisée telle quelle à l'étape suivante.

Etape 2: Dans un ballon de 10 mL, on met en suspension 150 mg du mélange obtenu à l'étape 1 dans 2 mL de dichlorométhane additionnés de 50 µL de diméthylformamide. On ajoute 70 mg de chlorure d'oxalyle et on agite le mélange réactionnel à température ambiante pendant 30 minutes. Après dilution par 10 mL de dichlorométhane, la solution obtenue est ajoutée goutte à goutte en 20 min et sous argon, à une suspension de 61 mg de 3,4-diaminopyridine et de 102 mg de triéthylamine dans 2 mL de dichlorométhane. Le précipité obtenu est filtré et lavé par 3 fois 20 mL de dicholorométhane pour conduire à 110 mg d'un mélange 65/35 de (3-amino-pyridin-4-yl)-amide de l'acide 7-bromo-9-oxo-9H-fluorène-4-carboxylique et de (3-amino-pyridin-4-yl)-amide de l'acide 2,7-dibromo-9-oxo-9H-fluorène-4-carboxylique sous la forme d'une poudre jaune.

Etape 3 : Une suspension de 80 mg du mélange, obtenu à l'étape 2, dans 2 mL d'acide acétique est chauffée à reflux pendant 45 minutes. Le milieu réactionnel est additionné de 30 mL d'eau puis amené à pH = 9 par addition d'une solution aqueuse 1N d'hydroxyde de sodium. Le précipité jaune formé est filtré, lavé par 3 fois 20 mL d'eau et séché à l'étuve sous vide à 50°C pendant 4 heures. La poudre jaune obtenue est purifiée par flash chromatographie sur gel de silice en éluant avec un gradient de mélanges de dichlorométhane et de méthanol (de 100/0 à 90/10 en volumes) pour conduire :
- à 29 mg de 2-bromo-5-(3H-imidazo[4,5-c]pyridin-2-yl)-9H-fluorèn-9-one (exemple 243), sous la forme d'une poudre jaune dont les caractéristiques sont les suivantes:
   Spectres de masse (EI) : m/z = 376 (M+.)
   Spectre de RMN (300 MHz, □ en ppm, DMSO-d6) : 7,62 (t, J = 7,5 Hz, 1H) ; 7,69 (d large, J = 5,5 Hz, 1H) ; 7,72 (dd partiellement masqué, J = 2,0 et 8,5 Hz, 1H) ; de 7,79 à 7,86 (m, 3H) ; 7,95 (d large, J = 7,5 Hz, 1H) ; 8,39 (d, J = 5,5 Hz, 1H) ; 9,05 (s large, 1H) ; 13,5 (m très étalé, 1H).
- et à 14 mg de 2,7-dibromo-5-(3H-imidazo[4,5-c]pyridin-2-yl)-9H-fluorèn-9-one (exemple 244), sous la forme d'une poudre jaune dont les caractéristiques sont les suivantes :
   Spectre de masse (EI) : m/z = 454 (M+.)

### Exemple 245 : Synthèse du [4-(6-diméthylamino-1H-benzimidazol-2-yl)-9H-fluorèn-9(R,S)-yl]-amide de l'acide 1H-pyrrolo[2,3-b]pyridine-4-carboxylique

Dans un monocol de 20 mL, on introduit successivement, sous atmosphère d'argon, 23 mg de N4,N4-diméthyl-benzène-1,2,4-triamine, qui peut être obtenue par hydrogénation catalytique de la N4,N4-diméthyl-2-nitrobenzène-1,4-diamine en présence de palladium à 10% sur charbon sous une pression initiale de 5 bars d'hydrogène à 20°C, 53 mg de (4-formyl-9H-fluorèn-9(R,S)-yl)-amide de l'acide 1H-pyrrolo[2,3-b]pyridine-4-carboxylique, préparé comme à l'étape 6 de l'exemple 234, et 24 mg de chlorure ferrique et 2 mL de diméthylformamide. Après 3h d'agitation à 20°C, on concentre la solution sous pression réduite, puis on reprend le résidu obtenu dans 4 mL d'eau. Après essorage de l'insoluble formé sur plaque poreuse, lavage par 2 mL d'acétate d'éthyle et séchage à l'air, on obtient ainsi 41 mg de [4-(6-dimethylamino-1H-benzimidazol-2-yl)-9H-fluoren-9(R,S)-yl]-amide de l'acide 1H-pyrrolo[2,3-b]pyridine-4-carboxylique, sous forme de poudre violette dont les caractéristiques sont les suivantes :
Spectre de masse (E/I) : m/z = 487 (M+)
Spectre RMN 1H (400 MHz, □ en ppm, DMSO) : 3,08 (s large, 6H) ; 6,41 (d, J = 8,5 Hz, 1H) ; 6,89 (m large, 1H) ; de 6,95 à 7,34 (m étalé, 3H) ; 7,31 (t, J = 7,5 Hz, 1H) ; 7,41 (t, J = 7,5 Hz, 1H) ; 7,47 (d, J = 5,0 Hz, 1H) ; 7,60 (t, J = 7,5 Hz, 1H) ; 7,63 (d, J = 3,0 Hz, 1H) ; 7,68 (d, J = 7,5 Hz, 1H) ; 7,73 (d, J = 8,5 Hz, 1H) ; 7,77 (d, J = 7,5 Hz, 1H) ; 7,89 (d, J = 7,5 Hz, 1H) ; 8,31 (d, J = 5,0 Hz, 1H) ; 9,34 (d, J = 8,5 Hz, 1H) ; 11,9 (m large, 1H) ; 14,8 (m étalé, 1H).

### Exemple 246 : Synthèse du {4-[6-(4-méthyl-pipérazin-1-yl)-1H-benzimidazol-2-yl]-9H-fluorèn-9(R,S)-yl}-amide de l'acide 1H-pyrrolo[2,3-b]pyridine-4-carboxylique

Etape 1: On opère comme à l'étape 1 de l'exemple 1, mais à partir de 2,06 g de 4-(4-méthyl-pipérazin-1-yl)benzène-1,2-diamine, 2,85 mL de triéthylamine et 2,43 g de chlorure de l'acide 9H-fluorène-9-one-4-carboxylique dans 50 mL de dichlorométhane, pendant 20 heures à température ambiante. Après purification par flash-chromatographie sur gel de silice (40-63µm), en éluant avec un mélange de dichlorométhane et de méthanol (95/5 en volumes), puis empâtage à l'éther diisopropylique, on obtient ainsi 4 g de [2-amino-4-(4-méthyl-pipérazin-1-yl)-phényl]-amide de l'acide 9-oxo-9H-fluorène-4-carboxylique, sous forme d'une poudre orange utilisée telle quelle à l'étape suivante, dont les caractéristiques sont les suivantes:
Spectre de masse (LC/MS/ES/+/-): m/z = 412 (M+)

Etape 2 : On opère comme dans l'exemple 68. Dans un ballon de 250 mL sous atmosphère d'argon, on chauffe à reflux pendant 6 heures, une suspension de 4 g du [2-amino-4-(4-méthyl-pipérazin-1-yl)-phényl]-amide de l'acide 9-oxo-9H-fluorène-4-carboxylique, obtenu à l'étape précédente, dans 64 mL d'acide acétique. Après purification par empâtage à l'éther diisopropylique, on obtient ainsi 2,5 g de 4-[6-(4-méthyl-pipérazin-1-yl)-1H-benzimidazol-2-yl]-9H-fluorène-9-one, sous forme d'une poudre orange utilisée telle quelle à l'étape suivante, dont les caractéristiques sont les suivantes :
Spectre de masse (LCMS) : m/z = 394 (M+)

Etape 3 : On opère comme à l'exemple 5, mais à partir de 2,5 g de 4-[6-(4-méthyl-pipérazin-1-yl)-1H-benzimidazol-2-yl]-9H-fluorène-9-one, obtenue à l'étape précédente, de 1,32 g de chlorhydrate d'hydroxylamine et de 2,6 g d'acétate de sodium dans 130 mL d'éthanol agités pendant 20 heures à température ambiante. Après purification par empâtage à l'éther diisopropylique, on obtient ainsi 1,5 g de 4-[6-(4-méthyl-pipérazin-1-yl)-1H-benzimidazol-2-yl]-9H-fluorène-9-one oxime (Z,E) en mélange 50-50 des isomères Z et E, sous forme d'une poudre brune utilisée telle quelle à l'étape suivante, dont les caractéristiques sont les suivantes:
Spectre de masse (LCMS) : m/z = 409 (M+)

Etape 4 : On opère comme à l'exemple 6, mais à partir de 1,5 g de 4-[6-(4-méthyl-pipérazin-1-yl)-1H-benzimidazol-2-yl]-9H-fuorène-9-one oxime (Z,E) obtenue à l'étape précédente, et de 229 mg de Nickel de Raney dans 35 mL d'éthanol et 35 mL de tétrahydrofurane pendant 48 heures à 60°C sous une pression initiale d'un bar d'hydrogène. Après filtration du catalyseur, concentration à sec pression réduite et purification par empâtage dans l'éther diisopropylique, on obtient ainsi 1,3 g de 4-[6-(4-méthyl-pipérazin-1-yl)-1H-benzimidazol-2-yl]-9H-fluorène-9(R,S)-yl-amine, sous forme d'une poudre orange utilisée telle quelle à l'étape suivante, dont les caractéristiques sont les suivantes :
Spectre de masse (LCMS) : m/z = 427 (M+)

Etape 5: On opère comme dans l'exemple 14, mais à partir de 416mg de 4-[6-(4-méthyl-pipérazin-1-yl)-1H-benzimidazol-2-yl]-9H-fluorène-9(R,S)-yl-amine, obtenue à l'étape précédente, 162 mg d'acide 1H-pyrrolo-[2,3-b]pyridine-4-carboxylique, 211 mg de chlorhydrate de 1-(3-diméthylaminopropyl)-3-éthylcarbodiimide (EDCI) et 148 mg de 1-hydroxybenzotriazole (HOBT) dans 7 mL de diméthylformamide, pendant 20 heures à température ambiante. Après purification par flash-chromatographie sur gel de silice (40-63µm), en éluant avec un mélange de dichlorométhane et de méthanol ammoniacal 7N (97,5/2,5 en volumes), puis empâtage à l'éther diisopropylique, on obtient ainsi 250 mg de {4-[6-(4-méthyl-pipérazin-1-yl)-1H-benzimidazol-2-yl]-9H-fluorèn-9(R,S)-yl}-amide de l'acide 1H-pyrrolo[2,3-b]pyridine-4-carboxylique, sous forme d'une poudre beige dont les caractéristiques sont les suivantes :
Spectre de masse (LC/MS/ES+/-) : m/z = 539 (M+)
Spectre RMN 1H (400 MHz, □en ppm, DMSO-d6): pour ce composé on observe un mélange de tautomères 50 % - 50 % avec : 2,25 (s, 3H) ; 2,51 (m partiellement masqué, 4H) ; 3,15 (m, 4H) ; 6,40 (d, J = 8,5 Hz, 1H) ; 6,91 (d, J = 3,5 Hz, 1H); de 6,93 à 7,10 (m étalé, 1,5H) ; de 7,15 à 7,70 (m étalé partiellement masqué, 1,5 H) ; 7,25 (t, J = 7,5 Hz, 1H) ; 7,33 (t, J = 7,5 Hz, 1H) ; 7,47 (d, J = 5,0 Hz, 1H) ; 7,49 (t partiellement masqué, J = 7,5 Hz, 1H) ; de 7,57 à 7,64 (m, 3H) ; 7,67 (d, J = 8,5 Hz, 1H) ; 7,72 (d, J = 7,5 Hz, 1H) ; 8,29 (d, J = 5,0 Hz, 1H) ; 9,27 (d, J = 8,5 Hz, 1H); 11,85 (m large, 1H) ; 12,6 (m étalé, 0,5H) ; 12,65 (m étalé, 0,5H).

### Exemple 247 : [[6-(méthyl-4(5)(R,S)-imidazolin-2-yl)-1H-benzimidazol-2-yl]-9H-fluorèn-9(R,S)-yl]-amide de l'acide 1H-pyrrolo[2,3-b]pyridine-4-carboxylique

On agite, à température ambiante sous argon, un mélange de 94 mg de (4-formyl-9H-fluorèn-9(R,S)-yl)-amide de l'acide 1H-pyrrolo[2,3-b]pyridine-4-carboxylique, préparé comme à l'étape 6 de l'exemple 234, de 60 mg de chlorhydrate de 2-(3,4-diaminophenyl)-4(5)(R,S)-méthyl-2-imidazoline, qui peut-être préparée selon le brevet DE 2,804,835, et 43 mg de trichlorure ferrique dans 3 mL de DMF sec. Après 50 heures, on évapore à sec le milieu réactionnel et on purifie le résidu par flash-chromatographie sur gel de silice en éluant avec un mélange de méthanol et de dichlorométhane (10/90 en volumes). On obtient ainsi 105 mg de [[6-(méthyl-4(5) (R,S)-imidazolin-2-yl)-1H-benzimidazol-2-yl]-9H-fluorén-9(R,S)-yl]-amide de l'acide 1H-pyrrolo[2,3-b]pyridine-4-carboxylique sous forme d'une mousse beige dont les caractéristiques sont les suivantes :
CCM (SiO2) Rf=0,3 (méthanol-dichlorométhane 20 : 80 en volumes)
Spectre de RMN (400 MHz, □ en ppm, DMSO-d6) : 1,41 (d, J = 6,5 Hz, 3H) ; 3,62 (dd, J = 8,0 et 11,0 Hz, 1H) ; 4,16 (t, J = 11,0 Hz, 1H) ; 4,50 (m, 1H) ; 6,41 (d, J = 8,5 Hz, 1H) ; 6,90 (dd, J = 2,0 et 3,5 Hz, 1H) ; 7,24 (t, J = 7,5 Hz, 1H) ; 7,36 (t, J = 7,5 Hz, 1H) ; de 7,43 à 7,68 (m masqué, 1H) ; 7,46 (d, J = 5,0 Hz, 1H) ; 7,55 (t, J = 7,5 Hz, 1H) ; de 7,60 à 7,66 (m, 2H) ; 7,72 (d, J = 7,5 Hz, 1H) ; 7,81 (d, J = 7,5 Hz, 1H) ; de 7,85 à 7,98 (m étalé, 2H) ; 8,30 (d, J = 5,0 Hz, 1H) ; 8,46 (m étalé, 1H); 9,29 (d, J = 8,5 Hz, 1H) ; 10,6 (m étalé, 2H) ; 11,9 (m large, 1H) ; 13,6 (m étalé, 1H).

### Exemple 248 : Synthèse du {{4-{5-[(3-diméthylamino-propyl)aminocarbonyl]-1H-benzimidazol-2-yl}-9H-fluorène-9(R,S)-yl}}-amide de l'acide 1H-pyrrolo[2,3-b]pyridine-4-carboxylique

Etape1 : On opère comme dans l'exemple 233, mais à partir de 400 mg de [4-(5-carboxy-1H-benzimidazol-2-yl)-9H-fluorène-9-one, obtenue à l'étape 1 de l'exemple 241 220 µL de N,N-diméthyl-1,3-propane diamine, 225 mg de chlorhydrate de 1-(3-diméthylaminopropyl)-3-éthylcarbodiimide (EDCI) et 79 mg de 1-hydroxybenzotriazole (HOBT) dans 4 mL de diméthylformamide, pendant 20 heures à température ambiante. Après purification par flash-chromatographie sur gel de silice (40-63µm), en éluant avec un mélange de dichlorométhane et d'ammoniac en solution 7N dans le méthanol (95/5 puis 9/1 en volumes), puis empâtage à l'éther diisopropylique, on obtient ainsi 273 mg de 4-{5-[(3-diméthylamino-propyl)aminocarbonyl]-1H-benzimidazol-2-yl]-9H-fluorène-9-one, sous forme d'un solide jaune utilisé tel quel à l'étape suivante dont les caractéristiques sont les suivantes:
Spectre de masse (LC/MS/ES+/-) : m/z = 424 (M+)

Etape 2 : On opère comme dans l'exemple 5, à partir de 273 mg de 4-[5-(3-diméthylamino-propyl-aminocarbonyl)-1H-benzimidazol-2-yl]-9H-fluorène-9-one, obtenue à l'étape précédente, de 134 mg de chlorhydrate d'hydroxylamine et de 264 mg d'acétate de sodium dans 10 mL d'éthanol, agités pendant 20h à température ambiante. Après concentration du solvant sous pression réduite, le résidu est versé sur de l'eau contenant quelques mL d'ammoniac en solution 7N dans le méthanol puis extrait par de l'acétate d'éthyle. La phase organique est séchée sur du sulfate de magnésium et amenée à sec sous pression réduite. Le produit brut est empâté à l'éther diisopropylique, filtré et rincé. On obtient ainsi 229 mg de 4-{5-[(3-diméthylamino-propyl) aminocarbonyl]-1H-benzimidazol-2-yl}-9H-fluorène-9-one oxime (Z,E), sous forme d'un solide jaune pâle utilisé tel quel à l'étape suivante dont les caractéristiques sont les suivantes:
Spectre de masse (LC/MS/ES/+/-) : m/z = 439 (M+).

Etape 3 : On opère comme dans l'exemple 216. Dans un ballon de 10 mL sous atmosphère d'argon, on dissout 229 mg de 4-{5-[(3-diméthylamino-propyl)aminocarbonyl]-1H-benzimidazol-2-yl}-9H-fluorène-9-one oxime (Z,E), obtenue à l'étape précédente, dans un mélange de 1,2 mL d'éthanol, 1,2 mL de d'eau et 1,2 mL d'acide acétique. A température ambiante, on ajoute en trois fois 136 mg de zinc. Entre chaque ajout, on agite environ une heure à deux heures. On ajoute de la Célite et on filtre. Le filtrat est concentré sous pression. Le produit brut est purifié par flash-chromatographie sur gel de silice (40-63µm) en éluant avec un mélange de dichlorométhane et d'ammoniac en solution 7N dans le méthanol (9/1 en volumes). Après empâtage à l'éther diisopropylique, on obtient ainsi 150 mg de 4-{5-[(3-diméthylamino-propyl)aminocarbonyl]-1H-benzimidazol-2-yl}-9H-fluorène-9(R,S)-amine sous forme d'une poudre blanc cassé, utilisée telle quelle à l'étape suivante, dont les caractéristiques sont les suivantes :
Spectre de masse (EI/CI) : m/z = 425 (M+)

Etape 4 : On opère comme dans l'exemple 14, mais à partir de 150 mg de 4-{5-[(3-diméthylamino)propyl-aminocarbonyl]-1H-benzimidazol-2-yl}-9H-fluorène-9(R,S)-amine, obtenue à l'étape précédente, 57 mg d'acide 1H-pyrrolo-[2,3-b]pyridine-4-carboxylique, 68 mg de chlorhydrate de 1-(3-diméthylaminopropyl)-3-éthylcarbodiimide (EDCI) et 24 mg de 1-hydroxybenzotriazole (HOBT) dans 2,5 mL de diméthylformamide, pendant 20 heures à température ambiante. Après purification par flash-chromatographie sur gel de silice (40-63µm) en éluant avec un mélange de dichlorométhane et d'ammoniac en solution 7N dans le méthanol (9/1 puis 85/15 en volumes), puis empâtage à l'éther diisopropylique, on obtient ainsi 32 mg de {{4-{5-[(3-diméthylamino-propyl)aminocarbonyl]-1H-benzimidazol-2-yl}-9H-fluorène-9(R,S)-yl}}-amide de l'acide 1H-pyrrolo[2,3-b]pyridine-4-carboxylique, sous forme d'une poudre blanc cassé dont les caractéristiques sont les suivantes :
Spectre de masse (LC/MS/ES+/-) : m/z = 569 (M+)
Spectre RMN 1H (400 MHz, □en ppm, DMSO-d6): 1,70 (m, 2H) ; 2,16 (s, 6H) ; 2,30 (t, J = 7,0 Hz, 2H) ; 3,32 (m partiellement masqué, 2H) ; 6,41 (d, J = 8,5 Hz, 1H) ; 6,91 (dd, J = 2,0 et 3,5 Hz, 1H) ; 7,25 (t, J = 7,5 Hz, 1H) ; 7,35 (t, J = 7,5 Hz, 1H) ; de 7,42 à 7,88 (m masqué, 1H) ; 7,47 (d, J = 5,0 Hz, 1H) ; 7,53 (t, J = 7,5 Hz, 1H) ; 7,62 (m, 2H) ; 7,69 (d, J = 7,5 Hz, 1H) ; 7,77 (d, J = 7,5 Hz, 1H) ; 7,81 (m étalé, 1H) ; de 8,05 à 8,35 (m étalé, 1H) ; 8,30 (d, J = 5,0 Hz, 1H) ; 8,55 (m étalé, 1H) ; 9,28 (d, J = 8,5 Hz, 1H) ; 11,85 (m large, 1H) ; 13,2 (m étalé, 1H).

### Exemple 249 : Synthèse du {4-[5-fluoro-6-(4-méthyl-pipérazin-1-yl)-1H-benzimidazol-2-yl]-9H-fluorèn-9(R,S)-yl}-amide de l'acide 1H-pyrrolo[2,3-b]pyridine-4-carboxylique

Etape 1 : On opère comme à l'étape 1 de l'exemple 1, mais à partir de 2,69 g de 4-fluoro-5-(4-méthyl-pipérazin-1-yl)-benzène-1,2-diamine, qui peut être préparé selon J. Het. Chem. 2005, 42(1), 67-71, 2,82 mL de triéthylamine et 2,43 g de chlorure de l'acide 9H-fluorène-9-one-4-carboxylique dans 50 mL de dichlorométhane pendant 20 heures à température ambiante. Après purification par flash-chromatographie sur gel de silice (40-63µm), en éluant avec un mélange de dichlorométhane et de méthanol (95/5 en volumes), puis empâtage à l'éther diisopropylique, on obtient ainsi 1 g de [2-amino-4-fluoro-5-(4-méthyl-pipérazin-1-yl)-phényl]-amide de l'acide 9-oxo-9H-fluorène-4-carboxylique sous forme d'une poudre orange utilisée telle quelle à l'étape suivante, dont les caractéristiques sont les suivantes:
Spectre de masse (LC/MS/ESl+/-): m/z = 430 (M+)

On obtient également 1 g de [2-amino-5-fluoro-4-(4-méthyl-pipérazin-1-yl)-phenyl]-amide de l'acide 9-oxo-9H-fluorène-4-carboxylique, sous forme d'une poudre orange utilisée telle quelle à l'étape suivante, dont les caractéristiques sont les suivantes :
Spectre de masse (LC/MS/ES/+/-): m/z = 430 (M+)

Etape 2 : On opère comme dans l'exemple 68. Dans un ballon de 250 mL sous atmosphère d'argon, on chauffe à reflux pendant 6 heures, une suspension de 1 g du [2-amino-5-fluoro-4-(4-méthyl-pipérazin-1-yl)-phényl]-amide de l'acide 9-oxo-9H-fluorène-4-carboxylique et de 1 g du [2-amino-4-fluoro-5-(4-méthyl-pipérazin-1-yl)-phényl]-amide de l'acide 9-oxo-9H-fluorène-4-carboxylique, obtenus à l'étape précédente, dans 30 mL d'acide acétique. Après purification par empâtage à l'éther diisopropylique, on obtient ainsi 1,9 g de 4-[5-fluoro-6-(4-méthyl-pipérazin-1-yl)-1H-benzimidazol-2-yl]-9H-fluorène-9-one, sous forme d'une poudre orange utilisée telle quelle à l'étape suivante, dont les caractéristiques sont les suivantes:
Spectre de masse (LCMS) : m/z = 412 (M+)

étape 3 : On opère comme à l'exempte 5, mais à partir de 1,9 g de 4-[5-fluoro-6-(4-méthyl-pipérazin-1-yl)-1H-benzimidazol-2-yl]-9H-fluorène-9-one, obtenue à l'étape précédente, de 960 mg de chlorhydrate d'hydroxylamine et de 1,89 g d'acétate de sodium dans 95 mL d'éthanol agités pendant 20h à température ambiante. Après purification par empâtage à l'éther diisopropylique, on obtient ainsi 1,95 g de 4-[5-fluoro-6-(4-méthyl-pipérazin-1-yl)-1H-benzimidazol-2-yl]-9H-fluorène-9-one oxime (Z,E) en mélange 50-50 des isomères Z et E, sous forme d'une poudre orange utilisée telle quelle à l'étape suivante.
Spectre de masse (LCMS) : m/z = 427 (M+)

Etape 4 : On opère comme à l'exemple 6, à partir de 1,95 g de 4-[5-fluoro-6-(4-méthyl-pipérazin-1-yl)-1H-benzimidazol-2-yl]-9H-fluorène-9-one oxime (Z,E), obtenue à l'étape précédente, et de 285mg de Nickel de Raney dans 40 mL d'éthanol et 40 mL de tétrahydrofurane pendant 16 heures à 60°C sous une pression initiale d'un bar d'hydrogène. Après filtration du catalyseur, concentration à sec sous pression réduite et purification par empâtage à l'éther diisopropylique, on obtient ainsi 1,7 g de 4-[5-fluoro-6-(4-méthyl-pipérazin-1-yl)-1H-benzimidazol-2-yl]-9H-fluorène-9(R,S)-yl-amine, sous forme d'une poudre orange utilisée telle quelle à l'étape suivante, dont les caractéristiques sont les suivantes :
Spectre de masse (LCMS) : m/z = 413 (M+)

Ce composé contient environ 50% d'un analogue N-acétylé inséparable à cette étape.

Etape 5: On opère comme dans l'exemple 14, mais à partir de 689 mg de 4-[5-fluoro-6-(4-méthyl-pipérazin-1-yl)-1H-benzimidazol-2-yl]-9H-fluorène-9(R,S)-yl-amine, obtenue à l'étape précédente en mélange à 50% avec un analogue N-acétylé, 162 mg d'acide 1H-pyrrolo-[2,3-b]pyridine-4-carboxylique, 211 mg de chlorhydrate de 1-(3-diméthylaminopropyl)-3-éthylcarbodiimide (EDCI) et 148 mg de 1-hydroxybenzotriazole (HOBT) dans 7 mL de diméthylformamide, pendant 20 heures à température ambiante. Après purification par flash-chromatographie sur gel de silice (40-63µm), en éluant avec un mélange de dichlorométhane et de méthanol ammoniacal 7N (97,5/2_{;}5 en volumes), puis empâtage à l'éther diisopropylique, on obtient ainsi 175 mg de {4-[5-fluoro-6-(4-méthyl-pipérazin-1-yl)-1H-benzimidazol-2-yl]-9H-fluorèn-9(R,S)-yl}-amide de l'acide 1H-pyrrolo[2,3-b]pyridine-4-carboxylique, sous forme d'une poudre beige dont les caractéristiques sont les suivantes :
Spectre de masse (LC/MS/ES+/-) : m/z = 557 (M+)
Spectre RMN 1H (400 MHz, □ en ppm, DMSO-d6) : 2,26 (s, 3H); 2,54 (m partiellement masqué, 4H) ; 3,06 (m, 4H) ; 6,40 (d, J = 8,5 Hz, 1H) ; 6,91 (d, J = 3;5 Hz, 1H) ; de 7,10 à 7,80 (m masqué, 2H) ; 7,26 (t, J = 7,5 Hz, 1H) ; 7,34 (t, J = 7,5 Hz, 1H) ; 7,46 (d, J = 5,0 Hz, 1H) ; 7,50 (t, J = 7,5 Hz, 1H) ; de 7,59 à 7,66 (m, 4H) ; 7,73 (d, J = 7,5 Hz, 1H) ; 8,29 (d, J = 5,0 Hz, 1H) ; 9,26 (d, J = 8,5 Hz, 1H) ; 11,9 (m large, 1H) ; 12,85 (m étalé, 1H).

### Exemple 250: Synthèse du N-{4-[5-Fluoro-6-(4-methyl-piperazin-1-yl)-1H-benzimidazol-2-yl]-9H-fluorèn-9(R,S)-yl}-acétamide

Lors de la purification par flash-chromatographie du produit de l'étape 5 de l'exemple 249, on isole un produit secondaire. Après empâtage à l'oxyde diisopropyle, on obtient ainsi 135 mg N-{4-[5-fluoro-6-(4-méthyl-pipérazin-1-yl)-1H-benzimidazol-2-yl]-9H-fluorèn-9(R,S)-yl}-acétamide, sous forme d'une poudre beige dont les caractéristiques sont les suivantes:
Spectre de masse (LC/MS/ES+/-) : m/z = 455 (M+)

### Exemple 251 : Synthèse du {{4-{5-[(3-diméthylamino-propyl)carbonyloxy]-1H-benzimidazol-2-yl}-9H-fluorène-9(R,S)-yl}}-amide de l'acide 1H-pyrrolo[2,3-b]pyridine-4-carboxylique

Etape1 : On opère comme dans l'exemple 233, mais à partir de 500 mg de [4-(5-carboxy-1H-benzimidazol-2-yl)-9H-fluorène-9-one, obtenue à l'étape 1 de l'exemple 241, 261 µL de 3-diméthylaminopropanol, 280 mg de chlorhydrate de 1-(3-diméthylaminopropyl)-3-éthylcarbodiimide (EDCI), 50 mg de N,N diméthylaminopyridine (DMAP) dans un mélange de 4 mL de dichlorométhane, 2 mL de tétrahydrofurane et 1 mL de diméthylformamide, pendant 20 heures à température ambiante. Après purification par flash-chromatographie sur gel de silice (40-63µm), en éluant avec un mélange de dichlorométhane et de méthanol (9/1 en volumes) puis de dichlorométhane et d'ammoniac en solution 7N dans le méthanol ( 9/1 en volumes), on obtient ainsi 582 mg de 4-{5-[(3-diméthylamino-propyl)carbonyloxy]-1H-benzimidazol-2-yl}-9H-fluorène-9-one, sous forme d'une résine jaune utilisée telle quelle à l'étape suivante dont les caractéristiques sont les suivantes:
Spectre de masse (LC/MSIES+/-) : m/z = 425 (M+)

Etape 2 : On opère comme dans l'exemple 5, mais à partir de 582 mg de 4-{5-[(3-diméthylamino-propyl)carbonyloxy]-1H-benzimidazol-2-yl}-9H-fluorène-9-one, obtenue à l'étape précédente, de 283 mg de chlorhydrate d'hydroxylamine et de 561 mg d'acétate de sodium dans 30 mL d'éthanol agités pendant 20h à température ambiante. Après empâtage à l'éther diisopropylique, on obtient ainsi 468 mg de 4-{5-[(3-diméthylamino-propyl)carbonyloxy]-1H-benzimidazol-2-yl}-9H-fluorène-9-one oxime (Z,E), sous forme d'un solide jaune pâle utilisé tel quel à l'étape suivante dont les caractéristiques sont les suivantes :
Spectre de masse (LC/MS/ESI+/-) : m/z = 440 (M+).

Etape 3 : On opère comme dans l'exemple 216. Dans un ballon de 20 mL sous atmosphère d'argon, on dissout 468 mg de de 4-{5-[(3-diméthylamino-propyl)carbonyloxy]-1H-benzimidazol-2-yl}-9H-fluorène-9-one oxime (Z,E), obtenue à l'étape précédente, dans un mélange de 2,5 mL d'éthanol, 2,5 mL de d'eau et 2,5 mL d'acide acétique, à température ambiante. On ajoute en trois fois 278 mg de zinc. Entre chaque ajout, on agite environ une heure à deux heures. On ajoute de la Célite 545 et on filtre. Le filtrat est concentré sous pression. Après purification par flash-chromatographie sur gel de silice (40-63µm), en éluant avec un mélange de dichlorométhane et d'ammoniac en solution 7N dans le méthanol (9/1 en volumes), puis empâtage à l'éther diisopropylique, on obtient ainsi 327 mg de 4-{5-[(3-diméthylamino-propyl)carbonyloxy]-1H-benzimidazol-2-yl}-9H-fluorène-9(R,S)-amine, sous forme d'un solide blanc cassé, utilisé tel quel à l'étape suivante, dont les caractéristiques sont les suivantes :
Spectre de masse (LC/MS/ES+/-) : m/z = 426 (M+)

Etape 4: On opère comme dans l'exemple 14, mais à partir de 327 mg de 4-{5-[(3-diméthylamino-propyl)carbonyloxy]-1H-benzimidazol-2-yl}-9H-fluorène-9(R,S)-amine, obtenue à l'étape précédente, 124 mg d'acide 1H-pyrrolo-[2,3-b]pyridine-4-carboxylique, 147 mg de chlorhydrate de 1-(3-diméthylaminopropyl)-3-éthylcarbodiimide (EDCI) et 52 mg de 1-hydroxybenzotriazole (HOBT) dans 3,5 mL de diméthylformamide, pendant 20 heures à température ambiante. Après purification par flash-chromatographie sur gel de silice (40-63µm), en éluant avec un mélange de dichlorométhane et d'ammoniac en solution 7N dans le méthanol (95/5 puis 9/1 en volumes), puis empâtage à l'éther diisopropylique, on obtient ainsi 258 mg de {{4-{5-[(3-diméthytamino-propyl)carbonyloxy]-1H-benzimidazol-2-yl}-9H-fluorène-9(R,S)-yl}}-amide de l'acide 1H-pyrrolo[2,3-b]pyridine-4-carboxylique, sous forme d'un solide blanc cassé dont les caractéristiques sont les suivantes :
Spectre de masse (LC/MS/ES+/-) : m/z = 570 (M+)
Spectre RMN 1H (400 MHz, □en ppm, DMSO-d6) : 1,90 (m, 2H) ; 2,17 (s, 6H) ; 2,39 (t, J = 6,5 Hz, 2H) ; 4,35 (t, J = 6,5 Hz, 2H) ; 6,42 (d, J = 8,5 Hz, 1H) ; 6,91 (m large, 1H) ; 7,26 (t, J = 7,5 Hz, 1H) ; 7,35 (t, J = 7,5 Hz, 1H) ; 7,47 (d, J = 5,0 Hz, 1H) ; 7,53 (t, J = 7,5 Hz, 1H) ; de 7,57 à 7,64 (m, 3H) ; 7,70 (d, J = 7,5 Hz, 1H) ; 7,75 (m étalé, 1H) ; 7,78 (d, J = 7,5 Hz, 1H) ; 7,93 (d, J = 8,5 Hz, 1H) ; 8,28 (m étalé, 1H) ; 8,30 (d, J = 5,0 Hz, 1H) ; 9,28 (d, J = 8,5 Hz, 1H) ; 11,85 (m large, 1H) ; 13,3 (m étalé, 1H).

### Exemple 252 : Synthèse du [4-(6-nitro-1H-benzimidazol-2-yl)-9H-fluorèn-9(R,S)-yl]-amide de l'acide 1H-pyrrolo[2,3-b]pyridine-4-carboxylique.

Dans un monocol de 20 mL, on introduit successivement, sous atmosphère d'argon, 23 mg de 4-nitro-benzène-1,2-diamine, 53 mg de (4-formyl-9H-fluorèn-9-yl)-amide de l'acide 1H-pyrrolo[2,3-b]pyridine-4-carboxylique, préparé comme à l'étape 6 de l'exemple 234, 24 mg de chlorure ferrique et 2 mL de diméthylformamide. Après 16h d'agitation à 20°C, on concentre la solution sous pression réduite, puis on reprend le résidu obtenu dans 5 mL d'eau. Après essorage de l'insoluble formé sur plaque poreuse, lavage par 4 fois 2 mL d'eau et séchage à 40°C sous pression réduite, on obtient 72 mg de [4-(6-nitro-1H-benzimidazol-2-yl)-9H-fluorèn-9(R,S)-yl]-amide de l'acide 1H-pyrrolo[2,3-b]pyridine-4-carboxylique, sous forme de poudre violette dont les caractéristiques sont les suivantes :
Spectre de masse (E/I) : m/z = 486 (M+)
Spectre RMN 1H (300 MHz, □ en ppm, DMSO) : Pour ce composé, on observe un mélange 70 % - 30 % de rotamères avec : 6,41 (d, J = 8,5 Hz, 1H) ; 6,91 (s large, 1H) ; de 7,00 à 8,00 (m étalé masqué, 1H) ; 7,27 (t, J = 7,5 Hz, 1H) ; 7,37 (t, J = 7,5 Hz, 1H) ; 7,47 (d, J = 5,0 Hz, 1H) ; 7,56 (t, J = 7,5 Hz, 1H) ; 7,58 (d large, J = 8,5 Hz, 1H) ; 7,63 (m, 2H) ; 7,72 (d, J = 7,5 Hz, 1H) ; 7,81 (d, J = 7,5 Hz, 1H) ; 8,22 (m large, 1H) ; 8,30 (d, J = 5,0 Hz, 1H) ; 8,45 (m large, 0,3H) ; 8,68 (m large, 0,7H) ; 9,29 (d, J = 8,5 Hz, 1H) ; 11,86 (m large, 1H) ; 13,7 (m étalé, 1H).

### Exemple 253 : Synthèse du 4-(6-méthyl-1H-benzimidazol-2-yl)-9H-fluorène-9(R,S)-yl]-amide de l'acide 1H-pyrrolo[2,3-b]pyridine-4-carboxylique

Etape 1 : On opère comme à l'étape 1 de l'exemple 1, mais à partir de 5 g de 1,2-diamino-4-méthylbenzène, 12 mL de triéthylamine et 10 g de chlorure de l'acide fluorène-4-one-9-carboxylique dans 300 mL de dichlorométhane. Après 20 heures d'agitation à température ambiante, le mélange réactionnel est lavé par de l'eau, séché sur sulfate de magnésium, et concentré à sec sous pression réduite. Le résidu est purifié par flash-chromatographie sur gel de silice (40-63µm) en éluant avec un mélange de dichlorométhane et de méthanol (95/5 en volumes). On obtient ainsi 11,5 g du (2-amino-4-méthyl-phényl)amide de l'acide 9-oxo-9H-fluorène-4-carboxylique sous forme d'un solide orange utilisé tel quel à l'étape suivante, dont la caractéristique est la suivante :
Spectre de masse (El) : m/z = 328 (M+)

Etape 2 : On opère comme dans l'exemple 68. Dans un ballon de 500 mL sous atmosphère d'argon, on chauffe à 110°C pendant 6 heures, une solution de 11,5 g de (2-amino-4-méthyl-phényl) amide de l'acide 9-oxo-9H-fluorène-4-carboxylique, obtenu à l'étape précédente, dans 300 mL d'acide acétique. Après refroidissement, on amène à sec sous pression réduite. Le solide brut obtenu est purifié par flash-chromatographie sur gel de silice (40-63µm) en éluant avec un mélange de dichlorométhane et de méthanol (99/1 en volumes). On obtient ainsi 5,3 g de 4-(6-méthyl-1H-benzimidazol-2yl)-9H-fluorène-9-one, sous forme d'une poudre jaune utilisée telle quelle à l'étape suivante, dont les caractéristiques sont les suivantes:
Spectre de masse (El): m/z = 310 (M+)

Etape 3 : On opère comme à l'exemple 5, mais à partir de 1 g de 4-(6-méthyl-1H-benzimidazol-2-yl)-9H-fluorène-9-one, obtenu à l'étape précédente, de 672 mg de chlorhydrate d'hydroxylamine et de 1,3 g d'acétate de sodium dans 45 mL d'éthanol agités pendant 20h à température ambiante. Après concentration du solvant sous pression réduite, le résidu est repris successivement par de l'eau, filtré et rincé à l'éther isopropylique. On obtient ainsi 1g (95%) de 4-(6-méthyl-1H-benzimidazol-2-yl)-9H-fluorène-9-one oxime (Z,E) en mélange 50-50 des isomères Z et E, sous forme d'un solide beige utilisé tel quel à l'étape suivante, dont les caractéristiques sont les suivantes :
Spectre de masse (EI) : m/z = 325 (M+).

Etape 4 : On opère comme dans l'exemple 6. Dans un autoclave de 100 mL, on dissout 1 g de 4-(6-méthyl-1H-benzimidazol-2-yl)-9H-fluorène-9-one oxime (Z,E) obtenu à l'étape précédente, dans un mélange de 40 mL d'éthanol et 40 mL de tétrahydrofuranne, on ajoute une spatule de Nickel activé selon Raney puis on soumet à une pression initiale d'hydrogène de 1 bar et on chauffe l'autoclave à 60° C pendant 3 heures. Après refroidissement, le volume d'hydrogène absorbé est de 142 mL. Après filtration du catalyseur sur Célite, le filtrat est concentré sous pression réduite puis repris dans de l'éther de pétrole et filtré. On obtient ainsi 720 mg de 4-(6-méthyl-1H-benzimidazol-2-yl)-9H-fluorène-9(R,S)-amine, sous forme d'un solide beige utilisé tel quel à l'étape suivante, dont la caractéristique est la suivante :
Spectre de masse (LC/MS) : m/z = 311 (M+)

Etape 5 : On opère comme dans l'exemple 14, mais à partir de 370 mg de 4-(6-méthyl-1H-benzimidazol-2-yl)-9H-fluoréne-(R,S)-amine, obtenue à l'étape précédente, 162 mg d'acide 1H-pyrrolo-[2,3-b]pyridine-4-carboxylique, 210 mg de chlorhydrate de 1-(3-diméthylaminopropyl)-3-éthylcarbodiimide (EDCI) et 148 mg de 1-hydroxybenzotriazole (HOBT) dans 7 mL de diméthylformamide, pendant 20 heures à température ambiante. On amène à sec le diméthylformamide, on ajoute de l'eau et on essore le précipité formé qui est ensuite lavé à l'eau puis avec une solution saturée d'hydrogénocarbonate de sodium à 10% et enfin à l'eau. Le solide brut obtenu est séché puis purifié par flash-chromatographie sur gel de silice (40-63µm) en éluant avec un mélange de dichlorométhane et de méthanol (95/5 en volumes). On obtient ainsi 290 mg de 4-(6-méthyl-1H-benzimidazol-2-yl)-9H-fluorène-9(R,S)-yl]-amide de l'acide 1H-pyrrolo[2,3-b]pyridine-4-carboxylique, sous forme d'un solide beige dont les caractéristiques sont les suivantes :
Spectre de masse (LC/MS) : m/z = 455 (M+)
Spectre RMN 1H (400 MHz, □ en ppm, DMSO-d6) : Pour ce composé, on observe un mélange 50 % - 50 % de rotamères avec : De 2,47 à 2,51 (s masqué, 3H) ; 6,41 (d, J = 8,5 Hz, 1H) ; 6,91 (dd, J = 1,5 et 3,5 Hz, 1H) ; 7,10 (m, 1H) ; 7,24 (t, J = 7,5 Hz, 1H) ; de 7,30 à 7,37 (m, 2H) ; de 7,43 à 7,67 (m, 7H) ; 7,74 (d, J = 7,5 Hz, 1H) ; 8,29 (d, J = 5,0 Hz, 1H) ; 9,26 (d, J = 8,5 Hz, 1H); 11,85 (m large, 1H) ; 12,7 (m large, 0,5H) ; 12,8 (m large, 0,5H).

### Exemple 254 : Synthèse de la 5-(1H-benzimidazol-2-yl)-2-bromo-9H-fluorèn-9-one :

Etape 1 : Dans un ballon tricol de 100 mL, équipé d'une agitation mécanique, on agite 4.48 g d'acide 9H-fluorèn-9-one-4-carboxylique en suspension dans 40 mL d'acide trifluoroacétique et de 12 mL d'acide sulfurique concentré. À ce mélange réactionnel sombre, on ajoute 5.33 g de N-bromosuccinimide sur une période de 9 h. Le précipité jaune formé est filtré sur fritté, lavé par 3 fois 20 mL d'acide trifluoroacétique et 6 fois 50 mL d'eau puis séché à l'étuve 50°C sous vide poussé. On obtient 6.6 g d'une poudre jaune, composée d'un mélange 55/45 d'acide 2-bromo-9-oxo-9H-fluorène-4-carboxylique et d'acide 2,7-dibromo-9-oxo-9H-fluorène-carboxylique, utilisé tel quel à l'étape suivante.

Etape 2 : Dans un ballon de 250 mL on agite 3.80 g du mélange obtenu à l'étape 1 dans 100 mL de dichlorométhane additionnés de 1 mL de diméthylformamide. On ajoute 1.93 g de chlorure d'oxalyle et on agite le mélange réactionnel à température ambiante pendant 30 minutes. La solution jaune obtenue est ajoutée sous argon et goutte à goutte en 20 min, à une solution de 1.78 g d'orthophénylènediamine et de 3.12 g de triéthylamine dans 100 mL de dichlorométhane. Le précipité formé est filtré et lavé par 2 fois 50 mL de dicholorométhane et 50 mL d'eau pour conduire, après séchage à 50°C à l'étuve sous vide, à 4.90 g d'un mélange 60/40 de 2-aminobenzoylamide de l'acide 7-bromo-9-oxo-9H-fluorène-4-carboxylique et de 2-aminobenzoylamide de l'acide 2,7-dibromo-9-oxo-9H-fluorène-4-carboxylique sous la forme d'une poudre jaune, utilisé tel quel à l'étape suivante.

Etape 3 : Une suspension de 4.30 g du mélange obtenu à l'étape 2 dans 200 mL d'acide acétique est chauffée à reflux pendant 1 heure et 30 minutes. L'évaporation de l'acide acétique sous pression réduite conduit à un pâte jaune qui est reprise dans 250 mL d'eau. Le pH du surnageant est amené à 9 par addition d'une solution aqueuse 5 N d'hydroxyde de sodium. Le précipité jaune obtenu est filtré sur fritté, lavé à l'eau et séché à 50°C à l'étuve sous vide. Après recristallisation dans 100 mL de diméthylformamide, le produit étant isolé par filtration à une température de 80°C, on obtient ainsi 1.57 g de 5-(1H-benzimidazol-2-yl)-2-bromo-9H-fluorèn-9-one sous la forme de cristaux jaunes, dont les caractéristiques sont les suivantes.
Spectre de masse (El) : m/z = 375 (M+.)
Spectre de RMN (400 MHz, □ en ppm, DMSO-d6) : 7,30 (m, 2H) ; 7,61 (t, J = 7,5 Hz, 1H) ; 7,69 (m, 2H); 7,73 (dd, J = 2,0 et 8,0 Hz, 1H) ; 7,80 (d, J = 2,0 Hz, 1H) ; 7,81 (d large, J = 7,5 Hz, 1H) ; 7,90 (d, J = 8,0 Hz, 1H) ; 7,92 (d large, J = 7,5 Hz, 1H) ; 13,0 (m étalé, 1H).

### Exemple 255 : Synthèse du {{4-{5-[(3-hydroxy-propyl)aminocarbonyl]-1H-benzimidazole-2-yl}-9H-fluorène-9(R,S)-yl}}-amide de l'acide 1H-pyrrolo[2,3-b]pyridine-4-carboxylique

Dans un ballon de 30 mL sous argon, on dissout 300 mg de [4-(5-méthoxycarbonyl-1H-benzimidazole-2-yl)-9H-fluorène-9(R,S)-yl]-amide de l'acide 1H-pyrrolo[2,3-b]pyridine-4-carboxylique, obtenu dans l'exemple 205, dans 5 mL de 3-aminopropanol, puis on chauffe à 170°C pendant 2h30. Le milieu réactionnel est versé sur de l'eau contenant quelques mL d'ammoniac en solution 7N dans le méthanol de chlorure de sodium, on extrait par de l'acétate d'éthyle, la phase organique est ensuite lavée avec une solution saturée chlorure de sodium, séchée sur sulfate de magnésium et amenée à sec sous pression réduite. Le solide brut obtenu est purifié par flash-chromatographie sur gel de silice (40-63µm) en éluant avec un mélange de dichlorométhane et de méthanol (95/5 puis 9/1 en volumes). Après empâtage à l'éther diisopropylique, on obtient ainsi 38 mg de {{4-{5-[(3-hydroxy-propyl)aminocarbonyl]-1H-benzimidazole-2-yl}-9H-fluorène-9(R,S)-yl}}-amide de l'acide 1H-pyrrolo[2,3-b]pyridine-4-carboxylique, sous forme d'une poudre blanc cassé dont les caractéristiques sont les suivantes:
Point de fusion (Kofler) >260°C .
Spectre de masse (LC/MS/ES+/-) : m/z = 542 (M+)
Spectre RMN 1H (400 MHz, □ en ppm, DMSO-d6) : 1,73 (m, 2H); 3,38 (q, J = 6,5 Hz, 2H) ; 3,50 (m, 2H) ; 4,49 (t, J = 5,5 Hz, 1H) ; 6,41 (d, J = 8,5 Hz, 1H) ; 6,91 (dd, J = 1,5 et 3,5 Hz, 1H) ; 7,25 (t, J = 7,5 Hz, 1H) ; 7,35 (t, J = 7,5 Hz, 1H) ; 7,47 (d, J = 5,0 Hz, 1H) ; de 7,49 à 7,71 (m masqué, 1H) ; 7,53 (t, J = 7,5 Hz, 1H) ; 7,62 (m, 3H) ; 7,69 (d, J = 7,5 Hz, 1H) ; 7,78 (d, J = 7,5 Hz, 1H) ; 7,82 (m large, 1H) ; de 8,00 à 8,39 (m étalé, 1H) ; 8,30 (d, J = 5,0 Hz, 1H) ; 8,48 (t large, J = 6,5 Hz, 1H) ; 9,29 (d, J = 8,5 Hz, 1H) ; 11,85 (m large, 1H) ; 13,15 (m étalé, 1H).

### Exemple 256 : Synthèse du 4-[5-aminométhyl-1H-benzimidazol-2-yl]-9H-fluorène-9(R,S)-yl}-amide de l'acide 1H-pyrrolo[2,3-b]pyridine-4-carboxylique

Dans un autoclave de 67 mL, on dissout 425 mg de [4-(5-cyano-1H-benzimidazol-2-yl)-9H-fluorène-9(R,S)-yl]-amide de l'acide 1H-pyrrolo[2,3-b]pyridine-4-carboxylique, obtenu dans l'exemple 216, dans un mélange de 15 mL d'éthanol et 15 mL de tétrahydrofurane, on ajoute une spatule de Nickel activé selon Raney puis on soumet à une pression initiale d'hydrogène de 1 bar et on chauffe l'autoclave à 60° pendant 10 heures. Après refroidissement, le volume d'hydrogène absorbé est de 69 mL. Après filtration du catalyseur sur célite, le filtrat est concentré sous pression réduite, le solide brut obtenu est purifié par flash-chromatographie sur gel de silice (40-63µm) en éluant avec un mélange de dichlorométhane et d'ammoniac ensolution 7N dans le méthanol (95/5 puis 9/1 en volumes). Après à l'éther diisopropylique, on obtient ainsi 220 mg de 4-[5-aminométhyl-1H-benzimidazol-2-yl]-9H-fluorène-9(R,S)-yl}-amide de l'acide 1H-pyrrolo[2,3-b]pyridine-4-carboxylique sous forme d'un solide blanc, dont les caractéristiques sont les suivantes :
Point de fusion (Kofler) = >260°C.
spectre de masse (LC/MS/ES+/-) : m/z = 470 (M+)
Spectre RMN 1H (400 MHz, □ en ppm, DMSO-d6) : 3,93 (s large, 2H) ; 6,41 (d, J = 8,5 Hz, 1H) ; 6,91 (dd, J = 1,5 et 3,5 Hz, 1H) ; 7,25 (t, J = 7,5 Hz, 1H) ; 7,31 (m masqué, 1H); 7,35 (t, J = 7,5 Hz, 1H) ; de 7,42 à 7,57 (m masqué, 1H) ; 7,47 (d, J = 5,0 Hz, 1H); 7,52 (t, J = 7,5 Hz, 1H) ; de 7,58 à 7,78 (m, 4H) ; de 7,70 à 7,81 (m, 2H) ; 8,30 (d, J = 5,0 Hz, 1H) ; 9,28 (d, J = 8,5 Hz, 1H) ; 11,85 (m large, 1H) ; 12,85 (m large, 1H) .

### Exemples 257 et 258: Dédoublement des énantiomères du 4-[5-cyano-1H-benzimidazol-2-yl]-9H-fluorène-9(R,S)-yl}-amide de l'acide 1H-pyrrolo[2,3-b]pyridine-4-carboxylique.

En opérant comme à l'exemple 153, à partir de 422 mg 4-[5-cyano-1H-benzimidazol-2-yl]-9H-fluorène-9(R,S)-yl}-amide de l'acide 1H-pyrrolo[2,3-b]pyridine-4-carboxylique, obtenu comme à l'exemple 216, mais en utilisant une colonne chirale de type Chiralcel OJ20 µm, de longueur 25 cm et de diamètre 5 cm, en éluant avec une phase mobile supercritique composée de dioxyde de carbone, d'éthanol et d'acide trifluoroacétique (75/25/0,1) à un débit de 150 mL/mn et sous une pression de 124 bars, et en détectant les produits élués par spectroscopie UV à 254 nM, on obtient en récupérant et en concentrant sous pression réduite les premières fractions éluées 205 mg d'énantiomère dextrogyre du 4-[5-cyano-1H-benzimidazol-2-yl]-9H-fluorène-9(R,S)-yl}-amide de l'acide 1H-pyrrolo[2,3-b]pyridine-4-carboxylique, sous la forme d'un solide amorphe blanc, dont les caractéristiques sont les suivantes:
□D20 = + 157 +/- 2,3° (c = 0,468; DMSO).

En récupérant et concentrant sous pression réduite les secondes fractions éluées, on obtient 206 mg d'énantiomère lévogyre du 4-[5-cyano-1H-benzimidazol-2-yl]-9H-fluorène-9(R,S)-yl}-amide de l'acide 1H-pyrrolo[2,3-b]pyridine-4-carboxylique, contenant environ 1,2 % d'énantiomère dextrogyre, sous la forme d'un solide amorphe blanc, dont les caractéristiques sont les suivantes :
□D20 = - 156,7 +/- 2,2° (c = 0,56; DMSO).

### Exemple 259: Synthèse du [4-(3H-imidazo[4,5-c]pyridin-2-yl)-9H-fluorèn-9(R,S)-yl]-amide de l'acide 7H-pyrrolo[2,3-d]pyrimidine-4-carboxylique.

Etape 1 : Dans un tricol de 1 L, on introduit sous agitation 11,8 g de 4-chloro-7H-pyrrolo[2,3-d]pyrimidine et 137 mL de toluène, puis 16,11 g de chloure 4-méthyl-benzènesulfonyle, 5.2 g d'hydrogénosulfate de tétra-n-butyle-ammonium, puis on coule goutte à goutte 275 mL d'une solution aqueuse contenant 30,7 g d'hydroxyde de sodium. Après 30 minutes à température ambiante, on ajoute 200mL d'acétate d'éthyle et 200mL d'eau. La phase organique est décantée, séchée sur sulfate de magnésium, concentrée sous pression réduite. Après purification par flash-chromatographie sur gel de silice, en éluant au dichlorméthane, on obtient 22,6 g de 4-chloro-7-(4-méthyl-benzènesulfonyl)-7H-pyrrolo[2,3-d]pyrimidine, sous forme d'un solide blanc dont les caractéristiques sont les suivantes:
Spectre de masse (E/I) : m/z = 307 (M+)

Etape 2 : Dans un autoclave, on introduit successivement 1 g de 4-chloro-7-(4-méthylbenzène-sulfonyl)-7H-pyrrolo[2,3-d]pyrimidine, obtenu à l'étape précédente, 0,18 g 1,1'-bis(diphénylphosphino)ferrocène, 0,073 g d'acétate de palladium(II), 0,53 g d'acétate de sodium et 20 mL d'éthanol. On agite pendant 24 heures à 100°C sous une pression de 20 bars de monoxyde de carbone. Après évaporation du solvant et flash-chromatographie sur gel de silice, en éluant avec un mélange d'acétate d'éthyle et de dichlorométhane (95/5 en volumes), on obtient 0,78 g d'ester éthylique de l'acide 7-(4-méthyl-benzènesulfonyl)-7H-pyrrolo[2,3-d]pyrimidine-4-carboxylique, sous forme d'un solide blanc dont les caractéristiques sont les suivantes :
Spectre de masse (E/I) : m/z = 345 (M+)

Etape 3 : On dissout 0,16 g d'ester éthylique de l'acide 7-(4-méthyl-benzènesulfonyl)-7H-pyrrolo[2,3-d]pyrimidine-4-carboxylique, obtenu à l'étape précédente, dans 10 mL de tétrahydrofurane ; puis on coule, à 20°C en 20 minutes, 1,38 mL d'une solution molaire dans le tétrahydrofurane de fluorure de tétra-n-butylammonium. Après 2 h à 20°C, on reprend le milieu réactionnel par 10 mL d'eau et 20 mL d'acétate d'éthyle. Après décantation, lavage à l'eau de la phase organique, séchage sur sulfate de magnésium, et concentration à sec sous pression réduite, on obtient 70 mg d'ester éthylique de l'acide 7H-pyrrolo[2,3-d]pyrimidine-4-carboxylique, sous forme d'un solide blanchâtre dont les caractéristiques sont les suivantes:
Spectre de masse (E/I) : m/z = 191 (M+)
Spectre de RMN (400 MHz, □ en ppm, DMSO-d6) : 1,40 (t, J = 7,5 Hz, 3H) ; 4,44 (q, J = 7,5 Hz, 2H) ; 6,89 (d, J = 3,5 Hz, 1H) ; 7,80 (d, J = 3,5 Hz, 1H) ; 8,90 (s, 1H) ; 12,5 (m étalé, 1H).

Etape 4 : Dans un tricol de 100 mL, on dissout 100 mg de 4-(1H-imidazo[4,5-c]pyridin-2-yl)-9H-fluorèn-9(R,S)-ylamine, obtenue comme à l'exemple 6, et 64 mg de d'ester éthylique de l'acide 7H-pyrrolo[2,3-d]pyrimidine-4-carboxylique, obtenu à l'étape précédente, dans 10 mL de tétrahydrofurane ; puis on coule, goutte à goutte, 6 mL d'une solution 2,0 M de triméthylaluminium dans le toluène et on agite à 20°C pendant 5 heures. Le milieu réactionnel est repris avec 50 mL d'eau et 50 mL d'acétate d'éthyle. Après décantation, lavage à l'eau de la phase organique, séchage sur sulfate de magnésium, et concentration à 30°C sous pression réduite, on obtient 133 mg d'un résidu qui est remis en suspension dans 5 mL d'acétate d'éthyle. Le précipité est essoré, lavé 2 fois par 2 mL d'acétate d'éthyle et séché, on obtient ainsi 93 mg de [4-(3H-imidazo[4,5-c]pyridin-2-yl)-9H-fluorèn-9(R,S)-yl]-amide de l'acide 7H-pyrrolo[2,3-d]pyrimidine-4-carboxylique, sous forme d'une poudre grisâtre dont les caractéristiques sont les suivantes :
Point de fusion (Kofler) = 260°C
Spectre de masse (E/I) : m/z = 443 (M+)
Spectre RMN 1H (300 MHz, □ en ppm, DMSO) : 6,27 (d, J = 8,5 Hz, 1H) ; 7,10 (d, J = 3,5 Hz, 1H) ; 7,25 (t, J = 7,5 Hz, 1H) ; 7,31 (t large, J = 7,5 Hz, 1H) ; 7,50 (t, J = 7,5 Hz, 1H) ; 7,51 (m masqué, 1H) ; 7,56 (d, J = 7,5 Hz, 1H) ; 7,67 (m masqué, 1H) ; 7,68 (d, J = 7,5 Hz, 1H) ; 7,73 (d, J = 7,5 Hz, 1H) ; 7,78 (d, J = 3,5 Hz, 1H) ; 8,40 (d, J = 5,0 Hz, 1H) ; 8,83 (s, 1H) ; 9,04 (s, 1H) ; 9,51 (d, J = 8,5 Hz, 1H) ; 12,4 (m large, 1H) ; 13,4 (m étalé, 1H).

### Exemple 260 : Synthèse de l'ester méhylique de l'acide 2-{9-[(Z,E)-hydroxyimino]-9H-fluorèn-4-yl}-1H-imidazo[4,5-c]pyridine-6-carboxylique

Etape 1 : Dans un ballon de 500 mL sous atmosphère d'argon, on chauffe à reflux pendant 24 heures, une suspension de 3 g de monohydrate de l'acide 4-amino-5-nitro-pyridine-2-carboxylique dans 300 mL de méthanol en présence de 6 mL d'acide sulfurique concentré à 98%. Après refroidissement, on amène à sec sous pression réduite. On reprend le résidu dans 200 mLd'eau, on refroidit dans un bain de glace, on neutralise à pH = 8 avec une solution aqueuse saturée d'hydrogénocarbonate de sodium. Le précipité formé est filtré, lavé à l'eau, et rincé à l'éther diisopropylique. On obtient ainsi 2,5g de l'ester méthylique de l'acide 4-amino-5-nitro-pyridine-2-carboxylique, sous forme d'une poudre beige utilisée telle quelle à l'étape suivante, dont les caractéristiques sont les suivantes :
Spectre de masse (LC/MS/ES/+/-): m/z = 197 (M+)

Etape 2 : Dans un autoclave, on dissout 2,5g d'ester méthylique de l'acide 4-amino-5-nitro-pyridine-2-carboxylique, obtenu à l'étape précédente, dans 300 mL d'éthanol et on agite en présence de 179 mg de palladium à 10% sur charbon pendant 6 heures à 60°C sous une pression initiale d'un bar d'hydrogène. Après filtration du catalyseur, concentration du filtrat sous pression réduite et empâtage à l'éther diisopropylique, on obtient ainsi 2,1g de l'ester méthylique de l'acide 4,5-diamino-pyridine-2-carboxylique, sous forme d'une poudre beige utilisée telle quelle à l'étape suivante, dont les caractéristiques sont les suivantes :
Spectre de masse (LC/MS/ES/+/-): m/z = 167 (M+)

Etape 3: On opère comme à l'étape 1 de l'exemple 1, mais à partir de 2,1 g de l'ester méthylique de l'acide 4,5-diamino-pyridine-2-carboxylique, de 3,55 mL de triéthylamine et 3,05 g de chlorure de l'acide fluorène-9-one-4-carboxylique dans 50 mL de dichlorométhane pendant 20 heures à température ambiante. Après purification par flash-chromatographie sur gel de silice (40-63µm), en éluant avec un mélange de dichlorométhane et de méthanol (95/5 en volumes), puis empâtage à l'éther diisopropylique, on obtient ainsi 2,5g de l'ester méthylique de l'acide 5-amino-4-[(9-oxo-9H-fluorèn-4-yl)carboxamido]-pyridine-2-carboxylique, sous forme d'une poudre orange utilisée telle quelle à l'étape suivante, dont les caractéristiques sont les suivantes :
Spectre de masse (LC/MS/ES/+/-): m/z = 373 (M+)

Etape 4: On opère comme dans l'exemple 68. Dans un ballon de 250 mL sous atmosphère d'argon, on chauffe, à reflux pendant 30 heures, une suspension de 2,5g de l'ester méthylique de l'acide 5-amino-4-[(9-oxo-9H-fluorèn-4-yl)carboxamido]-pyridine-2-carboxylique, obtenu à l'étape précédente, dans 44 mL d'acide acétique. Après refroidissement, on amène à sec sous pression réduite. On reprend le résidu à l'eau, on neutralise à pH = 8 par addition d'une solution aqueuse saturée d'hydrogénocarbonate de sodium. Le précipité formé est filtré, lave à l'eau, on rince à l'éther diisopropylique. Le produit brut recristallisé dans un mélange de dichlorométhane et de méthanol (7/3 en volume). On obtient 1,5 g d'ester méthylique de l'acide 2-(9-oxo-9H-fluorèn-4-yl)-1H-imidazo[4,5-c]pyridine-6-carboxylique, sous forme d'une poudre jaune utilisée telle quelle à l'étape suivante, dont les caractéristiques sont les suivantes :
Spectre de masse (LCMS) : m/z = 355 (M+)

Etape 5 : On opère comme à l'exemple 5, mais à partir de 1,35 g d'ester méthylique de l'acide 2-(9-oxo-9H-fluorèn-4-yl)-1H-imidazo[4,5-c]pyridine-6-carboxylique, obtenu à l'étape précédente, de 792 mg de chlorhydrate d'hydroxylamine et de 1,55g d'acétate de sodium dans 80 mL d'éthanol agités pendant 20h à température ambiante. Après purification par flash-chromatographie sur gel de silice (40-63µm), en éluant avec un mélange de dichlorométhane et de méthanol (95/5 en volumes), puis empâtage à l'éther diisopropylique, on obtient ainsi 950 mg d'ester méthylique de l'acide 2-{9-[(Z,E)-hydroxyimino]-9H-fluorèn-4-yl}-1H-imidazo[4,5-c]pyridine-6-carboxylique, en mélange 50-50 des isomères Z et E, sous forme d'une poudre beige dont les caractéristiques ont les suivantes :
Spectre de masse (LCMS) : m/z = 370 (M+)
Spectre de RMN (400 MHz, □ en ppm, DMSO-d6) : Pour ce composé, on observe un mélange 50% - 50% d'isomères Z et E avec : 3,92 (s, 3H) ; 7,24 (m, 1H); de 7,31 à 7,36 (m, 1H) ; 7,40 (t, J = 7,5 Hz, 0,5H) ; 7,49 (d, J = 7,5 Hz, 0,5H) ; 7,54 (t, J = 7,5 Hz, 0,5H) ; 7,60 (t, J = 7,5 Hz, 0,5H) ; 7,72 (d, J = 7,5 Hz, 0,5 H) ; 7,76 (d, J = 7,5 Hz, 0,5H) ; 7,78 (d, J = 7,5 Hz, 0,5H) ; 7,96 (d, J = 7,5 Hz, 0,5H) ; 8,36 (s large, 1H) ; 8,44 (d, J = 7,5 Hz, 0,5H) ; 8,64 (d, J = 7,5 Hz, 0,5H) ; 9,11 (s large, 1H) ; 12,78 (s, 0,5H) ; 12,82 (s, 0,5H) ; 13,8 (m étalé, 1H) .

### Exemple 261 : [4-(6-amino-1H-benzimidazol-2-yl)-9H-fluorèn-9(R,S)-yl]-amide de l'acide 1 -pyrrolo[2,3-b]pyridine-4-carboxylique

Dans un autoclave d'hydrogénation, on introduit successivement 3 mg de palladium à 10% sur charbon, 20 mg de [4-(6-nitro-1H-benzimidazol-2-yl)-9H-fluorèn-9(R,S)-yl]-amide de l'acide 1H-pyrrolo[2,3-b]pyridine-4-carboxylique, obtenu comme à l'exemple 252, 10mL d'alcool éthylique. On agite pendant 18 heures à 15°C sous une pression initiale de 1 bar d'hydrogène. Après filtration du catalyseur, puis concentration à sec sous pression réduite, on obtient, après séchage à l'étuve à 40°C, 15 mg de [4-(6-amino-1H-benzimidazol-2-yl)-9H-fluorèn-9(R,S)-yl]-amide de l'acide 1H-pyrrolo[2,3-b]pyridine-4-carboxylique, sous forme d'un solide beige dont les caractéristiques sont les suivantes :
Spectre de masse (E/I) : m/z = 456 (M+)
Spectre RMN 1H (300 MHz, □ en ppm, DMSO) : 6,27 (d, J = 8,5 Hz, 1H); 7,10 (d, J = 3,5 Hz, 1H) ; 7,25 (t, J = 7,5 Hz, 1H) ; 7,31 (t large, J = 7,5 Hz, 1H) ; 7,50 (t, J = 7,5 Hz, 1H) ; 7,51 (m masqué, 1H) ; 7,56 (d, J = 7,5 Hz, 1H) ; 7,67 (m masqué, 1H) ; 7,68 (d, J = 7,5 Hz, 1H) ; 7,73 (d, J = 7,5 Hz, 1H) ; 7,78 (d, J = 3,5 Hz, 1H) ; 8,40 (d, J = 5,0 Hz, 1H) ; 8,83 (s, 1H) ; 9,04 (s, 1H) ; 9,51 (d, J = 8,5 Hz, 1H) ; 12,4 (m large, 1H) ; 13,4 (m étalé, 1H).

### Exemple 262 : Synthèse du [5-(1H-benzimidazol-2-yl)-2-bromo-9H-fluorèn-9(R,S)-yl]-amide de l'acide 1H-pyrrolo[2,3-b]pyridine-4-carboxylique :

Etape 1 : On opère comme à l'exemple 5, mais à partir de 5-(1H-benzimidazol-2-yl)-2-bromo-9H-fluorèn-9-one, obtenue comme à l'exemple 254 , 285 mg de chlorhydrate d'hydroxylamine et 560 mg d'acétate de sodium, à reflux pendant 14 heures dans 30 mL d'éthanol. La poudre jaune, obtenue après évaporation du solvant sous pression réduite, est reprise dans 80 mL d'eau, filtrée, lavée par 2 fois 50 mL d'eau et séchée à 50°C à l'étuve sous vide, on obtient ainsi 530 mg de 5-(1H-benzimidazol-2-yl)-2-bromo-9H-fluorèn-9-one oxime, en mélange 50/50 des isomères Z et E, sous la forme d'une poudre blanc-cassé qui est utilisée telle quelle à l'étape suivante.

Etape 2: On met en suspension 500 mg de 5-(1H-benzimidazol-2-yl)-2-bromo-9H-fluorèn-9-one oxime(Z,E) dans un mélange de 3,5 mL d'eau, 7 mL d'éthanol et 3,5 mL d'acide acétique. On ajoute 335 mg de poudre de zinc et on agite à température ambiante pendant 19 heures. Les résidus insolubles sont filtrés sur fritté et rincés à l'éthanol. L'évaporation du solvant sous pression réduite conduit à une mousse incolore qui est reprise en suspension dans 100 mL d'eau. Le pH du surnageant est alors amené à 9 par addition d'une solution aqueuse 1N d'hydroxyde de sodium, et le précipité persistant est filtré, lavé par 3 fois 50 mL d'eau et 2 fois 100 mL d'éther isopropylique. Après séchage à 50 °C en l'étuve sous vide, on obtient ainsi 1,08 g de 5-(1H-benzimidazol-2-yl)-2-bromo-9H-fluorène-9(R,S)-amine, sous forme d'une poudre blanche utilisée telle quelle à l'étape suivante.

Etape 3 : On opère comme à l'exemple 14, mais à partir de 500 mg de 5-(1H-benzimidazole-2-yl)-2bromo-9H-fluorène-9(R,S)-amine, obtenue à l'étape précédente, 237 mg d'acide 1H-pyrrolo[2,3-b]pyridine-4-carboxylique, 280 mg de chlorhydrate de 1-(3-diméthylaminopropyl)-3-éthylcarbodiimide (EDCI) et 203 mg d'hydrate d'hydroxybenzotriazole (HOBT) dans 10 mL de diméthylformamide pendant 1h30. Après purification par flash-chromatographie sur colonne de silice, en éluant avec un gradient de mélange de dichlorométhane et de méthanol (de 100/0 à 90/10 en volumes), on obtient ainsi 169 mg de [5-(1H-benzimidazol-2-yl)-2-bromo-9H-fluorèn-9(R,S)-yl]-amide de l'acide 1H-pyrrolo[2,3-b]pyridine-4-carboxylique, sous la forme d'une poudre blanc cassé, dont les caractéristiques sont les suivantes : Spectre de masse (ESI) : m/z = 520 (MH+)
Spectre de RMN (400 MHz, □ en ppm, DMSO-d6) : 6,40 (d , J = 8,5 Hz, 1H) ; 6,91 (dd, J = 1,5 et 3,5 Hz, 1H) ; 7,29 (m large, 2H) ; 7,48 (d, J = 5,0 Hz, 1H) ; 7,49 (m masqué, 1H) ; 7,55 (t, J = 7,5 Hz, 1H) ; 7,60 (m étalé, 1H) ; 7,63 (t, J = 3,5 Hz, 1H) ; 7,71 (d, J = 7,5 Hz, 1H) ; de 7,72 à 7,80 (m, 4H) ; 8,30 (d, J = 5,0 Hz, 1H) ; 9,34 (d, J = 8,5 Hz, 1H) ; 11,9 (m large, 1H) ; 12,95 (m large, 1H).

### Exemples 263 et 264 : Dédoublement des énantiomères du 4-[5-fluoro-1H-benzimidazol-2-yl]-9H-fluorène-9(R,S)-yl}-amide de l'acide 1H-pyrrolo[2,3-b]pyridine-4-carboxylique.

En opérant comme à l'exemple 153, à partir de 2,65 g 4-[5-fluoro-1H-benzimidazol-2-yl]-9H-fluorène-9(R,S)-yl}-amide de l'acide 1H-pyrrolo[2,3-b]pyridine-4-carboxylique, obtenu comme à l'exemple 188, mais en utilisant une colonne chirale de type Chiralcel OJ20 µm, de longueur 35 cm et de diamètre 5 cm, en éluant avec une phase mobile supercritique composée de dioxyde de carbone et de méthanol (70/30) à un débit de 250 mUmn et sous une pression de 120 bars, en 16 injections successives et en détectant les produits élués par spectroscopie UV à 254 nM, on obtient en récupérant et en concentrant sous pression réduite les premières fractions éluées 1,15 g d'énantiomère dextrogyre du 4-[5-fluoro-1H-benzimidazol-2-yl]-9H-fluorène-9(R,S)-yl}-amide de l'acide 1H-pyrrolo[2,3-b]pyridine-4-carboxylique, sous la forme d'un solide amorphe blanc, dont les caractéristiques sont les suivantes :
□D20 = + 146,2 +/- 1,9 ° (c = 0,51; méthanol)

En récupérant et concentrant sous pression réduite les secondes fractions éluées, on obtient 1,01 g d'énantiomère lévogyre du 4-[5-fluoro-1H-benzimidazol-2-yl]-9H-fluorène-9(R,S)-yl}-amide de l'acide 1H-pyrrolo[2,3-b]pyridine-4-carboxylique, sous la forme d'un solide amorphe blanc, dont les caractéristiques sont les suivantes :
□D20 = - 159,3 +/- 2,1° (c = 0,48; methanol).

### Exemple 265 : Synthèse de l'ester méthylique de l'acide 2-{9(R,S)-[(1H-pyrrolo[2,3-b]pyridine-4-carbonyl)-amino]-9H-fluorèn-4-yl}-1H-imidazo[4,5-c]pyridine-6-carboxylique

Etape 1 : On opère comme à l'exemple 6, à partir de 800 mg de l'ester méthylique de l'acide 2-{9-[(Z,E)-hydroxyimino]-9H-fluorèn-4-yl}-1H-imidazo[4,5-c]pyridine-6-carboxylique, obtenu à l'exemple 260, et de 135 mg de Nickel de Raney dans 21 mL d'éthanol et 21 mL de tétrahydrofurane pendant 8 heures à 60°C sous une pression initiale d'un bar d'hydrogène. Après filtration du catalyseur, concentration du filtrat sous pression réduite et purification par empâtage dans l'éther diisopropylique, on obtient ainsi 700 mg de l'ester méthylique de l'acide 2-(9(R,S)-amino-9H-fluorèn-4-yl)-1H-imidazo[4,5-c]pyridine-6-carboxylique, sous forme d'une poudre orange utilisée telle quelle à l'étape suivante, dont les caractéristiques sont les suivantes .
Spectre de masse (LCMS) : m/z = 356 (M+)

Etape 2 : On opère comme dans l'exemple 14, mais à partir de 700mg de l'ester méthylique de l'acide 2-(9(R,S)-amino-9H-fluorèn-4-yl)-1H-imidazo[4,5-c]pyridine-6-carboxylique obtenue à l'étape précédente, 319 mg d'acide 1H-pyrrolo-[2,3-b]pyridine-4-carboxylique, 414 mg de chlorhydrate de 1-(3-diméthylaminopropyl)-3-éthylcarbodiimide (EDCI) et 292 mg de 1-hydroxybenzotriazole (HOBT) dans 14 mL de diméthylformamide, pendant 20 heures à température ambiante. Après purification par flash-chromatographie sur gel de silice (40-63µm), en éluant avec un mélange de dichlorométhane et de méthanol (95/5 en volumes) et empâtage dans l'éther diisopropylique, on obtient 650 mg de solide beige qui contient un mélange d'ester méthylique et d'ester éthylique du produit attendu. Après une chromatographie supplémentaire sur colonne Kromasil C8 20 µm, de longueur 35 cm et de diamètre 8 cm, en éluant avec une phase un mélange de tampon queux acétate d'ammonium (pH= 4,9) et d'acétonitrile (65/35) à un débit de 70 mL/mn , et en détectant le produit élué par spectroscopie UV à 254 nM, on obtient 87 mg d'ester méthylique de l'acide 2-{9(R,S)-[(1H-pyrrolo[2,3-b]pyridine-4-carbonyl)-amino]-9H-fluorèn-4-yl}-1H-imidazo[4,5-c]pyridine-6-carboxylique sous forme d'un solide blanc dont les caractéristiques sont les suivantes :
Spectre de masse (LC/MS/ES+/-) : m/z = 500 (M+)
Spectre RMN 1H (400 MHz, □ en ppm, DMSO-d6) : 3,88 (s, 3H) ; 6,39 (d , J = 8,5 Hz, 1H) ; 6,91 (s, 1H) ; 7,23 (t, J = 7,5 Hz, 1H) ; 7,31 (t, J = 7,5 Hz, 1H); de 7,36 à 7,51 (m, 2H) ; 7,59 (d, J = 7,5 Hz, 1H) ; 7,62 (s, 1H) ; 7,69 (d, J = 7,5 Hz, 1H) ; 7,81 (d, J = 7,5 Hz, 1H) ; 8,01 (m étalé, 1H) ; 8,28 (s, 1H) ; 8,29 (d, J = 5,0 Hz, 1H) ; 8,94 (s, 1H) ; 9,26 (d, J = 8,5 Hz, 1H) ; 11,85 (m large, 1H) ; 12,9 (m large, 1H).

### Exemple 266 : Synthèse du [5-(1H-benzimidazol-2-yl)-2-cyano-9H-fluorén-9(R,S)-yl]-amide de l'acide 1H-pyrrolo[2,3-b]pyridine-4-carboxyfique.

Etape 1 : Une suspension de 250 mg de 5-(1H-benzimidazol-2-yl)-2-bromo-9H-fluorèn-9-one, obtenue à l'étape 3 de l'exemple 254, et de 179 mg de cyanure de cuivre dans 15 mL de N-méthylpyrrolidinone est agitée en tube scellé à 220 °C sous micro-ondes pendant 50 minutes. Le mélange réactionnel est versé sur 250 mL de glace. Après 15 minutes d'agitation à température ambiante, le précipité vert formé est filtré, lavé par 3 fois 50 mL d'eau puis séché à 50 °C à l'étuve sous vide. La poudre verte ainsi obtenue est reprise dans 50 mL de diméthylformamide et la suspension est chauffée à reflux pendant 30 minutes. Les résidus insolubles sont filtrés à chaud et lavés par 50 mL de diméthylformamide bouillant. L'évaporation du filtrat conduit à 210 mg d'une poudre orangée qui est reprise dans 50 mL d'un mélange de dichlorméthane et de méthanol (90/10 en volumes). La suspension est chauffée à reflux pendant 30 minutes. Le précipité persistant est filtré, lavé par 2 fois 25 mL de dichlorométhane et d'éther isopropylique. On obtient ainsi 140 mg de 5-(1H-benzimidazol-2-yl)-9-oxo-9H-fluorène-2-carbonitrile, sous la forme d'une poudre jaune qui est utilisée sans autre purification.

Etape 2 : On agite 136 mg de 5-(1H-benzimidazol-2-yl)-9-oxo-9H-fluorène-2-carbonitrile, obtenu à l'étape précédente, en suspension dans 3 mL de N-méthylpyrrolidinone et on ajoute 88 mg de chlorhydrate d'hydroxylamine et 173 mg d'acétate de sodium. Le mélange est chauffé à 60 °C pendant 1 heure 30 minutes. La solution jaune-clair obtenue est versée dans 100 mL d'eau glacée. Le précipité jaune formé est filtré, lavé à l'eau puis séché à 50°C à l'étuve sous vide pendant 18 heures. On obtient ainsi 83 mg d'une poudre jaune, dont l'analyse par LC/MS montre la présence de 30 % environ de 5-(1H-benzimidazol-2-yl)-9(Z,E)-oximino-9H-fluorène-2-carbonitrile, et qui est utilisée sans purification à l'étape suivante.

Etape 3: Le mélange obtenu à l'étape précédente est dissout dans un mélange de 2 mL d'éthanol, 1 mL d'eau et 1 mL d'acide acétique. On ajoute 58 mg de poudre de zinc et le mélange réactionnel est agité à température ambiante pendant 1 heure 30 minutes. L'excès de zinc est filtré sur célite et lavé à l'éthanol. L'évaporation du filtrat sous pression réduite conduit à une poudre blanche qui est reprise dans 50 mL d'une solution aqueuse saturée d'hydrogénocarbonate de sodium et 50 mL d'un mélange de dichlorométhane et de méthanol (90/10 en volumes). L'insoluble est filtré, et la phase aqueuse du filtrat est extraite par 3 fois 50 mL d'un mélange de dichlorométhane et de méthanol (90 /10 en volumes). Les phases organiques sont rassemblées, séchées sur sulfate de magnésium et évaporées à sec sous pression réduite. On obtient ainsi 33 mg d'une poudre blanche, qui est utilisée telle quelle à l'étape suivante.

Etape 4 : La poudre obtenue à l'étape précedente est dissoute dans 3 mL de diméthylformamide puis on ajoute successivement 16 mg d'acide 1H-pyrrolo[2,3-b]pyridine-4-carboxylique, 13 mg d'hydrate d'hydroxybenzotriazole (HOBT) et 19 mg de chlorhydrate de 1-(3-diméthylaminopropyl)-3-éthylcarbodiimide (EDCI). Le mélange réactionnel est agité 17 heures à température ambiante. L'évaporation du solvant sous pression réduite conduit à une huile jaune qui est purifiée chromatographie flash sur gel de silice, en éluant avec un gradient de mélanges de dichlorométhane et de méthanol (de 100/0 à 90/10 en volumes). On obtient ainsi 19 mg de [5-(1H-benzimidazol-2-yl)-2-cyano-9H-fluorèn-9(R,S)-yl]-amide de l'acide 1H-pyrrolo[2,3-b]pyridine-4-carboxylique, sous la forme d'une poudre beige clair, dont la caractéristique est la suivante.
Spectre de masse (ESI+) : m/z = 467 (MH+)

### Exemple 267: Synthèse du (3-diéthylamino-propyl)-amide de l'acide 2-{9(R,S)-[(1H-pyrrolo[2,3-b]pyridine-4-carbonyl)-amino]-9H-fluorèn-4-yl}-1H-benzimidazole-5-carboxylique

Etape 1 : On dissout 500 mg de 4-(5-carboxy-1H-benzimidazol-2-yl)-fluorène-9-one, obtenue comme à l'étape 1 de l'exemple 241, dans 10 mL de diméthylformamide. On ajoute successivement 191 mg de 3-diéthylaminopropylamine, 225 mg d'hydrate d'hydroxybenzotriazole (HOBT) et 281 mg de chlorhydrate de 1-(3-diméthylaminopropyl)-3-éthylcarbodiimide (EDCI). La soution obtenue est agitée 17 heures à température ambiante. La pâte jaune obtenue après évaporation du solvant sous pression réduite est reprise dans un mélange de 50 mL d'eau et de 50 mL d'une solution aqueuse saturée d'hydrogénocarbonate de sodium et extraite par 4 fois 100 mL d'une solution composée de 90 mL de dichlorométhane et 10 mL de méthanol ammoniacal 7N. Les phases organiques jointes sont séchées sur sulfate de magnésium. L'huile jaune obtenue après évaporation du solvant sous pression réduite est purifiée par flash chromatographie sur gel de silice, en éluant avec un gradient de dichlorméthane et d'une solution 7N d'ammoniac dans le méthanol (de 100/0 à 90/10 en volumes). On obtient 457 mg du (3-diéthylamino-propyl)-amide de l'acide 2-(9-oxo-9H-fluorèn-4-yl)-1H-benzimidazole-5-carboxylique sous la forme d'une poudre jaune utilisée telle quelle à l'étape suivante.

Etape 2 : On dissout 340 mg du (3-diéthylamino-propyl)-amide de l'acide 2-(9-oxo-9H-fluorèn-4-yl)-1H-benzimidazole-5-carboxylique, obtenu à l'étape précédente, dans 20 mL d'éthanol. La solution jaune obtenue est additionnée de 308 mg d'acétate de sodium et de 156 mg de chlorhydrate d'hydroxylamine. La suspension est agitée 17 h à température ambiante. L'éthanol est évaporé sous pression réduite et la poudre blanche obtenue est reprise dans 100 ml d'eau puis extraite par 3 fois 100 mL d'une solution composée de 90 mL de dichlorométhane et 10 mL de méthanol ammoniacal 7N. Les phases organiques jointes sont ensuite séchée sur sulfate de magnésium. Après évaporation du solvant sous pression réduite, on obtient 346 mg du (3-diéthylamino-propyl)-amide de l'acide 2-{9(Z,E)-[hydroxyimino]-9H-fluorèn-4-yl}-1H-benzimidazole-5-carboxylique, en mélange 50/50 des isomères Z et E, sous la forme d'une poudre blanc-cassé.

Etape 3 : On dissout 333 mg de (3-diéthylamino-propyl)-amide de l'acide 2-{9(Z,E)-[hydroxyimino]-9H-fluorèn-4-yl}-1H-benzimidazole-5-carboxylique, obtenu à l'étape précédente, dans un mélange de 2 mL d'eau, de 4 mL d'éthanol et de 1.9 mL d'acide acétique. La solution incolore obtenue est additionnée de 186 mg de poudre de zinc. La suspension est agitée à température ambiante pendant 2 heures. L'huile rosée obtenue après évaporation du solvant sous pression réduite est solubilisée dans 90 mL de dichlorométhane et 10 mL de méthanol ammoniacal 7N. Cette solution est lavée par 100 mL d'une solution aqueuse saturée d'hydrogénocarbonate de sodium. La phase aqueuse est séparée et réextraite par deux fois 100 mL d'une solution composée de 90 mL de dichlorométhane et 10 mL de méthanol ammoniacal 7N. Les phases organiques sont rassemblées et séchées sur sulfate de magnésium. Après évaporation du solvant sous pression réduite, on obtient 330 mg du (3-diéthylamino-propyl)-amide de l'acide 2-(9(R,S)-amino-9H-fluorèn-4-yl)-1H-benzimidazole-5-carboxylique sous la forme d'une poudre légèrement bleutée utilisée telle quelle à l'étape suivante.

Etape 4: On dissout 330 mg de (3-diéthylamino-propyl)-amide de l'acide 2-(9(R,S)-amino-9H-fluorèn-4-yl)-1H-benzimidazole-5-carboxylique, obtenu à (9(R,S)-amino-9H-fluorèn-4-yl)-1H-benzimidazole-5-carboxylique, obtenu à l'étape précédente, dans 6 mL de diméthylformamide. On ajoute 118 mg de l'acide 1H-pyrrolo[2,3-b]pyridine-4-carboxylique, 56 mg d'hydrate d'hydroxybenzotriazole (HOBT) et 139 mg de chlorhydrate de 1-(3-diméthylaminopropyl)-3-éthylcarbodiimide (EDCI). La solution jaune obtenue est agitée à température ambiante pendant 1 heure 30 minutes. La gomme jaune obtenue après évaporation du solvant sous pression réduite est reprise dans un mélange de 100 mL d'une solution aqueuse saturée d'hydrogénocarbonate de sodium, 90 mL de dichlorométhane et 10 mL de méthanol ammoniacal 7N. Le précipité formé est filtré, lavé à l'eau, puis au dichlorométhane et au méthanol pour conduire, après 16 heures de séchage à 50 °C à l'étuve sous vide, à 112 mg du (3-diéthylamino-propyl)-amide de l'acide 2-{9(R,S)-[(1H-pyrrolo[2,3-b]pyridine-4-carbonyl)-amino]-9H-fluorén-4-yl}-1H-benzimidazole-5-carboxylique sous la forme d'une poudre blanche, dont les caractéristiques sont les suivantes :
Spectre de masse (ESI+) : m/z = 598 (MH+).
Spectre RMN 1H (400 MHz , □ en ppm DMSO-d6) : 3,93 (s, 3H) ; 6,40 (d , J = 8,5 Hz, 1H) ; 6,91 (dd, J = 1,5 et 3,5 Hz, 1H) ; 7,23 (m masqué, 1H) ; 7,25 (t, J = 7,5 Hz, 1H) ; 7,34 (t, J = 7,5 Hz, 1H) ; 7,46 (d, J = 5,0 Hz, 1H) ; 7,50 (t, J = 7,5 Hz, 1H) ; 7,57 (m étalé, 1H) ; 7,62 (m, 4H) ; 7,74 (d, J = 7,5 Hz, 1H) ; 8,30 (d, J = 5,0 Hz, 1H) ; 9,27 (d, J = 8,5 Hz, 1H) ; 11,85 (m large, 1H) ; 12,9 (m large, 1H) .

### Exemple 268 : Synthèse du [4-(6-fluoro-5-methoxy-1H-benzimidazol-2-yl)-9H-fluorène-9(R,S)-yl]-amide de l'acide 1H-pyrrolo[2,3-b]pyridine-4-carboxylique

Etape 1 :On opère comme à l'étape 1 de l'exemple 1, à partir de 1 g de 4-fluoro-5-methoxy-2-nitro-phenylamine, qui peut être préparée selon Chem Pharm Bull 37(6), 1517-1523,1989, dans 20 mL de dichlorométhane et 10 mL de tétrahydrofurane, 1,5 mL de triéthylamine et 1,3 g de chlorure de l'acide fluorène-4-one-9-carboxylique, pendant 20 heures à température ambiante puis 5 heures au reflux 5h et enfin 72 heures à température ambiante. Après purification par empâtage à l'éther diisopropylique, on obtient ainsi 700 mg du (4-fluoro-5-méthoxy-2-nitro-phényl)amide de l'acide 9-oxo-9H-fluorène-4-carboxylique, sous forme d'une poudre jaune utilisée telle quelle à l'étape suivante, dont les caractéristiques sont les suivantes:
Spectre de masse (LC/MS) : m/z = 392 (M+)

Etape 2 : Dans un ballon de 100 mL sous atmosphère d'argon, on chauffe, à 90°C pendant 4 heures, une solution de 700 mg de (4-fluoro-5-méthoxy-2-nitro-phényl)amide de l'acide 9-oxo-9H-fluorène-4-carboxylique, obtenu à l'étape précédente, et de 300 mg de fer, dans un mélange de 45 mL d'éthanol, 5 mL d'eau et 70 µl d'une solution aqueuse à 37% d'acide chlorhydrique. Après refroidissement, on ajuste à pH=9 par addition d'une solution aqueuse saturée de carbonate de sodium, on filtre l'insoluble qui est lavé avec une solution aqueuse saturée d'hydrogénocarbonate de sodium, puis à l'eau, on amène à sec sous pression réduite. Les phases aqueuses sont extraites deux fois à de l'acétate d'éthyle. Les phases organiques sont lavées à l'eau, séchées sur le sulfate de magnésium et amenées à sec sous pression réduite. Après purification par empâtage à l'éther diisopropylique, on obtient 200 mg de (2-amino-4-fluoro-5-méthoxy-phenyl)amide de l'acide 9-oxo-9H-fluorène-4-carboxylique, sous forme d'une poudre jaune utilisée telle quelle à l'étape suivante, dont les caractéristiques sont les suivantes :
Spectre de masse (EI) : m/z = 362 (M+)

Etape 3 : On opère comme dans l'exemple 68. Dans un ballon de 25 mL sous atmosphère d'argon, on chauffe, à 140°C pendant 1 heure, une solution de 200 mg de (2-amino-4-fluoro-5-méthoxy-phényl)amide de l'acide 9-oxo-9H-fluorène-4-carboxylique , obtenu à l'étape précédente, dans 9 mL d'acide acétique. Après refroidissement, on amène à sec sous pression réduite. Après purification par empâtage à l'éther diisopropylique, on obtient 170 mg de 4-(5-fluoro-6-méthoxy-1H-benzimidazol-2-yl)-9H-fluorène-9-one, sous forme d'une poudre jaune pâle utilisée telle quelle à l'étape suivante, dont les caractéristiques sont les suivantes :
Spectre de masse (EI) : m/z = 344 (M+)

Etape 4 : On opère comme à l'exemple 5, à partir de 170 mg de 4-(5-fluoro-6-méthoxy-1H-benzimidazol-2-yl)-9H-fluorène-9-one, obtenue à l'étape précédente, de 103 mg de chlorhydrate d'hydroxylamine et de 203 mg d'acétate de sodium dans 3 mL d'éthanol agités pendant 20 heures à température ambiante. Après concentration du solvant sous pression réduite, le résidu est repris successivement par de l'eau, puis du toluène, amené à sec. On obtient ainsi 141 mg de 4-(5-fluoro-6-méthoxy-1H-benzimidazol-2-yl)-9H-fluorène-9-one oxime (Z,E), en mélange 50-50 des isomères Z et E, sous forme d'une poudre beige utilisée telle quelle à l'étape suivante, dont les caractéristiques sont les suivantes:
Spectre de masse (EI/CI) : m/z = 359 (M+).

Etape 5 : On opère comme à l'étape 4 de l'exemple 216. Dans un ballon de 30 mL sous atmosphère d'argon, on dissout 140 mg de mélange équimoléculaire de 4-(5-fluoro-6-méthoxy-1H-benzimidazol-2-yl)-9H-fluorène-9-one oxime (Z,E), obtenue à l'étape précédente, dans un mélange de 0,7 mL d'éthanol, 0,7 mL de d'eau et 0,7mL d'acide acétique. A température ambiante, on ajoute en trois fois 100 mg de zinc, entre chaque ajout, on agite environ une heure à deux heures. Après purification par flash-chromatographie sur gel de silice (40-63µm), en éluant avec un mélange de dichlorométhane et d'ammoniac en solution 7N dans le méthanol (95/5 en volumes), on obtient ainsi 127 mg de 4-(5-fluoro-6-méthoxy-1H-benzimidazol-2-yl)-9H-fluorène-9(R,S)-amine, sous forme d'une résine beige utilisée telle quelle à l'étape suivante, dont les caractéristiques sont les suivantes:
Spectre de masse (LC/MS) : m/z = 345 (M+).

Etape 6 : On opère comme dans l'exemple 14. Dans un ballon de 10 mL, on dissout 127 mg de 4-(5-fluoro-6-méthoxy-1H-benzimidazol-2-yl)-9H-fluorène-9(R,S)-amine, obtenue à l'étape précédente, 60 mg d'acide 1H-pyrrolo-[2,3-b]pyridine-4-carboxylique, 70 mg de chlorhydrate de 1-(3-diméthylaminopropyl)-3-éthylcarbodiimide (EDCI) et 25 mg de 1-hydroxybenzotriazole (HOBT), dans 2 mL de diméthylformamide pendant 20 heures à température ambiante. Après purification par flash-chromatographie sur gel de silice (40-63µm), en éluant avec un mélange de dichlorométhane et de méthanol (98/2 puis 95/5 en volumes), puis empâtage à l'éther diisopropylique, on obtient ainsi 95,5 mg de [4-(5-fluoro-6-méthoxy-1H-benzimidazol-2-yl)-9H-fluorène-9(R,S)-yl]-amide de l'acide 1H-pyrrolo[2,3-b]pyridine-4-carboxylique, sous forme d'une poudre beige dont les caractéristiques sont les suivantes :
Point de fusion (Kofler) = 200-210°C (gommeux).
Spectre de masse (LC/MS) : m/z = 489 (M+)

### Exemple 269 : Synthèse du [4-(6-fluoro-5-hydroxy-1H-benzimidazol-2-yl)-9H-fluorène-9(R,S)-yl]-amide de l'acide 1H-pyrrolo[2,3-b]pyridine-4-carboxylique

Etape 1 : On opère comme dans l'étape 1 de l'exemple 230. Dans un ballon de 50 mL sous argon, on dissout 1,07 g de 4-(5-fluoro-6-méthoxy-1H-benzimidazole-2-yl)-9H-fluorène-9-one, obtenue à l'étape 3 de l'exemple 268, dans 20 mL de dichlorométhane, on refroidit à 0°C et l'on ajoute 7,8 mL d'une solution molaire dans le dichlorométhane de tribromure de bore. Après 20 heures d'agitation, le milieu réactionnel est versé sur de l'eau, l'insoluble est essoré, lavé à l'eau puis séché. Après purification par empâtage à l'éther diisopropylique, on obtient ainsi 914 mg de 4-(5-fluoro-6-hydroxy-1H-benzimidazol-2-yl)-9H-fluorène-9-one, sous forme d'une poudre marron, utilisée telle quelle à l'étape suivante, dont les caractéristiques sont les suivantes :
Spectre de masse (LC/MS/ES/+/-) : m/z = 330 (M+).

Etape 2 : On opère comme dans l'exemple 5, mais à partir de 914 mg de 4-(5-fluoro-6-hydroxy-1H-benzimidazol-2-yl)-9H-fluorène-9-one, obtenue à l'étape précédente, de 580 mg de chlorhydrate d'hydroxylamine et de 1,1 g d'acétate de sodium dans 17 mL d'éthanol agités pendant 20h à température ambiante. Après concentration du solvant sous pression réduite, le résidu est repris successivement par de l'eau, puis du toluène et amené à sec. Après purification par flash-chromatographie sur gel de silice (40-63µm), en éluant avec un mélange de dichlorométhane et de méthanol (95/5 puis 9/1 en volumes), puis empâtage à l'éther diisopropylique, on obtient ainsi 667 mg de 4-(5-fluoro-6-hydroxy-1H-benzimidazol-2-yl)-9H-fluorène-9-one oxime (Z,E), en mélange 50/50 des isomères Z et E, sous forme d'une poudre beige utilisée telle quelle à l'étape suivante, dont les caractéristiques sont les suivantes :
Spectre de masse (EI/CI) : m/z = 345 (M+).

Etape 3 : On opère comme à l'étape 4 de l'exemple 216. Dans un ballon de 30 mL sous atmosphère d'argon, on dissout 667 mg de mélange équimoléculaire de 4-(5-fluoro-6-hydroxy-1H-benzimidazol-2-yl)-9H-fluorène-9-one oxime (Z,E), obtenue à l'étape précédente, dans un mélange de 3,5 mL d'éthanol et 3,5 mL de d'eau et 3,5 mL d'acide acétique ; à température ambiante on ajoute, en trois fois 500 mg de zinc. Entre chaque ajout, on agite environ une heure à deux heures. Après purification par flash-chromatographie sur gel de silice (40-63µm), en éluant avec un mélange de dichlorométhane et d'ammoniac en solution 7N dans le méthanol (9/1 puis 85/15 en volumes), on obtient ainsi 456 mg de 4-(5-fluoro-6-hydroxy-1H-benzimidazol-2-yl)-9H-fluorène-9(R,S)-amine, sous forme d'une poudre beige, utilisée telle quelle à l'étape suivante, dont les caractéristiques sont les suivantes :
spectre de masse (LC/MS): m/z = 331 (M+)

Etape 4 : On opère comme dans l'exemple 14, mais à partir de 450 mg de 4-(5-fluoro-6-hydroxy-1H-benzimidazole-2-yl)-9H-fluorène-9(R,S)-amine, obtenue à l'étape précédente, 220 mg d'acide 1H-pyrrolo-[2,3-b]pyridine-4-carboxylique, 260 mg de chlorhydrate de 1-(3-diméthylaminopropyl)-3-éthylcarbodiimide (EDCI) et 92 mg de 1-hydroxybenzotriazole (HOBT) dans 7 mL de diméthylformamide, pendant 20 heures à température ambiante. Après purification par flash-chromatographie sur gel de silice (40-63µm), en éluant avec un mélange de dichlorométhane et d'ammoniac 7N dans le méthanol (95/5 puis 9/1 en volumes), puis empâtage à l'éther diisopropylique, on obtient ainsi 492 mg de [4-(5-fluoro-6-hydroxy-1H-benzimidazol-2-yl)-9H-fluorèn-9(R,S)-yl]-amide de l'acide 1H-pyrrolo[2,3-b]pyridine-4-carboxylique, sous forme d'une poudre blanche dont les caractéristiques sont les suivantes :
Point de fusion (Kofler) = >260°C
Spectre de masse (LC/MS) : m/z = 475 (M+)

### Exemple 270 : Synthèse du {4-[5-fluoro-6-(3-méthoxy-propoxy)-1H-benzimidazol-2-yl]-9H-fluorèn-9(R,S)-yl}-amide de l'acide 1H-pyrrolo[2,3-b]pyridine-4-carboxylique

Etape 1 : Dans un tricol de 250 mL, sous atmosphère d'argon, 1 g de 3-méthoxy-1-propanol dans 20 mL de tétrahydrofurane sont refroidis à 0°C à l'aide d'un bain de glace puis 450 mg d'hydrure de sodium à 50% dans l'huile de vaseline sont introduits par fractions. Après 15 minutes d'agitation à 0°C, on ajoute, en 5 minutes, une solution de 1,5 g de 4,5-difluoro-2-nitroaniline dans 20 mL de tétrahydrofurane puis on chauffe au voisinage de 70°C pendant 1 heure 30 minutes. Le mélange réactionnel est versé sur 200 mL d'eau, extrait par trois fois 50 mL d'acétate d'éthyle. Les phases organiques jointes sont séchées sur du sulfate de magnésium et amenées à sec sous pression réduite. Après purification par flash-chromatographie sur gel de silice (15-20 µm), en éluant avec un mélange de cyclohexane et d'acétate d'éthyle (80/20 en volumes), on obtient ainsi 1 g de 4-fluoro-5-(3-méthoxy-propoxy)-2-nitro-phénylamine sous forme d'un solide rouge, utilisé tel quel dans l'étape suivante et dont la caractéristique est la suivante :
Spectre de masse (LCMS) : m/z = 244 (M+)

Etape 2 : Dans un autoclave de 25 mL, on dissout 330 mg de 4-fluoro-5-(3-méthoxy-propoxy)-2-nitro-phénylamine, obtenue à l'étape précédente, dans 20 mL d'éthanol, on ajoute 40 mg de palladium sur charbon à 10% puis on soumet à une pression initiale d'hydrogène de 1 bar et à 20°C pendant 16 heures. Après refroidissement, le volume d'hydrogène absorbé est de 91 mL. Après filtration du catalyseur puis concentration à sec sous pression réduite, on obtient 290 mg de 4-fluoro-5-(3-méthoxy-propoxy)-benzène-1,2-diamine, sous forme d'une huile visqueuse noire utilisée telle quelle à l'étape suivante et dont la caractéristique est la suivante :
Spectre de masse (EI) : m/z = 214 (M+)

Etape 3 : On agite, à température ambiante sous argon, un mélange de 100 mg de (4-formyl-9H-fluorèn-9(R,S)-yl)-amide de l'acide 1H-pyrrolo[2,3-b]pyridine-4-carboxylique, préparé comme à l'exemple 234, de 60 mg de 4-fluoro-5-(3-méthoxy-propoxy)-benzène-1,2-diamine, préparée à l'étape précédente, et 46 mg de trichlorure ferrique dans 5 mL de DMF anhydre. Après 48 heures, on évapore à sec le milieu réactionnel et on purifie le résidu par flash-chromatographie sur gel de silice (20-40 µm) en éluant avec un mélange de méthanol et de dichlorométhane (10/90 en volumes). On obtient ainsi 58 mg de {4-[5-fluoro-6-(3-méthoxy-propoxy)-1H-benzimidazol-2-yl]-9H-fluorèn-9(R,S)-yl}-amide de l'acide 1H-pyrrolo[2,3-b]pyridine-4-carboxylique sous forme d'un solide orange et dont les caractéristiques sont les suivantes : Point de fusion (Kofler) : >260°C
Spectre de masse (EI): m/z = 547 (M+)

### Exemple 271 : (4-[6-(3-diméthylamino-propoxy)-5-fluoro-1H-benzimidazol-2-yl]-9H-fluorèn-9(R,S)-yl)-amide de l'acide 1H-pyrrolo[2,3-b]pyridine-4-carboxylique

Etape 1 : Dans un tricol de 250 mL, sous atmosphère d'argon, 5,3 g de 3-diméthylamino-1-propanol, en solution dans 100 mL de tétrahydrofurane, sont refroidis à 0°C à l'aide d'un bain de glace puis on introduit 2,3 g d'hydrure de sodium à 50% dans l'huile de vaseline. Après 1 heure d'agitation à 0°C, on ajoute rapidement une solution de 3 g de 4,5-difluoro-2-nitroaniline dans 100 mL de tétrahydrofurane puis on chauffe au voisinage de 70°C pendant 1 heure. Le mélange réactionnel est versé sur 100 mL d'eau, extrait par trois fois 100 mL d'acétate d'éthyle. La phase organique est séchée sur du sulfate de magnésium et amenée à sec sous pression réduite. Le solide obtenu est lavé au pentane, filtré et séché sous hotte. On obtient ainsi 3,5 g de 5-(3-diméthylamino-propoxy)-4-fluoro-2-nitro-phénylamine sous forme de solide jaune dont les caractéristiques sont les suivantes :
Point de fusion (Kofler) : 128°C
Spectre de masse (LCMS) : m/z = 257 (M+)

Etape 2 : Dans un autoclave de 25 mL, on dissout 350 mg de 5-(3-diméthylamino-propoxy)-4-fluoro-2-nitro-phénylamine, obtenue à l'étape précédente, dans 20 mL d'éthanol on ajoute 40 ma de Palladium sur charbon à 10% puis on soumet à une pression initiale d'hydrogène de 1 bar et à 20°C pendant 16 heures. Après refroidissement, le volume d'hydrogène absorbé est de 123 mL. Après filtration du catalyseur puis concentration à sec sous pression réduite, on obtient 280 mg de 4-(3-diméthylamino-propoxy)-5-fluoro-benzène-1,2-diamine, sous forme d'une huile visqueuse noire utilisée telle quelle à l'étape suivante et dont la caractéristique est la suivante :
Spectre de masse (EI) : m/z = 227 (M+)

Etape 3 : On agite, à température ambiante sous argon, un mélange de 100 mg de (4-formyl-9H-fluorèn-9(R,S)-yl)-amide de l'acide 1H-pyrrolo[2,3-b]pyridine-4-carboxylique, préparé comme à l'exemple 234, de 65 mg de 4-(3-diméthylamino-propoxy)-5-fluoro-benzène-1,2-diamine, préparée à l'étape précédente et 46 mg de trichlorure ferrique dans 5 mL de diméthylformamide anhydre. Après 48 heures, on évapore à sec le milieu réactionnel puis on purifie le résidu par flash-chromatographie sur gel de silice (20-40 µm), en éluant avec un mélange de dichlorométhane et d'ammoniac en solution 7N dans le méthanol (95/5 puis 9/1 en volumes). On obtient ainsi 42 mg de {4-[6-(3-diméthylamino-propoxy)-5-fluoro-1H-benzimidazol-2-yl]-9H-fluorèn-9(R,S)-yl}-amide de l'acide 1H-pyrrolo[2,3-b]pyridine-4-carboxylique sous forme d'un solide jaune et dont les caractéristiques sont les suivantes :
Point de fusion (Kofler) : 226°C
Spectre de masse (El) : m/z = 560 (M+)

### Exemple 272: composition pharmaceutique:

On a préparé des comprimés répondant à la formule suivante :

| | |
|---|---|
| Produit de l'exemple 87 | 0,2 g |
| Excipient pour un comprimé terminé à | 1 g |

(détail de l'excipient: lactose, talc, amidon, stéarate de magnésium).

### Exemple 273 : composition pharmaceutique

On a préparé des comprimés répondant à la formule suivante :

| | |
|---|---|
| Produit de l'exemple 92 | 0,2 g |
| Excipient pour un comprimé terminé à | 1 g |

(détail de l'excipient : lactose, talc, amidon, stéarate de magnésium).

### Tests biologiques permettant de caractériser biologiquement les produits de l'invention :

Le phosphate inorganique libéré au cours de l'hydrolyse de l'ATP par l'activité ATPasique de Hsp82 est quantifié par la méthode du green Malachite. En présence de ce réactif, il y a formation du complexe phosphate inorganique-molybdate-vert de malachite qui absorbe à une longueur d'onde de 620 nm.

Les produits à évaluer sont incubés dans un volume réactionnel de 30 µl, en présence de 1µM Hsp82 et de 250 µM de substrat (ATP) dans un tampon composé de 50 mM Hepes-NaOH (pH 7.5), 1 mM DTT, 5 mM MgC12 et 50 mM KCI à 37°C pendant 60 min. Parallèlement, une gamme de phosphate inorganique comprise entre 1 à 40 µM est constituée dans le même tampon.

L'activité ATPasique est ensuite révélée par l'addition de 60 µl du réactif biomol green (Tebu). Après 20 min d'incubation à température ambiante, l'absorbance des différents puits est mesurée à l'aide d'un lecteur de microplaque à 620 nm. La concentration en phosphate inorganique de chaque échantillon est alors calculée à partir de la courbe d'étalonnage.

L'activité ATPasique d' Hsp82 est exprimée en concentration de phosphate inorganique produit en 60 min.L'effet des divers produits testés est exprimé en pourcentage d'inhibition de l'activité ATPasique.

Dans le test ci-dessus, le composé A000187458 a une concentration inhibitrice 50% (Cl50) égale à 2.5 µM

La formation d'ADP due à l'activité ATPasique de Hsp82 a été utilisée pour mettre au point une autre méthode d'évaluation de l'activité enzymatique de cette enzyme par application d'un système de couplage enzymatique faisant intervenir la pyruvate kinase (PK) et la lactate deshydrogensase (LDH). Dans cette méthode spectrophotométrique de type cinétique, la PK catalyse la formation d'ATP et de pyruvate à partir de phosphoenol-pyruvate (PEP) et de l'ADP produit par HSP82. Le pyruvate formé, substrat de la LDH, est ensuite transformé en lactate en présence de NADH. Dans ce cas, la diminution de la concentration en NADH, mesurée par la diminution de l'absorbance à la longueur d'onde de 340 nm est proportionnelle à la concentration en ADP produit par HSP82.

Les produits testés sont incubés dans un volume réactionnel de 100 µl de tampon composé de 100 mM Hepes-NaOH (pH 7.5), 5 mM MgCl2, 1mM DTT, 150 mM KCI, 0.3 mM NADH, 2.5 mM PEP et 250 µM ATP. Ce mélange est preincubé à 37°C pendant 30 min avant addition de 3.77 unités de LDH et 3.77 unités de PK. La réaction est initiée par addition du produit à évaluer, en concentrations variables, et de Hsp82, à la concentration de 1µM. La mesure de l'activité enzymatique de Hsp82 est alors réalisée, en continu, dans un lecteur de microplaque, à 37°C, à la longueur d'onde de 340nm. La vitesse initiale de la réaction est obtenue par la mesure de la pente de la tangente à l'origine de la courbe enregistrée. L'activité enzymatique est exprimée en µM d'ADP formé par minute. L'effet des divers produits testés est exprimé en pourcentage d'inhibition de l'activité ATPasique selon la codification ci-dessous :
A : IC50 < 1µM
B : 1µM < IC50 < 10µM
C : 10µM < IC50 < 100 µM

**Tableau de résultats**

| Ex | Structure | Hsp82 ATPase IC50 µM | Ex | Structure | Hsp82 ATPase IC50 µM |
|---|---|---|---|---|---|
| 1 | 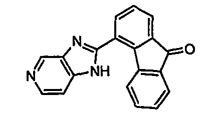 | B | 2 | 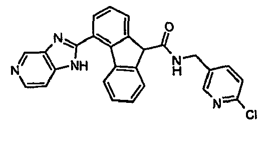 | C |
| 3 | 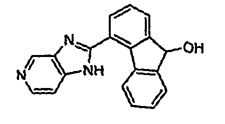 | C | 4 | 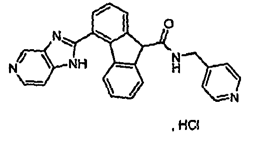 | C |
| 5 | 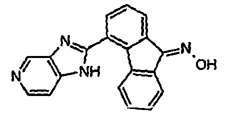 | B | 6 | 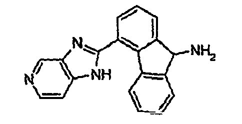 | C |
| 7 | 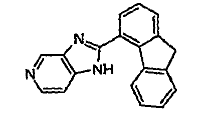 | C | 8 | 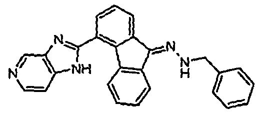 | B |
| 9 | 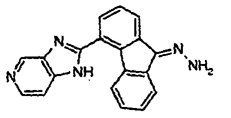 | B | 10 | 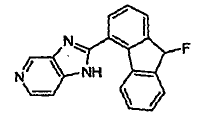 | B |
| 11 | 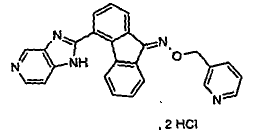 | B | 12 | 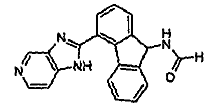 | C |
| 13 | 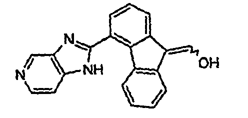 | C | 14 | 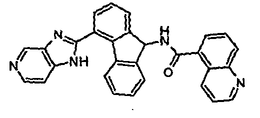 | A |
| 14A | 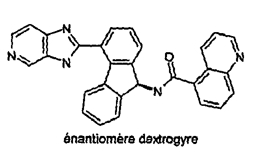 | A | 14B | 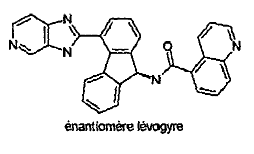 | C |
| 15 | 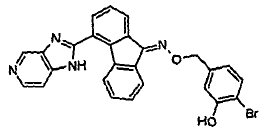 | B | 16 | 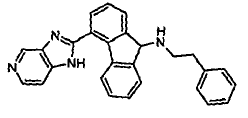 | C |
| 17 | 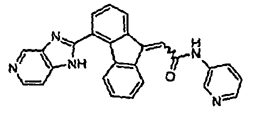 | B | 18 | 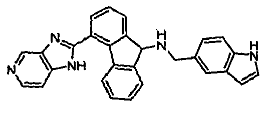 | C |
| 19 | 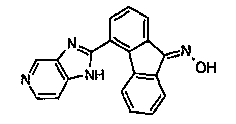 | B | 20 | 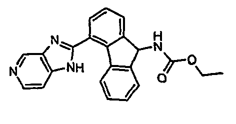 | B |
| 21 | 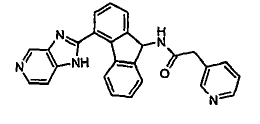 | B | 22 | 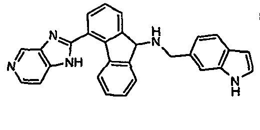 | C |
| 23 | 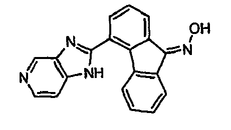 | A | 24 | 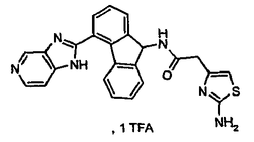 | B |
| 25 | 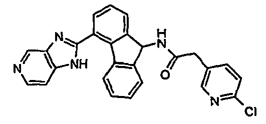 | B | 26 | 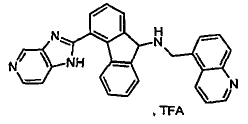 | B |
| 27 | 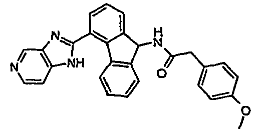 | B | 28 | 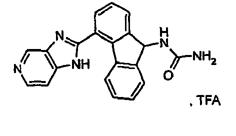 | C |
| 29 | 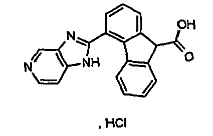 | C | 30 | 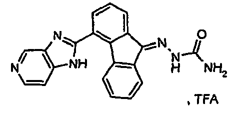 | C |
| 31 | 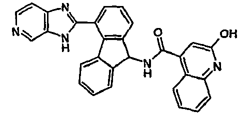 | B | 32 | 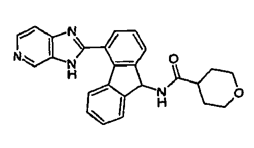 | C |
| 33 | 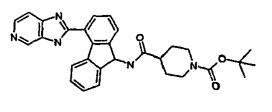 | B | 34 | 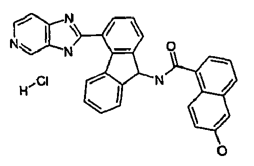 | B |
| 35 | 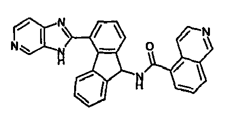 | C | 36 | 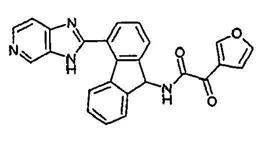 | B |
| 37 | 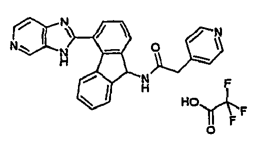 | B | 38 | 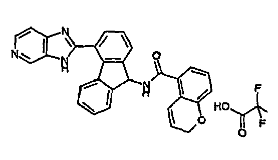 | B |
| 39 | 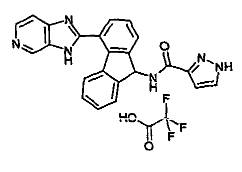 | B | 40 | 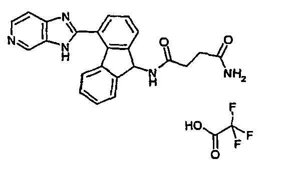 | B |
| 41 | 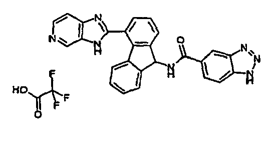 | A | 42 | 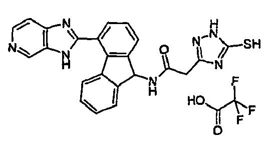 | B |
| 43 | 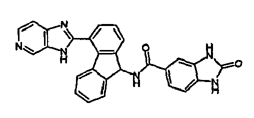 | C | 44 | 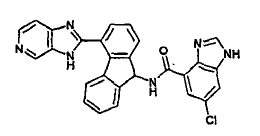 | B |
| 45 | 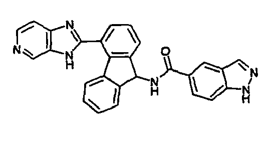 | B | 46 | 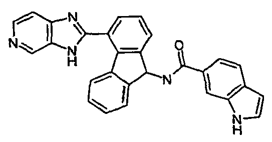 | B |
| 47 | 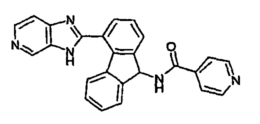 | A | 48 | 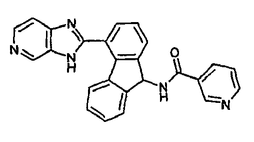 | B |
| 49 | 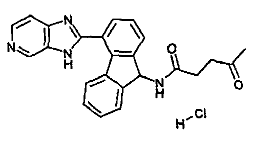 | C | 50 | 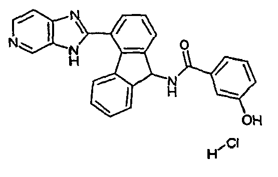 | B |
| 51 | 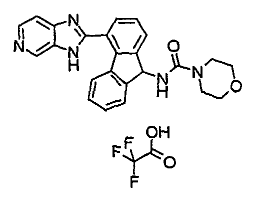 | B | 52 | 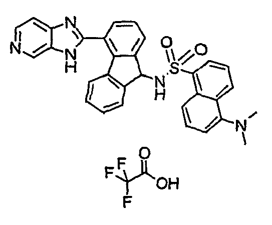 | C |
| 53 | 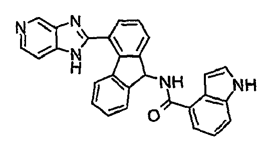 | B | 54 | 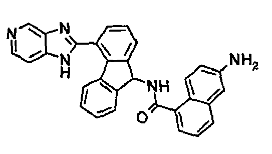 | B |
| 55 | 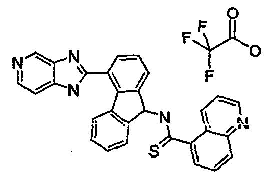 | A | 56 | 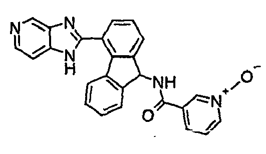 | B |
| 57 | 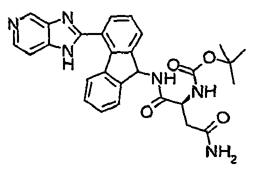 | B | 58 | 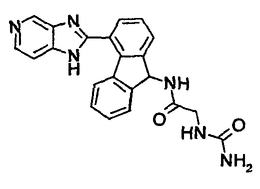 | B |
| 59 | 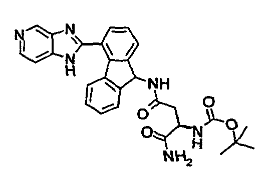 | B | 60 | 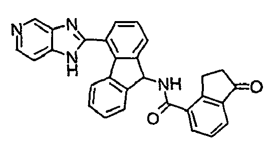 | B |
| 61 | 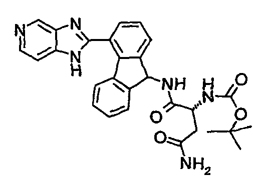 | B | 62 | 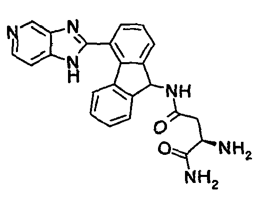 | B |
| 63 | 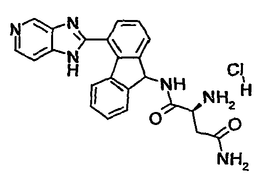 | B | 64 | 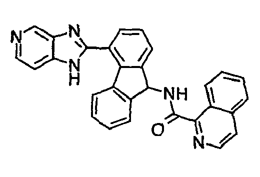 | C |
| 65 | 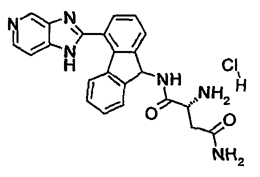 | B | 66 | 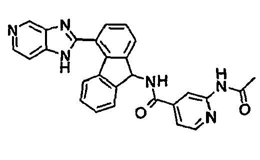 | A |
| 67 | 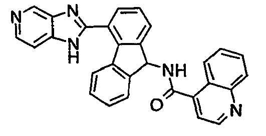 | A | 68 | 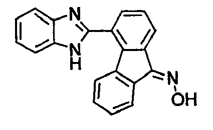 | C |
| 69 | 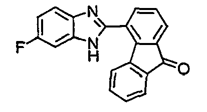 | C | 70 | 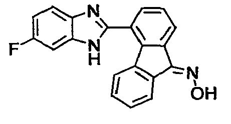 | B |
| 71 | 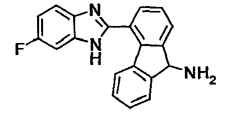 | C | 72 | 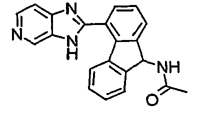 | B |
| 73 | 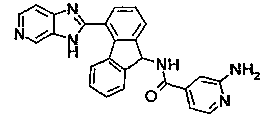 | A | 74 | 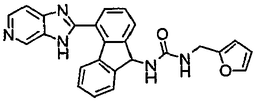 | C |
| 75 | 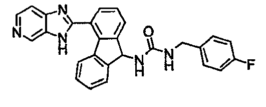 | C | 76 | 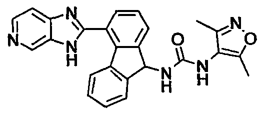 | C |
| 77 | 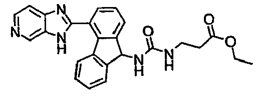 | B | 78 | 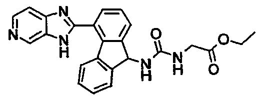 | C |
| 79 | 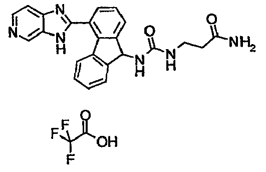 | C | 80 | 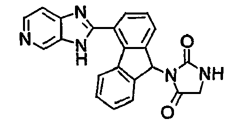 | C |
| 81 | 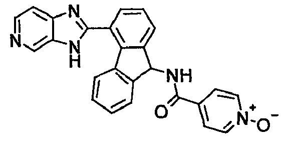 | B | 82 | 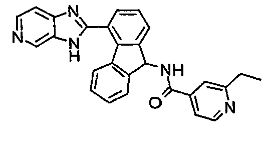 | A |
| 83 | 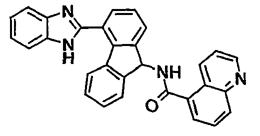 | C | 84 | 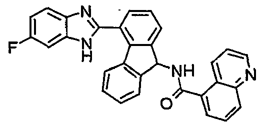 | B |
| 85 | 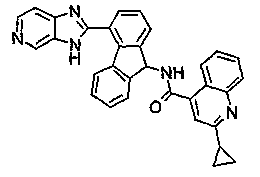 | B | 86 | 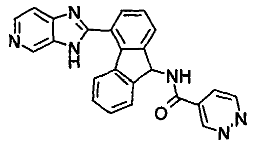 | B |
| 87 | 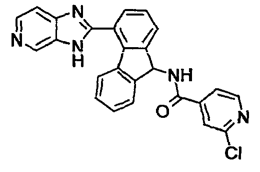 | B | 88 | 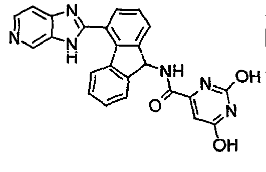 | B |
| 89 | 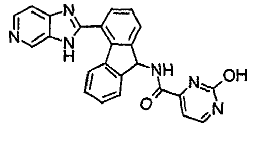 | B | 90 | 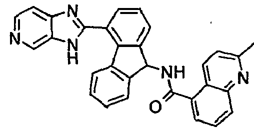 | B |
| 91 | 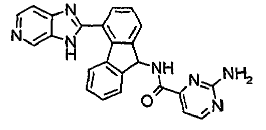 | A | 92 | 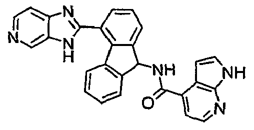 | A |
| 93 | 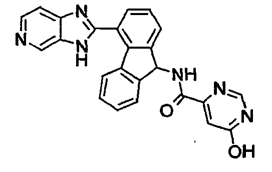 | B | 94 | 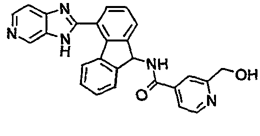 | B |
| 95 | 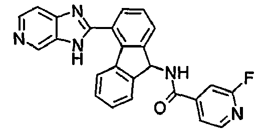 | B | 96 | 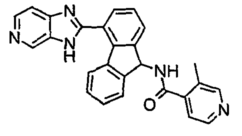 | B |
| 97 | 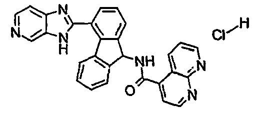 | B | 98 | 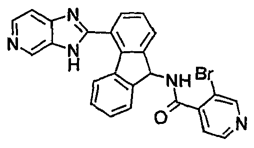 | B |
| 99 | 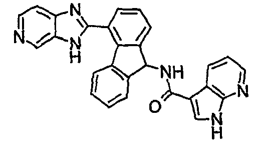 | A | 100 | 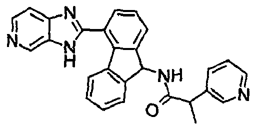 | C |
| 101 | 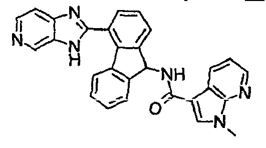 | B | 102 | 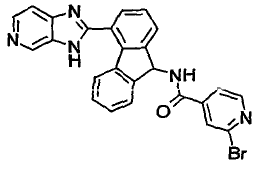 | A |
| 103 | 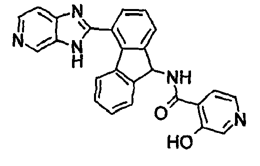 | A | 104 | 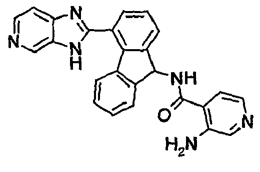 | A |
| 105 | 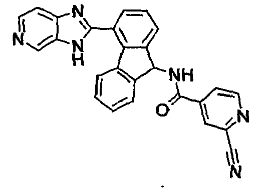 | B | 106 | 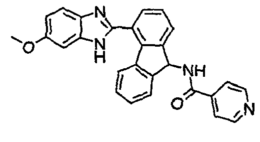 | B |
| 107 | 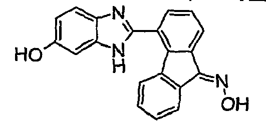 | C | 108 | 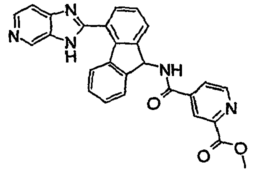 | A |
| 109 | 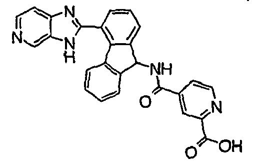 | C | 110 | 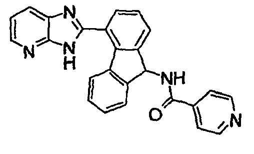 | C |
| 111 | 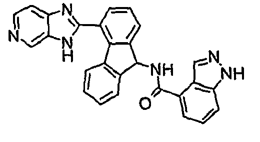 | A | 112 | 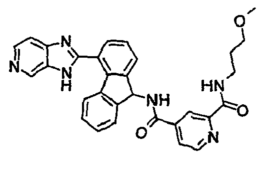 | B |
| 113 | 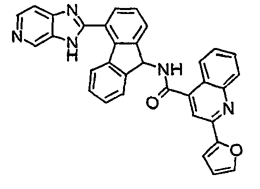 | B | 114 | 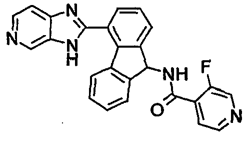 | B |
| 115 | 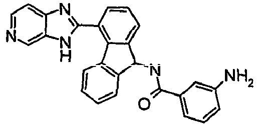 | C | 116 | 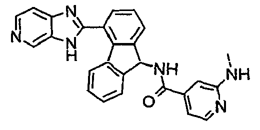 | B |
| 117 | 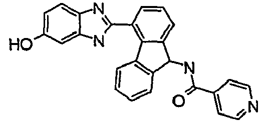 | B | 118 | 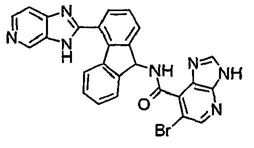 | A |
| 119 | 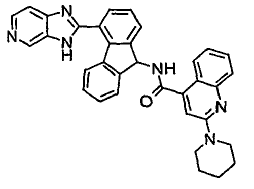 | B | 120 | 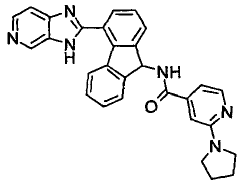 | B |
| 121 | 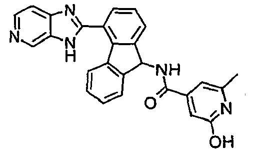 | B | 122 | 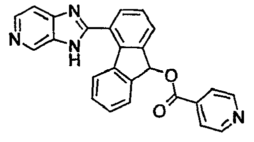 | A |
| 123 | 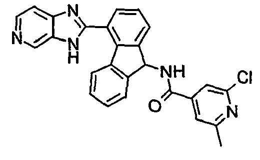 | B | 124 | 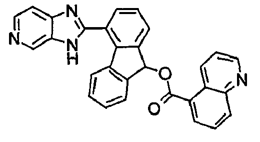 | B |
| 125 | 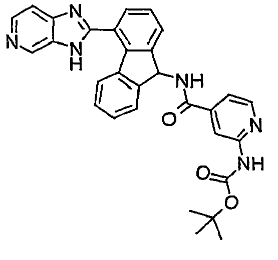 | A | 126 | 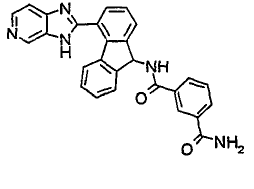 | B |

## Revendications

1. Produits de formule (I) : 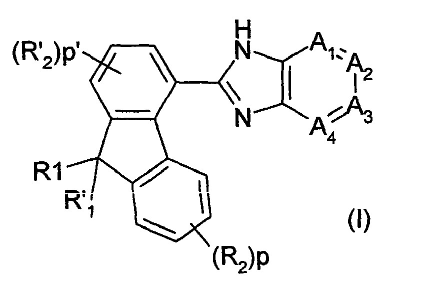 dans laquelle :
A1, A2, A3 et A4, identiques ou différents, représentent CRa ou N ou NRb; avec Rb représente alkyle, alcoxy ou OH,
R1 et R1' sont tels que : soit R1 et R1' identiques ou différents sont tels que l'un de R1 et R1' représente un atome d'hydrogène ou d'halogène ou un radical choisi parmi C1C3-alkyle, C1C3-alcoxy, alkyl-OH, CF3, cyano, carboxy et carboxamido ;
et l'autre de R1 et R1' est choisi dans le groupe constitué par H ; halogène ; CF3 ; hydroxyle ; mercapto ; nitro ; amino ; NH-OH ; NH-CO-H ; NH-CO-OH, NH-CO-Oalkyle , NH-CO-NH2 ; carboxy ; CN ; CO-NH2 ; X-(CH2)m-alkyle; X-(CH2)m-cycloalkyle ; X-(CH2)m-hétérocycloalkyle ; X-(CH2)m-aryle ou X-(CH2)m-hétéroaryle avec X = simple liaison, CH2, CH=CH, CH2-O, CH2-NH, CH2-C(O), CH2-C(O)-O, CH2-C(O)-NH, CH2-NH-(CO), CH2-NH-S(O), CH2-NH-S(O)2, O, S, NH, O-C(O), C(O)-NH, -NH-C(O), -NH-C(O)-C(O)-, -NH-C(O)-NH-, ; NH-CS, NH-S(O) ou NH-S(O)2, -NH-CO-CH2-O- ; -NH-CO-CH2-S-CH2-CO-NH- ; -NH-CO-(CH2)2-S02- ; -NH-CO-CH2-N(CH3)-CO- ; avec m = 0, 1 ou 2, tous les radicaux alkyle, cycloalkyle, hétérocycloalkyle, aryle et hétéroaryle étant éventuellement substitués, le radical cycloalkyle contenant de 3 à 10 chainons, le radical aryle contenant de 6 à 10 chainons, et les radicaux hétérocycloalkyle et hétéroaryle, éventuellement substitués, contenant de 4 à 10 chainons dont 1 à 4 hétéroatomes choisis parmi O, S, N ou NR3 avec R3 représente H ou alkyle lui-même éventuellement substitué, soit R1 et R'1 forment ensemble avec l'atome de carbone auquels ils sont liés un radical =O ; =S ; =N-OH ; =N-NH2 ; =N-NH-CO-NH2, =CH-OH ; =Y1-(CH2)m-aryle ou =Y1-(CH2)m -hétéroaryle, dans lequel Y1 représente CH, CH-CO-, CH-CO-NH, N, N-O ou N-NH-, avec m = 0, 1 ou 2 et dans lesquels aryle et hétéroaryle sont tels que définis ci-dessus et éventuellement substitués,
soit R1 et R'1 forment ensemble avec l'atome de carbone auquels ils sont liés un cycle partiellement saturé constitué de 4 à 6 chainons et contenant éventuellement de 1 à 3 hétéroatomes choisis parmi O, S, N ou NR4 avec R4 représente H ou alkyle lui-même éventuellement substitué,
R2 et R'2, identiques ou différents, sont indépendamment sélectionnés dans le groupe constitué par H, halogène, CF3, nitro, cyano, alkyle, hydroxy, mercapto, amino, alkylamino, dialkylamino, alkoxy, alkylthio (méthylthio), carboxy libre ou estérifié par un radical alkyle , carboxamide, CO-NH(alkyl) et CO N(alkyl)2, tous les radicaux alkyle, alcoxy et alkylthio étant éventuellement substitués,
p et p' identiques ou différents représentent respectivement les entiers 1 à 4 et 1 à 3 ;
Ra est sélectionné dans le groupe constitué par H ; halogène ; CF3 ; hydroxy; OCF3; S02-NH2 ; S02-NH(alk), S02-N(alk)2 ; mercapto ; nitro ; amino ; NH(alk); N(alk)2; NH-OH ; NH-CO-H ; NH-CO-NH2 ; carboxy libre ou estérifié par un radical alkyle lui-même éventuellement substitué; CN; CO-NH2 ; Y-(CH2)n-alkyle ; Y-(CH2)n-cycloalkyle, Y-(CH2)n-hétérocycloalkyle, Y-(CH2)n-aryle ou Y-(CH2)n-hétéroaryle, avec Y représente une simple liaison ou bien = O, S, NH, O-C(O), C(O)-NH, -C(O)N(CH3)- ; CO ; NH-C(O), NH-S(O) ou NH-S(O)2, avec n = 0, 1, 2, ou 3 radicaux dans lesquels les radicaux alkyle, cycloalkyle, hétérocycloalkyle, aryle et hétéroaryle sont éventuellement substitués, le radical cycloalkyle contient de 3 à 10 chainons, le radical aryle contient de 6 à 10 chainons, et les radicaux hétérocycloalkyle et hétéroaryle, éventuellement substitué, contiennent de 4 à 10 chainons dont 1 à 4 hétéroatomes choisis parmi O, S, N ou NR3 avec R3 représente H ou alkyle lui-même éventuellement substitué,
tous les radicaux alkyle, alcoxy, alkylthio, cycloalkyle, hétérocycloalkyle, aryle et hétéroaryle des substituants des produits de formule (I) étant éventuellement substitués,
lesdits produits de formule (I) étant sous toutes les formes tautomères et isomères possibles
racémiques, énantiomères et diastéréo-isomères, ainsi que les sels d'addition avec les acides minéraux et organiques ou avec les bases minérales et organiques desdits produits de formule (I).

2. Produits de formule (I) tels que définis à la revendication 1 dans lesquels
A1, A2, A3 et A4, identiques ou différents, représentent CRa ou N ou NRb avec Rb représente CH3 ou OH ;
Ra est sélectionné dans le groupe constitué par H ;halogène ; CF3 ; hydroxy ; OCF3; SO2-NH2 ; SO2-NHCH3, S02-N(CH3)2; mercapto ; nitro; amino ; NH(CH3); N(CH3)2; NH-OH ; NH-CO-H ; NH-CO-NH2; carboxy libre ou estérifié par un radical alkyle lui-même éventuellement substitué; CO2-CH3 ; CO2-(CH2)3-N(CH3)2 ; CN ; CO-NH2 ; Y-(CH2)n-alkyle ; Y-(CH2)n-cycloalkyle, Y-(CH2)n-hétérocycloalkyle, Y-(CH2)n-aryle ou Y-(CH2)n-hétéroaryle, avec Y représente une simple liaison ou bien = O, -C(O)-NH-, - C(O)N(CH3)- ; CO, avec n = 0, 1, 2, ou 3 radicaux dans lesquels les radicaux alkyle, cycloalkyle, hétérocycloalkyle, aryle et hétéroaryle sont éventuellement substitués, le radical cycloalkyle contient de 3 à 10 chainons, le radical aryle contient de 6 à 10 chainons, et les radicaux hétérocycloalkyle et hétéroaryle, éventuellement substitué, contiennent de 4 à 10 chainons dont 1 à 4 hétéroatomes choisis parmi O, S, N ou NR3 avec R3 représente H ou alkyle lui-même éventuellement substitué,
les autres substituants R1,R1',R2,R2' desdits produits de formule (I) étant choisis parmi l'une quelconque des définitions ci-dessus,
tous les radicaux alkyle, alcoxy, alkylthio, cycloalkyle, hétérocycloalkyle, aryle et hétéroaryle des substituants des produits de formule (I) étant éventuellement substitués,
lesdits produits de formule (I) étant sous toutes les formes tautomères et isomères possibles racémiques, énantiomères et diastéréo-isomères, ainsi que les sels d'addition, avec les acides minéraux et organiques ou avec les bases minérales et organiques desdits produits de formule (I).

3. Produits de formule (I) tels que définis à la revendication 1 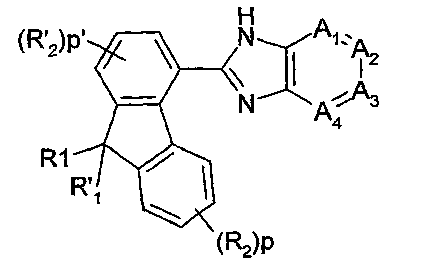 dans lesquels :
A1, A2, A3 et A4, identiques ou différents, représentent CRa ou N ;
R1 et R1' sont tels que : soit R1 et R1' identiques ou différents sont tels que l'un de R1 et R1' représente un atome d'hydrogène ou d'halogène ou un radical choisi parmi C1C3-atkyle, C1C3-alcoxy, alkyl-OH, CF3, cyano, carboxy et carboxamido ;
et l'autre de R1 et R1' est choisi dans le groupe constitué par H ; halogène CF3 ; hydroxyle ; mercapto ; nitro ; amino ; NH-OH ; NH-CO-H ; NH-CO-OH, NH-CO-Oalkyle , NH-CO-NH2 ; carboxy ; CN ; CO-NH2 ; X-(CH2)m-alkyle; X-(CH2)m-cycloalkyle; X-(CH2)m-hétérocycloalkyle ; X-(CH2)m-aryle ou X-(CH2)m-hétéroaryle avec X = simple liaison, CH2, CH=CH, CH2-O, CH2-NH, CH2-C(O), CH2-C(O)-O, CH2-C(O)-NH, CH2-NH-(CO), CH2-NH-S(O), CH2-NH-S(O)2, O, S, NH, O-C(O), C(O)-NH, -NH-C(O), -NH-C(O)-C(O)-, -NH-C(O)-NH-, ; NH-CS, NH-S(O) ou NH-S(O)2, avec m = 0, 1 ou 2, tous les radicaux alkyle, cycloalkyle, hétérocycloalkyle, aryle et hétéroaryle étant éventuellement substitués, le radical cycloalkyle contenant de 3 à 10 chainons, le radical aryle contenant de 6 à 10 chainons, et les radicaux hétérocycloalkyle et hétéroaryle, éventuellement substitués, contenant de 4 à 10 chaînons dont 1 à 4 hétéroatomes choisis parmi O, S, N ou NR3 avec R3 représente H ou alkyle lui-même éventuellement substitué,
soit R1 et R'1 forment ensemble avec l'atome de carbone auquels ils sont liés un radical =O ; =S ; =N-OH ; =N-NH2 ; =N-NH-CO-NH2, =CH-OH ; =Y1-(CH2)m-aryle ou =Y1-(CH2)m -hétéroaryle, dans lequel Y1 représente CH, CH-CO-, CH-CO-NH, N, N-O ou N-NH-, avec m = 0, 1 ou 2 et dans lesquels aryle et hétéroaryle sont tels que définis ci-dessus et éventuellement substitués,
soit R1 et R'1 forment ensemble avec l'atome de carbone au quels ils sont liés un cycle partiellement saturé constitué de 4 à 6 chaînons et contenant éventuellement de 1 à 3 hétéroatomes choisis parmi O, S, N ou NR4 avec R4 représente H ou alkyle lui-même éventuellement substitué,
R2 et R'2, identiques ou différents, sont indépendamment sélectionnés dans le groupe constitué par H, halogène, CF3, nitro, cyano, alkyle, hydroxy, mercapto, amino, alkylamino, dialkylamino, alkoxy, alkylthio (méthylthio), carboxy libre ou estérifié, carboxamide, CO-NH(alkyl) et CO N(alkyl)2,
p et p' identiques ou différents représentent respectivement les entiers 1 à 4 et 1 à 3 ;
Ra est sélectionné dans le groupe constitué par H ; halogène ; CF3 ; hydroxy ; mercapto ; nitro ; amino ; NH-OH ; NH-CO-H ; NH-CO-NH2 ; carboxy ; CN ; CO-NH2 ; Y-(CH2)n-alkyle ; Y-(CH2)n-cycloalkyle, Y-(CH2)n-hétérocycloalkyle, Y-(CH2)n-aryle ou Y-(CH2)n-hétéroaryle, avec Y = O, S, NH, O-C(O), C(O)-NH, NH-C(O), NH-S(O) ou NH-S(O)2, avec n = 0, 1, 2, ou 3 radicaux dans lesquels les radicaux alkyle, cycloalkyle, hétérocycloalkyle, aryle et hétéroaryle sont éventuellement substitués, le radical cycloalkyle contient de 3 à 10 chaînons, le radical aryle contient de 6 à 10 chainons, et les radicaux hétérocycloalkyle et hétéroaryle, éventuellement substitué, contiennent de 4 à 10 chaînons dont 1 à 4 hétéroatomes choisis parmi O, S, N ou NR3 avec R3 représente H ou alkyle lui-même éventuellement substitué,
tous les radicaux alkyle, alcoxy, alkylthio, cycloalkyle, hétérocycloalkyle, aryle et hétéroaryle des substituants des produits de formule (I) étant éventuellement substitués,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

4. Produits de formule (I) tels que définis à la revendication 1 dans lesquels
A1, A2, A3 et A4, identiques ou différents, représentent CRa ou N ;
Ra est sélectionné dans le groupe constitué par H ; halogène ; hydroxy ; mercapto; amino ; Y-(CH2)n-alkyle ; Y-(CH2)n-cycloalkyle, Y-(CH2)n-hétérocycloalkyle, Y-(CH2)n-aryle ou Y-(CH2)n-hétéroaryle, avec Y = O, avec n = 0, 1, 2, ou 3, radicaux dans lesquels tous les radicaux alkyle, cycloalkyle, hétérocycloalkyle, aryle et hétéroaryle sont éventuellement substitués et les radicaux hétérocycloalkyle et hétéroaryle contiennent de 4 à 10 chainons dont 1 à 4 hétéroatomes choisis parmi O, S, N ou NR3 avec R3 représente H ou alkyle lui-même éventuellement substitué,
les autres substituants R1,R1',R2,R2' desdits produits de formule (I) étant choisis parmi l'une quelconque des définitions ci-dessus,
lesdits produits de formule (1) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (1).

5. Produits de formule (1) tels que définis à l'une quelconque des autres revendications dans lesquels A1, A2, A3 et A4, identiques ou différents, représentent CRa ou N ;
Ra est sélectionné dans le groupe constitué par H ; halogène ; hydroxy ; Y-(CH2)n-alkyle ; Y-(CH2)n-cycloalkyle, Y-(CH2)n-hétérocycloalkyle, Y-(CH2)n-aryle ou Y-(CH2)n-hétéroaryle, avec Y = O, et avec n = 2 ou 3, tous les radicaux alkyle, cycloalkyle, hétérocycloalkyle, aryle et hétéroaryle étant éventuellement substitués, et les radicaux hétérocycloalkyle et hétéroaryle, contenant de 4 à 10 chainons dont 1 à 4 hétéroatomes choisis parmi O, S, N ou NR3 avec R3 représente H ou alkyle lui-même éventuellement substitué, les autres substituants R1,R1',R2,R2' desdits produits de formule (I) étant choisis parmi l'une quelconque des définitions ci-dessus,
lesdits produits de formule (I) étant sous toutes les formes formes tautomères et isomères possibles racémiques, énantiomères et diastéréo-isomères, ainsi que les sels d'addition avec les acides minéraux et organiques ou avec les bases minérales et organiques desdits produits de formule (I).

6. Produits de formule (I) tels que définis à l'une quelconque des autres revendications dans lesquels A1, A2, A3 et A4 sont tels que soit tous les 4 représentent CRa soit l'un représente CRa et les trois autres, identiques ou différents, représentent N ou CRa, avec Ra représente H ; halogène ; hydroxy ou alcoxy;
les autres substituants R1,Rl',R2,R2' desdits produits de formule (I) étant choisis parmi l'une quelconque des définitions ci-dessus,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

7. Produits de formule (I) tels que définis à l'une quelconque des autres revendications dans lesquels R2 et R'2, identiques ou différents, sont indépendamment sélectionnés dans le groupe constitué par H, halogène, méthyle, éthyle, amino, méthoxy, CH2-NH2, CH2-NHalk, CH2-N(alk)2, CH2-OH, CH2-Oalk,.
p et p' identiques ou différents représentent respectivement les entiers 1 à 4 et 1 à 3;
les autres substituants A1, A2, A3, A4, R1 et R1' desdits produits de formule (I) étant choisis parmi l'une quelconque des définitions ci-dessus,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

8. Produits de formule (I) tels que définis à l'une quelconque des autres revendications dans lesquels R2 et R'2, identiques ou différents, sont indépendamment sélectionnés dans le groupe constitué par H, halogène, méthyle, éthyle, amino, méthoxy,.
p et p' identiques ou différents représentent respectivement les entiers 1 à 4 et 1 à 3 ;
les autres substituants A1, A2, A3, A4, R1 et R1' desdits produits de formule (I) étant choisis parmi l'une quelconque des définitions ci-dessus,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

9. Produits de formule (I) tels que définis à l'une quelconque des autres revendications dans lesquels R2 et R'2, identiques ou différents, sont indépendamment sélectionnés dans le groupe constitué par H, méthyle,
p et p' identiques ou différents représentent respectivement les entiers 1 à 4 et 1 à 3 ;
les autres substituants A1, A2, A3, A4, R1 et R1' desdits produits de formule (I) étant choisis parmi l'une quelconque des définitions ci-dessus,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

10. Produits de formule (I) tels que définis à l'une quelconque des autres revendications dans lesquels :
R1 et R1' sont tels que : soit R1 et R1' identiques ou différents sont tels que l'un de R1 et R1' représente un atome d'hydrogène,
et l'autre de R1 et R1' est choisi dans le groupe constitué par H ; halogène ; hydroxyle; amino ; NH-CO-H ; NH-CO-OH, NH-CO-Oalkyle , NH-CO-NH2 ; carboxy; CO-NH2 ; X-(CH2)m-alkyle; X-(CH2)m-cycloalkyle ; X-(CH2)m-hétérocycloalkyle; X-(CH2)m-aryle et X-(CH2)m-hétéroaryle, avec X = simple liaison, CH2, CH=CH, CH2-C(O), NH, O-C(O), C(O)-NH, -NH-C(O), -NH-C(O)-C(O)-, -NH-C(O)-NH-, ; NH-CS, NH-S(O) ou NH-S(O)2, avec m = 0, tous les radicaux alkyle, cycloalkyle, hétérocycloalkyle, aryle et hétéroaryle étant éventuellement substitués, le radical cycloalkyle contenant de 3 à 10 chainons, le radical aryle contenant de 6 à 10 chainons, et les radicaux hétérocycloalkyle et hétéroaryle, éventuellement substitués contenant de 4 à 10 chainons dont 1 à 4 hétéroatomes choisis parmi O, S, N ou NR3 avec R3 représente H ou alkyle lui-même éventuellement substitué,
soit R1 et R'1 forment ensemble avec l'atome de carbone auquels ils sont liés un radical =O ; =S ; =N-OH ; =N-NH2 ; =N-NH-CO-NH2, =CH-OH ; =Y1-(CH2)m-aryle ou =Y1-(CH2)m -hétéroaryle, dans lequel Y1 représente CH, CH-CO-,
CH-CO-NH, N, N-O ou N-NH-, avec m = 0, 1 ou 2 et dans lesquels aryle, et hétéroaryle sont tels que définis ci-dessus et éventuellement substitués,
soit R1 et R'1 forment ensemble avec l'atome de carbone auquels ils sont liés un hétérocycle partiellement saturé constitué de 5 à 6 chainons et contenant de 1 à 3 hétéroatomes choisis parmi O, S, N ou NR4 avec R4 représente H ou alkyle lui-même éventuellement substitué,
les autres substituants A1, A2, A3, A4, R2 et R2' desdits produits de formule (I) étant choisis parmi l'une quelconque des définitions ci-dessus,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

11. Produits de formule (I) tels que définis à la revendication 1 dans lesquels
A1, A2, A3 et A4, identiques ou différents, représentent CRa ou N ou NRb avec Rb représente CH3 ou OH ;
Ra est sélectionné dans le groupe constitué par H ; CH3; CH2-NH2 ; halogène ; CF3 ; hydroxy ; OCF3; SO2-NH2 ; S02-N(CH3)2; mercapto ; nitro; amino ; NH(CH3); N(CH3)2; NH-OH ; NH-CO-H ; NH-CO-NH2 ; carboxy libre ou estérifié par un radical alkyle lui-même éventuellement substitué; CO2-CH3 ; CO2-(CH2)3-N(CH3)2 ; CN ; CO-NH2 ; CO-N(CH3)2 ; CO-CH3, CO-(CH2)3-O-CH3) ; morpholinyle ; pipérazinyl-CH3; imidazolinyl-CH3; diazépin-CH3 ; -CO-pipérazinyl-CH3 ; -CO-pyrrolidinyle; Y-(CH2)n-alkyle ; Y-(CH2)n-cycloalkyle, Y-(CH2)n-hétérocycloalkyle, Y-(CH2)n-aryle ou Y-(CH2)n-hétéroaryle, avec Y représente une simple liaison ou bien = O, -C(O)-NH-, - C(O)N(CH3)- ; CO, avec n = 0, 1, 2, ou 3 radicaux dans lesquels les radicaux alkyle, cycloalkyle, hétérocycloalkyle, aryle et hétéroaryle sont éventuellement substitués, le radical cycloalkyle contient de 3 à 10 chainons, le radical aryle contient de 6 à 10 chainons, et les radicaux hétérocycloalkyle et hétéroaryle, éventuellement substitué, contiennent de 4 à 10 chainons dont 1 à 4 hétéroatomes choisis parmi O, S, N ou NR3 avec R3 représente H ou alkyle lui-même éventuellement substitué,
R1 et R1' sont tels que : soit l'un de R1 et R1' représente un atome d'hydrogène
et l'autre de R1 et R1' est choisi dans le groupe constitué par X-(CH2)m-hétérocycloalkyle, X-(CH2)m-aryle et X-(CH2)m-hétéroaryle, et notammment X-(CH2)m-hétéroaryle , avec X représente -O-C(O), -NH-C(O), NH-CS, - NH-CO-CH2-O- ; -NH-CO-CH2-S-CH2-CO-NH- ; -NH-CO-(CH2)2-SO2-; - NH-CO-CH2-N(CH3)-CO- ; et m = 0,
soit R1 et R'1 forment ensemble avec l'atome de carbone auquels ils sont liés un radical =N-OH ou =N-NH₂,
R2 et R'2, identiques ou différents, sont indépendamment sélectionnés dans le groupe constitué par H, halogène, méthyle, éthyle, amino, méthoxy, CH2-NH2, CH2-NHalk, CH2-N(alk)2, CH2-OH, CH2-Oalk,.
p et p' identiques ou différents représentent respectivement les entiers 1 à 4 et 1 à 3 ;
tous les radicaux alkyle, alcoxy, alkylthio, cycloalkyle, hétérocycloalkyle, aryle et hétéroaryle des substituants des produits de formule (I) étant éventuellement substitués,
) lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

12. Produits de formule (I) tels que définis à l'une quelconque des autres revendications dans lesquels :
R1 et R1' sont tels que : soit l'un de R1 et R1' représente un atome d'hydrogène
et l'autre de R1 et R1' est choisi dans le groupe constitué par X-(CH2)m-hétérocycloalkyle, X-(CH2)m-aryle et X-(CH2)m-hétéroaryle, et notammment X-(CH2)m-hétéroaryle , avec X représente -O-C(O), -NH-C(O) ou NH-CS et m=0,
soit R1 et R'1 forment ensemble avec l'atome de carbone auquels ils sont liés un radical =N-OH ou =N-NH2,
les radicaux hétérocycloalkyle, aryle et hétéroaryle étant éventuellement substitués,
les autres substituants A1, A2, A3, A4, R2 et R2' desdits produits de formule (I) étant choisis parmi l'une quelconque des définitions ci-dessus,
lesdits produits de formule (I) étant sous toutes les formes tautomères et
isomères possibles racémiques, énantiomères et diastéréo-isomères, ainsi que les sels d'addition avec les acides minéraux et organiques ou avec les bases minérales et organiques desdits produits de formule (I).

13. Produits de formule (I) telle que définis à l'une quelconque des revendications précédentes dont les noms suivent :
- la 4-(3H-imidazo[4,5-c]pyridin-2-yl)-9H-fluorène-9-one oxime (Z, E).
- la N-[4-(1H-imidazo[4,5-c]pyridin-2-yl)-9H-fluorèn-9-ylidène]-hydrazine, mélange 60/40 d'isomères E et Z
- le [4-(3H-imidazo[4,5-c]pyridin-2-yl)-9H-fluorèn-9(R,S)-yl]-amide de l'acide quinoléine-5-carboxylique.
- énantiomère dextrogyre du [4-(3H-imidazo[4,5-c]pyridin-2-yl)-9H-fluorèn-9(R,S)-yl]-amide de l'acide quinoléine-5-carboxylique
- la 4-(3H-lmidazo[4,5-c]pyridin-2-yl)-9H-fluorène-9-one oxime (E).
- le trifluoroacétate du [4-(3H-imidazo[4,5-c]pyridin-2-yl)-9H-fluorèn-9(R,S)-yl]-amide de l'acide 1H-pyrazole-4-carboxylique
- le trifluoroacétate du N-[4-(3H-imidazo[4,5-c]pyridin-2-yl)-9H-fluorèn-9(R,S)-yl]-succinamide.
- le trifluoroacétate du [4-(3H-imidazo[4,5-c]pyridin-2-yl)-9H-fluorèn-9(R,S)-yi]-amide de l'acide 1H-benzotriazole-5-carboxylique.
- ie [4-(3H-imidazo[4,5-c]pyridin-2-yl)-9H-fluorèn-9(R,S)-yl]-amide de l'acide 1H-indazole-5-carboxylique
- le [4-(3H-imidazo[4,5-c]pyridin-2-yl)-9H-fluorèn-9(R,S)-yl]-amide de l'acide 1H-indole-6-carboxylique
- le N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide 1H-indole-4-carboxylique
- le trifluoroacétate du [4-(3H-imidazo[4,5-c]pyridin-2-yl)-9H-fluorèn-9(R,S)-yl]-amide de l'acide quinoléine-5-carbothioique
- le 2-acétylamino-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide quinoléine-4-carboxylique
- le 2-amino-N-[4-(3H-imidazo[4,5-c]pyridin-2-yl)-9H-fluorèn-9(R,S)-yl]-isonicotinamide.
- le 2-éthyl-N-[4-(3H-imidazo[4,5-c]pyridin-2-yl)-9H-fluorèn-9(R,S)-yl]-isonicotinamide
- le [4-(6-fluoro-1H-benzimidazol-2-yi)-9H-fluorèn-9(R,S)-yl]-amide de l'acide quinoléine-5-carboxylique
- le 2-chloro-N-[4-(3H-imidazo[4,5-c]pyridin-2-yl)-9H- fluorèn-9(R,S)-yl]-isonicotinamide
- le [4-(3H-imidazo[4,5-c]pyridin-2-yl)-9H-fluoren-9(R,S)-yl]-amide de l'acide 2-amino-pyrimidine-4-carboxylique
- le [4-(3H-imidazo[4,5-c]pyridin-2-yl)-9H-fluorèn-9(R,S)-yl]-amide de l'acide 1H-pyrrolo[2,3-b]pyridine-4-carboxylique
- le 2-hydroxyméthyl-N-[4-(3H-imidazo[4,5-c]pyridin-2-yl)-9H-fluorèn-9(R,S)-yl]-isonicotinamide
- le N-[4-(3H-imidazo[4,5-c]pyridin-2-yl)-9H-fluorèn-9(R,S)-yl]-3-méthyl-isonicotinamide
- le chlorhydrate du [4-(3H-imidazo[4,5-c]pyridin-2-yl)-9H-fluorèn-9(R,S)-yl]-amide de l'acide 1,8-naphtyridine-4-carboxylique
- ie [4-(3H-imidazo[4,5-c]pyridin-2-yl)-9H-fiuorèn-9(R,S)-yl]-amide de l'acide 1H-pyrrolo[2,3-b]pyridine-3-carboxylique
- le [4-(3H-imidazo[4,5-c]pyridin-2-yl)-9H-fluorèn-9(R,S)-yl]-amide de l'acide 1-méthyl-1H-pyrrolo[2,3-b]pyridine-3-carboxylique
- le 2-bromo-N-[4-(3H-imidazo[4,5-c]pyridin-2-yl)-9H-fluorèn-9(R,S)-yl]-isonicotinamide
- le 3-hydroxy-N-[4-(3H-imidazo[4,5-c]pyridin-2-yl)-9H-fluorèn-9(R,S)-yl]-isonicotinamide
- le 3-amino-N-[4-(3H-imidazo[4,5-c]pyridin-2-yl)-9H-fluorèn-9(R,S)-yl]-isonicotinamide
- ester de méthyle de l'acide 4-[4-(3H-imidazo[4,5-c]pyridin-2-yl)-9H-fluorèn-9(R,S)-ylcarbamoyl]-pyridine-2-carboxylique
- le [4-(3H-imidazo[4,5-c]pyridin-2-yl)-9H-fluorèn-9(R,S)-yl]-amide de l'acide 1H-indazole-4-carboxylique
- le N-[4-(3H-imidazo[4,5-c]pyridin-2-yl)-9H-fluorèn-9(R,S)-yl]-2-méthylamino-isonicotinamide
- le N-[4-(6-hydroxy-1H-benzimidazol-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide 6-bromo-3H-imidazo[4,5-b]pyridine-7-carboxylique
- ester de 4-(3H-imidazo[4,5-c]pyridin-2-yl)-9H-fluoren-9(R,S)-yle de l'acide isonicotinique,
- le N-[4-(3H-imidazo[4,5-c]pyridin-2-yl)-9H-fluorèn-9(R,S)-yl]-amide de l'acide 2-tert-butoxycarbonylamino-isonicotinique.
- le N-[4-(3H-imidazo[4,5-c]pyridin-2-yl)-9H-fluorèn-9(R,S)-yl]-amide de l'acide 3-chloro-6-méthoxy-quinoléine-4-carboxylique.
- le N-[4-(3H-imidazo[4,5-c]pyridin-2-yl)-9H-fluorèn-9(R,S)-yl]-amidede l'acide 3-hydroxy-quinoléine-4-carboxylique.
- le N-[4-(3H-imidazo[4,5-c]pyridin-2-yl)-9H-fluorèn-9(R,S)-yl]-amide de l'acide 2-amino-5-chloro-pyrimidine-4-carboxylique.
- le [4-(3H-imidazo[4,5-c]pyridin-2-yl)-9H-fluorèn-9(R,S)-yl]-amide de l'acide 3-bromo-1H-pyrrolo[2,3-b]pyridine-4-carboxylique.
- l'énantiomère dextrogyre du [4-(3H-imidazo[4,5-c]pyridin-2-yl)-9H-fluorèn-9(R,S)-yl]-amide de l'acide 1H-pyrrolo[2,3-b]pyridine-4-carboxylique.
- le N-[4-(3H-imidazo[4,5-c]pyridin-2-yl)-9H-fluorèn-9(R,S)-yl]-amide de l'acide 9H-purine-6-carboxylique.
- le N-[4-(3H-imidazo[4,5-c]pyridin-2-yl)-9H-fluorèn-9(R,S)-yl]-amide de l'acide 2-amino-6-méthyl-pyrimidine-4-carboxylique.
- le [4-(3H-imidazo[4,5-c]pyridin-2-yl)-9H-fluorène-9(R,S)-yl]-amide de l'acide 5-amino-3H-1,2,3-triazole-4-carboxylique.
- le N-[4-(3H-imidazo[4,5-c]pyridin-2-yl)-9H-fluorèn-9(R,S)-yl]-amide de l'acide 6-méthyl-2-méthylamino-pyrimidine-4-carboxylique.
- le N-[4-(3H-imidazo[4,5-c]pyridin-2-yl)-9H-fluorèn-9(R,S)-yl]-amide de l'acide 6-méthoxy-quinoléine-4-carboxylique.
- le 3,5-dihydroxy-N-[4-(3H-imidazo[4,5-c]pyridin-2-yl)-9H-fluorèn-9(R,S)-yl]-benzamide.
- le N-[4-(3H-imidazo[4,5-c]pyridin-2-yl)-9H-fluorèn-9(R,S)-yl]-amide de l'acide pyrimidine-4-carboxylique.
- le 4-hydroxy-N-[4-(3H-imidazo[4,5-c]pyridin-2-yl)-9H-fluorèn-9(R,S)-yl]-benzamide.
- le 2,4-dihydroxy-N-[4-(3H-imidazo[4,5-c]pyridin-2-yl)-9H-fluorèn-9(R,S)-yl]-benzamide.
- le [4-(5-cyano-6-fluoro-1H-benzimidazol-2-yl)-9H-fluorén-9(R,S)-yl]-amide de l'acide 1H-pyrrolo[2,3-b]pyridine-4-carboxylique.
- le 4-(1H-benzimidazol-2-yl)-9H-fluorène-9(R,S)-yl]-amide de l'acide 2-amino-5-chloro-pyrimidine-4-carboxylique.
- le [4-(5-fluoro-1H-benzimidazol-2-yl)-9H-fluorène-9(R,S)-yl]-amide de l'acide 2-amino-5-chloro-pyrimidine-4-carboxylique.
- le [4-(5-fluoro-1H-benzimidazol-2-yl)-9H-fluorène-9(R,S)-yl]-amide de l'acide 1H-pyrrolo[2,3-b]pyridine-4-carboxylique.
- le [4-(3H-imidazo[4,5-c]pyridin-2-yl)-9H-fluorène-9(R,S)-yl]-amide de l'acide 2,3-diméthyl-quinoxaline-5-carboxylique.
- le [4-(3H-imidazo[4,5-c]pyridin-2-yl)-9H-fluorène-9(R,S)-yl]-amide de l'acide 3-amino-1H-pyrazole-4-carboxylique.
- l'énantiomère dextrogyre du N-[4-(3H-imidazo[4,5-c]pyridin-2-yl)-9H-fluorèn-9(R,S)-yl]-amide de l'acide 2-amino-5-chloro-pyrimidine-4-carboxylique.
- l'énantiomère lévogyre du N-[4-(3H-imidazo[4,5-c]pyridin-2-yl)-9H-fluorèn-9(R,S)-yl]-amide de l'acide 2-amino-5-chloro-pyrimidine-4-carboxylique.
- le [4-(3H-imidazo[4,5-c]pyridin-2-yl)-9H-fluorène-9(R,S)-yl]-amide de l'acide 3-méthyl-quinoxaline-5-carboxylique.
- le [4-(3H-imidazo[4,5-c]pyridin-2-yl)-9H-fluorène-9(R,S)-yl]-amide de l'acide quinoxaline-5-carboxylique.
- le [4-(9H-purine-8-yl)-9H-fluorène-9(R,S)-yl]-amide de l'acide 1H-pyrrolo[2,3-b]pyridine-4-carboxylique
- le [4-(1H-benzimidazol-2-yl)-9H-fluorène-9(R,S)-yl]-amide de l'acide 1H-pyrrolo[2,3-b]pyridine-4-carboxylique
- le [4-(5-trifluorométhyl-1H-benzimidazol-2-yl)-9H-fluorène-9(R,S)-yl]-amide de l'acide 1H-pyrrolo[2,3-b]pyridine-4-carboxylique
- le [4-(5-méthoxycarbonyl-1 H-benzimidazol-2-yl)-9H-fluorène-9(R,S)-yl]-amide de l'acide 1H-pyrrolo[2,3-b]pyridine-4-carboxylique
- le [4-(5-carboxy-1H-benzimidazol-2-yl)-9H-fluorène-9(R,S)-yl]-amide de l'acide 1H-pyrrolo[2,3-b]pyridine-4-carboxylique
- le [4-(4-méthyl-1H-benzimidazol-2-yl)-9H-fluorène-9(R,S)-yl]-amide de l'acide 1H-pyrrolo[2,3-b]pyridine-4-carboxylique
- le [4-(5-carboxamido-1H-benzimidazol-2-yl)-9H-fluorène-9(R,S)-yl]-amide de l'acide 1H-pyrrolo[2,3-b]pyridine-4-carboxylique
- le [4-(6-sulfamoyl-1H-benzimidazol-2-yl)-9H-fluorèn-9(R,S)-yl]-amide de l'acide 1H-pyrrolo[2,3-b]pyridine-4-carboxylique
- le [4-(5-trifluorométhoxy-1H-benzimidazol-2-yl) -9H-fluorène-9(R,S)-yl]-amide de l'acide 1H-pyrrolo[2,3-b]pyridine-4-carboxylique
- le [4-(5-cyano-1H-benzimidazol-2-yl)-9H-fluorène-9(R,S)-yl]-amide de l'acide 1H-pyrrolo[2,3-b]pyridine-4-carboxylique
- le diastéréosisomère D du [4-(3H-imidazo[4,5-c]pyridin-2-yl)-9H-fluorèn-9(R,S)-yl]-amide de l'acide 2-(3-acétyl-2,2-diméthyl-cyclobutan-1-yl) acétique
- le (6-chloro-1H-benzimidazol-2-yl)-9H-fluorèn-9(R,S)-yl]-amide de l'acide 1H-pyrrolo[2,3-b]pyridine-4-carboxylique :
- le 4-(5-fluoro-6-morpholino-1H-benzimidazole-2-yl)-9H-fluorène-9(R,S)-yl]-amide de l'acide 1H-pyrrolo[2,3-b]pyridine-4-carboxylique
- le 4-(6-chloro-5-fluoro-1H-benzimidazole-2-yl)-9H-fluorène-9(R,S)-yl]-amide de l'acide 1H-pyrrolo[2,3-b]pyridine-4-carboxylique
- le [4-(4,5,6,7-tétrahydro-1H-benzimidazol-2-yl)-9H-fluorèn-9(R,S)-yl]-amide de l'acide 1H-pyrrolo[2,3-b]pyridine-4-carboxylique
- le [2-amino-5-(1H-benzimidazol-2-yl)-9H-fluorène-9(R,S)-yl]-amide de l'acide 1H-pyrrolo[2,3-b]pyridine-4-carboxylique
- le [4-(5-hydroxy-5H-imidazo[4,5-c]pyridin-2-yl)-9H-fluorèn-9(R,S)-yl]-amide de l'acide 1H-pyrrolo[2,3-b]pyridine-4-carboxylique
- le 4-(5-hydroxy-1H-benzimidazole-2-yl)-9H-fluorène-9(R,S)-yl]-amide de l'acide 1H-pyrrolo[2,3-b]pyridine-4-carboxylique
- le [4-(5-méthylaminocarbonyl-1H-benzimidazol-2-yl)-9H-fluorène-9(R,S)-yl]-amide de l'acide 1H-pyrrolo[2,3-b]pyridine-4-carboxylique
- le [4-(5-diméthylaminocarbonyl-1H-benzimidazol-2-yl)-9H-fluorène-9(R,S)-yl]-amide de l'acide 1H-pyrrolo[2,3-b]pyridine-4-carboxylique
- le [4-5-(2-diméthylamino-éthyl)aminocarbonyl-1H-benzimidazol-2-yl)-9H-fluorène-9(R,S)-yl]-amide de l'acide 1H-pyrrolo[2,3-b]pyridine-4-carboxylique
- le [4-(4,5,6,7-tétrafluoro-1H-benzimidazol-2-yl)-9H-fluorèn-9(R,S)-yl]-amide de l'acide 1H-pyrrolo[2,3-b]pyridine-4-carboxylique
- le 4-[5-(3-méthoxypropyl)aminocarbonyl-1H-benzimidazol-2-yl)-9H-fluorène-9(R,S)-yl]-amide de l'acide 1H-pyrrolo[2,3-b]pyridine-4-carboxylique
- le 4-[5-(4-méthyl-pipérazine-1-yl)carbonyl-1H-benzimidazol-2-yl)-9H-fluorène-9(R,S)-yl]-amide de l'acide 1H-pyrrolo[2,3-b]pyridine-4-carboxylique
- le [4-(6-diméthylsulfamoyl-1H-benzimidazol-2-yl)-9H-fluorèn-9(R,S)-yl]-amide de l'acide 1H-pyrrolo[2,3-b]pyridine-4-carboxylique
- le 4-[5-(pyrrolidin-1-yl)carbonyl)-1H-benzimidazol-2-yl)-9H-fluorène-9(R,S)-yl]-amide de l'acide 1H-pyrrolo[2,3-b]pyridine-4-carboxylique
- l'énantiomère dextrogyre de l'acide 2-{9(R,S)-[(1H-pyrrolo[2,3-b]pyridine-4-carbonyl)-amino]-9H-fluorèn-4-yl}-1H-benzimidazole-5-carboxylique
- le {{4-{5-[2-(pyrrolidin-1-yl)éthylaminocarbonyl]-1H-benzimidazole-2-yl}-9H-fluorène-9(R,S)-yl}}amide de l'acide 1H-pyrrolo[2,3-b]pyridine-4-carboxylique
- le [4-(6-diméthyiamino-1H-benzimidazol-2-yl)-9H-fluoren-9(R,S)-yl]-amide de l'acide 1H-pyrrolo[2,3-b]pyridine-4-carboxylique
- le {4-[6-(4-méthyl-pipérazin-1-yl)-1H-benzimidazol-2-yl]-9H-fluorèn-9(R,S)-yl}-amide de l'acide 1H-pyrrolo[2,3-b]pyridine-4-carboxylique
- le [[6-(méthyl-4(5)-imidazolin-2-yl)-1H-benzimidazol-2-yl]-9H-fluorèn-9(R,S)-yl]-amide de l'acide 1H-pyrrolo[2,3-b]pyridine-4-carboxylique
- le {{4-{5-[(3-diméthylamino-propyl)aminocarbonyl]-1H-benzimidazol-2-yl}-9H-fluorène-9(R,S)-yl}}-amide de l'acide 1H-pyrrolo[2,3-b]pyridine-4-carboxylique
- le {4-[5-fluoro-6-(4-méthyl-pipérazin-1-yl)-1H-benzimidazol-2-yl]-9H-fluorèn-9(R,S)-yl}-amide de l'acide 1H-pyrrolo[2,3-b]pyridine-4-carboxylique
- le {{4-{5-[(3-diméthylamino-propyl)carbonyloxy]-1H-benzimidazol-2-yl}-9H-fluorène-9(R,S)-yl}}-amide de l'acide 1H-pyrrolo[2,3-b]pyridine-4-carboxylique
- le [4-(6-amino-1H-benzimidazol-2-yl)-9H-fluorèn-9-(R,S)yl]-amide de l'acide 1H-pyrrolo[2,3-b]pyridine-4-carboxylique.
- le 4-(6-méthyl-1H-benzimidazol-2-yl)-9H-fluorène-9(R,S)-yl]-amide de l'acide 1H-pyrrolo[2,3-b]pyridine-4-carboxylique
- le {{4-{5-[(3-hydroxy-propyl)aminocarbonyl]-1H-benzimidazole-2-yl}-9H-fluorène-9(R,S)-yl}}-amide de l'acide 1H-pyrrolo[2,3-b]pyridine-4-carboxylique
- l'énantiomère dextrogyre du 4-[5-cyano-1H-benzimidazol-2-yl]-9H-fluorène-9(R,S)-yl}-amide de l'acide 1H-pyrrolo[2,3-b]pyridine-4-carboxylique
- le 4-(3H-imidazo[4,5-c]pyridin-2-yl)-9H-fluorèn-9(R,S)-yl]-amide de l'acide 7H-pyrrolo[2,3-d]pyrimidine-4-carboxylique.
- le [4-(6-amino-1H-benzimidazol-2-yl)-9H-fluorèn-9(R,S)-yl]-amide de l'acide 1H-pyrrolo[2,3-b]pyridine-4-carboxylique
- l'énantiomère dextrogyre du 4-[5-fluoro-1H-benzimidazol-2-yl]-9H-fluorène-9(R,S)-yl}-amide de l'acide 1H-pyrrolo[2,3-b]pyridine-4-carboxylique
- l'ester méthylique de l'acide2-{9(R,S)-[(1H-pyrrolo[2,3-b]pyridine-4-carbonyl)-amino]-9H-fluorèn-4-yl}-1H-imidazo[4,5-c]pyridine-6-carboxylique
- le [4-(6-fluoro-5-méthoxy-1H-benzimidazol-2-yl)-9H-fluorène-9(R,S)-yl]-amide de l'acide 1H-Pyrrolo[2,3-b]pyridine-4-carboxylique
- le [4-(6-fluoro-5-hydroxy-1H-benzimidazol-2-yl)-9H-fluorène-9(R,S)-yl]-amide de l'acide 1H-pyrrolo[2,3-b]pyridine-4-carboxylique
- le {4-[5-fluoro-6-(3-méthoxy-propoxy)-1H-benzimidazol-2-yl]-9H-fluorèn-9(R,S)-yl}-amide de l'acide 1H-pyrrolo[2,3-b]pyridine-4-carboxylique
- le {4-[6-(3-diméthylamino-propoxy)-5-fluoro-1H-benzimidazol-2-yl]-9H-fluorèn-9(R,S)-yl}-amide de l'acide 1H-pyrrolo[2,3-b]pyridine-4-carboxylique lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

14. Produits de formule (I) telle que définis à l'une quelconque des revendications précédentes dont les noms suivent :
- la 4-(3H-imidazo[4,5-c]pyridin-2-yl)-fluorène-9-one oxime (Z, E).
- la N-[4-(1H-imidazo[4,5-c]pyridin-2-yl)-fluorèn-9-ylidène]-hydrazine, mélange 60/40 d'isomères E et Z
- le [4-(3H-imidazo[4,5-c]pyridin-2-yl)-9H-fluorèn-9(R,S)-yl]-amide de l'acide quinoléine-5-carboxylique.
- énantiomère dextrogyre du [4-(3H-imidazo[4,5-c]pyridin-2-yl)-9H-fluorèn-9(R,S)-yl]-amide de l'acide quinoléine-5-carboxylique
- la 4-(3H-Imidazo[4,5-c]pyridin-2-yl)-fluorène-9-one oxime (E).
- le trifluoroacétate du [4-(3H-imidazo[4,5-c]pyridin-2-yl)-9H-fluorèn-9(R,S)-yl]-amide de l'acide 1H-pyrazole-4-carboxylique
- le trifluoroacétate du N-[4-(3H-imidazo[4,5-c]pyridin-2-yl)-9H-fluorèn-9(R,S)-yl]-succinamide.
- le trifluoroacétate du [4-(3H-imidazo[4,5-c]pyridin-2-yl)-9H-fluorèn-9(R,S)-yl]-amide de l'acide 1H-benzotriazole-5-carboxylique.
- le [4-(3H-imidazo[4,5-c]pyridin-2-yl)-9H-fluorèn-9(R,S)-yl]-amide de l'acide 1H-indazole-5-carboxylique
- le [4-(3H-imidazo[4,5-c]pyridin-2-yl)-9H-fluorèn-9(R,S)-yl]-amide de l'acide 1H-indole-6-carboxylique
- le N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide 1H-indole-4-carboxylique
- le trifluoroacétate du [4-(3H-imidazo[4,5-c]pyridin-2-yl)-9H-fluorèn-9(R,S)-yl]-amide de l'acide quinoléine-5-carbothioique
- le 2-acétylamino-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide quinoléine-4-carboxylique
- le 2-amino-N-[4-(3H-imidazo[4,5-c]pyridin-2-yl)-9H-fluorèn-9(RS-yl]-isonicotinamide.
- le 2-éthyl-N-[4-(3H-imidazo[4,5-c]pyridin-2-yl)-9H-fluorèn-9(R,S)-yl]-isonicotinamide
- le [4-(6-fluoro-1H-benzimidazol-2-yl)-9H-fluorèn-9(R,S)-yl]-amide de l'acide quinoléine-5-carboxylique
- le 2-chloro-N-[4-(3H-imidazo[4,5-c]pyridin-2-yl)-9H- fluorèn-9(R,S)-yl]-isonicotinamide
- le [4-(3H-imidazo[4,5-c]pyridin-2-yl)-9H-fluoren-9-yl]-amide de l'acide 2-amino-pyrimidine-4-carboxylique
- le [4-(3H-imidazo[4,5-c]pyridin-2-yl)-9H-fluorèn-9(R,S)-yl]-amide de l'acide 1H-pyrrolo[2,3-b]pyridine-4-carboxylique
- le 2-hydroxyméthyl-N-[4-(3H-imidazo[4,5-c]pyridin-2-yl)-9H-fluorèn-9(R,S)-yl]-isonicotinamide
- le N-[4-(3H-imidazo[4,5-c]pyridin-2-yl)-9H-fluorèn-9(R,S)-yl]-3-méthyl-isonicotinamide
- le chlorhydrate du [4-(3H-imidazo[4,5-c]pyridin-2-yl)-9H-fluorèn(R,S)-9-yl]-amide de l'acide 1,8-naphtyridine-4-carboxylique
- le [4-(3H-imidazo[4,5-c]pyridin-2-yl)-9H-fluorèn(R,S)-9-yl]-amide de l'acide 1H-pyrrolo[2,3-b]pyridine-3-carboxylique
- le [4-(3H-imidazo[4,5-c]pyridin-2-yl)-9H-fluorèn(R,S)-9-yl]-amide de l'acide 1-méthyl-1H-pyrrolo[2,3-b]pyridine-3-carboxylique
- le 2-bromo-N-[4-(3H-imidazo[4,5-c]pyridin-2-yl)-9H-fluorèn-9(R,S)-yl]-isonicotinamide
- le 3-hydroxy-N-[4-(3H-imidazo[4,5-c]pyridin-2-yl)-9H-fluorèn-9(R,S)-yl]-isonicotinamide
- le 3-amino-N-[4-(3H-imidazo[4,5-c]pyridin-2-yl)-9H-fluorèn-9(R,S)-yl]-isonicotinamide
- ester de méthyle de l'acide 4-[4-(3H-imidazo[4,5-c]pyridin-2-yl)-9H-fluorèn-9(R,S)-ylcarbamoyl]-pyridine-2-carboxylique
- le [4-(3H-imidazo[4,5-c]pyridin-2-yl)-9H-fluorèn(R,S)-9-yl]-amide de l'acide 1H-indazole-4-carboxylique
- le N-[4-(3H-imidazo[4,5-c]pyridin-2-yl)-9H-fluorèn-9(R,S)-yl]-2-méthylamino-isonicotinamide
- le N-[4-(6-hydroxy-1H-benzimidazol-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide 6-bromo-3H-imidazo[4,5-b]pyridine-7-carboxylique
- ester de 4-(3H-imidazo[4,5-c]pyridin-2-yl)-9H-fluoren-9-y!e de l'acide isonicotinique,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

15. Produits de formule (I) telle que définis à l'une quelconque des revendications précédentes dont les noms suivent :
- le [4-(3H-imidazo[4,5-c]pyridin-2-yl)-9H-fluorèn-9(R,S)-yl]-amide de l'acide quinoléine-5-carboxylique.
- énantiomère dextrogyre du [4-(3H-imidazo[4,5-c]pyridin-2-yl)-9H-fluorèn-9(R,S)-yl]-amide de l'acide quinoléine-5-carboxylique
- la 4-(3H-Imidazo[4,5-c]pyridin-2-yl)-fluorène-9-one oxime (E).
- le trifluoroacétate du [4-(3H-imidazo[4,5-c]pyridin-2-yl)-9H-fluorèn-9(R,S)-yl]-amide de l'acide 1H-benzotriazole-5-carboxylique.
- le N-[4-(3H-imidazo[4,5-c]pyridin-2-yl)-9H-fluorèn-9(R,S)-yl]-isonicotinamide
- le trifluoroacétate du [4-(3H-imidazo[4,5-c]pyridin-2-yl)-9H-fluorèn-9(R,S)-yl]-amide de l'acide quinoléine-5-carbothioique
- ie 2-acétyiamino-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide quinoléine-4-carboxylique
- le 2-amino-N-[4-(3H-imidazo[4,5-c]pyridin-2-yl)-9H-fluorèn-9(RS-yl]-isonicotinamide.
- le 2-éthyl-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluoren-9-yl]-amide de l'acide 2-amino-pyrimidine-4-carboxylique
- le [4-(3H-imidazo[4,5-c]pyridin-2-yl)-9H-fluorèn-9(R,S)-yl]-amide de l'acide 1H-pyrrolo[2,3-b]pyridine-4-carboxylique
- le [4-(3H-imidazo[4,5-c]pyridin-2-yl)-9H-fluorèn(R,S)-9-yl]-amide de l'acide 1H-pyrrolo[2,3-b]pyridine-3-carboxylique
- le 2-bromo-N-[4-(3H-imidazo[4,5-c]pyridin-2-yl)-9H-fluorèn-9(R,S)-yl]-isonicotinamide
- le 3-hydroxy-N-[4-(3H-imidazo[4,5-c]pyridin-2-yl)-9H-fluorèn-9(R,S)-yl]-isonicotinamide
- le 3-amino-N-[4-(3H-imidazo[4,5-c]pyridin-2-yl)-9H-fluorèn-9(R,S)-yl]-isonicotinamide
- ester de méthyle de l'acide 4-[4-(3H-imidazo[4,5-c]pyridin-2-yl)-9H-fluorèn-9(R,S)-ylcarbamoyl]-pyridine-2-carboxylique
- le [4-(3H-imidazo[4,5-c]pyridin-2-yl)-9H-fluorèn(R,S)-9-yl]-amide de l'acide 1H-indazole-4-carboxylique
- le [4-(3H-imidazo[4,5-c]pyridin-2-yl)-9H-fluorèn-9(R,S)-yl]-amide de l'acide 6-bromo-3H-imidazo[4,5-b]pyridine-7-carboxylique
- ester de 4-(3H-imidazo[4,5-c]pyridin-2-yl)-9H-fluoren-9-yle de l'acide isonicotinique,
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

16. Produits de formule (I) telle que définie à l'une quelconque des revendications précédentes dont les noms suivent :
- le [4-(3H-imidazo[4,5-c]pyridin-2-yl)-9H-fluorèn-9(R,S)-yl]-amide de l'acide quinoléine-5-carboxylique.
- le N-[4-(3H-imidazo[4,5-c]pyridin-2-yl)-9H-fluorèn-9(R,S)-yl]-isonicotinamide
- le 2-amino-N-[4-(3H-imidazo[4,5-c]pyridin-2-yl)-9H-fluorèn-9(RS-yl]-isonicotinamide.
- le 2-éthyl-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluoren-9-yl]-amide de l'acide 2-amino-pyrimidine-4-carboxylique
- le [4-(3H-imidazo[4,5-c]pyridin-2-yl)-9H-fluorèn-9(R,S)-yl]-amide de l'acide 1H-pyrrolo[2,3-b]pyridine-4-carboxylique
- le [4-(3H-imidazo[4,5-c]pyridin-2-yl)-9H-fluorèn(R,S)-9-yl]-amide de l'acide 1H-pyrrolo[2,3-b]pyridine-3-carboxylique
- le 3-amino-N-[4-(3H-imidazo[4,5-c]pyridin-2-yl)-9H-fluorèn-9(R,S)-yl]-isonicotinamide
- le [4-(3H-imidazo[4,5-c]pyridin-2-yl)-9H-fluorèn-9(R,S)-yl]-amide de l'acide 6-bromo-3H-imidazo[4,5-b]pyridine-7-carboxylique
lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques desdits produits de formule (I).

17. A titre de médicaments, les produits de formule (I) telle que définie aux revendications 1 à 12, ainsi que leurs prodrugs, lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

18. A titre de médicaments, les produits de formule (I) telle que définie aux revendications 13 à 16, ainsi que leurs prodrugs, lesdits produits de formule (I) étant sous toutes les formes tautomères et isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

19. Les compositions pharmaceutiques contenant à titre de principe actif, l'un au moins des médicaments tels que définis aux revendications 17 et 18.

20. Compositions pharmaceutiques telles que définies aux revendications précédentes contenant en plus, des principes actifs d'autres médicaments de chimiothérapie contre le cancer.

21. Compositions pharmaceutiques selon l'une quelconque des revendications précédentes **caractérisées en ce qu'**elles sont utilisées comme médicaments, en particulier pour la chimiothérapie de cancers.

22. Utilisation de produits de formule (I) telle que définie à l'une quelconque des revendications précédentes ou de sels pharmaceutiquement acceptables desdits produits de formule (I) pour la préparation d'un médicament destiné à prévenir ou traiter une maladie **caractérisée par** le dérèglement de l'activité de la protéine HSP90.

23. Utilisation de produits de formule (I) selon la revendication précédente dans laquelle la maladie à prévenir ou traiter est chez un mammifère.

24. Utilisation de produits de formule (I) telle que définie à l'une quelconque des revendications précédentes ou de sels pharmaceutiquement acceptables desdits produits de formule (I) pour la préparation d'un médicament destiné à prévenir ou traiter une maladie appartenant au groupe suivant : désordres de la prolifération de vaisseaux sanguins, désordres fibrotiques, désordres de la prolifération de cellules mésangiales, désordres métaboliques, allergies, asthme, thromboses, maladies du système nerveux, rétinopathies, psoriasis, arthrite rhumatoïde, diabètes, dégénération musculaire, maladies en oncologie, cancers.

25. Utilisation de produits de formule (I) telle que définie à l'une quelconque des revendications précédentes ou de sels pharmaceutiquement acceptables desdits produits de formule (I) pour la préparation d'un médicament destiné à traiter des cancers.

26. Utilisation de produits de formule (I) selon la revendication précédente dans laquelle la maladie à traiter est un cancer de tumeurs solides ou liquides.

27. Utilisation de produits de formule (I) selon la revendication précédente dans laquelle la maladie à traiter est un cancer résistant aux agents cytotoxiques.

28. Utilisation de produits de formule (I) telle que définie à l'une quelconque des revendications précédentes ou de sels pharmaceutiquement acceptables desdits produits de formule (I) pour la préparation d'un médicament destiné à traiter des cancers parmi lesquels les cancers du poumon, du sein et de l'ovaire, les glioblastomes, les leucémies myéloîdes chroniques, les leucémies lymphoblastiques aigûes, les cancers de la prostate, du pancréas et du colon, les mélanomes métastatiques, les tumeurs de la thyroïde et les carcinomes rénaux.

29. Utilisation de produits de formule (I) telle que définie à l'une quelconque des revendications précédentes ou de sels pharmaceutiquement acceptables desdits produits de formule (I) pour la préparation d'un médicament destiné à la chimiothérapie de cancers.

30. Utilisation de produits de formule (I) telle que définie à l'une quelconque des revendications précédentes ou de sels pharmaceutiquement acceptables desdits produits de formule (I) pour la préparation de médicaments destinés à la chimiothérapie de cancers utilisés seuls ou en association.

31. Utilisation de produits de formule (I) telle que définie à l'une quelconque des revendications précédentes ou de sels pharmaceutiquement acceptables desdits produits de formule (I) pour la préparation de médicaments destinés à être utilisés seuls ou en association avec chimiothérapie ou radiothérapie ou alternativement en association avec d'autres agents thérapeutiques.

32. Utilisation de produits de formule (I) selon la revendication précédente dans laquelle les agents thérapeutiques peuvent être des agents anti-tumoraux utilisés communément.

## Claims

1. Products of formula (I): 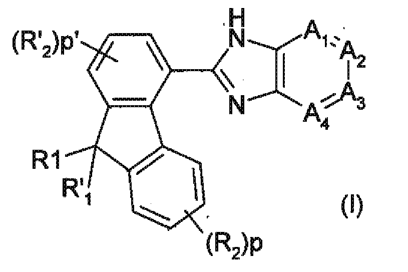 in which:
A1, A2, A3 and A4, which may be identical or different, represent CRa or N or NRb; where Rb represents alkyl, alkoxy, or OH,
R1 and R1' are such that: either R1 and R1', which may be identical or different, are such that one of R1 and R1' represents a hydrogen or halogen atom or a radical selected from C1C3-alkyl, C1C3-alkoxy, alkyl-OH, CF3, cyano, carboxy and carboxamido;
and the other one of R1 and R1' is selected from the group comprising H; halogen; CF3; hydroxyl; mercapto; nitro; amino; NH-OH; NH-CO-H; NH-CO-OH, NH-CO-Oalkyl, NH-CO-NH2; carboxy; CN; CO-NH2 ; X-(CH2)m-alkyl; X-(CH2)m-cycloalkyl; X-(CH2)m-heterocycloalkyl; X-(CH2)m-aryl or X-(CH2)m-heteroaryl with X = single bond, CH2, CH=CH, CH2-O, CH2-NH, CH2-C(O), CH2-C(O)-O, CH2-C(O)-NH, CH2-NH-(CO), CH2-NH-S(O), CH2-NH-S(O)2, O, S, NH, O-C(O), C(O)-NH, -NH-C(O), -NH-C(O)-C(O)-, -NH-C(O)-NH-; NH-CS, NH-S(O) or NH-S(O)2, -NH-CO-CH2-O-; - NH-CO-CH2-S-CH2-CO-NH-; -NH-CO(CH2) 2-SO2-; -NH-CO-CH2-N(CH3)-CO-; with m = 0, 1 or 2, all the alkyl, cycloalkyl, heterocycloalkyl, aryl and heteroaryl radicals being optionally substituted, the cycloalkyl radical containing from 3 to 10 ring members, the aryl radical containing from 6 to 10 ring members, and the heterocycloalkyl and heteroaryl radicals, optionally substituted, containing from 4 to 10 ring members including 1 to 4 heteroatoms selected from O, S, N or NR3 where R3 represents H or alkyl, itself optionally substituted,
or R1 and R1' form, together with the carbon atom to which they are bound, an =O =S; =N-OH; =N-NH2; =N-NH-CO-NH2, =CH-OH; =Y1-(CH2)m-aryl or =Y1-(CH2)m-heteroaryl radical, in which Y1 represents CH, CH-CO-, CH-CO-NH, N, N-O or N-NH-, with m = 0, 1 or 2 and in which aryl and heteroaryl are as defined above and optionally substituted,
or R1 and R1' form, together with the carbon atom to which they are bound, a partially saturate ring made up of 4 to 6 ring members and optionally containing from 1 to 3 heteroatoms selected from O, S, N or NR4 where R4 represents H or alkyl, itself optionally substituted,
R2 and R2', which may be identical or different, are selected independently from the group comprising H, halogen, CF3, nitro, cyano, alkyl, hydroxy, mercapto, amino, alkylamino, dialkylamino, alkoxy, alkylthio (methylthio), free carboxy or carboxy esterified with an alkyl radical, carboxamide, CO-NH(alkyl) and CO N(alkyl)2, all the alkyl, alkoxy and alkylthio radicals being optionally substituted,
p and p', which may be identical or different, represents the integers 1 to 4 and 1 to 3, respectively; Ra is selected from the group comprising H; halogen; CF3; hydroxy; OCF3; SO2-NH2; SO2-NH(alk), SO2-N(alk)2; mercapto; nitro; amino; NH(alk) ; N(alk)2; NH-OH; NH-CO-H; NH-CO-NH2; free carboxy or carboxy esterified with an alkyl radical, itself optionally substituted; CN; CO-NH2; Y-(CH2)n-alkyl; Y-(CH2)n-cycloalkyl, Y-(CH2)n-heterocycloalkyl, Y-(CH2)n-aryl or Y-(CH2)n-heteroaryl, where Y represents a single bond or else = O, S, NH, O-C(O), C(O)-NH, -C(O)N(CH3)-; CO; NH-C(O), NH-S(O) or NH-S(O)2, with n = 0, 1, 2 or 3, and in said radicals the alkyl, cycloalkyl, heterocycloalkyl, aryl and heteroaryl radicals are optionally substituted, the cycloalkyl radical contains from 3 to 10 ring members, the aryl radical contains from 6 to 10 ring members,
and the heterocycloalkyl and heteroaryl radicals, optionally substituted, contain from 4 to 10 ring members, including 1 to 4 heteroatoms selected from O, S, N or NR3 where R3 represents H or alkyl, itself optionally substituted,
all the alkyl, alkoxy, alkylthio, cycloalkyl, heterocycloalkyl, aryl and heteroaryl radicals of the substituents of the products of formula (I) being optionally substituted,
said products of formula (I) being in all the possible tautomeric and isomeric forms: racemic, enantiomeric and diastereoisomeric, as well as the salts of addition with inorganic and organic acids or with inorganic and organic bases of said products of formula (I).

2. Products of formula (I) as defined in Claim 1 in which
A1, A2, A3 and A4, which may be identical or different, represent CRa or N or NRb, where Rb represents CH3 or OH;
Ra is selected from the group comprising H; halogen; CF3; hydroxy; OCF3; S02-NH2; SO2-NHCH3, SO2-N(CH3)2; mercapto; nitro; amino ; NH(CH3); N(CH3)2; NH-OH ; NH-CO-H; NH-CO-NH2; free carboxy or carboxy esterified with an alkyl radical, itself optionally substituted; CO2-CH3; CO2-(CH2)3-N(CH3)2; CN; CO-NH2; Y-(CH2)n-alkyl; Y-(CH2)n-cycloalkyl, Y-(CH2)n-heterocycloalkyl, Y-(CH2)n-aryl or Y-(CH2)n-heteroaryl, where Y represents a single bond or else = O, -C(O)-NH-, -C(O)N(CH3)-; CO,
with n = 0, 1, 2, or 3, and in said radicals the alkyl, cycloalkyl, heterocycloalkyl, aryl and heteroaryl radicals are optionally substituted, the cycloalkyl radical contains from 3 to 10 ring members, the aryl radical contains from 6 to 10 ring members, and the heterocycloalkyl and heteroaryl radicals, optionally substituted, contain from 4 to 10 ring members, including 1 to 4 heteroatoms selected from O, S, N or NR3 where R3 represents H or alkyl, itself optionally substituted,
the other substituents R1, R1', R2, R2' of the said products of formula (I) being selected from any one of the above definitions,
all the alkyl, alkoxy, alkylthio, cycloalkyl, heterocycloalkyl, aryl and heteroaryl radicals of the substituents of the products of formula (I) being optionally substituted,
said products of formula (I) being in all the possible tautomeric and isomeric forms: racemic, enantiomeric and diastereoisomeric, as well as the salts of addition with inorganic and organic acids or with inorganic and organic bases of said products of formula (I).

3. Products of formula (I) as defined in claim 1 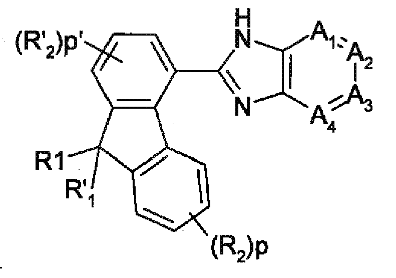 in which:
A1, A2, A3 and A4, which may be identical or different, represent CRa or N;
R1 and R1' are such that: either R1 and R1', which may be identical or different, are such that one of R1 and R1' represents a hydrogen or halogen atom or a radical selected from C1C3-alkyl, C1C3-alkoxy, alkyl-OH, CF3, cyano, carboxy and carboxamido;
and the other one of R1 and R1' is selected from the group comprising H; halogen; CF3 ; hydroxyl; mercapto; nitro; amino; NH-OH; NH-CO-H; NH-CO-OH, NH-CO-Oalkyl , NH-CO-NH2; carboxy; CN; CO-NH2; X-(CH2)m-alkyl; X-(CH2)m-cycloalkyl; X-(CH2)m-heterocycloalkyl; X-(CH2)m-aryl or X-(CH2)m-heteroaryl with X = single bond, CH2, CH=CH, H2-O, CH2-NH, CH2-C(O), CH2-C(O)-O, CH2-C(O) - NH, CH2-NH-(CO), CH2-NH-S(O), CH2-NH-S(O)2, O, S, NH, O-C(O), C(O)-NH, -NH-C(O), -NH-C(O)-C(O)-, -NH-C(O)-NH- . ; NH-CS, NH-S(CO) or NH-S(O)2, with m = 0, 1 or 2, all the alkyl, cycloalkyl, heterocycloalkyl, aryl and heteroaryl radicals being optionally substituted, the cycloalkyl radical containing from 3 to 10 ring members, the aryl radical containing from 6 to 10 ring members, and the heterocycloalkyl and heteroaryl radicals, optionally substituted, containing from 4 to 10 ring members including 1 to 4 heteroatoms selected from O, S, N or NR3 where R3 represents H or alkyl, itself optionally substituted,
or R1 and R1' form, together with the carbon atom to which they are bound, an =O; =S; =N-OH; =N-NH2; =N-NH-CO-NH2, =CH-OH; =Y1-(CH2)m-aryl or =Y1-(CH2)m - heteroaryl, radical, in which Y1 represents CH, CH-CO-, CH-CO-NH, N, N-O or N-NH-, with m = 0, 1 or 2 and in which aryl and heteroaryl are as defined above and optionally substituted,
or R1 and R1' form, together with the carbon atom to which they are bound, a partially saturated ring made up of 4 to 6 ring members and optionally containing from 1 to 3 heteroatoms selected from O, S, N or NR4 where R4 represents H or alkyl, itself optionally substituted,
R2 and R2', which may be identical or different, are selected independently from the group comprising H, halogen, CF3, nitro, cyano, alkyl, hydroxy, mercapto, amino, alkylamino, dialkylamino, alkoxy, alkylthio (methylthio), free or esterified carboxy, carboxamide, CO-NH(alkyl) and CO N(alkyl)2,
p and p', which may be identical or different, represent the integers 1 to 4 and 1 to 3, respectively; Ra is selected from the group comprising H; halogen; CF3; hydroxy; mercapto; nitro; amino; NH-OH; NH-CO-H; NH-CO-NH2; carboxy; CN; CO-NH2; Y-(CH2)n-alkyl; Y-(CH2)n-cycloalkyl, Y-(CH2)n-heterocycloalkyl, Y-(CH2)n-aryl or Y-(CH2)n-heteroaryl, with Y = O, S, NH, O-C(O), C(O)-NH, NH-C(O), NH-S(O) or NH-S(O)2; with n = 0, 1, 2, or 3 radicals in which the alkyl, cycloalkyl, heterocycloalkyl, aryl and heteroaryl radicals are optionally substituted, the cycloalkyl radical contains from 3 to 10 ring members, the aryl radical contains from 6 to 10 ring members, and the heterocycloalkyl and heteroaryl radicals, optionally substituted, contain from 4 to 10 ring members including 1 to 4 heteroatoms selected from O, S, N or NR3 where R3 represents H or alkyl, itself optionally substituted,
all the alkyl, alkoxy, alkylthio, cycloalkyl, heterocycloalkyl, aryl and heteroaryl radicals of the substituents of the products of formula (I) being optionally substituted,
said products of formula (I) being in all the possible tautomeric and isomeric forms: racemic, enantiomeric and diastereoisomeric, as well as the salts of addition with inorganic and organic acids or with inorganic and organic bases of said products of formula (I).

4. Products of formula (I) as defined in Claim 1 in which
A1, A2, A3 and A4, which may be identical or different, represent CRa or N;
Ra is selected from the group comprising H; halogen; hydroxy; mercapto; amino; Y-(CH2)n-alkyl; Y-(CH2)n-cycloalkyl, Y-(CH2)n-heterocycloalkyl, Y-(CH2)n-aryl or Y-(CH2)n-heteroaryl, with Y = O, with n = 0, 1, 2, or 3, radicals in which all the alkyl, cycloalkyl, heterocycloalkyl, aryl and heteroaryl radicals are optionally substituted and the heterocycloalkyl and heteroaryl radicals contain from 4 to 10 ring members including 1 to 4 heteroatoms selected from O, S, N or NR3 where R3 represents H or alkyl, itself optionally substituted,
the other substituents R1, R1', R2, R2' of said products of formula (I) being selected from any one of the above definitions,
said products of formula (I) being in all the possible tautomeric and isomeric forms: racemic, enantiomeric and diastereoisomeric, as well as the salts of addition with inorganic and organic acids or with inorganic and organic bases of said products of formula (I),

5. Products of formula (I) as defined in any one of the other claims in which A1, A2, A3 and A4, which may be identical or different, represent CRa or N;
Ra is selected from the group comprising H; halogen; hydroxy; Y-(CH2)n-alkyl; Y-(CH2)n-cycloalkyl, Y-(CH2)n-heterocycloalkyl, Y-(CH2)n-aryl or Y-(CH2)n-heteroaryl, with Y = O, and with n = 2 or 3, all the alkyl, cycloalkyl, heterocycloalkyl, aryl and heteroaryl radicals being optionally substituted, and the heterocycloalkyl and heteroaryl radicals contain from 4 to 10 ring members including 1 to 4 heteroatoms selected from O, S, N or NR3 where R3 represents H or alkyl, itself optionally substituted,
the other substituents R1, R1', R2, R2' of said products of formula (I) being selected from any one of the above definitions,
said products of formula (I) being in all the possible tautomeric and isomeric forms: racemic, enantiomeric and diastereoisomeric, as well as the salts of addition with inorganic and organic acids or with inorganic and organic bases of said products of formula (I).

6. Products of formula (I) as defined in any one of the other claims in which A1, A2, A3 and A4 are such that either all 4 represent CRa or one of them represents CRa and the other three, which may be identical or different, represent N or CRa, where Ra represents H; halogen; hydroxy or alkoxy;
the other substituents R1, R1', R2, R2' of said products of formula (I) being selected from any one of the above definitions,
said products of formula (I) being in all the possible tautomeric and isomeric forms: racemic, enantiomeric and diastereoisomeric, as well as the salts of addition with inorganic and organic acids or with inorganic and organic bases of said products of formula (I)

7. Products of formula (I) as defined in any one of the other claims in which R2 and R2' which may be identical or different, are selected independently from the group comprising H, halogen, methyl, ethyl, amino, methoxy, CH2-NH2, CH2-NHalk, CH2-N(alk)2, CH2-OH and CH2-Oalk,
p and p', which may be identical or different, represent the integers 1 to 4 and 1 to 3, respectively; the other substituents A1, A2, A3, A4, R1 and R1' of said products of formula (I) being selected from any one of the above definitions,
said products of formula (I) being in all the possible tautomeric and isomeric forms: racemic, enantiomeric and diastereoisomeric, as well as the salts of addition
with inorganic and organic acids or with inorganic and organic bases of said products of formula (I).

8. Products of formula (I) as defined in any one of the other claims in which R2 and R2', which may be identical or different, are selected independently from the group comprising H, halogen, methyl, ethyl, amino, methoxy,
p and p', which may be identical or different, represent the integers 1 to 4 and 1 to 3, respectively; the other substituents A1, A1, A3, A4, R1 and R1' of said products of formula (I) being selected from any one of the above definitions,
said products of formula (I) being in all the possible tautomeric and isomeric forms: racemic, enantiomeric and diastereoisomeric, as well as the salts of addition with inorganic and organic acids or with inorganic and organic bases of said products of formula (I).

9. Products of formula (I) as defined in any one of the other claims, in which R2 and R2', which may be identical or different, are selected independently from the group comprising H, methyl,
p and p', which may be identical or different, represent the integers 1 to 4 and 1 to 3, respectively; the other substituents A1, A2, A3, A4, R1 and R1' of said products of formula (I) being selected from any one of the above definitions,
said products of formula (I) being in all the possible tautomeric and isomeric forms: racemic, enantiomeric and diastereoisomeric, as well as the salts of addition with inorganic and organic acids or with inorganic and organic bases of said products of formula (I)

10. Products of formula (I) as defined in any one of the other claims in which:
R1 and R1' are such that: either R1 and R1', which may be identical or different, are such that one of R1 and
R1' represents a hydrogen atom,
and the other one of R1 and R1' is selected from the group comprising H; halogen; hydroxyl; amino; NH-CO-H; NH-CO-OH, NH-CO-Oalkyl, NH-CO-NH2; carboxy; CO-NH2; X-(CH2)m-alkyl; X-(CH2)m-cycloalkyl; X-(CH2)m-heterocycloalkyl; X-(CH2)m-aryl and X-(CH2)m-heteroaryl, with X = single bond, CH2, CH=CH, CH2-C(O), NH, O-C(O), C(O)-NH, -NH-C(O), -NH-C(O)-C(O)-, -NH-C(O)-NH- .; NH-CS, NH-S(O) or NH-S(O)2, with m = 0, all the alkyl, cycloalkyl, heterocycloalkyl, aryl and heteroaryl radicals being optionally substituted, the cycloalkyl radical containing from 3 to 10 ring members, the aryl radical containing from 6 to 10 ring members, and the heterocycloalkyl and heteroaryl radicals, optionally substituted, containing from 4 to 10 ring members including 1 to 4 heteroatoms selected from O, S, N or NR3 where R3 represents H or alkyl itself optionally substituted,
or R1 and R1' form, together with the carbon atom to which they are bound, an =O; =S; =N-OH; =N-NH2; =N-NH-CO-NH2, =CH-OH; =Y1-(CH2)m-aryl or =Y1-(CH2)m heteroaryl radical, in which Y1 represents CH, CH-CO-, CH-CO-NH, N, N-O or N-NH-, with m = 0, 1 or 2 and in which aryl and heteroaryl are as defined above and optionally substituted,
or R1 and R1' form, together with the carbon atom to which they are bound, a partially saturated heterocycle made up of 5 to 6 ring members and containing from 1 to 3 heteroatoms selected from O, S, N or NR4 where R4 represents H or alkyl, itself optionally substituted,
the other substituents A1, A2, A3, A4, R2 and R2' of said products of formula (I) being selected from any one of the above definitions,
said products of formula (I) being in all the possible tautomeric and isomeric forms: racemic, enantiomeric and diastereoisomeric, as well as the salts of addition with inorganic and organic acids or with inorganic and organic bases of said products of formula (I).

11. Products of formula (I) as defined in Claim 1 in which
A1, A2, A3 and A4, which may be identical or different, represent CRa or N or NRb where Rb represents CH3 or OH;
Ra is selected from the group comprising H; CH3; CH2-NH2; halogen; CF3 ; hydroxy; OCF3 ; SO2-NH2 ; SO2-N(CH3)2 ; mercapto; nitro; amino; NH(CH3); N(CH3)2; NH-OH; NH-CO-H; NH-CO-NH2; free carboxy or carboxy esterified with an alkyl radical, itself optionally substituted; CO2-CH3; CO2-(CH2)3-N(CH3)2; CN; CO-NH2; CO-N(CH3)2; CO-CH3, CO-(CH2)3-O-CH3); morpholinyl; piperazinyl-CH3; imidazolinyl-CH3; diazepin-CH3; -CO-piperazinyl-CH3 - CO-pyrrolidinyl; Y-(CH2)n-alkyl; Y-(CH2)n-cycloalkyl, Y-(CH2)n-heterocycloalkyl, Y-(CH2)n-aryl or Y-(CH2)n-heteroaryl, where Y represents a single bond or else = O, -C(O)-NH-, -C(O)N(CH3)-; CO, with n = 0, 1, 2 or 3,
and in said radicals the alkyl, cycloalkyl, heterocycloalkyl, aryl and heteroaryl radicals are optionally substituted, the cycloalkyl radical contains from 3 to 10 ring members, the aryl radical contains from 6 to 10 ring members, and the heterocycloalkyl and heteroaryl radicals, optionally substituted, contain from 4 to 10 ring members, including to 4 heteroatoms selected from O, S, N or NR3 where R3 represents H or alkyl, itself optionally substituted,
R1 and R1' are such that: either one of R1 and R1' represents a hydrogen atom,
and the other one of R1 and R1' is selected from the group comprising X-(CH2)m-heterocycloalkyl, X-(CH2)m-aryl and X-(CH2)m-heteroaryl, and in particular X-(CH2)m-heteroaryl, where X represents -O-C(O), -NH-C(O), NH-CS, -NH-CO-CH2-O-; -NH-CO-CH2-S-CH2-CO-NH-;-NH-CO-(CH2)2-SO2-; -NH-CO-CH2-N(CH3)-CO-; and m = 0,
or R1 and R1' form, together with the carbon atom to which they are bound an =N-OH or =N-NH2 radical,
R2 and R2', which may be identical or different, are selected independently from the group comprising H, halogen, methyl, ethyl, amino, methoxy, CH2-NH2, CH2-NHalk, CH2-N(alk)2, CH2-OH and CH2-Oalk,
p and p', which may be identical or different, represent the integers 1 to 4 and 1 to 3, respectively; all the alkyl, alkoxy, alkylthio, cycloalkyl, heterocycloalkyl, aryl and heteroaryl radicals of the substituents of the products of formula (I) being optionally substituted,
said products of formula (I) being in all the possible tautomeric and isomeric forms: racemic, enantiomeric and diastereoisomeric, as well as the salts of addition
with inorganic and organic acids or with inorganic and organic bases of said products of formula (I).

12. Products of formula (I) as defined in any one of the other claims in which:
R1 and R1' are such that: either one of R1. and R1' represents a hydrogen atom
and the other one of R1 and R1' is selected from the group comprising X-(CH2)m-heterocycloalkyl, X-(CH2)m-aryl and X-(CH2)m-heteroaryl, and notably X-(CH2)m-heteroaryl, where X represents -O-C(O), -NH-C(O) or NH-CS and m = 0,
or R1 and R1' form, together with the carbon atom to which they are bound, a radical =N-OH or =N-NH2,
the heterocycloalkyl, aryl and heteroaryl radicals being optionally substituted,
the other substituents A1, A2, A3, A4, R2 and R2' of said products of formula (I) being selected from any one of the above definitions,
said products of formula (I) being in all the possible tautomeric and isomeric forms: racemic, enantiomeric and diastereoisomeric, as well as the salts of addition with inorganic and organic acids or with inorganic and organic bases of said products of formula (I).

13. Products of formula (I) as defined in any one of the preceding claims with the following names:
- 4-(3H-imidazo[4,5-c]pyridin-2-yl)-9H-fluoren-9-one oxime (Z, E).
- N-[4-(1H-imidazo[4,5-c]pyridin-2-yl)-9H-fluoren-9-ylidene]-hydrazine, 60/40 mixture of E and Z isomers
- [4-(3H-imidazo[4,5-c]pyridiri-2-yl)-9H-fluoren-9(R,S)-yl]amide of quinoline-5-carboxylic acid
- dextrorotatory enantiomer of [4-(3H-imidazo[4,5-c]pyridin-2-yl)-9H-fluoren-9(R,S)-yl]amide of quinoline-5-carboxylic acid
- 4-(3H-imidazo[4,5-c]pyridin-2-yl)-9H-fluoren-9-one oxime (E).
- trifluoroacetate of [4-(3H-imidazo[4,5-c]pyridin-2-yl)-9H-fluoren-9(R,S)-yl]amide of 1H-pyrazole-4-carboxylic acid
- trifluoroacetate of N-[4-(3H-imidazo[4,5-clpyridin-2-yl)-9H-fluoren-9(R,S)-yl]-succinamide.
- trifluoroacetate of [4-(3H-imidazo[4,5-c]pyridin-2-yl)-9H-fluoren-9(R,S)-yl]amide of 1H-benzotriazole-5-carboxylic acid
- [4-(3H-imidazo[4,5-c]pyridin-2-yl)-9H-fluoren-9(R,S)-yl]amide of 1H-indazole-5-carboxylic acid
- [4-(3H-imidazo[4,5-c]pyridin-2-yl)-9H-fluoren-9 (R,S)-yl] amide of 1H-indole-6-carboxylic acid
- N-[4-(3H-imidazo[4,5-c]pyridin-2-yl)-9H-fluoren-9(R,S)-yl]-isonicotinamide
- [4-(3H-imidazo[4,5-c]pyridin-2-yl)-9H-fluoren-9(R,S)-yl]amide of 1H-indole-4-carboxylic acid
- trifluoroacetate of [4-(3H-imidazo[4,5-c]pyridin-2-yl)-9H-fluoren-9(R,S)-yl]amide of quinoline-5-carbothioic acid
- 2-acetylamino-N-[4-(3H-imidazo[4,5-c]pyridin-2-yl)-9H-fluoren-9(R,S)-yl]-isonicotinamide
- [4-(3H-imidazo[4,5-c]pyridin-2-yl)-9H-fluoren-9-(R,S)-yl]amide of quinoline-4-carboxylic acid
- 2-amino-N-[4-(3H-imidazo[4,5-c]pyridin-2-yl)-9H-fluoren-9(R,S)-yl]-isonicotinamide.
- 2-ethyl-N-[4-(3H-imidazo[4,5-c]pyridin-2-yl)-9H-fluoren-9(R,S)-yl]-isonicotinamide
- [4-(6-fluoro-1H-benzimidazol-2-yl)-9H-fluoren-9(R,S)-yl]amide of quinoline-5-carboxylic acid
- 2-chloro-N-[4-(3H-imidazo[4,5-c]pyridin-2-yl)-9H-fluoren-9(R,S)-yl]-isonicotinamide
- [4-(3H-imidazo[4,5-c]pyridin-2-yl)-9H-fluoren-9(R,S)-yl]amide of 2-amino-pyrimidine-4-carboxylic acid
- [4-(3H-imidazo[4,5-c]pyridin-2-yl)-9H-fluoren-9(R,S)-yl]amide of 1H-pyrrolo[2,3-b]pyridine-4-carboxylic acid
- 2-hydroxymethyl-N-[4-(3H-imidazo[4,5-c]pyridin-2-yl)-9H-fluoren-9(R,S)-yl]-isonicotinamide
- N-[4-(3H-imidazo[4,5-c]pyridin-2-yl)-9H-fluoren-9(R,S)-yl]-3-methyl-isonicotinamide
- hydrochloride of [4-(3H-imidazo[4,5-c]pyridin-2-yl)-9H-fluoren-9(R,S)-yl]amide of 1,8-naphthyridine-4-carboxylic acid
- [4-(3H-imidazo[4,5-c]pyridin-2-yl)-9H-fluoren-9(R,S)-yl]amide of 1H-pyrrolo[2,3-b]pyridine-3-carboxylic acid
- [4-(3H-imidazo[4,5-c]pyridin-2-yl)-9H-fluoren-9(R,S)-yl] amide of 1-methyl-1H-pyrrolo[2,3-b]pyridine-3-carboxylic acid
- 2-bromo-N-[4-(3H-imidazo[4,5-c]pyridin-2-yl)-9H-fluoren-9(R,S)-yl]-isonicotinamide
- 3-hydroxy-N-[4-(3H-imidazo[4,5-c]pyridin-2-yl)-9H-fluoren-9(R,S)-yl]-isonicotinamide
- 3-amino-N-[4-(3H-imidazo[4,5-c]pyridin-2-yl)-9H-fluoren-9(R,S)-yl]-isonicotinamide
- methyl ester of 4-[4-(3H-imidazo[4,5-c]pyridin-2-yl)-9H-fluoren-9(R,S)-ylcarbamoyl]-pyridine-2-carboxylic acid
- [4-(3H-imidazo[4,5-c]pyridin-2-yl)-9H-fluoren-9(R,S)-yl]amide of 1H-indazole-4-carboxylic acid
- N-[4-(3H-imidazo[4,5-c]pyridin-2-yl)-9H-fluoren-9(R,S)-yl]-2-methylamino-isonicotinamide
- N-[4-(6-hydroxy-1H-benzimidazol-2-yl)-9H-fluoren-9(R,S)-yl]isonicotinamide
- [4-(3H-imidazo[4,5-c]pyridin-2-yl)-9H-fluoren-9(R,S)-yl]amide of 6-bromo-3H-imidazo[4,5-b]pyridine-7-carboxylic acid
- 4-(3H-imidazo[4,5-c]pyridin-2-yl)-9H-fluoren-9(R,S)-yl ester of isonicotinic acid,
- N-[4-(3H-imidazo[4,5-c]pyridin-2-yl)-9H-fluoren-9(R,S)-yl]amide of 2-tert-butoxycarbonylamino-isonicotinic acid
- N-[4-(3H-imidazo[4,5-c]pyridin-2-yl)-9H-fluoren-9(R,S)-yl]amide of 3-chloro-6-methoxy-quinoline-4-carboxylic acid
- N-[4-(3H-imidazo[4,5-c]pyridin-2-yl)-9H-fluoren-9(R,*S)*-yl] amide of 3-hydroxy-quinoline-4-carboxylic acid
- N-[4-(3H-imidazo[4,5-c]pyridin-2-yl)-9H-fluoren-9(R,S)-yl]amide of 2-amino-5-chloro-pyrimidine-4-carboxylic acid
- [4-(3H-imidazo[4,5-c]pyridin-2-yl)-9H-fluoren-9(R,S)-yl]amide of 3-bromo-1H-pyrrolo[2,3-b]pyridine-4-carboxylic acid
- dextrorotatory enantiomer of [4-(3H-imidazo[4,5-c]pyridin-2-yl)-9H-fluoren-9(R,S)-yl] amide of 1H-pyrrolo[2,3-b]pyridine-4-carboxylic acid
- N-[4-(3H-imidazo[4,5-c]pyridin-2-yl)-9H-fluoren-9(R,S)-yl]amide of 9H-purine-6-carboxylic acid
- N-[4-(3H-imidazo[4,5-c]pyridin-2-yl)-9H-fluoren-9(R,S)-yl]amide of 2-amino-6-methyl-pyrimidine-4-carboxylic acid
- [4-(3H-imidazo[4,5-c]pyridin-2-yl)-9H-fluoren-9(R,S)-yl]amide of 5-amino-3H-1,2,3-triazole-4-carboxylic acid
- N-[4-(3H-imidazo[4,5-c]pyridin-2-yl)-9H-fluoren-9(R,S)-yl]amide of 6-methyl-2-methylamino-pyrimidine-4-carboxylic acid
- N-[4-(3H-imidazo[4,5-c]pyridin-2-yl)-9H-fluoren-9 (R, S) -yl] amide of 6-methoxy-quinoline-4-carboxylic acid
- 3,5-dihydroxy-N-[4-(3H-imidazo[4,5-c]pyridin-2-yl)-9H-fluoren-9(R,S)-yl]-benzamide.
- N-[4-(3H-imidazo[4,5-c]pyridin-2-yl)-9H-fluoren-9(R,S)-yl]amide of pyrimidine-4-carboxylic acid
- 4-hydroxy-N-[4-(3H-imidazo[4,5-c]pyridin-2-yl)-9H-fluoren-9(R,S)-yl]-benzamide.
- 2,4-dihydroxy-N-[4-(3H-imidazo[4,5-c]pyridin-2-yl) - 9H-fluoren-9(R,S)-yl]-benzamide.
- [4-(5-cyano-6-fluoro-1H-benzimidazol-2-yl)-9H-fluoren-9(R,S)-yl]amide of 1H-pyrrolo[2,3-b] pyridine-4-carboxylic acid
- 4-(1H-benzimidazol-2-yl)-9H-fluoren-9(R,S)-yl]amide of 2-amino-5-chloro-pyrimidine-4-carboxylic acid
- [4-(5-fluoro-1H-benzimidazol-2-yl)-9H-fluoren-9(R,S)-yl]amide of 2-amino-5-chloro-pyrimidine-4-carboxylic acid
- [4-(5-fluoro-1H-benzimidazol-2-yl)-9H-fluoren-9(R,S)-yl]amide of 1H-pyrrolo[2,3-b]pyridine-4-carboxylic acid
- [4-(3H-imidazo[4,5-c]pyridin-2-yl)-9H-fluoren-9(R,S)-yl]amide of 2,3-dimethyl-quinoxaline-5-carboxylic acid
- [4-(3H-imidazo[4,5-c]pyridin-2-yl)-9H-fluoren-9(R,S)-yl]amide of 3-amino-1H-pyrazole-4-carboxylic acid
- dextrorotatory enantiomer of N-[4-(3H-imidazo[4,5-c]pyridin-2-yl)-9H-fluoren-9(R,S)-yl]amide of 2-amino-5-chloro-pyrimidine-4-carboxylic acid
- levorotatory enantiomer of N-[4-(3H-imidazo[4,5-c]pyridin-2-yl)-9H-fluoren-9(R,S)-yl]amide of 2-amino-5-chloro-pyrimidine-4-carboxylic acid
- [4-(3H-imidazo[4,5-c]pyridin-2-yl)-9H-fluoren-9(R,S)-yl]amide of 3-methyl-quinoxaline-5-carboxylic acid
- [4-(3H-imidazo[4,5-c]pyridin-2-yl)-9H-fluoren-9(R,S)-yl]amide of quinoxaline-5-carboxylic acid
- [4-(9H-purine-8-yl)-9H-fluoren-9(R,S)-yl]amide of 1H-pyrrolo[2,3-b]pyridine-4-carboxylic acid
- [4-(1H-benzimidazol-2-yl)-9H-fluoren-9(R,S)-yl]amide of 1H-pyrrolo[2,3-b]pyridine-4-carboxylic acid
- [4-(5-trifluoromethyl-1H-benzimidazol-2-yl)-9H-fluoren-9(R,S)-yl]amide of 1H-pyrrolo[2,3-b]pyridine-4-carboxylic acid
- [4-(5-methoxycarbonyl-1H-benzimidazol-2-yl)-9H-fluoren-9(R,S)-yl]amide of 1H-pyrrolo[2,3-b]pyridine-4-carboxylic acid
- [4-(5-carboxy-1H-benzimidazol-2-yl)-9H-fluoren-9(R,S)-yl]amide of 1H-pyrrolo[2,3-b]pyridine-4-carboxylic acid
- [4-(4-methyl-1H-benzimidazol-2-yl)-9H-fluoren-9(R,S)-yl]amide of 1H-pyrrolo[2,3-b]pyridine-4-carboxylic acid
- [4-(5-carboxamido-1H-benzimidazol-2-yl)-9H-fluoren-9(R,S)-yl] amide of 1H-pyrrolo[2,3-b]pyridine-4-carboxylic acid
- [4-(6-sulphamoyl-1H-benzimidazol-2-yl)-9H-fluoren-9(R,S)-yl]amide of 1H-pyrrolo[2,3-b]pyridine-4-carboxylic acid
- [4-(5-trifluoromethoxy-1H-benzimidazol-2-yl) -9H-fluoren-9(R,S)-yl]amide of 1H-pyrrolo[2,3-b]pyridine-4-carboxylic acid
- [4-(5-cyano-1H-benzimidazol-2-yl)-9H-fluoren-9(R,S)-yl]amide of 1H-pyrrolo[2,3-b]pyridine-4-carboxylic acid
- D diastereoisomer of [4-(3H-imidazo[4,5-c]pyridin-2-yl)-9H-fluoren-9(R,S)-yl]amide of 2-(3-acetyl-2,2-dimethyl-cyclobutan-1-yl) acetic acid
- [4-(6-chloro-1H-benzimidazol-2-yl)-9H-fluoren-9(R,S)-yl]amide of 1H-pyrrolo[2,3-b]pyridine-4-carboxylic acid :
- [4-(5-fluoro-6-morpholino-1H-benzimidazole-2-yl)-9H-fluoren-9(R,S)-yl]amide of 1H-pyrrolo[2,3-b]pyridine-4-carboxylic acid
- [4-(6-chloro-5-fluoro-1H-benzimidazole-2-yl)-9H-fluoren-9 (R,S)-yl] amide of 1H-pyrrolo[2,3-b]pyridine-4-carboxylic acid
- [4-(4,5,6,7-tetrahydro-1H-benzimidazol-2-yl)-9H-fluoren-9(R,S)-yl]amide of 1H-pyrrolo[2,3-b]pyridine-4-carboxylic acid
- [2-amino-5-(1H-benzimidazol-2-yl)-9H-fluoren-9(R,S)-yl]amide of 1H-pyrrolo[2,3-b]pyridine-4-carboxylic acid
- [4-(5-hydroxy-5H-imidazo [4,5-c]pyridin-2-yl)-9H-fluoren-9(R,S)-yl]amide of 1H-pyrrolo[2,3-b]pyridine-4-carboxylic acid
- [4-(5-hydroxy-1H-benzimidazole-2-yl)-9H-fluoren-9(R,S)-yl]amide of 1H-pyrrolo[2,3-b]pyridine-4-carboxylic acid
- [4-(5-methylaminocarbonyl-1H-benzimidazol-2-yl)-9H-fluoren-9(R,S)-yl]amide of 1H-pyrrolo[2,3-b]pyridine-4-carboxylic acid
- [4-(5-dimethylaminocarbonyl-1H-benzimidazol-2-yl)-9H-fluoren-9(R,S)-yl]amide of 1H-pyrrolo[2,3-b]pyridine-4-carboxylic acid
- [4-5-(2-dimethylamino-ethyl)aminocarbonyl-1H-benzimidazol-2-yl)-9H-fluoren-9(R,S)-yl]amide of 1H-pyrrolo[2,3-b]pyridine-4-carboxylic acid
- [4-(4,5,6,7-tetrafluoro-1H-benzimidazol-2-yl)-9H-fluoren-9(R,S)-yl]amide of 1H-pyrrolo[2,3-b]pyridine-4-carboxylic acid
- 4-[5-(3-methoxypropyl)aminocarbonyl-1H-benzimidazol-2-yl)-9H-fluoren-9(R,S)-yl]amide of 1H-pyrrolo[2,3-b]pyridine-4-carboxylic acid
- 4-[5-(4-methyl-piperazine-1-yl)carbonyl-1H-benzimidazol-2-yl)-9H-fluoren-9(R,S)-yl]amide of 1H-pyrrolo[2,3-b]pyridine-4-carboxylic acid
- [4-(6-dimethylsulphamoyl-1H-benzimidazol-2-yl)-9H-fluoren-9(R,S)-yl]amide of 1H-pyrrolo[2,3-b]pyridine-4-carboxylic acid
- [4-[5-(pyrrolidin-1-yl)carbonyl)-1H-benzimidazol-2-yl)-9H-fluoren-9(R,S)-yl]amide of 1H-pyrrolo[2,3-blpyridine-4-carboxylic acid
- dextrorotatory enantiomer of 2-{9(R,S)-[(1H-pyrrolo[2,3-b]pyridine-4-carbonyl)-amino]-9H-fluoren-4-yl}-1H-benzimidazole-5-carboxylic acid
- {{4-{5-[2-(pyrrolidin-1-yl)ethylaminocarbonyl]-1H-benzimidazole-2-yl}-9H-fluoren-9(R,S)-yl}}amide of 1H-pyrrolo[2,3-b]pyridine-4-carboxylic acid
- [4-(6-dimethylamino-1H-benzimidazol-2-yl)-9H-fluoren-9 (R,S) -yl] amide of 1H-pyrrolo[2,3-b]pyridine-4-carboxylic acid
- {4-[6-(4-methyl-piperazin-1-yl)-1H-benzimidazol-2-yl]-9H-fluoren-9(R,S)-yl}-amide of 1H-pyrrolo[2,3-b]pyridine-4-carboxylic acid
- [[6-(methyl-4(5)-imidazolin-2-yl)-1H-benzimidazol-2-yl]-9H-fluoren-9(R,S)-yl]amide of 1H-pyrrolo[2,3-b]pyridine-4-carboxylic acid
- {{4-{5-[{3-dimethylamino-propyl)aminocarbonyl]-1H-benzimidazol-2-yl}-9H-fluoren-9(R,S)-yl}}-amide of 1H-pyrrolo[2,3-b]pyridine-4-carboxylic acid
- {4-[5-fluoro-6-(4-methyl-piperazin-1-yl)-1H-benzimidazol-2-yl]-9H-fluoren-9(R,S)-yl}-amide of 1H-pyrrolo[2,3-b]pyridine-4-carboxylic acid
- {{4-{5-[(3-dimethylamino-propyl)carbonyloxy]-1H-benzimidazol-2-yl}-9H-fluoren-9(R,S)-yl}}-amide of 1H-pyrrolo[2,3-b]pyridine-4-carboxylic acid
- [4-(6-amino-1H-benzimidazol-2-yl)-9H-fluoren-9-(R,S]yl]amide of 1H-pyrrolo[2,3-b]pyridine-4-carboxylic acid
- [4-(6-methyl-1H-benzimidazol-2-yl)-9H-fluoren-9(R,S)-yl]amide of 1H-pyrrolo[2,3-b]pyridine-4-carboxylic acid
- {{4-{5-[(3-hydroxy-propyl)aminocarbonyl]-1H-benzimidazole-2-yl}-9H-fluoren-9(R,S)-yl}}-amide of 1H-pyrrolo[2,3-b]pyridine-4-carboxylic acid
- dextrorotatory enantiomer of {4-[5-cyano-1H-benzimidazol-2-yl]-9H-fluoren-9(R,S)-yl}-amide of 1H-pyrrolo[2,3-b]pyridine-4-carboxylic acid
- [4-(3H-imidazo[4,5-c]pyridin-2-yl)-9H-fluoren-9(R,S)-yl]amide of 7H-pyrrolo[2,3-d]pyrimidine-4-carboxylic acid
- [4-(6-amino-1H-benzimidazol-2-yl)-9H-fluoren-9(R,S) - yl]amide of 1H-pyrrolo[2,3-b]pyridine-4-carboxylic acid
- dextrorotatory enantiomer of {4-[5-fluoro-1H-benzimidazol-2-yl]-9H-fluoren-9(R,S)-yl}-amide of 1H-pyrrolo[2,3-b]pyridine-4-carboxylic acid
- methyl ester of 2-{9(R,S)-[(1H-pyrrolo[2,3-b]pyridine-4-carbonyl)-amino]-9H-fluoren-4-yl}-1H-imidazo[4,5-c]pyridine-6-carboxylic acid
- [4-(6-fluoro-5-methoxy-1H-benzimidazol-2-yl)-9H-fluoren-9(R,S)-yl] amide of 1H-pyrrolo[2,3-b]pyridine-4-carboxylic acid
- [4-(6-fluoro-5-hydroxy-1H-benzimidazol-2-yl)-9H-fluoren-9(R,S)-yl]amide of 1H-pyrrolo[2,3-b]pyridine-4-carboxylic acid
- {4-[5-fluoro-6-(3-methoxy-propoxy-1H-benzimidazol-2-yl]-9H-fluoren-9(R,S)-yl}-amide of 1H-pyrrolo[2,3-b]pyridine-4-carboxylic acid
- {4-[6-(3-dimethylamino-propoxy)-5-fluoro-1H-benzimidazol-2-yl]-9H-fluoren-9(R,S)-yl}-amide of 1H-pyrrolo[2,3-b]pyridine-4-carboxylic acid said products of formula (I) being in all the possible tautomeric and isomeric forms: racemic, enantiomeric and diastereoisomeric, as well as the salts of addition with inorganic and organic acids or with inorganic and organic bases of said products of formula (I).

14. Products of formula (I) as def ined in any one of the preceding claims with the following names:
- 4-(3H-imidazo[4,5-c]pyridin-2-yl)-fluoren-9-one oxime (Z, E).
- N-[4-(1H-imidazo[4,5-c]pyridin-2-yl)-fluoren-9-ylidene]-hydrazine, 60/40 mixture of E and Z isomers
- [4-(3H-imidazo[4,5-c]pyridin-2-yl)-9H-fluoren-9(R,S)-yl]amide of quinoline-5-carboxylic acid.
- dextrorotatory enantiomer of [4-(3H-imidazo[4,5-c]pyridin-2-yl)-9H-fluoren-9(R,S)-yl]amide of quinoline-5-carboxylic acid
- 4- (3H-imidazo[4,5-c]pyridin-2-yl)-fluoren-9-one oxime (E).
- trifluoroacetate of [4-(3H-imidazo[4,5-c]pyridin-2-yl)-9H-fluoren-9(R,S)-yl]amide of 1H-pyrazole-4-carboxylic acid
- trifluoroacetate of N-[4-(3H-imidazo[4,5-c]pyridin-2-yl)-9H-fluoren-9(R,S)-yl]-succinamide.
- trifluoroacetate of [4-(3H-imidazo[4,5-c]pyridin-2-yl)-9H-fluoren-9(R,S)-yl]amide of 1H-benzotriazole-5-carboxylic acid.
- [4-(3H-imidazo[4,5-c]pyridin-2-yl)-9H-fluoren-9(R,S)-yl]amide of 1H-indazole-5-carboxylic acid
- [4-(3H-imidazo[4,5-c]pyridin-2-yl)-9H-fluoren-9(R,S)-yl]amide of 1H-indole-6-carboxylic acid
- N-[4-(3H-imidazo[4,5-c]pyridin-2-yl)-9H-fluoren-9(R,S)-yl]-isonicotinamide
- [4-(3H-imidazo[4,5-c]pyridin-2-yl)-9H-fluoren-9(R,S)-yl]amide of 1H-indole-4-carboxylic acid
- trifluoroacetate of [4-(3H-imidazo[4,5-c]pyridin-2-yl)-9H-fluoren-9(R,S)-yl]amide of quinoline-5-carbothioic acid
- 2-acetylamino-N-[4-(3H-imidazo[4,5-c]pyridin-2-yl)-9H-fluoren-9(R,S)-yl]-isonicotinamide
- [4-(3H-imidazo[4,5-c]pyridin-2-yl)-9H-fluoren-9(R,S)-yl]amide of quinoline-4-carboxylic acid
- 2-amino-N-[4-(3H-imidazo[4,5-c]pyridin-2-yl)-9H-fluoren-9(R,S)-yl]-isonicotinamide.
- 2-ethyl-N-[4-(3H-imidazo[4,5-c]pyridin-2-yl)-9H-fluoren-9(R,S)-yl]-isonicotinamide
- [4-(6-fluoro-1H-benzimidazol-2-yl)-9H-fluoren-9(R,S) - yl]amide of quinoline-5-carboxylic acid
- 2-chloro-N-[4-(3H-imidazo[4,5-c]pyridin-2-yl)-9H-fluoren-9(R,S)-yl]-isonicotinamide
- [4-(3H-imidazo[4,5-c]pyridin-2-yl)-9H-fluoren-9-yl]amide of 2-amino-pyrimidine-4-carboxylic acid
- [4-(3H-imidazo[4,5-c]pyridin-2-yl)-9H-fluoren-9(R,S)-yl]amide of 1H-pyrrolo[2,3-b]pyridine-4-carboxylic acid
- 2-hydroxymethyl-N-[4-(3H-imidazo[4,5-c]pyridin-2-yl)-9H-fluoren-(R,S)-yl]-isonicotinamide
- N-[4-(3H-imidazo[4,5-c]pyridin-2-yl)-9H-fluoren-9(R,S)-yl]-3-methyl-isonicotinamide
- hydrochloride of [4-(3H-imidazo[4,5-c]pyridin-2-yl)-9H-fluoren(R,S)-9-yl]amide of 1,8-naphthyridine-4-carboxylic acid
- [4-(3H-imidazo[4,5-c]pyridin-2-yl)-9H-fluoren(R,S)-9-yl]amide of 1H-pyrrolo[2,3-b]pyridine-3-carboxylic acid
- [4-(3H-imidazo[4,5-c]pyridin-2-yl)-9H-fluoren(R,S)-9-yl]amide of 1-methyl-1H-pyrrolo[2,3-b]pyridine-3-carboxylic acid
- 2-bromo-N-[4-(3H-imidazo[4,5-c]pyridin-2-yl)-9H-fluoren-9(R,S)-yl]-isonicotinamide
- 3-hydroxy-N-[4-(3H-imidazo[4,5-c]pyridin-2-yl)-9H-fluoren-9(R,S)-yl]-isonicotinamide
- 3-amino-N-[4-(3H-imidazo[4,5-c]pyridin-2-yl)-9H-fluoren-9(R,S)-yl]-isonicotinamide
- methyl ester of 4-[4-(3H-imidazo[4,5-c]pyridin-2-yl)-9H-fluoren-9(R,S)-ylcarbamoyl]-pyridine-2-carboxylic acid
- [4-(3H-imidazo[4,5-c]pyridin-2-yl)-9H-fluoren(R,S)-9-yl]amide of 1H-indazole-4-carboxylic acid
- N-[4-(3H-imidazo[4,5-c]pyridin-2-yl)-9H-fluoren-9(R,S)-yl]-2-methylamino-isonicotinamide
- N-[4-(6-hydroxy-1H-benzimidazol-2-yl)-9H-fluoren-9(R,S)-yl]-isonicotinamide
- [4-(3H-imidazo[4,5-c]pyridin-2-yl)-9H-fluoren-9(R,S)-yl]amide of 6-bromo-3H-imidazo[4,5-b]pyridine-7-carboxylic acid
- 4-(3H-imidazo[4,5-c]pyridin-2-yl)-9H-fluoren-9-yl ester of isonicotinic acid,
said products of formula (I) being in all the possible tautomeric and isomeric forms: racemic, enantiomeric
and diastereoisomeric, as well as the salts of addition with inorganic and organic acids or with inorganic and organic bases of said products of formula (I).

15. Products of formula (I) as defined in any one of the preceding claims with the following names:
- [4-(3H-imidazo[4,5-c]pyridin-2-yl)-9H-fluoren-9(R,S) - yl]amide of quinoline-5-carboxylic acid.
- dextrorotatory enantiomer of [4-(3H-imidazo[4,5-c]pyridin-2-yl)-9H-fluoren-9(R,S)-yl]amide of quinoline-5-carboxylic acid
- 4-(3H-imidazo[4,5-c]pyridin-2-yl)-fluoren-9-one oxime (E).
- trifluoroacetate of [4-(3H-imidazo[4,5-c]pyridin-2-yl)-9H-fluoren-9(R,S)-yl]amide of 1H-benzotriazole-5-carboxylic acid.
- N-[4-(3H-imidazo[4,5-c]pyridin-2-yl)-9H-fluoren-9(R,S)-yl]-isonicotinamide
- trifluoroacetate of [4-(3H-imidazo[4,5-c]pyridin-2-yl)-9H-fluoren-9(R,S)-yl]amide of quinoline-5-carbothioic acid
- 2-acetylamino-N-[4-(3H-imidazo[4,5-c]pyridin-2-yl)-9H-fluoren-9(R,S)-yl]-isonicotinamide
- [4-(3H-imidazo[4,5-c]pyridin-2-yl)-9H-fluoren-9(R,S)-yl]amide of quinoline-4-carboxylic acid
- 2-amino-N-[4-(3H-imidazo[4,5-c]pyridin-2-yl)-9H-fluoren-9(R,S)-yl]-isonicotinamide.
- 2-ethyl-N-[4-(3H-imidazo[4,5-c]pyridin-2-yl)-9H-fluoren-9(R,S)-yl]-isonicotinamide
- [4-(3H-imidazo[4,5-c]pyridin-2-yl)-9H-fluoren-9-yl]amide of 2-amino-pyrimidine-4-carboxylic acid
- [4-(3H-imidazo[4,5-c]pyridin-2-yl)-9H-fluoren-9(R,S)-yl]amide of 1H-pyrrolo[2,3-b]pyridine-4-carboxylic acid
- [4-(3H-imidazo[4,5-c]pyridin-2-yl)-9H-fluoren(R,S)-9-yl]amide of 1H-pyrrolo[2,3-b]pyridine-3-carboxylic acid
- 2-bromo-N-[4-(3H-imidazo[4,5-c]pyridin-2-yl)-9H-fluoren-9(R,S)-yl]-isonicotinamide
- 3-hydroxy-N-[4-(3H-imidazo[4,5-c]pyridin-2-yl)-9H-fluoren-9(R,S)-yl]-isonicotinamide
- 3-amino-N-[4-(3H-imidazo[4,5-c]pyridin-2-yl)-9H-fluoren-9(R,S)-yl]-isonicotinamide
- methyl ester of 4-[4-(3H-imidazo[4,5-c]pyridin-2-yl)-9H-fluoren-9(R,S)-ylcarbamoyl]-pyridine-2-carboxylic acid
- [4-(3H-imidazo[4,5-c]pyridin-2-yl)-9H-fluoren(R,S)-9-yl]amide of 1H-indazole-4-carboxylic acid
- [4-(3H-imidazo[4,5-c]pyridin-2-yl)-9H-fluoren-9(R,S)-yl]amide of 6-bromo-3H-imidazo[4,5-b]pyridine-7-carboxylic acid
- 4-(3H-imidazo[4,5-c]pyridin-2-yl)-9H-fluoren-9-yl ester of isonicotinic acid,
said products of formula (I) being in all the possible tautomeric and isomeric forms: racemic, enantiomeric and diastereoisomeric, as well as the salts of addition with inorganic and organic acids or with inorganic and
organic bases of said products of formula (T).

16. Products of formula (I) as defined in any one of the preceding claims with the following names:
- [4-(3H-imidazo[4,5-c]pyridin-2-yl)-9H-fluoren-9(R,S)-yl]amide of quinoline-5-carboxylic acid.
- N-[4-(3H-imidazo[4,5-c]pyridin-2-yl)-9H-fluoren-9(R,S)-yl]-isonicotinamide
- 2-amino-N-[4-(3H-imidazo[4,5-c]pyridin-2-yl)-9H-fluoren-9(R,S)-yl]-isonicotinamide.
- 2-ethyl-N-[4-(3H-imidazo[4,5-c]pyridin-2-yl)-9H-fluoren-9(R,S)-yl]-isonicotinamide
- [4-(3H-imidazo[4,5-c]pyridin-2-yl)-9H-fluoren-9-yl]amide of 2-amino-pyrimidine-4-carboxylic acid
- [4-(3H-imidazo[4,5-c]pyridin-2-yl)-9H-fluoren-9(R,S)-yl]amide of 1H-pyrrolo[2,3-b]pyridine-4-carboxylic acid
- [4-(3H-imidazo[4,5-c]pyridin-2-yl)-9H-fluoren(R,S)-9-yl]amide of 1H-pyrrolo[2,3-b]pyridine-3-carboxylic acid
- 3-amino-N-[4-(3H-imidazo[4,5-c]pyridin-2-yl)-9H-fluoren-9(R,S)-yl]-isonicotinamide
- [4-(3H-imidazo[4,5-c]pyridin-2-yl)-9H-fluoren-9(R,S)-yl]amide of 6-bromo-3H-imidazo[4,5-b]pyridine-7-carboxylic acid
said products of formula (I) being in all the possible tautomeric and isomeric forms: racemic, enantiomeric and diastereoisomeric, as well as the salts of addition with inorganic and organic acids or with inorganic and organic bases of said products of formula (I).

17. As medicinal products, the products of formula (I) as defined in Claims 1 to 12, as well as their prodrugs, said products of formula (I) being in all the possible tautomeric and isomeric forms: racemic, enantiomeric and diastereoisomeric, as well as the pharmaceutically acceptable salts of addition with inorganic and organic acids or with inorganic and organic bases of said products of formula (I).

18. As medicinal products, the products of formula (I) as defined in Claims 13 to 16, as well as their prodrugs, said products of formula (I) being in all the possible tautomeric and isomeric forms: racemic, enantiomeric and diastereoisomeric, as well as the pharmaceutically acceptable salts of addition with inorganic and organic acids or with inorganic and organic bases of said products of formula (I).

19. Pharmaceutical compositions containing as active principle, at least one of the medicinal products as defined in Claims 17 and 18.

20. Pharmaceutical compositions as defined in the preceding claims additionally containing active principles of other medicinal products for cancer chemotherapy.

21. Pharmaceutical compositions according to any one of the preceding claims, **characterized in that** they are used as medicinal products, in particular for chemotherapy of cancers.

22. Use of products of formula (I) as defined in any one of the preceding claims or of pharmaceutically acceptable salts of said products of formula (I) for the preparation of a medicinal product intended for preventing or treating a disease **characterized by** disturbance of the activity of the HSP90 protein.

23. Use of products of formula (I) according to the preceding claim in which the disease to be prevented or treated is in a mammal.

24. Use of products of formula (I) as defined in any one of the preceding claims or of pharmaceutically acceptable salts of said products of formula (I) for the preparation of a medicinal product intended for preventing or treating a disease in the following group: disorders of proliferation of blood vessels, fibrotic disorders, disorders of proliferation of mesangial cells, metabolic disorders, allergies, asthma, thromboses, diseases of the nervous system, retinopathies, psoriasis, rheumatoid arthritis, diabetes, muscular degeneration, oncological diseases, cancers.

25. Use of products of formula (I) as defined in any one of the preceding claims or of pharmaceutically acceptable salts of said products of formula (I) for the preparation of a medicinal product intended for treating cancers.

26. Use of products of formula (I) according to the preceding claim in which the disease to be treated is a cancer of solid or liquid tumours.

27. Use of products of formula (I) according to the preceding claim in which the disease to be treated is a cancer that is resistant to cytotoxic agents.

28. Use of products of formula (I) as defined in any one of the preceding claims or of pharmaceutically acceptable salts of said products of formula (I) for the preparation of a medicinal product intended for treating cancers including lung, breast and ovarian cancers, glioblastomas, chronic myeloid leukaemias, acute lymphoblastic leukaemias, cancers of the prostate, pancreas and colon, metastatic melanomas, thyroid tumours and renal carcinomas.

29. Use of products of formula (I) as defined in any one of the preceding claims or of pharmaceutically acceptable salts of said products of formula (I) for the preparation of a medicinal product intended for chemotherapy of cancers.

30. Use of products of formula (I) as defined in any one of the preceding claims or of pharmaceutically acceptable salts of said products of formula (I) for the preparation of medicinal products intended for chemotherapy of cancers, used alone or in combination.

31. Use of products of formula (I) as defined in any one of the preceding claims or of pharmaceutically acceptable salts of said products of formula (I) for the preparation of medicinal products intended to be used alone or in combination with chemotherapy or radiotherapy or alternatively in combination with other therapeutic agents.

32. Use of products of formula (I) according to the preceding claim in which the therapeutic agents can be commonly-used antitumour agents.

## Patentansprüche

1. Produkte der Formel (I) : 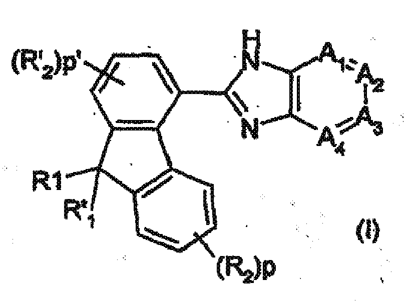 worin:
A1, A2, A3 und A4 gleich oder verschieden sind und für CRa oder N oder NRb stehen;
wobei Rb für Alkyl, Alkoxy oder OH steht;
R1 und R1' so beschaffen sind, daß: entweder R1 und R1' gleich oder verschieden sind und so beschaffen sind, daß eine der Gruppen R1 und R1' für ein Wasserstoff- oder Halogenatom oder einen unter C₁-C₃-Alkyl, C₁-C₃-Alkoxy, Alkyl-OH, CF₃, Cyano, Carboxy und Carboxamido ausgewählten Rest steht
und die andere der Gruppen R1 und R1' ausgewählt ist aus der Gruppe bestehend aus H; Halogen; CF₃; Hydroxyl; Mercapto, Nitro; Amino; NH-OH; NH-CO-H; NH-CO-OH, NH-CO-O-Alkyl, NH-CO-NH₂; Carboxy; CN; CO-NH₂; X-(CH₂)ₘ-Alkyl; X-(CH₂)ₘ-Cycloalkyl; X-(CH₂)ₘ-Heterocycloalkyl; X-(CH₂)ₘ-Aryl oder X-(CH₂)ₘ-Heteroaryl mit X = Einfachbindung, CH₂, CH=CH, CH₂-O, CH₂-NH, CH₂-C(O)**,** CH₂-C(O)-O, CH₂-C(O)-NH, CH₂-NH-(CO), CH₂-NH-S(O), CH₂-NH-S(O)₂, O, S, NH, O-C(O), C(O)-NH, -NH-C(O), -NH-C(O)-C(O)-, -NH-C(O)-NH-, NH-CS, NH-S(O) oder NH-S(O)₂, -NH-CO-CH₂-O-; -NH-CO-CH₂-S-CH₂-CO-NH-; -NH-CO-(CH₂)₂-SO₂-; -NH-CO-CH₂-N(CH₃)-CO-; mit m = 0, 1 oder 2, wobei alle Alkyl-, Cycloalkyl-, Heterocycloalkyl-, Aryl- und Heteroarylreste gegebenenfalls substituiert sind, der Cycloalkylrest 3 bis 10 Glieder enthält, der Arylrest 6 bis 10 Glieder enthält und die Heterocycloalkyl- und Heteroarylreste gegebenenfalls substituiert sind und 4 bis 10 Glieder enthalten, von denen 1 bis 4 Heteroatome sind, die unter O, S, N oder NR3 ausgewählt sind, wobei R3 für H oder Alkyl, das gegebenenfalls selbst substituiert ist, steht,
oder R1 und R'1 zusammen mit dem Kohlenstoffatom, an das sie gebunden sind, einen =O-; =S-; =N-OH-; =N-NH₂- ; =N-NH-CO-NH₂-, =CH-OH-; =Y₁-(CH₂)ₘ-Aryl- oder =Y₁-(CH₂)ₘ-Heteroarylrest, worin Y₁ für CH, CH-CO-, CH-CO-NH, N, N-O oder N-NH- steht, mit m = 0, 1 oder 2, wobei Aryl und Heteroaryl wie oben definiert und gegebenenfalls substituiert sind, bilden,
oder R1 und R'1 zusammen mit dem Kohlenstoffatom, an das sie gebunden sind, einen teilweise gesättigten Ring bilden, der aus 4 bis 6 Gliedern besteht und gegebenenfalls 1 bis 3 unter O, S, N oder NR4 ausgewählte Heteroatome enthält, wobei R4 für H oder Alkyl, das gegebenenfalls selbst substituiert ist, steht,
R2 und R'2 gleich oder verschieden sind und unabhängig voneinander ausgewählt sind aus der Gruppe bestehend aus H, Halogen, CF₃, Nitro, Cyano, Alkyl, Hydroxy, Mercapto, Amino, Alkylamino, Dialkylamino, Alkoxy, Alkylthio (Methylthio), freiem Carboxy oder mit einem Alkylrest verestertem Carboxy, Carboxamid, CO-NH(Alkyl) und CON(Alkyl)_{2,} wobei alle Alkyl- Alkoxy- und Alkylthioreste gegebenenfalls substituiert sind,
p und p' gleich oder verschieden sind und für ganze Zahlen mit einem Wert von 1 bis 4 bzw. 1 bis 3 stehen;
Ra ausgewählt ist aus der Gruppe bestehend aus H; Halogen; CF₃; Hydroxy; OCF₃; SO₂-NH₂; SO₂-NH(alk), SO₂-N(alk)₂; Mercapto; Nitro; Amino; NH(alk); N(alk)₂; NH-OH; NH-CO-H; NH-CO-NH₂; freiem Carboxy oder mit einem Alkylrest, der gegebenenfalls selbst substituiert ist, verestertem Carboxy; CN; CO-NH₂; Y-(CH₂)ₙ-Alkyl; Y-(CH₂)ₙ-Cycloalkyl, Y-(CH₂)ₙ-Heterocycloalkyl, Y-(CH₂)ₙ-Aryl oder Y-(CH₂)ₙ-Heteroaryl, wobei Y für eine Einfachbindung steht oder auch = O, S, NH, O-C(O), C(O)-NH, -C(O)N(CH₃) -; CO; NH-C(O), NH-S(O) oder NH-S(O)₂, mit n = 0, 1, 2 oder 3, wobei in diesen Resten die Alkyl-, Cycloalkyl-, Heterocycloalkyl-, Aryl- und Heteroarylreste gegebenenfalls substituiert sind, der Cycloalkylrest 3 bis 10 Glieder enthält, der Arylrest 6 bis 10 Glieder enthält und die Heterocycloalkyl- und Heteroarylreste gegebenenfalls substituiert sind und 4 bis 10 Glieder enthalten, von denen 1 bis 4 Heteroatome sind, die unter O, S, N oder NR3 ausgewählt sind, wobei R3 für H oder Alkyl, das gegebenenfalls selbst substituiert ist, steht,
wobei alle Alkyl-, Alkoxy-, Alkylthio-, Cycloalkyl-, Heterocycloalkyl-, Aryl- und Heteroarylreste der Substituenten der Produkte der Formel (I) gegebenenfalls substituiert sind,
wobei die Produkte der Formel (I) in allen möglichen tautomeren und isomeren Formen, Racemate, Enantiomere und Diastereoisomere, vorliegen, sowie die Additionssalze der Produkte der Formel (I) mit anorganischen und organischen Säuren oder mit anorganischen und organischen Basen.

2. Produkte der Formel (I) nach Anspruch 1, worin
A1, A2, A3 und A4 gleich oder verschieden sind und für CRa oder N oder NRb stehen, wobei Rb für CH₃ oder OH steht;
Ra ausgewählt ist aus der Gruppe bestehend aus H; Halogen; CF₃; Hydroxy; OCF₃; SO₂-NH₂; SO₂-NHCH₃, SO₂-N(CH₃)₂; Mercapto; Nitro; Amino; NH(CH₃) ; N(CH₃)₂; NH-OH; NH-CO-H; NH-CO-NH₂; freiem Carboxy oder mit einem Alkylrest, der gegebenenfalls selbst substituiert ist, verestertem Carboxy; CO₂-CH₃; CO₂- (CH₂)₃-N(CH₃)₂; CN; CO-NH₂; Y- (CH₂)ₙ-Alkyl; Y-(CH₂)ₙ₋Cycloalkyl, Y-(CH₂)ₙ-Heterocycloalkyl, Y-(CH₂)ₙ-Aryl oder Y-(CH₂)ₙ-Heteroaryl, wobei Y für eine Einfachbindung steht oder auch = O, -C(O)-NH-, -C(O)N(CH₃)-; CO; mit n = 0, 1, 2 oder 3, wobei in diesen Resten die Alkyl-, Cycloalkyl-, Heterocycloalkyl-, Aryl- und Heteroarylreste gegebenenfalls substituiert sind, der Cycloalkylrest 3 bis 10 Glieder enthält, der Arylrest 6 bis 10 Glieder enthält und die Heterocycloalkyl- und Heteroarylreste gegebenenfalls substituiert sind und 4 bis 10 Glieder enthalten, von denen 1 bis 4 Heteroatome sind, die unter O, S, N oder NR3 ausgewählt sind, wobei R3 für H oder Alkyl, das gegebenenfalls selbst substituiert ist, steht, wobei die anderen Substituenten R1, R1'_{,} R2 und R2' der Produkte der Formel (I) unter einer der obigen Definitionen ausgewählt sind,
wobei alle Alkyl-, Alkoxy-, Alkylthio-, Cycloalkyl-, Heterocycloalkyl-, Aryl- und Heteroarylreste der Substituenten der Produkte der Formel (I) gegebenenfalls substituiert sind,
wobei die Produkte der Formel (I) in allen möglichen tautomeren und isomeren Formen, Racemate, Enantiomere und Diastereoisomere, vorliegen, sowie die Additionssalze der Produkte der Formel (I) mit anorganischen und organischen Säuren oder mit anorganischen und organischen Basen.

3. Produkte der Formel (I) nach Anspruch 1 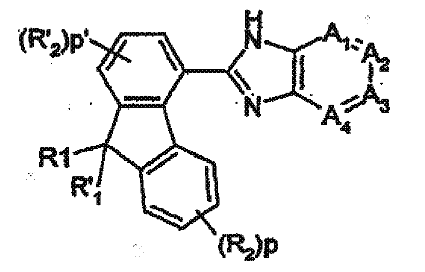 worin:
A1, A2, A3 und A4 gleich oder verschieden sind und für CRa oder N stehen;
R1 und R1' so beschaffen sind, daß: entweder R1 und R1' gleich oder verschieden sind und so beschaffen sind, daß eine der Gruppen R1 und R1' für ein Wasserstoff- oder Halogenatom oder einen unter C₁-C₃-Alkyl, C₁-C₃-Alkoxy, Alkyl-OH, CF₃, Cyano, Carboxy und Carboxamido ausgewählten Rest steht
und die andere der Gruppen R1 und R1' ausgewählt ist aus der Gruppe bestehend aus H; Halogen; CF₃; Hydroxyl; Mercapto; Nitro; Amino; NH-OH; NH-CO-H; NH-CO-OH, NH-CO-O-Alkyl, NH-CO-NH₂; Carboxyl; CN; CO-NH₂; X-(CH₂)ₘ-Alkyl; X-(CH₂)-Cycloalkyl; X-(CH₂)ₘ-Heterocycloalkyl; X-(CH₂)ₘ-Aryl oder X-(CH₂)ₘ-Heteroaryl mit X = Einfachbindung, CH₂, CH=CH, CH₂-O, CH₂-NH, CH₂-C(O), CH₂-C(O)-O, CH₂-C(O)-NH, CH₂-NH-(CO), CH₂-NH-S(O), CH₂-NH-S(O)₂, O, S, NH, O-C(O), C(O)-NH, -NH-C(O), -NH-C(O)-C(O)-, -NH-C(O)-NH-, NH-CS, NH-S(O) oder NH-S(O)₂, mit m = 0, 1 oder 2, wobei alle Alkyl-, Cycloalkyl-, Heterocycloalkyl-, Aryl- und Heteroarylreste gegebenenfalls substituiert sind, der Cycloalkylrest 3 bis 10 Glieder enthält, der Arylrest 6 bis 10 Glieder enthält und die Heterocycloalkyl- und Heteroarylreste gegebenenfalls substituiert sind und 4 bis 10 Glieder enthalten, von denen 1 bis 4 Heteroatome sind, die unter O, S, N oder NR3 ausgewählt sind, wobei R3 für H oder Alkyl, das gegebenenfalls selbst substituiert ist, steht,
oder R1 und R'1 zusammen mit dem Kohlenstoffatom, an das sie gebunden sind, einen =O-; =S-; =N-OH-; =N-NH₂-; =N-NH-CO-NH₂-, =CH-OH-; =Y₁-(CH₂)ₘ-Aryl- oder =Y₁-(CH₂)ₘ-Heteroarylrest, worin Y₁ für CH, CH-CO-, CH-CO-NH, N, N-O oder N-NH- steht, mit m = 0, 1 oder 2, wobei Aryl und Heteroaryl wie oben definiert und gegebenenfalls substituiert sind, bilden,
oder R1 und R'1 zusammen mit dem Kohlenstoffatom, an das sie gebunden sind, einen teilweise gesättigten Ring bilden, der aus 4 bis 6 Gliedern besteht und gegebenenfalls 1 bis 3 unter O, S, N oder NR4 ausgewählte Heteroatome enthält, wobei R4 für H oder Alkyl, das gegebenenfalls selbst substituiert ist, steht,
R2 und R'2 gleich oder verschieden sind und unabhängig voneinander ausgewählt sind aus der Gruppe bestehend aus H, Halogen CF₃, Nitro, Cyano, Alkyl, Hydroxy, Mercapto, Amino, Alkylamino, Dialkylamino, Alkoxy, Alkylthio (Methylthio), freiem Carboxy oder verestertem Carboxy, Carboxamid, CO-NH(Alkyl) und CON(Alkyl)₂,
p und p' gleich oder verschieden sind und für ganze Zahlen mit einem Wert von 1 bis 4 bzw. 1 bis 3 stehen;
Ra ausgewählt ist aus der Gruppe bestehend aus H; Halogen; CF₃; Hydroxy; Mercapto; Nitro; Amino; NH-OH; NH-CO-H; NH-CO-NH₂; Carboxy; CN; CO-NH₂; Y-(CH₂)ₙ-Alkyl; Y-(CH₂)ₙ-Cycloalkyl, Y-(CH₂)ₙ- Heterocycloalkyl, Y-(CH₂)ₙ-Aryl oder Y-(CH₂)ₙ-Heteroaryl mit Y = O, S, NH, O-C(O), C(O)-NH, NH-C(O), NH-S(O) oder NH-SCO)₂, mit n = 0, 1, 2 oder 3, wobei in diesen Resten die Alkyl-, Cycloalkyl-, Heterocycloalkyl-, Aryl- und Heteroarylreste gegebenenfalls substituiert sind, der Cycloalkylrest 3 bis 10 Glieder enthält, der Arylrest 6 bis 10 Glieder enthält und die Heterocycloalkyl- und Heteroarylreste gegebenenfalls substituiert sind und 4 bis 10 Glieder enthalten, von denen 1 bis 4 Heteroatome sind, die unter O, S, N oder NR3 ausgewählt sind; wobei R3 für H oder Alkyl das gegebenenfalls selbst substituiert ist, steht,
wobei alle Alkyl-, Alkoxy-, Alkylthio-, Cycloalkyl- Heterocycloalkyl-, Aryl- und Heteroarylreste der Substituenten der Produkte der Formel (I) gegebenenfalls substituiert sind,
wobei die Produkte der Formel (I) in allen möglichen tautomeren und isomeren Formen, Racemate, Enantiomere und Diastereoisomere, vorliegen, sowie die Additionssalze der Produkte der Formel (I) mit anorganischen und organischen Säuren oder mit anorganischen und organischen Basen.

4. Produkte der Formel (I) nach Anspruch 1, worin
A1, A2, A3 und A4 gleich oder verschieden sind und für CRa oder N stehen;
Ra ausgewählt ist aus der Gruppe bestehend aus H; Halogen; Hydroxy; Mercapto; Amino; Y-(CH₂)ₙ-Alkyl; Y-(CH₂)ₙ-Cycloalkyl, Y-(CH₂)ₙ-Heterocycloalkyl, Y-(CH₂)ₙ-Aryl oder Y-(CH₂)ₙ-Heteroaryl mit Y = O, mit n = 0, 1, 2 oder 3, wobei in diesen Resten alle Alkyl-, Cycloalkyl-, Heterocycloalkyl-, Aryl- und Heteroarylreste gegebenenfalls substituiert sind und die Heterocycloalkyl- und Heteroarylreste 4 bis 10 Glieder enthalten, von denen 1 bis 4 Heteroatome sind, die unter O, S, N oder NR3 ausgewählt sind, wobei R3 für H oder Alkyl, das gegebenenfalls selbst substituiert ist, steht, wobei die anderen Substituenten R1, R1', R2 und R2' der Produkte der Formel (I) unter einer der obigen Definitionen ausgewählt sind,
wobei die Produkte der Formel (I) in allen möglichen tautomeren und isomeren Formen, Racemate, Enantiomere und Diastereoisomere, vorliegen, sowie die Additionssalze der Produkte der Formel (I) mit anorganischen und organischen Säuren oder mit anorganischen und organischen Basen.

5. Produkte der Formel (I) nach einem der anderen Ansprüche, worin A1, A2, A3 und A4 gleich oder verschieden sind und für CRa oder N stehen;
Ra ausgewählt ist aus der Gruppe bestehend aus H; Halogen; Hydroxy; Y-(CH₂)ₙ-Alkyl; Y- (CH₂)ₙ-Cycloalkyl, Y-(CH₂)ₙ-Heterocycloalkyl, Y-(CH₂)ₙ-Aryl oder Y-(CH₂)ₙ-Heteroaryl mit Y = O und mit n = 2 oder 3, wobei alle Alkyl-, Cycloalkyl-, Heterocycloalkyl-, Aryl- und Heteroarylreste gegebenenfalls substituiert sind und die Heterocycloalkyl- und Heteroarylreste 4 bis 10 Glieder enthalten, von denen 1 bis 4 Heteroatome sind, die unter O, S, N oder NR3 ausgewählt sind, wobei R3 für H oder Alkyl, das gegebenenfalls selbst substituiert ist, steht,
wobei die anderen Substituenten R1, R1', R2 und R2' der Produkte der Formel (I) unter einer -der obigen Definitionen ausgewählt sind,
wobei die Produkte der Formel (I) in allen möglichen tautomeren und isomeren Formen, Racemate, Enantiomere und Diastereoisomere, vorliegen, sowie die Additionssalze der Produkte der Formel (I) mit anorganischen und organischen Säuren oder mit anorganischen und organischen Basen.

6. Produkte der Formel (I) nach einem der anderen Ansprüche, worin A1, A2, A3 und A4 so beschaffen sind, daß entweder alle 4 Gruppen für CRa stehen oder eine Gruppe für CRa steht und die anderen drei Gruppen gleich oder verschieden sind und für N oder CRa stehen, wobei Ra für H; Halogen; Hydroxy oder Alkoxy steht;
wobei die anderen Substituenten R1, R1', R2 und R2' der Produkte der Formel (I) unter einer der obigen Definitionen ausgewählt sind,
wobei die Produkte der Formel (I) in allen möglichen tautomeren und isomeren Formen, Racemate, Enantiomere und Diastereoisomere, vorliegen, sowie die Additionssalze der Produkte der Formel (I) mit anorganischen und organischen Säuren oder mit anorganischen und organischen Basen.

7. Produkte der Formel (I) nach einem der anderen Ansprüche, worin R2 und R'2 gleich oder verschieden sind und unabhängig voneinander aus der Gruppe bestehend aus H, Halogen, Methyl, Ethyl, Amino, Methoxy, CH₂-NH₂ , CH₂-NHalk, CH₂-N(alk) ₂, CH₂-OH, CH₂-Oalk ausgewählt sind,
p und p' gleich oder verschieden sind und für ganze Zahlen mit einem Wert von 1 bis 4 bzw. 1 bis 3 stehen;
wobei die anderen Substituenten A1, A2, A3, A4, R1 und R1' der Produkte der Formel (I) unter einer der obigen Definitionen ausgewählt sind,
wobei die Produkte der Formel (I) in allen möglichen tautomeren und isomeren Formen, Racemate, Enantiomere und Diastereoisomere, vorliegen, sowie die Additionssalze der Produkte der Formel (I) mit anorganischen und organischen Säuren oder mit anorganischen und organischen Basen.

8. Produkte der Formel (I) nach einem der anderen Ansprüche, worin R2 und R'2 gleich oder verschieden sind und unabhängig voneinander aus der Gruppe bestehend aus H, Halogen, Methyl, Ethyl, Amino, Methoxy ausgewählt sind,
p und p' gleich oder verschieden sind und für ganze Zahlen mit einem Wert von 1 bis 4 bzw. 1 bis 3 stehen;
wobei die anderen Substituenten A1, A2, A3, A4, R1 und R1' der Produkte der Formel (I) unter einer der obigen Definitionen ausgewählt sind,
wobei die Produkte der Formel (I) in allen möglichen tautomeren und isomeren Formen, Racemate, Enantiomere und Diastereoisomere, vorliegen, sowie die Additionssalze der Produkte der Formel (I) mit anorganischen und organischen Säuren oder mit anorganischen und organischen Basen.

9. Produkte der Formel (I) nach einem der anderen Ansprüche, worin R2 und R'2 gleich oder verschieden sind und unabhängig voneinander aus der Gruppe bestehend aus H, Methyl ausgewählt sind,
p und p' gleich oder verschieden sind und für ganze Zahlen mit einem Wert von 1 bis 4 bzw. 1 bis 3 stehen;
wobei die anderen Substituenten A1, A2, A3, A4, R1 und R1' der Produkte der Formel (I) unter einer der obigen Definitionen ausgewählt sind,
wobei die Produkte der Formel (I) in allen möglichen tautomeren und isomeren Formen, Racemate, Enantiomere und Diastereoisomere, vorliegen, sowie die Additionssalze der Produkte der Formel (I) mit anorganischen und organischen Säuren oder mit anorganischen und organischen Basen.

10. Produkte der Formel (I) nach einem der anderen Ansprüche, worin:
R1 und R1' so beschaffen sind, daß: entweder R1 und R1' gleich oder verschieden sind und so beschaffen sind, daß eine der Gruppen R1 und R1' für ein Wasserstoffatom steht
und die andere der Gruppen R1 und R1' ausgewählt ist aus der Gruppe bestehend aus H; Halogen; Hydroxyl; Amino; NH-CO-H; NH-CO-OH, NH-CO-O-Alkyl, NH-CO-NH₂; Carboxy; CO-NH₂; X-(CH₂)ₘ-alkyl; X- (CH₂)ₘ-Cycloalkyl; X-(CH₂)ₘ-Heterocycloalkyl; X-(CH₂)ₘ-Aryl und X-(CH₂)ₘ-Heteroaryl mit X = Einfachbindung, CH₂, CH=CH, CH₂-C(O), NH, O-C(O), C(O)-NH, -NH-C(O), -NH-C(O)-C(O)-, -NH-C(O)-NH-, NH-CS, NH-S(O) oder NH-S(O)₂, mit m = 0, wobei alle Alkyl-, Cycloalkyl-, Heterocycloalkyl-, Aryl- und Heteroarylreste gegebenenfalls substituiert sind, der Cycloalkylrest 3 bis 10 Glieder enthält, der Arylrest 6 bis 10 Glieder enthält und die Heterocycloalkyl- und Heteroarylreste gegebenenfalls substituiert sind und 4 bis 10 Glieder enthalten, von denen 1 bis 4 Heteroatome sind,die unter O, S, N oder NR3 ausgewählt sind, wobei R3 für H oder Alkyl, das gegebenenfalls selbst substituiert ist, steht,
oder R1 und R'1 zusammen mit dem Kohlenstoffatom, an das sie gebunden sind, einen =O-; =S-; =N-OH-; =N-NH₂"; =N-NH-CO-NH₂-, =CH-OH-; =Y₁-(CH₂)ₘ-Aryl- oder =Y₁-(CH₂)ₘ-Heteroarylrest, worin Y₁ für CH, CH-CO-, CH-CO-NH, N, N-O oder N-NH- steht, mit m = 0, 1 oder 2, wobei Aryl und Heteroaryl wie oben definiert und gegebenenfalls substituiert sind, bilden,
oder R1 und R'1 zusammen mit dem Kohlenstoffatom, an das sie gebunden sind, einen teilweise gesättigten Heterocyclus bilden, der aus 5 bis 6 Gliedern besteht und gegebenenfalls 1 bis 3 unter O, S, N oder NR4 ausgewählte Heteroatome enthält, wobei R4 für H oder Alkyl, das gegebenenfalls selbst substituiert ist, steht,
wobei die anderen substituenten A1, A2, A3, A4, R2 wobei die der Produkte der Formel (I) Unter A4, R2 und R2' der Produkte der Formel (I) unter einer der obigen Definitionen ausgewählte sind,
wobei die Produkte der Formel (I) in allen möglichen tautomeren und isomeren Formen, Racemate, Enantiomere und Diastereoisomere, vorliegen, sowie die Additionssalze der Produkte der Formel (I) mit anorganischen und organischen Säuren oder mit anorganischen und organischen Basen.

11. Produkte der Formel (I) nach Anspruch 1, worin A1, A2, A3 und A4 gleich oder verschieden sind und für CRa oder N oder NRb stehen, wobei Rb für CH₃ oder OH steht;
Ra ausgewählt ist aus der Gruppe bestehend aus H; CH₃; CH₂-NH₂; Halogen; CF₃; Hydroxy,; OCF₃; SO₂-NH₂; SO₂-N(CH₃)₂; Mercapto; Nitro; Amino; NH(CH₃); N(CH₃)₂; NH-OH; NH-CO-H; NH-CO-NH₂; freiem Carboxy oder mit einem Alkylrest, der gegebenenfalls selbst substituiert ist, verestertem Carboxy; CO₂ CH₃; CO₂-(CH₂)₃-N(CH₃)₂; CN; CO-NH₂; CO-N (CH₃)₂; CO-CH₃, CO-(CH₂)₃-O-CH₃); Morpholinyl; Piperazinyl-CH3; Imidazolinyl-CH₃ Diazepin-CH₃; -CO-Piperazinyl-CH₃ ; -CO-Pyrrolidinyl ; Y- (CH₂)ₙ-Alkyl; Y-(CH₂)ₙ-Cycloalkyl, Y-(CH₂)ₙ-Heterocycloalkyl, Y-(CH2)ₙ-Aryl oder Y- (CH₂)ₙ-Heteroaryl, wobei Y für eine Einfachbindung steht oder auch = O, -C(O)-NH-, -C(O)N(CH₃)-; CO; mit n = 0, 1, 2 oder 3, wobei in diesen Resten die Alkyl-, Cycloalkyl-, Heterocycloalkyl-, Aryl- und Heteroarylreste gegebenenfalls substituiert sind, der Cycloalkylrest 3 bis 10 Glieder enthält, der Arylrest 6 bis 10 Glieder enthält und die Heterocycloalkyl- und Heteroarylreste gegebenenfalls substituiert sind und 4 bis 10 Glieder enthalten, von denen 1 bis 4 Heteroatome sind, die unter O, S, N oder NR3 ausgewählt sind, wobei R3 für H oder Alkyl, das gegebenenfalls selbst substituiert ist, steht,
R1 und R1' so beschaffen sind, daß: eine der Gruppen R1 und R1' für ein Wasserstoffatom steht und die andere der Gruppen R1 und R1' ausgewählt ist aus der Gruppe bestehend aus X-(CH₂)ₘ-Heterocycloalkyl ; X- (CH₂)ₘ-Aryl und X-(CH₂)ₘ-Heteroaryl, und insbesondere X-(CH₂)ₘ-Heteroaryl, wobei X für -O-C(O), -NH-C(O), NH-CS, -NH-CO-CH₂-O- ; -NH-CO-CH₂-S-CH₂-CO-NH-; -NH-CO-(CH₂)₂-SO₂; -NH-CO-CH₂-N(CH₃)-CO- steht und m = 0
oder R1 und R'1 zusammen mit dem Kohlenstoffatom, an das sie gebunden sind, einen =N-OH- oder =N-NH₂-Rest bilden,
R2 und R'2 gleich oder verschieden sind und unabhängig voneinander ausgewählt sind aus der Gruppe bestehend aus H, Halogen, Methyl, Ethyl, Amino, Methoxy, CH₂-NH₂, CH₂-NHalk, CH₂-N(alk)₂, CH₂-OH, CH₂-Oalk,
p und p' gleich oder verschieden sind und für ganze Zahlen mit einem Wert von 1 bis 4 bzw. 1 bis 3 stehen;
wobei alle Alkyl-, Alkoxy-, Alkylthio-, Cycloalkyl-, Heterocycloalkyl-, Aryl- und Heteroarylreste der Substituenten der Produkte der Formel (I) gegebenenfalls substituiert sind,
wobei die Produkte der Formel (I) in allen möglichen tautomeren und isomeren Formen, Racemate, Enantiomere und Diastereoisomere, vorliegen, sowie die Additionssalze der Produkte der Formel (I) mit anorganischen und organischen Säuren oder mit anorganischen und organischen Basen.

12. Produkte der Formel (I) nach einem der anderen Ansprüche, worin:
R1 und R1' so beschaffen sind, daß: eine der Gruppen R1 und R1' für ein Wasserstoffatom steht und die andere der Gruppen R1 und R1' ausgewählt ist aus der Gruppe bestehend aus X-(CH₂)ₘ-Heteracycloalkyl ; X- (CH₂)ₘ-Aryl und X-(CH₂)m-Heteroaryl, und insbesondere X-(CH₂)ₘ-Heteroaryl, wobei X für -O-C(O), -NH-C(O) oder NH-CS steht und m = 0
oder R1 und R'1 zusammen mit dem Kohlenstoffatom, an das sie gebunden sind, einen =N-OH- oder =N-NH₂-Rest bilden,
wobei die Heterocycloalkyl-, Aryl- und Heteroarylreste gegebenenfalls substituiert sind,
wobei die anderen Substituenten A1, A2, A3, A4, R2 und R2' der Produkte der Formel (I) unter einer der obigen Definitionen ausgewählt sind,
wobei die Produkte der Formel (I) in allen möglichen tautomeren und isomeren Formen, Racemate, Enantiomere und Diastereoisomere, vorliegen, sowie die Additionssalze der Produkte der Formel (I) mit anorganischen und organischen Säuren oder mit anorganischen und organischen Basen.

13. Produkte der Formel (I) nach einem der vorhergehenden Ansprüche mit den folgenden Namen:
- 4-(3H-Imidazo[4,5-c]pyridin-2-yl)-9H-fluoren-9-onoxim (Z, E)
- N-[4-(1H-Imidazo[4,5-c]pyridin-2-yl)-9H-fluoren-9-yliden]hydrazin, 60/40-Gemisch von E- und Z-Isomer
- [4-(3H-Imidazo[4,5-c]pyridin-2-yl-9H-fluoren-9(R,S)-yl]amid von Chinolin-5-carbonsäure rechtsdrehendes Enantiomer des [4-(3H-Imidazo[4,5-c]pyridin-2-yl)-9H-fluoren-9(R,S)-yl]-amids von Chinolin-5-carbonsäure
- 4-(3H-Imidazo[4,5-c]pyridin-2-y1)-9H-fluoren-9-onoxim (E).
- Trifluoracetat des [4-(3H-Imidazo[4,5-c]pyridin-2-yl)-9H-fluoren-9(R,S)-yl]amids von 1H-Pyrazol-4-carbonsäure
- Trifluoracetat von N-[4-(3H-Imidazo[4,5-c]-pyridin-2-yl)-9H-fluoren-9(R,S)-yl]succinamid
- Trifluoracetat des- [4-(3H-zmidazo[4,5-c]pyridin-2-yl)-9H-fluoren-9(R,S)-yl]amids, von 1.H-Benzotriazol-5-carbonsäure
- [4-(3H-Imidazo[4,5-c]pyridin-2-yl)-9H-fluoren-9(R,S)-yl]amid von 1H-Indazol-5-carbonsäure
- [4-(3H-Imidazo[4,5-c]pyridin-2-yl)-9H-fluoren-9(R,S)-yl]amid von 1H-Indol-6-carbonsäure
- N-[4-(3H-Imidazo[4,5-c]pyridin-2-yl)-9H-fluoren-9(R,S)-yl]isonicotinamid
- [4-(3H-Imidazo[4,5-c]pyridin-2-yl)-9H-fluoren-9(R,S)-yl]amid von 1H-Indol-4-carbonsäure
- Trifluoracetat des [4-(3H-Imidazo[4,5-c]pyridin-2-yl)-9H-fluoren-9(R,S)-yl]amids von Chinolin-5-thiocarbonsäure
- 2-Acetylamino-N-[4-(3H-imidazo[4,5-c]pyridin-2-yl)-9H-fluoren-9(R,S)-yl]isonicotinamid
- [4-(3H-Imidazo [4,5-c] pyridin-2-yl)-9H-fluoren-9(R,S)-yl]amid von Chinolin-4-carbonsäure
- 2-Amino-N- [4- (3H-imidazo [4,5-c] pyridin-2-yl) -9H-fluoren-9(R,S)-yl]isonicotinamid
- 2-Ethyl-N-[4-(3H-imidazo[4,5-c]pyridin-2-yl)-9H-fluoren-9(R,S)-yl]isonicotinamid
- [4-(6-Fluor-1H-benzimidazol-2-yl)-9H-fluoren-9(R,S)-yl]amid von Chinolin-5-carbonsäure
- 2-Chlor-N-[4-(3H-imidazo[4,5-c]pyridin-2-yl)-9H-fluoren-9(R,S)-yl]isonicotinamid
- [4-(3H-imidazo[4,5-c]pyridin-2-yl)-9H-fluoren-9(R,S)-yl]amid von 2-Aminopyrimidin-4-carbonsäure
- [4-(3H-Imidazo[4,5-c]pyridin-2-yl)-9H-fluoren-9(R,S)-yl]amid von 1H-Pyrrolo[2,3-b]pyridin-4-carbonsäure
- 2-Hydroxymethyl-N-[4-(3H-imidazo[4,5-c]pyridin-2-yl)-9H-fluoren-9(R,S)-yl]isonicotinamid
- N-[4-(3H-Imidazo[4,5-c]pyridin-2-yl)-9H-fluoren-9(R,S)-yl]-3-methylisonicotinamid
- Hydrochlorid des [4-(3H-Imidazo[4,5-c]pyridin-2-yl)-9H-fluoren-9(R,S)-yl]amids von 1,8-Naphthyridin-4-carbonsäure
- [4-(3H-Imidazo[4,5-c]pyridin-2-yl)-9H-fluoren-9(R,S)-yl]amid von 1H-Pyrrolo[2,3-b]pyridin-3-carbonsäure
- [4-(3H-Imidazo[4,5-c]pyridin-2-yl)-9H-fluoren-9(R,S)-yl]amid von 1-Methyl-1H-pyrrolo[2,3-b]-pyridin-3-carbonsäure
- 2-Brom-N-[4-(3H-imidazo[4,5-c]pyridin-2-yl)-9H-fluoren-9(R,S)-yl]isonicotinamid
- 3-Hydroxy-N-[4-(3H-imidazo[4,5-c]pyridin-2-yl)-9H-fluoren-9(R,S)-yl]isonicotinamid
- 3-Amino-N-[4-(3H-imidazo[4,5-c]pyridin-2-yl)-9H-fluoren-9(R,S)-yl]isonicotinamid
- Methylester von 4-[4-(3H-Imidazo[4,5-c]pyridin-2-yl)-9H-fluoren-9(R,S)-ylcarbamoyl]pyridin-2-carbonsäure
- [4-(3H-Imidazo[4,5-c]pyridin-2-yl)-9H-fluoren-9(R,S)-yl]amid von 1H-Indazol-4-carbonsäure
- N-[4-(3H-Imidazo[4,5-c]pyridin-2-yl)-9H-fluoren-9(R,S)-yl]-2-methylaminaisonicotinamid
- N-[4-(6-Hydroxy-1H-benzimidazol-2-yl)-9H-fluoren-9 (R,S)-yl]isonicotinamid
- [4-(3H-Imidazo[4,5-c]pyridin-2-yl)-9H-fluoren-9(R,S)-yl]amid von 6-Brom-3H-imidazo[4,5-b]-pyridin-7-carbonsäure
- 4-(3H-Imidazo[4,5-c]pyridin-2-yl)-9H-fluoren-9(R,S)-ylester von Isonicotinsäure,
- N-[4-(3H-Imidazo[4,5-c]pyridin-2-yl)-9H-fluoren-9(R,S)-yl]amid von 2-tert.-Butoxycarbonylamino-isonikotinsäure
- N-[4-(3H-Imidazo[4,5-c]pyridin-2-yl)-9H-fluoren-9(R,S)-yl]amid von 3-Chlor-6-methoxychinolin-4-carbonsäure
- N-[4-(3H-Imdazo[4,5-c]pyridin-2-yl)-9H-fluoren-9(R,S)-yl]amid von 3-Hydroxychinolin-4-carbonsäure
- N-[4-(3H-Imidazo[4,5-c]pyridin-2-yl)-9H-fluoren-9(R,S)-yl]amid von 2-Amino-5-chlorpyrimidin-4-carbonsäure
- [4-(3H-Imidazo[4,5-c]pyridin-2-yl)-9H-fluoren-9(R,S)-yl]amid von 3-Brorn-1H-pyrrolo[2,3-b]-pyridin-4-carbonsäure
- rechtsdrehendes Enantiomer des [4-(3H-Imidazo-[4,5-c]pyridin-2-yl)-9H-fluoren-9(R,S)-yl]amids von 1H-Pyrrolo[2,3-b]pyridin-4-carbonsäure
- N-[4-(3H-Imidazo[4,5-c]pyridin-2-yl)-9H-fluoren-9(R,S)-yl]amid von 9H-Purin-6-carbonsäure
- N-[4-(3H-Imidazo[4,5-c]pyridin-2-yl)-9H-fluoren-9(R,S)-yl]amid von 2-Amino-6-methylpyrimid-4-carbonsäure
- [4-(3H-Imidazo[4,5-c]pyridin-2-yl)-9H-fluoren-9(R,S)-yl]amid von 5-Amino-3H-1,2,3-triazol-4-carbonsäure
- N-[4-(3H-Imidazo[4,5-c]pyridin-2-yl)-9H-fluoren-9(R,S)-yl]amid von 6-Methyl-2-methylamino-pyrimidin-4-carbonsäure
- N-[4-(3H-Imidazo[4,5-c]pyridin-2-yl)-9H-fluoren-9(R,S)-yl]amid von 6-Methoxychinolin-4 -carbonsäure 3,5-Dihydroxy-N-[4-(3H-imidazo[4,5-c]pyridin-2-yl)-9H-fluoren-9(R,S)-yl]benzamid.
- N-[4-(3H-Imidazo[4,5-c]pyridin-2-yl)-9H-fluoren-9(R,S)-yl]amid von Pyrimidin-4-carbonsäure
- 4-Hydroxy-N-[4-(3H-imidazo [4,5-c] pyridin-2-yl)-9H-fluoren-9(R,S)-yl]benzamid
- 2,4-Dihydroxy-N-[4-(3H-imidazo[4,5-c]pyridin-2-yl)-9H-fluoren-9(R,S)-yl] benzamid
- [4-(5-Cyano-6-fluor-1H-benzimidazol-2-yl)-9H-fluoren-9(R,S)-yl]amid von 1H-Pyrrolo[2,3-b]-pyridin-4-carbonsäure
- 4-(1H-Benzimidazol-2-yl)-9H-fluoren-9(R,S)-yl]-amid von 2-Amino-5-chlorpyrimidin-4-carbonsäure
- [4-(5-Fluor-1H-benzimidazol-2-yl)-9H-fluoren-9(R,S)-yl]amid von 2-Amino-5-chlorpyrimidin-4-carbonsäure
[4-(S-Fluor-1H-benzimidazol-2-yl)-9H-fluoren-9(R,S)-yl]amid von 1H-Pyrrolo[2,3-b]pyridin-4-carbonsäure
[4-(3H-Imidazo[4,5-c]pyridin-2-yl)-9H-fluoren-9(R,S)-yl]amid von 2,3-Dimethyldhinaxalin-5-carbonsäure
- [4-(3H-Imidazo[4,5-c]pyridin-2-yl)-9H-fluoren-9(R,*S)*-yl]amid von 3-Amino-1H-pyrazol-4-carbonsäure
- rechtsdrehendes Enantiomer des N-[4-(3H-Imidazo[4,5-c]pyridin-2-yl)-9H-fluoren-9(R,S)-yl]-amids von 2-Amino-5-chlorpyrimidin-4-carbonsäure
- linksdrehendes Enantiomer des N-[4-(3H-Imidazo[4,5-c]pyridin-2-yl)-9H-fluoren-9-(R,S)-yl]-amids von 2-Amino-5-chlorpyrimidin-4-carbonsäure
- [4-(3H-Imidazo[4,5-c]pyridin-2-yl)-9H-fluoren-9(R,S)-yl]amid von 3-Methylchinoxalin-5-carbonsäure
- [4-(3H-Imidazo[4,5-c]pyridin-2-yl)-9H-fluoren-9(R,S)-yl]amid von Chinoxalin-5-carbonsäure
- [4-(9H-Purin-8-yl)-9H-fluoren-9(R,S)-yl]amid von 1H-Pyrrolo[2,3-b]pyridin-4-carbonsäure
- [4-(1H-Benzimidazol-2-yl)-9H-fluoren-9(R,S)-yl]-amid von 1H-Pyrrolo[2,3-b]pyridin-4-carbonsäure
- [4-(5-Trifluormethyl-1H-benzimidazol-2-yl)-9H-fluoren-9 (R,S)-yl]amid von 1H-Pyrrolo[2,3-b]-pyridin-4-carbonsäure
- [4-(5-Methoxycarbonyl-1H-benzimidazol-2-yl)-9H-fluoren-9(R,S)-yl]amid von 1H-Pyrrolo[2,3-b]-pyridin-4-carbonsäure
- [4-(5-Carboxy-1H-benzimidazol-2-yl)-9H-fluoren-9(R,S)-yl]amid von 1H-Pyrrolo[2,3-b]pyridin-4-carbonsäure
- [4-(4-Methyl-1H-benzimidazol-2-yl)-9H-fluoren-9(R,S)-yl]amid von 1H,Pyrrolo[2,3-b]pyridin-4-carbonsäure
- [4-(5-Carboxamido-1H-benzimidazol-2-yl)-9H-fluoren-9(R,S)-yl]amid von 1H-Pyrrolo[2,3-b]-pyridin-4-carbonsäure
- [4-(6-Sulfamoyl-1H-benzimidazol-2-yl)-9H-fluoren-9(R,S)-yl]amid von 1H-Pyrrolo[2,3-b] pyridin-4-carbonsäure
- [4-(5-Trifluormethoxy-1H-benzimidazol-2-yl)-9H-fluoren-9(R,S)-yl]amid von 1H-Pyrrolo[2,3-b]-pyridin-4-carbonsäure
- [4-(5-Cyano-1H-benzimidazol-2-yl)-9H-fluoren-9(R,S)-yl]amid von 1H-pyrrolo[2,3-b]pyridin-4-carbonsäure
- D-Diastereoisomer des [4-(3H-Imidazo[4,5-c]-pyridin-2-yl)-9H-fluoren-9(R,S)-yl]amids von 2-(3-Acetyl-2,2-dimethylcyclobutan-1-yl)essigsäure
- [4-(6-Chlor-1H-benzimidazol-2-yl)-9H-fluoren-9(R,S)-yl]amid von 1H-Pyrrolo[2,3-b]pyridin-4 carbonsäure
- [4-(5-Fluor-6-morpholino-1H-benzimidazol-2-yl)-9H-fluoren-9(R,S)-yl]amid von 1H-Pyrrolo[2,3-b] pyridin-4-carbonsäure
- [4-(6-Chlor-5-fluor-1H-benzimidazol-2-yl)-9H-fluoren-9(R,S)-yl]amid von 1H-Pyrrolo[2,3-b]-pyridin-4-carbonsäure
- [4-(4,5,6,7-Tetrahydro-1H-benzimidazol-2-yl)-9H-fluoren-9(R,S)-yl]amid von 1H-Pyrrolo[2,3-b] pyridin-4-carbonsäure
- [2-Amino-5-(1H-benzimidazol-2-yl)-9H-fluoren-9 (R, S) -yl] amid von 1H-Pyrrolo[2,3-b]pyridin-4-carbonsäure
- [4-(5-Hydroxy-5H-imidazo[4,5-c]pyridin-2-yl)-9H-fluoren-9(R,S)-yl]amid von 1H-Pyrrolo[2,3-b]-pyridin-4-carbonsäure
- [4-(5-Hydroxy-1H-benzimidazol-2-yl)-9H-fluoren-9(R,S)-yl]amid von 1H-Pyrrolo[2,3-b]pyridin-4-carbonsäure
- [4-(5-Methylaminocarbonyl-1H-benzimidazol-2-yl)-9H-fluoren-9(R,S)-yllamid von 1H-Pyrrolo[2,3-b]-pyridin-4-carbonsäure
- [4-(5-Dimethylaminocarbonyl-1H-benzimidazol-2-yl)-9H-fluoren-9(R,S)-yl]amid von 1H-Pyrrolo[2,3-b]pyridin-4-carbonsäure
- [4-5-(2-Dimethylamino-ethyl)aminocarbonyl-1H-benzimidazol-2-yl)-9H-fluoren-9(R,S)-yl]amid von 1H-Pyrrolo[2,3-b]pyridin-4-carbonsäure
- [4-(4,5,6,7-Tetrafluor-1H-benzimidazol-2-yl)-9H-fluoren-9(R,S)-yl]amid von 1H-pyrrolo[2,3-b]-pyridin-4-carbonsäure
- 4-[5-(3-Methoxypropyl)aminocarbonyl-1H-benzimidazol-2-yl)-9H-fluoren-9(R,S)-yl]amid von 1H-Pyrrolo[2,3-b]pyridin-4-carbonsäure
- 4-[5-(4-Methylpiperazin-1-yl)carbonyl-1H-benzimidazol-2-yl)-9H-fluoren-9(R,S)-yl] amid von 1H-Pyrrolo[2,3-b]pyridin-4-carbonsäure
- [4-(6-Dimethylsulfamoyl-1H-benzimidazol-2-yl) 9H-fluoren-9(R,S)-yl]amid von 1H-Pyrrolo[2,3-b]-pyridin-4-carbonsäure
- [4-[-5-(Pyrrolidin-1-yl)carbonyl)-1H-benzimidazol-2-yl)-9H-fluoren-9(R,S)-yl]amid von 1H-Pyrrolo[2,3-b]pyridin-4-carbonsaure
- rechtsdrehendes Enantiomer von 2-{9(R,S)-[(1H-Pyrrolo[2,3-b]pyridin-4-carbonyl)amino]-9H-fluoren-4-yl}-1H-benzimidazol-5-carbonsäure
- ({4-{5-[2-(Pyrrolidin-1-yl)ethylaminocarbonyl]-1H-benzimidazol-2-yl}-9H-fluoren-9(R,S)-yl}}amid von 1H-Pyrrolo[2,3-b]pyridin-4-carbonsaure
- [4-(6-Dimethylamino-1H-benzimidazol-2-yl)-9H-fluoren-9(R,S)-yl]amid von 1H-Pyrrolo[2,3-b]-pyridin-4-carbonsäure
- {4-[6-(4-Methylpiperazin-1-yl)-1H-benzimidazol-2-yl]-9H-fluoren-9(R,S)-yl}-amid von 1H-Pyrrolo-[2,3-b]pyridin-4-carbonsäure
- [[6-(Methyl-4 (5)-imidazolin-2-yl)-1H-benzimidazol-2-yl]-9H-fluoren-9(R,S)-yl]amid von 1H-Pyrrolo[2,3-b]pyridin-4-carborisäure
- {{4-{5-[(3-Dimethylaminopropyl)aminocarbonyl]-1H-benzimidazol-2yl-}-9H-fluoren-9(R,S)-yl}}amid von IH-Pyrrolo[2,3-b]pyridin-4-carbonsäure
- {4-[5-Fluor-6-(4-methylpiperazin-1-yl)-1H-benzimidazol-2-yl]-9H-fluoren-9(R,S)-yl}-amid von 1H-Pyrrolo[2,3-b]pyridin-4-carbonsäure
- {{4-{5-[(3-Dimethylaminopropyl)carbonyloxy]-1H-benzimidazol-2-yl}-9H-fluoren-9(R,S)-yl}}-amid von 1H-Pyrrolo[2,3-b]pyridin-4-carbonsäure
- [4-(6-Amino-1H-benzimidazol-2-yl)-9H-fluoren-9-(R,S)yl] amid von 1H-Pyrrolo[2,3-b]pyridin-4-carbonsäure
- [4-(6-Methyl-1H-benzimidazol-2-yl)-9H-fluoren-9(R,S)-yl]amid von 1H-Pyrrolo[2,3-b]pyridin-4-carbonsäure
- {{4-{5-[(3-Hydroxypropyl)aminocarbonyl]-1H-benzimidazol-2-yl}-9H-fluoren-9(R,S)-yl}}-amid von 1H-Pyrrolo[2,3-b]pyridin-4-carbonsäure
- rechtsdrehendes Enantiomer des {4-[5-cyano-1H-benzimidazol-2-yl]-9H-fluoren-9(R,S)-yl}amids von 1H-Pyrrolo[2,3-b]pyridin-4-carbonsäure [4-(3H-Imidazo[4,5-c]pyridin-2-yl)-9H-fluoren-9(R,S)-yl]amid von 7H-Pyrrolo[2,3-d]pyrimidin-4-carbonsäure
- [4-(6-Amino-1H-benzimidazol-2-yl)-9H-fluoren-9(R,S)-yl]amid von 1H-Pyrrolo[2,3-b]pyridin-4-carbonsäure
- rechtsdrehendes Enantiomer des {4-[5-Fluor-1H-benzimidazol-2-yl]-9H-fluoren-9(R,S)-yl}-amids von 1H-Pyrrolo[2,3-b]pyridin-4-carbonsäure
- Methylester von 2-{9(R,S)-[(1H-Pyrrolo[2,3-b]-pyridin-4-carbonyl)amino]-9H-fluoren-4-yl}-1H-imidazo[4,5-c]pyridin-6-carbonsäure
- [4-(6-Fluor-5-methoxy-1H-benzimidazol-2-yl)-9H-fluoren-9(R,S)-yl]amid von 1H-Pyrrolo[2,3-b]-pyridin-4-carbonsäure
- [4-(6-Fluor-5-hydroxy-1H-benzimidazol-2-yl)-9H-fluoren-9(R,S)-yl]amid von 1H-Pyrrolo[2,3-b]-pyridin-4-carbonsäure
- {4-[5-Fluor-6-(3-methoxypropoxy)-1H-benzimidazol-2-yl]-9H-fluoren-9(R,S)-yl}-amid von 1H-pyrrolo[2,3-b]pyridin-4-carbonsäure
- {4-[6-(3-Dimethylaminopropoxy)-5-fluor-1H-benzimidazol-2-yl]-9H-fluoren-9(R,S),-yl}-amid von 1H-Pyrrolo[2,3-b]pyridin-4-carbonsäure,
wobei die Produkte der Formel (I) in allen möglichen tautomeren und isomeren Formen, Racemate, Enantiomere und Diastereoisomere, vorliegen, sowie die Additionssalze der Produkte der Formel (I) mit anorganischen und organischen Säuren oder mit anorganischen und organischen Basen.

14. Produkte der Formel (I) nach einem der vorhergehenden Ansprüche mit den folgenden Namen:
- 4-(3H-Imidazo[4,5-c]pyridin-2-yl)-fluoren-9-on-oxim (Z, E)
- N-[4-(1H-Imidazo[4,5-c]pyridin-2-yl)-fluoren-9-yliden]hydrazin, 60/40-Gemisch von E- und Z- Isomer
- [4-(3H-Imidazo [4,5-c]pyridin-2-yl)-9H-fluoren-9(R,S)-yl]amid von Chinolin-5-carbonsäure
- rechtsdrehendes Enantiomer des [4-(3H-Imidazo-[4,5-c]pyridin-2-yl)-9H-fluoren-9(R,S)-yl]amids von Chinolin-5-carbonsäure
- 4-(3H-Imidazo[4,5-c]pyridin-2-yl)-fluoren-9-on-oxim (E)
- trifluoracetat des [4-(3H-Imidazo[4,5-c]pyridin-2-yl)-9H-fluoren-9(R,S)-yl]amids von 1H-Pyrazol-4-carbonsäure
- Trifluoracetat von N-[4-(3H-imidazo[4,5-c]-pyridin-2-yl)-9H-fluoren-9(R,S)-yl]succinamid
- Trifluoracetat des [4-(3H-Imidazo[4,5-c]pyridin-2-yl)-9H-fluoren-9(R,S)-yl]amids von 1H-Benzotriazol-5-carbonsäure
- [4-(3H-Imidazo[4,5-c]pyridin-2-yl)-9H-fluoren-5(R,S)-yl]amid von 1H-Indazol-5-carbonsäure
- [4-(3H-Imidazo[4,5-c]pyridin-2-yl)-9H-fluoren-9(R,S)-yl]amid von 1H-Indol-6-carbonsäure
- N-[4-(3H-Imidazo[4,5-c]pyridin-2-yl)-9H-fluoren-9(R,S)-yl]isonicotinamid
- [4-(3H-Imidazo[4,5-c]pyridin-2-yl)-9H-fluoren-9(R,S)-yl]amid von 1H-Indol-4-carbonsäure
- Trifluoracetat des [4-(3H-Imidazo[4,5-c]pyridin-2-yl)-9H-fluoren-9(R,S)-yl]amids von Chinolin-5-thiocarbonsäure
- 2-Acetylamino-N-[4-(3H-imidazo[4,5-c]pyridin-2-yl)-9H-fluoren-9(R,S)-yl]isonicotinamid
- [4-(3H-Imidazo[4,5-c]pyridin-2-yl)-9H-fluoren-9(R,S)-yl]amid von Chinolin-4-carbonsäure
- 2-Amino-N-[4-(3H-imidazo[4,5-c]pyridin-2-yl)-9H-fluoren-9(R,S)-yl]isonicotinamid
- 2-Ethyl-N-[4-(3H-imidazo[4,5-c]-pyridin-2-yl)-9H-fluoren-9(R,S)-yl]isonicotinamid
- [4-(6-Fluor-1H-benzimidazol-2-yl)-9H-fluoren-9(R,S)-yl]amid von Chinolin-5-carbonsäure
- 2-Chlor-N-[4-(3H-imidazo[4,5-c]pyridin-2-yl)-9H-fluoren-9(R,S)-yl]isonicotinamid
- [4-(3H-Imidazo[4,5-c]pyridin-2-yl)-9H-fluoren-9-yl]amid von 2-Aminopyrimidin-4-carbonsäure
- [4-(3H-Imidazo[4,5-c]pyridin-2-yl)-9H-fluoren-9(R,S)-yl]amid von 1H-Pyrrolo[2,3-b]pyridin-4-carbonsäure
- 2-Hydroxymethyl-N-[4-(3H-imidazo[4,5-clpyridin-2-yl)-9H-fluoren-9(R,S)-yl]isonicotinamid
- N-[4-(3H-Imidazo[4,5-c]pyridin-2-yl)-9H-fluoren-9(R,S)-yl]-3-methylisonicotin amid
- Hydrochlorid von [4-(3H-Imidazo[4,5-c]pyridin-2-yl)-9H-fluoren(R,S)-9-yl]amid von 1,8-Naphthyridin-4-carbonsäure
- [4-(3H-Imidazo[4,5-c]pyridin-2-yl)-9H-fluoren-(R,S)-9-yl]amid von 1H-Pyrrolo[2,3-blpyridin-3-carbonsäure
- [4-(3H-Imidazo[4,5-c]pyridin-2-y)-9H-fluoren-(R,S)-9-yl]amid von 1-Methyl-1H-pyrrolo[2,3-b]-pyridin-3-carbonsäure
- 2-Brom-N-[4-(3H-imidazo[4,5-c]pyridin-2-yl)-9H-fluoren-9(R,S)-yl]isonicotinamid
- 3-Hydroxy-N- [4- (3H-imidazo [4,5-c] pyridin-2-yl) - 9H-fluoren-9(R,S)-yl]isonicotinamid,
- 3-Amino-N-[4-(3H-imidazo[4,5-c]pyridin-2-yl)-9H-fluoren-9(R,S)-yl]isonicotinamid
- Methylester von 4-[4-(3H-imidazo[4,5-c]pyridin-2-yl)-9H-fluoren-9(R,S)-ylcarbamoyl]pyridin-2-carbonsäure
- [4-(3H-Imidazo[4,5-c]pyridin-2-yl)-9H-fluoren-(R,S)-9-yl]amid von 1H-Indazol-4-carbonsäure
- N-[4-(3H-Imidazo[4,5-c]pyridin-2-yl)-9H-fluoren-9(R,S)-yl]-2-methylaminoisonicotinamid
- N-[4-(6-Hydroxy-1H-benzimidazol-2-yl)-9H-fluoren-9(R,S)-yl]isonicotinamid
- [4-(3H-Imidazo[4,5-c]pyridin-2-yl)-9H-fluoren-9(R,S)-yl]amid von 6-Brom-3H-imidazo[4,5-b]-pyridin-7-carbonsäure
- 4-(3H-Imidazo[4,5-c]pyridin-2-yl)-9H-fluoren-9-ylester von Isonicotinsäure,
wobei die Produkte der Formel (I) in allen möglichen tautomeren und isomeren Formen, Racemate, Enantiomere und Diastereoisomere, vorliegen, sowie die Additionssalze der Produkte der Formel (I) mit anorganischen und organischen Säuren oder mit anorganischen und organischen Basen.

15. Produkte der Formel (I) nach einem der vorhergehenden Ansprüche mit den folgenden Namen:
- [4-(3H-Imidazo[4,5-c]pyridin-2-yl)-9H-fluoren-9(R,S)-yl]amid von Chinolin-5-carbonsäure rechtsdrehendes Enantiomer des [4-(3H-Imidazo-[4,5-c]pyridin-2-yl)-9H-fluoren-9(R,S)-yl]amids von Chinolin-5-carbonsäure
- 4-(3H-Imidazo[4,5-c]pyridin-2-yl)-fluoren-9-on-oxim (E)
- Trifluoracetat des [4-(3H-Imidazo[4,5-c]pyridin-2-yl)-9H-fluoren-9(R,S)-yl]amids von 1H-Benzotriazol-5-carbonsäure
- N-[4-(3H-Imidazo[4,5-c]pyridin-2-yl)-9H-fluoren-9(R,S)-yl]isonicotinamid
- Trifluoracetat des [4-(3H-Imidazo[4,5-c]pyridin-2-yl)-9H-fluoren-9(R,S)-yl]amids von Chinolin-5-thiocarbonsäure
- 2-Acetylamino-N-[4-(3H-imidazo[4,5-c]pyridin-2-yl)-9H-fluoren-9(R,S)-yl]isonicotinamid
- [4-(3H-Imidazo[4,5-c]pyridin-2-yl)-9H-fluoren-9(R,S)-yl]amid von Chinolin-4-carbonsäure
- 2-Amino-N-[4-(3H-imidazo[4,5-c]pyridin-2-yl)-9H-fluoreh-9(R,S)-yl]isonicotinamid
- 2-Ethyl-N-[4-(3H-imidazo[4,5-c]pyridin-2-yl)-9H-fluoren-9(R,S)-yl]isonicotinamid
- [4-(3H-Imidazo[4,5-c]pyridin-2-yl)-9H-fluoren-9-yl]amid von 2-Aminopyrimidin-4-carbonsäure
- [4-(3H-Imidazo[4,5-c]pyridin-2-yl)-9H-fluoren-9 (R,S)-yl] amid von 1H-Pyrrolo[2,3-blpyridin-4-carbonsäure
- [4-(3H-Imidazo[4,5-c]pyridin-2-yl)-9H-fluoren-(R,S)-9-yl]amid von 1H-Pyrrolo[2,3-b]pyridin-3-carbonsäure
- 2-Brom-N-[4-(3H-imidazo[4,5-c]pyridin-2-yl)-9H-fluoren-9(R,S)-yl]isonicotinamid
- 3-Hydroxy-N-[4-(3H-imidazo[4,5-c]pyridin-2-yl)-9H-fluoren-9(R,S)-yl]isonicotinamid
- 3-Amino-N-[4-(3H-imidazo[4,5-c]pyridin-2-yl)-9H-fluoren-9(R,S)-yl]isonicotinamid
- Methylester von 4-[4-(3H-Imidazo[4,5-c]pyridin-2-yl)-9H-fluoren-9(R,S)-ylcarbamoyl]pyridin-2-carbonsäure
- [4-(3H-Imidazo[4,5-c]pyridin-2-yl)-9H-fluoren-(R,S)-9-yl]amid von 1H-Indazol-4-carbonsäure
- [4-(3H-Imidazo[4,5-c]pyridin-2-yl)-9H-fluoren-9(R,S)-yl] amid von 6-Brom-3H-imidazo[4,5-b]-pyridin-7-carbonsäure
- 4-(3H-Imidazo[4,5-c]pyridin-2-yl)-9H-fluoren-9-ylester von Isonicotinsäure,
wobei die Produkte der Formel (I) in allen möglichen tautomeren und isomeren Formen, Racemate, Enantiomere und Diastereoisomere, vorliegen, sowie die Additionssalze der Produkte der Formel (I) mit anorganischen und organischen Säuren oder mit anorganischen und organischen Basen.

16. Produkte der Formel (I) nach einem der vorhergehenden Ansprüche mit den folgenden Namen:
- [4-(3H-Imidazo[4,5-c]pyridin-2-yl)-9H-fluoren-9(R,S)-yl]amid von Chinolin-5-carbonsäure
- N-[4-(3H-Imidazo[4,5-c]pyridin-2-yl)-9H-fluoren-9(R,S)-yl]isonicotinamid
- 2-Amino-N-[4-(3H-imidazo[4,5-c]pyridin-2-yl)-9H-fluoren-9(R,S)-yl]isonicotinamid
- 2-Ethyl-N-[4-(3H-imidazo[4,5-c]pyridin-2-yl)-9H-fluoren-9(R,S)-yl]isonicotinamid
- [4-(3H-Imidazo[4,5-c]pyridin-2-yl)-9H-fluoren-9-yl]amid von 2-Aminopyrimidin-4-carbonsäure [4-(3H-Imidazo[4,5-c]pyridin-2-yl)-9H-fluoren-9 (R,S)-yl] amid von 1H-Pyrrolo[2,3-b] pyridin-4-carbonsäure
- [4-(3H-Imidazo[4,5-c]pyridin-2-yl)-9H-fluoren-(R,S)-9-yl]amid von 1H-Pyrrolo[2,3-b]pyridin-3-carbonsäure
- 3-Amino-N-[4-(3H-imidazo[4,5-c.]pyridin-2-yl)-9H-fluoren-9(R,S)-yl]isonicotinamid
- [4-(3H-Imidazo[4,5-c]pyridin-2-yl)-9H-fluoren-9(R,B)-yl]amid von 6-Brom-3H-imidazo[4,5-b]-pyridin-7-carbonsäure,
wobei die Produkte der Formel (I) in allen möglichen tautomeren und isomeren Formen, Racemate, Enantiomere und Diastereoisomere, vorliegen, sowie die Additionssalze der Produkte der Formel (I) mit anorganischen und organischen Säuren oder mit anorganischen und organischen Basen.

17. Produkte der Formel (I) nach den Ansprüchen 1 bis 12 sowie ihre Prodrugs, wobei die Produkte der Formel (I) in allen möglichen tautomeren und isomeren Formen, Racemate, Enantiomere und Diastereoisomere, vorliegen, sowie die pharmazeutisch unbedenklichen Additionssalze der Produkte der Formel (I) mit anorganischen und organischen Säuren oder mit anorganischen und organischen Basen als Medikamente.

18. Produkte der Formel (I) nach den Ansprüchen 13 bis 16 sowie ihre Prodrugs, wobei die Produkte der Formel (I) in allen möglichen tautomeren und isomeren Formen, Racemate, Enantiomere und Diastereoisomere, vorliegen, sowie die pharmazeutisch unbedenklichen Additionssalze der Produkte der Formel (I) mit anorganischen und organischen Säuren oder mit anorganischen und organischen Basen als Medikamente.

19. Pharmazeutische Zusammensetzungen, die als Wirkstoff mindestens eines der Medikamente nach den Ansprüchen 17 und 18 enthalten.

20. Pharmazeutische Zusammensetzungen nach den vorhergehenden Ansprüchen, die außerdem Wirkstoffe anderer Medikamente für die Krebschemotherapie enthalten.

21. Pharmazeutische Zusammensetzungen nach einem der vorhergehenden Ansprüche, **dadurch gekennzeichnet, daß** sie als Medikamente verwendet werden, insbesondere für die Krebschemotherapie.

22. Verwendung von Produkten der Formel (I) nach einem der vorhergehenden Ansprüche oder von pharmazeutisch unbedenklichen Salzen der Produkte der Formel (I) zur Herstellung eines Medikaments zur Prävention oder Behandlung einer Erkrankung, die durch die Störung der Aktivität des HSP90-Proteins **gekennzeichnet** ist.

23. Verwendung von Produkten der Formel (I) nach dem vorhergehenden Anspruch, wobei die zu verhindernde oder zu behandelnde Erkrankung bei einem Säugetier auftritt.

24. Verwendung von Produkten der Formel (I) nach einem der vorhergehenden Ansprüche oder von pharmazeutisch unbedenklichen Salzen der Produkte der Formel (I) zur Herstellung eines Medikaments zur Prävention oder Behandlung einer Erkrankung aus der folgenden Gruppe: Störungen der Blutgefäßproliferation, fibrotische Störungen, Störungen der Mesangialzellenproliferation, Stoffwechselstörungen, Allergien, Asthma, Thrombosen, Erkrankungen des Nervensystems, Retinopathien, Psoriasis, rheumatoide Arthritis, Diabetes, Muskeldegeneration, onkologische Erkrankungen, Krebserkrankungen.

25. Verwendung von Produkten der Formel (I) nach einem der vorhergehenden Ansprüche oder von pharmazeutisch unbedenklichen Salzen der Produkte der Formel (I) zur Herstellung eines Medikaments zur Behandlung von Krebserkrankungen.

26. Verwendung von Produkten der Formel (I) nach dem vorhergehenden Anspruch, wobei es sich bei der zu behandelnden Erkrankung um Krebs mit soliden oder flüssigen Tumoren handelt.

27. Verwendung von Produkten der Formel (I) nach dem vorhergehenden Anspruch, wobei es sich bei der zu behandelnden Erkrankung um einen Krebs handelt, der gegenüber Cytotoxika resistent ist.

28. Verwendung von Produkten der Formel (I) nach einem der vorhergehenden Ansprüche oder von pharmazeutisch unbedenklichen Salzen der Produkte der Formel (I) zur Herstellung eines Medikaments zur Behandlung von Krebserkrankungen, u.a. Lungen-, Brust- und Eierstockkrebs, Glioblastomen, chronischen myeloischen Leukämien, akuten lymphoblastischen Leukämien, Prostatakrebs, Bauchspeicheldrüsenkrebs und Kolonkrebs, metastatischen Melanomen, Schilddrüsentumoren und Nierenkarzinomen.

29. Verwendung von Produkten der Formel (I) nach einem der vorhergehenden Ansprüche oder von pharmazeutisch unbedenklichen Salzen der Produkte der Formel (I) zur Herstellung eines Medikaments für die Krebschemotherapie.

30. Verwendung von Produkten der Formel (I) nach einem der vorhergehenden Ansprüche oder von pharmazeutisch unbedenklichen Salzen der Produkte der Formel (I) zur Herstellung von Medikamenten für die Krabschemotherapie, die allein oder in Kombination verwendet werden.

31. Verwendung von Produkten der Formel (I) nach einem der vorhergehenden Ansprüche oder von pharmazeutisch unbedenklichen Salzen der Produkte der Formel (I) zur Herstellung von Medikamenten zur alleinigen Verwendung oder zur Verwendung in Kombination mit Chemotherapie oder Strahlentherapie oder alternativ dazu in Kombination mit anderen Therapeutika.

32. Verwendung von Produkten der Formel (I) nach dem vorhergehenden Anspruch, wobei es sich bei den Therapeutika um gängige Antitumormittel handeln kann.
